# EUROPEAN PATENT APPLICATION

(11) **EP 4 190 361 A1**
(43) Date of publication of application: **07.06.2023**
(21) Application number: 21851077.4
(22) Date of filing: 21.07.2021
(51) Int. Cl.: A61K 47/55, A61K 47/60, A61K 31/517, A61P 35/00

(54) **POLYETHYLENE GLYCOL CONJUGATE DRUG, AND PREPARATION METHOD THERFOR AND USE THEREOF**

(30) Priority: 28.07.2020 CN 202010738638
(71) Applicant: Chongqing Upgra Biotechnology Co., Ltd., Chongqing 401120 (CN)
(72) Inventor: LI, Gaoquan, Chongqing 401120 (CN); LIU, Nian, Chongqing 401120 (CN); PENG, Yongchen, Chongqing 401120 (CN); ZENG, Xiafan, Chongqing 401120 (CN); MEI, Gang, Chongqing 401120 (CN); GUAN, Sheng, Chongqing 401120 (CN); GAO, Yang, Chongqing 401120 (CN); YANG, Shuai, Chongqing 401120 (CN); YIN, Yifeng, Chongqing 401120 (CN); LOU, Jie, Chongqing 401120 (CN); CHEN, Huiyu, Chongqing 401120 (CN); QIAN, Kun, Chongqing 401120 (CN); WEI, Yusong, Chongqing 401120 (CN); ZHANG, Qian, Chongqing 401120 (CN); LI, Dajun, Chongqing 401120 (CN); DING, Xiaoling, Chongqing 401120 (CN); YANG, Xiangwei, Chongqing 401120 (CN); HUANG, Liqun, Chongqing 401120 (CN); LIU, Xi, Chongqing 401120 (CN); LIU, Liwei, Chongqing 401120 (CN); LI, Zhenwei, Chongqing 401120 (CN); HU, Kaixiong, Chongqing 401120 (CN); LIU, Hua, Chongqing 401120 (CN); TU, Tao, Chongqing 401120 (CN)
(74) Representative: Cabinet Nony
(86) International application number: PCT/CN2021/107625
(87) International publication number: WO 2022/022360

(57) **Abstract**

Disclosed are a polyethylene glycol conjugate drug, and a preparation method therefor and the use thereof. Specifically, the present invention relates to a polyethylene glycol conjugate drug represented by formula A or a pharmaceutically acceptable salt thereof, a method for preparing the polyethylene glycol conjugate drug or the pharmaceutically acceptable salt thereof, an intermediate for preparing the polyethylene glycol conjugate drug or the pharmaceutically acceptable salt thereof, a pharmaceutical composition comprising the polyethylene glycol conjugate drug or the pharmaceutically acceptable salt thereof, and the use of the polyethylene glycol conjugate drug or the pharmaceutically acceptable salt thereof in the preparation of a drug.

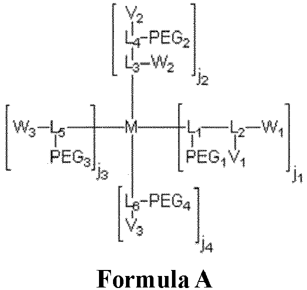

## Description

### TECHNICAL FIELD

The present invention belongs to the technical field of medicine, and in particular to a polyethylene glycol conjugated drug, preparation method thereof and use thereof.

### BACKGROUND

Polymer conjugated drug can greatly increase water solubility of drug molecules, and can prevent or reduce the formation of caking, immunogenicity and antigenicity of drugs. Most small-molecule drugs can only stay in the blood circulation for a few minutes, while polymer conjugated drugs may stay for tens or hundreds of hours or even longer, which is beneficial for the "enhanced permeability and retention" effect (i.e., the EPR effect) caused by leakage of tumor capillaries. Due to the increased hydrodynamic volume of the polymer conjugated drugs, the renal elimination of the drugs is weakened, the drugs are protected from enzymatic degradation, the half-life of the drugs in plasma is extended, and the bioavailability of the drugs is increased. Moreover, the anticancer drugs can be highly enriched in diseased organs, tissues or cells through active targeting or the EPR passive targeting, thereby greatly reducing the toxic side effects caused by small molecule anticancer drugs spreading all over the body. In addition, the polymer conjugated drugs can limit the cell absorption of drugs to the endocytic pathway, which is conducive to drug delivery to the lysosome, thereby avoiding drug resistance caused by p-glycoprotein pumping out; the polymer conjugated drugs can also stimulate or restore immune function, and this is conducive to killing cancer cells. Polyethylene glycol is the most successful carrier in the field of polymer conjugated drug nano medicine, and is called as a "gold standard" carrier. In the last 30 years, the technology of pegylated drugs has achieved tremendous success, and there are 17 pegylated drugs which have been approved by the FDA in USA to enter the market, and 1 pegylated drug which has been approved by the NMPA in China to enter the market. In addition, nearly 40 new clinical drugs are in the first-phase, second-phase, third-phase clinical trials or in the NDA phase, wherein half of the new clinical drugs are pegylated small-molecule drugs.

Using small molecules such as folic acid (FA) and RGD short peptides to create active targeting polymer conjugated drugs is a popular strategy of targeting the drugs to tumor cells at present. Small molecule targeting ligands are superior to antibodies and other ligands because of their advantages of low immunogenicity, good storage stability, small size, easy modification, easy conjugation with polymer carriers, good compatibility with various organic and water solvents, and low cost.

Folic acid (FA) is the most widely studied small molecular targeting ligand at present. It is a vitamin that can specifically bind to FA receptors on the cell surface. The FA receptors cannot enter healthy cells during circulation, but they are overexpressed in many cancer cells, such as ovarian cancer cell, lung cancer cell, breast cancer cell, kidney cancer cell, endometrial cancer cell and colon cancer cell, etc. As an active targeting ligand, FA has a unique advantage, and it has a high binding affinity to the FA receptors (K_{d}~10⁻¹⁰M). Because of its small size and simple structure, the interference of FA to pharmacokinetics is negligible. In addition, the active carboxyl group in the FA structure is highly reactive and easily conjugated with polymer carriers containing hydroxyl group or amino group, and this conjugation will not damage the inherent activity of FA.

Certain short peptides are able to specifically target cancer cells or tumor vascular endothelial cells, and some of these peptides, called cell-targeting peptides, exhibit a high degree of specificity and affinity to the corresponding target cells by interacting with the receptors. Cell-targeting peptides have been used in various therapeutic assays, and the most widely studied sequences is Arg-Gly-Asp (RGD), which was the first homing peptide discovered. RGD can bind to upregulated integrins in tumor cells and vascular endothelial cells, and an RGD-mediated drug delivery system can improve the efficiency of target delivery in chemotherapy. The recently developed internalized RGD peptide (iRGD, CRGDK/RGPD/EC), which is a tumor-targeting and cell-penetrating cyclic peptide, can increase the capacity of conjugates to enter extravascular tumor tissues in a tumor-specific manner depending on neuropeptide-1. Compared with conventional cyclic peptides, iRGD-regulated polymer conjugated drugs can significantly improve the efficacy of tumor therapy by enhancing drug enrichment and penetration at a tumor site.

### SUMMARY

The present invention is intended to solve one of the technical problems in the related technologies to at least some extent. In view of this, the present invention provides a polyethylene glycol conjugated drug with excellent tumor inhibition activity. In addition, the inventors graft small molecule targeting ligands (such as FA and iRGD peptide) and anticancer drugs on the same polyethylene glycol polymeric carrier at the same time through a special chemical synthesis method to improve the efficacy of polyethylene glycol conjugated drugs. Through the preparation method of the present invention, the polyethylene glycol conjugated drug of the present invention can be prepared efficiently and conveniently.

In view of this, in the first aspect of the present invention, the present invention provides a polyethylene glycol conjugated drug of formula (A) or a pharmaceutically acceptable salt thereof, 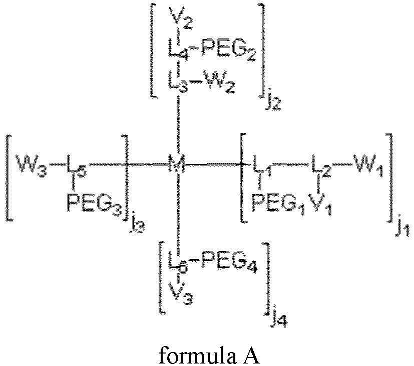 wherein:
M is selected from 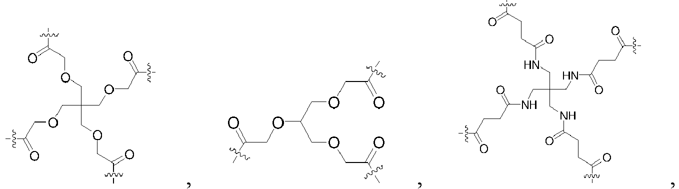 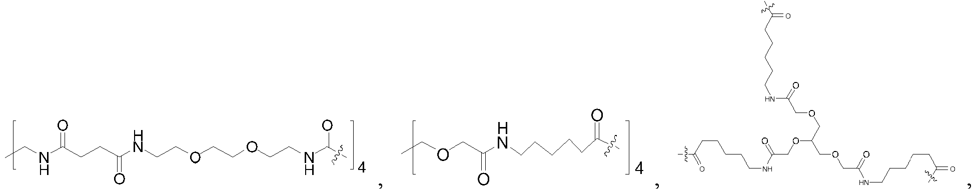 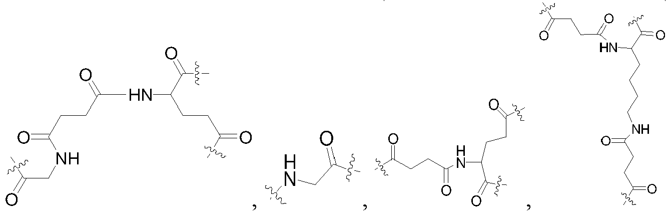 or PEGₘ; PEGₘ is a single-arm or multi-arm (such as four-arm, eight-arm, preferably four-arm) polyethylene glycol segment; PEGₘ is connected to L₁, L₃, L₅ or L₆ through a carbonyl group; the number-average molecular weight of PEGₘ is 5k-40k, preferably 5k-10k or 10k-40k, more preferably 5k or 10k;
j₁, j₂, j₃ and j₄ each independently are 0, 1, 2, 3, 4, 5 or 6, preferably 0, 1, 2, 3 or 4, and j₁, j₂, j₃ and j₄ are not 0 at the same time;
PEG₁, PEG₂, PEG₃ and PEG₄ are single-arm polyethylene glycol segments; PEG₁, PEG₂, PEG₃ and PEG₄ are connected to L₁, L₄, L₅ and L₆ through a carbonyl group respectively; each of the number-average molecular weight of PEG₁, PEG₂, PEG₃ and PEG₄ independently is 5k-40k, preferably 5k-10k or 10k-40k, more preferably 5k, 10k or 40k;
L₁, L₂, L₃, L₄, L₅ and L₆ each independently are a direct bond, 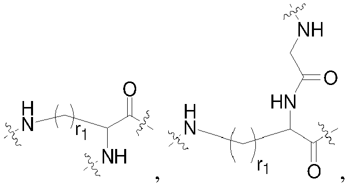 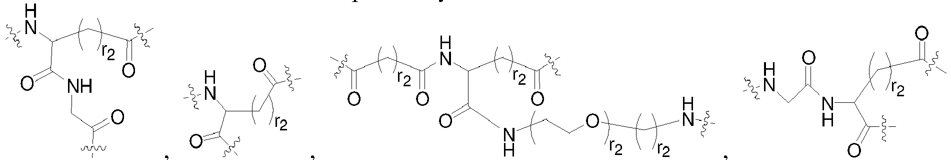 or 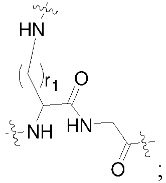 L₁, L₂, L₃, L₄, L₅ and L₆ are not direct bonds at the same time, and when L₆ is a direct bond, PEG₄ does not exist, and V3 is directly connected to M;
each r₁ independently is 1, 2, 3, 4, 5 or 6, each r₁ independently is preferably 1, 2, 3 or 4, each r₁ independently is more preferably 3 or 4,
each r₂ independentlyis 1, 2, 3, 4, 5 or 6, each r₂ independently is preferably 1, 2, 3 or 4, each r₂ independently is more preferably 1 or 2;
V₁ is -L_{1V}-T₁ or 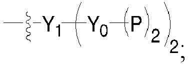
V₂ is -L_{2V}-T₂;
V₃ is -P₃, 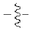 Y₀-(P₃)₂ or 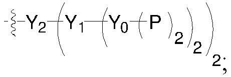
P is -Lv-T;
P₃ is -L_{3V}-T₃;
L_{1V}, L_{2V} and L_{3V} each independently are 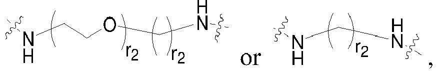 each r₂ independently is 1, 2, 3, 4, 5 or 6, each r₂ independently is preferably 1, 2, 3 or 4, each r₂ independently is more preferably 1 or 2;
T₁, T₂ and T₃ are 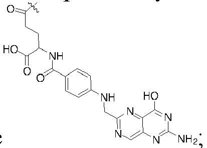
Y₂, Y₁ and Y₀ each independently are 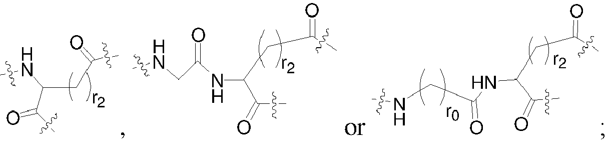 r₀ is 1, 2, 3, 4, 5 or 6, preferably 3, 4, 5 or 6, and more preferably 5 or 6; each r₂ independently is 1, 2, 3, 4, 5 or 6, each r₂ independently is preferably 1, 2, 3 or 4, each r₂ independently is more preferably 1 or 2;
L_{V} is 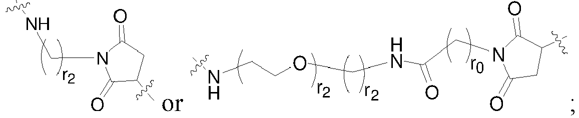 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
T is 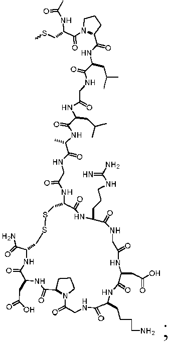
W₁, W₂ and W₃ each independently are 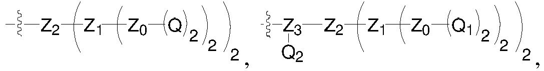 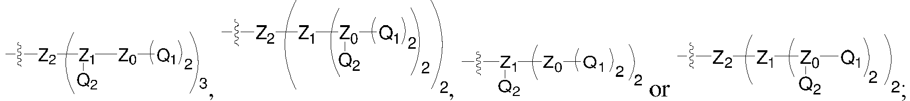
Z₃, Z₂, Z₁ and Z₀ each independently are 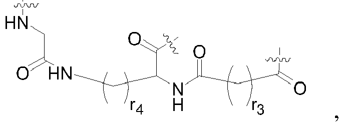 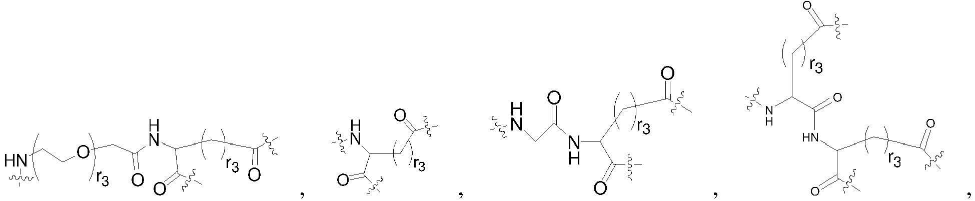 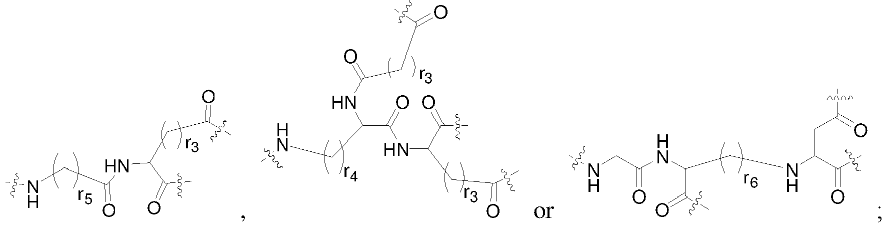 each r₃ independently is 1, 2, 3, 4, 5 or 6, each r₃ independently is preferably 1, 2, 3 or 4, each r₃ independently is more preferably 1 or 2; each r₄ independently is 1, 2, 3, 4, 5 or 6, each r₄ independently is preferably 1, 2, 3 or 4, each r₄ independently is more preferably 3 or 4; r₅ is 1, 2, 3, 4, 5 or 6, preferably 3, 4, 5 or 6, more preferably 5 or 6; r₆ is 1, 2, 3, 4, 5 or 6, preferably 2, 3, 4 or 5, more preferably 3 or 4;
Q is -N-AC;
Q1 is -N1-AC1;
Q2 is -N2-AC2;
N, N1 and N2 each independently are 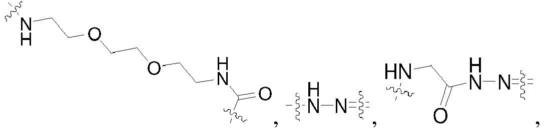 G or GFLG;
AC, AC1 and AC2 are anti-cancer drug molecules; preferably, AC, AC1 and AC2 each independently are SB7, NPB, SN38, LPT, PCB, DOX, PTX or AXT.

In some embodiments, L₁, L₂, L₃, L₄, L₅ and L₆ each independently are a direct bond, 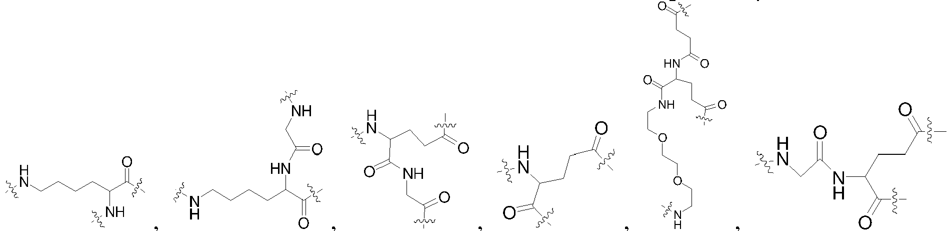 or 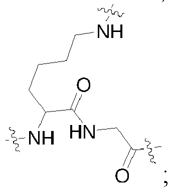 L₁, L₂, L₃, L₄, L₅ and L₆ are not direct bonds at the same time, and when L₆ is a direct bond, PEG₄ does not exist, and V3 is directly connected to M.

In some embodiments, L₁ is 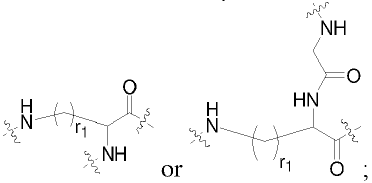 each r₁ independently is 1, 2, 3, 4, 5 or 6, each r₁ independently is preferably 1, 2, 3 or 4, each r₁ independently is more preferably 3 or 4.

In some embodiments, L₁ is

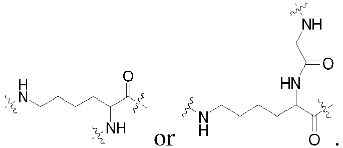

In some embodiments, L₂ is 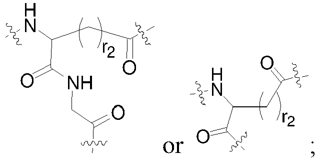 each r₂ independently is 1, 2, 3, 4, 5 or 6, each r₂ independently is preferably 1, 2, 3 or 4, each r₂ independently is more preferably 1 or 2.

In some embodiments, L₂ is

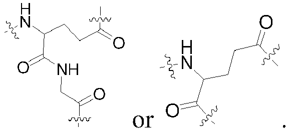

In some embodiments, L₃ is 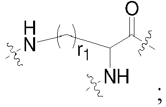 r₁ is 1, 2, 3, 4, 5 or 6, preferably 1, 2, 3 or 4, more preferably 3 or 4.

In some embodiments, L₃ is

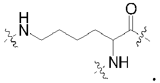

In some embodiments, L₄ is 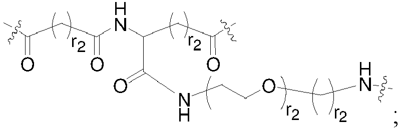 each r₂ independently is 1, 2, 3, 4, 5 or 6, each r₂ independently is preferably 1, 2, 3 or 4, each r₂ independently is more preferably 1 or 2.

In some embodiments, L₄ is

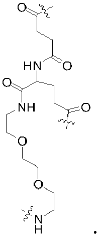

In some embodiments, L₅ is 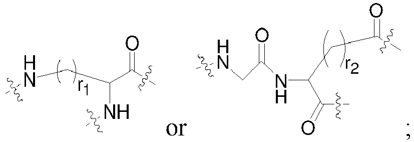 r₁ is 1, 2, 3, 4, 5 or 6, preferably 1, 2, 3 or 4, more preferably 3 or 4; r₂ is 1, 2, 3, 4, 5 or 6, preferably 1, 2, 3 or 4, more preferably 1 or 2.

In some embodiments, L₅ is

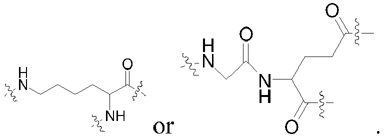

In some embodiments, L₆ is a direct bond, 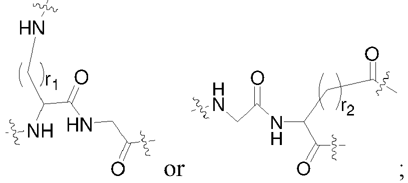 r₁ is 1, 2, 3, 4, 5 or 6, preferably 1, 2, 3 or 4, more preferably 3 or 4; r₂ is 1, 2, 3, 4, 5 or 6, preferably 1, 2, 3 or 4, more preferably 1 or 2; and when L₆ is a direct bond, PEG₄ does not exist, and V3 is directly connected to M.

In some embodiments, L₆ is a direct bond, 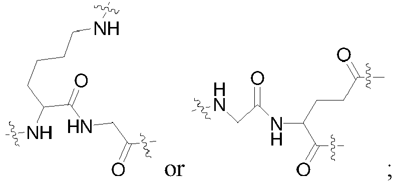 when L₆ is a direct bond, PEG₄ does not exist, and V3 is directly connected to M.

In some embodiments, L_{1V}, L_{2V} and L_{3V} each independently are

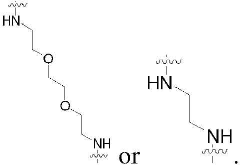

In some embodiments, Y₂, Y₁ and Y₀ each independently are 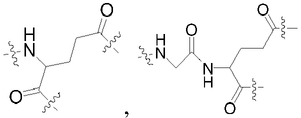 or

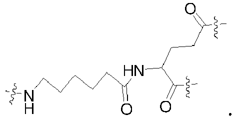

In some embodiments, L_{V} is

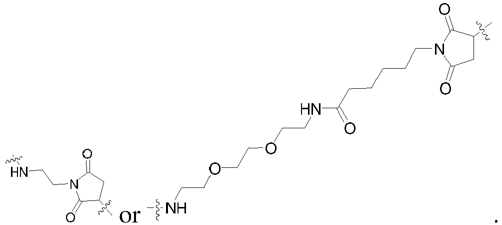

In some embodiments, Z₃, Z₂, Z₁ and Z₀ each independently are

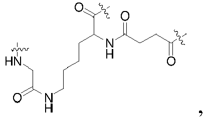

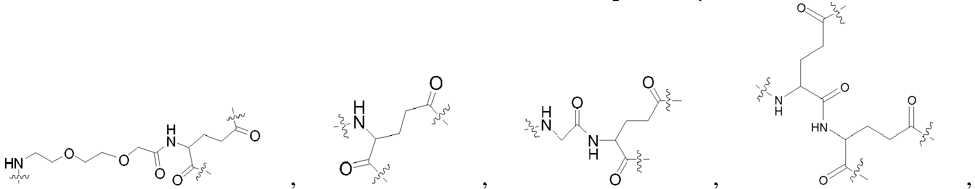

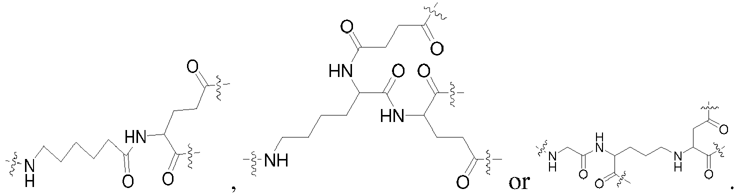

In the second aspect of the present invention, the present invention provides a polyethylene glycol conjugated drug of formula (A') or a pharmaceutically acceptable salt thereof, 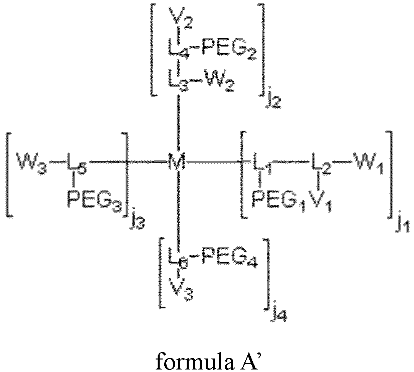 wherein:
M is selected from 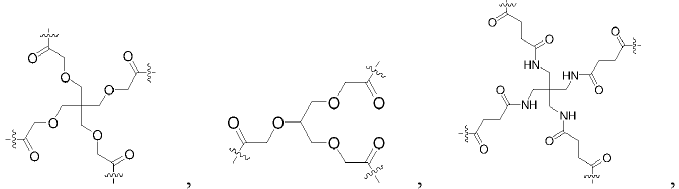 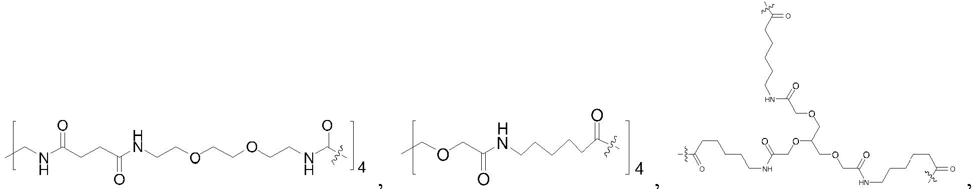 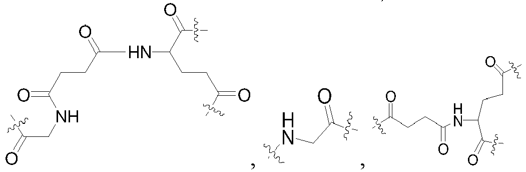 or PEGₘ; PEGₘ is a single-arm or multi-arm (such as four-arm, eight-arm, preferably four-arm) polyethylene glycol segment; PEGₘ is connected to L₁, L₃, L₅ or L₆ through a carbonyl group; the number-average molecular weight of PEGₘ is 5k-40k, preferably 5k-10k or 10k-40k, more preferably 5k or 10k;
j₁, j₂, j₃ and j₄ each independently are 0, 1, 2, 3, 4, 5 or 6, preferably 0, 1, 2, 3 or 4, and j₁, j₂, j₃ and j₄ are not 0 at the same time;
PEG₁, PEG₂, PEG₃ and PEG₄ are single-arm polyethylene glycol segments; PEG₁, PEG₂, PEG₃ and PEG₄ are connected to L₁, L₄, L₅ and L₆ through a carbonyl group respectively; each of the number-average molecular weight of PEG₁, PEG₂, PEG₃ and PEG₄ independently is 5k-40k, preferably 5k-10k or 10k-40k. more preferably 5k, 10k or 40k;
L₁, L₂, L₃, L₄, L₅ and L₆ each independently are a direct bond, 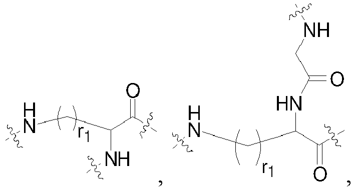 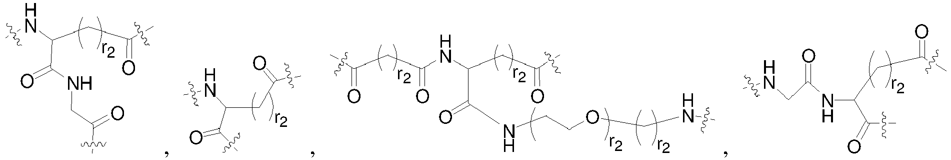 or 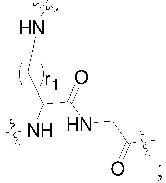 L₁, L₂, L₃, L₄, L₅ and L₆ are not direct bonds at the same time, and when L₆ is a direct bond, PEG₄ does not exist, and V3 is directly connected to M;
each r₁ independently is 1, 2, 3, 4, 5 or 6, each r₁ independently is preferably 1, 2, 3 or 4, each r₁ independently is more preferably 3 or 4;
each r₂ independently is 1, 2, 3, 4, 5 or 6, each r₂ independently is preferably 1, 2, 3 or 4, each r₂ independently is more preferably 1 or 2;
V₁ is -L_{1V}-T₁ or 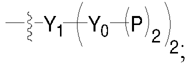
V₂ is -L_{2V}-T₂;
V₃ is -L_{3V}-T₃ or 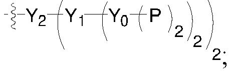
P is -Lv-T;
L_{1V}, L_{2V} and L_{3V} each independently are 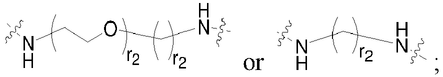 each r₂ independently is 1, 2, 3, 4, 5 or 6, each r₂ independently is preferably 1, 2, 3 or 4, each r₂ independently is more preferably 1 or 2;
T₁, T₂ and T₃ are 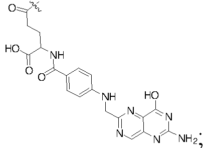
Y₂, Y₁ and Y₀ each independently are 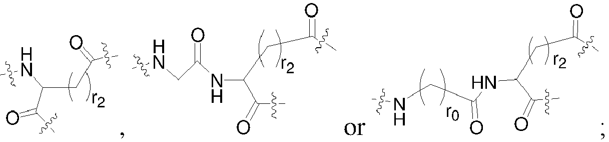 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2
L_{V} is 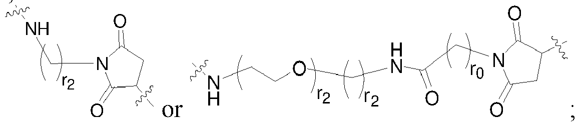 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
T is 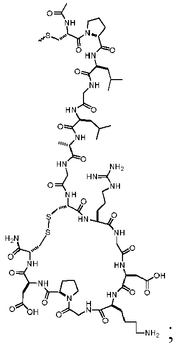
W₁, W₂ and W₃ each independently are 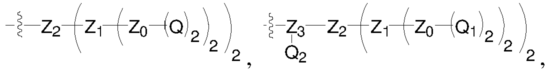 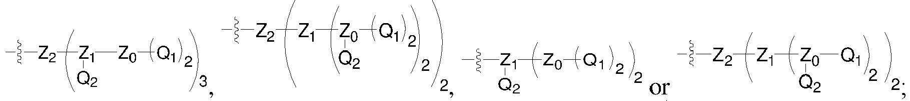
Z₃, Z₂, Z₁ and Z₀ each independently are 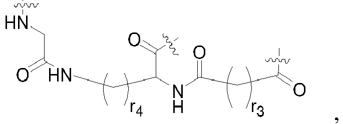 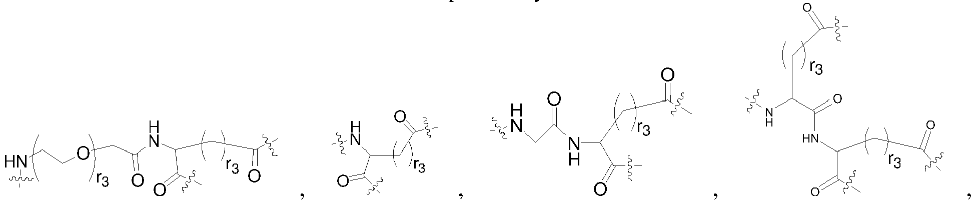 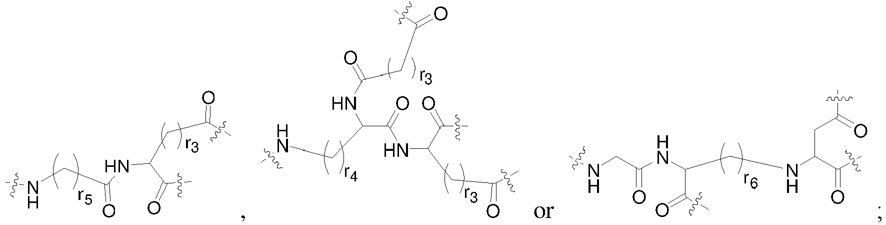 each r₃ independently is 1, 2, 3, 4, 5 or 6, each r₃ independently is preferably 1, 2, 3 or 4, each r₃ independently is more preferably 1 or 2; each r₄ independently is 1, 2, 3, 4, 5 or 6, each r₄ independently is preferably 1, 2, 3 or 4, each r₄ independently is more preferably 3 or 4; r₅ is 1, 2, 3, 4, 5 or 6, preferably 3, 4, 5 or 6, more preferably 5 or 6; r₆ is 1, 2, 3, 4, 5 or 6, preferably 2, 3, 4 or 5, more preferably 3 or 4;
Q is -N-AC;
Q1 is -N1-AC1;
Q2 is -N2-AC2;
N, N1 and N2 each independently are 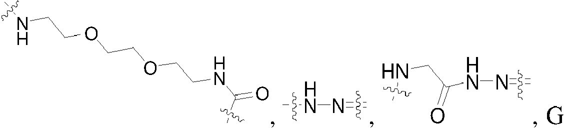 or GFLG;
AC, AC1 and AC2 are anti-cancer drug molecules, preferably, AC, AC1 and AC2 each independently are SB7, NPB, SN38, LPT, PCB, DOX, PTX or AXT.

In some embodiments, L₁, L₂, L₃, L₄, L₅ and L₆ each independently are a direct bond, 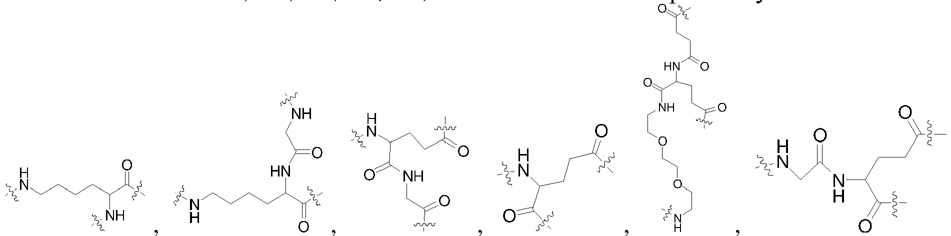 or 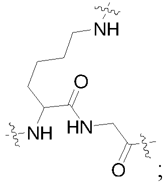 L₁, L₂, L₃, L₄, L₅ and L₆ are not direct bonds at the same time, and when L₆ is a direct bond, PEG₄ does not exist, and V3 is directly connected to M;

In some embodiments, L₁ is 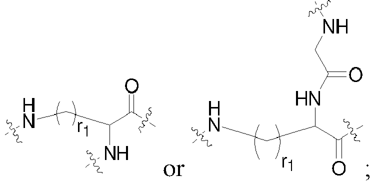 each r₁ independently is 1, 2, 3, 4, 5 or 6, each r₁ independently is preferably 1, 2, 3 or 4, each r₁ independently is more preferably 3 or 4.

In some embodiments, L₁ is

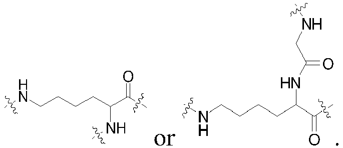

In some embodiments, L₂ is 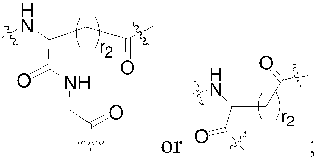 each r₂ independently is 1, 2, 3, 4, 5 or 6, each r₂ independently is preferably 1, 2, 3 or 4, each r₂ independently is more preferably 1 or 2.

In some embodiments, L₂ is

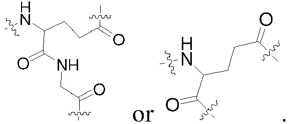

In some embodiments, L₃ is 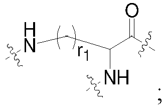 r₁ is 1, 2, 3, 4, 5 or 6, preferably 1, 2, 3 or 4, more preferably 3 or 4.

In some embodiments, L₃ is

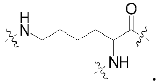

In some embodiments, L₄ is 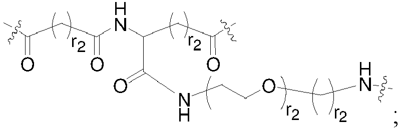 each r₂ independently is 1, 2, 3, 4, 5 or 6, each r₂ independently is preferably 1, 2, 3 or 4, each r₂ independently is more preferably 1 or 2.

In some embodiments, L₄ is

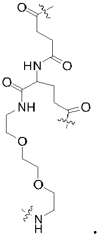

In some embodiments, L₅ is 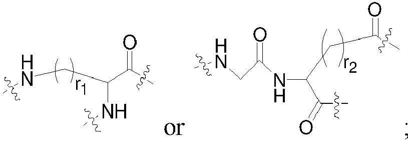 r₁ is 1, 2, 3, 4, 5 or 6, preferably 1, 2, 3 or 4, more preferably 3 or 4; r₂ is 1, 2, 3, 4, 5 or 6, preferably 1, 2, 3 or 4, more preferably 1 or 2.

In some embodiments, L₅ is

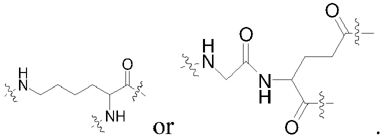

In some embodiments, L₆ is a direct bond or 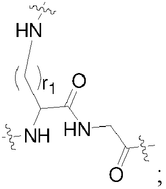 r₁ is 1, 2, 3, 4, 5 or 6, preferably 1, 2, 3 or 4, more preferably 3 or 4, and when L₆ is a direct bond, PEG₄ does not exist, and V3 is directly connected to M.

In some embodiments, L₆ is a direct bond or 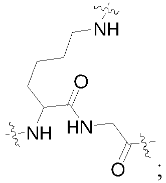 when L₆ is a direct bond, PEG₄ does not exist, and V3 is directly connected to M.

In some embodiments, L_{1V}, L_{2V} and L_{3V} each independently are 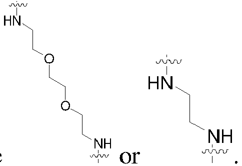 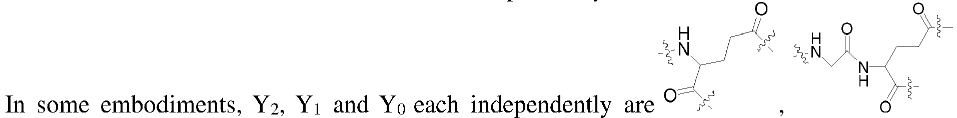 or

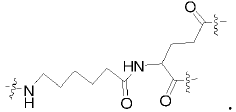

In some embodiments, L_{V} is

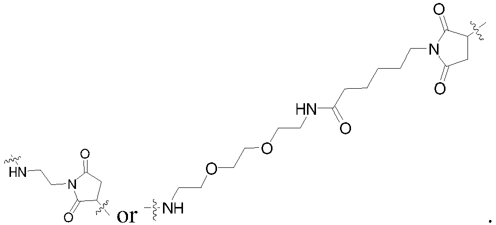

In some embodiments, Z₃, Z₂, Z₁ and Z₀ each independently are

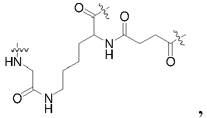

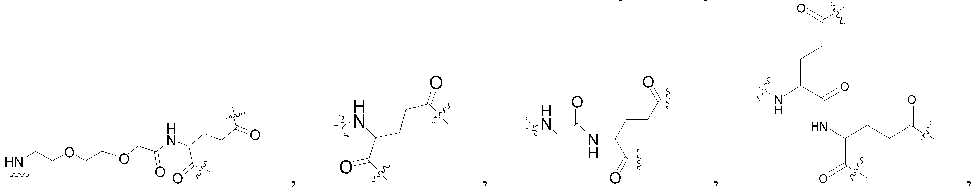

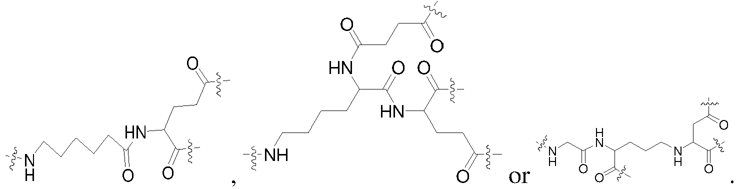

In the third aspect of the present invention, the present invention provides a polyethylene glycol conjugated drug of formula (I) or a pharmaceutically acceptable salt thereof, 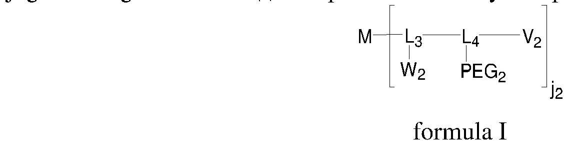 wherein:
M is selected from 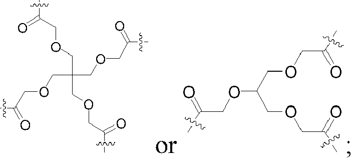 j₂ is 3 or 4;
PEG₂ is a single-arm polyethylene glycol segment; PEG₂ is connected to L₄ through a carbonyl group; the number-average molecular weight of PEG₂ is 5k-40k, preferably 5k-10k or 10k-40k, more preferably 10k;
L₃ is 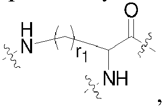 r₁ is 1, 2, 3, 4, 5 or 6, preferably 1, 2, 3 or 4, more preferably 3 or 4,
L₄ is 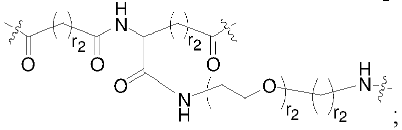 each r₂ independently is 1, 2, 3, 4, 5 or 6, each r₂ independently is preferably 1, 2, 3 or 4, each r₂ independently is more preferably 1 or 2,
V₂ is -L_{2V}-T₂,
L_{2V} is 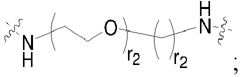 each r₂ independently is 1, 2, 3, 4, 5 or 6, each r₂ independently is preferably 1, 2, 3 or 4, each r₂ independently is more preferably 1 or 2,
T₂ is 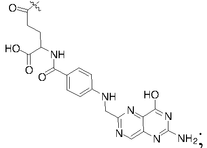
W₂ is 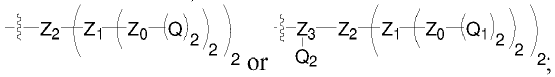
Z₃, Z₂, Z₁ and Z₀ each independently are 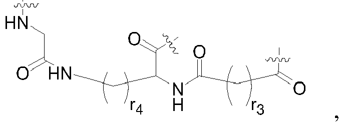 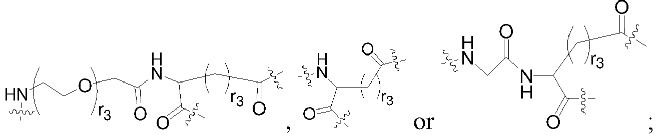 each r₃ independently is 1, 2, 3, 4, 5 or 6, each r₃ independently is preferably 1, 2, 3 or 4, each r₃ independently is more preferably 1 or 2; r₄ is 1, 2, 3, 4, 5 or 6, preferably 1, 2, 3 or 4, more preferably 3 or 4;
Q is -N-AC, Q1 is -N1-AC1, Q2 is -N2-AC2;
N, N1 and N2 each independently are G or GFLG, preferably, N, N1 and N2 are GFLG;
AC, AC1 and AC2 each independently are SB7, NPB, SN38, LPT, PCB, DOX, PTX or AXT; preferably, AC, AC1 and AC2 each independently are LPT or PCB; more preferably, AC1 is LPT, AC and AC2 is PCB.

In some embodiments, L₃ is

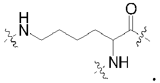

In some embodiments, L₄ is

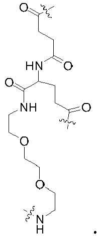

In some embodiments, L_{2V} is

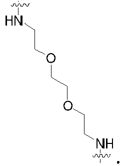

In some embodiments, Z₃ is 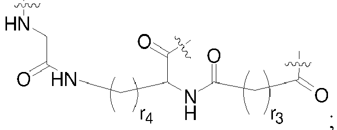 r₃ is 1 or 2, r₄ is 3 or 4.

In some embodiments Z₃ is

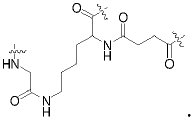

In some embodiments, Z₂ is 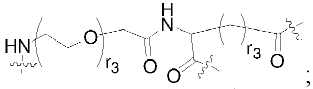 each r₃ independently is 1 or 2.

In some embodiments, Z₂ is

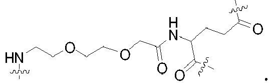

In some embodiments, Z₁ is 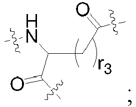 r₃ is 1 or 2.

In some embodiments, Z₁ is

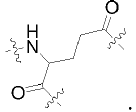

In some embodiments, Z₀ is 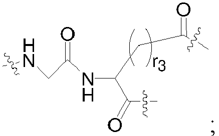 r₃ is 1 or 2.

In some embodiments, Z₀ is

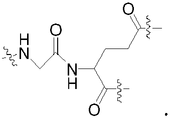

In the fourth aspect of the present invention, the present invention provides a polyethylene glycol conjugated drug of formula (II) or a pharmaceutically acceptable salt thereof, 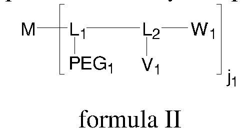 wherein:
M is selected from 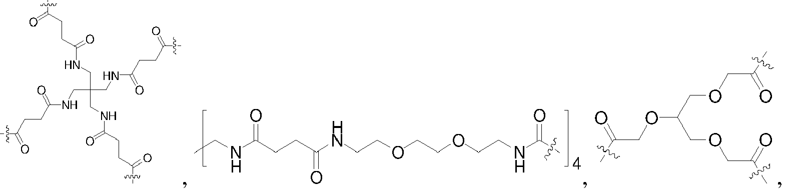 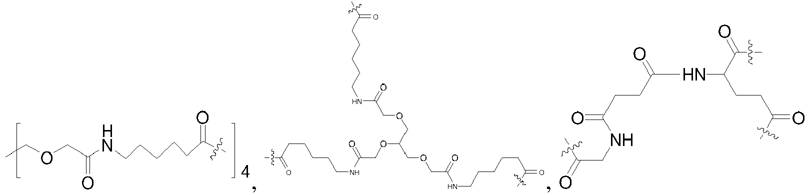 or PEGₘ; PEGₘ is a single-arm or multi-arm (such as four-arm, eight-arm, preferably four-arm) polyethylene glycol segment; PEGₘ is connected to L₁ through a carbonyl group; the number-average molecular weight of PEGₘ is 5k-40k, preferably 5k-10k or 10k-40k, more preferably 5k or 10k;
j₁ is 3 or 4;
PEG₁ is a single-arm polyethylene glycol segment; PEG₁ is connected to L₁ through a carbonyl group; the number-average molecular weight of PEG₁ is 5k-40k, preferably 5k-10k or 10k-40k, more preferably 5k, 10k or 40k;
L₁ is 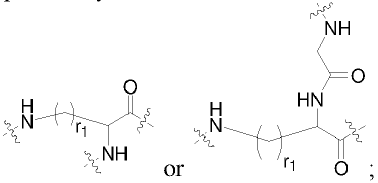 each r₁ independently is 1, 2, 3, 4, 5 or 6, each r₁ independently is preferably 1, 2, 3 or 4, each r₁ independently is more preferably 3 or 4;
L₂ is 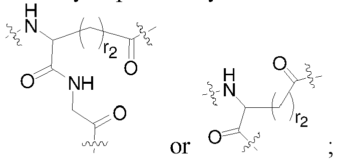 each r₂ independently is 1, 2, 3, 4, 5 or 6, each r₂ independently is preferably 1, 2, 3 or 4, each r₂ independently is more preferably 1 or 2;
V₁ is -L_{1V}-T₁,
L_{1V} is 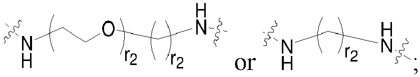 each r₂ independently is 1, 2, 3, 4, 5 or 6, each r₂ independently is preferably 1, 2, 3 or 4, each r₂ independently is more preferably 1 or 2;
T₁ is 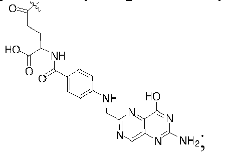
W₁ is 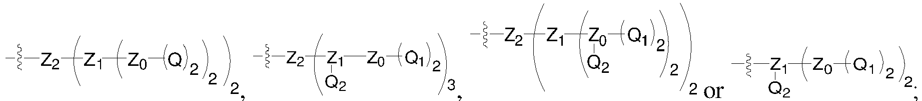
Z₂, Z₁ and Z₀ each independently are 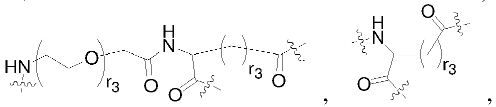 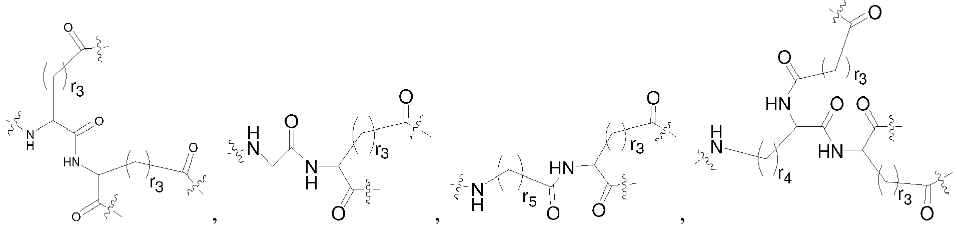 or 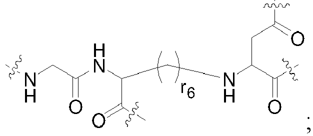 each r₃ independently is 1, 2, 3, 4, 5 or 6, each r₃ independently is preferably 1, 2, 3 or 4, each r₃ independently is more preferably 1 or 2; r₄ is 1, 2, 3, 4, 5 or 6, preferably 1, 2, 3 or 4, more preferably 3 or 4; r₅ is 1, 2, 3, 4, 5 or 6, preferably 3, 4, 5 or 6, more preferably 5 or 6; r₆ is 1, 2, 3, 4, 5 or 6, preferably 2, 3, 4 or 5, more preferably 3 or 4;
Q is -N-AC, Q1 is -N1-AC1, Q2 is -N2-AC2;
N, N1 and N2 each independently are G or GFLG;
AC, AC1 and AC2 each independently are SB7, NPB, SN38, LPT, PCB, DOX, PTX or AXT; preferably, AC, AC1 and AC2 each independently are SB7, NPB, SN38, LPT or PCB; more preferably, AC is SB7, NPB or SN38, AC1 is NPB or LPT, and AC2 is PCB.

In some embodiments, L₁ is

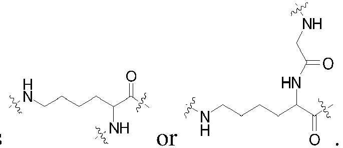

In some embodiments, L₂ is

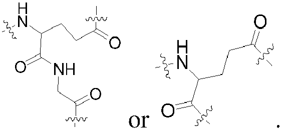

In some embodiments, Liv is

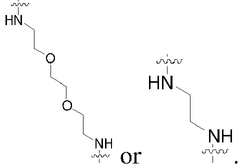

In some embodiments, Z₂ is 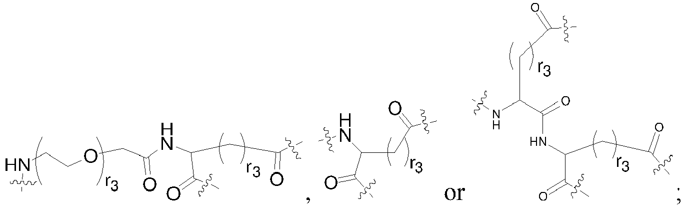 each r₃ independently is 1 or 2.

In some embodiments, Z₂ is

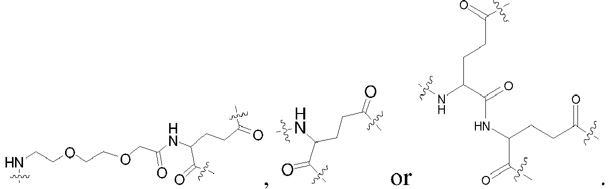

In some embodiments, Z₁ is 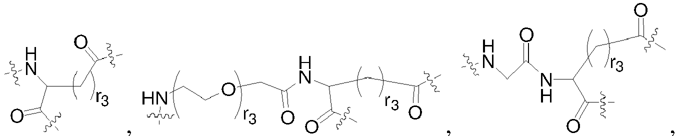 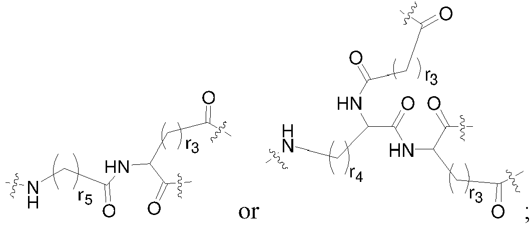 each r₃ independently is 1 or 2, r₄ is 3 or 4, r₅ is 5 or 6.

In some embodiments, Z₁ is

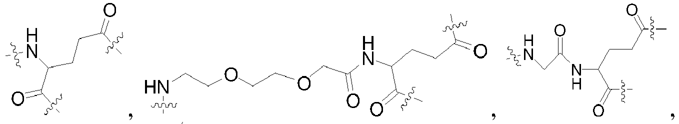

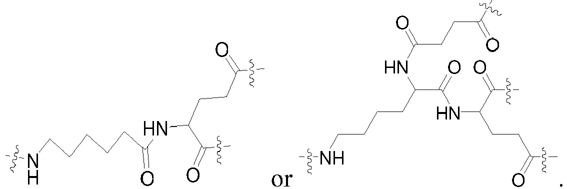

In some embodiments, Z₀ is 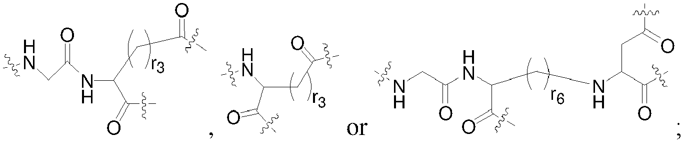 each r₃ independently is 1 or 2, r₆ is 3 or 4.

In some embodiments, Z₀ is

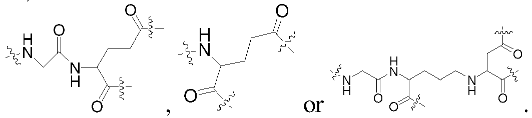

In the fifth aspect of the present invention, the present invention provides a polyethylene glycol conjugated drug of formula (III) or a pharmaceutically acceptable salt thereof, 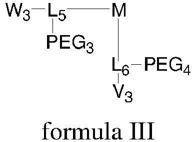 wherein:
Mis 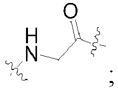 PEG₃ and PEG₄ are single-arm polyethylene glycol segments; PEG₃ and PEG₄ are connected to L₅ and L₆ through a carbonyl group respectively; each of the number-average molecular weight of PEG₃ and PEG₄ independently is 5k-40k, preferably 5k-10k or 10k-40k, more preferably 5k or 10k;
L₅ is 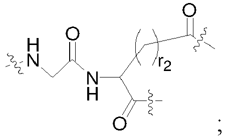 r₂ is 1, 2, 3, 4, 5 or 6, preferably 1, 2, 3 or 4, more preferably 1 or 2,
L₆ is 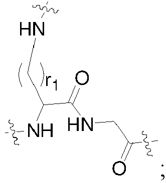 r₁ is 1, 2, 3, 4, 5 or 6, preferably 1, 2, 3 or 4, more preferably 3 or 4, V₃ is -L_{3V}-T₃;
L_{3V} is 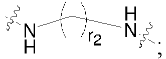 r₂ is 1, 2, 3, 4, 5 or 6, preferably 1, 2, 3 or 4, more preferably 1 or 2,
T₃ is 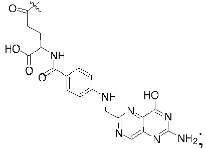
W₃ is 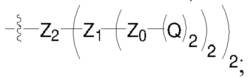
Z₂, Z₁ and Z₀ each independently are 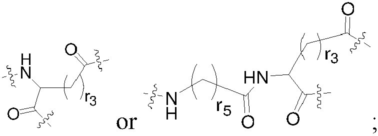 each r₃ independently is 1, 2, 3, 4, 5 or 6, each r₃ independently is preferably 1, 2, 3 or 4, each r₃ independently is more preferably 1 or 2; r₅ is 1, 2, 3, 4, 5 or 6, preferably 3, 4, 5 or 6, more preferably 5 or 6;
Q is -N-AC;
Nis G, 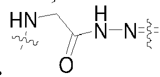 or GFLG; preferably, N is G or 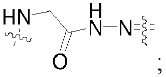
AC is SB7, NPB, SN38, LPT, PCB, DOX, PTX or AXT; preferably, AC is DOX or PTX.

In some embodiments, L₅ is

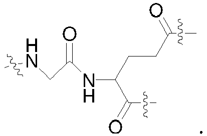

In some embodiments, L₆ is

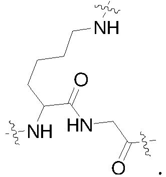

In some embodiments, L_{3V} is

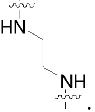

In some embodiments, Z₁ and Z₂ are 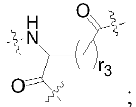 each r₃ independently is 1 or 2.

In some embodiments, Z₁ and Z₂ are

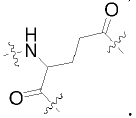

In some embodiments, Z₀ is 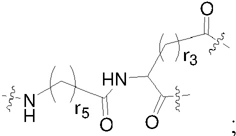 r₃ is 1 or 2, r₅ is 5 or 6.

In some embodiments, Z₀ is

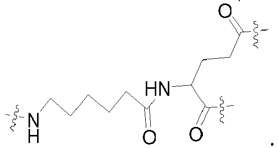

In the sixth aspect of the present invention, the present invention provides a polyethylene glycol conjugated drug of formula (IV) or a pharmaceutically acceptable salt thereof, 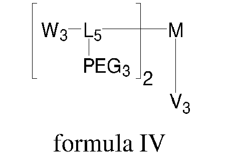 wherein:
Mis 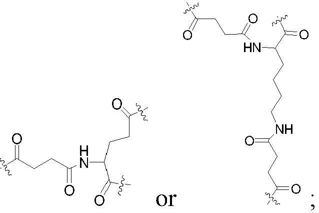
PEG₃ is a single-arm polyethylene glycol segment; PEG₃ is connected to L₅ through a carbonyl group; the number-average molecular weight of PEG₃ is 5k-40k, preferably 5k-10k or 10k-40k, more preferably 10k;
L₅ is 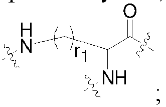 r₁ is 1, 2, 3, 4, 5 or 6, preferably 1, 2, 3 or 4, more preferably 3 or 4;
V₃ is 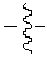 Y₀-(P₃)₂ or -P₃,
Y₀ is 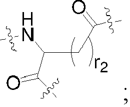 r₂ is 1, 2, 3, 4, 5 or 6, preferably 1, 2, 3 or 4, more preferably 1 or 2,
P₃ is -L_{3V}-T₃, L_{3V} is 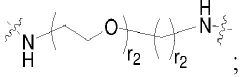 each r₂ independently is 1, 2, 3, 4, 5 or 6, each r₂ independently is preferably 1, 2, 3 or 4, each r₂ independently is more preferably 1 or 2,
T₃ is 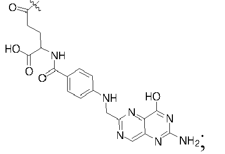
W₃ is 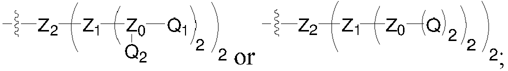
Z₂, Z₁ and Z₀ each independently are 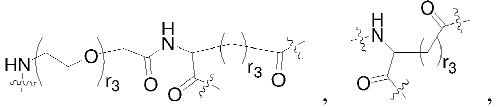 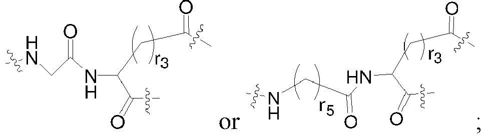 each r₃ independently is 1, 2, 3, 4, 5 or 6, each r₃ independently is preferably 1, 2, 3 or 4; each r₃ independently is more preferably 1 or 2; r₅ is 1, 2, 3, 4, 5 or 6, preferably 3, 4, 5 or 6, more preferably 5 or 6;
Q is -N-AC, Q1 is -N1-AC1, Q2 is -N2-AC2;
N, N1 and N2 each independently are G, GFLG or 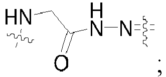 preferably, N1 and N2 are GFLG; preferably, N is G or 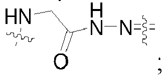
AC, AC1 and AC2 each independently are SB7, NPB, SN38, LPT, PCB, DOX, PTX or AXT; preferably, AC1 and AC2 each independently are PCB or LPT; preferably, AC is PTX or DOX.

In some embodiments, L₅ is

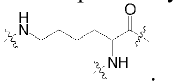

In some embodiments, Y₀ is

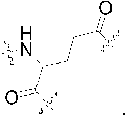

In some embodiments, L_{3V} is

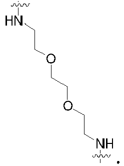

In some embodiments, Z₂ is 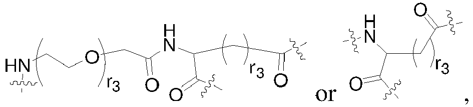 each r₃ independently is 1 or 2.

In some embodiments, Z₂ is

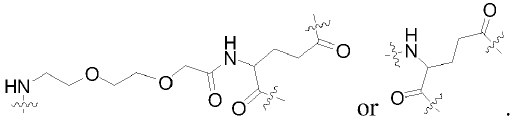

In some embodiments, Z₁ is 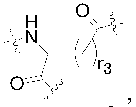 r₃ is 1 or 2.

In some embodiments, Z₁ is

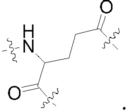

In some embodiments, Z₀ is 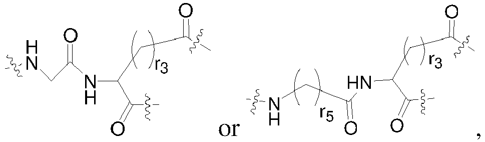 each r₃ independently is 1 or 2, r₅ is 5 or 6.

In some embodiments, Z₀ is

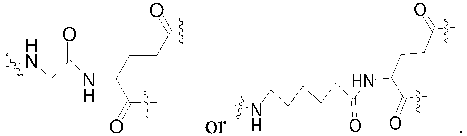

In the seventh aspect of the present invention, the present invention provides a polyethylene glycol conjugated drug of formula (IV') or a pharmaceutically acceptable salt thereof, 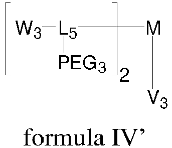 wherein:
Mis 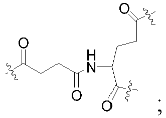
PEG₃ is a single-arm polyethylene glycol segment; PEG₃ is connected to L₅ through a carbonyl group; the number-average molecular weight of PEG₃ is 5k-40k, preferably 5k-10k or 10k-40k, more preferably 10k;
L₅ is 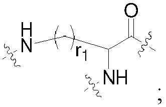 r₁ is 1, 2, 3, 4, 5 or 6, preferably 1, 2, 3 or 4, more preferably 3 or 4, V₃ is -L_{3V}-T₃,
L_{3V} is 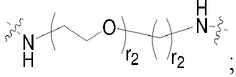 each r₂ independently is 1, 2, 3, 4, 5 or 6, each r₂ independently is preferably 1, 2, 3 or 4, each r₂ independently is more preferably 1 or 2;
T₃ is 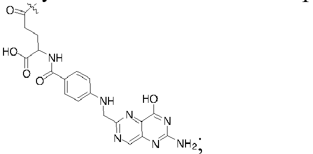
W₃ is 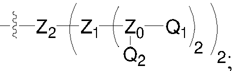
Z₂, Z₁ and Z₀ each independently are 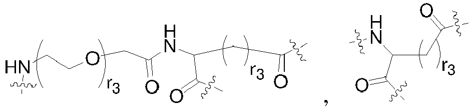 or 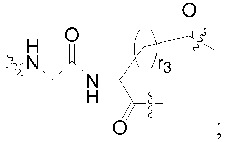 each r₃ independently is 1, 2, 3, 4, 5 or 6; each r₃ independently is preferably 1, 2, 3 or 4, each r₃ independently is more preferably 1 or 2;
Q1 is -N1-AC1, Q2 is -N2-AC2;
N1 and N2 each independently are G or GFLG, preferably, N1 and N2 are GFLG;
AC1 and AC2 each independently are SB7, NPB, SN38, LPT, PCB, DOX, PTX or AXT, preferably, AC1 and AC2 each independently are PCB or LPT.

In some embodiments, L₅ is

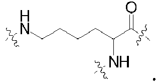

In some embodiments, L_{3V} is

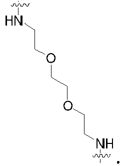

In some embodiments, Z₂ is 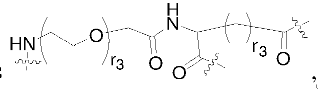 each r₃ independently is 1 or 2.

In some embodiments, Z₂ is

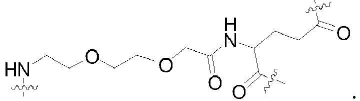

In some embodiments, Z₁ is 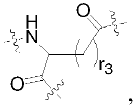 r₃ is 1 or 2.

In some embodiments, Z₁ is

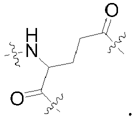

In some embodiments, Z₀ is 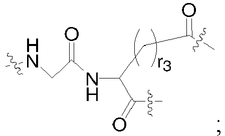 r₃ is 1 or 2.

In some embodiments, Z₀ is

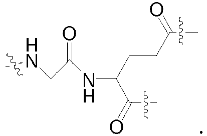

In the eighth aspect of the present invention, the present invention provides a polyethylene glycol conjugated drug of formula (V) or a pharmaceutically acceptable salt thereof, 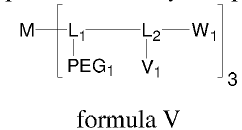 wherein:
Mis 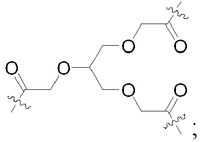 PEG₁ is a single-arm polyethylene glycol segment; PEG₁ is connected to L₁ through a carbonyl group; the number-average molecular weight of PEG₁ is 5k-40k, preferably 5k-10k or 10k-40k,
more preferably 10k;
L₁ is 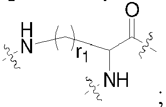 r₁ is 1, 2, 3 4, 5 or 6, preferably 1, 2, 3 or 4, more preferably 3 or 4;
L₂ is 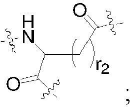 r₂ is 1, 2, 3, 4, 5 or 6, preferably 1, 2, 3 or 4, more preferably 1 or 2, V₁ is 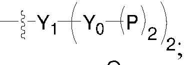
Y₁, Y₀ is 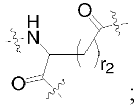 each r₂ independently is 1, 2, 3, 4, 5 or 6, each r₂ independently is preferably 1, 2, 3 or 4, each r₂ independently is more preferably 1 or 2,
P is -Lv-T,
L_{V} is 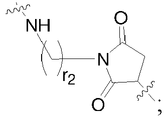 r₂ is 1, 2, 3, 4, 5 or 6, preferably 1, 2, 3 or 4, more preferably 1 or 2,
T is 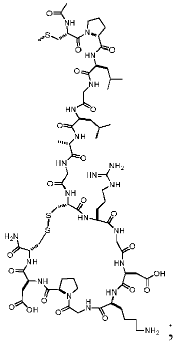
W₁ is 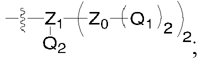
Z₁ and Z₀ each independently are 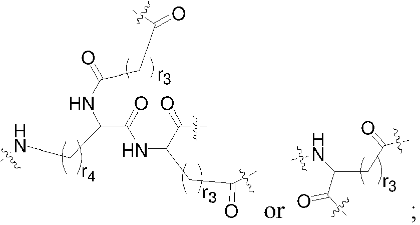 each r₃ independently is 1, 2, 3, 4, 5 or 6, each r₃ independently is preferably 1, 2, 3 or 4, each r₃ independently is more preferably 1 or 2; r₄ is 1, 2, 3, 4, 5 or 6, preferably 1, 2, 3 or 4, more preferably 3 or 4,
Q1 is -N1-AC1, Q2 is -N2-AC2;
N1 and N2 each independently are G or GFLG, preferably, N1 and N2 are GFLG;
AC1 andAC2 each independently are SB7, NPB, SN38, LPT, PCB, DOX, PTX or AXT; preferably,
AC1 and AC2 each independently are PCB or LPT; more preferably, AC1 is LPT, AC2 is PCB.

In some embodiments, L₁ is

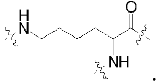

In some embodiments, L₂ is

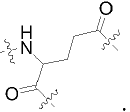

In some embodiments, Y₁ and Y₀ are

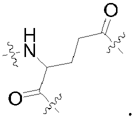

In some embodiments, L_{V} is

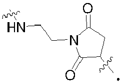

In some embodiments, Z₁ is 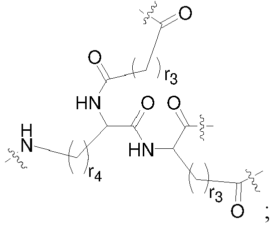 each r₃ independently is 1 or 2, r₄ is 3 or 4.

In some embodiments, Z₁ is

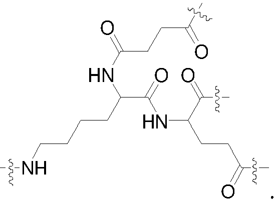

In some embodiments, Z₀ is 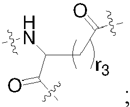 r₃ is 1 or 2.

In some embodiments, Z₀ is

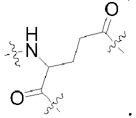

In the ninth aspect of the present invention, the present invention provides a polyethylene glycol conjugated drug of formula (VI) or a pharmaceutically acceptable salt thereof, 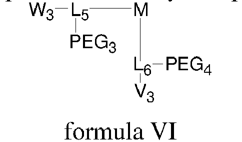 wherein:
Mis 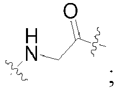 PEG₃ and PEG₄ are a single-arm polyethylene glycol segment; PEG₃ and PEG₄ are connected to L₅ and L₆ through a carbonyl group respectively; each of the number-average molecular weight of PEG₃ and PEG₄ independently is 5k-40k, preferably 5k-10k or 10k-40k, more preferably 10k;
L₅ and L₆ each independently are 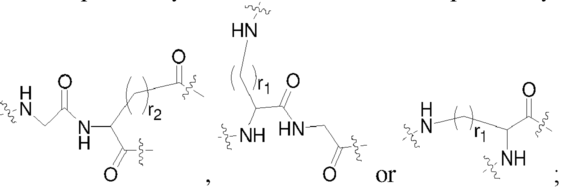 each r₁ independently is 1, 2, 3, 4, 5 or 6, each r₁ independently is preferably 1, 2, 3 or 4, each r₁ independently is more preferably 3 or 4; r₂ is 1, 2, 3, 4, 5 or 6, preferably 1, 2, 3 or 4, more preferably 1 or 2,
V₃ is 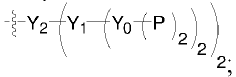
Y₂, Y₁ and Y₀ each independently are 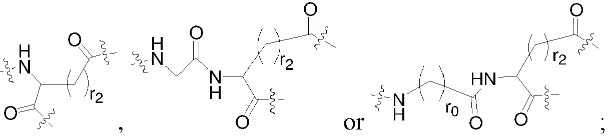 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
P is -L_{V}-T;
L_{V} is 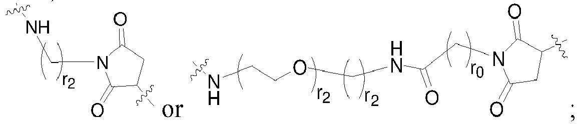 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
T is 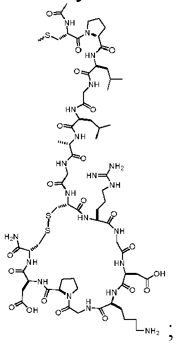
W₃ is 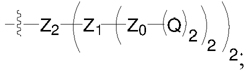
Z₂, Z₁ and Z₀ each independently are 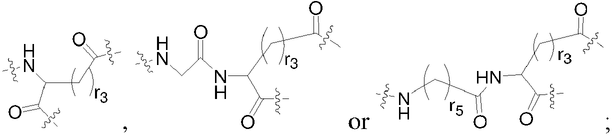 each r₃ independently is 1, 2, 3, 4, 5 or 6, each r₃ independently is preferably 1, 2, 3 or 4, each r₃ independently is more preferably 1 or 2; r₅ is 1, 2, 3, 4, 5 or 6, preferably 3, 4, 5 or 6, more preferably 5 or 6;
Q is -N-AC;
N is 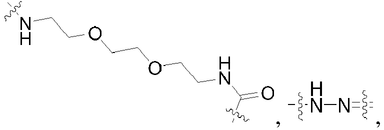 G or GFLG;
AC is SB7, NPB, SN38, LPT, PCB, DOX, PTX or AXT; preferably, AC is SB7, LPT, PTX, DOX or AXT.

In some embodiments, L₅ and L₆ each independently are

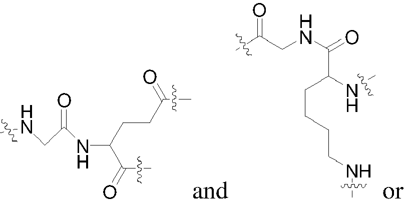

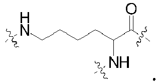

In some embodiments, Y₂ and Y₁ are 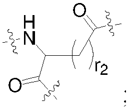 each r₂ independently is 1 or 2.

In some embodiments, Y₂ and Y₁ are

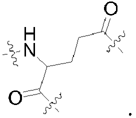

In some embodiments, Y₀ is 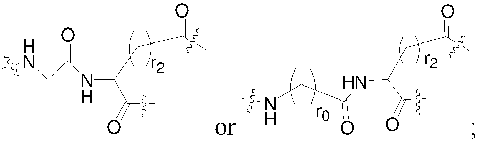 r₀ is 5 or 6, each r₂ independently is 1 or 2.

In some embodiments, Y₀ is

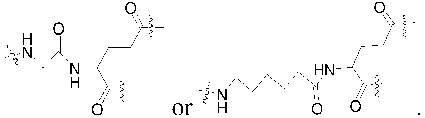

In some embodiments, L_{V} is

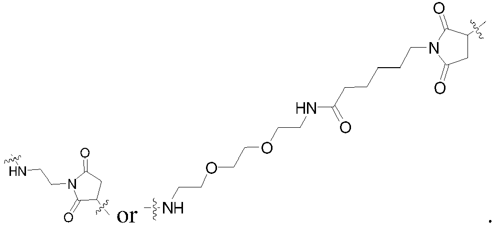

In some embodiments, Z₂ is 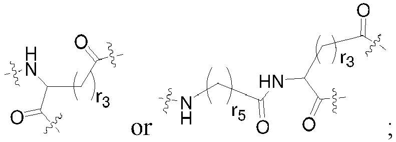 each r₃ independently is 1 or 2, rs is 5 or 6.

In some embodiments, Z₂ is

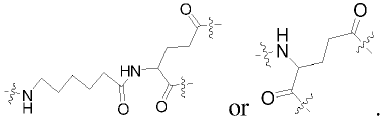

In some embodiments, Z₁ is 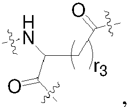 r₃ is 1 or 2.

In some embodiments, Z₁ is

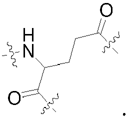

In some embodiments, Z₀ is 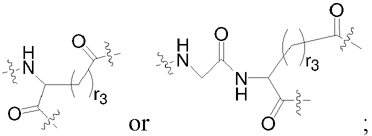 each r₃ independently is 1 or 2.

In some embodiments, Z₀ is

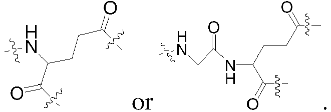

In the tenth aspect of the present invention, the present invention provides a polyethylene glycol conjugated drug of formula (VI') or a pharmaceutically acceptable salt thereof, 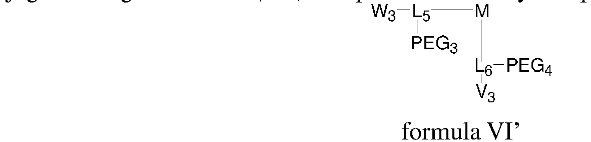 wherein:
Mis 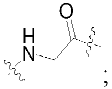 PEG₃ and PEG₄ are single-arm polyethylene glycol segments; PEG₃ and PEG₄ are connected to L₅ and L₆ through a carbonyl group respectively; each of the number-average molecular weight of PEG₃ and PEG₄ independently is 5k-40k, preferably 5k-10k or 10k-40k, more preferably 10k;
L₅ and L₆ each independently are 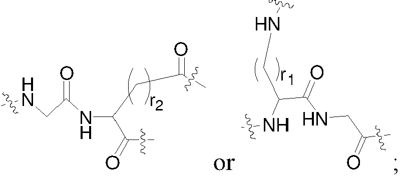 r₁ is 1, 2, 3, 4, 5 or 6, preferably 1, 2, 3 or 4, more preferably 3 or 4; r₂ is 1, 2, 3, 4, 5 or 6, preferably 1, 2, 3 or 4, more preferably 1 or 2;
V₃ is 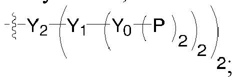
Y₂, Y₁ and Y₀ each independently are 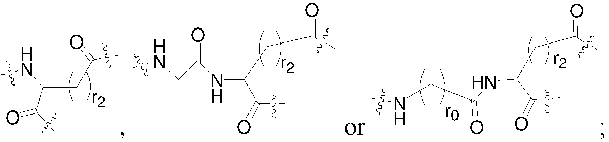 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
P is -Lv-T;
L_{V} is 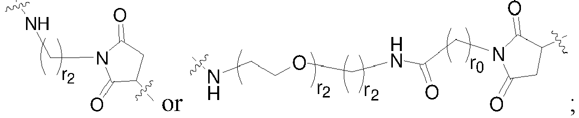 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
T is 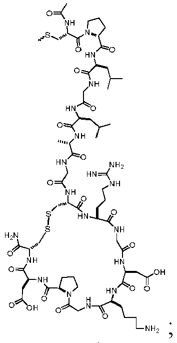
W₃ is 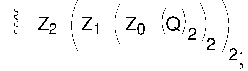
Z₂, Z₁ and Z₀ each independently are or 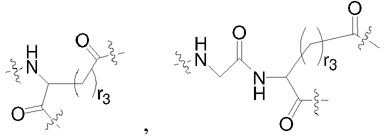 or 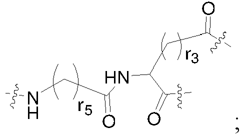 each r₃ independently is 1, 2, 3, 4, 5 or 6, each r₃ independently is preferably 1, 2, 3 or 4, each r₃ independently is more preferably 1 or 2; r₅ is 1, 2, 3, 4, 5 or 6, preferably 3, 4, 5 or 6, more preferably 5 or 6;
Q is -N-AC;
N is 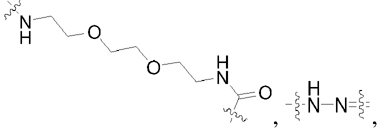 G or GFLG;
AC is SB7, NPB, SN38, LPT, PCB, DOX, PTX or AXT; preferably, AC is SB7, LPT, PTX, DOX or AXT; or preferably, AC is LPT, PTX, DOX or AXT.

In some embodiments, L₅ and L₆ each independently are

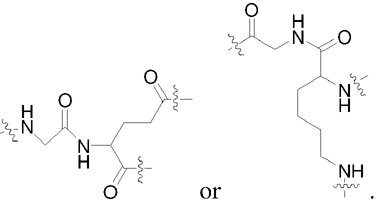

In some embodiments Y₂ and Y₁ are 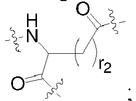 each r₂ independently is 1 or 2.

In some embodiments, Y₂ and Y₁ are

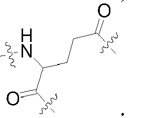

In some embodiments, Y₀ is 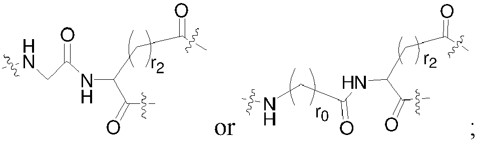 r₀ is 5 or 6; each r₂ independently is 1 or 2.

In some embodiments, Y₀ is

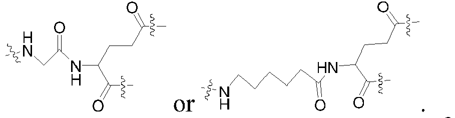

In some embodiments, L_{V} is

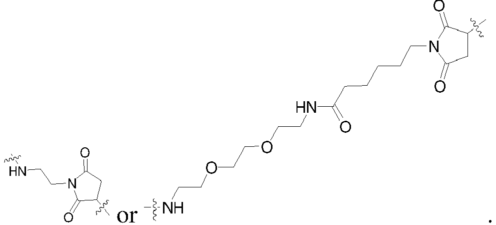

In some embodiments, Z₂ is 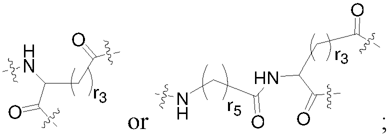 each r₃ independently is 1 or 2, r₅ is 5 or 6.

In some embodiments, Z₂ is

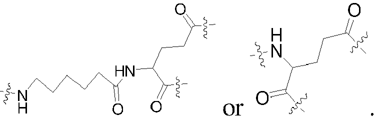

In some embodiments, Z₁ is 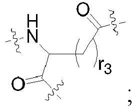 r₃ is 1 or 2.

In some embodiments, Z₁ is

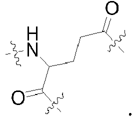

In some embodiments, Z₀ is 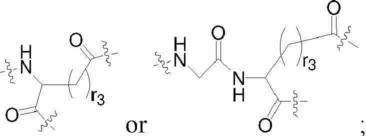 each r₃ independently is 1 or 2.

In some embodiments, Z₀ is

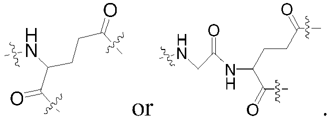

In the eleventh aspect of the present invention, the present invention provides a polyethylene glycol conjugated drug or a pharmaceutically acceptable salt thereof, wherein the polyethylene glycol conjugated drug is selected from:

| | | | | |
|---|---|---|---|---|
| | | | | |

| Number | | Structure | | |
|---|---|---|---|---|
| 37-184 | | 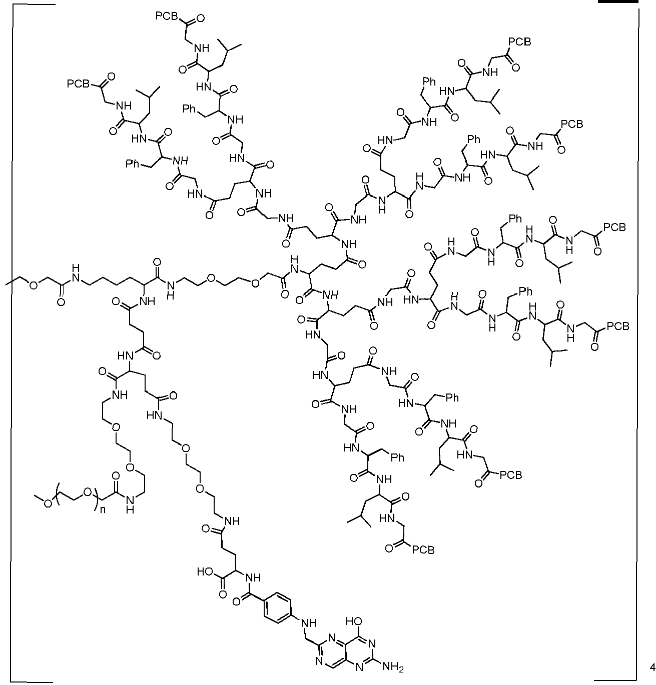 | | |
| | | wherein the number-average molecular weight of | 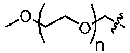 | is 10k; |
| 36-257 | | 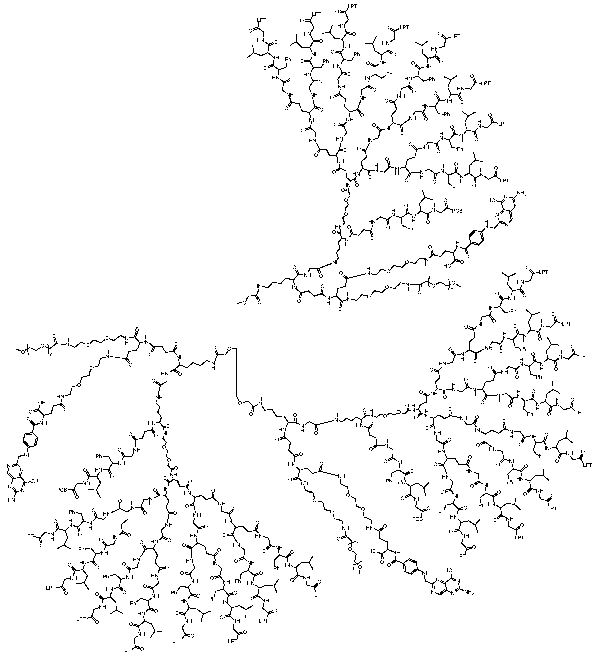 | | |
| | | wherein the number-average molecular weight of | 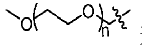 | is 10k; |
| 43-154 | | | | |
| | | 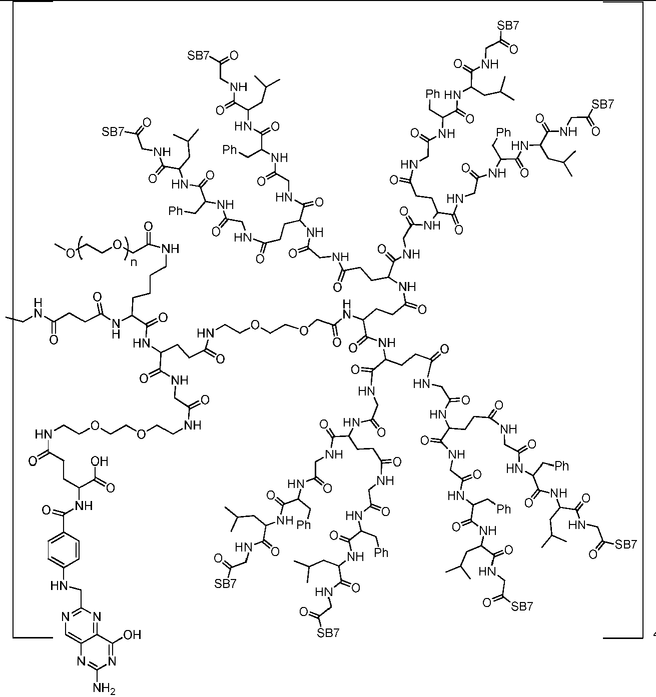 | | |
| | | wherein the number-average molecular weight of | 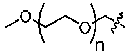 | is 10k; |
| 37-221 | | 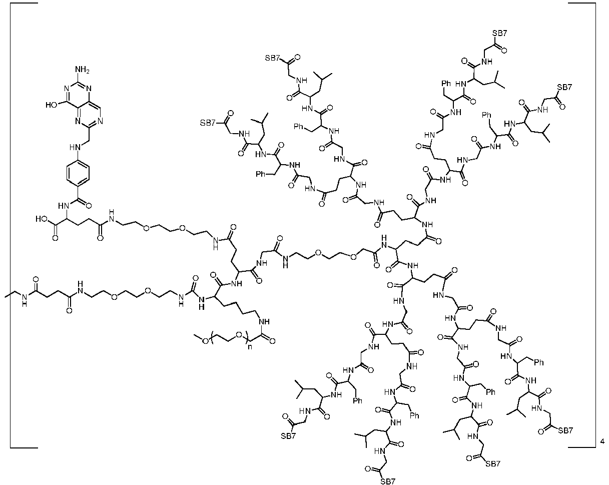 | | |
| | | wherein the number-average molecular weight of | 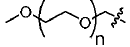 | is 10k; |
| 44-174 | | 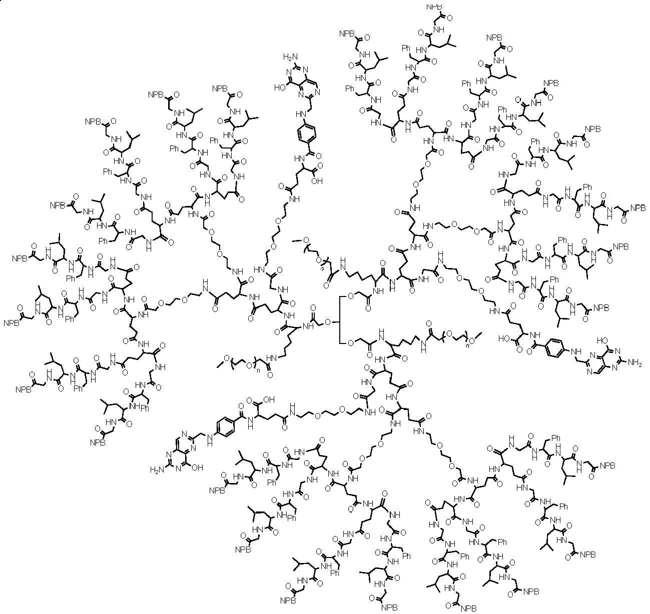 | | |
| | | wherein the number-average molecular weight of | 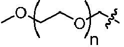 | is 10k; |
| 49-199 | | 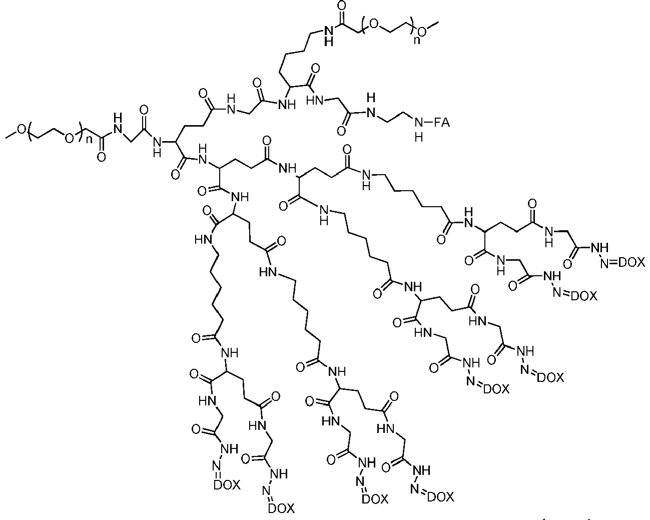 | | |
| | | wherein the number-average molecular weight of | 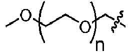 | is 5k; |
| 47-122 | | 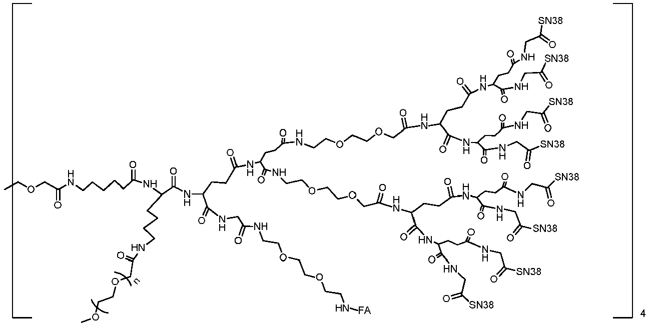 | | |
| | | wherein the number-average molecular weight of | 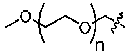 | is 5k; |
| 39-226 | | 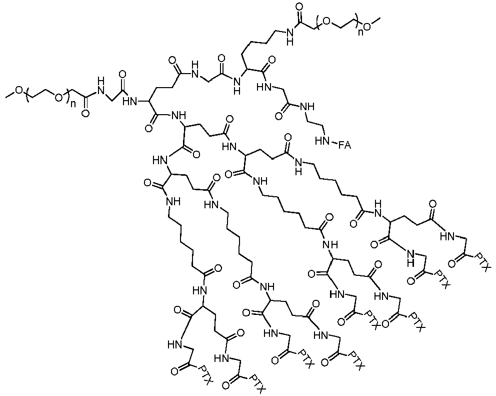 | | |
| | | wherein the number-average molecular weight of | 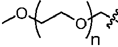 | is 10k; |
| 45-145 | | 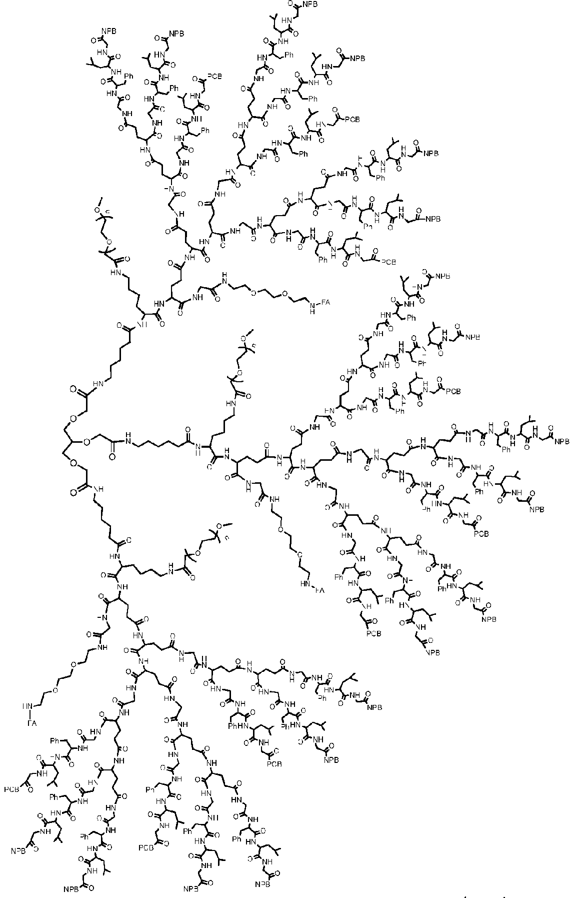 | | |
| | | wherein the number-average molecular weight of | 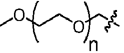 | is 10k; |
| 39-138 | | 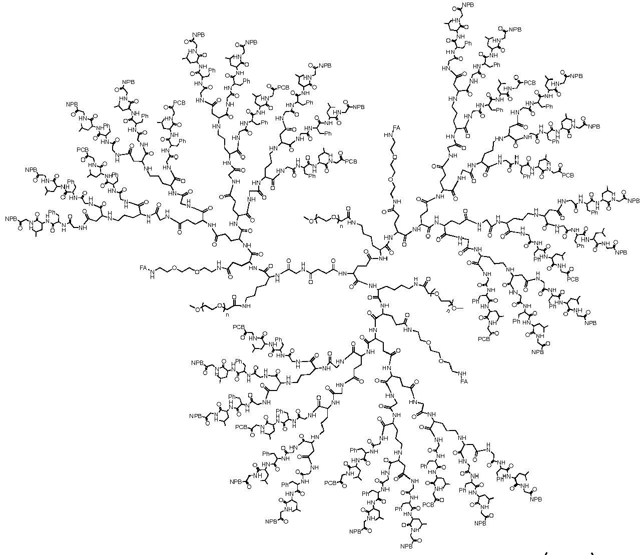 | | |
| | | wherein the number-average molecular weight of | 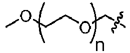 | is 10k; |
| 45-80 | | 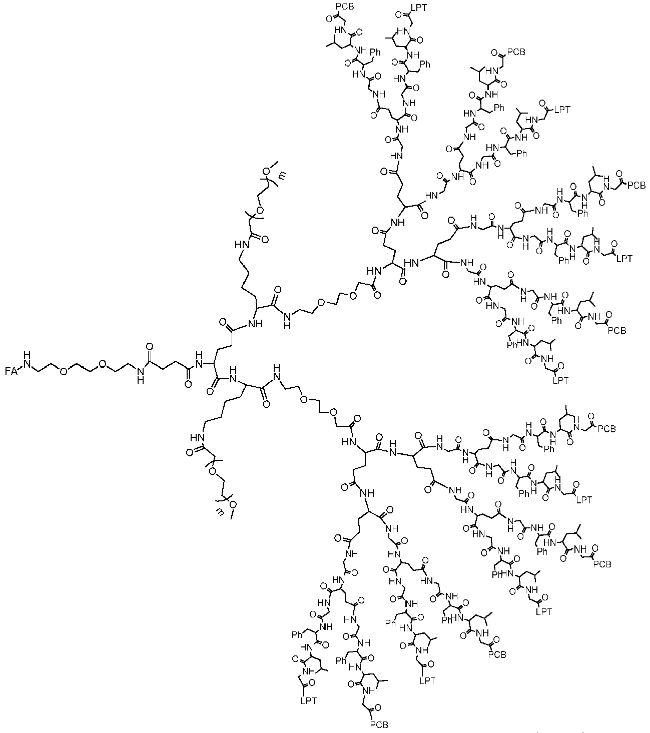 | | |
| | | wherein the number-average molecular weight of | 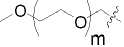 | is 10k; |
| 49-137 | 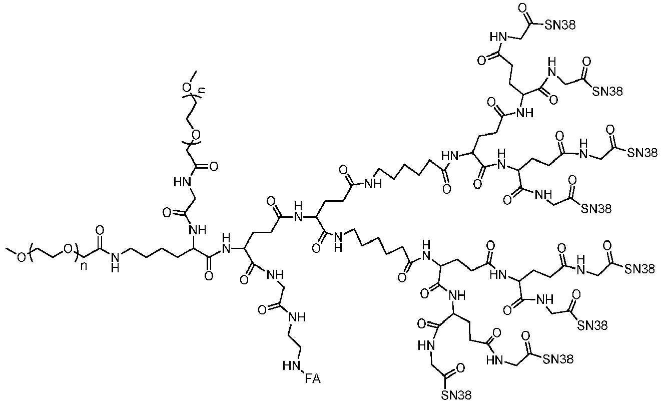 | | | |
| | wherein the number-average molecular weight of | | 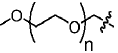 | is 5k; |
| 43-197 | 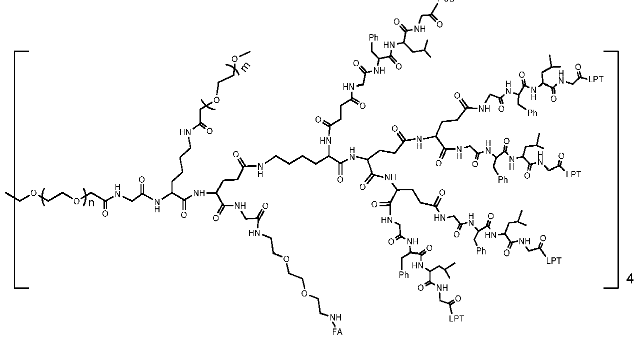 | | | |
| | wherein the number-average molecular weight of | | 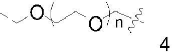 | is 10k, and the |
| | number-average molecular weight of | 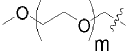 | is 5k; | |
| 50-47 | | 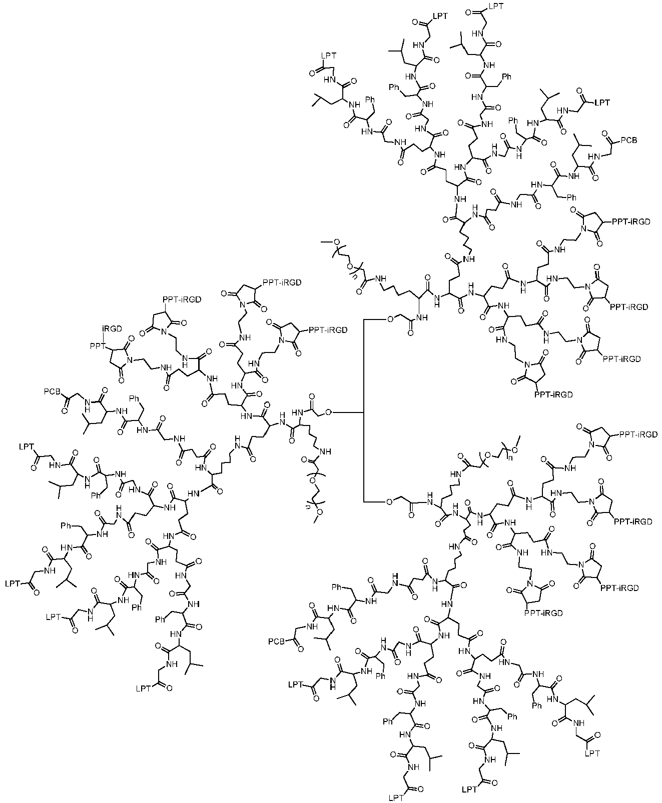 | | |
| | | wherein the number-average molecular weight of | 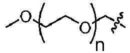 | is 10k; |
| 50-65 | | 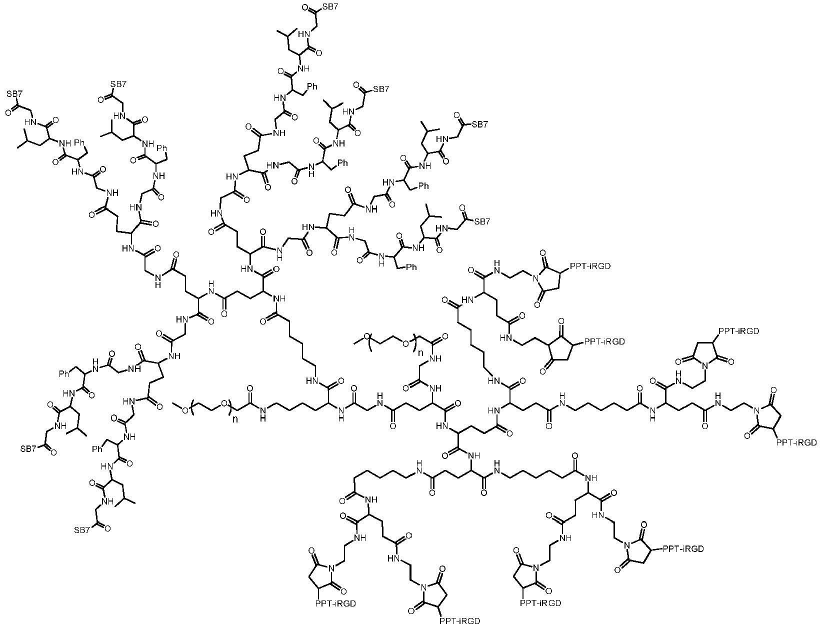 | | |
| | | wherein the number-average molecular weight of | 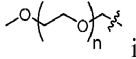 | is 10k; |
| 60-43 | | 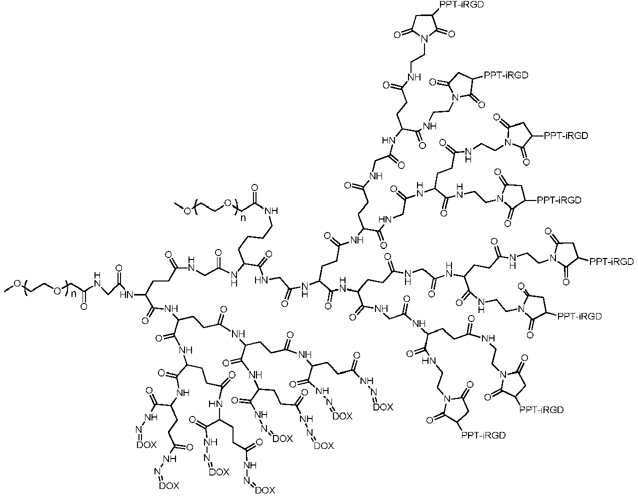 | | |
| | | wherein the number-average molecular weight of | 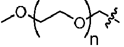 | is 10k; |
| 48-124 | | 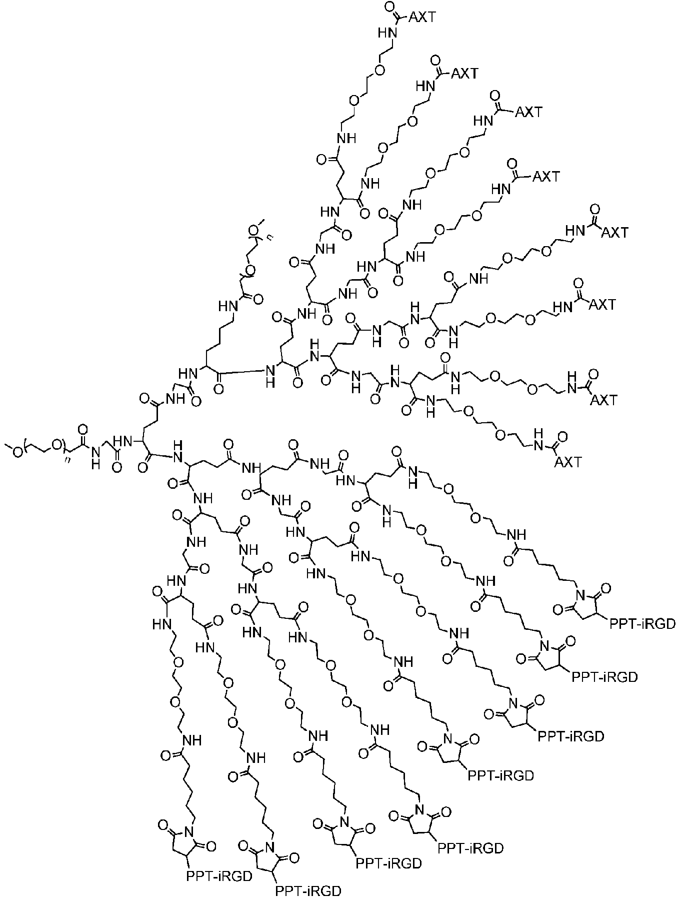 | | |
| | | wherein the number-average molecular weight of | 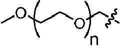 | is 10k; |
| 43-226 | | 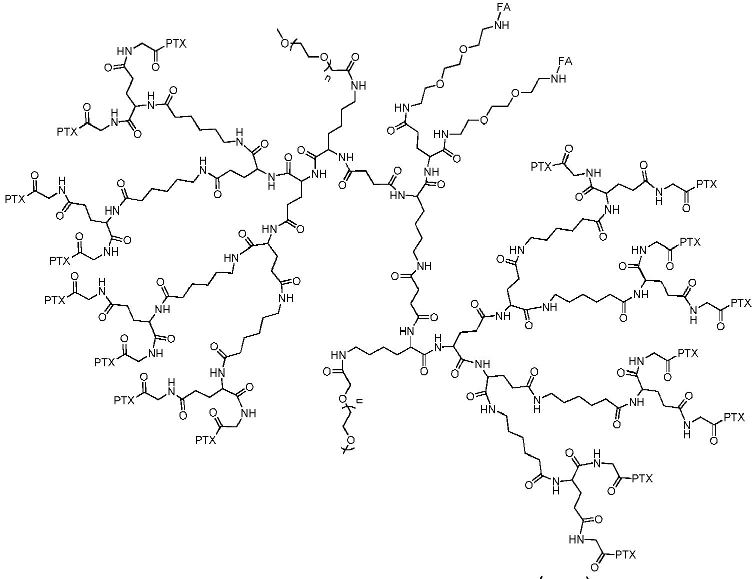 | | |
| | | wherein the number-average molecular weight of | 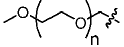 | is 10k; |
| 51-103 | | 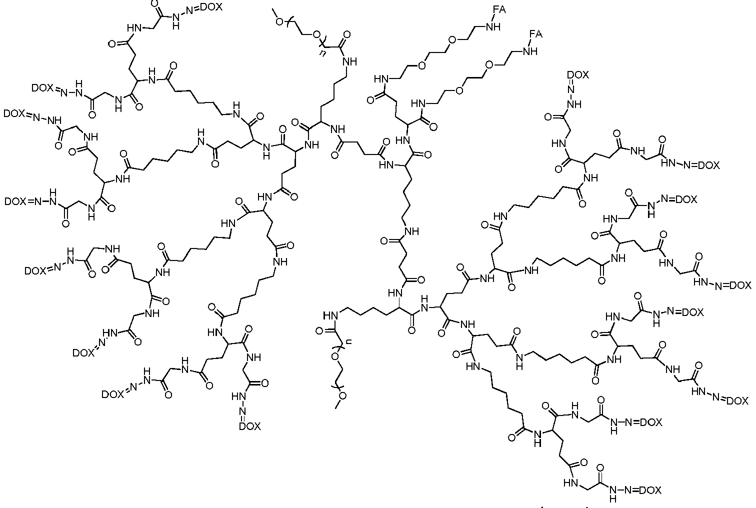 | | |
| | | wherein the number-average molecular weight of | 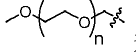 | is 10k. |

In the twelfth aspect of the present invention, the present invention provides an intermediate for preparing the above-mentioned polyethylene glycol conjugated drug or its pharmaceutically acceptable salt, the intermediate being selected from:

| Number | Structure |
|---|---|
| 37-166 | 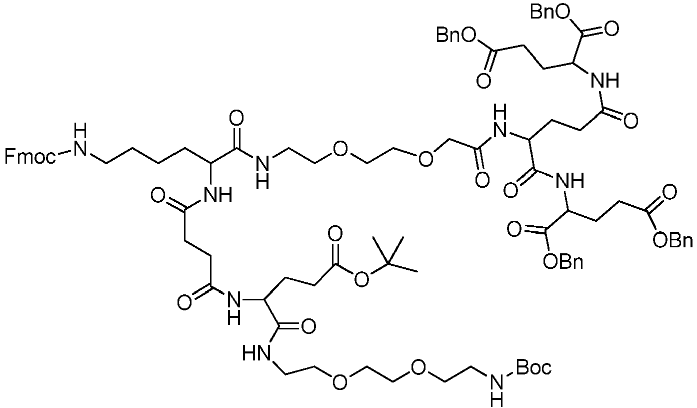 |
| 36-144 | 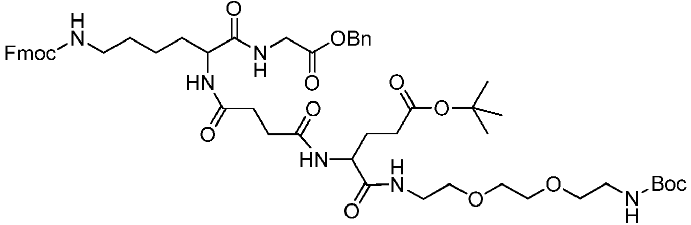 |
| 43-140 | 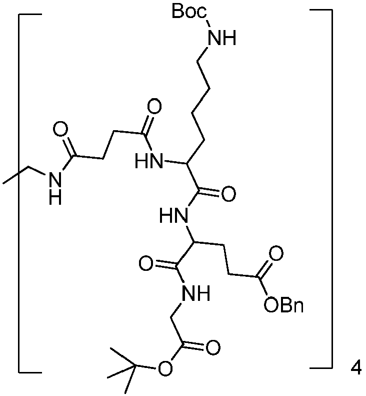 |
| 43-135 | 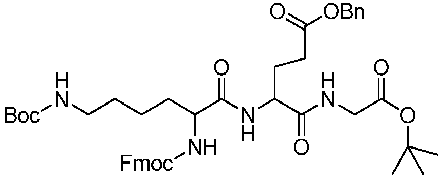 |
| 37-212 | 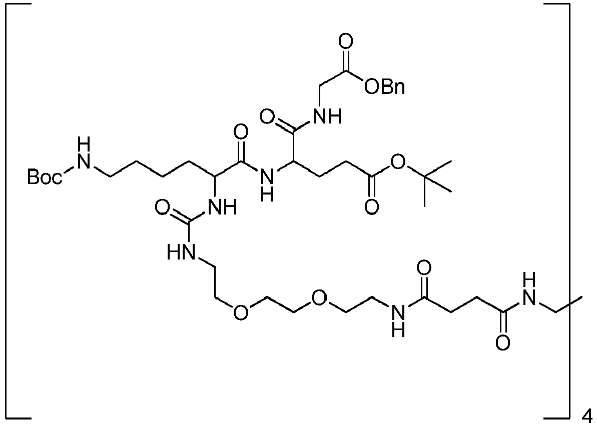 |
| 44-140 | 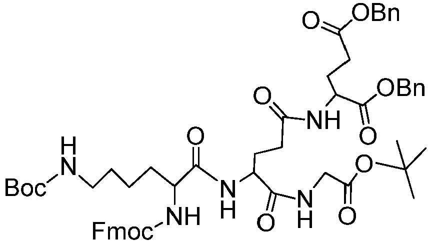 |
| 44-150 | 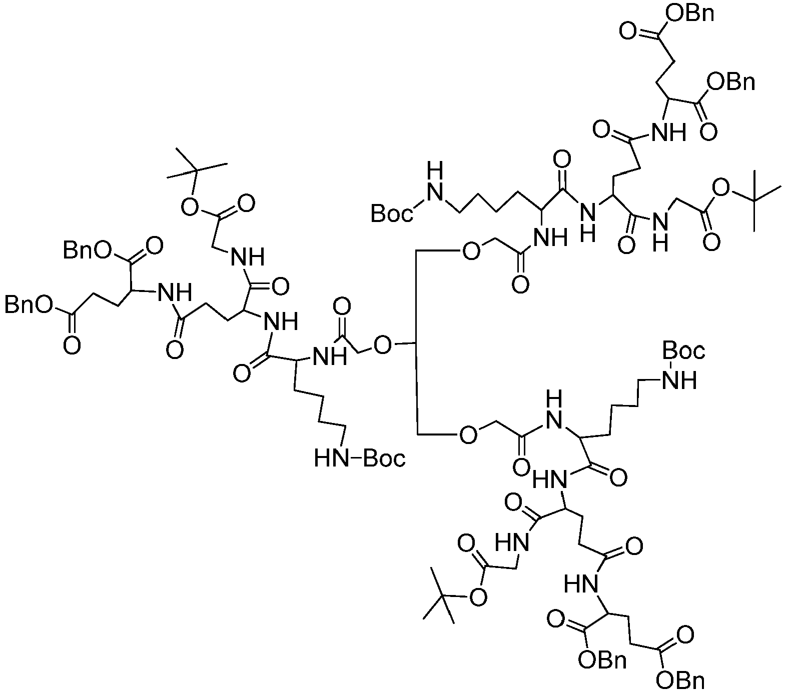 |
| 49-25 | 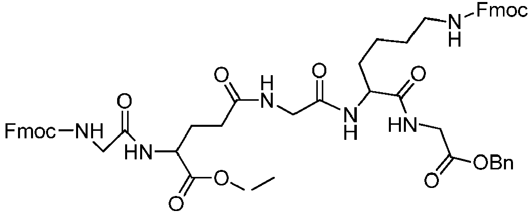 |
| 47-36 | 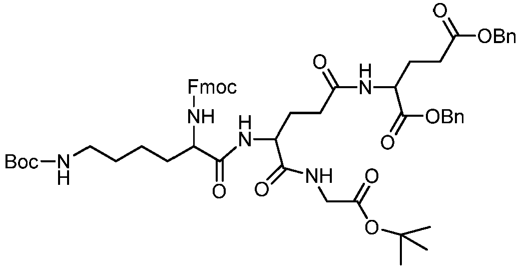 |
| 47-39 | 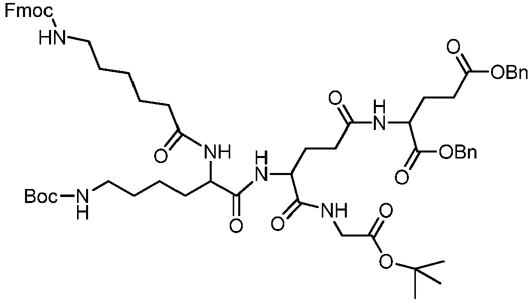 |
| 47-44 | 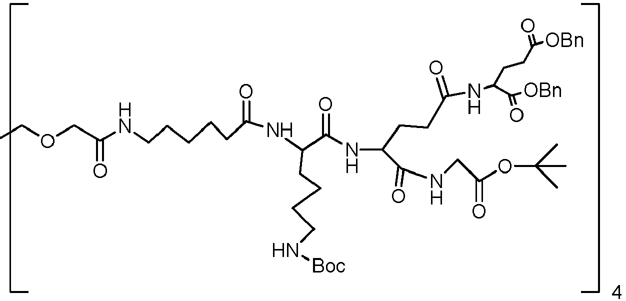 |
| 49-44 | 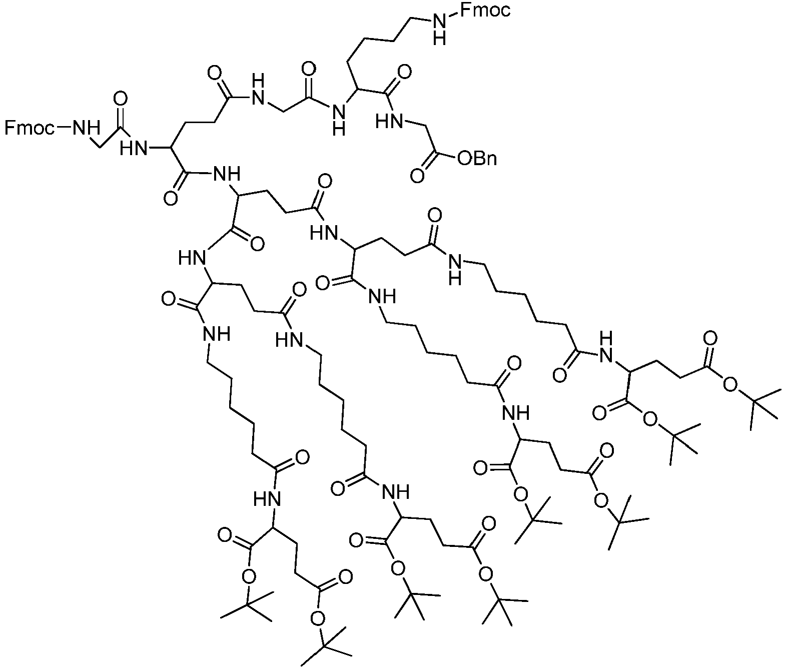 |
| 45-95 | 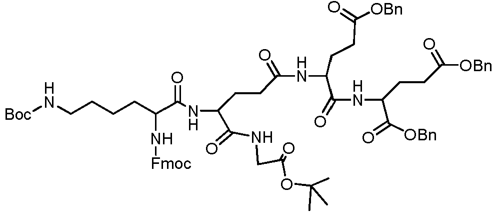 |
| 45-105 | 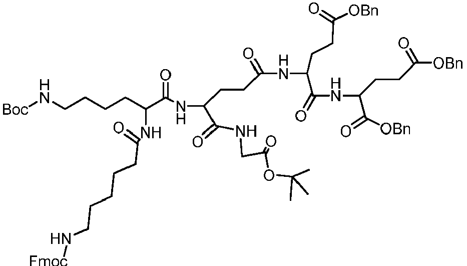 |
| 45-122 | 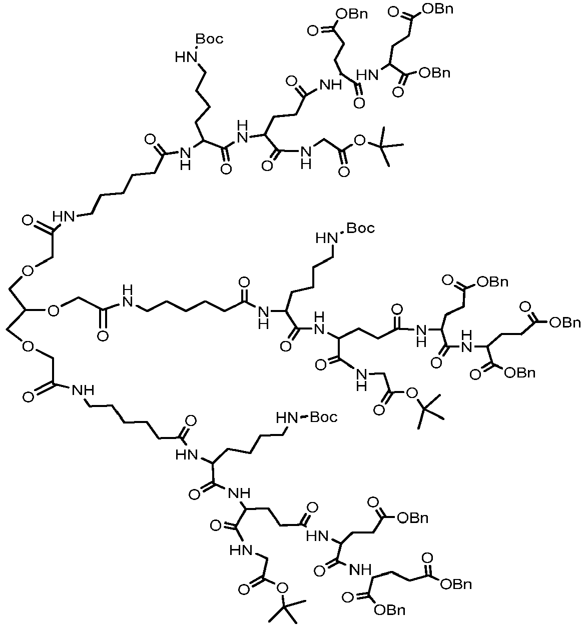 |
| 39-92 | 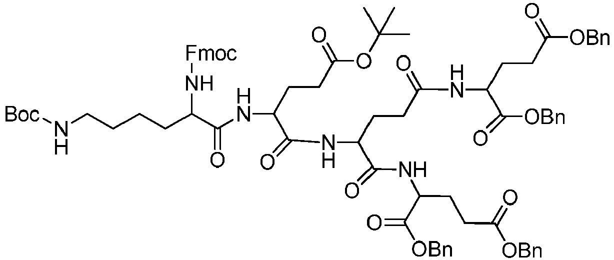 |
| 39-97 | 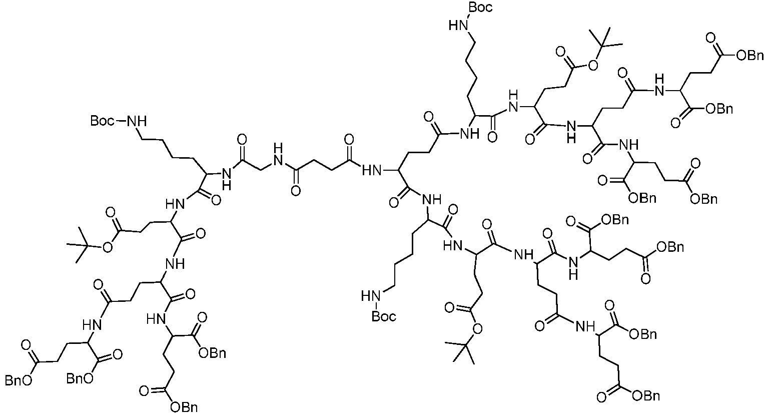 |
| 45-50 | 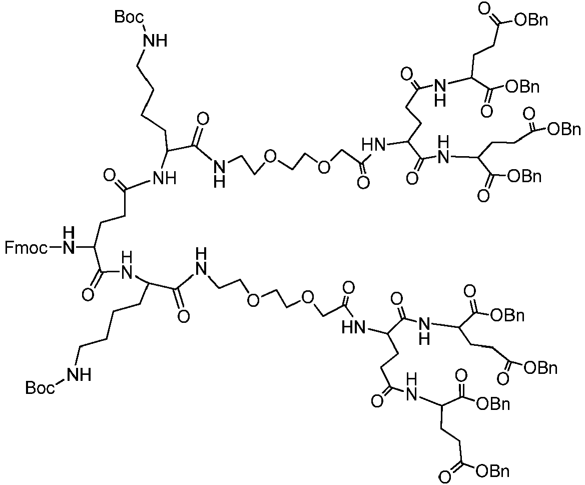 |
| 45-54 | 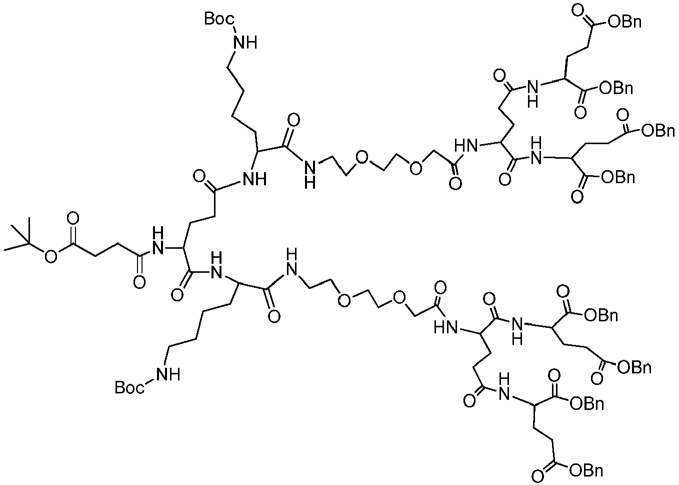 |
| 49-100 | 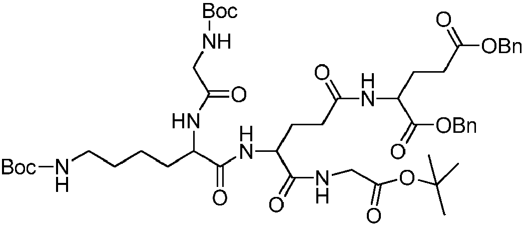 |
| 49-103 | 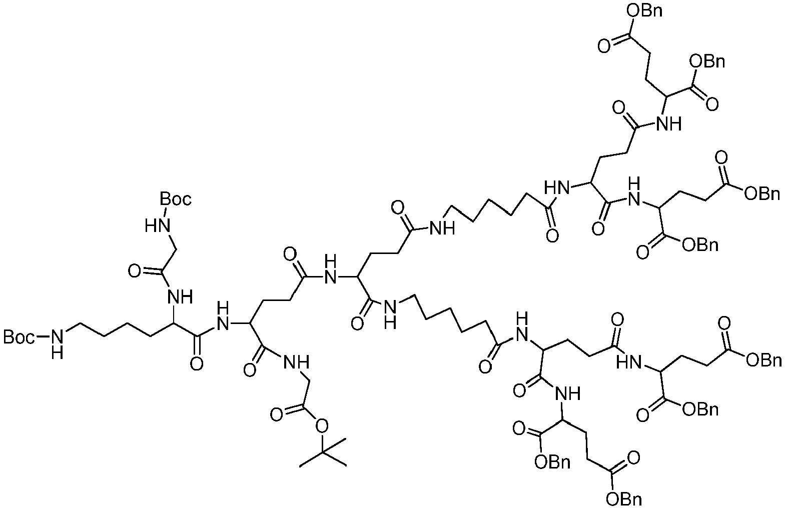 |
| 44-239 | 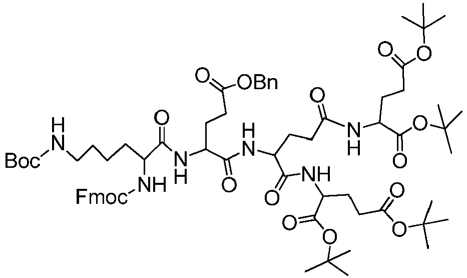 |
| 44-245 | 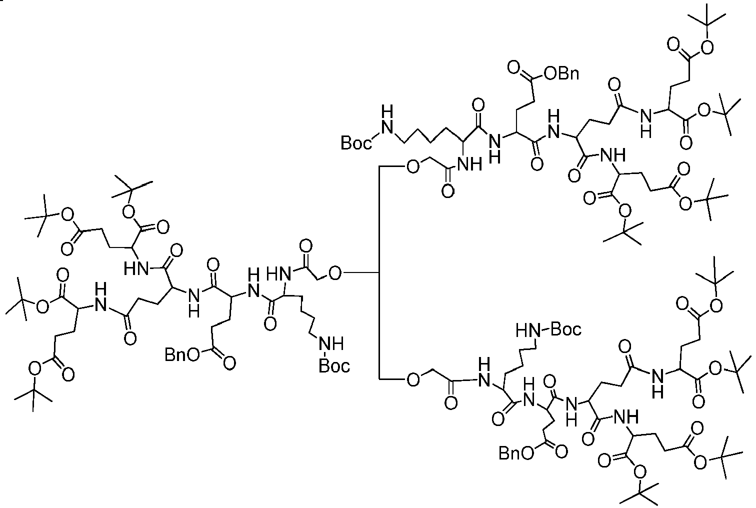 |
| 50-44 | 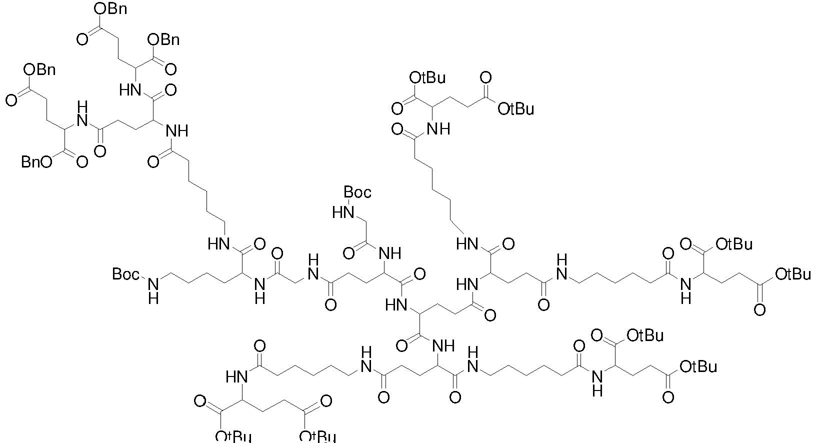 |
| 49-244 | 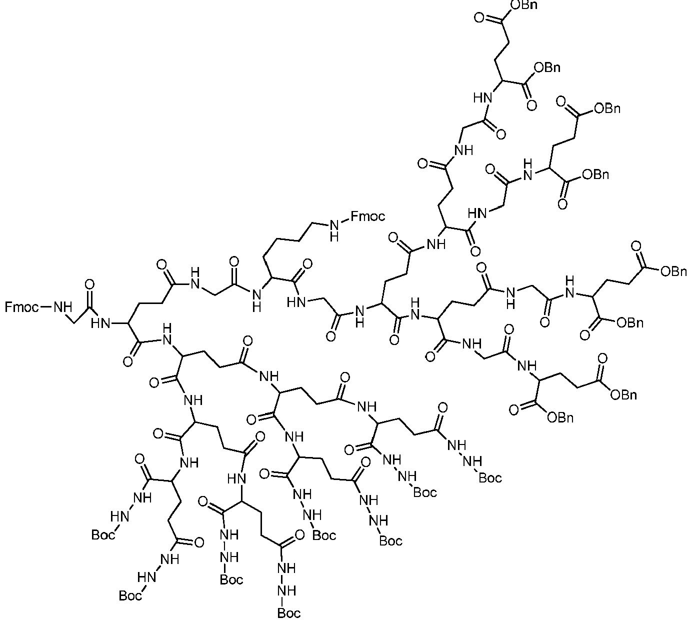 |
| 48-97 | 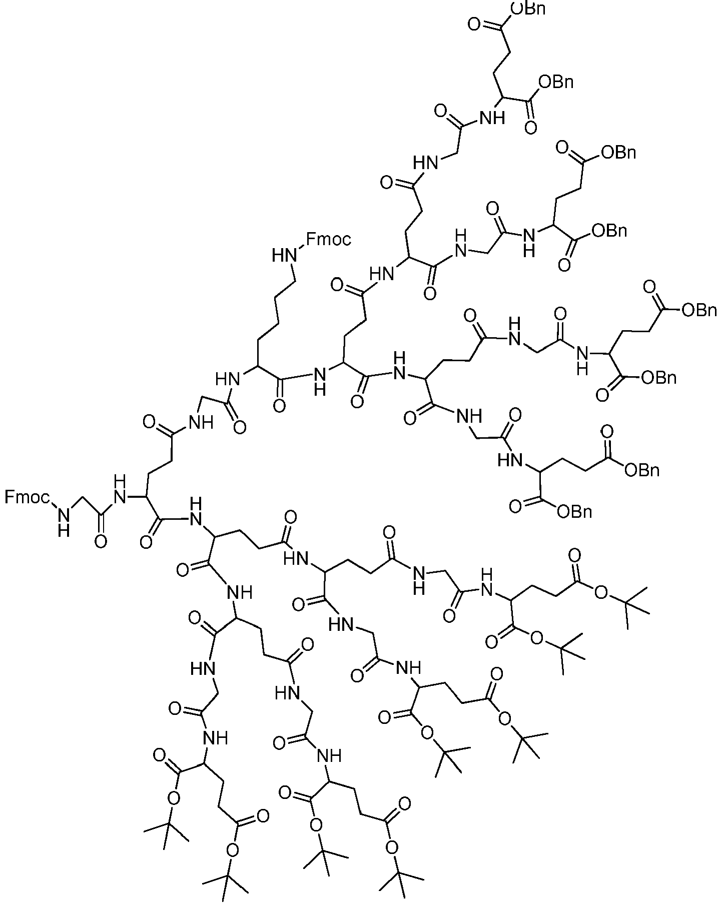 |

In the thirteenth aspect of the present invention, the present invention provides a method for preparing the above-mentioned polyethylene glycol conjugated drug or its pharmaceutically acceptable salt, comprising the following steps:
(1) preparing the intermediate 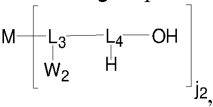 wherein, M, L₃, L₄, W₂ and j₂ are defined as above, and 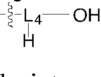 has carboxyl group and amino group at its terminal; preparing the intermediate V₂-H, wherem, V₂ is defined as above, and V₂-H has ammo group at its terminal;
(2) allowing PEG with carboxyl group or activated carboxyl group and the intermediate 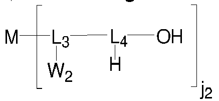 to carry out amidation reaction, to obtain the intermediate 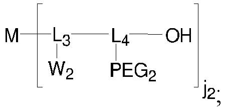 and
(3) allowing the intermediate V₂-H and the intermediate 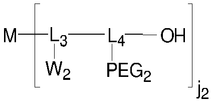 to carry out amidation reaction, to obtain the above-mentioned polyethylene glycol conjugated drug.

In the fourteenth aspect of the present invention, the present invention provides a method for preparing the above-mentioned polyethylene glycol conjugated drug or its pharmaceutically acceptable salt, comprising the following steps:
(1) preparing the intermediate 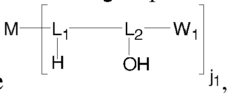 wherein, M, L₁, L₂, W₁ and j₁ are defined as above, and 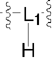 has amino group at its terminal, 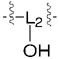 has carboxyl group at its terminal; preparing the intermediate V₁-H, wherein, Vi is defined as above, and V₁-H has amino group at its terminal;
(2) allowing PEG with carboxyl group or activated carboxyl group and the intermediate 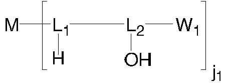 to carry out amidation reaction, to obtain the intermediate 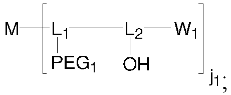 and
(3) allowing the intermediate V₁-H with amino group and the intermediate 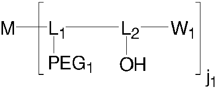 to carry out amindation reation, to obtain the above-mentioned polyethylene glycol conjugated drug.

In the fifteenth aspect of the present invention, the present invention provides a method for preparing the above-mentioned polyethylene glycol conjugated drug or its pharmaceutically acceptable salt, comprising the following steps:
(1) preparing the intermediate 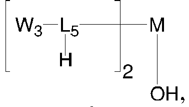 wherein, M, L₅ and W₃ are defined as above, and 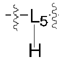 has amino group at its terminal, 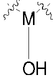 has carboxyl group at its terminal; preparing the intermediate V₃-H, wherein, V₃ is defined as above, and V₃-H has amino group at its terminal;
(2) allowing PEG with carboxyl group or activated carboxyl group and the intermediate 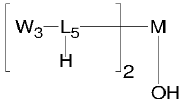 to carry out amidation reaction, to obtain the intermediate 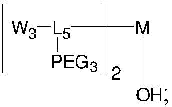 and
(3) allowing the intermediate V₃-H with amino group and the intermediate 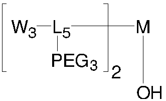 to carry out amidation reaction, to obtain the above-mentioned polyethylene glycol conjugated drug.

In the sixteenth aspect of the present invention, the present invention provides a method for preparing the above-mentioned polyethylene glycol conjugated drug or its pharmaceutically acceptable salt, comprising the following steps:
(1) preparing the intermediate 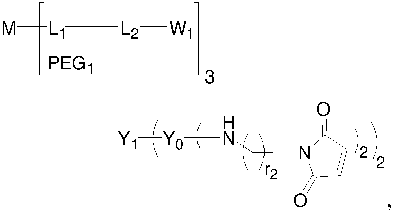 wherein, M, L₁, L₂, PEG₁, W₁, Y₁, Y₀ and r₂ are defined as above and
(2) allowing the intermediate 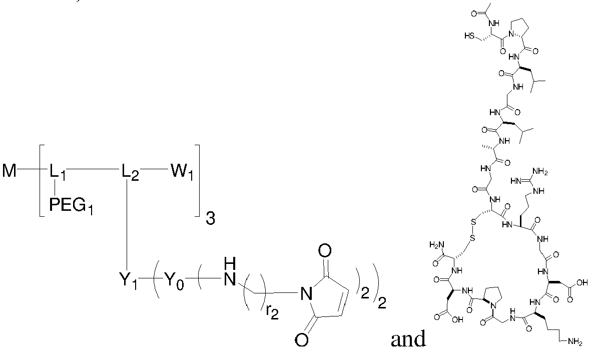 to carry out addition reaction, to obtain the above-mentioned polyethylene glycol conjugated drug.

In the seventeenth aspect of the present invention, the present invention provides a method for preparing the above-mentioned polyethylene glycol conjugated drug or its pharmaceutically acceptable salt, comprising the following steps:
(1) preparing the intermediate 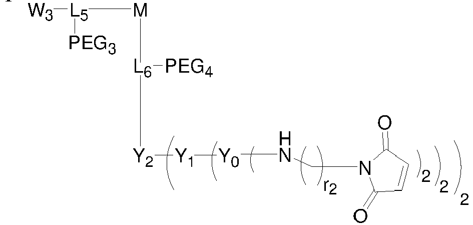 or 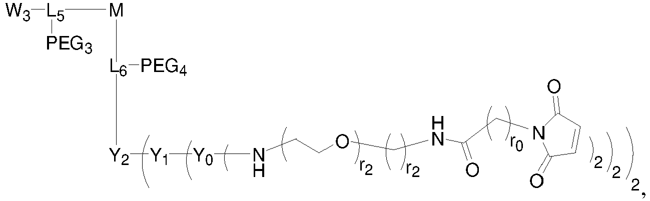 wherein, M, L₅, L₆, PEG₃, PEG₄, W₃, Y₂, Y₁, Y₀, r₀ and r₂ are defined as above; and
(2) allowing the intermediate 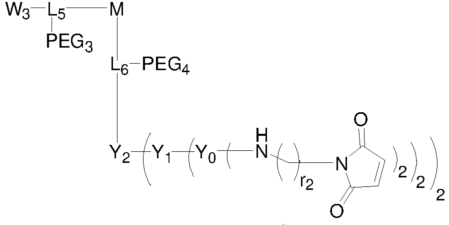 or 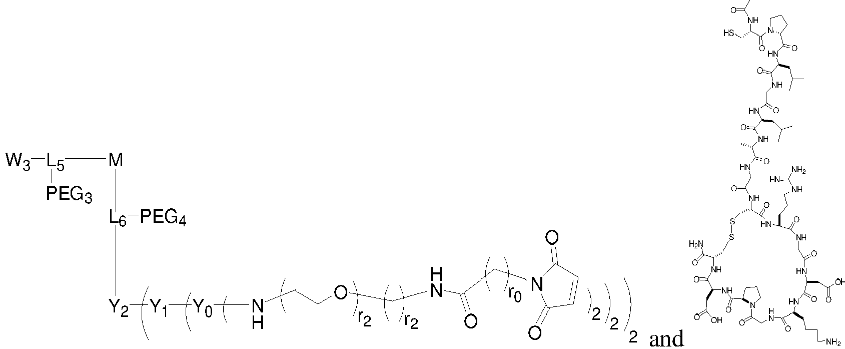 to carry out addition reaction, to obtain the above-mentioned polyethylene glycol conjugated drug.

In one aspect of the present invention, the present invention provides a pharmaceutical composition comprising therapeutically and/or prophylactically effective amount of the polyethylene glycol conjugated drug or a pharmaceutically acceptable salt thereof of the present invention; the composition further comprises one or more pharmaceutically acceptable excipients, such as carriers and/or vehicles. The carriers and/or vehicles include, but are not limited to: ion exchangers, alumina, aluminum stearate, lecithin, serum proteins such as human serum protein, buffer substances such as phosphate, glycerin, sorbic acid, potassium sorbate, a mixture of partial glycerides of saturated plant fatty acids, water, salt or electrolyte, such as protamine sulfate, disodium hydrogen phosphate, potassium hydrogen phosphate, sodium chloride, zinc salt, colloidal silica, magnesium trisilicate, polyvinylpyrrolidone, cellulose material, polyethylene glycol, sodium carboxymethyl cellulose, polyacrylate, beeswax, polyethylene- polyoxypropylene block polymer, and lanolin.

The pharmaceutical composition may be prepared into any pharmaceutically acceptable dosage form. The pharmaceutical composition may also be applied to individuals in need of such treatment in any suitable way of administration, such as oral, parenteral, rectal or pulmonary administration. In the case of oral administration, the pharmaceutical composition may be made into conventional solid preparations, such as tablets, capsules, pills, granules, etc.; it may also be made into oral liquid preparations, such as oral solutions and oral suspensions, and syrup. When the pharmaceutical composition is made into oral preparations, suitable fillers, binders, disintegrants, lubricants, etc. may be added. In the case of parenteral administration, the pharmaceutical composition may be made into injection preparations, including injection solutions, sterile powders for injection, and concentrated solutions for injection. When the pharmaceutical composition is made into injection preparations, they may be produced by a conventional method in the current pharmaceutical field. In the case of preparation of injection preparations, it is not required to add additives, or appropriate additives may be added according to the nature of the drug. In the case of rectal administration, the pharmaceutical composition may be made into suppositories and the like. In the case of pulmonary administration, the pharmaceutical composition may be made into an inhalant or a spray. Preferably, the pharmaceutical composition of the present invention may be made into an injection preparation, such as an injection solution. Alternatively, normal saline is used as the carrier of the injection solutions.

In another aspect of the present invention, the present invention provides use of the polyethylene glycol conjugated drug or a pharmaceutically acceptable salt thereof of the present invention in the preparation of a medicament for treating and/or preventing a disease (such as a cancer), wherein, the disease refers to a disease treated by the active ingredient in the polyethylene glycol conjugated drug.

In another aspect of the present, the present invention provides a method for treating and/or preventing a disease (such as a cancer), comprising administering an effective amount of the polyethylene glycol conjugated drug or a pharmaceutically acceptable salt thereof of the present invention to an individual in need thereof. The dosage regimen may be adjusted to provide the optimum desired response. For example, a single amount of drug may be administered, several divided doses may be administered over time, or the dose may be proportionally reduced or increased as indicated by the urgent need for the treatment. It should be noted that the dose value may vary with the type and severity of the condition to be alleviated, and may include single or multiple doses. It should be further understood that for any particular individual, the specific dosage regimen should be adjusted over time according to the individual's needs and the professional judgment of the person administering the composition or supervising the administration of the composition.

In another aspect of the present invention, the present invention provides the polyethylene glycol conjugated drug or a pharmaceutically acceptable salt thereof of the present invention for use in treating and/or preventing a disease (such as cancer).

In the present invention, cancer refers to a disease state characterized by cell proliferative, including but not limited to: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, etc., including metastasis of the aforementioned cancers.

In the present invention, "individual" includes a human or a non-human animal. Exemplary human individuals include human individuals suffering from diseases such as those described herein (referred to as patients) or normal individuals. In the present invention, "non-human animals" include all vertebrates, such as non-mammals (such as birds, amphibians, and reptiles) and mammals, such as non-human primates, livestock and/or domesticated animals (such as sheep, dogs, cats, cows, pigs, and etc.).

As used herein, the term "effective amount" refers to the amount of a compound that will relieve one or more symptoms of the disease being treated to a certain extent after being administered. As used herein, the term "treating" means reversing, alleviating, or inhibiting the disease or condition to which such term is applied or the progression of one or more symptoms of such a disease or condition, or preventing such a disease or condition or one or more symptoms of such a disease or condition.

In the polyethylene glycol conjugated drug of the present invention, multiple identical or different drug molecules are conjugated together by using an amino acid or a polypeptide as a linking chain, and a dicarboxylic acid or polycarboxylic acid with an amino group (for example, a natural amino acid with two carboxyl groups) or a carboxylic acid with two amino groups or multiple amino groups (for example, a natural amino acid with two amino groups) or a polycarboxylic acid as a linking bridge through the formation of an amide bond. The type, ratio and drug loading of the drug can be adjusted. In certain embodiments, activated PEG reacts with an amino group on the main chain through a carboxyl group to form an amide bond.

In the present invention, the active ingredient suitable for being conjugated with polyethylene glycol may be a drug molecule with at least one amino group, hydroxyl group, carboxyl group or acyl group, for example, a drug molecule having anti-tumor activity with at least one amino group, hydroxyl group, carboxyl group or acyl group, such as SB7, NPB, SN38, LPT, PCB, DOX, PTX or AXT, which represent the following meanings:

| Abbreviation | Name | CAS number or structural formula |
|---|---|---|
| LPT | Lapatinib | 231277-92-2 |
| PCB | Palbociclib | 571190-30-2 |
| SB7 | SB-743921 | 940929-33-9 |
| NPB | Niraparib (MK-4827) | 1038915-60-4 |
| SN38 | 7-ethyl-10-hydroxycamptothecin | 86639-52-3 |
| DOX | Adriamycin | 23214-92-8 |
| PTX | Paclitaxel | 33069-62-4 |
| AXT | Axitinib | 319460-85-0 |

In addition, FA represents folic acid and its structural formula is 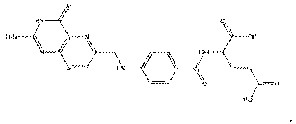 The connection site of the FA to other part of the overall structure of the polyethylene glycol conjugated drug is 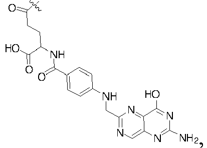 i.e., the position indicated by 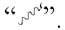 PPT-iRGD has the structural formula 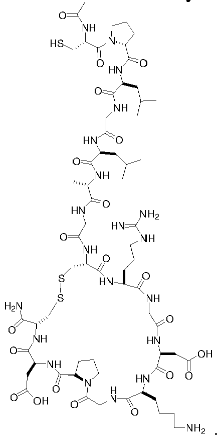 The connection site of the PPT-iRGD to other part of the overall structure of the polyethylene glycol conjugated drug is terminal sulfhydryl group 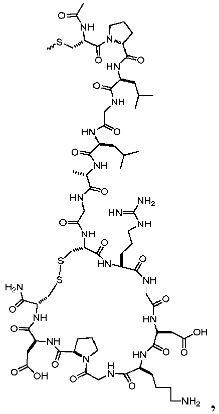 i.e., the position indicated by

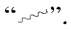

In addition, the connection sites of the following drug molecules to other part of the overall structure of the polyethylene glycol conjugated drug are shown in the following table, i.e., the positions indicated by

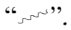

| Abbreviation | Connection site |
|---|---|
| LPT | 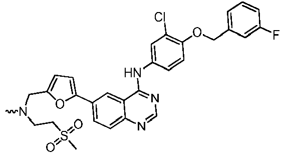 |
| PCB | 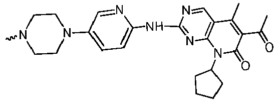 |
| SB7 | 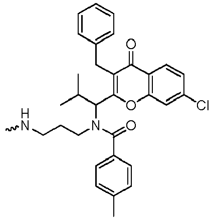 |
| NPB | 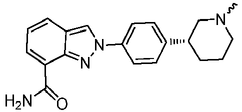 |
| SN38 | 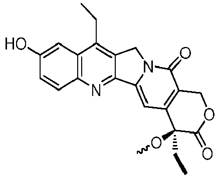 |
| DOX | 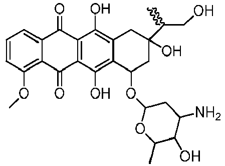 |
| PTX | 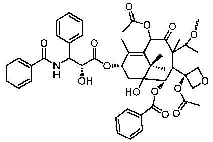 |
| AXT | 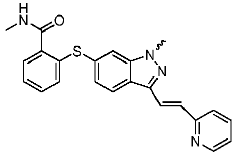 |

As used herein, "PEG" is an abbreviation for polyethylene glycol, which refers to a homopolymer with a repeating unit of -CH₂CH₂O-, including single-arm polyethylene glycol, multi-arm polyethylene glycol and their derivatives, such as a derivative with a reactive functional group such as amino or carboxyl group at the terminal. In the present invention, the arms of the multi-arm polyethylene glycol preferably have the same degree of polymerization. When referring to the molecular weight of a multi-arm polyethylene glycol, the molecular weight means the total molecular weight of each arm. In the structural formula of the present invention, the letter "m" or "n" in the subscript of the repeating unit of polyethylene glycol represents the degree of polymerization of polyethylene glycol. When the polyethylene glycol is a multi-arm polyethylene glycol, the letter "m" or "n" represents the degree of polymerization of each arm.

As used herein, unless it is clearly indicated in other ways, the expressions "each ... independently are" and "... and ... each independently are" used throughout this disclosure are interchangeable, and both should be understood in a broad sense. It can mean that the specific options expressed by the same symbol in different groups do not affect each other, or it can mean that the specific options expressed by the same symbol in the same group do not affect each other.

As used herein, a variable "direct bond" means that the linking group does not exist.

As used herein, the "pharmaceutically acceptable salt" of the compound of the present invention includes an acid addition salt and base addition salt of the compound, such as hydrochloride, hexafluorophosphate, and meglumine salt.

As used herein, the wavy line " 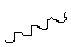 " in the structural formula means the position where another group is bonded to the structure represented by the structural formula.

### BRIEF DESCRIPTION OF THE DRAWINGS

FIG. 1 is a schematic diagram of the tumor growth trend of each group in the examples of the present invention.
FIG. 2 is a schematic diagram of the tumor weight inhibition rate of each group in the examples of the present invention.

### DETAILED DESCRIPTION

The embodiments of the present invention will be described in detail below in conjunction with examples. However, those skilled in the art will understand that the following examples are only used to illustrate the present invention, not to limit the scope of the present invention. Those without specific conditions among the examples are generally implemented under conventional conditions or under conditions recommended by the manufacturers. The reagents or instruments used without specifying the manufacturers are all conventional products that may be purchased commercially.

The meanings of abbreviations in the examples are as follows:

| | | | |
|---|---|---|---|
| G | Glycine residue | L | Leucine residue |
| F | Phenylalanine residue | Asp | Aspartic acid residue |
| E | Glutamate residue | Glu | Glutamate residue |
| DMF | N, N-dimethylformamide | TFA | Trifluoroacetic acid |
| t-Bu | Tert-butyl | Bn | Benzyl |
| Boc | Tert-butoxycarbonyl | Fmoc | Fluorenyl methoxycarbonyl |
| HOBT | 1-hydroxybenzotriazole | Ts | p-toluenesulfonyl |
| HBTU | o-Benzotriazole-N,N,N',N'-tetramethyl-uronium-hexafluoro-phosphate | LPT | Lapatinib |
| DIEA | N, N-diiso-propylethylamine | SB7 | SB-743921 |
| EA | Ethyl acetate | PCB | Palbociclib |
| TMP | 2, 4, 6-trimethylpyridine | NPB | Niraparib |
| PyAOP | (3H-1,2,3-triazolo[4,5-b]pyridinato-O)tri-1-pyrrolidinylphosphonium hexafluorophosphate | | |
| LC | NH₂-CH₂CH₂O-CH₂CH₂O-CH₂-COOH or -NH-CH₂CH₂O-CH₂CH₂O-CH₂-CO- | | |

The source and structure of some raw materials are as follows:
M-SCM-5K
JenKem, 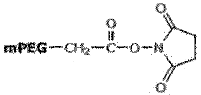
M-SCM-10K
JenKem, 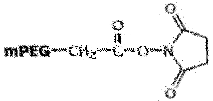
4ARM-SCM-40K
JenKem, 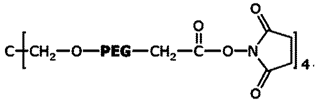
4ARM-SCM-10K
JenKem,

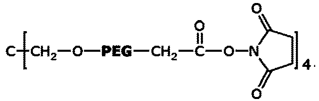

The present invention is further illustrated by the following examples.

### Example 1: Synthesis of compound

### Synthesis route of 37-184

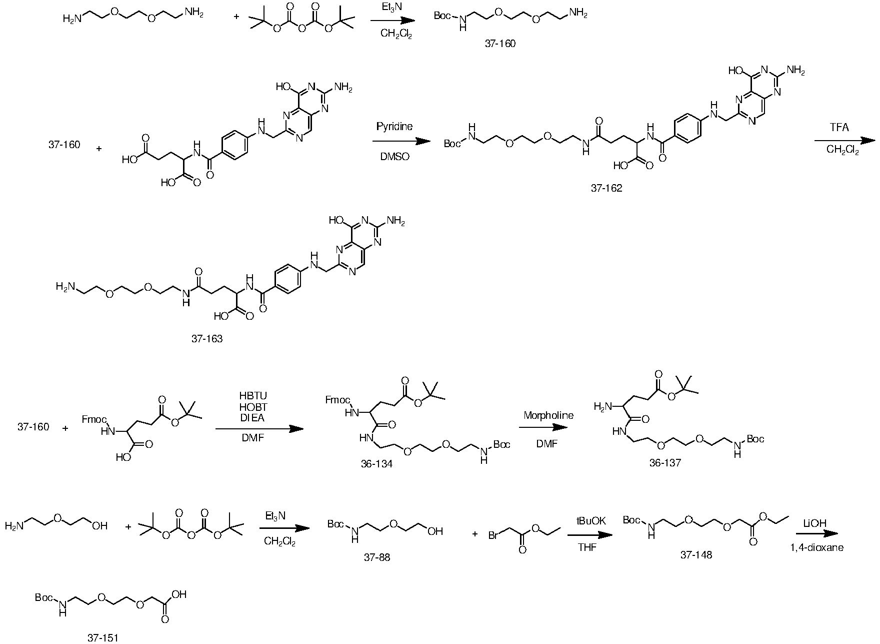

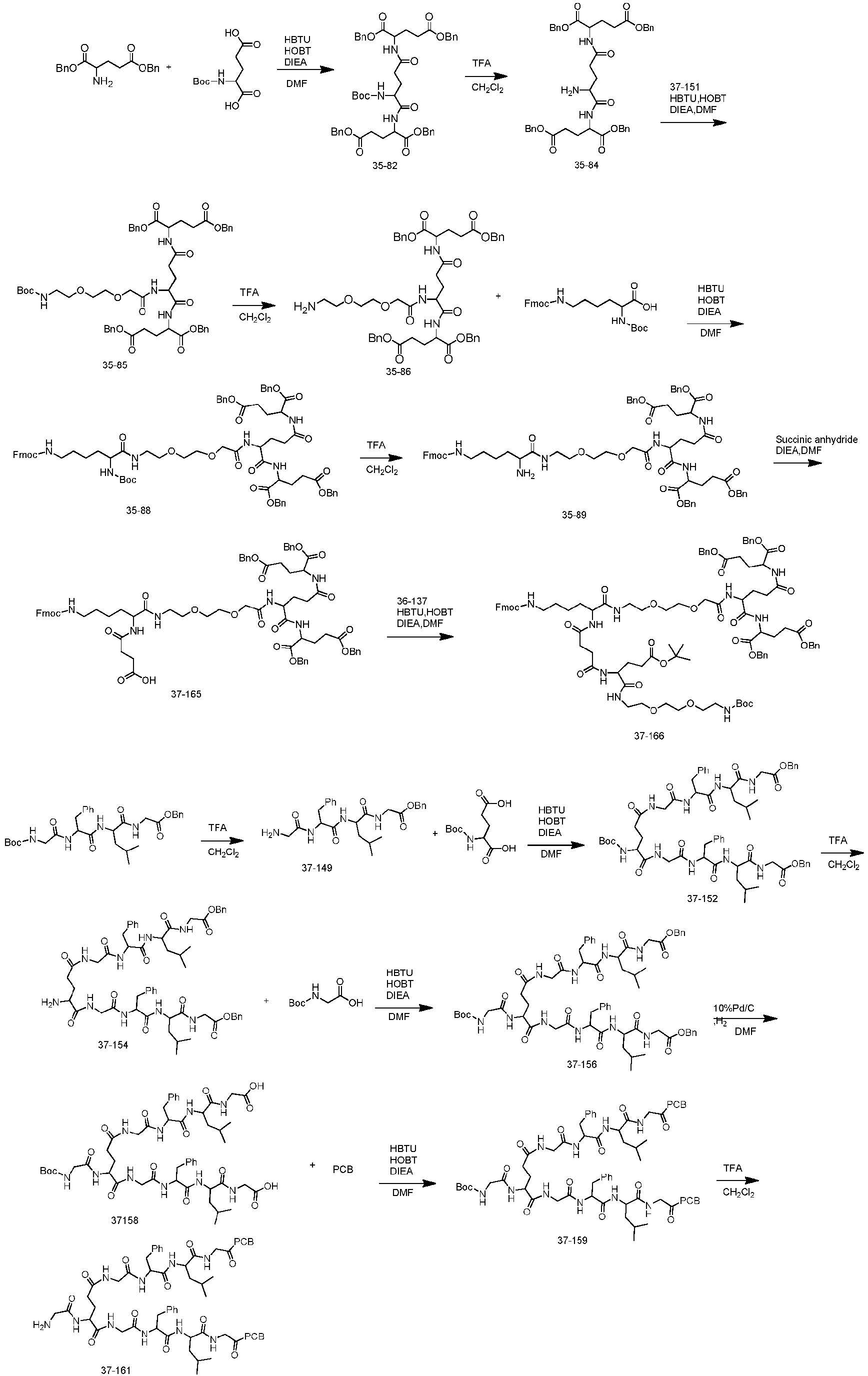

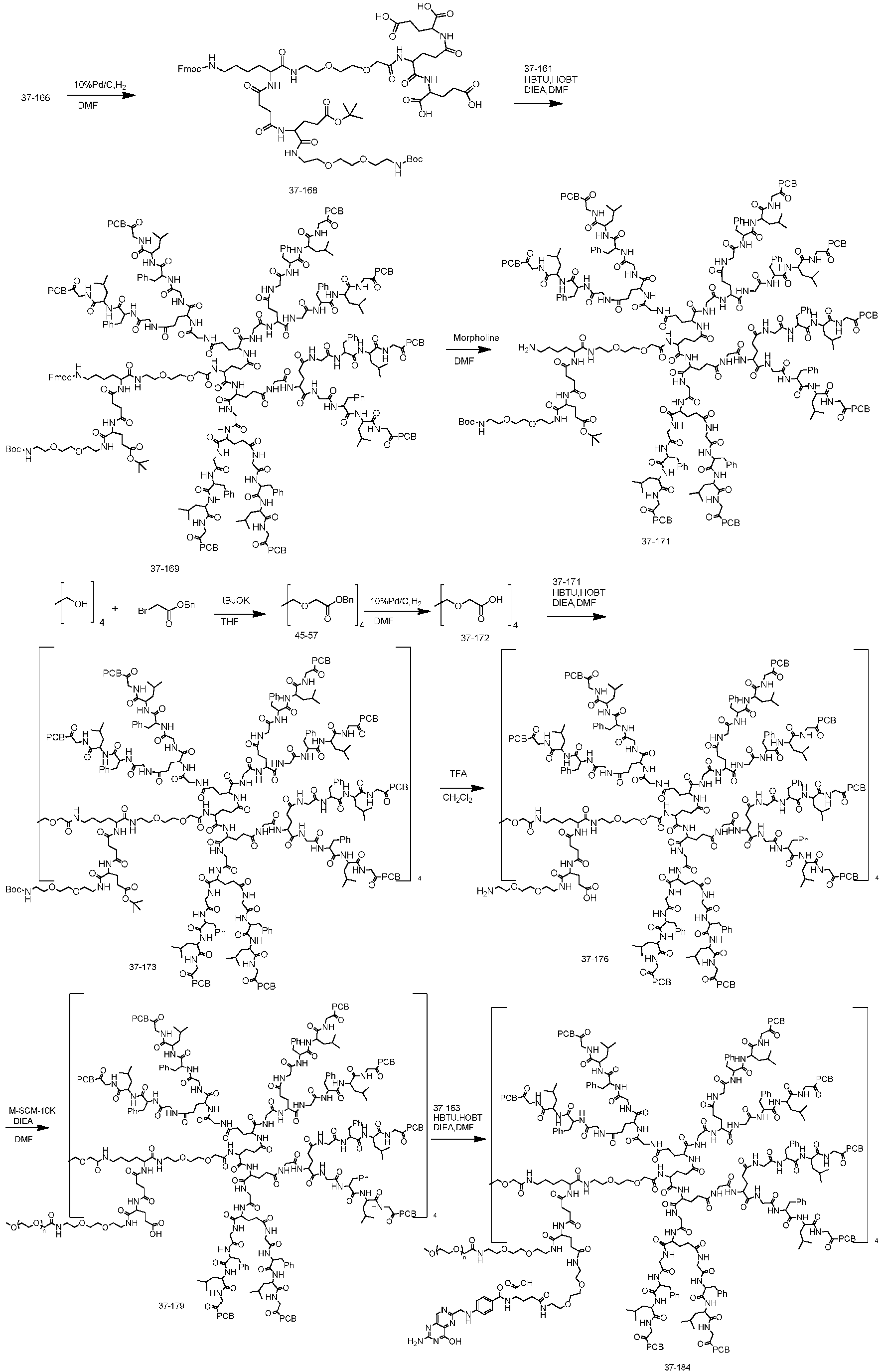

37-160

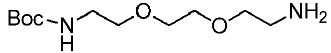

1,2-bis(2-a minoethoxy)ethane (50 g, 337.2 mmol, purchased from TCI) and triethylamine (37.6 mL, 270 mmol) were added to a 1 L flask, and the obtained solution was stirred at -5 °C for 10 min to react. Di-tert-butyl dicarbonate (56.5 g, 270 mmol, purchased from Adamas) was dissolved with dichloromethane, the resulting solution was added to the flask dropwise, and at the end of the addition, the obtained solution was further stirred for 10 min. At the end of the reaction, silica gel powder (60 g) was added, and the solution was then evaporated to dryness to obtain a powdery solid, the operations of dry sample loading, column chromatography, and elution with a mixed solution (1% ammonia water and 4% methanol/dichloromethane) were carried out, thus obtaining 28.7 g of Product 37-160 with a yield of 34%.
37-162

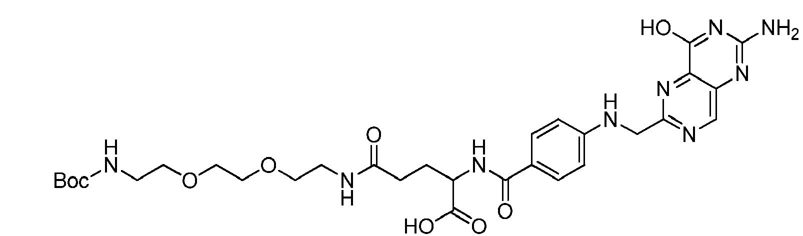

Folic acid (9.776 g, 22.1488 mmol, purchased from damas-beta) was added to a 500 mL flask and then dissolved with DMSO (300 mL), pyridine (16.285 mL, 201.35 mmol) was then added, and the mixed solution was stirred for 1 h at room temperature to react; Product 37-160 (5 g, 20.135 mmol) and DCC (2.07 g, 201.35 mmol) were then added, and the reaction solution was stirred overnight at room temperature to react. At the end of the reaction, ethyl acetate (200 mL), n-hexane (300 mL) and methyl tert-butyl ether (150 mL) were added to layer the reaction solution, the supernatant was discarded, ethyl acetate (200 mL) and n-hexane (300 mL) were further added to the lower oily solution and a powdery solid was then precipitated; the suction filtering was carried out and the filter cake was dried, thus obtaining 11.4 g of Product 37-162.
37-163

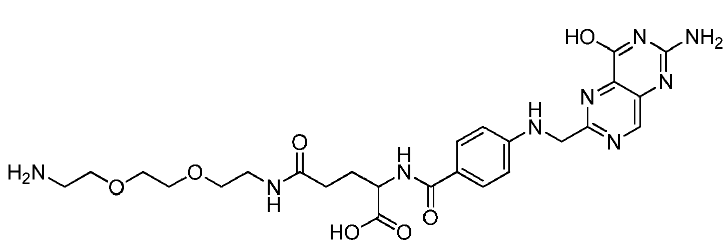

Product 37-162 (11.4 g, 16.972 mmol) was added to a 500 mL flask and then dissolved with dichloromethane (30 mL), trifluoroacetic acid (12.6 mL, 169.72 mmol) was then added, and the obtained solution was stirred overnight at room temperature to react. At the end of the reaction, methyl tert-butyl ether (200 mL) was added and a solid was then precipitated; the suction filtering was carried out and the filter cake was washed with methyl tert-butyl ether (50 mL × 2), and dried, thus obtaining 9.9 g of Product 37-163.
36-134

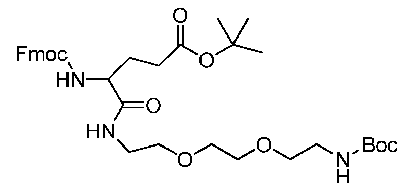

Fmoc-Glu(OtBu)-OH (4.93 g, 11.5859 mmol, purchased from Ark Pharm), Product 37-160 (2.74 g, 11.0342 mmol), HBTU (6.3 g, 16.5512 mmol) and HOBT (2.2 g, 16.5512 mmol) were added to a 500 mL flask and then dissolved with DMF (50 mL), and the obtained solution was stirred for about 10 min at 0 °C; then, DIEA (8.2 mL, 49.6537 mmol) was slowly added dropwise, and at the end of the addition, the obtained solution was further stirred at 0 °C overnight to react. At the end of the reaction, the reaction solution was transferred to a 1 L separatory funnel, deionized water (200 mL) and ethyl acetate (200 mL) were added, and the obtained solution was shaken and extracted. The aqueous phase was washed with ethyl acetate (150 mL × 2), the obtained organic phases were combined, washed with a saturated saline solution (200 mL × 2), then concentrated and evaporated to dryness, and dried in a vacuum oven, thus obtaining 7.235 g of Product 36-134.
36-137

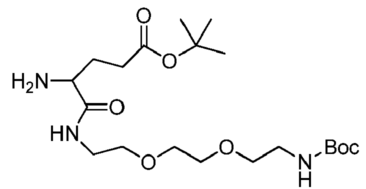

Product 36-134 (7.235 g, 11.0342 mmol) was added in a 250 mL flask and then dissolved with DMF (30 mL); morpholine (9.6 mL, 110.3415 mmol) was then added, and the reaction solution was stirred at room temperature for 1 h. At the end of the reaction, the reaction solution was transferred to a 1 L separatory funnel and extracted with deionized water (200 mL) and ethyl acetate (200 mL) to obtain an organic phase; the aqueous phase was washed with ethyl acetate (200 mL × 2), and the obtained organic phases were combined, washed with a saturated saline solution (200 mL × 2), then concentrated and evaporated to dryness, and the solid product was dissolved with methanol (30 mL) and dichloromethane (120 mL); silica gel powder (20 g) was added, and the solution was then evaporated to dryness to obtain a powdery solid; the operations of dry sample loading, column chromatography, and elution with a mixed solution (1%-5% of methanol/dichloromethane) were carried out, thus obtaining 4.2 g of Product 36-137 with a yield of 88%.
37-88

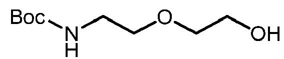

Diglycolamine (25.84 mL, 260.59 mmol, purchased from TCI), dichloromethane (50 mL) and triethylamine (72.64 mL, 521.18 mmol) were added in a 1 L flask and the mixed solution was stirred at 0 °C for 1 h. Tert-butyl dicarbonate was dissolved with dichloromethane and then the solution was slowly added to the flask dropwise; at the end of the addition, the mixed solution was further stirred at 0 °C overnight. At the end of the reaction, the reaction solution was concentrated to a small amount; silica gel powder (50 g) was then added to the obtained solution, and the solution was then evaporated to dryness to obtain a powdery solid; the operations of dry sample loading, column chromatography, and elution with a mixed solution of 50%-60% ethyl acetate in petroleum ether were carried out, thus obtaining 53.4861 g of Product 37-88.

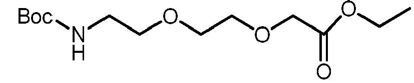

Product 37-88 (53.4861 g, 260.59 mmol) was added to a 1 L flask and the mixed solution was stirred at 0 °C for 30 min, a tetrahydrofuran solution of potassium tert-butoxide (286.64 mL, 1mol/L, 286.64 mmol) was then slowly added dropwise, and at the end of the addition, the mixed solution was further stirred for 1 h; ethyl bromoacetate (28.82 mL, 260.59 mmol) was slowly added dropwise, and at the end of the addition, the reaction solution was first stirred at 0 °C for 30 min, and then stirred at room temperature overnight. At the end of the reaction, the reaction solution was transferred to a 1 L separatory funnel and extracted with deionized water (200 mL) and ethyl acetate (200 mL) to obtain an organic phase; the aqueous phase was washed with ethyl acetate (200 mL × 1), the obtained organic phases were combined, then concentrated and evaporated to dryness, and the solid product was dissolved with methanol (30 mL) and dichloromethane (120 mL); silica gel powder (100 g) was then added to the obtained solution, and the solution was then evaporated to dryness to obtain a powdery solid; the operations of dry sample loading, column chromatography, and elution with a mixed solution of 20% ethyl acetate in petroleum ether were carried out, thus obtaining 52.1 g of Product 37-148 with a yield of 68.6%.
37-151

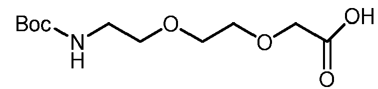

Product 37-148 (52.1 g, 178.8 mmol), 1,4-dioxane (100 mL) and lithium hydroxide (9.4 g, 393.4 mmol) were added to a 1 L flask and the mixed solution was stirred at room temperature for 30 min to react; purified water (200 mL) was added, and the obtained solution was further stirred for 2 h. At the end of the reaction, the reaction solution was transferred to a 1 L separatory funnel and extracted with n-hexane (125 mL) and methyl tert-butyl ether (125 mL); the aqueous phase was adjusted the PH to 2 with concentrated hydrochloric acid, then ethyl acetate (200 mL × 2) was added, and the obtained organic phases were combined; silica gel powder was added to the obtained solution, and the solution was evaporated to dryness; the operations of dry sample loading, column chromatography, and elution with a mixed solution of 50%-60% ethyl acetate in petroleum ether were carried out, thus obtaining 45.1 g of Product 37-151 with a yield of 96%.
35-82

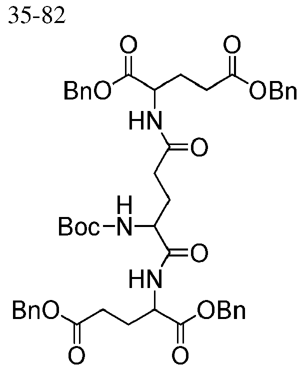

Boc-Glu-OH (20.0 g, 80.89 mmol, purchased from Ark Pharm), HBTU (92.02 g, 242.66 mmol), HOBT (32.8 g, 242.66 mmol) and H-Glu(OBn)₂·TsOH (84.861 g, 161.8 mmol, purchased from Ark Pharm) were added to a 1000 mL flask and then dissolved with DMF (200 mL), and the obtained solution was stirred for about 30 min at -5 °C; then, DIEA (120.32 mL, 728 mmol) was slowly added dropwise; at the end of the addition, the reaction solution was first stirred at -5 °C to react for 1 h, and then stirred at room temperature to further react overnight. At the end of the reaction, the reaction solution was transferred to a 2 L separatory funnel and extracted with a saturated sodium bicarbonate solution (600 mL) and ethyl acetate (300 mL); the aqueous phase was washed with ethyl acetate (200 mL × 1), and the obtained organic phases were combined, washed with a saturated saline solution (200 mL × 2), then concentrated and evaporated to dryness, thus obtaining 70 g of Product 35-82.
35-84

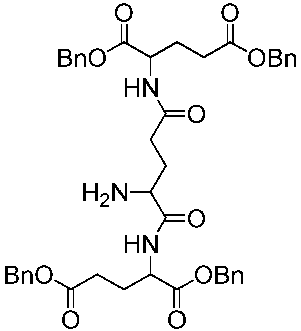

Product 35-82 (70 g, 80.89 mmol) was added in a 1000 mL round-bottomed flask and then dissolved with dichloromethane (50 mL), trifluoroacetic acid (300 mL, 4044.5 mmol) was then added with stirring, and the obtained solution was stirred at room temperature overnight. At the end of the reaction, the reaction solution was concentrated; ethyl acetate (300 mL) and a saturated sodium bicarbonate solution (300 mL) were added, a large number of bubbles were generated, and sodium bicarbonate solids were further added until the PH was greater than 7; the obtained solution was then extracted, the aqueous phase was washed with ethyl acetate (200 mL × 1), and the organic phases were combined, and evaporated to dryness, thus obtaining 62 g of Product 35-84.
35-85

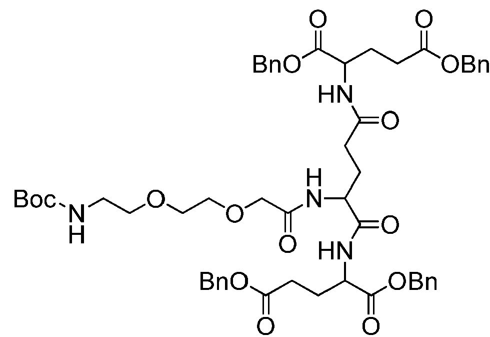

Product 37-151 (19.36 g, 73.5364 mmol), HBTU (41.83 g, 110.3045 mmol), HOBT (14.91 g, 110.3045 mmol) and Product 35-84 (61.95 g, 80.89 mmol) were added to a 1000 mL round-bottomed flask and dissolved with DMF (200 mL), and the obtained solution was stirred for about 30 min at -5 °C; DIEA (54.69 mL, 330.9136 mmol) was slowly added dropwise, the obtained solution was first stirred at -5 °C for 1 h, and then stirred overnight at room temperature to further react. At the end of the reaction, the reaction solution was transferred to a 2 L separatory funnel, a saturated sodium bicarbonate solution (500 mL) and ethyl acetate (300 mL) were then added, and the obtained solution was shaken and extracted to obtain an organic phase; the aqueous phase was washed with ethyl acetate (200 mL × 1), the obtained organic phases were combined and concentrated to a small amount; silica gel powder was added to the obtained solution, and the solution was then evaporated to dryness; the operations of dry sample loading, column chromatography, and elution with a mixed solution of 50%-70% ethyl acetate in petroleum ether were carried out, thus obtaining 51 g of Product 35-85 with a yield of 69%.
35-86

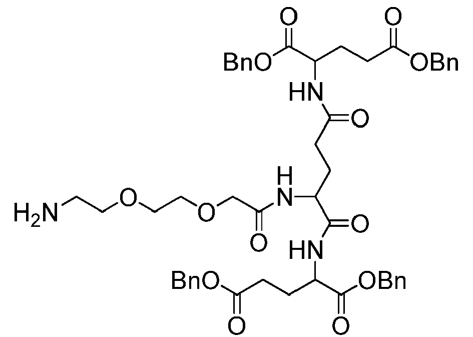

Product 35-85 (23.4 g, 23.14 mmol) was added in a 500 mL round-bottomed flask and then dissolved with dichloromethane (30 mL), trifluoroacetic acid (85.93 mL, 1157.13 mmol) was then added with stirring, and the obtained solution was stirred at room temperature overnight to react. At the end of the reaction, the reaction solution was concentrated; ethyl acetate (300 mL) and a saturated sodium bicarbonate solution (300 mL) were added, a large number of bubbles were generated, and sodium bicarbonate solids were further added until the PH was greater than 7; the obtained solution was then extracted, the aqueous phase was washed with ethyl acetate (200 mL × 1), and the obtained organic phases were combined and evaporated to dryness, thus obtaining 18.2 g of Product 35-86 with a yield of 86%.
35-88

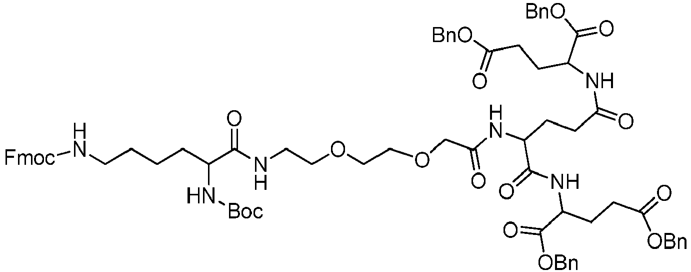

Product 35-86 (18.2 g, 19.98 mmol), HBTU (11.36 g, 29.97 mmol), HOBT (4.05 g, 29.97 mmol) and Boc-Lys (Fmoc)-OH (8.5 g, 18.16 mmol, purchased from Accela) were added to a 500 mL round-bottomed flask and dissolved with DMF (100 mL), and the obtained solution was stirred for about 30 min at 0 °C; DIEA (14.86 mL, 89.90 mmol) was slowly added dropwise, and at the end of the addition, the reaction solution was stirred overnight at 0 °C to further react. At the end of the reaction, the reaction solution was transferred to a 1 L separatory funnel, a saturated sodium bicarbonate solution (300 mL) and ethyl acetate (200 mL) were added, and the obtained solution was shaken and extracted to obtain an organic phase; the aqueous phase was washed with ethyl acetate (200 mL × 1), the obtained organic phases were combined and concentrated to a small amount; silica gel powder was added to the obtained solution, and the solution was evaporated to dryness, the operations of dry sample loading, column chromatography, and elution with a mixed solution of 80%-100% ethyl acetate in petroleum ether were carried out, thus obtaining 19.6 g of Product 35-88 with a yield of 79%.
35-89

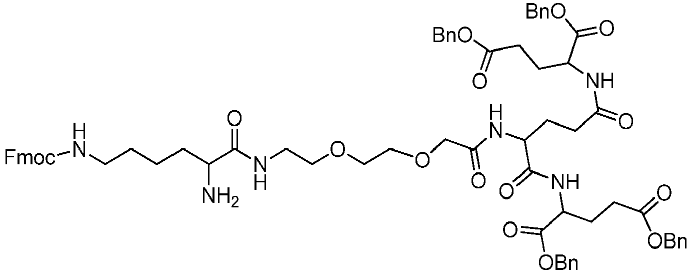

Product 35-88 (7.0 g, 5.1413 mmol) was added in a 250 mL flask and then dissolved with dichloromethane (20 mL), trifluoroacetic acid (5.7270 mL, 77.1191 mmol) was then added with stirring, and the reaction solution was stirred overnight at room temperature to react. At the end of the reaction, the reaction solution was concentrated, and then transferred to a 1 L separatory funnel; a saturated sodium bicarbonate solution (300 mL) and ethyl acetate (200 mL) were added, and the obtained solution was shaken and extracted to obtain an organic phase; the aqueous phase was washed with ethyl acetate (150 mL × 2), and the obtained organic phases were combined, washed with a saturated saline solution (200 mL × 2), then concentrated and evaporated to dryness, thus obtaining 6.4853 g of Product 35-89.
37-165

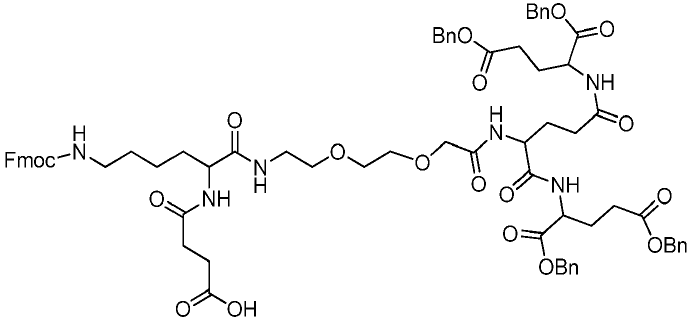

Product 35-89 (4.2 g, 3.33 mmol) was added in a 250 mL flask and then dissolved with DMF (30 mL); DIEA (4.4 mL, 26.64 mmol) was then added dropwise, and at the end of the addition, the mixed solution was stirred for 1 h; succinic anhydride (0.9997 g, 9.99 mmol) was then added, and the reaction solution was stirred at room temperature overnight to react. At the end of the reaction, the reaction solution was transferred to a 1 L separatory funnel and extracted with deionized water (200 mL) and ethyl acetate (200 mL) to obtain an organic phase; the aqueous phase was washed with ethyl acetate (200 mL × 4), and the obtained organic phases were combined, washed with a saturated saline solution (200 mL × 2), then concentrated and evaporated to dryness. 4 g of Product 37-165 was obtained with a yield of 89%.
37-166

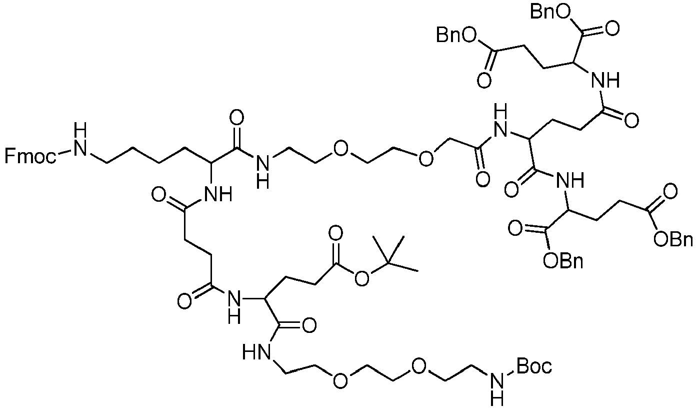

Product 37-165 (4 g, 2.938 mmol), Product 36-137 (1.2737 g, 2.938 mmol), HBTU (1.6713 g, 4.407 mmol) and HOBT (0.5955 g, 4.407 mmol) were added to a 500 mL flask and then dissolved with DMF (50 mL), and the obtained solution was stirred for about 10 min at 0 °C; DIEA (2.18 mL, 13.221 mmol) was slowly added dropwise, and at the end of the addition, the obtained solution was further stirred at 0 °C overnight to react. At the end of the reaction, the reaction solution was transferred to a 1 L separatory funnel; a saturated saline solution (200 mL) and ethyl acetate (250 mL) were added, and the obtained solution was shaken and extracted, then concentrated and evaporated to dryness, and dissolved with methanol (20 mL) and dichloromethane (100 mL); silica gel powder (20 g) was added to the obtained solution, and the solution was then evaporated to dryness to obtain a powdery solid; the operations of dry sample loading, column chromatography, and elution with a mixed solution (4%-7% methanol/dichloromethane) were carried out, and the elution product was dried in a vacuum oven, thus obtaining 3.9 g of Product 37-166 with a yield of 75%.

¹H-NMR (600 MHz, DMSO-d₆) δ 8.62- 8.43 (d, J = 7.4 Hz, 1H), 8.40 - 8.24 (d, J = 7.4 Hz, 1H), 8.08 - 7.85 (m, 7H), 7.75 - 7.62 (m, 3H), 7.46 - 7.23 (m, 24H), 6.75 (s, 1H), 5.19 - 5.00 (m, 8H), 4.48 - 4.13 (m, 9H), 4.00 - 3.79 (m, 3H), 3.66 - 3.45 (dd, J = 8.7, 4.3 Hz, 2H), 3.52 - 3.49 (d, J = 4.3 Hz, 2H), 3.47 (s, 4H), 3.40 - 3.36 (m, 6H), 3.23 - 3.14 (m, 4H), 3.09 - 3.02 (m, 2H), 2.97 - 2.92 (m, 2H), 2.46 - 2.35 (m, 8H), 2.24 - 2.15 (m, 4H), 2.04 (m, 2H), 1.97 - 1.82 (m, 4H), 1.78 - 1.61 (m, 3H), 1.47 - 1.23 (t, J = 7.8 Hz, 18H), 1.27 - 1.18 (m, 3H).
37-53

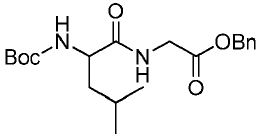

Boc-Leu-OH.H2O (40 g, 160.44 mmol, purchased from Innochem), Gly-OBn.TsOH (56.837 g, 168.462 mmol, purchased from Ark pharm), HBTU (66.93 g, 176.48 mmol) and HOBT (23.85 g, 176.48 mmol) were added to a 1000 mL flask and then dissolved with DMF (250 mL), and the obtained solution was stirred for about 20 min at -5 °C; then, DIEA (145.85 mL, 882.4356 mmol) was slowly added dropwise. At the end of the addition, the obtained solution was first stirred at - 5 °C for 1 h, and then stirred overnight at room temperature. At the end of the reaction, the reaction solution was transferred to a 2 L separatory funnel and extracted with a saturated sodium bicarbonate solution (250 mL) and ethyl acetate (300 mL) to obtain an organic phase; the aqueous phase was washed with ethyl acetate (200 mL × 3), and the obtained organic phases were combined, washed with a saturated saline solution (200 mL × 2), then concentrated and evaporated to dryness; the operations of dry sample loading, column chromatography, and elution with a mixed solution of 30%-40% ethyl acetate in petroleum ether were carried out, thus obtaining 60.7 g of Product 37-53.
37-54

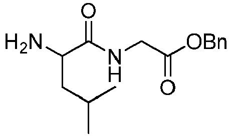

Product 37-53 (60.7 g, 160.44 mmol) was added in a 1000 mL flask, dichloromethane (40 mL) and TFA (95 mL, 1283.52.9 mmol) were then added, and the reaction solution was stirred overnight at room temperature to react. At the end of the reaction, the reaction solution was transferred to a 2 L separatory funnel and extracted with a saturated sodium bicarbonate solution (350 mL) and ethyl acetate (300 mL) to obtain an organic phase; the aqueous phase was washed with ethyl acetate (1500 mL × 2), and the obtained organic phases were combined, then concentrated and evaporated to dryness, thus obtaining 45 g of Product 37-54.
37-56

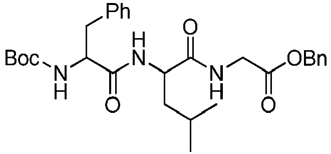

Product 37-54 (45 g, 160.44 mmol), Boc-Phe-OH (40.438 g, 152.42 mmol, purchased from aladdin), HBTU (66.93 g, 1276.48 mmol) and HOBT (23.85 g, 176.48 mmol) were added to a 1000 mL flask and then dissolved with DMF (250 mL), and the obtained solution was stirred for about 20 min at -5 °C; then, DIEA (119.85 mL, 722 mmol) was slowly added dropwise. At the end of the addition, the obtained solution was first stirred at -5 °C for 1 h, and then stirred overnight at room temperature. At the end of the reaction, the reaction solution was transferred to a 2 L separatory funnel and extracted with a saturated sodium bicarbonate solution (350 mL) and ethyl acetate (300 mL) to obtain an organic phase; the aqueous phase was washed with ethyl acetate (200 mL × 3), and the obtained organic phases were combined, washed with a saturated saline solution (250 mL × 2), then concentrated and evaporated to dryness, thus obtaining 84 g of Product 37-56.
37-59

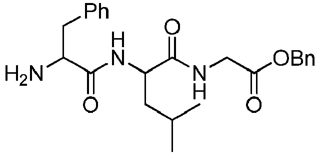

Product 37-56 (84 g, 160.44 mmol) was added in a 1000 mL flask, dichloromethane (40 mL) and TFA (95 mL, 1283.52.9 mmol) were then added, and the reaction solution was stirred overnight at room temperature to react. At the end of the reaction, the reaction solution was transferred to a 2 L conical flask, a saturated sodium bicarbonate solution (350 mL) was added and a large number of bubbles were generated, sodium bicarbonate solids were further added until the PH was greater than 7, and a powdery solid was then precipitated; the suction filtering was carried out and the filter cake was washed with purified water (100 mL × 2), and dried, thus obtaining 68 g of Product 37-59.
37-62

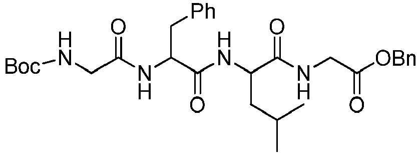

Product 37-59 (68.27 g, 152.42 mmol), Boc-Gly-OH (25.37 g, 144.799 mmol, purchased from aladdin), HBTU (63.58 g, 167.66 mmol) and HOBT (63.58 g, 167.66 mmol) were added to a 1000 mL flask and then dissolved with DMF (250 mL), and the obtained solution was stirred for about 20 min at -5 °C; then, DIEA (113.3 mL, 685.89 mmol) was slowly added dropwise. At the end of the addition, the obtained solution was first stirred at -5 °C for 1 h, and then stirred overnight at room temperature to further react. At the end of the reaction, the reaction solution was transferred to a 2 L separatory funnel and extracted with a saturated sodium bicarbonate solution (300 mL) and ethyl acetate (350 mL) to obtain an organic phase; the aqueous phase was washed with (200 mL × 3), and the obtained organic phases were combined, washed with a saturated saline solution (250 mL × 2), stayed still at room temperature for 1.5 h, and a powdery solid was then precipitated; the suction filtering was carried out and the filter cake was washed with ethyl acetate:petroleum ether (3: 7) (150 mL × 5), thus obtaining 72.8 g of Product 37-62.
37-149

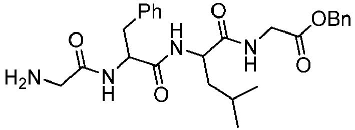

Product 37-62 (30 g, 51.4871 mmol) was added in a 250 mL flask and then dissolved with dichloromethane (20 mL), trifluoroacetic acid (30.6 mL, 411.9 mmol) was then added with stirring, and the reaction solution was stirred overnight at room temperature to react. At the end of the reaction, the reaction solution was concentrated; saturated sodium bicarbonate (200 mL) was added, and a large number of bubbles were generated; sodium bicarbonate solids were further added until the PH was greater than 7, and a powdery solid was then precipitated; the suction filtering was carried out and the filter cake was washed with deionized water (150 mL × 4), and dried, thus obtaining 24.85 g of Product 37-149.
37-152

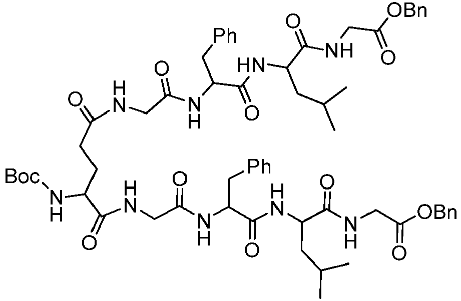

Boc-Glu-OH (5.7864 g, 23.4032 mmol, purchased from Ark pharm), Product 37-149 (24.85 g, 51.4871 mmol), HBTU (26.626 g, 70.2096 mmol) and HOBT (9.4874 g, 70.2096 mmol) were added to a 500 mL flask and then dissolved with DMF (150 mL), and the obtained solution was stirred for about 10 min at -5 °C; DIEA (34.8 mL, 210.628 mmol) was slowly added dropwise. At the end of the addition, the obtained solution was first stirred at -5 °C for 1 h, and then stirred overnight at room temperature. At the end of the reaction, the reaction solution was transferred to a 1 L separatory funnel; saturated sodium bicarbonate (200 mL) and ethyl acetate (300 mL) were added, and the obtained solution was shaken and extracted; the aqueous phase was washed with ethyl acetate (150 mL × 1), and the obtained organic phases were combined, then concentrated and evaporated to dryness, and the solid product was dissolved with methanol (20 mL) and dichloromethane (100 mL); silica gel powder (50 g) was then added to the obtained solution and the solution was evaporated to dryness to obtain a powdery solid; the operations of dry sample loading, column chromatography and gradient elution with a mixed solution (4%-8% methanol/dichloromethane) were carried out; the elution product was dried in a vacuum oven, thus obtaining 19.2 g of Product 37-152 with a yield of 69.8%.
37-154

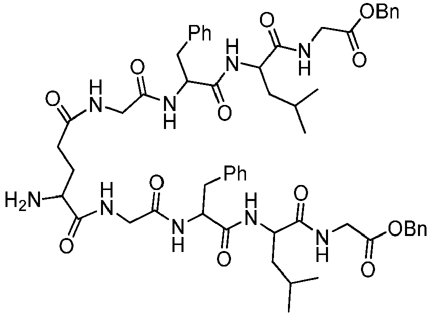

Product 37-152 (19.2 g, 16.3215 mmol), dichloromethane (30 mL) and trifluoroacetic acid (9.6966 mL, 130.5722 mmol) were added to a 1000 mL flask and the mixed solution was stirred at 0 °C overnight. At the end of the reaction, the reaction solution was concentrated to a small amount; methyl tert-butyl ether (200 mL) was added, and a solid was then precipitated, suction filtering was then carried out, and the filter cake was washed with methyl tert-butyl ether (100 mL × 3), and dried, thus obtaining 17.5 g of Product 37-154.
37-156

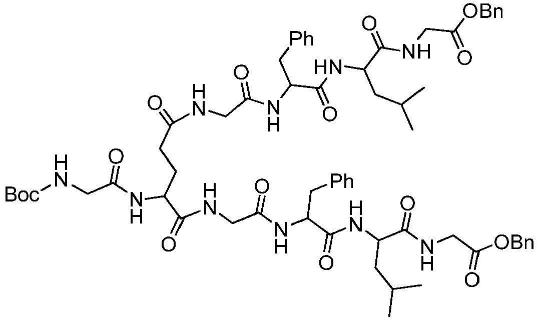

Boc-Gly-OH (3.4310 g, 19.5858 mmol, purchased from Ark Pharm), Product 37-154 (17.5 g, 16.3215 mmol), HBTU (9.2847 g, 24.4823 mmol) and HOBT (3.3083 g, 24.4823 mmol) were added to a 1000 mL flask and then dissolved with DMF (150 mL), and the obtained solution was stirred for about 10 min at -5 °C; then, DIEA (12.14 mL, 73.4468 mmol) was slowly added dropwise; at the end of the addition, the obtained solution was first stirred at -5 °C for 1 h, and then stirred overnight at room temperature to further react. At the end of the reaction, ethyl acetate (200 mL), methyl tert-butyl ether (200 mL) and n-hexane (300 mL) were added, and a powdery solid was then precipitated; the suction filtering was carried out and the filter cake was washed with methyl tert-butyl ether (100 mL × 3), and dried, thus obtaining 19.2 g of Product 37-156.
37-158

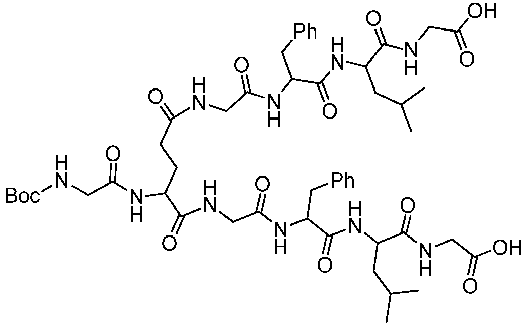

Product 37-156 (10.499 g, 8.5125 mmol) and 10% Pd/C (0.10 g) were added in a hydrogenation reactor and then dissolved with DMF (50 mL); hydrogen was introduced to a pressure of 1.8 MPa in the reactor, and the mixed solution then was stirred overnight at room temperature to react. At the end of the reaction, the reaction solution was filtered with diatomaceous earth; the diatomaceous earth was washed with DMF (20 mL × 3), and the DMF solution was combined, as the raw material for the next step.
37-159

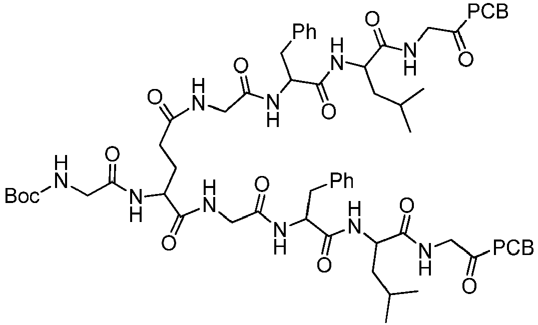

Product 37-158 (8.965 g, 0.4390 mmol), Palbociclib (Chinese name: palbociclib, PCB, 8 g, 17.8763 mmol), HBTU (9.6848 g, 25.5375 mmol) and HOBT (3.4509 g, 25.5375 mmol) were added to a 500 mL flask and then dissolved with DMF (130 mL), and the obtained solution was stirred for about 20 min at -5 °C; then, DIEA (12.6626 mL, 76.6125 mmol) was slowly added dropwise; at the end of the addition, the obtained solution was first stirred at -5 °C for 1 h, and then stirred overnight at room temperature to further react. At the end of the reaction, n-hexane (150 mL) and methyl tert-butyl ether (100 mL) were added, and a powdery solid was then precipitated; suction filtering was carried out and the filter cake was washed with methyl tert-butyl ether (100 mL × 3), and dried, thus obtaining 16.3 g of Product 37-159.
37-161

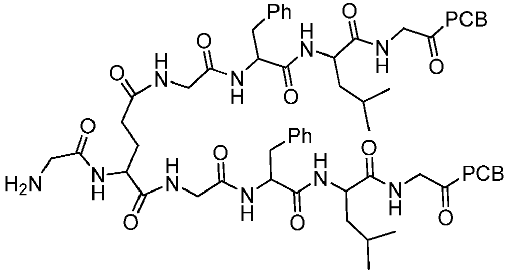

Product 37-159 (16.3 g, 8.5125 mmol) was added to a 500 mL flask and then dissolved with dichloromethane (30 mL), trifluoroacetic acid (18.964 mL, 255.375 mmol) was then added in the flask, and the mixed solution was stirred at room temperature to react overnight. At the end of the reaction, methyl tert-butyl ether (250 mL) was added and the powdery product was precipitated; suction filtering was then carried out, and the filter cake was washed with methyl tert-butyl ether (50 mL × 3), and then dissolved with dichloromethane (240 mL) and methanol (60 mL); silica gel powder (50 g) was added and the obtained solution was then evaporated to dryness to obtain a powdery solid; the operations of dry sample loading, column chromatography, and elution with a mixed solution (1% ammonia water: 5%-7% methanol/dichloromethane) were carried out; the elution product was then collected, concentrated, and evaporated to dryness, thus obtaining 11.2 g of Product 37-161 with a yield of 73%.
37-168

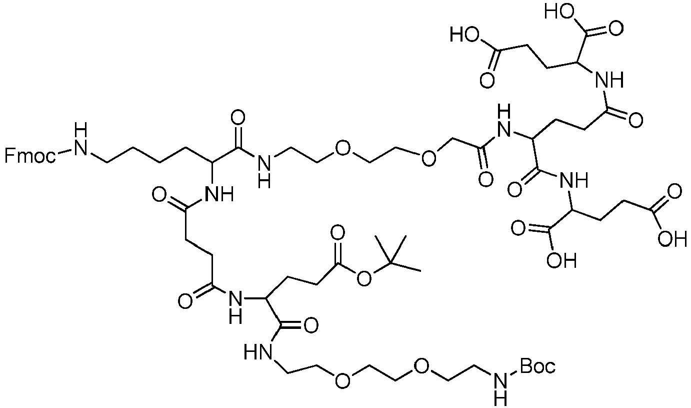

Product 37-166 (0.7801 g, 0.4390 mmol) and Pd/C (0.0500 g) were added in a hydrogenation reactor and then dissolved with DMF (30 mL); hydrogen was introduced to a pressure of 1.8 MPa in the reactor, and the mixed solution then was stirred overnight at room temperature to react. At the end of the reaction, the reaction solution was filtered with diatomaceous earth; the diatomaceous earth was washed with DMF (20 mL × 3), and the DMF solution was combined, as the raw material for the next step.
37-169

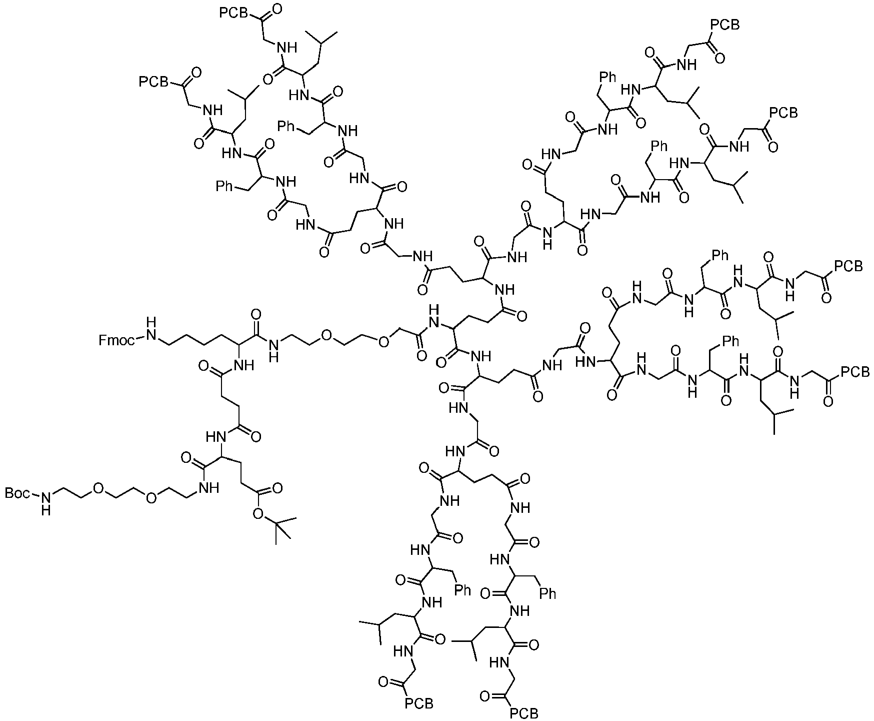

Product 37-168 (0.6218 g, 0.4390 mmol), Product 37-161 (3.5 g, 1.9315 mmol), HBTU (0.9988 g, 2.6338 mmol) and HOBT (0.3559 g, 2.6338 mmol) were added in a 250 mL flask and then dissolved with DMF (40 mL), and the obtained solution was stirred at -5 °C for 20 min; DIEA (1.306 mL, 7.9015 mmol) was slowly added dropwise; at the end of the addition, the reaction solution was first stirred at -5 °C for 40 min to react, and then stirred at room temperature overnight to further react. At the end of the reaction, n-hexane (150 mL) and methyl tert-butyl ether (40 mL) were added, and the reaction solution was precipitated twice to obtain a viscous oily product. Methyl tert-butyl ether (100 mL) was added to the obtained solution to separate out a solid; the solution was filtered and the filter cake was washed with methyl tert-butyl ether (50 mL × 2) and dried, thus obtaining 3.772 g of Product 37-169.
37-171

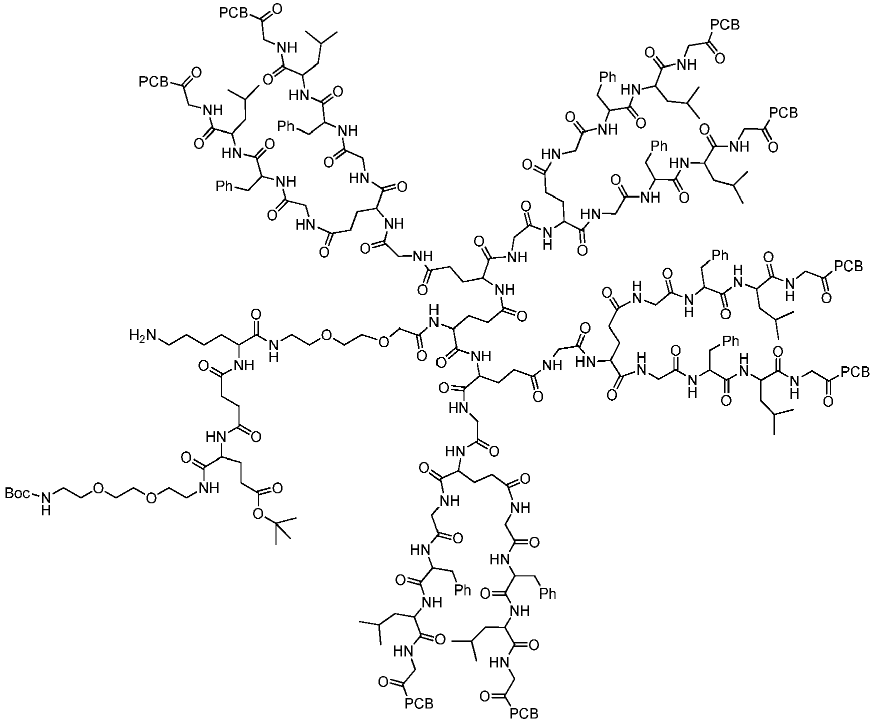

Product 37-169 (3.7772 g, 0.4390 mmol) was added in a 250 mL reaction flask and then dissolved with DMF (30 mL), morpholine (7.6 mL, 87.8 mmol) was then added, and the mixed solution was stirred for 1 h at room temperature to react. At the end of the reaction, methyl tert-butyl ether (200 mL) and n-hexane (300 mL) were added to layer the reaction solution, the supernatant was discarded, methyl tert-butyl ether (200 mL) and n-hexane (300 mL) were further added to the lower oily product, and such operations were repeated three times until a small amount of the oily product was obtained; methyl tert-butyl ether (250 mL) was added to precipitate a solid product, the solution was filtered and the filter cake was washed with methyl tert-butyl ether (150 mL × 2) and then dissolved with dichloromethane (160 mL) and methanol (40 mL); silica gel powder (30 g) was then added to the obtained solution and the solution was evaporated to dryness to obtain a powdery solid; the operations of dry sample loading, column chromatography and elution with a mixed solution (1% ammonia water and 5% - 8% methanol/dichloromethane) were carried out, the elution product was collected, concentrated and dried, thus obtaining 1.94 g of Product 37-171 with a yield of 53%.
45-57

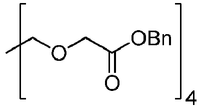

Pentaerythritol (2.5 g, 31.2344 mmol) was added to a 500 mL flask, tetrahydrofuran (150 mL) was then added, the solution was turbid, and the resulting solution was stirred at -5 °C for 30 min; a tetrahydrofuran solution (149 mL, 149 mmol) of potassium tert-butoxide (1 mol/L) was slowly added dropwise, and at the end of the addition, the obtained solution was further stirred for 5 h; benzyl bromoacetate (20.782 mL, 131.184 mmol) was slowly added dropwise, and the obtained solution was first stirred at -5 °C for 2 h, and then stirred at room temperature overnight. At the end of the reaction, the reaction solution was transferred to a 1 L separatory funnel; a saturated sodium bicarbonate solution (200 mL) and ethyl acetate (300 mL) were added, and the obtained solution was shaken and extracted; the aqueous phase was washed with ethyl acetate (150 mL × 1), the obtained organic phases were combined, then concentrated and evaporated to dryness, and the solid product was dissolved with methanol (20 mL) and dichloromethane (100 mL); silica gel powder (50 g) was then added to the obtained solution, and the solution was then evaporated to dryness to obtain a powdery solid; the operations of dry sample loading, column chromatography, and elution with a mixed solution (20%-40% petroleum ether/ethyl acetate) were carried out; the elution product was then collected, concentrated, and dried in a vacuum oven, thus obtaining 7.1 g of Product 45-57 with a yield of 31%.
37-172

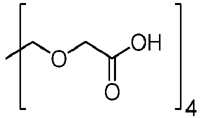

Product 45-57 (0.0412 g, 0.0565 mmol) and 10% Pd/C (0.10 g) were added in a hydrogenation reactor and then dissolved with DMF (50 mL); hydrogen was introduced to a pressure of 1.8 MPa in the reactor, and the mixed solution then was stirred overnight at room temperature to react. At the end of the reaction, the reaction solution was filtered with diatomaceous earth; the diatomaceous earth was washed with DMF (20 mL ×3 ), and the DMF solution was combined, as the raw material for the next step.
37-173

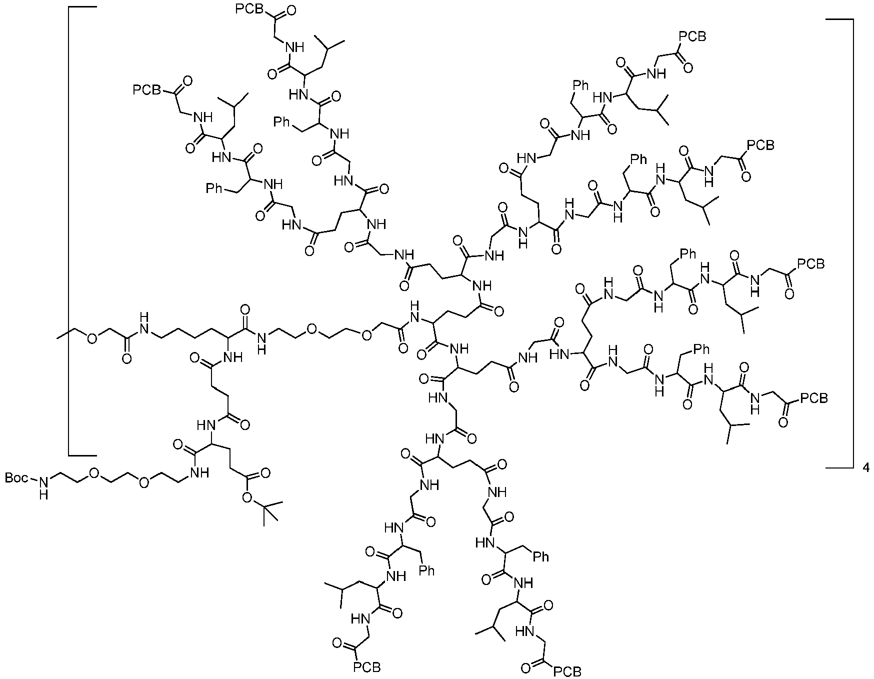

Product 37-172 (0.0208 g, 0.0565 mmol), Product 37-171 (1.94 g, 0.2318 mmol), HBTU (0.1287 g, 0.3393 mmol) and HOBT (0.0458 g, 0.3393 mmol) were added to a 250 mL flask and then dissolved with DMF (120 mL), and the obtained solution was stirred for about 15 min at -5 °C; then, DIEA (0.169 mL, 1.0177 mmol) was slowly added dropwise; at the end of the addition, the obtained solution was first stirred at -5 °C for 60 min to react, and then stirred at room temperature overnight to react. At the end of the reaction, n-hexane (200 mL) and methyl tert-butyl ether (300 mL) were added and a fibreized product was then precipitated; the suction filtering was carried out and the filter cake was washed with methyl tert-butyl ether (150 mL × 2), and dried, thus obtaining 1.91 g of Product 37-173.
37-176

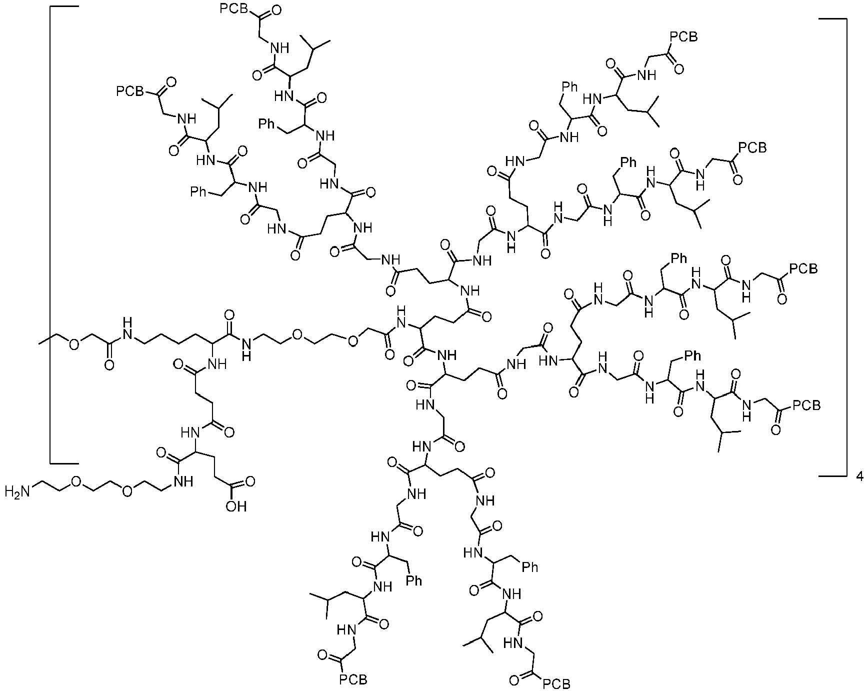

Product 37-173 (1.91 g, 0.05654 mmol), dichloromethane (15 mL) and trifluoroacetic acid (8.3976 mL, 113.08 mmol) were added to a 250 mL flask, and the reaction solution was stirred at room temperature overnight to react. At the end of the reaction, the reaction solution was concentrated to a small amount, methyl tert-butyl ether (150 mL) was added, and a powdery solid was then precipitated; the suction filtering was carried out and the filter cake was washed with methyl tert-butyl ether (50 mL × 3), and then dissolved with a solution of methanol (30 mL) and dichloromethane (120 mL); silica gel powder (15 g) was added to the obtained solution, and the solution was then evaporated to dryness to obtain a powdery solid; the operations of dry sample loading, column chromatography, and elution with a mixed solution (1% ammonia water/5%-10% methanol/dichloromethane) were carried out; the elution product was then collected, concentrated, and evaporated into a solid; the solid was dried in a vacuum oven, thus obtaining 1.143 g of Product 37-176 with a yield of 61%.
37-179

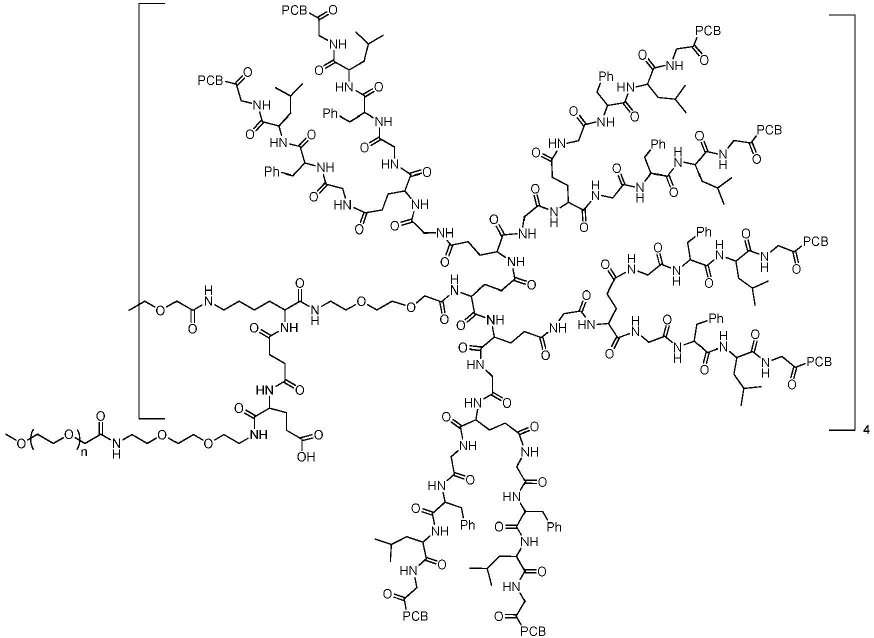

Product 37-176 (1.1435 g, 0.0345 mmol) was added to a 250 mL flask and then dissolved with DMF (80 mL); DIEA (0.479 mL, 2.898 mmol) was slowly added dropwise, M-SCM-10K (2.19 g, 0.2069 mmol, purchased from JenKem) was then added, and the obtained solution was slowly stirred at room temperature to react for 7 days in the dark. At the end of the reaction, n-hexane (200 mL) and methyl tert-butyl ether (100 mL) were added to layer the reaction solution, the supernatant was discarded; n-hexane (200 mL) and methyl tert-butyl ether (100 mL) were added again to the lower oily product, and such operations were repeated three times to obtain an oily product, and the oily product was dissolved with a solution of methanol (30 mL) and dichloromethane (120 mL); silica gel powder (20 g) was added to the obtained solution, and the solution was then evaporated to dryness to obtain a powdery solid; the operations of dry sample loading, column chromatography, and elution with a mixed solution (1% ammonia water/5%-8% methanol/dichloromethane) were carried out; the elution product was then collected, concentrated, and evaporated into a solid; the solid was dried in a vacuum oven, thus obtaining 1.05 g of Product 37-179 with a yield of 42%.
37-184

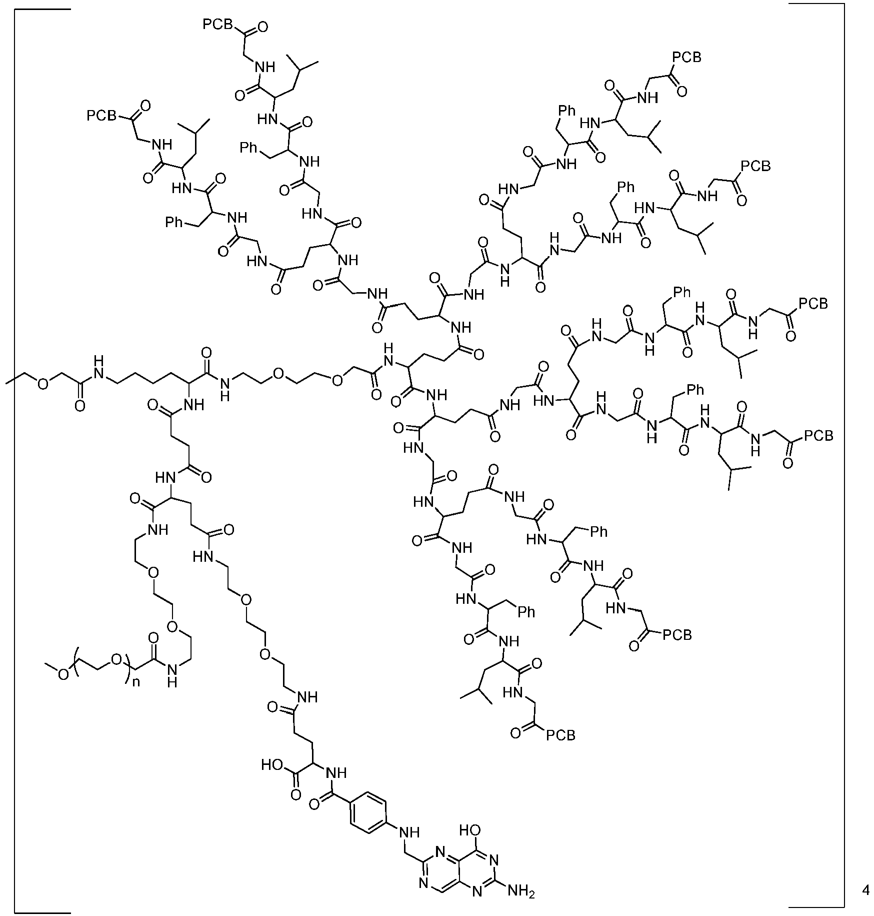

Product 37-179 (1.05 g, 0.0140 mmol), Product 37-163 (0.1599 g, 0.2798 mmol), HBTU (0.32 g, 0.84 mmol) and HOBT (0.1135 g, 0.84 mmol) were added to a 250 mL flask, and then dissolved with DMF (35 mL), and the obtained solution was stirred for about 20 min at -5 °C; DIEA (0.196 mL, 1.1190 mmol) was slowly added dropwise, and at the end of the addition, the obtained solution was stirred at -5 °C for 40 min, and then stirred at room temperature overnight to react. At the end of the reaction, n-hexane (150 mL) and methyl tert-butyl ether (50 mL) were added to layer the reaction solution, the supernatant was discarded; n-hexane (200 mL) and methyl tert-butyl ether (100 mL) were added again to the lower oily product; such operations were repeated three times to obtain a viscous oily product; the oily product was dissolved with a solution of methanol (30 mL) and dichloromethane (120 mL); silica gel powder (15 g) was added to the obtained solution, and the solution was then evaporated to dryness to obtain a powdery solid; the operations of dry sample loading, column chromatography, and elution with a mixed solution (1% ammonia water/7%-10% methanol/dichloromethane) were carried out; the elution product was then collected, concentrated, and evaporated into a solid; the solid was dried in a vacuum oven, and then dissolved in ethanol (5 mL) and dichloromethane (15 mL); methyl tert-butyl ether (50 mL) was added and a solid product was precipitated; the filter cake was washed with methyl tert-butyl ether (50 mL × 2) and dried, thus obtaining 0.36 g of Product 37-184 with a yield of 33%.

¹H-NMR (600 MHz, DMSO-d₆) δ 11.52- 11.23 (m, 10H), 11.15 - 11.02 (m, 2H), 10.76 - 10.41 (m, 3H), 10.25 -10.01 (m, 28H), 9.22 -8.86 (m, 29H), 8.75 -8.46 (m, 10H), 8.23 -8.04 (m, 256H), 7.76 - 7.45 (m, 60H), 7.37 - 6.91 (m, 179H), 6.73 - 6.45 (m, 22H), 5.92 -5.74 (m, 26H), 4.73 - 4.53 (m, 48H), 4.39 - 4.21 (m, 68H), 4.11 - 3.91 (m, 72H), 3.72 - 3.65 (m, 221H), 3.61 - 3.43 (m, 3847H), 3.25 - 3.12 (m, 161H), 3.05 - 2.92 (m, 46H), 2.83 - 2.71 (m, 67H), 2.62 - 2.53 (m, 11H), 2.52 - 2.43 (m, 93H), 2.40 - 2.34 (m, 89H), 2.23 -2.18 (m, 59H), 2.12 - 2.04 (m, 49H), 1.92 - 1.75 (m, 180H), 1.64 - 1.46 (m, 152H), 1.40 - 1.36 (m, 16H), 1.31 - 1.24 (m, 139H), 1.21 - 0.91 (m, 22H), 0.95 - 0.78 (m, 178H).

### Chemical synthesis route of 36-257

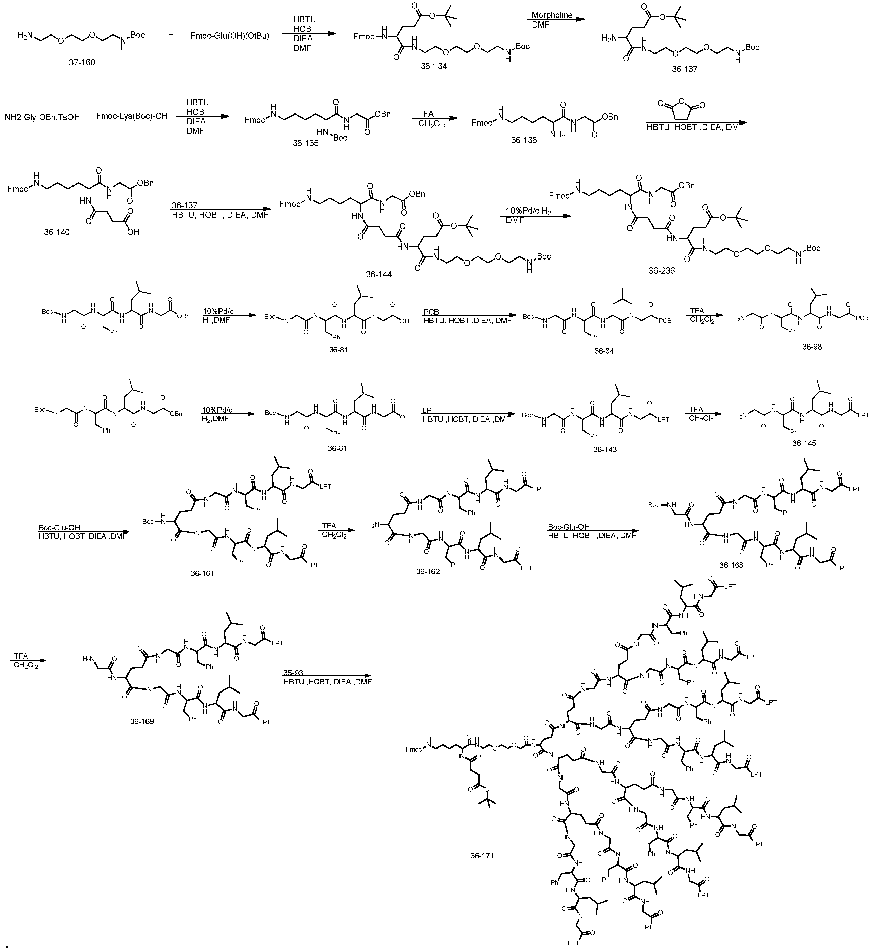

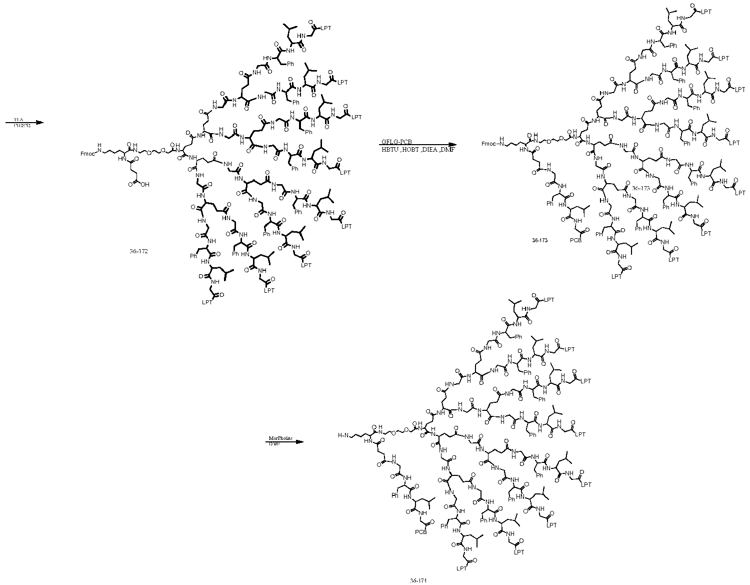

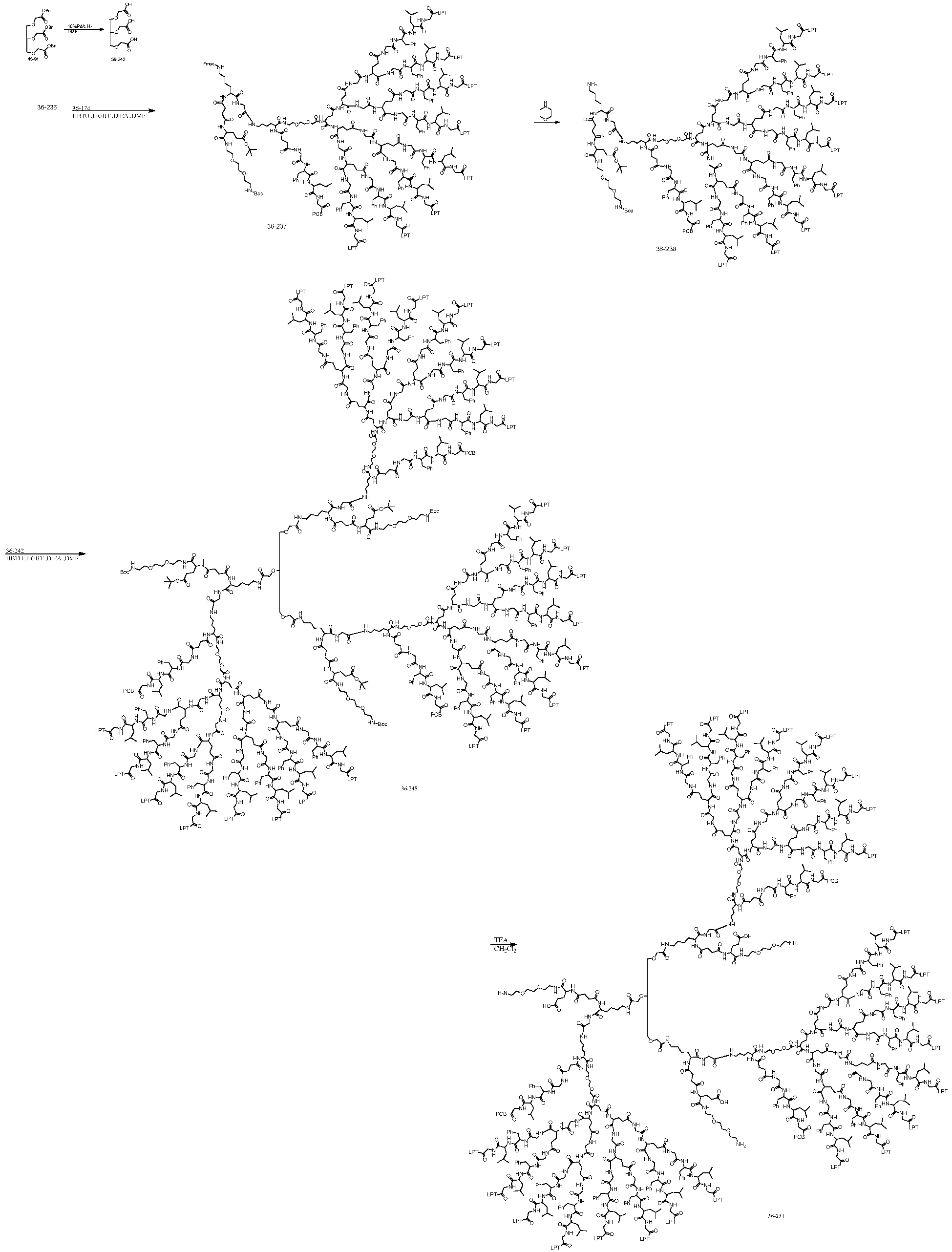

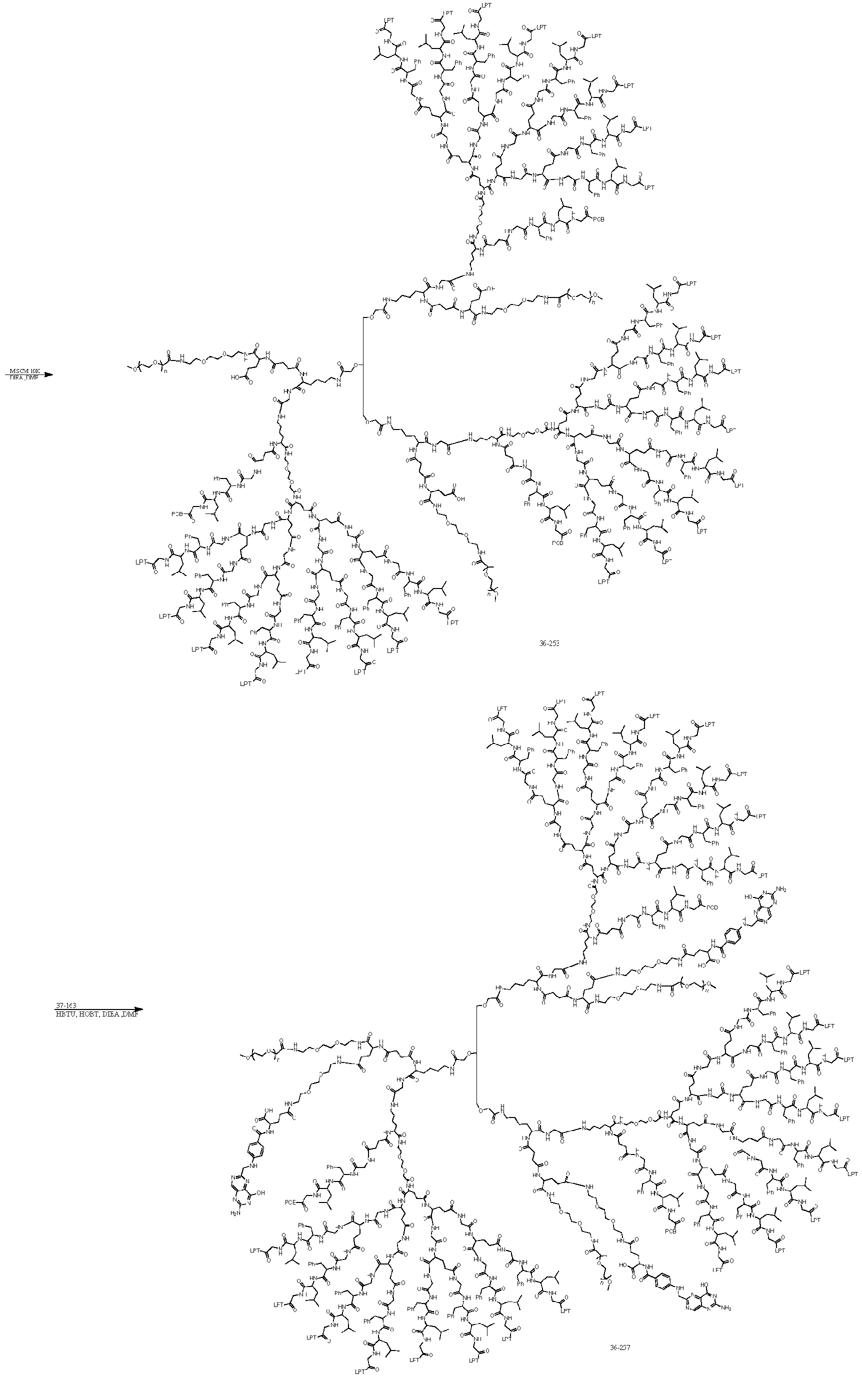

36-81

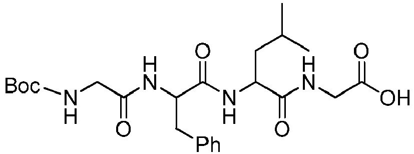

Boc-GFLG-OBn (14.95 g, 25.654 mmol, prepared according to the preparation method of Product 37-62) was added to a hydrogenation reactor, 10%Pd/C (0.300 g) was then added and the obtained mixture was dissolved with DMF (40 mL); the air in the reactor was then pumped out to reach a vacuum state by a water pump; hydrogen was introduced to a pressure of 0.16 MPa in the reactor, hydrogen was then discharged, and the reactor was pumped to reach a vacuum state by the water pump; hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth; the filter cake was washed with DMF (20 mL × 3), and the DMF solution was combined, as the raw material for the next step.
36-84

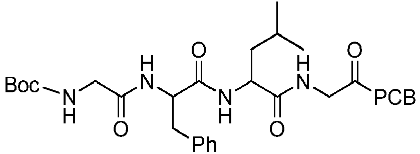

Product 36-81 (5.3 g, 10.7255 mmol), Palbociclib (PCB, 4 g, 8.9380 mmol), HBTU (5 g, 13.4069 mmol) and HOBT (1.8 g, 13.4069 mmol) were added in a 500 mL flask, and the obtained solution was stirred for about 20 min at -5 °C; then, DIEA (6.6 mL, 40.2208 mmol) was slowly added dropwise; the reaction continued at -5 °C for 1 h, and the reaction solution was stirred at room temperature overnight to react. At the end of the reaction, n-hexane (150 mL) and methyl tert-butyl ether (30 mL) were added for precipitation, the supernatant was discarded; n-hexane (150 mL) and methyl tert-butyl ether (50 mL) were added again to the lower oily solution, and such operations were repeated three times to obtain an oily product; methyl tert-butyl ether (200 mL) was added, and a solid product was precipitated; the obtained solid product was filtered by suction and dried, thus obtaining 15.9 g of Product 36-84.
36-98

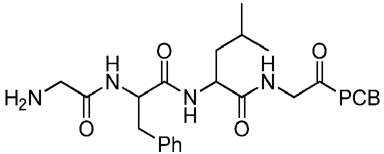

Product 36-84 (15.9 g, 8.938 mmol) was added to a 500 mL flask and then dichloromethane (10 mL) and TFA (6.6 mL, 89.38 mmol) were added in the flask, and the mixed solution was stirred at room temperature to react overnight. At the end of the reaction, the reaction solution was concentrated to 10 mL, methyl tert-butyl ether (200 mL) was then added and the powdery product was precipitated; suction filtering was then carried out, and the filter cake was washed with methyl tert-butyl ether (50 mL × 3), and then dissolved with 200 mL of a mixed solution (20% methanol: 80% dichloromethane); silica gel powder (60 mL) was added and the obtained solution was then evaporated to dryness to obtain a powdery solid; the operations of dry sample loading, column chromatography, and elution with a mixed solution (0.5% ammonia water: 2%-5% methanol/dichloromethane) were carried out; the elution product was then collected, concentrated, and evaporated to dryness, thus obtaining 8.5 g of Product 36-98 with a yield of 91.4%.
36-134

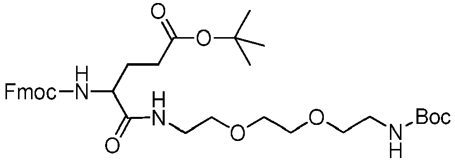

Product 37-160 (2.74 g, 11.0341 mmol), Fmoc-Glu(OH)(OtBu) (4.93 g, 11.5858 mmol, purchased from Innochem), HBTU (6.3 g, 16.5512 mmol) and HOBT (2.2 g, 16.5512 mmol) were added in a 500 mL flask, and the obtained solution was stirred for about 20 min at -5 °C; then, DIEA (8.2 mL, 49.6537 mmol) was slowly added dropwise; the reaction continued for 1 h, and the reaction solution was stirred at room temperature overnight to react. At the end of the reaction, the reaction solution was transferred to a 1 L separatory funnel and extracted with NaHCO₃(200 mL) and ethyl acetate (200 mL) to obtain an organic phase; the aqueous phase was washed with ethyl acetate (200 mL × 2), and the obtained organic phases were combined and washed with a saturated saline solution (200 mL × 2); the obtained organic phases were combined, washed with a saturated sodium chloride solution (100 ml × 2), and evaporated to dryness. 9.74 g of Product 36-134 was obtained.
36-135

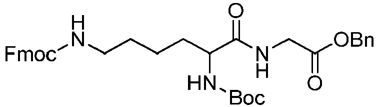

Fmoc-Lys(Boc)-OH (2 g, 4.2686 mmol), NH₂-Gly-OBn.TsOH (1.44 g, 4.2686 mmol, purchased from Innochem), HBTU (2.4 g, 6.4029 mmol) and HOBT (0.9 g, 6.4029 mmol) were added in a 500 mL flask, and the obtained solution was stirred for about 20 min at -5 °C; DIEA (3.2 mL, 19.2086 mmol) was slowly added dropwise; the reaction continued at -5 °C for 1 h, and the reaction solution was stirred at room temperature overnight to react. At the end of the reaction, the reaction solution was transferred to a 1 L separatory funnel and extracted with NaCl (200 mL) and ethyl acetate (200 mL) to obtain an organic phase; the aqueous phase was washed with ethyl acetate (200 mL × 2), and the obtained organic phases were combined and washed with a saturated saline solution (200 mL × 2); the obtained organic phases were combined and washed with a saturated sodium chloride solution (100 ml × 2), and evaporated to dryness. 4.04 g of Product of 36-135 was obtained.
36-140

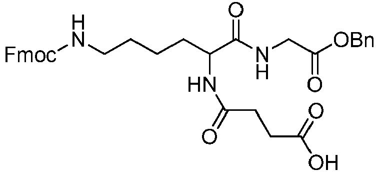

Product 36-136 (2.7 g, 4.2686 mmol) was added to a 250 mL flask and dissolved with 20 mL of DMF, and the obtained solution was stirred at -5 °C for 30 min to react; DIEA (3.5 mL, 21.3429 mmol) was slowly added dropwise, at the end of the addition, succinic anhydride (1.28 g, 12.8057 mmol) was then added when the temperature of the reaction solution was room temperature, and then the reaction solution was stirred overnight to react. At the end of the reaction, the reaction solution was transferred to a 1 L separatory funnel and extracted with saturated sodium chloride (300 mL) and ethyl acetate (200 mL) to obtain an organic phase; the aqueous phase was washed with ethyl acetate (200 mL × 2); the obtained organic phases were combined, washed with a saturated saline solution (200 mL × 2), and evaporated to dryness; the solid product was dissolved with methanol:dichloromethane (1:5); 40 ml of silica gel powder was added to the obtained solution, and the solution was evaporated to dryness, the operations of dry sample loading, column chromatography, and gradient elution with (5%-10% methanol: 95%-90% dichloromethane) were carried out; the elution product was then collected and concentrated, thus obtaining 1.9 g of Product 36-140 with a yield of 73%.
36-143

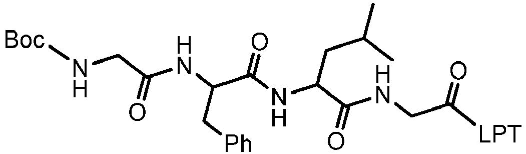

Product 36-81 (8.45 g, 17.1618 mmol), Lapatinib (LPT, 8.3 g, 14.3015 mmol), HBTU (8.1 g, 21.4522 mmol) and HOBT (2.9 g, 21.4522 mmol) were added in a 500 mL flask, and the obtained solution was stirred for about 20 min at -5 °C; DIEA (10.6 mL, 64.3567 mmol) was slowly added dropwise; the reaction continued at -5 °C for 1 h, and the reaction solution was stirred at room temperature overnight to react. At the end of the reaction, the reaction solution was transferred to a 1 L separatory funnel and extracted with NaHCO₃ (200 mL) and ethyl acetate (200 mL) to obtain an organic phase; the aqueous phase was washed with ethyl acetate (200 mL × 2), and the obtained organic phases were combined and washed with a saturated saline solution (200 mL × 2); the obtained organic phases were combined, washed with a saturated sodium chloride solution (100 ml × 2), and evaporated to dryness, thus obtaining 14 g of Product 36-143 with a yield of 92.8%.
36-144

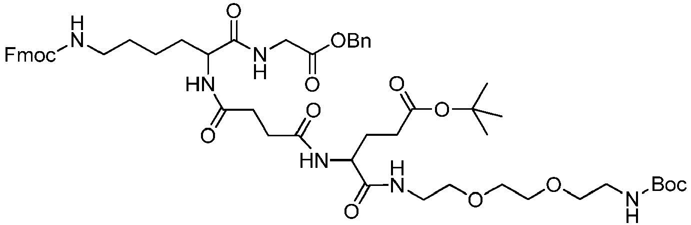

Product 36-140 (1.41 g, 2.2838 mmol), Product 36-137 (1 g, 3.3066 mmol), HBTU (1.3 g, 3.4256 mmol) and HOBT (0.5 g, 3.4256 mmol) were added to a 250 mL flask and dissolved with 30 mL of DMF, and the obtained solution was stirred at -5 °C for 30 min to react; DIEA (1.7 mL, 10.2769 mmol) was slowly added dropwise, and at the end of the addition, the reaction continued for 1 h, and the reaction solution was stirred at room temperature overnight to react. At the end of the reaction, the reaction solution was transferred to a 1 L separatory funnel and extracted with saturated sodium bicarbonate (200 mL) and ethyl acetate (200 mL) to obtain an organic phase; the aqueous phase was washed with ethyl acetate (200 mL × 2), and the obtained organic phases were combined, washed with a saturated saline solution (200 mL × 2), evaporated to dryness, and the solid product was dissolved with methanol:dichloromethane (1:5); 30 ml of silica gel powder was then added to the obtained solution, and the solution was then evaporated to dryness; the operations of dry sample loading, column chromatography, and gradient elution with (2%-4% methanol: 98%-96% dichloromethane) were carried out; the elution solution was then collected and concentrated, thus obtaining 1.398 g of Product 36-144 with a yield of 59.39%. ¹H-NMR (600 MHz, DMSO-d₆) δ 8.34 (s, 1H), 8.09 - 8.00 (m, 2H), 7.89 (m, 3H), 7.69 (m, 2H), 7.41 (s, 2H), 7.35 (m, 7H), 7.25 (s, 1H), 6.75 (s, 1H), 5.12 (s, 2H), 4.25 (m, 5H), 3.86 (s, 2H), 3.48 (s, 4H), 3.36 (s, 4H), 3.18 (m, 2H), 3.06 (m, 2H), 2.94 (m, 2H), 2.38 (s, 4H), 2.19 (s, 2H), 1.86 (s, 1H), 1.68 (m, 2H), 1.56 - 1.42 (m, 1H), 1.37 (m, 18H), 1.23 (s, 4H).
36-145

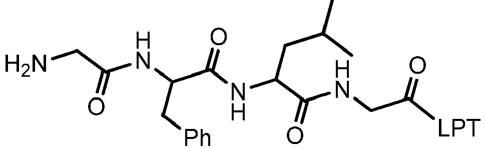

Product 36-143 (14 g, 14.3015 mmol) was added to a 500 mL flask and then dissolved with dichloromethane (20 mL), TFA (10.6 mL, 143.015 mmol) was then added in the flask, and the mixed solution was stirred at room temperature to react overnight. At the end of the reaction, the reaction solution was concentrated to 10 mL, methyl tert-butyl ether (200 mL) was then added and the powdery product was precipitated; suction filtering was then carried out, and the filter cake was washed with methyl tert-butyl ether (50 mL × 3), and then dissolved with 200 mL of a mixed solution (20% methanol: 80% dichloromethane; silica gel powder (60 mL) was added and the obtained solution was then evaporated to dryness to obtain a powdery solid; the operations of dry sample loading, column chromatography, and elution with a mixed solution (1% ammonia water: 5%-10% methanol/dichloromethane) were carried out; the elution product was then collected, concentrated, and evaporated to dryness, thus obtaining 6.2 g of Product 36-145 with a yield of 92.1%.
36-161

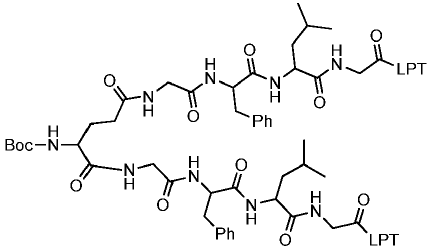

Product 36-145 (12.6 g, 13.1870 mmol), Boc-Glu-OH (1.55 g, 6.2795 mmol), HBTU (7.1 g, 18.8385 mmol) and HOBT (2.5 g, 18.8385 mmol) were added to a 250 mL flask and dissolved with 30 mL of DMF, and the obtained solution was stirred at -5 °C for 30 min to react; then, DIEA (9.3 mL, 56.5155 mmol) was slowly added dropwise, and at the end of the addition, the reaction continued for 1 h, and the reaction solution was stirred at room temperature overnight to react. At the end of the reaction, the reaction solution was transferred to a 1 L separatory funnel and extracted with NaHCO₃ (200 mL) and ethyl acetate (200 mL) to obtain an organic phase; the aqueous phase was washed with ethyl acetate (200 mL × 2); the obtained organic phases were combined, washed with a saturated saline solution (200 mL × 2); the obtained organic phases were combined, washed with a saturated sodium chloride solution (100 ml × 2), and evaporated to dryness, thus obtaining 18 g of Product 36-161.
36-162

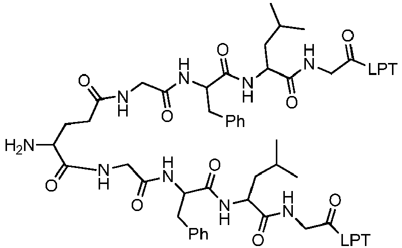

Product 36-161 (13.3 g, 6.2795 mmol) was added in a 500 mL flask, dichloromethane (20 mL) and TFA (4.66 mL, 62.795 mmol) were added in sequence, and the reaction solution was stirred overnight at room temperature to react. At the end of the reaction, the reaction solution was concentrated to 10 mL; methyl tert-butyl ether (200 mL) was then added and the powdery product was precipitated; suction filtering was then carried out, and the filter cake was washed with methyl tert-butyl ether (50 mL × 3); the solid was collected, thus obtaining 9.3 g of Product 36-162 with a yield of 70%.
36-168

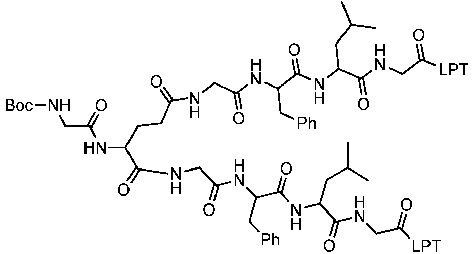

Product 36-162 (10.8 g, 5.3410 mmol), Boc-Gly-OH (1.03 g, 5.8751 mmol), HBTU (3 g, 8.0115 mmol) and HOBT (1.08 g, 8.0115 mmol) were added to a 250 mL flask and dissolved with 30 mL of DMF, and the obtained solution was stirred at -5 °C for 30 min to react; then, DIEA (4 mL, 24.0347 mmol) was slowly added dropwise; at the end of the addition, the reaction continued for 1 h, and the reaction solution was stirred at room temperature overnight to react. At the end of the reaction, n-hexane (100 mL × 3) was added to precipitate the reaction solution; the lower oily solid was dissolved with a small amount of dichloromethane; methyl tert-butyl ether was added, and a solid product was precipitated; the obtained solid product was dried, thus obtaining 13.6 g of Product 36-168.
36-169

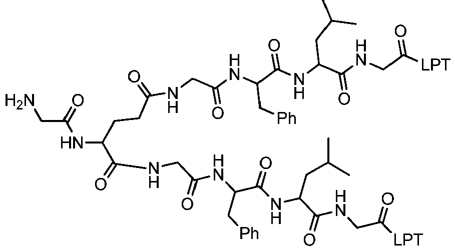

Product 36-168 (13.6 g, 5.3410 mmol) was added in a 500 mL flask, dichloromethane (20 mL) and TFA (3.8 mL, 53.410 mmol) were added in sequence, and the reaction solution was stirred overnight at room temperature to react. At the end of the reaction, the reaction solution was concentrated to 10 mL, methyl tert-butyl ether (200 mL) was then added and the powdery product was precipitated; suction filtering was then carried out, and the filter cake was washed with methyl tert-butyl ether (50 mL × 3), and then dissolved with a mixed solution (20% methanol: 80% dichloromethane) (200 mL); silica gel powder (100 mL) was added to the obtained solution, and the solution was then evaporated to dryness to obtain a powdery solid; the operations of dry sample loading, column chromatography, and elution with a mixed solution (0.5%-1% ammonia water/4%-7% methanol/dichloromethane) were carried out; the elution product was then collected, concentrated, and evaporated to dryness, thus obtaining 9.4 g of Product 36-169 with a yield of 84.7%.
36-171

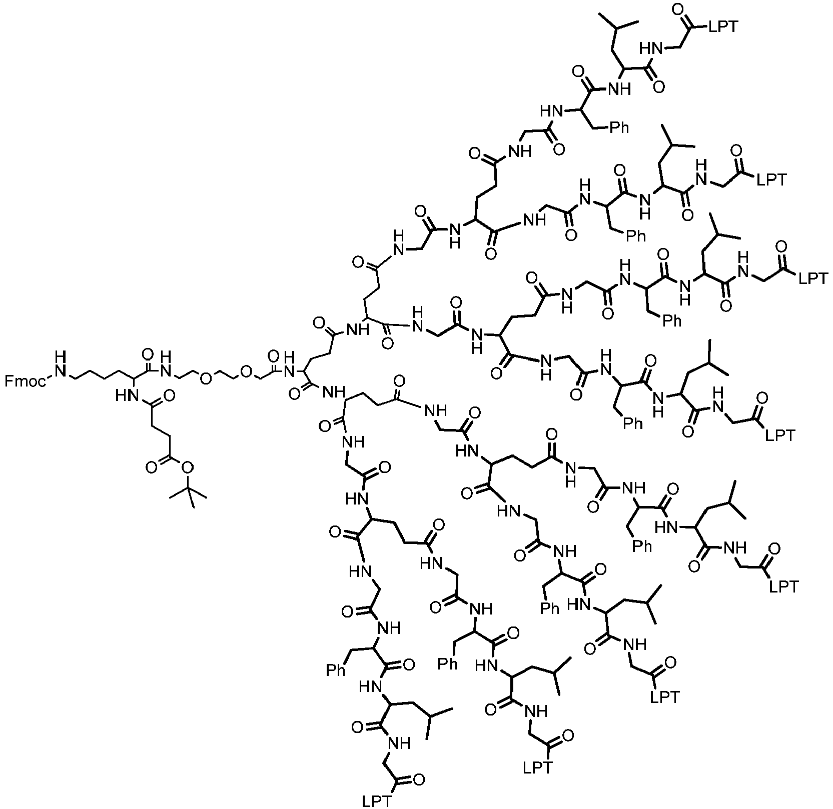

Product 35-93 (0.61 g, 0.5772 mmol), Product 36-169 (6 g, 2.8858 mmol), HBTU (1.3 g, 3.4630 mmol) and HOBT (0.46 g, 3.4630 mmol) were added to a 250 mL flask and dissolved with 30 mL of DMF, and the obtained solution was stirred at -5 °C for 30 min to react; then, DIEA (1.7 mL, 10.3889 mmol) was slowly added dropwise; at the end of the addition, the reaction continued for 1 h, and the reaction solution was stirred at room temperature overnight to react. At the end of the reaction, n-hexane (100 mL × 3) was added for precipitation; the lower oily solid was dissolved with a small amount of dichloromethane; methyl tert-butyl ether was added, and a solid product was precipitated; the obtained solid product was dried, thus obtaining 7.1 g of Product 36-171.
36-172

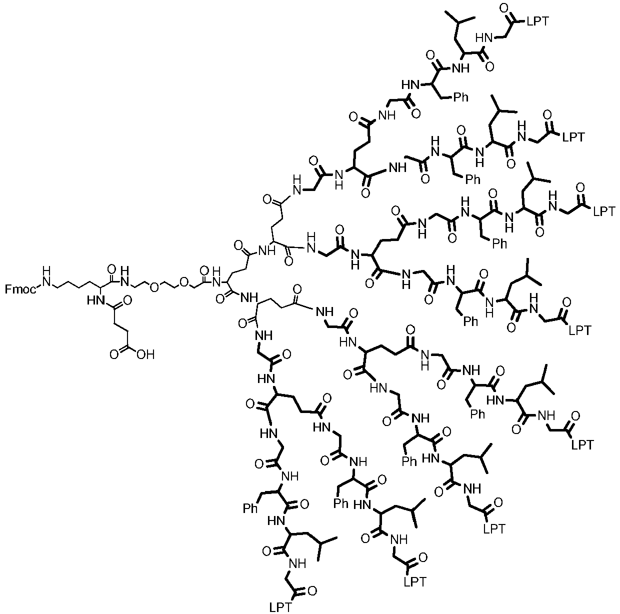

Product 36-171 (5.4 g, 0.5772 mmol) was added in a 250 mL flask and then dichloromethane (20 ml) and TFA (0.4 mL, 5.772 mmol) were added in sequence, and the mixed solution was stirred for 1 h at room temperature to react. At the end of the reaction, n-hexane (100 mL × 3) was added for precipitation, and the lower oily solid was dissolved with a small amount of dichloromethane; methyl tert-butyl ether was added, and a solid product was precipitated; the obtained solid product was filtered by suction and dried, thus obtaining 8 g of Product 36-172.
36-173

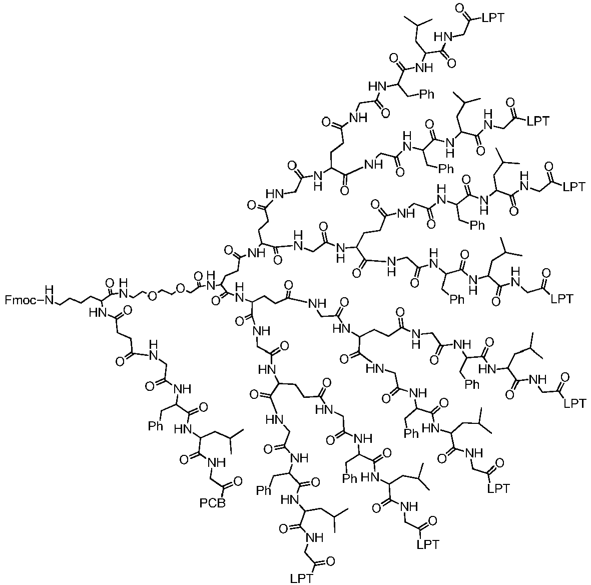

Product 36-98 (0.6 g, 0.6926 mmol), Product 36-172 (5.3 g, 0.5772 mmol), HBTU (0.3 g, 0.8658 mmol) and HOBT (0.1 g, 0.8658 mmol) were added to a 250 mL flask and dissolved with 30 mL of DMF, and the obtained solution was stirred at -5 °C for 30 min to react; then, DIEA (0.5 mL, 3.1746 mmol) was slowly added dropwise; at the end of the addition, the reaction continued for 1 h, and the reaction solution was stirred at room temperature overnight to react. At the end of the reaction, n-hexane (100 mL × 3) was added for precipitation, and the lower oily solid was dissolved with a small amount of dichloromethane; methyl tert-butyl ether was added, and a solid product was precipitated; the obtained solid product was dried, thus obtaining 9 g of Product 36-173.
36-174

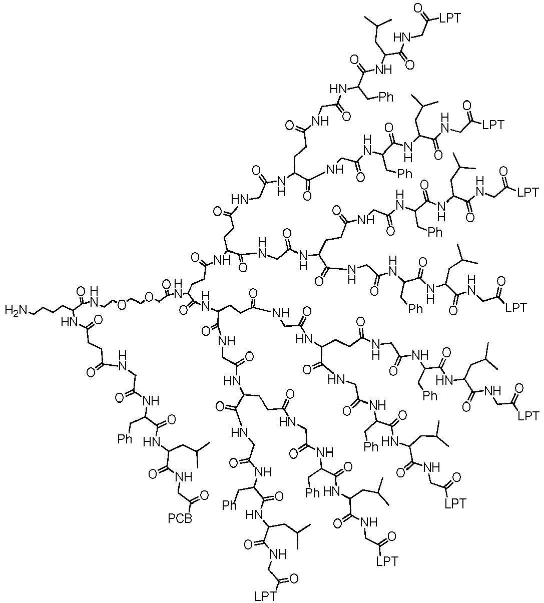

Product 36-173(9 g, 0.5772 mmol) was added in a 250 mL flask and then dissolved with DMF, morpholine (0.5 mL, 5.772 mmol) was added, and the reaction solution was stirred overnight at room temperature to react. At the end of the reaction, the reaction solution was concentrated to 10 mL, methyl tert-butyl ether (200 mL) was then added and the powdery product was precipitated; suction filtering was then carried out, and the filter cake was washed with methyl tert-butyl ether (50 mL× 3), and then dissolved with a mixed solution (20% methanol: 80% dichloromethane) (200 mL); silica gel powder (40 mL) was added to the obtained solution, and the solution was then evaporated to dryness to obtain a powdery solid; the operations of dry sample loading, column chromatography, and elution with a mixed solution (1% ammonia water/5%-10% methanol/dichloromethane) were carried out; the elution product was then collected, concentrated, and evaporated to dryness, thus obtaining 4 g of Product 36-174 with a yield of 70.5%.
36-236

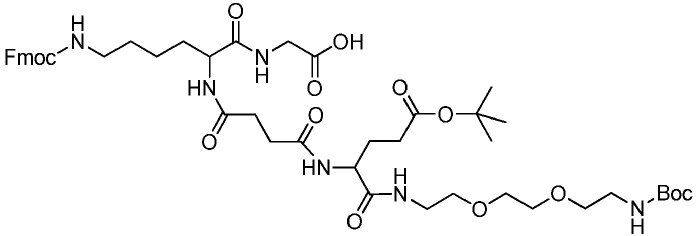

Product 36-144 (0.5 g, 0.4848 mmol) and 10% Pd/C (0.0200 g) were added in a reactor and then dissolved with DMF (30 mL); the air in the reactor was then pumped out to reach a vacuum state by a water pump; hydrogen was introduced to a pressure of 0.16 MPa in the reactor, and then discharged, and the reactor was pumped to reach a vacuum state by the water pump; hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth; the filter cake was washed with DMF (20 mL × 3), and the DMF solution was combined, as the raw material for the next step.
36-237

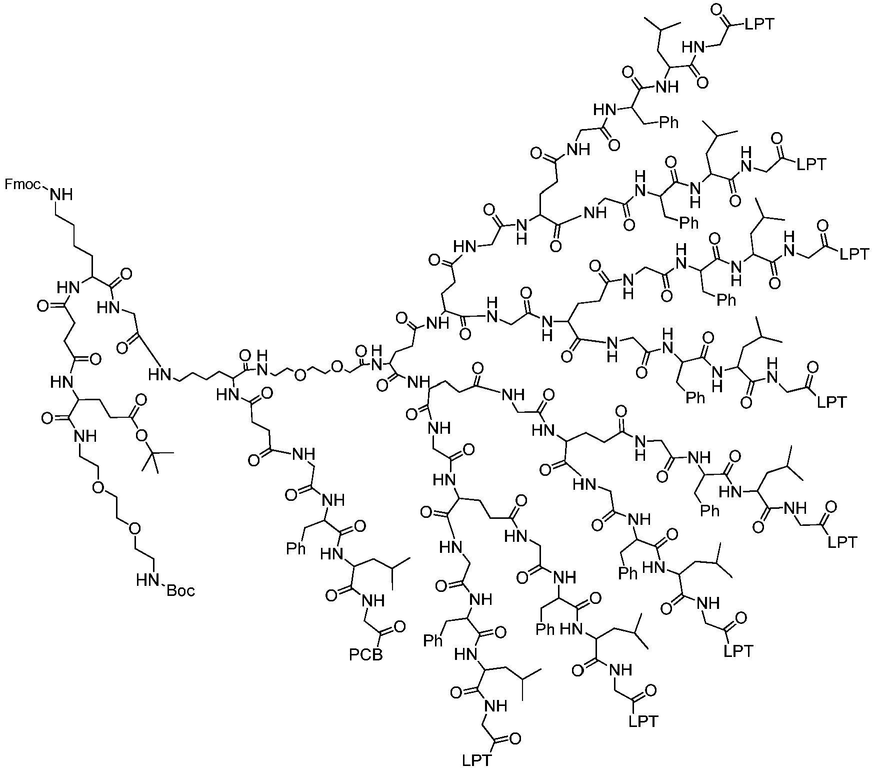

Product 36-236 (0.32 g, 0.3469 mmol), Product 36-174 (3.1 g, 0.3154 mmol), HBTU (0.18 g, 0.4731 mmol) and HOBT (0.06 g, 0.4731 mmol) were added to a 250 mL flask and then dissolved with 30 mL of DMF, and the obtained solution was stirred at -5 °C for 30 min to react; then, DIEA (0.23 mL, 1.4194 mmol) was slowly added dropwise; at the end of the addition, the reaction continued for 1 h, and the reaction solution was stirred at room temperature overnight to react. At the end of the reaction, n-hexane (100 mL × 3) was added for precipitation, and the lower oily solid was dissolved with a small amount of dichloromethane; methyl tert-butyl ether was added, and a solid product was precipitated; the obtained solid product was dried, thus obtaining 3.5 g of Product 36-237.
36-238

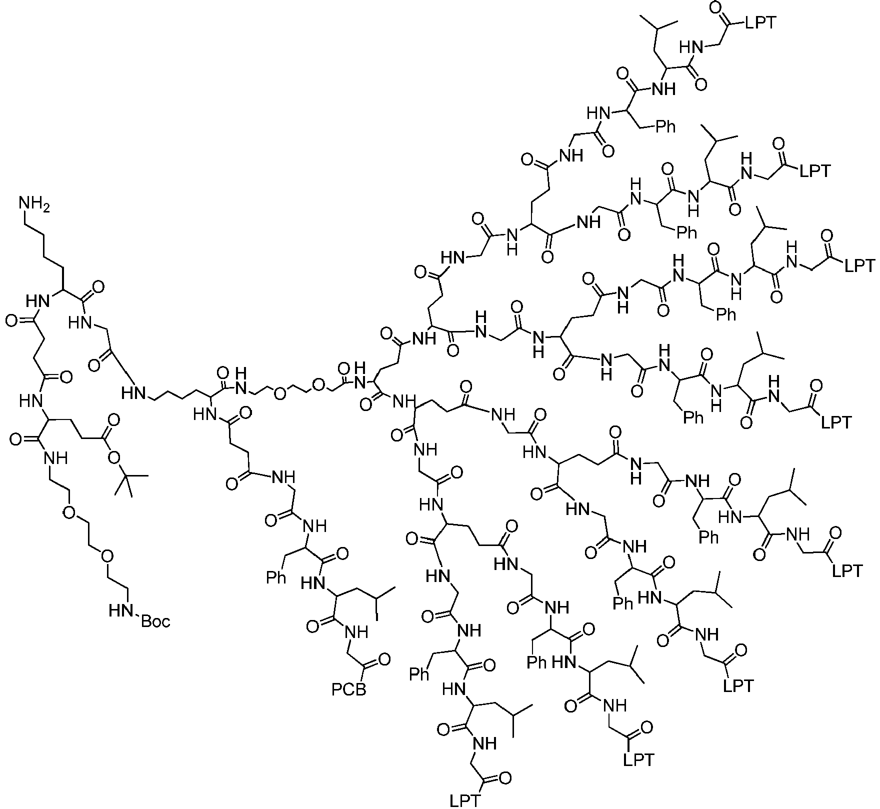

Product 36-237 (3.5 g, 0.3154 mmol) was added in a 250 mL flask and then dissolved with DMF, morpholine (0.27 mL, 3.1543 mmol) was added to the solution, and the reaction solution was stirred overnight at room temperature to react. At the end of the reaction, the reaction solution was concentrated to 10 mL, methyl tert-butyl ether (200 mL) was then added and the powdery product was precipitated; suction filtering was then carried out, and the filter cake was washed with methyl tert-butyl ether (50 mL × 3), and dissolved with a mixed solution (20% methanol: 80% dichloromethane) (200 mL); silica gel powder (40 mL) was added to the obtained solution, and the solution was then evaporated to dryness to obtain a powdery solid; the operations of dry sample loading, column chromatography, and elution with a mixed solution (1% ammonia water/5%-8% methanol/dichloromethane) were carried out; the elution product was then collected, concentrated, and evaporated to dryness, thus obtaining 2.3 g of Product 36-238 with a yield of 39.4%.
36-242

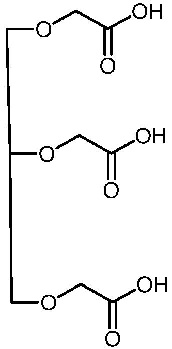

Product 45-91 (0.5 g, 0.9318 mmol, prepared according to the preparation method of Product 37-172, just replace the pentaerythritol to glycerin) and 10%Pd/C (0.0200 g) were added in a reactor and then dissolved with DMF (30 mL); the air in the reactor was then pumped out to reach a vacuum state by a water pump; hydrogen was introduced to a pressure of 0.16 MPa in the reactor, and then discharged, and the reactor was pumped to reach a vacuum state by the water pump; hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth; the filter cake was washed with DMF (20 mL × 3), and the DMF solution was combined, as the raw material for the next step.
36-248

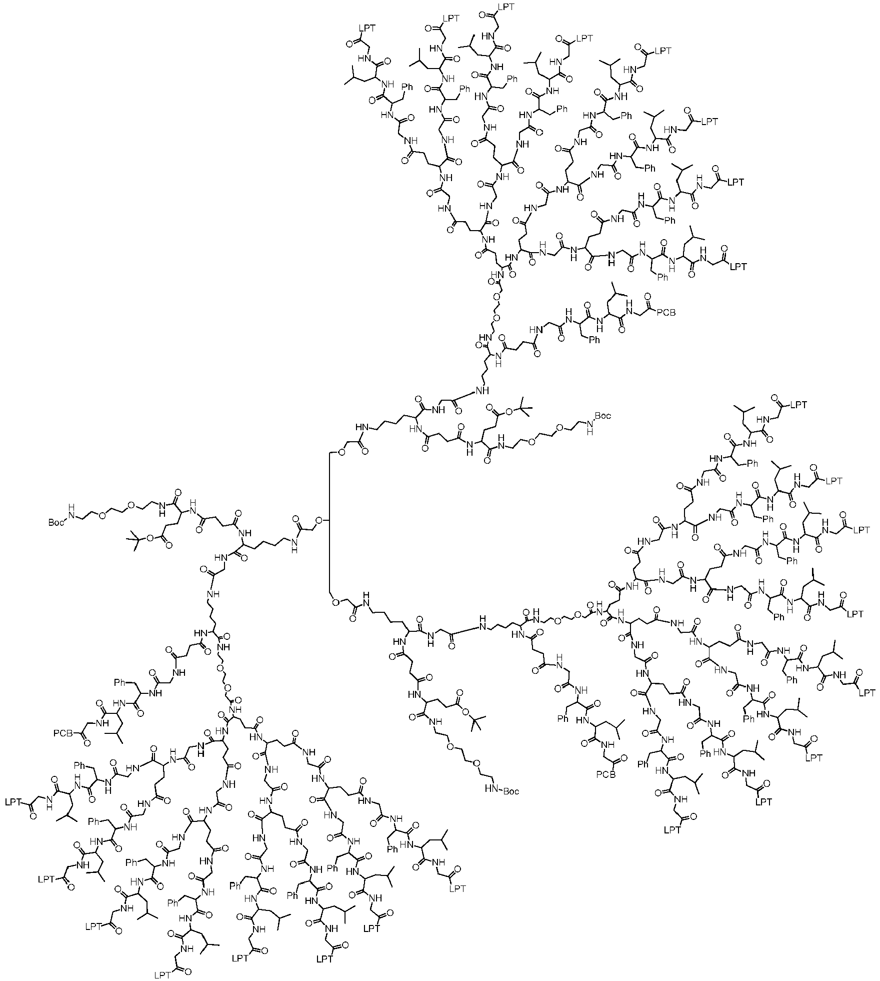

Product 36-238 (1.3 g, 0.1235 mmol), Product 36-242 (0.0082 g, 0.03087 mmol), HBTU (0.05 g, 0.1389 mmol) and HOBT (0.02 g, 0.1389 mmol) were added to a 250 mL flask and dissolved with 30 mL of DMF, and the obtained solution was stirred at -5 °C for 30 min to react; DIEA (0.07 mL, 0.4167 mmol) was slowly added dropwise, and at the end of the addition, the reaction continued for 1 h, and the reaction solution was stirred at room temperature overnight to react. At the end of the reaction, n-hexane (100 mL × 3) was added for precipitation, and the lower oily solid was dissolved with a small amount of dichloromethane; methyl tert-butyl ether was added, and a powdery solid was then precipitated; the suction filtering was carried out and the filter cake was washed with methyl tert-butyl ether (50 mL × 3), and then dissolved with a mixed solution (20% methanol: 80% dichloromethane) (200 mL); silica gel powder (40 mL) was added to the obtained solution, and the solution was then evaporated to dryness to obtain a powdery solid; the operations of dry sample loading, column chromatography, and elution with a mixed solution (1% ammonia water/5%-8% methanol/dichloromethane) were carried out; the elution product was then collected, concentrated, and evaporated to dryness, thus obtaining 0.54 g of Product 36-248 with a yield of 55%.
36-251

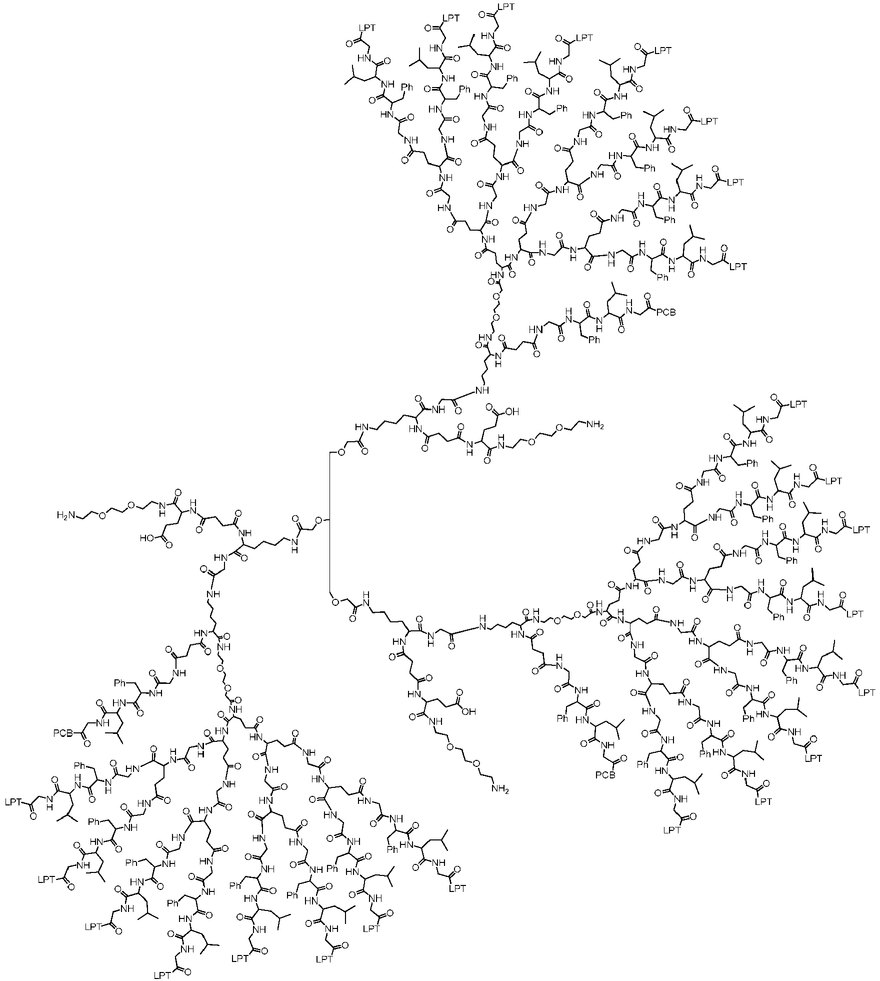

Product 36-248 (0.54 g, 0.01698 mmol) was added in a 500 mL flask, dichloromethane (20 mL) and TFA (0.12 mL, 1.6983 mmol) were added in sequence, and the reaction solution was stirred overnight at room temperature to react. At the end of the reaction, the reaction solution was concentrated to 10 mL; methyl tert-butyl ether (200 mL) was then added and the powdery product was precipitated; suction filtering was then carried out, and the resulting product was dried, thus obtaining 0.58 g of Product 36-251.
36-253

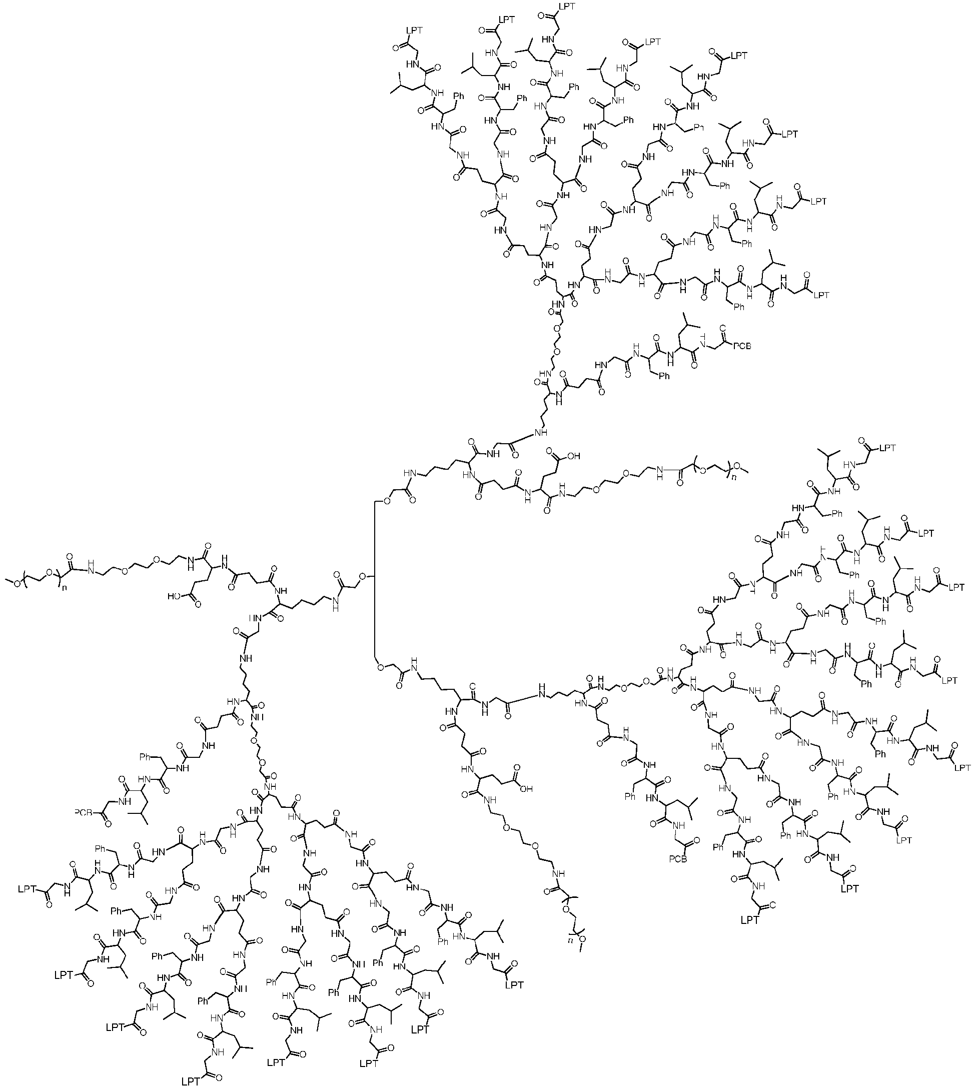

Product 36-251 (0.53 g, 0.01698 mmol) was added in a 500 mL flask and dissolved with 10 mL of DMF, and the obtained solution was stirred at -5 °C for about 30 min to react; DIEA (0.3 mL, 1.698 mmol) was slowly added dropwise; at the end of the addition, M-SCM-10K (0.7 g) was then added, and the obtained solution was slowly stirred at room temperature for a week to react. At the end of the reaction, n-hexane (50 mL × 3) was first added, when the lower oily solution was a small amount, methyl tert-butyl ether (20 mL) was added to precipitate the reaction solution, a solid product was then obtained, and the filter cake was dissolved with methanol/dichloromethane (1: 5); 30 mL of silica gel powder was then added to the obtained solution, and the solution was then evaporated to dryness; the operations of dry sample loading, column chromatography, and elution with an eluent (1% ammonia water: 5%-10% methanol: 94%-89% dichloromethane) were carried out; the elution solution was then collected, then concentrated and evaporated to dryness, thus obtaining 0.58 g of Product 36-253 with a yield of 54.7%.
36-257

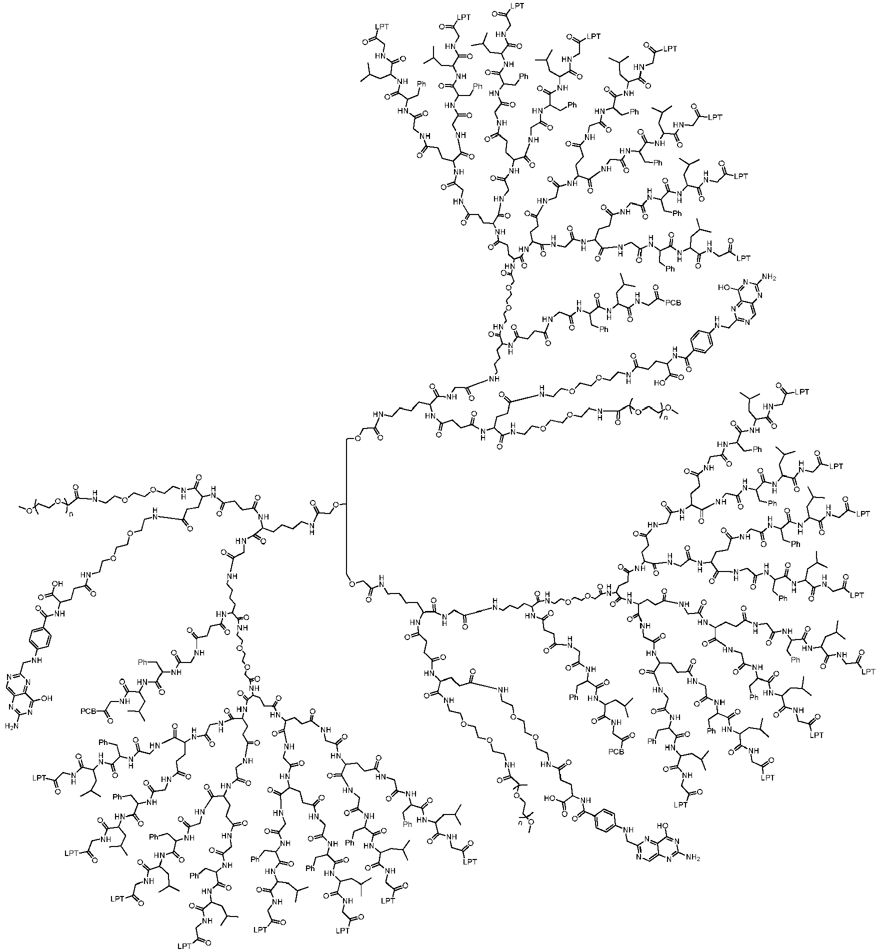

Product 36-253 (0.58 g, 0.00924 mmol), Product 37-163 (0.08 g, 0.1386 mmol), HBTU (0.35 g, 0.4758 mmol) and HOBT (0.11 g, 0.4758 mmol) were added to a 250 mL flask and then dissolved with an appropriate amount of DMF, and the obtained solution was stirred at -5 °C for 30 min to react; DIEA (0.2 mL, 1.3401 mmol) was slowly added dropwise, and at the end of the addition, the reaction continued for 1 h, and the reaction solution was stirred at room temperature overnight to react. At the end of the reaction, n-hexane (100 mL × 3) was added for precipitation, the volume of the lower oily layer was reduced; methyl tert-butyl ether was added, a solid product was precipitated, and the filter cake was dissolved with methanol/dichloromethane (1: 5); 30 mL of silica gel powder was then added to the obtained solution, and the solution was then evaporated to dryness; the operations of dry sample loading, column chromatography, and elution with an eluent (1% ammonia water: 5%-10% methanol: 94%-89% dichloromethane) were carried out; the elution solution was then collected, concentrated and evaporated to dryness. 0.14 g of Product 36-257 was obtained with a yield of 23.8%.

¹H-NMR (600 MHz, DMSO-d₆) δ 9.83 - 9.08 (m, 26H), 8.72 - 8.61 (m, 25H), 8.55 - 8.24 (m, 28H), 8.21 - 7.93 (m, 249H), 7.81 - 7.68 (m, 75H), 7.46 - 7.40 (m, 36H), 7.27 - 7.11 (m, 191H), 6.67 - 6.42 (m, 18H), 5.24 - 5.11 (m, 61H), 4.68 - 4.43 (m, 96H), 4.23 - 4.01 (m, 136H), 3.74 - 3.64 (m, 235H), 3.51 - 3.32 (m, 2465H), 3.25 - 3.22 (m,59H), 3.20 - 2.95 (m,259H), 2.89 - 2.83 (m, 36H), 2.80 - 2.68 (m, 82H), 2.62 - 2.56 (m, 13H), 2.45 - 2.28 (m, 92H), 2.12 - 2.06 (m, 85H), 1.81 - 1.77 (m, 90H), 1.54 -1.48(m, 151H), 1.17 - 1.09 (m, 70H), 0.90 - 0.75 (m, 241H). 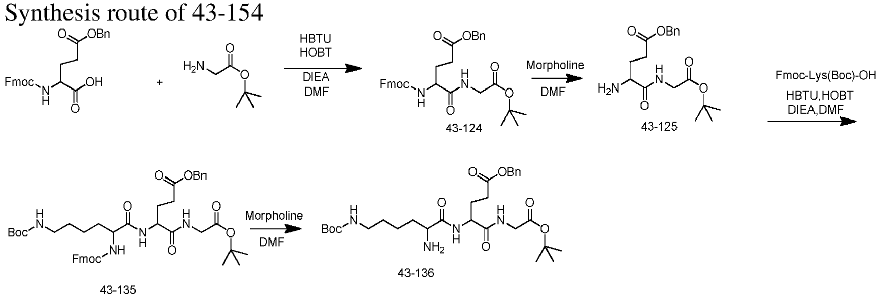 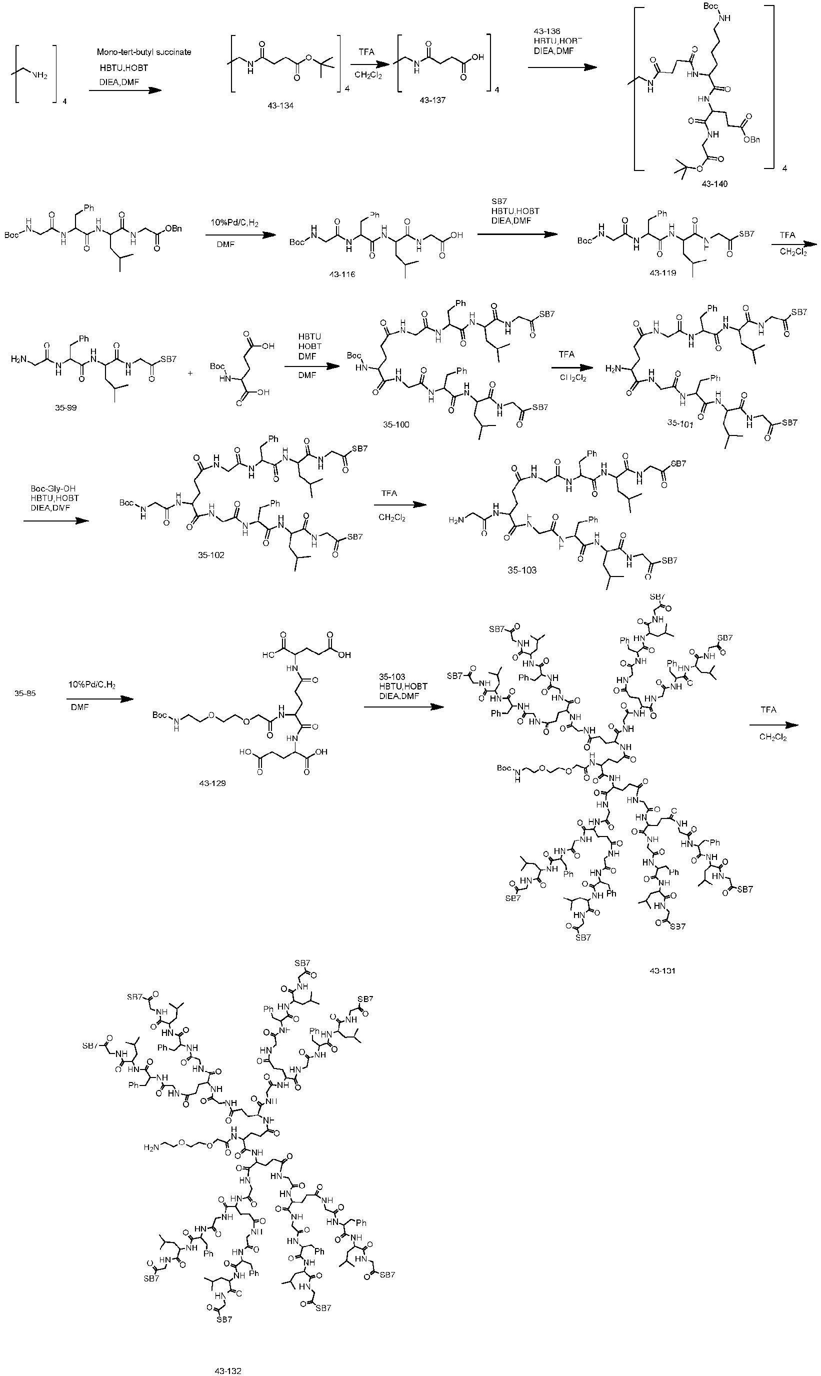 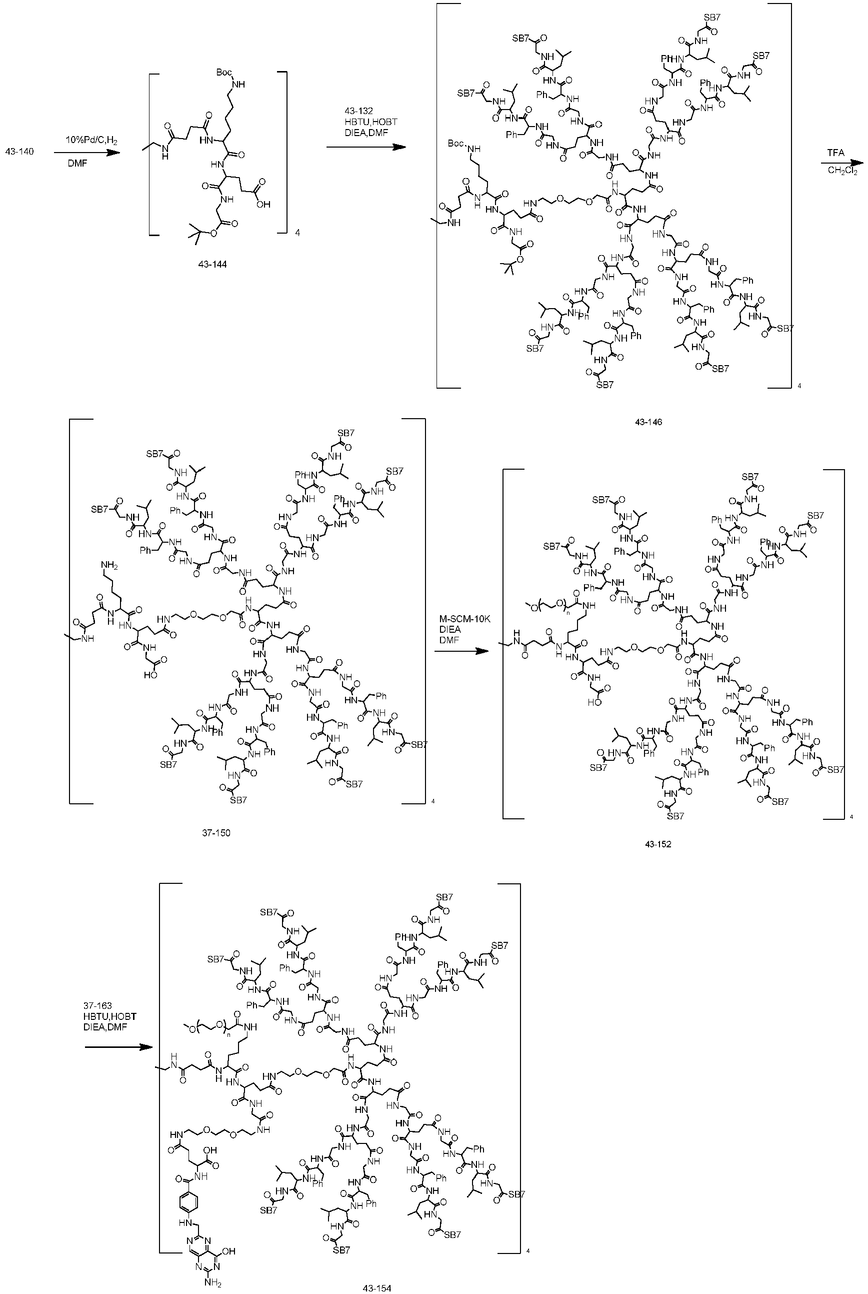 43-124

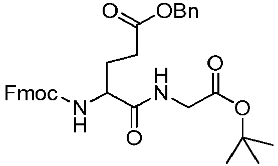

Gly-OtBu.HCl (3.6 g, 21.6 mmol, purchased from Accela), HBTU (12.28 g, 32.4 mmol), HOBT (4.4 g, 32.4 mmol) and Fmoc-Glu (OBn)-OH (10.12 g, 21.6 mmol, purchased from Ark Pharm) were added in a 500 mL round-bottomed flask and then dissolved with DMF (100 mL), and the mixed solution was stirred at 0 °C for 30 min to react; DIEA (19.6 mL, 118.8 mmol) was slowly added dropwise, and at the end of the addition, the obtained solution was further stirred at 0 °C overnight to react. At the end of the reaction, the reaction solution was first transferred to a 1 L separatory funnel; a saturated saline solution (300 mL) and ethyl acetate (200 mL) were added, and the obtained solution was shaken and extracted to obtain an organic phase; the aqueous phase was washed with ethyl acetate (200 mL × 1), and the obtained organic phases were combined, and evaporated to dryness, thus obtaining 12.37 g of Product 43-124.

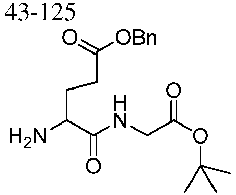

Product 43-124 (12.37 g, 21.6 mmol) was added in a 250 mL flask and then dissolved with DMF (30 mL), morpholine (18.82 mL, 216 mmol) was then added, and the mixed solution was stirred for 1 h at room temperature to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2); the obtained organic phases were combined, then concentrated and evaporated to dryness, thus obtaining 5.6 g of Product 43-125 with a yield of 74%.
43-135

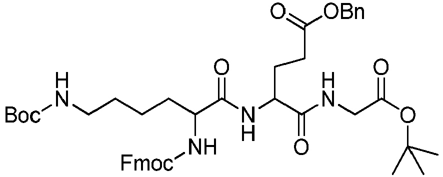

Product 43-125 (2.8 g, 8.0 mmol), HBTU (4.5 g, 12 mmol), HOBT (1.6 g, 12 mmol) and Fmoc-Lys (Boc)-OH (3.7 g, 8.0 mmol, purchased from Accela) were added in a 500 mL round-bottomed flask and then dissolved with DMF (40 mL), and the reaction solution was stirred at 0 °C for about 30 min; then, DIEA (4.6 mL, 96.4 mmol) was slowly added dropwise to further stirred at 0 °C overnight. At the end of the reaction, the reaction solution was first transferred to a 1 L separatory funnel, a saturated saline solution (300 mL) and ethyl acetate (200 mL) were added, and the obtained solution was shaken and extracted to obtain an organic phase; the aqueous phase was washed with ethyl acetate (200 mL × 1); the obtained organic phases were combined, and evaporated to dryness, thus obtaining 6.4 g of Product 43-135.
43-136

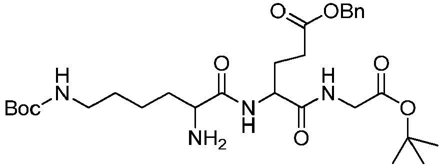

Product 43-135 (6.4 g, 8.0 mmol) was added in a 250 mL flask and then dissolved with DMF (30 mL), morpholine (6.9 mL, 80 mmol) was added to the solution, and the mixed solution was stirred for 1 h at room temperature to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, then concentrated and evaporated to dryness, and the solid product was dissolved with methanol (30 mL) and dichloromethane (120 mL); silica gel powder (20 g) was then added to the obtained solution and the solution was evaporated to dryness to obtain a powdery solid; the operations of dry sample loading, column chromatography and elution with a mixed solution (1% ammonia water and 2%-3% methanol/dichloromethane) were carried out. 1.6 g of Product 43-136 was obtained with a yield of 35%.
43-134

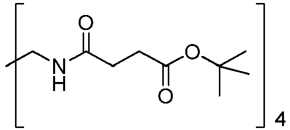

Pentatetramine disulfate (1.0 g, 3.05 mmol, purchased from Pharmaron), HBTU (6.94 g, 18.3 mmol), HOBT (2.47 g, 18.3 mmol) and mono-tert-butyl succinate (3.72 g, 21.34 mmol) were added in a 250 mL round-bottomed flask and then dissolved with DMF (30 mL), the obtained solution was stirred at -5 °C for 30 min to react; DIEA (9.07 mL, 54.9 mmol) was slowly added dropwise, and at the end of the addition, the obtained solution was first stirred at 15 °C for 1 h, and then stirred at room temperature overnight to react. At the end of the reaction, the reaction solution was first transferred to a 1 L separatory funnel, a saturated sodium bicarbonate solution (300 mL) and ethyl acetate (200 mL) were added, and the obtained solution was shaken and extracted to obtain an organic phase; the aqueous phase was washed with ethyl acetate (200 mL × 1), the obtained organic phases were combined and evaporated to dryness, and the solid product was dissolved with methanol (40 mL) and dichloromethane (160 mL); silica gel powder (30 g) was then added to the obtained solution and the solution was evaporated to dryness to obtain a powdery solid; the operations of dry sample loading, column chromatography and gradient elution with a mixed solution (3%-6% methanol/dichloromethane) were carried out. 2.3 g of Product 43-134 was obtained.
43-137

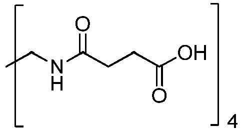

Product 43-134 (2.31 g, 3.05 mmol) was added to a 500 mL flask and then dissolved with dichloromethane (30 mL), trifluoroacetic acid (11.3 mL, 152.5 mmol) was added, and the reaction solution was stirred overnight at room temperature to react. At the end of the reaction, methyl tert-butyl ether (50 mL) and n-hexane (200 mL) were added, and the obtained solution was shaken and then stood still to be layered; the supernatant was discarded; methyl tert-butyl ether (50 mL) and n-hexane (200 mL) were added again to the lower oily product; such operations were repeated four times to obtain a half solid; the solid was dried, thus obtaining 1.6 g of Product 43-137.
43-140

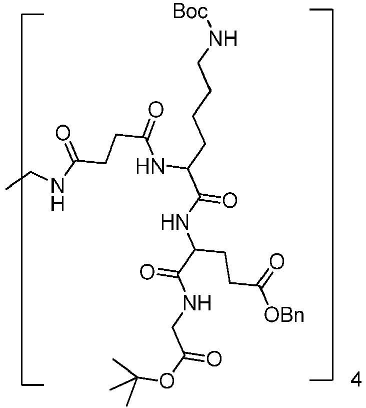

Product 43-136 (1.6 g, 2.7 mmol), HBTU (1.44 g, 3.6 mmol), HOBT (0.48 g, 3.6 mmol) and Product 43-137 (0.32 g, 0.6 mmol) were added in a 250 mL round-bottomed flask and then dissolved with DMF (100 mL), and the obtained solution was stirred at -5 °C for about 30 min to react. Then DIEA (1.84 mL, 10.8 mmol) was slowly added dropwise. At the end of the addition, the reaction solution was first stirred at 15 °C for 1 h, and then stirred at room temperature overnight to react. At the end of the reaction, the reaction solution was first transferred to a 1 L separatory funnel, a saturated sodium bicarbonate solution (300 mL) and ethyl acetate (200 mL) were added, and the obtained solution was shaken and extracted to obtain an organic phase; the aqueous phase was washed with ethyl acetate (200 mL × 1), the obtained organic phases were combined and evaporated to dryness, and the solid product was dissolved with methanol (40 mL) and dichloromethane (160 mL); silica gel powder (30 g) was then added to the obtained solution and the solution was evaporated to dryness to obtain a powdery solid; the operations of dry sample loading, column chromatography and gradient elution with a mixed solution (3%-6% methanol/dichloromethane) were carried out. 1.28 g of Product 43-140 was obtained with a yield of 60%.
43-116

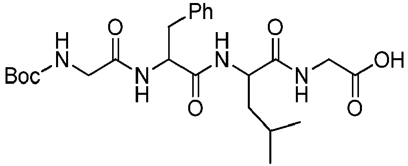

Boc-GFLG-OBn (9.9 g, 17 mmol, synthesized according to the synthesis method of Product 37-62) was added into a hydrogenation reactor and then dissolved with 10% Pd/C (0.025 g) and DMF (40 mL); hydrogen was introduced to a pressure of 2.1 MPa in the reactor, and the reaction solution was stirred overnight at room temperature to react. At the end of the reaction, the reaction solution was filtered with diatomaceous earth; the filter cake was washed three times with DMF (20 mL × 3), and the elution solution was added in a 500 mL round-bottomed flask, as the raw material for the next step.
43-119

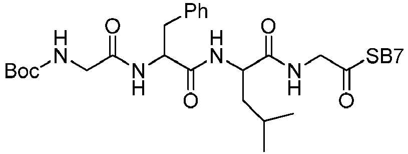

SB-743921 (SB7, 8.000 g, 15.4718 mmol, purchased from Nanjing PharmaBlock), HBTU (8.814 g, 23.2078 mmol) and HOBT (3.136 g, 23.2078 mmol) were added in the DMF (110 mL) of Product 43-116 (7.3 g, 17 mmol), and the obtained solution was stirred for about 20 min at -5 °C; then, DIEA (11.5 mL, 69.75 mmol) was slowly added dropwise over 10 min; the reaction continued at -5 °C for 1 h, and the reaction solution was stirred at room temperature overnight to react. At the end of the reaction, the reaction solution was transferred to a 1 L separatory funnel and extracted with a saturated sodium bicarbonate solution (250 mL) and ethyl acetate (300 mL) to obtain an organic phase; the aqueous phase was washed with ethyl acetate (200 mL × 2); the obtained organic phases were combined and washed with a saturated saline solution (200 mL × 2), evaporated to dryness to obtain a solid, and dried in a vacuum oven, thus obtaining 15.37 g of Product 43-119.
35-99

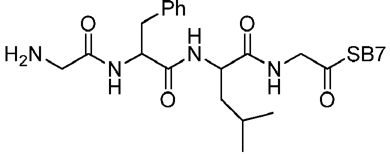

Product 43-119 (15.37 g, 15.5 mmol) was added in a 500 mL flask and then dissolved with dichloromethane (30 mL), TFA (11.51 mL, 155.0 mmol) was added, and the reaction solution was stirred overnight at room temperature to react. At the end of the reaction, the reaction solution was concentrated; methyl tert-butyl ether (300 mL) was then added and the powdery product was precipitated; suction filtering was then carried out, and the filter cake was washed with methyl tert-butyl ether (150 mL × 2) and dissolved with a solution of methanol (60 mL) and dichloromethane (240 mL); silica gel powder (50 g) was added to the obtained solution, and the solution was then evaporated to dryness to obtain a powdery solid; the operations of dry sample loading, column chromatography, and elution with a mixed solution (1% ammonia water/3% methanol/dichloromethane) were carried out; the elution product was then collected, concentrated, and evaporated into a solid, and the solid was dried in a vacuum oven. 10.7 g of Product 35-99 was obtained with a yield of 78%.
35-100

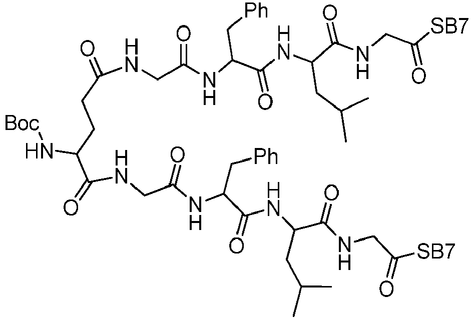

Product 35-99 (10.7 g, 12.0 mmol), Boc-Glu-OH (1.413 g, 5.72 mmol), HBTU (6.5 g, 17.15 mmol) and HOBT (2.32 g, 17.15 mmol) were added to a 500 mL flask and then dissolved with DMF (40 mL), and the obtained solution was stirred for about 10 min at -5 °C; then, DIEA (8.5 mL, 51.44 mmol) was slowly added dropwise; at the end of the addition, the reaction continued at -5 °C for 1 h, and the reaction solution was stirred at room temperature overnight to react. At the end of the reaction, the reaction solution was transferred to a 1 L separatory funnel and extracted with a saturated saline solution (200 mL) and ethyl acetate (300 mL) to obtain an organic phase; the organic phase was washed with a saturated saline solution (200 mL × 2), and evaporated to dryness to obtain a solid, thus obtaining 11.41 g of Product 35-100.
35-101

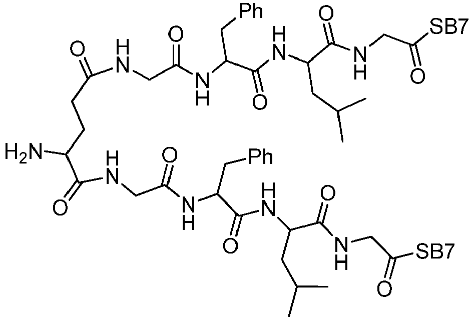

Product 35-100 (11.41 g, 5.72 mmol) was added to a 250 mL flask and then dissolved with dichloromethane (15 mL), TFA (4.2 mL, 57.2 mmol) was added then, and the reaction solution was stirred overnight at room temperature to react. At the end of the reaction, the reaction solution was concentrated; methyl tert-butyl ether (150 mL) was then added to obtain a powdery solid; suction filtering was then carried out, and the filter cake was washed with methyl tert-butyl ether (30 mL × 3), and dried in a vacuum oven, thus obtaining 10.8 g of Product 35-101.
35-102

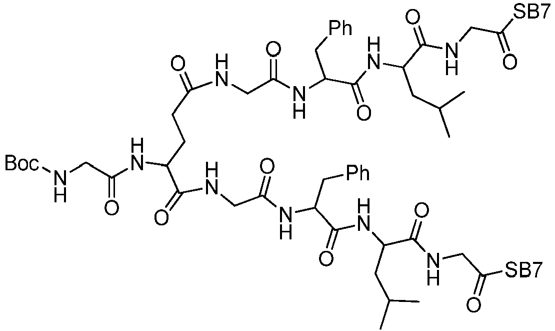

Product 35-101 (10.8 g, 5.72 mmol), Boc-Gly-OH (1.202 g, 6.864 mmol), HBTU (3.25 g, 8.58 mmol) and HOBT (1.16 g, 8.58 mmol) were added to a 500 mL flask and then dissolved with DMF (50 mL), and the obtained solution was stirred for about 10 min at -5 °C; then, DIEA (4.25 mL, 25.74 mmol) was slowly added dropwise; at the end of the addition, the reaction continued at - 5 °C for 1 h, and the reaction solution was stirred at room temperature overnight to react. At the end of the reaction, the reaction solution was transferred to a 1 L separatory funnel and extracted with a saturated saline solution (200 mL) and ethyl acetate (250 mL) to obtain an organic phase; the organic phase was washed with a saturated saline solution (200 mL × 2), and evaporated to dryness to obtain a solid, thus obtaining 11.7 g of Product 35-102.
35-103

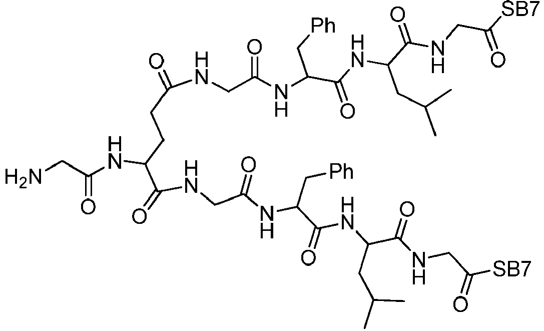

Product 35-102 (11.7 g, 5.72 mmol) was added to a 500 mL flask, dichloromethane (20 mL) and trifluoroacetic acid (4.2 mL, 57.2 mmol) were added, and the reaction solution was stirred overnight at room temperature to react. At the end of the reaction, the reaction solution was concentrated to a small amount, methyl tert-butyl ether (200 mL) was added, and a powdery solid was then precipitated; suction filtering was carried out and the filter cake was washed with methyl tert-butyl ether (50 mL × 3), and dissolved with methanol (30 mL) and dichloromethane (120 mL); silica gel powder (20 g) was added to the obtained solution, and the solution was then evaporated to dryness to obtain a powdery solid; the operations of dry sample loading, column chromatography, and elution with (1% ammonia water/4% methanol/dichloromethane) were carried out; the elution product was then collected, concentrated, evaporated to dryness, and dried. 9.0 g of Product 35-103 was obtained with a yield of 81%.
43-129

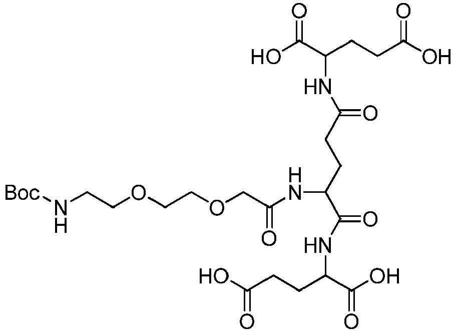

Product 35-85 (0.7 g, 0.69 mmol) and 10%Pd/C (0.030 g) were added in a hydrogenation reactor and then dissolved with DMF (30 mL); hydrogen was introduced to a pressure of 1.8 MPa in the reactor, and the mixed solution then was stirred overnight at room temperature to react. At the end of the reaction, the reaction solution was filtered with diatomaceous earth; the diatomaceous earth was washed with DMF (20 mL × 3), and the DMF solution was combined, as the raw material for the next step.
43-131

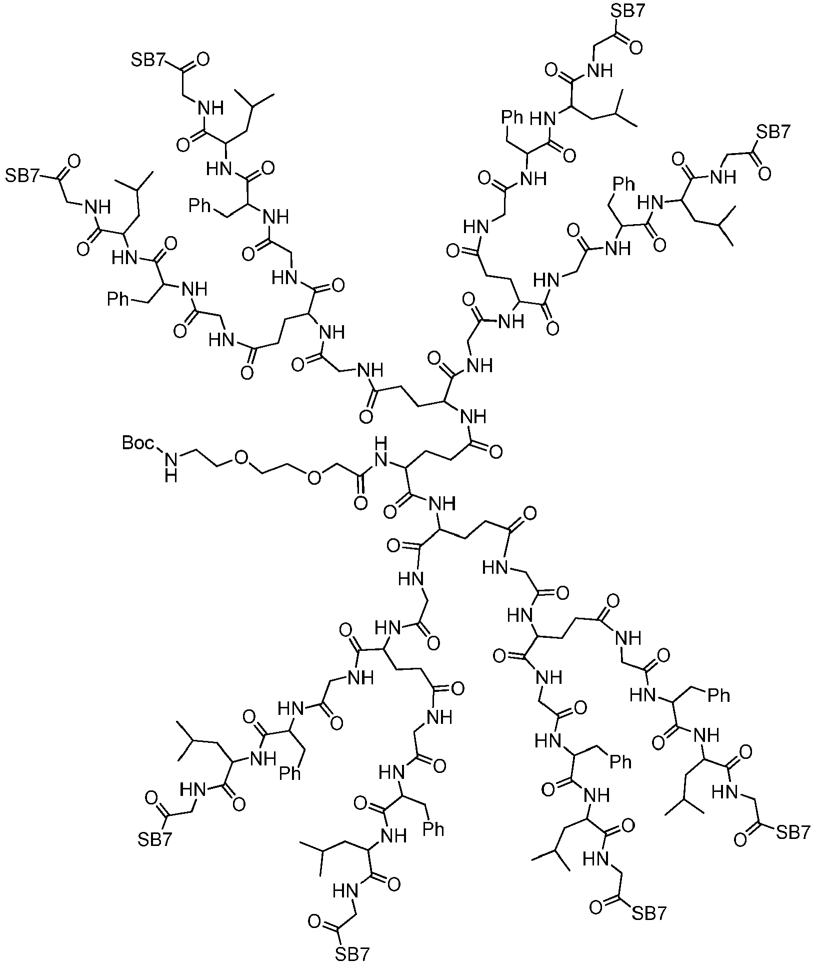

Product 43-129 (0.449 g, 0.69 mmol), HBTU (1.5 g, 4.14 mmol), HOBT (0.559 g, 4.14 mmol) and Product 35-103 (3 g, 1.5377 mmol) were added to a 250 mL flask and dissolved with DMF (30 mL), and the obtained solution was stirred at -5 °C for about 30 min to react. Then DIEA (2.0 mL, 6.2905 mmol) was slowly added dropwise, and at the end of the addition, the obtained solution was first stirred at 15 °C for 1 h, and then stirred at room temperature overnight to react. At the end of the reaction, the reaction solution was transferred to a 1 L separatory funnel and extracted with a saturated sodium bicarbonate solution (200 mL) and ethyl acetate (200 mL) to obtain an organic phase; the aqueous phase was washed with ethyl acetate (200 mL × 2), the obtained organic phases were combined, concentrated and evaporated to dryness, and the solid product was dissolved with methanol (20 mL) and dichloromethane (120 mL); silica gel powder (15 g) was then added to the obtained solution and the solution was evaporated to dryness to obtain a powdery solid; the operations of dry sample loading, column chromatography and gradient elution with a mixed solution (1% ammonia water and 3%-6% methanol/dichloromethane) were carried out. 4.8 g of Product 43-131 was obtained with a yield of 83%.
43-132

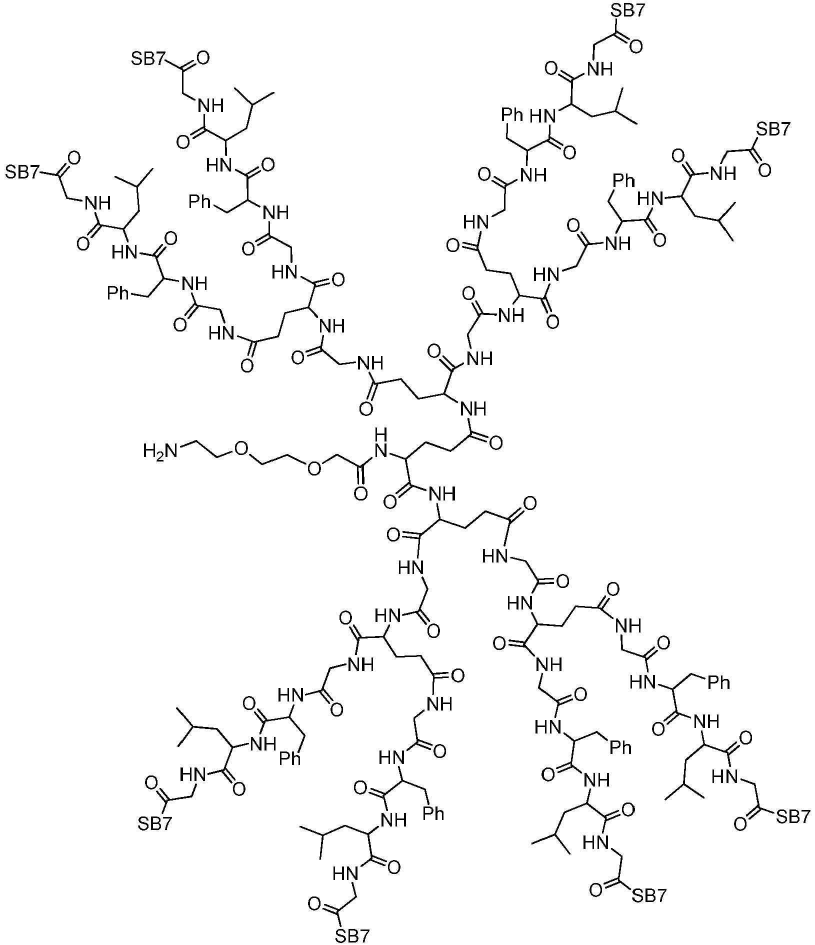

Product 43-131 (4.8 g, 0.57 mmol) was added to a 250 mL flask, dichloromethane (10 mL) and trifluoroacetic acid (6.35 mL, 85.5 mmol) were added in sequence, and the reaction solution was stirred overnight at room temperature to react. At the end of the reaction, the reaction solution was concentrated to a small amount, methyl tert-butyl ether (150 mL) was added, and a powdery solid was then precipitated; the suction filtering was carried out and the filter cake was washed with methyl tert-butyl ether (50 mL × 3), and then dissolved with a solution of methanol (30 mL) and dichloromethane (120 mL) ; silica gel powder (10 g) was added to the obtained solution, and the solution was then evaporated to dryness to obtain a powdery solid; the operations of dry sample loading, column chromatography, and elution with a mixed solution (1% ammonia water/7%-8% methanol/dichloromethane) were carried out; the elution product was then collected, concentrated, and evaporated into a solid. The solid was dried in a vacuum oven. 2.4 g of Product 43-132 was obtained with a yield of 51%.
43-144

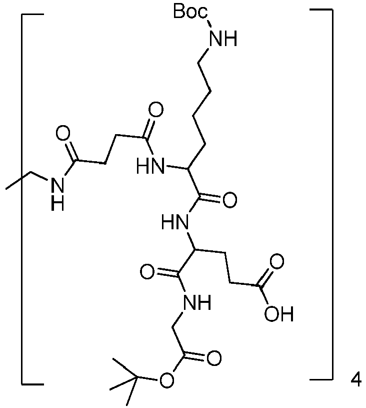

Product 43-140 (0.076 g, 0.0465 mmol) and 10% Pd/C (0.0320 g) were added in a hydrogenation reactor and then dissolved with DMF (30 mL); hydrogen was introduced to a pressure of 1.6 MPa in the reactor, and the reaction solution was stirred overnight at room temperature to react. At the end of the reaction, the reactor was taken out, and the reaction solution was filtered with diatomaceous earth, and washed with DMF (20 mL × 3), thus obtaining the DMF solution of Product 43-144.
43-146

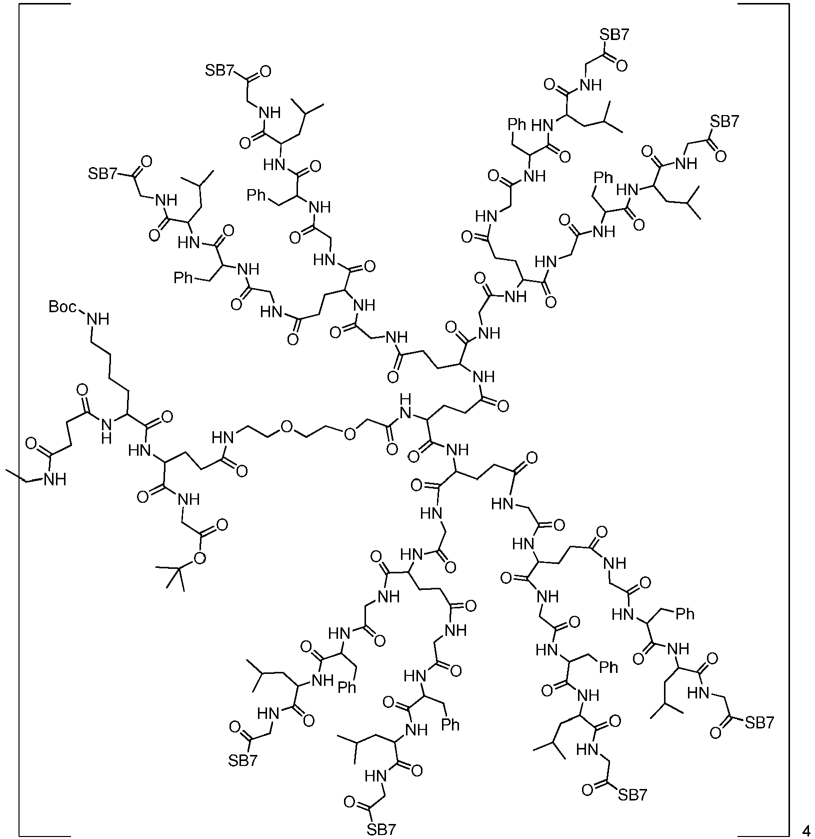

Product 43-132 (1.0 g, 0.121 mmol), HBTU (0.067 g, 0.178 mmol), HOBT (0.024 g, 0.178 mmol) and Product 43-144 (0.066 g, 0.027 mmol) were added to a 250 mL flask and dissolved with DMF (60 mL), and the obtained solution was stirred for about 30 min at -5 °C. Then DIEA (0.143 mL, 0.8694 mmol) was slowly added dropwise, and at the end of the addition, the obtained solution was first stirred at -5 °C for 1 h, and then stirred overnight at room temperature. At the end of the reaction, the reaction solution was transferred to a 1 L separatory funnel and extracted with a saturated sodium bicarbonate solution (150 mL) and ethyl acetate (200 mL) to obtain an organic phase; the aqueous phase was washed with ethyl acetate (200 mL × 2), and the obtained organic phases were combined, and the solid product was dissolved with methanol (20 mL) and dichloromethane (120 mL); silica gel powder (15 g) was then added to the obtained solution and the solution was evaporated to dryness to obtain a powdery solid; the operations of dry sample loading, column chromatography and gradient elution with a mixed solution (1% ammonia water and 4%-8% methanol/dichloromethane) were carried out. 1.07 g of Product 43-146 was obtained with a yield of 63%.
43-150

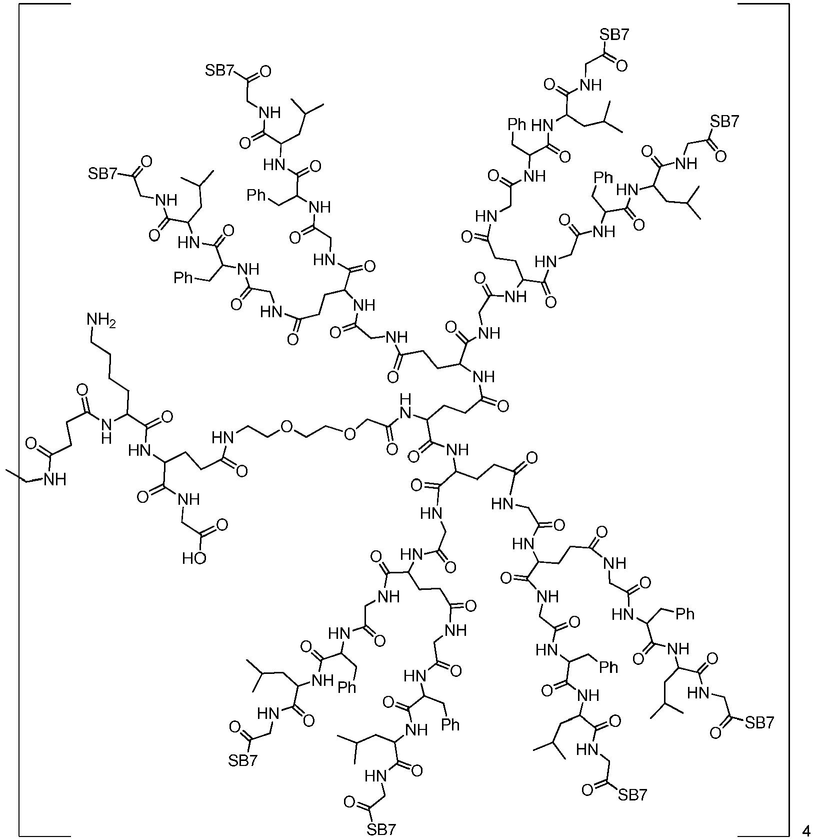

Product 43-146 (0.957 g, 0.027 mmol) was added to a 250 mL flask, dichloromethane (20 mL) and trifluoroacetic acid (10 mL, 134.98 mmol) were added in sequence, and the reaction solution was stirred overnight at room temperature to react. At the end of the reaction, the reaction solution was concentrated to a small amount, methyl tert-butyl ether (150 mL) was added, and a powdery solid was then precipitated; the suction filtering was carried out and the filter cake was washed with methyl tert-butyl ether (50 mL × 3), and then dissolved with a solution of methanol (30 mL) and dichloromethane (120 mL); silica gel powder (10 g) was added to the obtained solution, and the solution was then evaporated to dryness to obtain a powdery solid; the operations of dry sample loading, column chromatography, and elution with a mixed solution (1% ammonia water/7%-10% methanol/dichloromethane) were carried out; the elution product was then collected, concentrated, and evaporated into a solid. The solid was dried in a vacuum oven. 0.24 g of Product 43-150 was obtained with a yield of 26%.
43-152

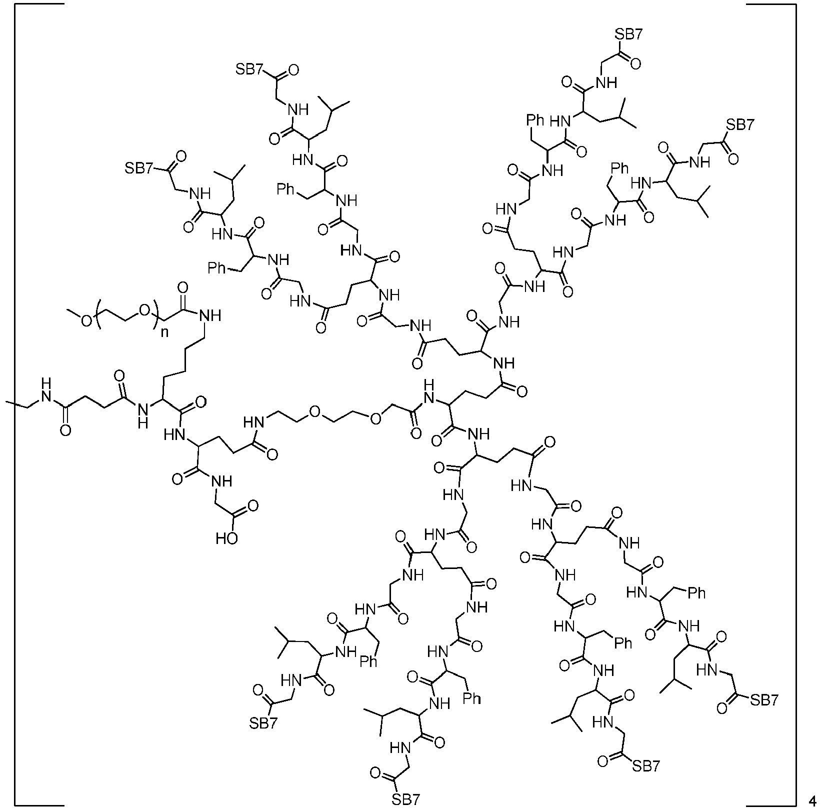

Product 43-150 (0.24 g, 0.0069 mmol) was added in a 250 mL flask and then dissolved with DMF (30 mL); DIEA (0.11 mL, 0.688 mmol) was slowly added dropwise, and at the end of the addition, the obtained solution was further stirred for 20 min, dissolved by ultrasonic with M-SCM-10K (0.4374 g, 0.0413 mmol), and slowly stirred at room temperature to react for 7 days in the dark. At the end of the reaction, n-hexane (120 mL) and methyl tert-butyl ether (40 mL) were added, the supernatant was discarded, n-hexane (120 mL) and methyl tert-butyl ether (40 mL) were added to the lower liquid, and such operations were repeated three times to obtain a viscous oily product; methyl tert-butyl ether (100 mL) was added, and a solid product was precipitated; suction filtering was then carried out, and the filter cake was washed with methyl tert-butyl ether (50 mL × 3), and dissolved with a solution of methanol (30 mL) and dichloromethane (120 mL); silica gel powder (10 g) was added to the obtained solution, and the solution was then evaporated to dryness to obtain a powdery solid; the operations of dry sample loading, column chromatography, and elution with a mixed solution (1% ammonia water/6%-7% methanol/dichloromethane) were carried out; the elution product was then collected, concentrated, and evaporated into a solid. The solid was dried in a vacuum oven, thus obtaining the product.
43-154

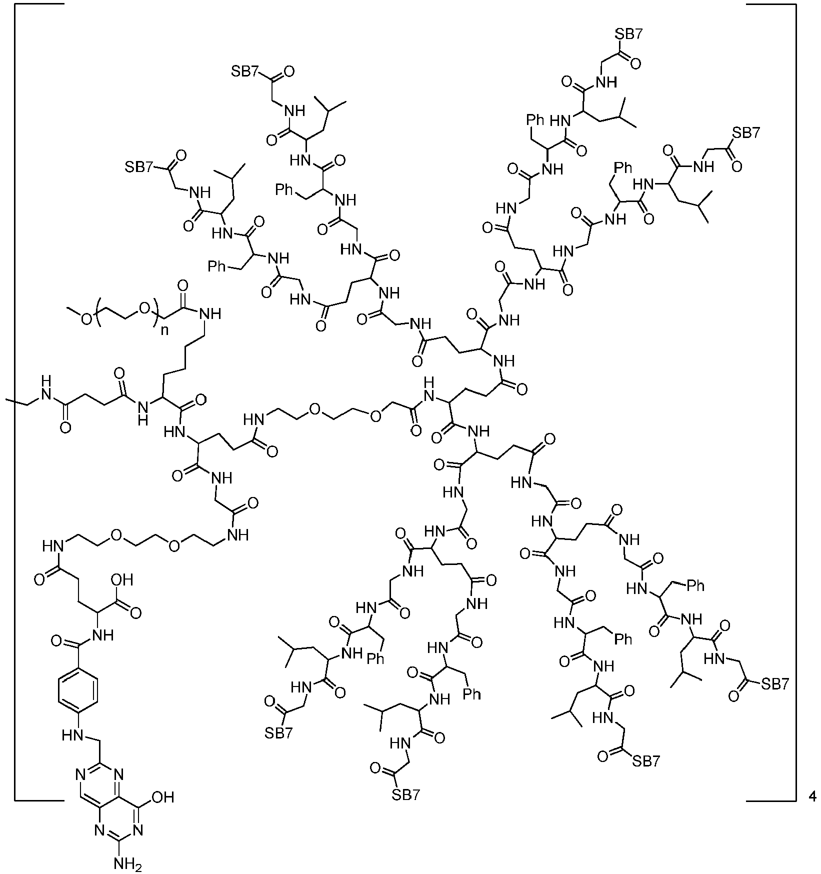

Product 43-152 (0.84 g, 0.0116 mmol), HBTU (0.264 g, 0.696 mmol), HOBT (0.094 g, 0.696 mmol) and Product 37-163 (0.1167 g, 0.232 mmol) were added to a 250 mL flask and dissolved with DMF (60 mL), and the obtained solution was stirred for about 30 min at -5 °C; then, DIEA (0.345 mL, 2.085 mmol) was slowly added dropwise; at the end of the addition, the obtained solution was first stirred at -5 °C for 1 h, and then stirred overnight at room temperature to further react. At the end of the reaction, n-hexane (100 mL) and methyl tert-butyl ether (60 mL) were added, and the supernatant was discarded; n-hexane (100 mL) and methyl tert-butyl ether (60 mL) were added to the lower liquid, and such operations were repeated three times to obtain a viscous oily product; methyl tert-butyl ether (100 mL) was added to obtain a solid; suction filtering was then carried out, and the filter cake was washed with methyl tert-butyl ether (50 mL × 3), and then dissolved with methanol (20 mL) and dichloromethane (120 mL); silica gel powder (15 g) was then added to the obtained solution and the solution was evaporated to dryness to obtain a powdery solid; the operations of dry sample loading, column chromatography and gradient elution with a mixed solution (1% ammonia water and 4%-8% methanol/dichloromethane) were carried out. 0.67 g of Product 43-154 was obtained with a yield of 72%.

¹H-NMR (600 MHz, DMSO-d₆) δ 8.18 -8.00 (m, 182H), 7.80-7.75 (m, 33H), 7.5-7.47 (m, 46H), 7.32 - 7.08(m, 726H), 4.24-4.15 (m, 165H), 3.51-3.49 (m, 6260H), 3.12 - 2.70 (m, 331H), 2.39-2.11(m, 296H), 1.59-1.46 (m, 182H), 0.90-0.78 (m, 384H).

### Synthesis route of 37-221

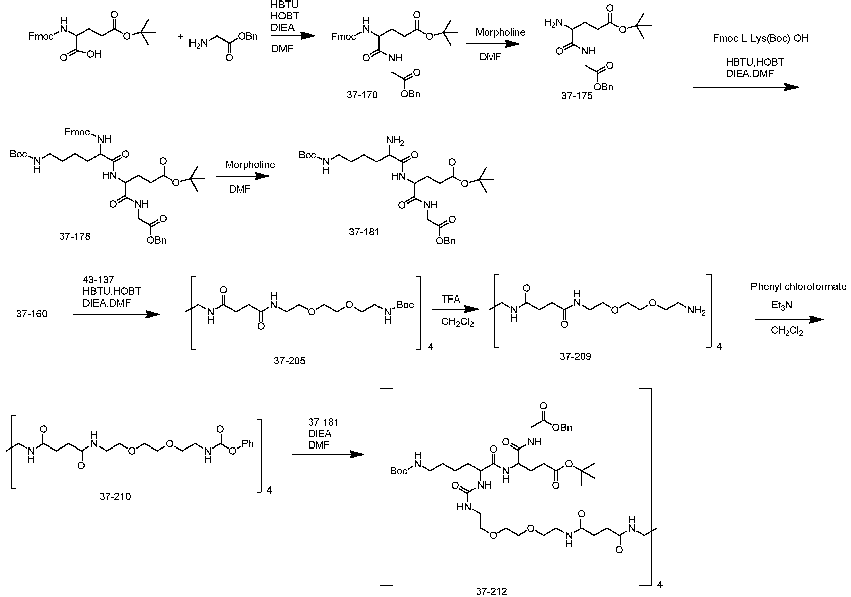

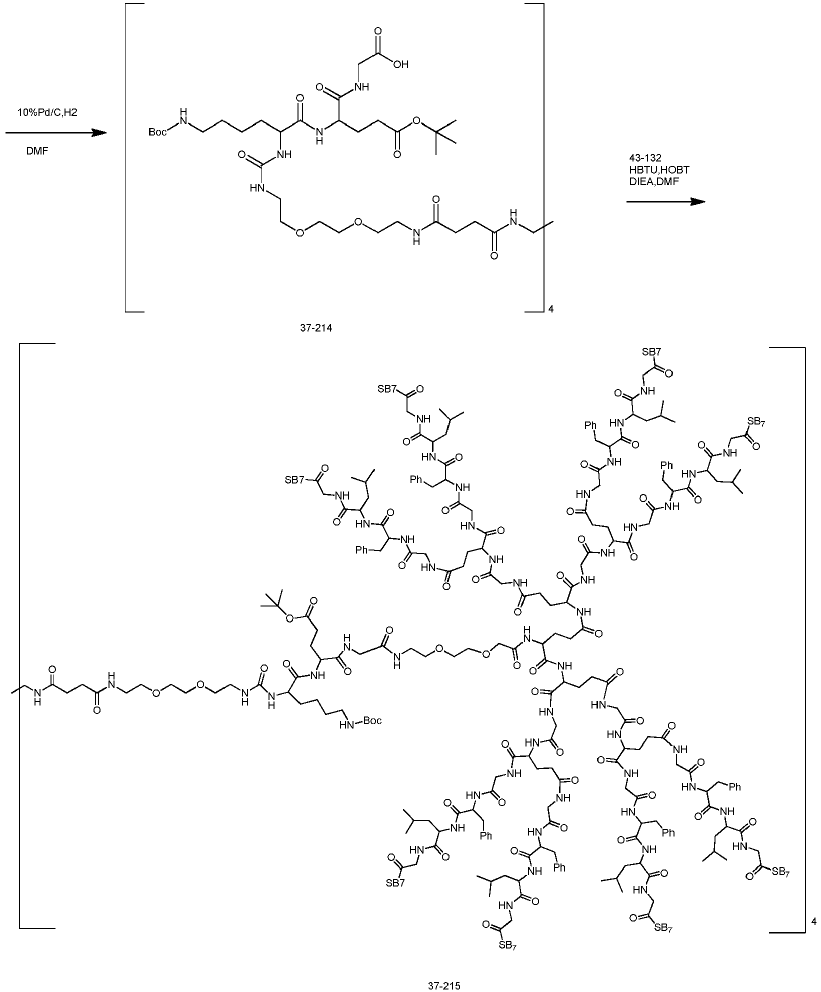

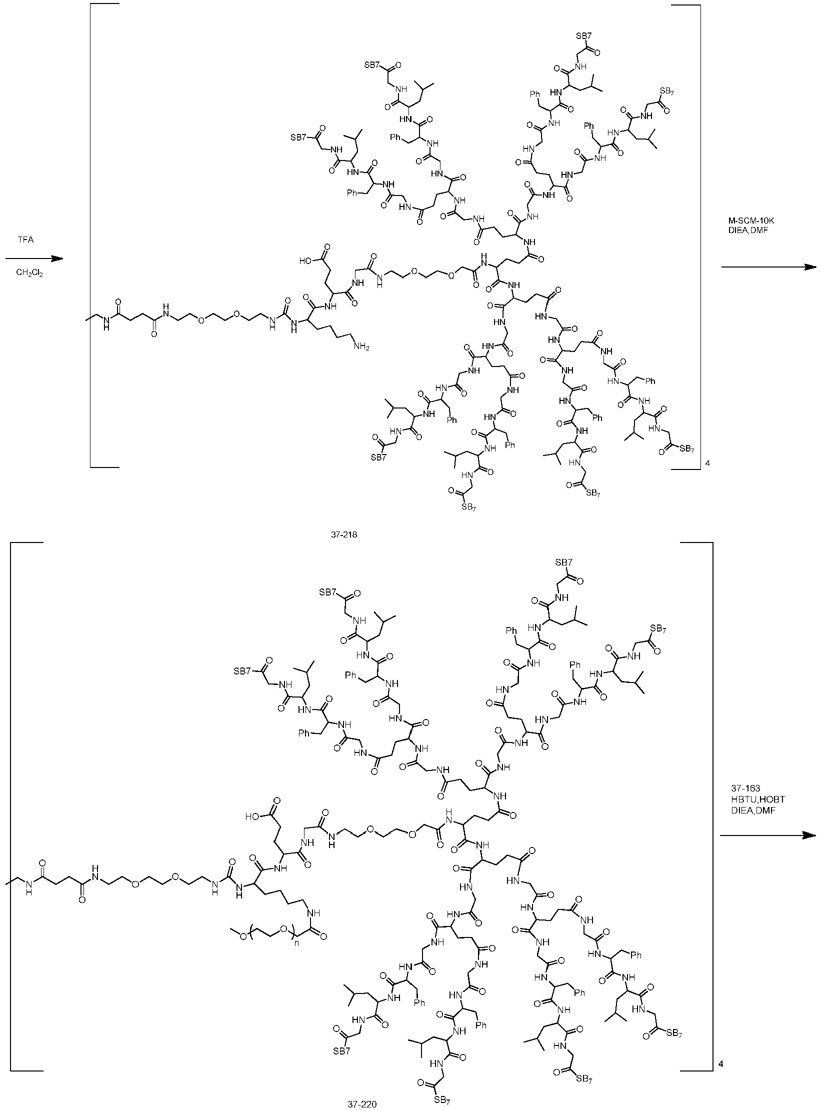

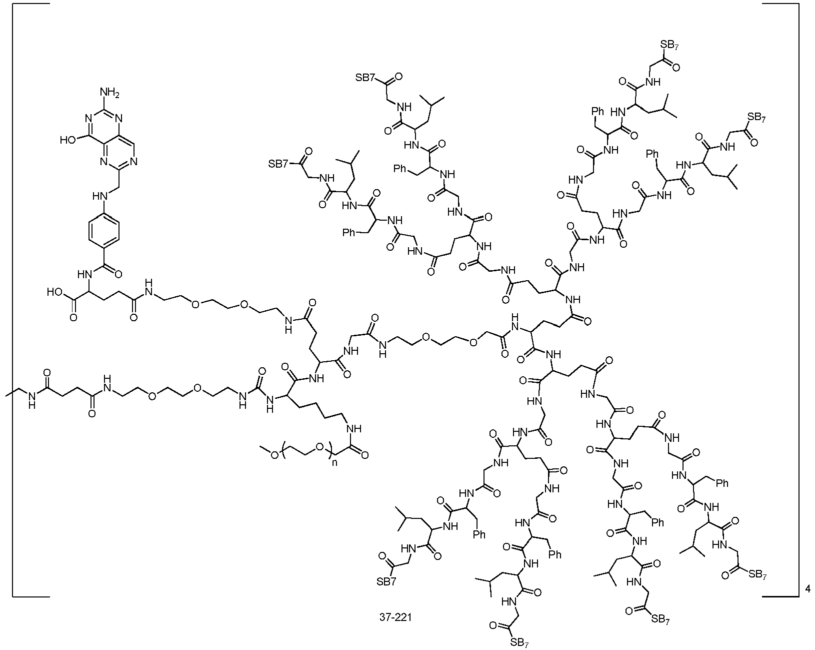

37-170

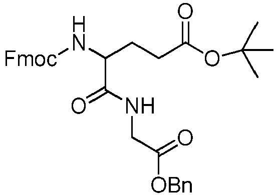

Gly-OBn.HCl (2.3697 g, 11.7517 mmol, purchased from Accela), HBTU (6.6851 g, 17.6276 mmol), HOBT (2.3820 g, 17.6276 mmol) and Fmoc-Glu(OtBu)-OH (5 g, 11.7517 mmol) were added to a 500 mL round-bottomed flask and then dissolved with DMF (50 mL), and the obtained solution was stirred for about 30 min at -5 °C; then, DIEA (8.7405 mL, 52.8827 mmol) was slowly added dropwise, and at the end of the addition, the obtained solution was further stirred at -5 °C overnight to react. At the end of the reaction, the reaction solution was transferred to a 1 L separatory funnel, a saturated sodium chloride solution (200 mL) and ethyl acetate (200 mL) were added; the obtained solution was shaken, extracted, concentrated to 100 mL, and washed with deionized water (150 mL × 2); the organic phase was evaporated to dryness, and dried, thus obtaining 6.73 g of Product 37-170.
37-175

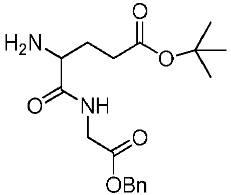

Product 37-170 (6.73 g, 11.7517 mmol) were added in a 250 mL flask and then dissolved with DMF (40 mL), morpholine (4.1 mL, 47.0068 mmol) was then added, and the mixed solution was stirred for 1 h at room temperature to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 5); the obtained organic phases were combined, concentrated to about 100 mL, washed with a saturated saline solution (200 mL × 1), then concentrated and evaporated to dryness, and dried in an oven, thus obtaining 4.12 g of Product 37-175.
37-178

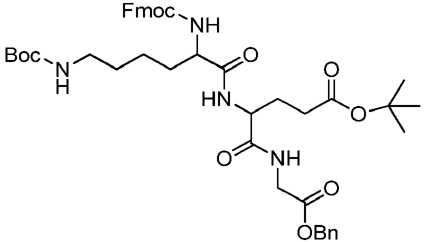

Fmoc-L-Lys (Boc)-OH (4.4 g, 11.1641 mmol, purchased from Accela), Product 37-175 (4.12 g, 11.7517 mmol), HBTU (6.6851 g, 17.6276 mmol) and HOBT (2.3820 g, 17.6276 mmol) were added to a 250 mL flask, and the obtained solution was stirred for about 30 min at 0 °C; then, DIEA (8.74 mL, 52.8827 mmol) was slowly added dropwise, and at the end of the addition, the obtained solution was further stirred at 0 °C overnight. At the end of the reaction, the reaction solution was transferred to a 1 L separatory funnel and extracted with a saturated saline solution (200 mL) and ethyl acetate (250 mL) to obtain an organic phase; the aqueous phase was washed with ethyl acetate (200 mL × 1); the obtained organic phases were combined, washed with a saturated saline solution (200 mL × 1), then concentrated and evaporated to dryness, thus obtaining 8.94 g of Product 37-178.
37-181

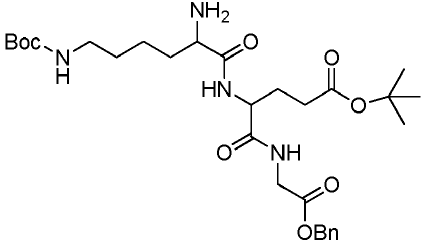

Product 37-178 (8.94 g, 11.7517 mmol) were added in a 250 mL flask and then dissolved with DMF (40 mL), morpholine (10.226 mL, 117.517 mmol) was then added, and the mixed solution was stirred for 1 h at room temperature to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 1), then concentrated and evaporated to dryness; silica gel powder (30 g) was added to the obtained solution, and the solution was then evaporated to dryness to obtain a powdery solid; the operations of dry sample loading, column chromatography, and elution with a mixed solution (1% ammonia water/3%-5% methanol/dichloromethane) were carried out; the elution product was then collected, concentrated, and evaporated to dryness, and the solid was dried in an oven. 4.0 g of Product 37-181 was obtained with a yield of 59%.
37-205

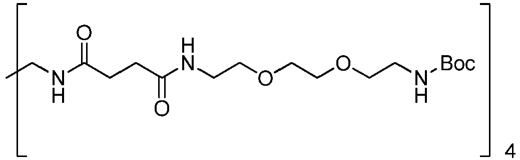

Product 37-160 (1 g, 4.027 mmol), HBTU (2.08 g, 1.35 mmol), HOBT (0.742 g, 1.35 mmol) and Product 43-137 (0.487 g, 0.915 mmol) were added to a 250 mL flask and dissolved with DMF (40 mL), and the obtained solution was at stirred -5 °C for about 30 min. Then DIEA (1.24 mL, 7.4819 mmol) was slowly added dropwise, and at the end of the addition, the obtained solution was first stirred at -5 °C for 1 h, and then stirred overnight at room temperature to further react. At the end of the reaction, the reaction solution was first transferred to a 1 L separatory funnel, a saturated sodium bicarbonate solution (200 mL) and ethyl acetate (200 mL) were added, and the obtained solution was shaken and extracted to obtain an organic phase; the aqueous phase was washed with ethyl acetate (200 mL × 1), the obtained organic phases were combined, concentrated to about 100 mL, washed with deionized water (150 mL × 1), and evaporated to dryness; the solid product was dissolved with methanol (40 mL) and dichloromethane (160 mL); silica gel powder (10 g) was then added to the obtained solution and the solution was evaporated to dryness to obtain a powdery solid; the operations of dry sample loading, column chromatography and gradient elution with a mixed solution (3%-5% methanol/dichloromethane) were carried out. 1.3 g of Product 37-205 was obtained.
37-209

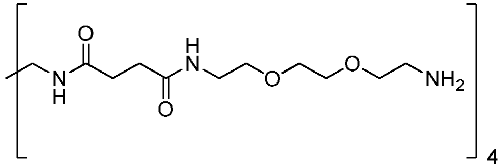

Product 37-205 (1.3 g, 0.915 mmol) was added to a 250 mL flask and dissolved with dichloromethane (10 mL), and trifluoroacetic acid (4.1 mL, 54.9 mmol) was then added dropwise, and at the end of the addition, the obtained solution was stirred at room temperature overnight to react. At the end of the reaction, n-hexane (80 mL) and methyl tert-butyl ether (30 mL) were added, the supernatant was discarded, n-hexane (80 mL) and methyl tert-butyl ether (30 mL) were added to the lower liquid, and such operations were repeated five times to obtain a viscous oily product, and the oily product was dissolved with methanol (20 mL) and dichloromethane (120 mL); silica gel powder (15 g) was then added to the obtained solution and the solution was evaporated to dryness to obtain a powdery solid; the operations of dry sample loading, column chromatography and gradient elution with a mixed solution (5%-10% methanol/dichloromethane) were carried out. 1 g of Product 37-209 was obtained.
37-210

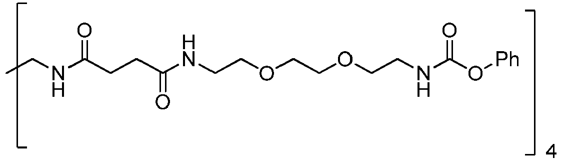

Product 37-209 (1.02 g, 0.965 mmol) was added to a 250 mL flask and dissolved with dichloromethane (30 mL), and triethylamine (1.26 mL, 8.9736 mmol) was then added, and the mixed solution was stirred at 0 °C for about 20 min; then, phenyl chloroformate (0.85 mL, 6.730 mmol) was slowly added dropwise, and at the end of the addition, the obtained solution was further stirred at 0 °C overnight to react. At the end of the reaction, the reaction solution was transferred to a 1 L separatory funnel, deionized water (100 mL) and ethyl acetate (150 mL) were added, and the obtained solution was shaken and extracted to obtain an organic phase; the aqueous phase was washed with dichloromethane (100 mL × 3), the obtained organic phases were combined, and concentrated; silica gel powder (10 g) was then added to the obtained solution and the solution was evaporated to dryness to obtain a powdery solid; the operations of dry sample loading, column chromatography and gradient elution with a mixed solution (3%-5% methanol/dichloromethane) were carried out. 0.9 g of Product 37-210 was obtained with a yield of 61%.
37-212

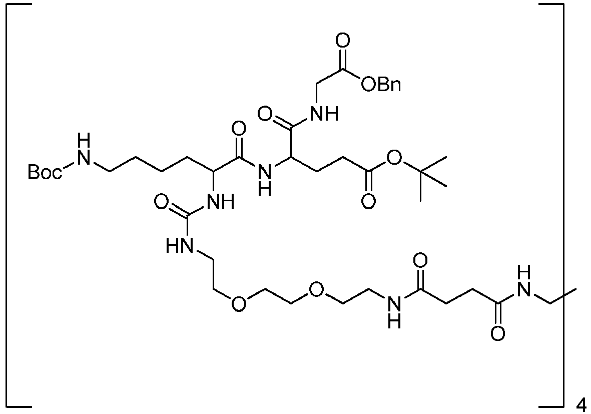

Product 37-210 (0.9 g, 0.5868 mmol) and Product 37-181 (1.494 g, 2.582 mmol) were added to a 250 mL flask and dissolved with DMF (40 mL), and the mixed solution was stirred at 110 °C for about 20 min; Then DIEA (3.1 mL, 18.778 mmol) was slowly added dropwise, and at the end of the addition, the obtained solution was further stirred at 110 °C overnight to react. At the end of the reaction, the reaction solution was transferred to a 1 L separatory funnel, a saturated sodium bicarbonate solution (200 mL) and ethyl acetate (200 mL) were added, and the obtained solution was shaken and extracted to obtain an organic phase; the aqueous phase was washed with ethyl acetate (200 mL × 1), the obtained organic phases were combined, concentrated to about 100 mL, washed with deionized water (150 mL × 1), and evaporated to dryness, and the solid product was dissolved with methanol (40 mL) and dichloromethane (160 mL); silica gel powder (10 g) was then added to the obtained solution and the solution was evaporated to dryness to obtain a powdery solid; the operations of dry sample loading, column chromatography and gradient elution with a mixed solution (4%-7% methanol/dichloromethane) were carried out. 0.8 g of Product 37-212 was obtained with a yield of 40%.

¹H-NMR (600 MHz, DMSO-d₆) δ 8.48 - 8.34 (m, 5H), 8.13 - 7.88 (m, 6H), 7.54 - 7.24 (m, 20H), 6.87 - 6.64 (m, 6H), 6.46 - 6.25 (m, 2H), 6.18 - 6.01 (m, 9H), 4.39 - 4.20 (m, 5H), 4.18 - 4.03 (m, 5H), 3.95 - 3.91 (m, 4H), 3.90 - 3.79 (m, 5H), 3.68 - 3.59 (m, 4H), 3.57 - 3.45 (m, 34H), 3.21 - 3.03 (m, 11H), 2.98 - 2.83 (m, 13H), 2.75 - 2.61 (m, 2H), 2.63 (m, 17H), 2.29 - 2.13 (m, 9H), 2.04 -1.91 (m, 5H), 1.80 - 1.67 (m, 5H), 1.61 - 1.48 (m, 16H), 1.46 - 1.33 (m, 72H), 1.35 - 1.31 (m, 9H), 1.27 - 1.15 (m, 17H).
37-214

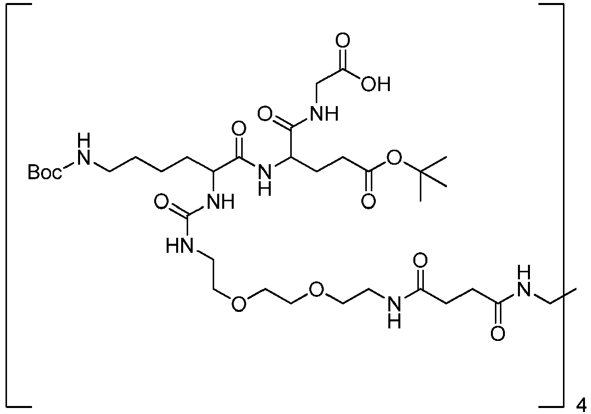

Product 37-212 (0.0953 g, 0.0823 mmol) and 10%Pd/C (0.0270 g) were added in a hydrogenation reactor and then dissolved with DMF (30 mL); hydrogen was introduced to a pressure of Pa=1.8 MPa in the reactor, and the mixed solution then was stirred overnight at room temperature to react. At the end of the reaction, the reaction solution was filtered with diatomaceous earth; the diatomaceous earth was washed with DMF (20 mL × 3), and the DMF solution was combined, as the raw material for the next step.
37-215

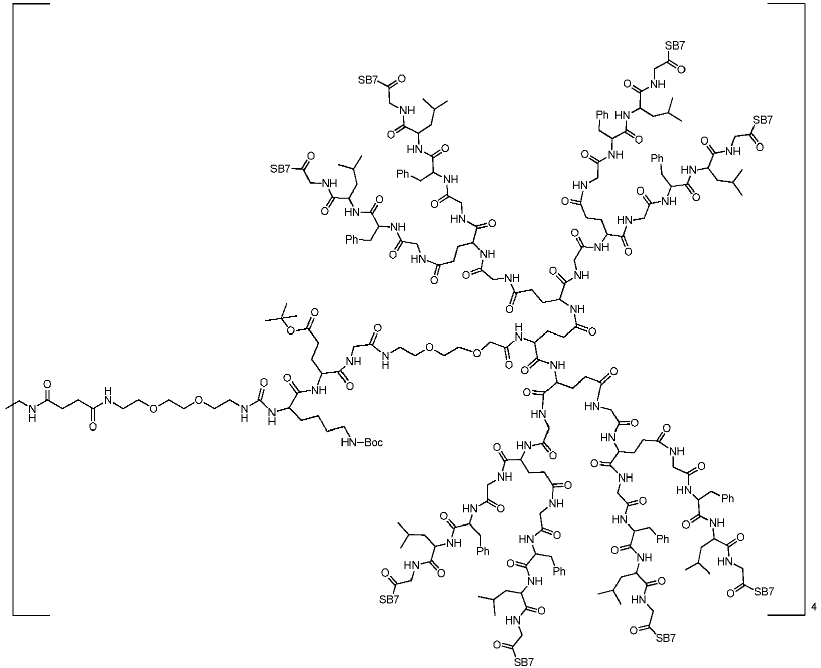

Product 37-214 (0.084 g, 0.027 mmol), Product 43-132 (1.0 g, 0.1207 mmol), HBTU (0.6144 g, 1.62 mmol) and HOBT (0.2189 g, 1.62 mmol) were added in a 250 mL flask, and the obtained solution was stirred for about 10 min at -5 °C; then, DIEA (0.8 mL, 4.86 mmol) was slowly added dropwise; the reaction continued at -5 °C for 1 h, and the reaction solution was stirred at room temperature overnight to react. At the end of the reaction, n-hexane (150 mL) and methyl tert-butyl ether (40 mL) were first added for precipitation; the supernatant was discarded, n-hexane (150 mL) and methyl tert-butyl ether (40 mL) were added again to the lower oily product, and such operations were repeated four times to obtain a viscous oily product; methyl tert-butyl ether (100 mL) was added, and a powdery solid was then precipitated; the suction filtering was carried out and the filter cake was washed with methyl tert-butyl ether (50 mL × 3), and dried, thus obtaining 0.9767 g of Product 37-215.
37-218

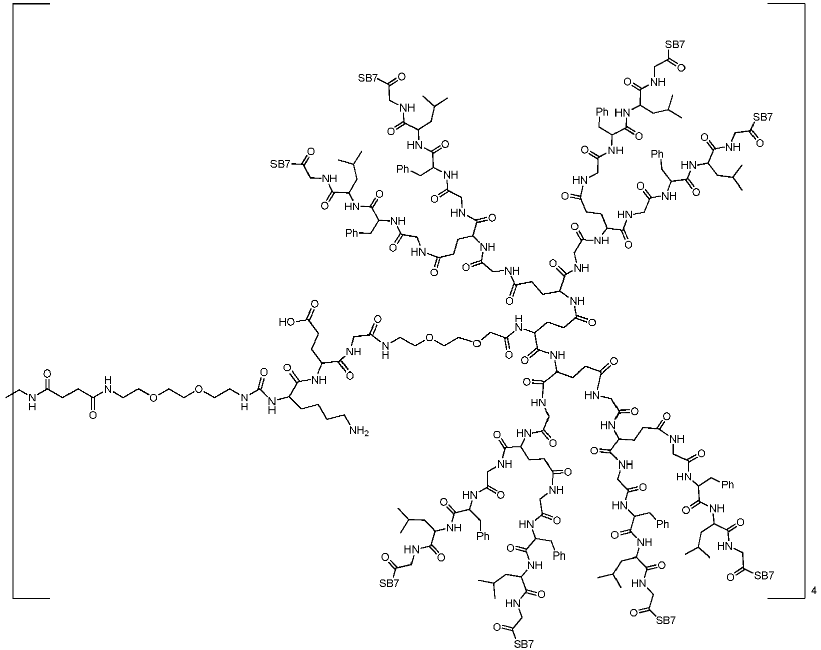

Product 37-215 (0.9767 g, 0.027 mmol) was added to a 100 mL flask, dichloromethane (15 mL) and TFA (4 mL, 27 mmol) were added in sequence, and the reaction solution was stirred overnight at room temperature to react. At the end of the reaction, the reaction solution was concentrated to a small amount; methyl tert-butyl ether (150 mL) was then added and the powdery product was precipitated; suction filtering was then carried out, and the filter cake was washed with methyl tert-butyl ether (50 mL × 3), and dissolved with a mixed solution of methanol (30 mL) and dichloromethane (120 mL); silica gel powder (10 g) was then added to the obtained solution and the solution was evaporated to dryness to obtain a powdery solid; the operations of dry sample loading, column chromatography and elution with a mixed solution (1% ammonia water: 6%-10% methanol/dichloromethane) were carried out; the elution product was collected, concentrated and evaporated to dryness. 0.66 g of Product 37-218 was obtained with a yield of 69%.
37-220

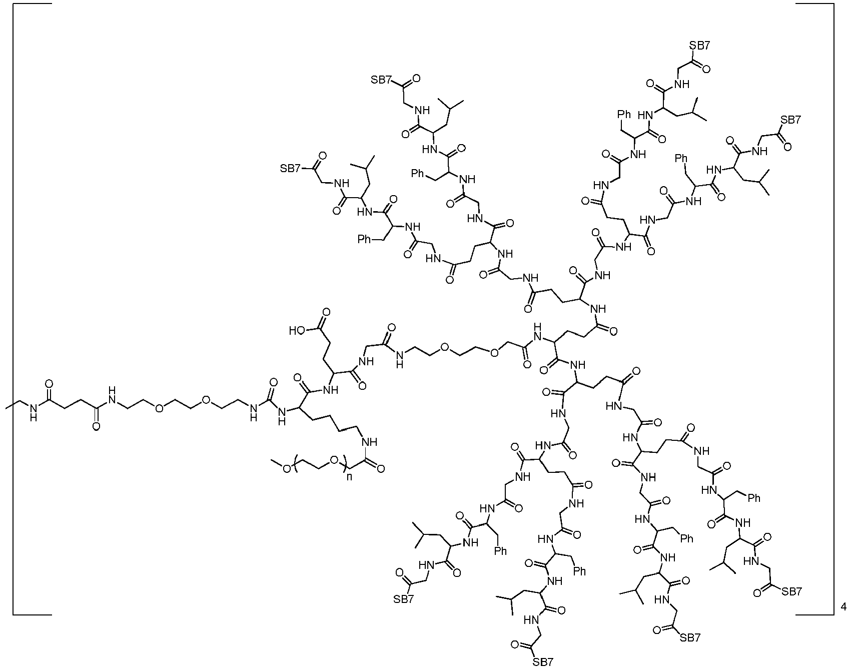

Product 37-218 (0.66 g, 0.0186 mmol) was added in a 250 mL flask and then dissolved with DMF (40 mL), and the obtained solution was stirred for about 30 min at -5 °C; then, DIEA (0.153 mL, 1.49 mmol) was slowly added dropwise, and the obtained solution was first stirred at low temperature for 10 min, after that, M-SCM-10K (1.18 g, 0.1114 mmol, purchased from JenKem) was added, and the obtained solution was then slowly stirred at room temperature to react for one week in the dark. At the end of the reaction, methyl tert-butyl ether (200 mL) and n-hexane (70 mL) were added to separate out a solid product, filtering suction was carried out and the filter cake was washed with methyl tert-butyl ether (40 mL × 3), and dissolved with a mixed solution of methanol/dichloromethane (1:4) (100 mL); silica gel powder (10 mL) was added to the obtained solution, and the solution was then evaporated to dryness to obtain a powdery solid; the operations of dry sample loading, column chromatography, and elution with a mixed solution (1% ammonia water/4%-8% methanol/dichloromethane) were carried out; the elution product was then collected, concentrated, and dried in a vacuum oven. 0.98 g of Product 37-220 was obtained with a yield of 70%.
37-221

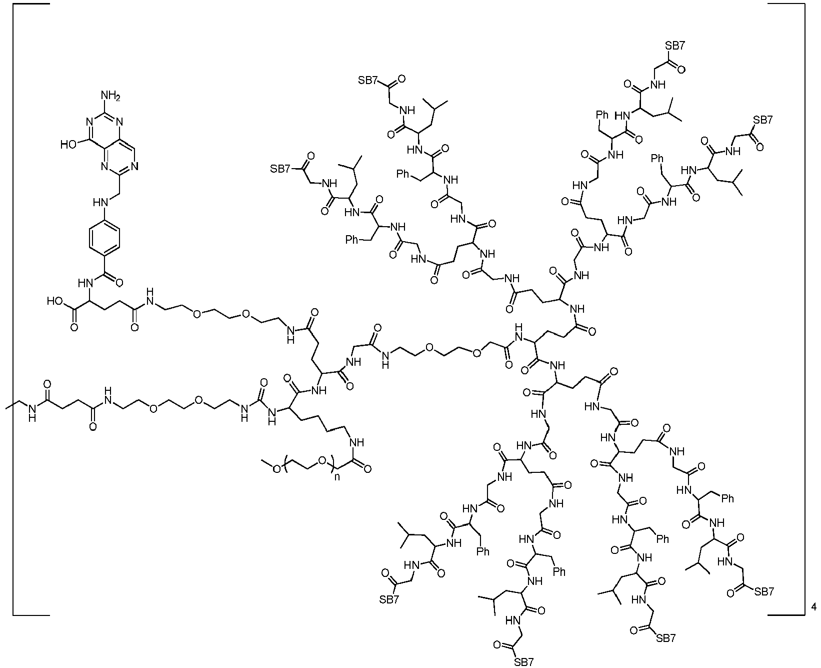

Product 37-220 (1.27 g, 0.0164 mmol), Product 37-163(0.1874 g, 0.367 mmol), HBTU (0.373 g, 0.984 mmol) and HOBT (0.133 g, 0.984 mmol) were added in a 250 mL flask, and the obtained solution was stirred for about 10 min at -5 °C; then, DIEA (0.488 mL, 2.952 mmol) was slowly added dropwise, and the reaction continued at -5 °C for 1 h, and the reaction solution was stirred at room temperature overnight to react. At the end of the reaction, n-hexane (150 mL) and methyl tert-butyl ether (40 mL) were first added for precipitation, the supernatant was discarded, n-hexane (150 mL) and methyl tert-butyl ether (40 mL) were added again to the lower oily product, and such operations were repeated four times to obtain a viscous oily product; methyl tert-butyl ether (100 mL) was added, and a powdery solid was then precipitated; the suction filtering was carried out and the filter cake was washed with methyl tert-butyl ether (50 mL × 3), and dissolved with a mixed solution of methanol (40 mL) and dichloromethane (160 mL); silica gel powder (10 g) was then added to the obtained solution and the solution was evaporated to dryness to obtain a powdery solid; the operations of dry sample loading, column chromatography and elution with a mixed solution (1% ammonia water: 5%-10% methanol/dichloromethane) were carried out; the elution product was collected, concentrated and evaporated to dryness. 0.65 g of Product 37-221 was obtained with a yield of 65%.

¹H-NMR (600 MHz, DMSO-d₆) δ 8.19 - 8.14 (m, 59H), 8.12 - 8.03 (m, 108H), 8.01 - 7.96 (m, 24H), 7.91 - 7.77 (m, 29H), 7.64 - 7.54 (m, 32H), 7.50 - 7.47 (m, 16H), 7.34- 7.27 (m, 118H), 7.24 - 7.15 (m, 230H), 7.14 - 7.01 (m, 208H), 4.63- 4.42 (m, 52H), 4.29- 4.03 (m, 130H), 3.98 - 3.85 (m, 76H), 3.78 - 3.58 (m, 211H), 3.56 - 3.43 (m, 3817H), 3.34- 3.13 (m, 17H), 3.11- 3.05 (m, 85H), 2.92- 2.73 (m, 63H), 2.69 - 2.52 (m, 60H), 2.41 - 2.38 (m, 30H), 2.36 - 2.27 (m, 80H), 2.22- 2.09 (m, 45H), 1.83- 1.73 (m, 47H), 1.60- 1.43(m, 102H), 1.34- 1.23 (m, 190H), 1.19 - 1.13 (m, 136H), 1.08 - 1.97 (m, 9H), 0.95 - 0.87 (m, 87H), 0.85 - 0.82 (m, 99H), 0.81 - 0.73 (m, 90H), 0.60- 0.54 (m, 73H).

### Synthesis route of 44-174:

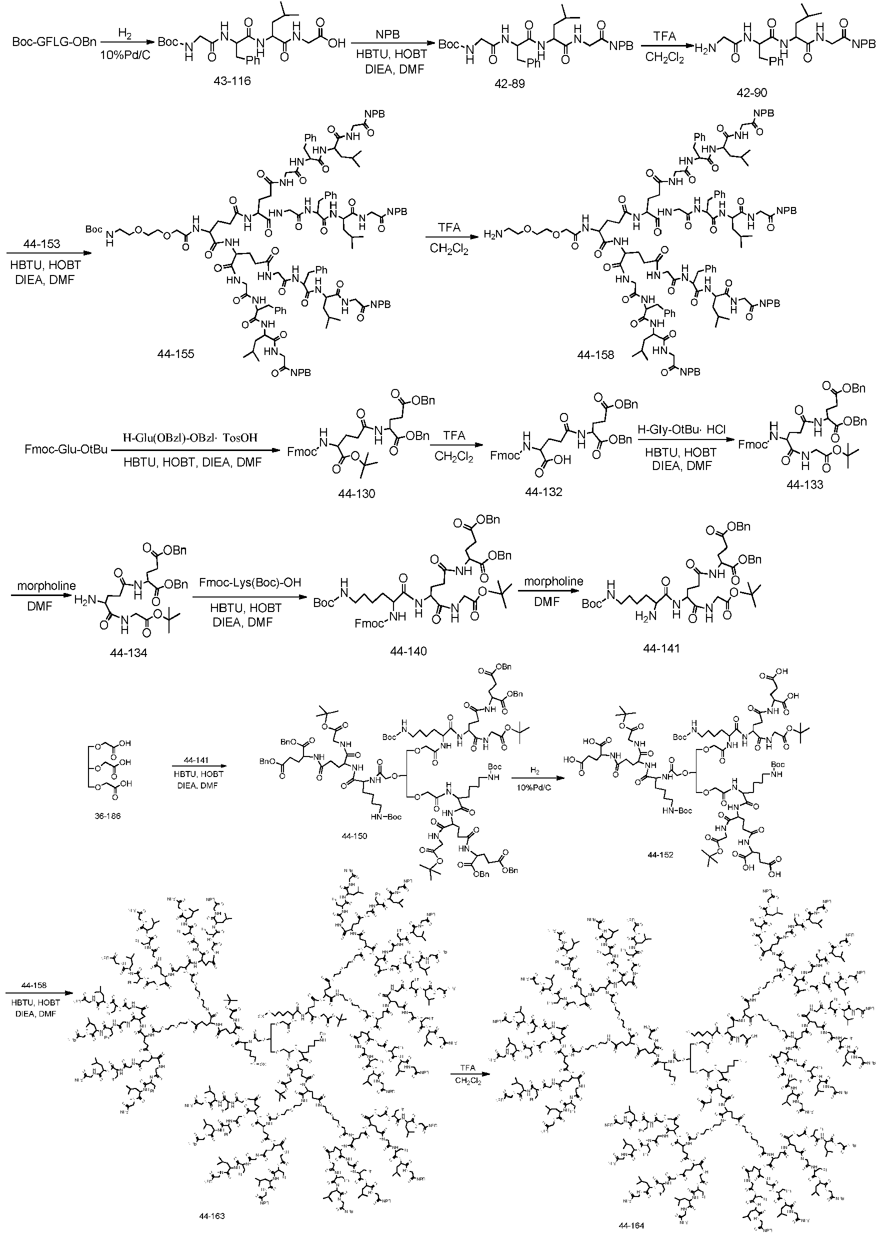

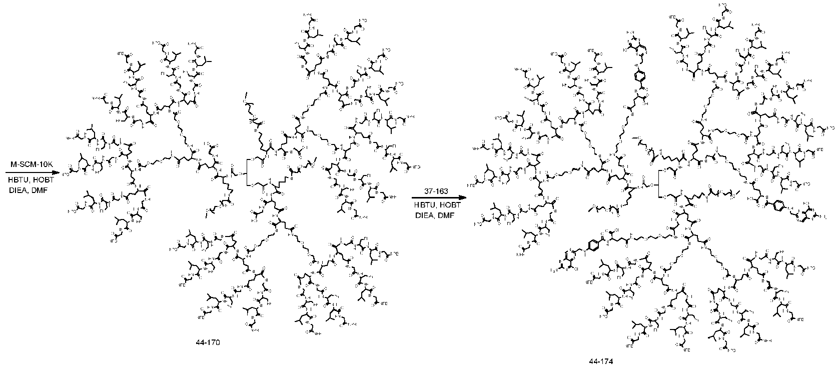

42-89

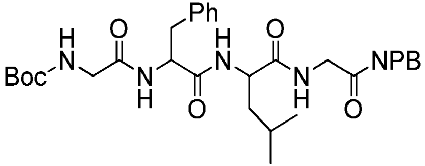

The DMF solution of Product 43-116 (8.4534 g, 17.1618 mmol), Niraparib (NPB, 4.5821 g, 14.3015 mmol), HBTU (8.1356 g, 21.4523 mmol) and HOBT (2.8986 g, 21.4523 mmol) were added to a 250 mL round-bottomed flask and then dissolved with DMF (60 mL); the obtained solution in the flask was stirred at -5 °C for about 30 min and DIEA (10.6 mL, 64.3568 mmol) was slowly added dropwise. At the end of the addition, the reaction solution in the flask was stirred at -5 °C for 1 h, and then stirred overnight at room temperature. At the end of the reaction, the reaction solution was transferred to a 1 L separatory funnel, a saturated sodium chloride aqueous solution (300 mL) and ethyl acetate (200 mL) were added, and the obtained solution was shaken and extracted; the aqueous phase was separated; ethyl acetate (200 mL) was added to the aqueous phase, and the obtained solution was shaken and extracted; the aqueous phase was separated; the obtained organic phases were combined, a saturated sodium chloride aqueous solution (300 mL) was added again to the organic phase, and the obtained solution was shaken and extracted; the aqueous phase was separated. After that, deionized water (300 mL) was added to the organic phase, and the obtained solution was shaken and extracted; the aqueous phase was separated. Finally, the organic phase was concentrated, evaporated to dryness, and then dried in an oven, thus obtaining 11.3695 g of Product 42-89.
42-90

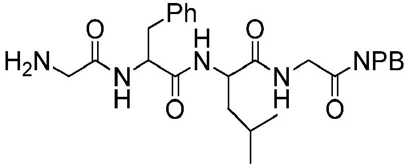

Product 42-89 (11.3695 g, 14.3015 mmol) was added in a 250 mL round-bottomed flask and then dissolved with dichloromethane (30 mL), TFA (15.9 mL, 214.5225 mmol) was then added with stirring, and the obtained solution in the flask was stirred at room temperature overnight to react. At the end of the reaction, the reaction solution was concentrated and evaporated to remove dichloromethane. Then, methyl tert-butyl ether (150 mL) was added for precipitation, a powdery solid was obtained, and the filtering was carried out. The filter cake was washed with methyl tert-butyl ether (60 mL) and dissolved with a mixed solvent (20% methanol/dichloromethane) (60 mL); silica gel powder (65 mL) was added to the obtained solution, and the solution was then evaporated to dryness; the operations of dry sample loading, column chromatography, and elution with an eluent (0%-2% methanol: 100%-98% dichloromethane) were carried out; the elution product was then collected, concentrated, evaporated to dryness, and dried. 8.6 g of Product 42-90 was obtained with a yield of 86.6%.
44-153

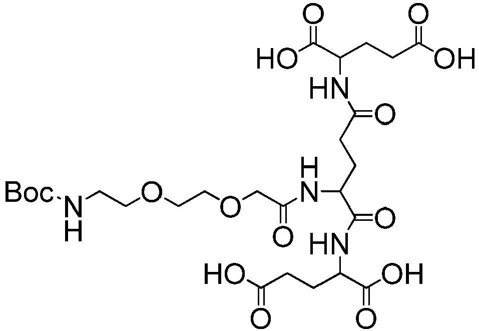

Product 35-85 (1.1433 g, 0.6586 mmol, home-made) and 10% Pd/C (120 mg) were added in a hydrogenation reactor and then dissolved with DMF (40 mL), and the hydrogenation reactor was then sealed; hydrogen was introduced to a pressure of 2.0 MPa in the reactor; the hydrogenation reactor was placed at room temperature and the rection solution was stirred overnight. At the end of the reaction, the reactor was taken out; the reaction solution was evenly added dropwise to a sand core funnel filled with compacted diatomaceous earth, and then suction filtering was carried out, and the diatomaceous earth was washed with DMF until it did not contain any product, thus obtaining the reaction solution of the product.
44-155

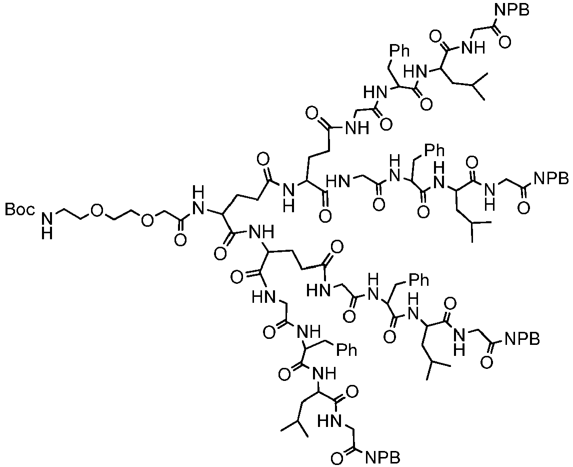

Solution of Product 44-153 (1.7026 g, 2.6168 mmol), Product 42-90 (8.3637 g, 12.0373 mmol), HBTU (5.9543 g, 15.7006 mmol) and HOBT (2.1215 g, 15.7006 mmol) were added to a 500 mL round-bottomed flask and then dissolved with DMF (80 mL); the obtained solution in the flask was stirred at -5 °C for about 30 min. Then DIEA (7.8 mL, 47.1018 mmol) was slowly added dropwise; at the end of the addition, the reaction solution was first stirred at -5 °C for 1 h to react, and finally stirred at room temperature for 3 h to react. At the end of the reaction, n-hexane (150 mL) and methyl tert-butyl ether (30 mL) were first added to precipitate the reaction solution; the supernatant was discarded; n-hexane (150 mL) and methyl tert-butyl ether (30 mL) were added again to precipitate the lower oily product; the process of precipitation was repeated three times to obtain an oily solid. The oily solid was dissolved with dichloromethane (10 mL); methyl tert-butyl ether (150 mL) was added for precipitation, and a powdery solid was obtained; filtering was carried out, and a solid product was obtained; the solid product was washed with methyl tert-butyl ether (60 mL) and finally dried in an oven, thus obtaining 8.7868 g of Product 44-155.
44-158

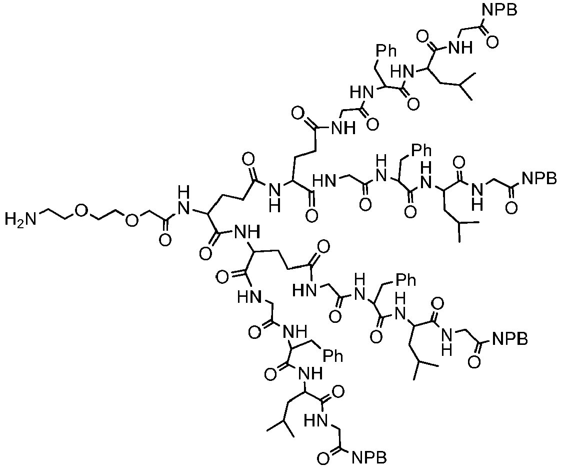

Product 44-155 (8.7868 g, 2.6168 mmol) was added in a 500 mL round-bottomed flask and then dissolved with dichloromethane (20 mL), TFA (15.6 mL, 209.3440 mmol) was then added with stirring, and the obtained solution in the flask was stirred at room temperature to react. At the end of the reaction, the reaction solution was concentrated at reduced pressure to remove dichloromethane. Then, methyl tert-butyl ether (150 mL) was added for precipitation, a powdery solid was obtained, and the filtering was carried out. The filter cake was washed with methyl tert-butyl ether (60 mL) and dissolved with a mixed solvent (100 mL) (20% methanol/dichloromethane); silica gel powder (60 mL) was added to the obtained solution, and the solution was then evaporated to dryness; the operations of dry sample loading, column chromatography, and elution with an eluent (1% ammonia water/1%-8% methanol/98%-91% dichloromethane) were carried out; the elution product was then collected, concentrated, evaporated to dryness, and dried. 6.8199 g of Product 44-158 was obtained with a yield of 80%.
44-130

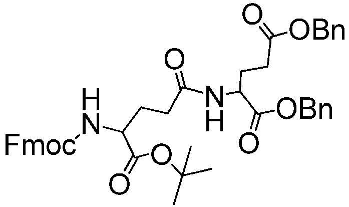

Fmoc-Glu-OtBu (8.0 g, 18.8027 mmol, purchased from Ark Pharm), HBTU (10.6961 g, 28.2041 mmol), HOBT (3.8110 g, 28.2041 mmol) and H-Glu(OBzl)-OBzl·TosOH (9.8631 g, 19.7429 mmol, purchased from Ark Pharm) were added to a 250 mL round-bottomed flask and then dissolved with DMF (80 mL); the obtained solution in the flask was stirred at -5 °C for about 30 min; then, DIEA(17.1 mL, 103.4150 mmol) was slowly added dropwise; at the end of the addition, the reaction solution in the flask was first stirred at -5 °C for 1 h to react, and finally stirred at room temperature for 3 h to react. At the end of the reaction, the reaction solution was transferred to a 1 L separatory funnel, a saturated sodium chloride aqueous solution (300 mL) and ethyl acetate (200 mL) were then added, the obtained solution was shaken and extracted, and the aqueous phase and the organic phase were separated; ethyl acetate (100 mL) was then added to the aqueous phase, the obtained solution was shaken and extracted, and the aqueous phase and the organic phase were separated. Then the obtained organic phases were combined, a saturated sodium chloride aqueous solution (300 mL) was then added to the organic phase, the obtained solution was shaken and extracted, and the aqueous phase and the organic phase were separated; deionized water (300 mL) was then added to the organic phase, the obtained solution was shaken and extracted, and the aqueous phase and the organic phase were separated. Finally, the organic phase was concentrated, evaporated to dryness, and dried, thus obtaining 13.8168 g of Product 44-130.
44-132

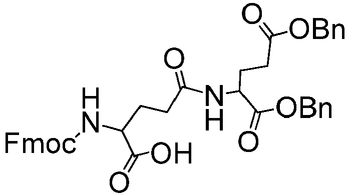

Product 44-130 (13.8168 g, 18.8027 mmol) was added in a 250 mL round-bottomed flask and then dissolved with dichloromethane (20 mL), TFA (14.0 mL, 188.027 mmol) was then added with stirring, and the obtained solution in the flask was stirred at room temperature to react. At the end of the reaction, the reaction solution was concentrated at reduced pressure to remove dichloromethane, and then transferred to a 1 L separatory funnel; a saturated sodium chloride aqueous solution (300 mL) and ethyl acetate (200 mL) were then added, the obtained solution was shaken and extracted, and the aqueous phase and the organic phase were separated; ethyl acetate (100 mL) was then added to the aqueous phase, the obtained solution was shaken and extracted, and the aqueous phase and the organic phase were separated. Then, the obtained organic phases were combined, a saturated sodium chloride aqueous solution (300 mL) was then added to the organic phase, the obtained solution was shaken and extracted, and the aqueous phase was separated; then, deionized water (300 mL) was added to the organic phase, the obtained solution was shaken and extracted, and the aqueous phase was separated. Finally, the organic phase was concentrated, evaporated to dryness, and dried, thus obtaining 12.7620 g of Product 44-132.
44-133

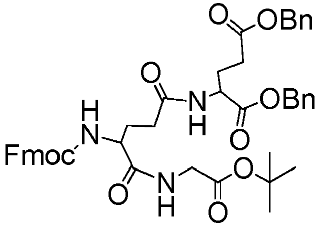

Product 44-132 (12.7620 g, 18.8027 mmol), H-Gly-OtBu·HCl (3.3095 g, 19.7428 mmol, purchased from Accela), HBTU (10.6961 g, 28.2041 mmol) and HOBT (3.8109 g, 28.2041 mmol) were added to a 250 mL round-bottomed flask and then dissolved with DMF (80 mL); the obtained solution in the flask was stirred at -5 °C for about 30 min. Then DIEA (21.8 mL, 131.6189 mmol) was slowly added dropwise; at the end of the addition, the reaction solution in the flask was further stirred at -5 °C. At the end of the reaction, the reaction solution was first transferred to a 1 L separatory funnel; a saturated sodium chloride aqueous solution (300 mL) and ethyl acetate (200 mL) were added, and the obtained solution was shaken and extracted; the aqueous phase was separated; ethyl acetate (100 mL) was added to the aqueous phase, and the obtained solution was shaken and extracted; the aqueous phase was separated; then, the obtained organic phases were combined; a saturated sodium chloride aqueous solution (300 mL) was added again to the organic phase, and the obtained solution was shaken and extracted; the aqueous phase was separated; then, deionized water (300 mL) was added to the organic phase, and the obtained solution was shaken and extracted; the aqueous phase was separated. Finally, the organic phase was concentrated, evaporated to dryness, and dried, thus obtaining 14.8895 g of Product 44-133.
44-134

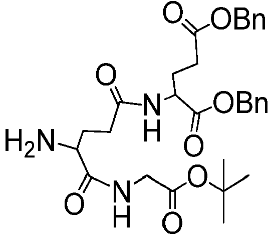

Product 44-133 (14.8895 g, 18.8027 mmol) was added in a 500 mL round-bottomed flask and then dissolved with DMF (30 mL), piperidine (18.6 mL, 188.027 mmol) was then added with stirring; the obtained solution in the flask was stirred at room temperature for 2 h to react. At the end of the reaction, the reaction solution was first transferred to a 1 L separatory funnel; a saturated sodium chloride aqueous solution (300 mL) and ethyl acetate (200 mL) were added, and the obtained solution was shaken and extracted; the aqueous phase was separated; ethyl acetate (100 mL) was added to the aqueous phase, and the obtained solution was shaken and extracted; the aqueous phase was separated; then, the obtained organic phases were combined, and a saturated sodium chloride aqueous solution (300 mL) was added to the organic phase, and the obtained solution was shaken and extracted; the aqueous phase was separated; then, deionized water (300 mL) was added to the organic phase, and the obtained solution was shaken and extracted; the aqueous phase was separated. Finally, the organic phase was concentrated, evaporated to dryness, and dried, thus obtaining 10.7110 g of Product 44-134.
44-140

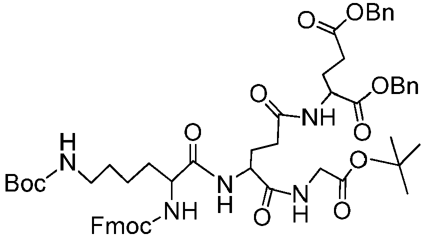

Fmoc-Lys (Boc)-OH (7.3415 g, 15.6689 mmol), Product 44-134 (10.7110 g, 18.8027 mmol), HBTU (8.9134 g, 23.5034 mmol) and HOBT (3.1758 g, 23.5034 mmol) were added to a 500 mL round-bottomed flask and then dissolved with DMF (80 mL); the obtained solution in the flask was stirred at -5 °C for about 30 min. Then DIEA (13.0 mL, 78.3445 mmol) was slowly added dropwise; at the end of the addition, the reaction solution in the flask was stirred at -5 °C for 1 h, and then stirred overnight at room temperature to further react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product, and the process of precipitation was repeated three times to obtain an oily solid. The oily solid was dissolved with dichloromethane (10 mL), precipitated with methyl tert-butyl ether (150 mL) to obtain a powdery solid, and filtered to obtain a solid product; the solid product was washed with methyl tert-butyl ether (60 mL) and dried in an oven. 15.9849 g of Product 44-140 was obtained.

¹H-NMR (600 MHz, DMSO-d₆) δ 8.27 - 8.13 (m, 3H), 8.05 - 7.94 (m, 3H), 7.89 (s, 1H), 7.76 - 7.69 (m, 2H), 7.49 - 7.46 (m, 1H), 7.44 - 7.41 (m, 2H), 7.37 - 7.29 (m, 11H), 2.47 - 2.37 (m, 1H), 5.18 - 5.02 (m, 4H), 4.38 - 4.18 (m, 5H), 3.81 - 3.54 (m, 6H), 3.19 - 3.11 (m, 4H), 2.38-2.4 (m, 2H), 2.23 -2.18 (m, 2H), 2.04 - 1.73 (m, 4H), 1.67 - 1.47 (m, 3H), 1.39 - 1.36 (m, 18H).
44-141

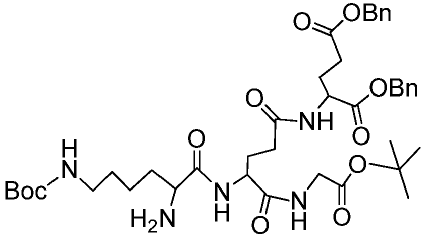

Product 44-140 (7.6 g, 7.4474 mmol) was added in a 250 mL round-bottomed flask and then dissolved with DMF (30 mL), morpholine (9.7 mL, 111.7110 mmol) was then added with stirring; the reaction flask was finally placed at room temperature and the reaction solution was stirred for 2 h to react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product, and the process of precipitation was repeated three times to obtain an oily solid. The oily solid was dissolved with dichloromethane (10 mL), precipitated with methyl tert-butyl ether (150 mL) to obtain a powdery solid, and then filtered to obtain a solid product; the solid product was washed with methyl tert-butyl ether (60 mL) and dried in an oven. 6.3 g of Product 44-141 was obtained with a yield of 50.39%.
44-150

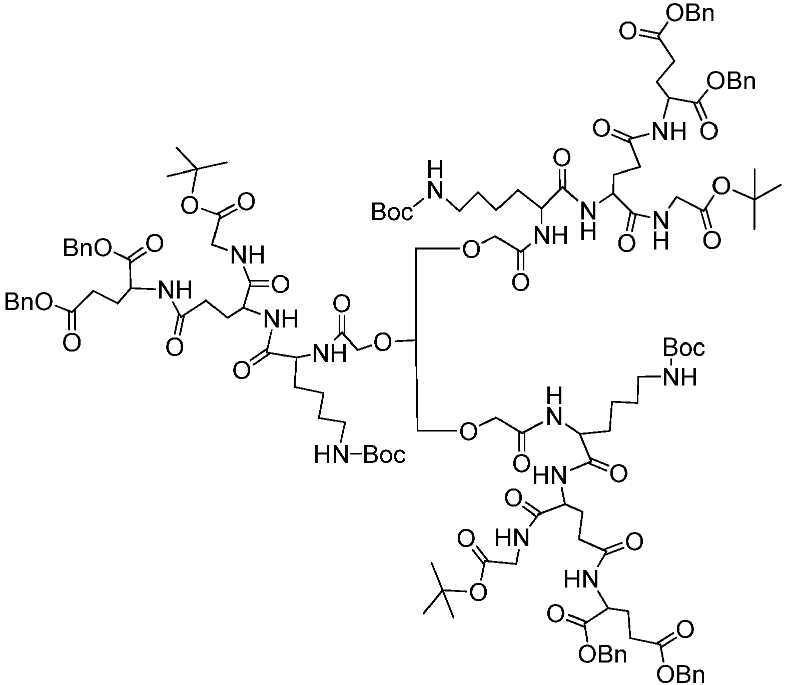

The DMF solution of Product 36-186 (0.5551 g, 2.0853 mmol, synthesized according to the synthesis method of Product 37-172, just replace the pentaerythritol to glycerin), Product 44-141 (5.99 g, 7.5069 mmol), HBTU (3.5586 g, 9.3836 mmol) and HOBT (1.2679 g, 9.3836 mmol) were added to a 250 mL round-bottomed flask and then dissolved with DMF (80 mL), and the obtained solution in the flask was stirred at -5 °C for about 30 min; then, DIEA (4.7 mL, 28.1516 mmol) was slowly added dropwise, at the end of the addition, the reaction solution in the flask was stirred for 1 h to react, and finally stirred at room temperature. At the end of the reaction, the reaction solution was transferred to a 1 L separatory funnel, a saturated sodium bicarbonate aqueous solution (400 mL) and ethyl acetate (300 mL) were added, the obtained solution was shaken and extracted, and the aqueous phase and the organic phase were separated. Then, deionized water (300 mL) was added to the organic phase, the obtained solution was shaken and extracted, and the aqueous phase and the organic phase were separated. Then, a saturated sodium chloride aqueous solution (300 mL) was added to the organic phase, the obtained solution was shaken and extracted, and the aqueous phase and the organic phase were separated. Finally, the organic phase was concentrated, evaporated to dryness, and the organic phase was dissolved with a mixed solvent (100 mL) (20% methanol/dichloromethane); silica gel powder (50 mL) was added to the obtained solution, and the solution was then evaporated to dryness; the operations of dry sample loading, column chromatography, and elution with an eluent (1%-6% methanol: 99%-94% dichloromethane) were carried out; the elution product was then collected, concentrated, evaporated to dryness and dried. 3.8827 g of Product 44-150 was obtained with a yield of 71.45%. ¹H-NMR (600 MHz, DMSO-d₆) δ 8.65 - 8.08 (m, 15H), 7.73 - 7.16 (m, 30H), 5.25 - 4.92 (m, 12H), 4.63 - 4.25 (m, 15H), 3.86 - 3.64 (m, 11H), 3.22-3.18 (m, 6H), 2.47 - 2.38 (m, 12H), 2.07 - 2.05 (m, 12H), 1.84 - 1.47 (m, 12H), 1.4 - 1.21 (m, 60H).
44-152

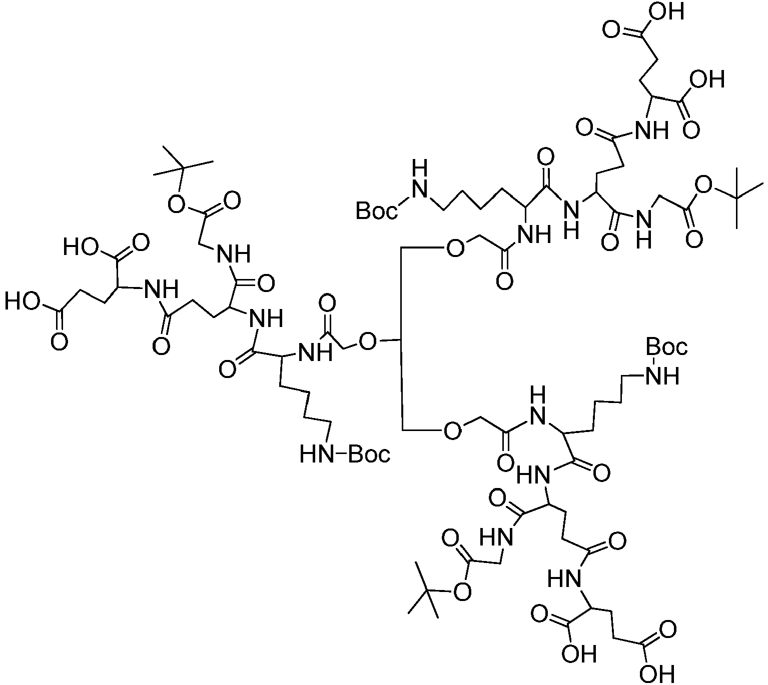

Product 44-150 (0.9221 g, 0.3539 mmol, home-made) and 10%Pd/C (50 mg) were added in a hydrogenation reactor and then dissolved with DMF (30 mL), and the hydrogenation reactor was then sealed; hydrogen was introduced to a pressure of 1.6 MPa in the reactor; the hydrogenation reactor was placed at room temperature and the rection solution was stirred overnight. At the end of the reaction, the reactor was taken out, and the reaction solution was evenly added dropwise to a sand core funnel filled with compacted diatomaceous earth; suction filtering was carried out, and the diatomaceous earth was washed with DMF until it did not contain any product, thus obtaining the reaction solution of the product.
44-163

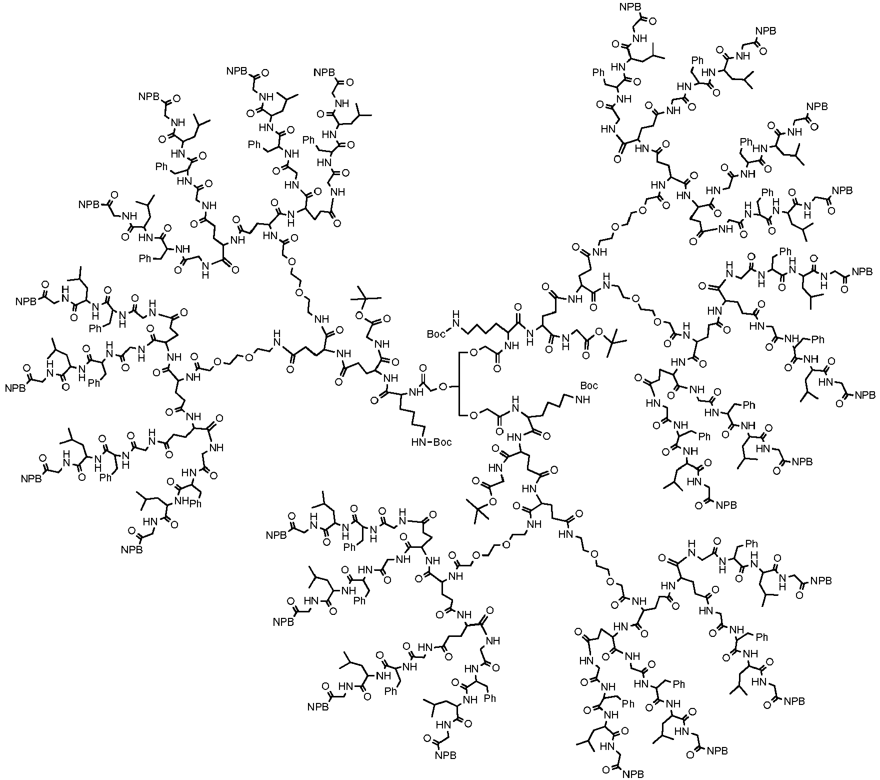

Solution of Product 44-152 (0.7308 g, 0.3539 mmol), Product 44-158 (7.6092 g, 2.3357 mmol), HBTU (1.2079 g, 3.1851 mmol) and HOBT (0.4304 g, 3.1851 mmol) were added to a 250 mL round-bottomed flask and then dissolved with DMF (80 mL); the obtained solution in the flask was stirred at -5 °C for about 30 min. Then DIEA (1.6 mL, 9.5553 mmol) was slowly added dropwise; at the end of the addition, the reaction solution in the flask was stirred at -5 °C for 1 h to react, and finally stirred at room temperature. At the end of the reaction, n-hexane (150 mL) and methyl tert-butyl ether (30 mL) were first added to precipitate the reaction solution; the supernatant was discarded; n-hexane (150 mL) and methyl tert-butyl ether (30 mL) were added again to precipitate the lower oily product, and the process of precipitation was repeated three times to obtain an oily solid. The oily solid was dissolved with dichloromethane (10 mL), precipitated with methyl tert-butyl ether (150 mL) to obtain a powdery solid, and filtered to obtain a solid product. The solid product was washed with methyl tert-butyl ether (60 mL) and dried in an oven. 7.6101 g of Product 44-163 was obtained.
44-164

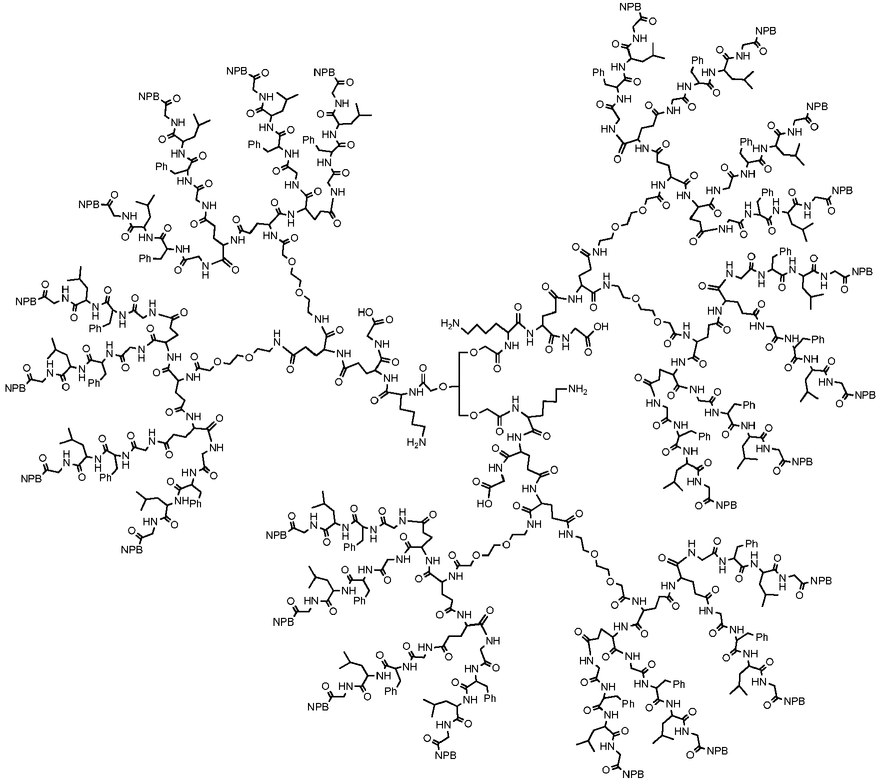

Product 44-163 (7.6101 g, 0.3539 mmol) was added in a 250 mL round-bottomed flask and then dissolved with dichloromethane (20 mL), TFA (3.9 mL, 503.085 mmol) was then added with stirring, the obtained solution in the flask was stirred at room temperature to react. At the end of the reaction, the reaction solution was first concentrated at reduced pressure to remove dichloromethane. Then, methyl tert-butyl ether (150 mL) was added for precipitation, a powdery solid was obtained, and the filtering was carried out. The filter cake was washed with methyl tert-butyl ether (60 mL) and dissolved with a mixed solvent (100 mL) (20%methanol/dichloromethane); silica gel powder (50 mL) was added to the obtained solution, and the solution was then evaporated to dryness; the operations of dry sample loading, column chromatography, and elution with an eluent (1% ammonia water/2%-8% methanol/97%-91% dichloromethane) were carried out; the elution product was then collected, concentrated, evaporated to dryness, and dried. 1.2542 g of Product 44-164 was obtained with a yield of 26.27 %.

¹H-NMR (600 MHz, DMSO-d₆) δ 9.30 - 9.26 (m, 20H), 8.57 - 8.52 (m, 24H), 8.33 - 7.79 (m, 217H), 7.74 - 7.63 (m, 10H), 7.60 - 7.44 (m, 46H), 7.35 - 6.95 (m, 190H), 4.57 - 4.36 (m, 75H), 4.03-3.82 (m, 78H), 3.76 - 3.45 (m, 76H), 3.23 - 2.92 (m, 74H), 2.70 - 2.57 (m, 26H), 2.30 (s, 15H), 2.24 - 1.39 (m, 263H), 1.35 - 1.34 (m, 20H), 1.31- 1.13 (m, 71H), 0.86 - 0.81 (m, 144H).
44-170

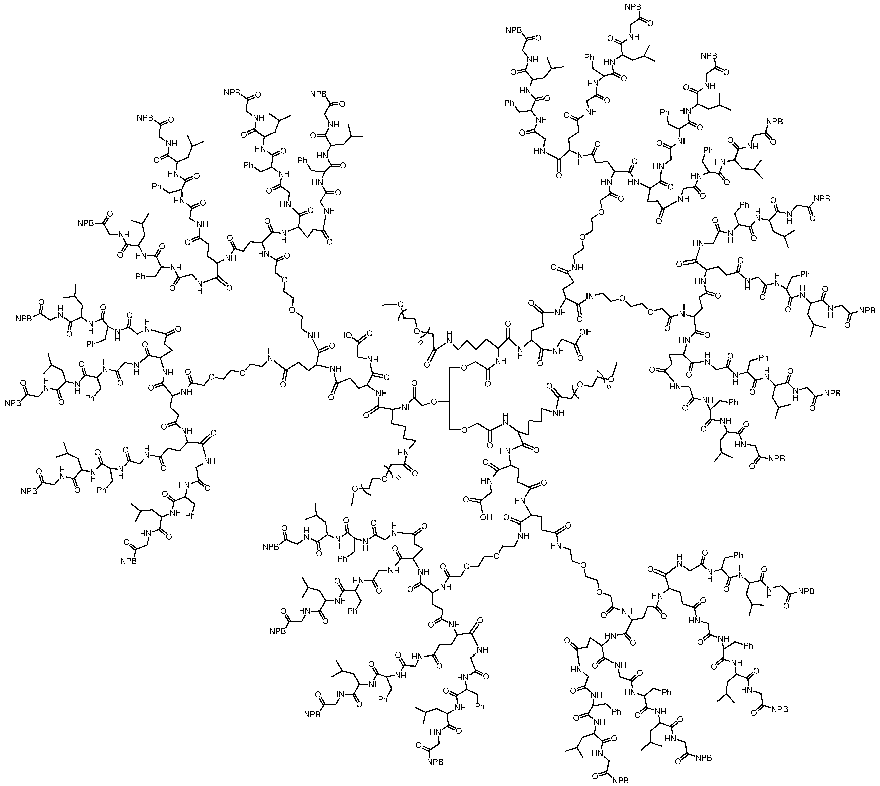

Product 44-164 (1.2542 g, 0.0596 mmol) and M-SCM-10K (2.8419 g, 0.2683 mmol, purchased from JenKem) were added to a 500 mL round-bottomed flask and then dissolved with DMF (10 mL), and the obtained solution in the flask was stirred at -5 °C for about 30 min; then, DIEA (1.2 mL, 7.1549 mmol) was slowly added dropwise, and at the end of the addition, the reaction solution was first stirred at -5 °C for 10 min to react, and then reacted at room temperature for one week. At the end of the reaction, n-hexane (150 mL) and methyl tert-butyl ether (20 mL) were added in the reaction solution for precipitation, the supernatant was discarded, n-hexane (150 mL) and methyl tert-butyl ether (30 mL) were added again to precipitate the lower oily product, and the process of precipitation was repeated three times to obtain an oily solid. The oily solid was dissolved with dichloromethane (10 mL), precipitated with methyl tert-butyl ether (60 mL) to obtain a powdery solid, and filtered to obtain a solid product; the filter cake was dissolved with a mixed solvent (100 mL) (20% methanol/dichloromethane); 50 mL of silica gel powder was added to the obtained solution, and the solution was then evaporated to dryness, the operations of dry sample loading, column chromatography and elution with an eluent (1% ammonia water: 4%-12% methanol: 95%-87% dichloromethane) were carried out; the elution product was then collected, concentrated, evaporated to dryness, and dried, thus obtaining 1.0155 g of Product 44-170 with a yield of 32.48%.

¹H-NMR (600 MHz, DMSO-d₆) δ 9.31 - 9.27 (m, 14H), 8.58 - 8.51 (m, 15H), 8.34 - 7.70 (m, 137H), 7.56 - 7.47 (m, 27H), 7.32 - 7.07 (m, 100H), 4.70 - 4.29 (m, 47H), 4.03 - 3.84 (m, 68H), 3.66 - 3.33 (m, 2880H), 3.28 - 3.22 (m, 12H), 3.06 - 2.02 (m, 23H), 2.89 - 2.72 (m, 40H), 2.62 - 2.60 (m, 17H), 2.19 - 2.12 (m, 24H), 2.01 - 1.85 (m, 40H), 1.79 - 1.75 (m, 41H), 1.50 - 1.44 (m, 68H), 1.37 - 1.06 (m, 504H), 0.85 - 0.81 (m, 144H).
44-174

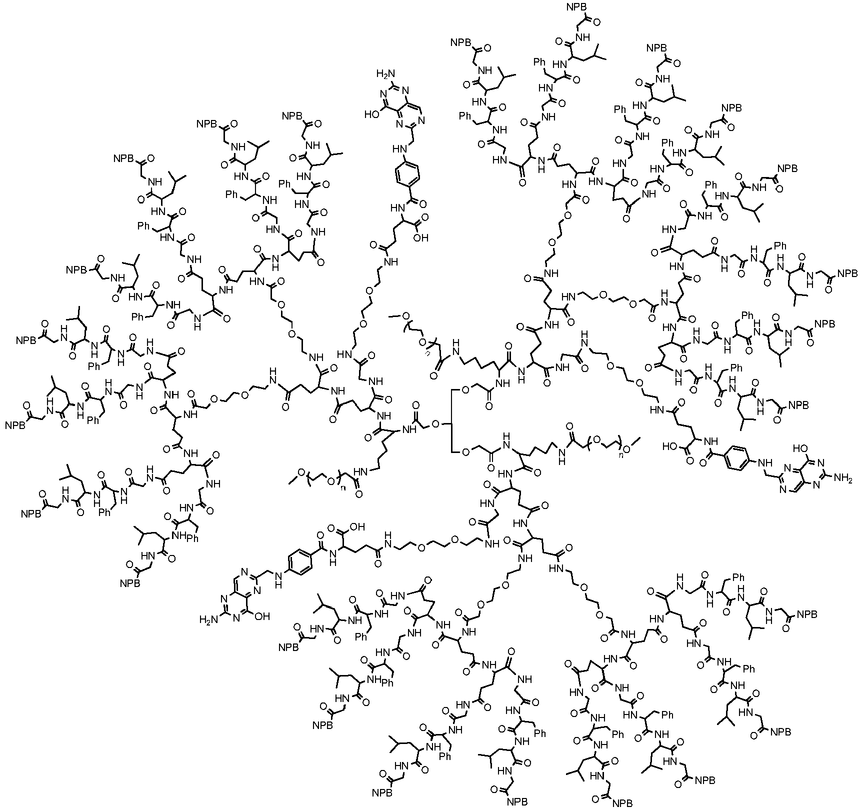

Product 44-170 (0.7714 g, 0.0147 mmol), Product 37-163 (0.1681 g, 0.2941 mmol), HBTU (0.3346 g, 0.8822 mmol) and HOBT (0.1192 g, 0.8822 mmol) were added to a 250 mL round-bottomed flask and then dissolved with DMF (50 mL); the obtained solution in the flask was stirred at -5 °C for about 30 min; then, DIEA (0.1 mL, 0.5516 mmol) was slowly added dropwise, and at the end of the addition, the reaction solution in the flask was stirred at -5 °C for 1 h to react, and finally stirred at room temperature. At the end of the reaction, n-hexane (150 mL) and methyl tert-butyl ether (30 mL) were first added to precipitate the reaction solution, the supernatant was discarded; n-hexane (150 mL) and methyl tert-butyl ether (30 mL) were added again to precipitate the lower oily product, and the process of precipitation was repeated three times to obtain an oily solid. The oily solid was dissolved with dichloromethane (10 mL), precipitated with methyl tert-butyl ether (150 mL) to obtain a powdery solid, and filtered to obtain a solid product; the obtained solid product was washed with methyl tert-butyl ether (60 mL), and dissolved with a mixed solvent (60 mL) (20% methanol/dichloromethane); silica gel powder (70 mL) was added to the obtained solution, and the solution was then evaporated to dryness; the operations of dry sample loading, column chromatography, and elution with an eluent (1% ammonia water/3%-8% methanol/96%-91% dichloromethane) were carried out; the elution product was then collected, concentrated, evaporated to dryness, and dried. 0.4393 g of Product 44-174 was obtained with a yield of 55.21%. ¹H-NMR (600 MHz, DMSO-d₆) δ 9.32 - 9.26 (m, 22H), 8.61 - 8.57 (m, 22H), 8.19 - 7.88 (m, 238H), 7.56 - 7.48 (m, 48H), 7.32 - 6.94 (m, 284H), 4.66 - 4.32 (m, 78H), 4.27 - 3.78 (m, 122H), 3.65 - 3.61 (m, 37H), 3.59 - 3.43 (m, 2802H), 3.25 - 3.01 (m, 88H), 2.92 - 2.87 (m, 12H), 2.80 - 2.74 (m, 74H), 2.65 - 2.61 (m, 39H), 2.39 - 2.37 (m, 7H), 2.12 - 1.41 (m, 280H), 0.86 - 0.81 (m, 144H).

### Synthesis of Compound 49-199

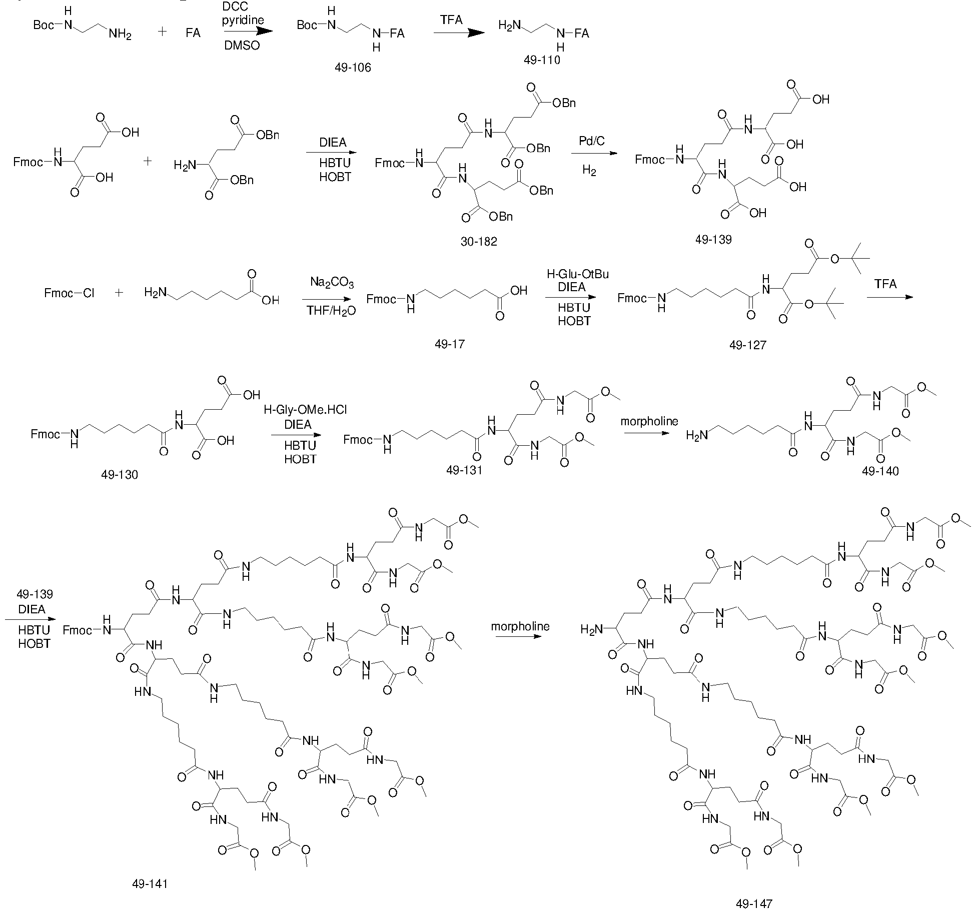

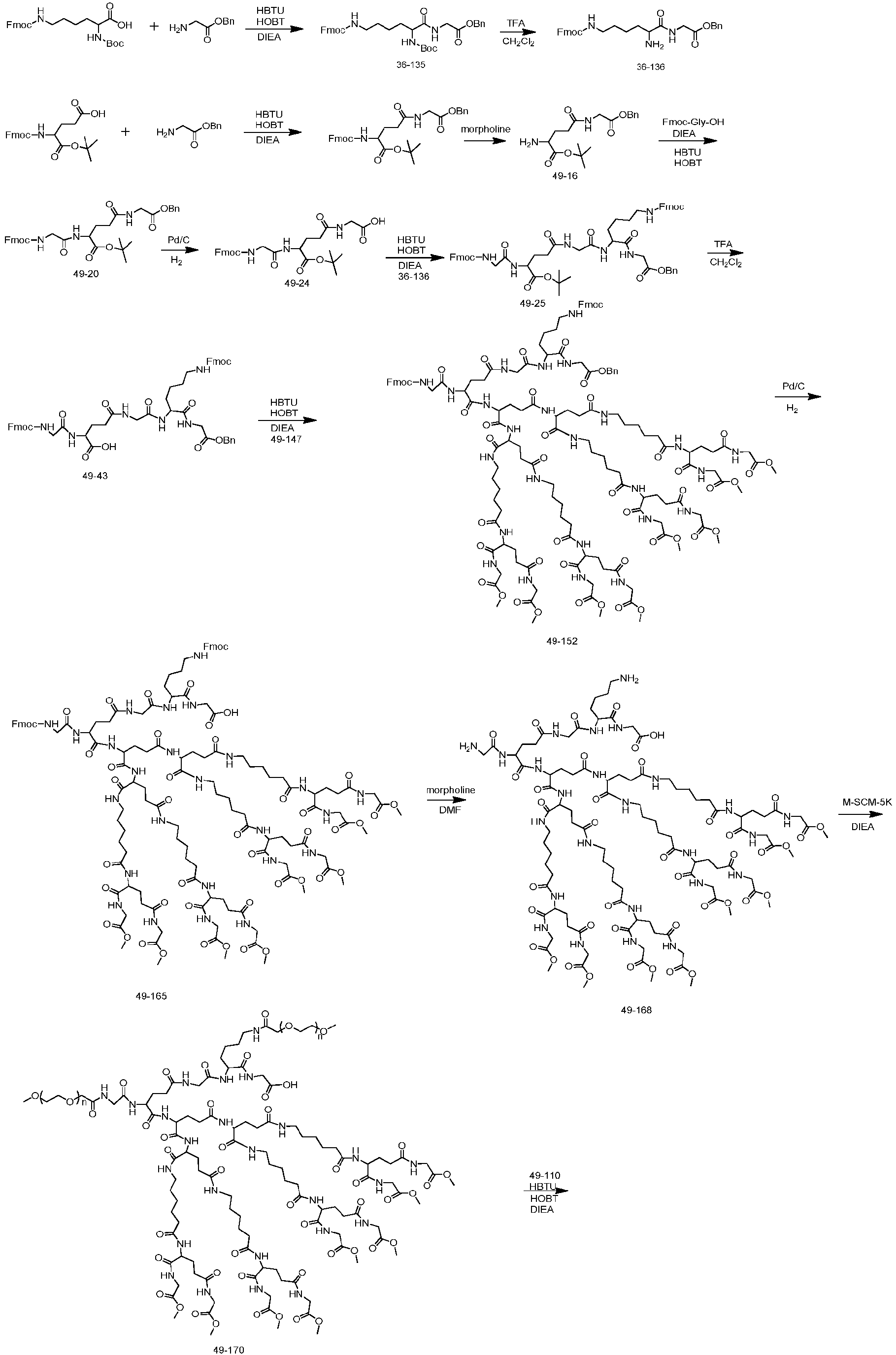

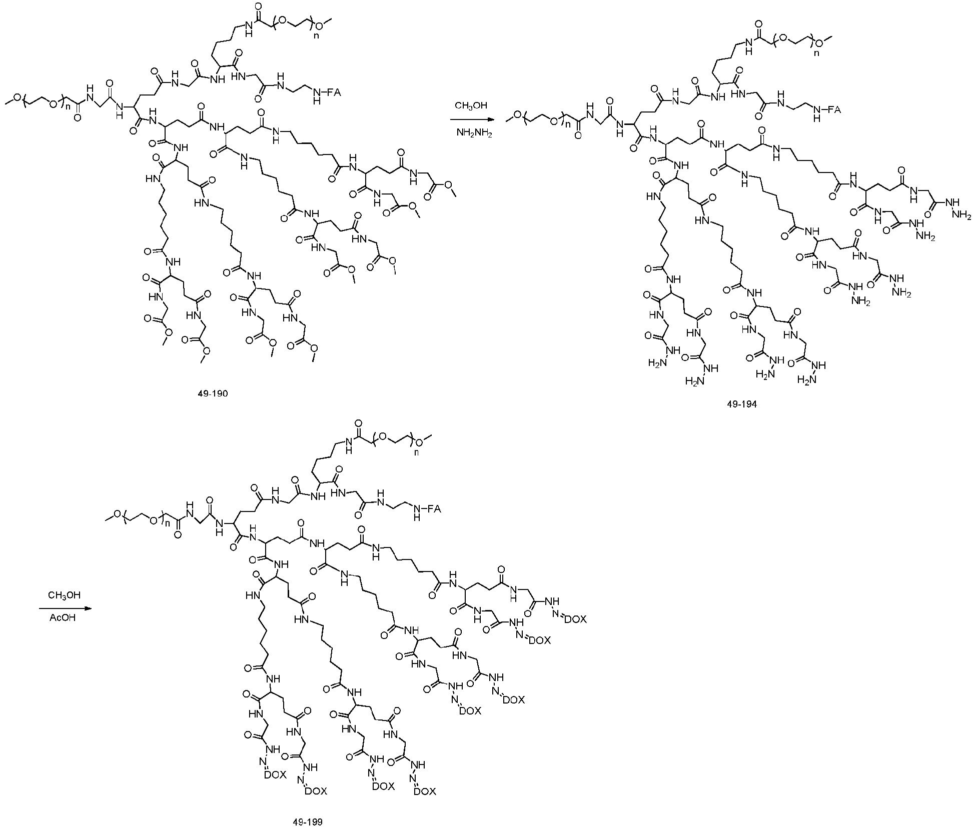

49-17

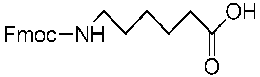

6-aminocaproic acid (4.61 g, 35.1407 mmol) was weighed and added in a 1 L flask and then dissolved completely with 150 mL of a mixed solution THF:H₂O=1:1, and the mixed solution was stirred at 0 °C; sodium carbonate solid (7.45 g, 70.2814 mmol) was added, the obtained solution was dissolved by ultrasonic, and then stirred at 0 °C for 30 min; after that, Fmoc-Cl (10 g, 38.6548 mmol) was dissolved in 30 mL of THF, and then slowly added dropwise to the reaction solution; at the end of the addition, the reaction solution was taken out and stirred at room temperature to react. At the end of the reaction, 50 g of citric acid was weighed and dissolved in 450 mL of deionized water, the reaction solution was added to adjust the pH to 3, and then transferred to a 1 L separatory funnel; extraction with EA (300 mL × 3) was carried out, the organic phases were collected, then concentrated and evaporated to dryness; the solid product was then dissolved with a mixed solution of methanol/dichloromethane (1:4) (100 mL); silica gel powder (50 mL) was added to the obtained solution, and the solution was evaporated to dryness to obtain a powdery solid, the operations of dry sample loading, column chromatography, and elution with a mixed solution of 2% methanol/dichloromethane were carried out; the elution product was collected, concentrated and dried in a vacuum oven, thus obtaining the product (7.7 g, 86.51 %).
49-106

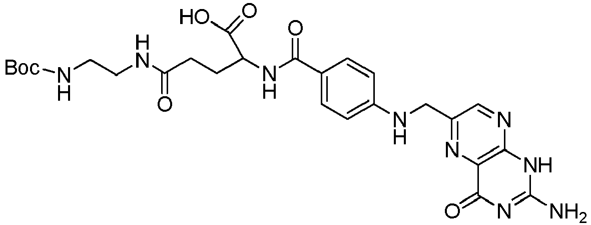

FA (2.2 g, 5.0 mmol) was weighed and added in a 1 L flask and dissolved completely with DMSO (80 mL); Boc-ethylenediamine (0.78 g, 5.0 mmol), DCC (1.68 g, 8.14 mmol), and pyridine (32 mL) were added to the flask; the obtained solution was stirred overnight at room temperature. At the end of the reaction, the reaction solution was filtered, and the filtrate was collected and placed at 0 °C; methyl tert-butyl ether was slowly added dropwise to the filtrate and stirred quickly until a large number of yellow solids appeared. The solids were filtered, and the filter cake was washed twice with methyl tert-butyl ether (60 mL), and then collected and dried, thus obtaining the product (2.75 g, 94.48%).
49-110

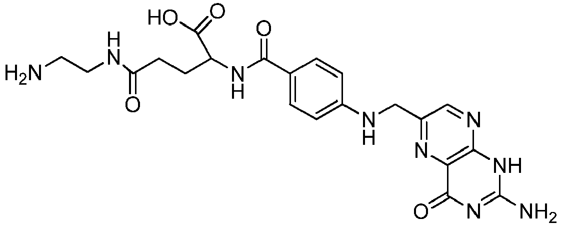

TFA (6.99 ml, 94.166 mmol) was added in a flask with Product 49-106 (2.75 g, 4.7083 mmol), and the obtained solution was stirred overnight at room temperature to react. At the end of the reaction, the reaction solution was concentrated at reduced pressure, and dissolved with a small amount of anhydrous DMF; then, pyridine was slowly added dropwise at 0 °C; a small amount of methyl tert-butyl ether was added to separate out a yellow solid product, and the solid product was filtered; the filter cake was washed twice with methyl tert-butyl ether (60 mL), and then collected and dried, thus obtaining the product (2.18 g, 96.03%).
49-15

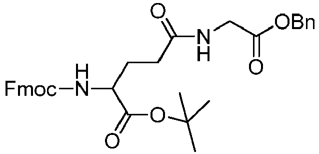

Fmoc-Glu(OtBu)-OH (3 g, 7.0510 mmol), HBTU (4.01 g, 10.5765 mmol) and HOBT (1.42 g, 10.5765 mmol) were weighed and added in a flask with H-Gly-OBn (2.38 g, 7.0510 mmol, purchased from Accela), and then dissolved with an appropriate amount of DMF, and the obtained solution was placed at -5 °C; then, DIEA (5.2 mL, 31.7295 mmol) was slowly added dropwise; after addition and reaction for 30 min, the reaction solution was taken out, and the obtained solution was stirred overnight at room temperature to react. At the end of the reaction, extraction with a saturated NaCl solution (200 mL) and EA (200 mL) was carried out, the reaction solution was stood still to be layered, and the organic phases were collected; the aqueous phase was then washed with EA (200 mL × 3), the obtained organic phases were combined, evaporated into a solid, and dried in a vacuum oven, thus obtaining the product (7.5 g being extra-quota product).
49-16

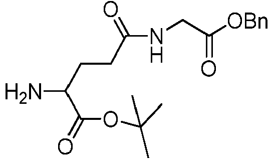

Product 49-15 (4.03 g, 7.0510 mmol) in a flask was dissolved completely with DMF by ultrasonic vibration; then morpholine (6.14 mL, 70.510 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (5.6 g being extra-quota product).
49-20

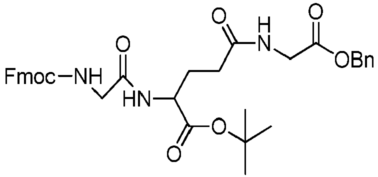

Fmoc-Gly-OH (2.09 g, 7.0510 mmol, purchased from Accela), HBTU (4.01 g, 10.5765 mmol) and HOBT (1.42 g, 10.5765 mmol) were weighed and added in a flask with Product 49-16 (2.47 g, 7.0510 mmol) and then dissolved with an appropriate amount of DMF, and the obtained solution was placed at -5 °C; then, DIEA (5.2 mL, 31.7295 mmol) was slowly added dropwise for titration, after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and the solid product was dissolved with a mixed solution of methanol/dichloromethane (1:4) (100 mL); silica gel powder (100 mL) was added to the obtained solution, and the solution was then evaporated to dryness to obtain a powdery solid; the operations of dry sample loading, column chromatography, and elution with a mixed solution (1% methanol/dichloromethane) were carried out; the elution product was then collected, concentrated, and dried in a vacuum oven, thus obtaining the product (2.64 g, 59.45 %).
49-24

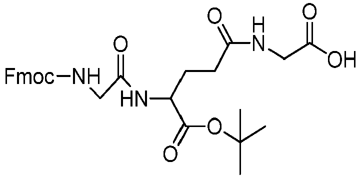

Product 49-20 (2 g, 3.1761 mmol) was added in a hydrogenation reactor and then 10% Pd/C (0.05 g) was added, and the mixture was dissolved with DMF (20 mL); hydrogen was introduced to a pressure of 300 Psi in the reactor, and the reaction solution was stirred overnight at room temperature to react. At the end of the reaction, the reaction solution was filtered with diatomaceous earth; the filter cake was washed three times with DMF (15 mL × 3); the elution solution was added in a 250 mL round-bottomed flask, and the product was obtained, as the raw material for the next step.
36-135

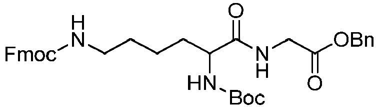

Fmoc-Lys(Boc)-OH (2 g, 4.2686 mmol, purchased from Accela), H-Gly-OBzl.TosOH (1.44 g, 4.2686 mmol, purchased from Ark Pharm), HBTU (2.4 g, 6.4029 mmol) and HOBT (0.9 g, 6.4029 mmol) were added in a 500 mL flask, and the obtained solution was stirred for about 20 min at - 5 °C; then, DIEA (3.2 mL, 19.2086 mmol) was slowly added dropwise; the reaction continued at -5 °C for 1 h, and the reaction solution was stirred at room temperature overnight to react. At the end of the reaction, the reaction solution was transferred to a 1 L separatory funnel and extracted with a saturated NaCl solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined, washed with a saturated sodium chloride solution (100 ml × 2), and evaporated to dryness, thus obtaining 4.04 g of Product 36-135.
36-136

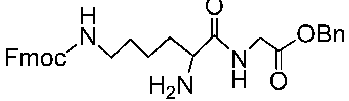

Product 36-135 (11.8271 g, 19.2086 mmol) was added in a 500 mL round-bottomed flask and then dissolved with dichloromethane (40 mL), TFA (21.4 mL, 288.129 mmol) was then added with stirring, and the obtained solution in the flask was stirred at room temperature overnight to react. At the end of the reaction, the reaction solution was first concentrated and evaporated to remove dichloromethane; methyl tert-butyl ether (150 mL) was added to precipitate the reaction solution, and a powdery solid was obtained; the obtained solid was filtered, and the filter cake was washed with methyl tert-butyl ether (60 mL), and finally dried in an oven, thus obtaining 9.9040 g of the product.
49-25

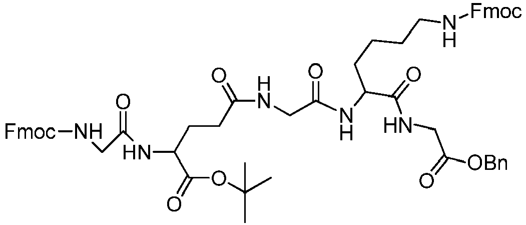

Product 36-136 (1.80 g, 3.4937 mmol), HBTU (1.80 g, 4.7642 mmol) and HOBT (0.64 g, 4.7642 mmol) were weighed and added in a flask with Product 49-24 (1.71 g, 3.1761 mmol) and then dissolved with an appropriate amount of DMF, and the obtained solution was placed at -5 °C; then, DIEA (12.36 mL, 14.2925 mmol) was slowly added dropwise for titration, after addition and reaction for 30 min, the reaction solution was taken out, and stirred overnight at room temperature to react. At the end of the reaction, saturated NaCl (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and the solid product was dissolved with a mixed solution of methanol/dichloromethane (1:4) (100 mL); silica gel powder (100 mL) was added, and the obtained solution was evaporated to dryness to obtain a powdery solid; the operations of dry sample loading, column chromatography, and elution with 3% methanol/dichloromethane were carried out; the elution product was collected, concentrated and dried in a vacuum oven, thus obtaining the product (1.5 g, 45.59%).

¹H-NMR (600 MHz, DMSO-*d*₆) δ 8.40 - 8.38 (m, 1H), 8.16 - 8.13 (m, 1H), 8.09 - 7.85 (m, 7H), 7.76 - 7.60 (m, 4H), 7.52 - 7.49 (m, 1H), 7.44 - 7.29 (m, 13H), 7.25 - 7.21 (m, 1H), 5.11 (s, 2H), 4.36 - 4.09 (m, 8H), 3.93 - 3.85 (m, 2H), 3.80 - 3.57 (m, 5H), 2.99 - 2.71 (m, 3H), 2.21 - 2.16 (m, 2H), 2.01 - 1.88 (m, 1H), 1.84 - 1.72 (m, 2H), 1.68 - 1.63 (m, 1H), 1.45 - 1.36 (m, 9H), 1.28 - 1.22 (m, 3H).
49-43

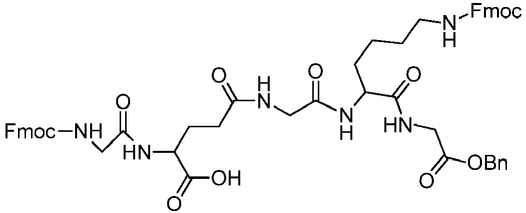

Dichloromethane was added in a flask with Product 49-25 (0.6 g, 0.5785 mmol) and dissolved completely by ultrasonic vibration; TFA (0.43 mL, 5.785 mmol) was added, and the reaction solution was stirred at room temperature overnight to react. At the end of the reaction, dichloromethane and most of the TFA were evaporated to dryness; saturated NaCl (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (3.95 g being extra-quota product).
49-127

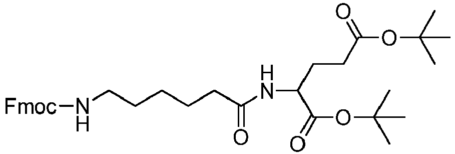

Product 49-17 (4 g, 11.32 mmol), HBTU (6.44 g, 16.98 mmol) and HOBT (2.29 g, 16.98 mmol) were weighed and added in a flask with H-Glu-(OtBu)₂ (3.35 g, 11.32 mmol, purchased from Leyan) and then dissolved with an appropriate amount of DMF, and the obtained solution was placed at -5 °C; then, DIEA (8.41 mL, 50.93 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution; extraction was carried out and the reaction solution was stood still to be layered, and the organic phases were collected; the aqueous phase was then washed with EA (200 mL × 3); the obtained organic phases were combined, collected, concentrated and dried in a vacuum oven, thus obtaining the product (8.9 g being extra-quota product).
49-130

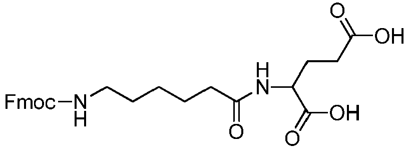

Dichloromethane was added in a flask with Product 49-127 (6.73 g, 11.32 mmol) and dissolved completely by ultrasonic vibration flask; TFA (8.40 mL, 113.2 mmol) was added, and the reaction solution was stirred at room temperature overnight to react. At the end of the reaction, n-hexane (25 mL) and methyl tert-butyl ether (200 mL) were first added for precipitation, and the supernatant was discarded; n-hexane and methyl tert-butyl ether were added again for precipitation, and such operations were repeated three times; then suction filtering was carried out, and the solid product was collected, and dried in a vacuum oven, thus obtaining the product (7.6 g being extra-quota product).
49-131

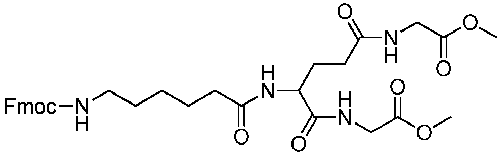

Glycine methyl ester hydrochloride (3.12 g, 24.90 mmol, purchased from damas-beta), HBTU (12.88 g, 33.96 mmol) and HOBT (4.59 g, 33.96 mmol) were weighed and added in a flask with Product 49-130 (5.45 g, 11.32 mmol), and then dissolved with an appropriate amount of DMF, and the obtained solution was placed at -5 °C; then, DIEA (19.48 mL, 117.8361 mmol) was slowly added dropwise; after addition and reaction for 30 min, the reaction solution was taken out, and the obtained solution was stirred overnight at room temperature to react. At the end of the reaction, the reaction solution was precipitated with n-hexane (25 mL) and methyl tert-butyl ether (200 mL) and then the supernatant was discarded. n-hexane and methyl tert-butyl ether was added again for further precipitation, and the operation of precipitation was repeated three times; suction filtering was then carried out to obtain a solid product; the obtained solid product was dissolved with a mixed solvent (dichloromethane and methanol), the operations of dry sample loading, column chromatography, and elution with a mixed solution (6%-10% methanol/dichloromethane) were carried out; the elution product was collected, concentrated and dried in a vacuum oven, thus obtaining the product (5.2 g, 73.55%).
49-140

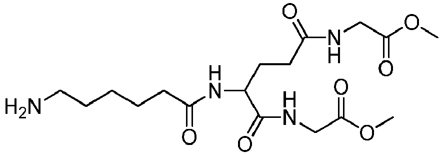

Product 49-131 (5.2 g, 8.33 mmol) in a flask was dissolved completely with DMF by ultrasonic vibration; morpholine (7.26 mL, 83.33 mmol) was added, and the reaction solution was stirred at room temperature overnight for 2 h to react. At the end of the reaction, the reaction solution was precipitated with n-hexane (25 mL) and methyl tert-butyl ether (200 mL) and then the supernatant was discarded; n-hexane and methyl tert-butyl ether was added again for further precipitation, and the operation of precipitation was repeated three times; suction filtering was then carried out to obtain a solid product; the obtained solid product was collected and dried in a vacuum oven. The product (5.1 g being extra-quota product) was obtained.
30-182

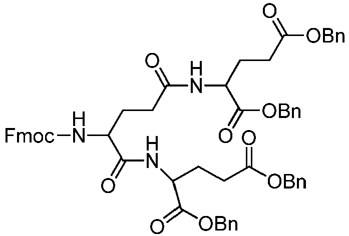

Fmoc-Glu-OH (2.5 g, 7.1551 mmol, purchased from Aladdin), HBTU (8.1405 g, 21.4654 mmol) and HOBT (2.9 g, 21.4654 mmol) were weighed and added in a flask with H-Glu-(OBn)₂ (7.86 g, 15.7412 mmol, purchased from Ark Pharm), and then dissolved with an appropriate amount of DMF, and the obtained solution was placed at -5 °C; then, DIEA (10.64 mL, 64.3961 mmol) was slowly added dropwise for titration; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, and evaporated into a solid; the solid product was dissolved with a mixed solution of methanol/dichloromethane (1:4); silica gel powder (100 mL) was added to the obtained solution, and the solution was then evaporated to dryness to obtain a powdery solid; the operations of dry sample loading, column chromatography, and elution with a mixed solution (2%-4% methanol/dichloromethane) were carried out; the elution product was collected, concentrated and dried in a vacuum oven, thus obtaining the product (6.6 g, 83.54 %).
49-139

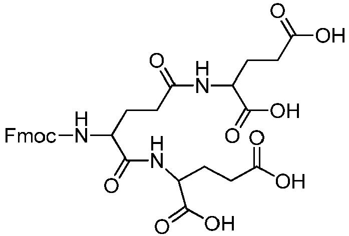

Product 30-182 (6.6 g, 5.9945 mmol) was added in a hydrogenation reactor and then 10% Pd/C (0.05 g) was added, and the mixture was dissolved with DMF (20 mL); hydrogen was introduced to a pressure of 300 Psi in the reactor, and the reaction solution was stirred overnight at room temperature to react. At the end of the reaction, the reaction solution was filtered with diatomaceous earth; the filter cake was washed three times with DMF (15 mL × 3); the elution solution was added in a 250 mL round-bottomed flask, and the product was obtained, as the raw material for the next step.
49-141

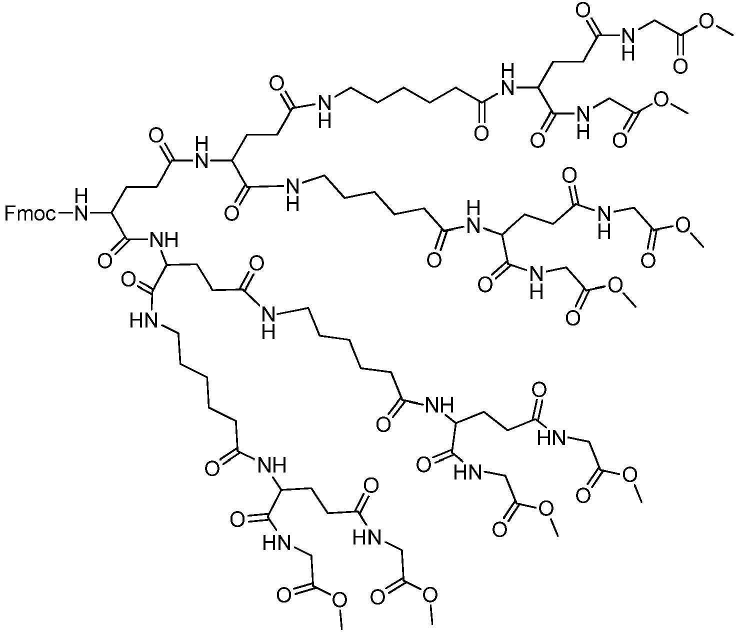

Product 49-140 (5 g, 11.5207 mmol), HBTU (5.95 g, 15.7101 mmol) and HOBT (2.12 g, 15.7101 mmol) were weighed and added in a flask with Product 49-139 (1.64 g, 2.6183 mmol), and then dissolved with an appropriate amount of DMF, and the obtained solution was placed at -5 °C; then, DIEA (7.79 mL, 47.1303 mmol) was slowly added dropwise for titration; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, and evaporated into a solid; the solid product was then dissolved with a mixed solution of methanol/dichloromethane (1:4); silica gel powder (100 mL) was added to the obtained solution, and the solution was then evaporated to dryness to obtain a powdery solid; the operations of dry sample loading, column chromatography, and elution with a mixed solution (2%-4% methanol/dichloromethane) were carried out; the elution product was collected, concentrated and dried in a vacuum oven, thus obtaining the product (3.2 g, 53.33 %).
49-147

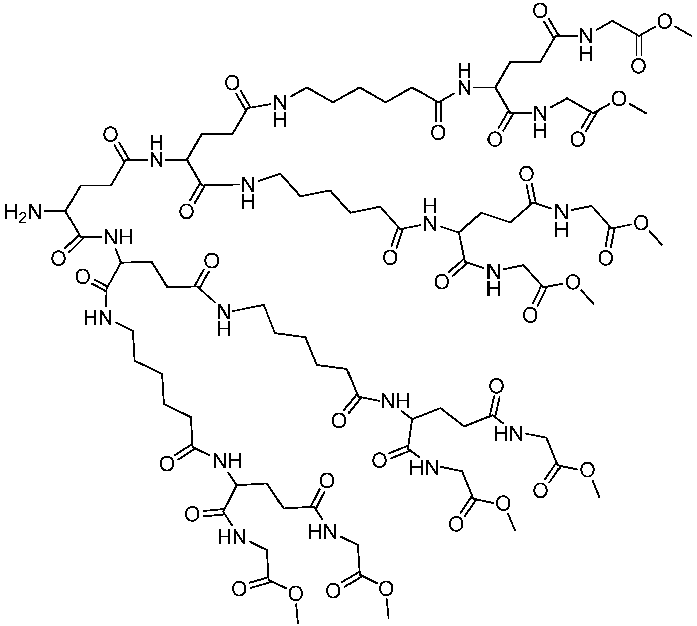

Product 49-141 (3.2 g, 1.3968 mmol) in a flask was dissolved completely with DMF by ultrasonic vibration; morpholine (1.21 mL, 13.968 mmol) was added, and the reaction solution was stirred at room temperature for 2 h to react. At the end of the reaction, a saturated NaCl solution (200 mL) and EA (200 mL) were added to the reaction solution; extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (5.6 g being extra-quota product).
49-152

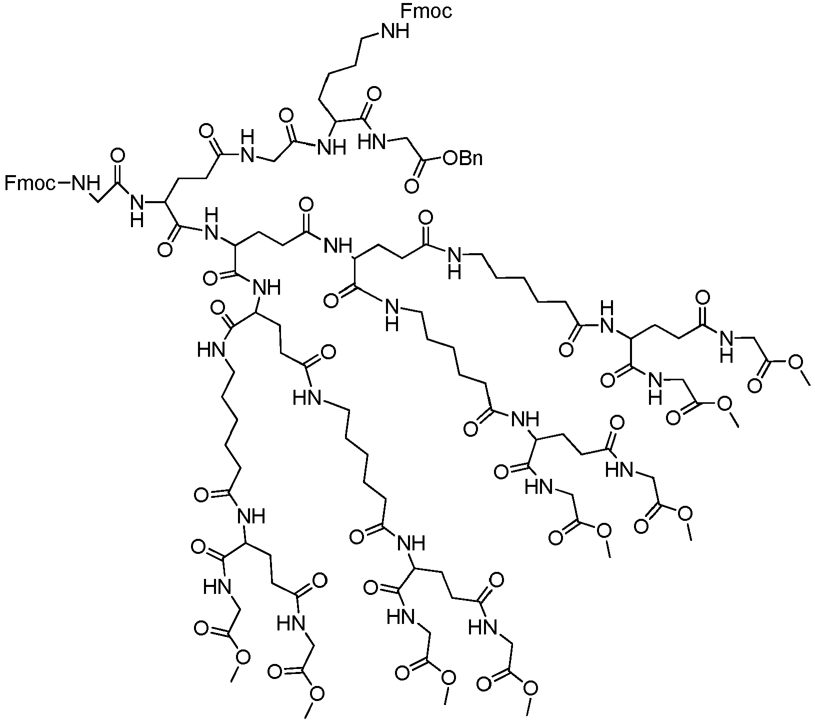

Product 49-43 (1.64 g, 1.6761 mmol), HBTU (0.79 g, 2.0952 mmol) and HOBT (0.28 g, 2.0952 mmol) were weighed and added in a flask with Product 49-147 (2.89 g, 1.3968 mmol) and then dissolved with an appropriate amount of DMF, and the obtained solution was placed at -5 °C; then, DIEA (1.04 mL, 6.2856 mmol) was slowly added dropwise for titration; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, and evaporated into a solid; the solid product was then dissolved with a mixed solution of methanol/dichloromethane (1:4); silica gel powder (100 mL) was added to the obtained solution, and the solution was then evaporated to dryness to obtain a powdery solid; the operations of dry sample loading, column chromatography, and elution with a mixed solution (3%-7% methanol/dichloromethane) were carried out; the elution product was collected, concentrated and dried in a vacuum oven. The product (2.8 g, 66.19 %) was obtained.
49-165

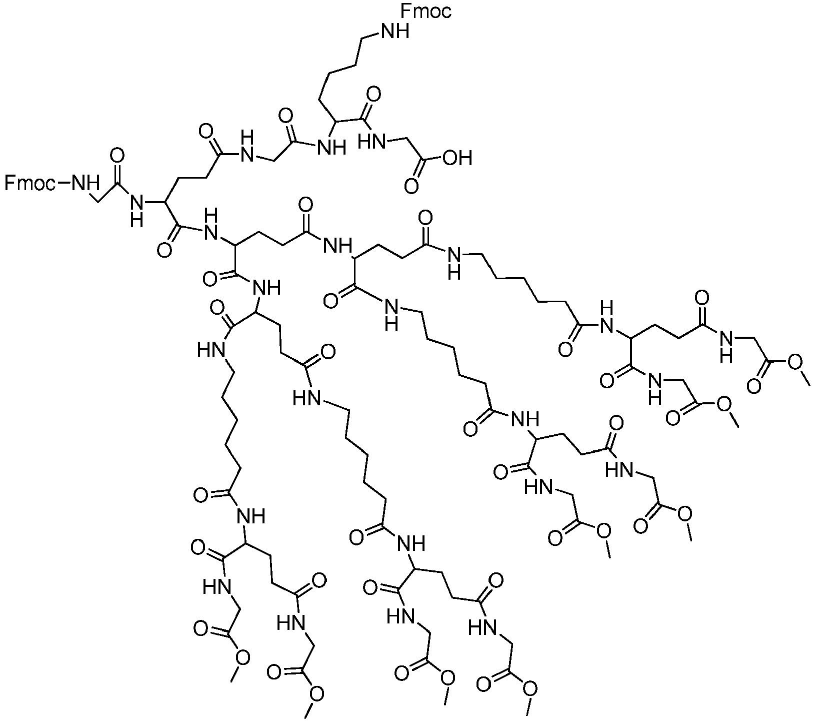

Product 49-152 (2.8 g, 0.9232 mmol) was added in a hydrogenation reactor and then 10% Pd/C (0.05 g) was added, and the mixture was dissolved with DMF (20 mL); hydrogen was introduced to a pressure of 300 Psi in the reactor, and the reaction solution was stirred overnight at room temperature to react. At the end of the reaction, the reaction solution was filtered with diatomaceous earth; the filter cake was washed three times with DMF (15 mL × 3); the elution solution was added in a 250 mL round-bottomed flask, and the product was obtained, as the raw material for the next step.
49-168

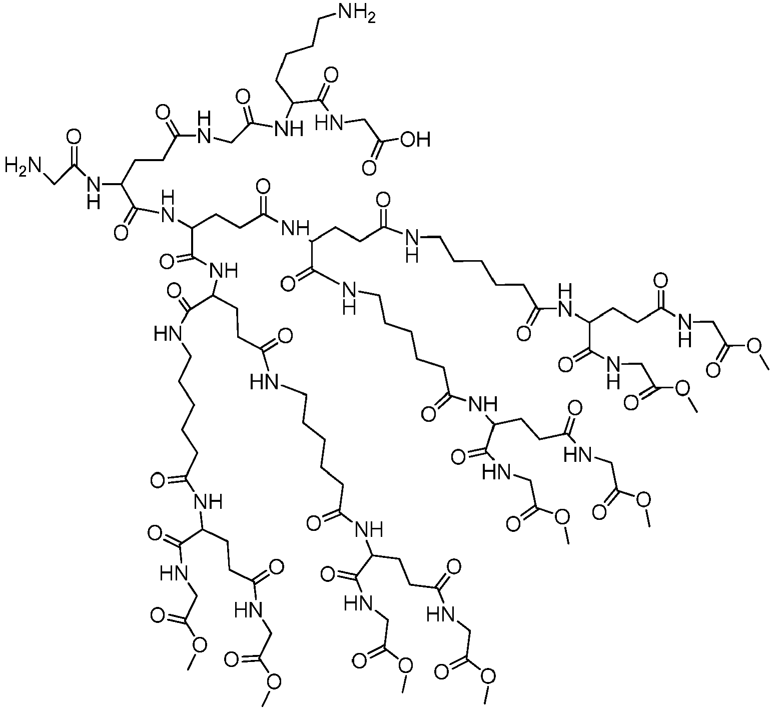

Product 49-165 (2.60 g, 0.9232 mmol) in a flask was dissolved completely with DMF by ultrasonic vibration; piperidine (1.57 mL, 18.464 mmol) was added, and the reaction solution was stirred at room temperature for 2 h to react. At the end of the reaction, an overdose of DIEA and methylbenzene were added, and the reaction solution was concentrated at reduced pressure; DMF solution of the product was obtained, as the raw material for the next step.
49-170

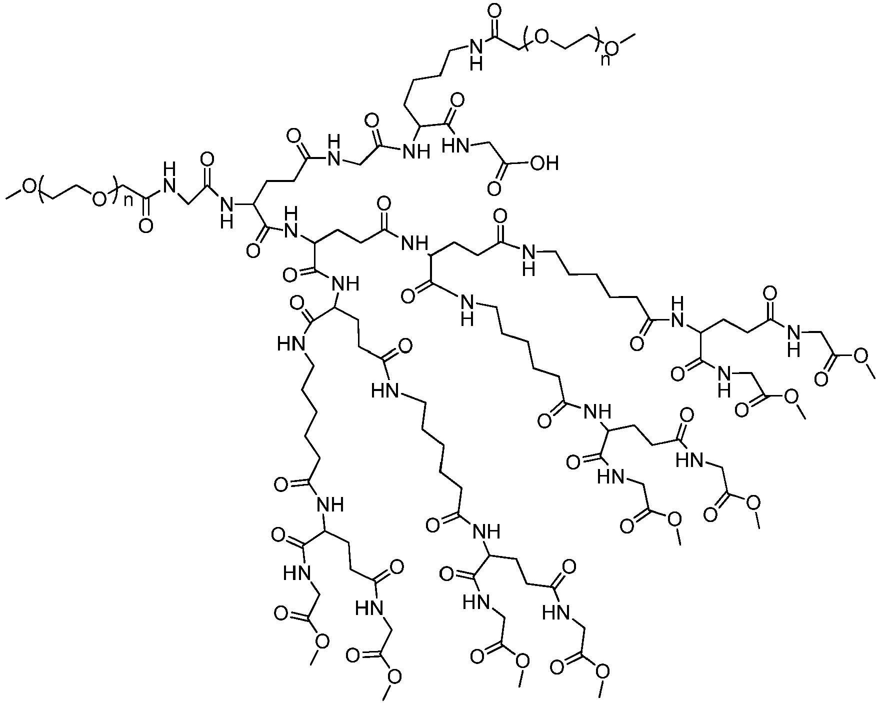

Product 49-168 (0.31 g, 0.1311 mmol) was added in a 250 mL flask and then dissolved with DMF (20 mL), and DIEA (1.38 mL, 8.3904 mmol) was added dropwise slowly at -5 °C; after addition and reaction for 30 min, M-SCM-5K (1.44 g, 0.2884 mmol, purchased from JenKem) was added and dissolved by ultrasonic vibration, and the obtained solution was slowly stirred at room temperature to react for one week in the dark. After the reaction was completed, n-hexane (25 mL) and methyl tert-butyl ether (200 mL) were first added for precipitation, and the supernatant was discarded; n-hexane and methyl tert-butyl ether were added again for precipitation, and such operations were repeated three times; then suction filtering was carried out to obtain a solid product, and the obtained solid product was dissolved with a mixed solvent (dichloromethane and methanol); the operations of dry sample loading, column chromatography, and elution with a mixed solution (1% ammonia water/6-10% methanol/dichloromethane) were carried out; the elution product was collected, concentrated and dried in a vacuum oven. The product (0.81 g, 48.21%) was obtained.
49-190

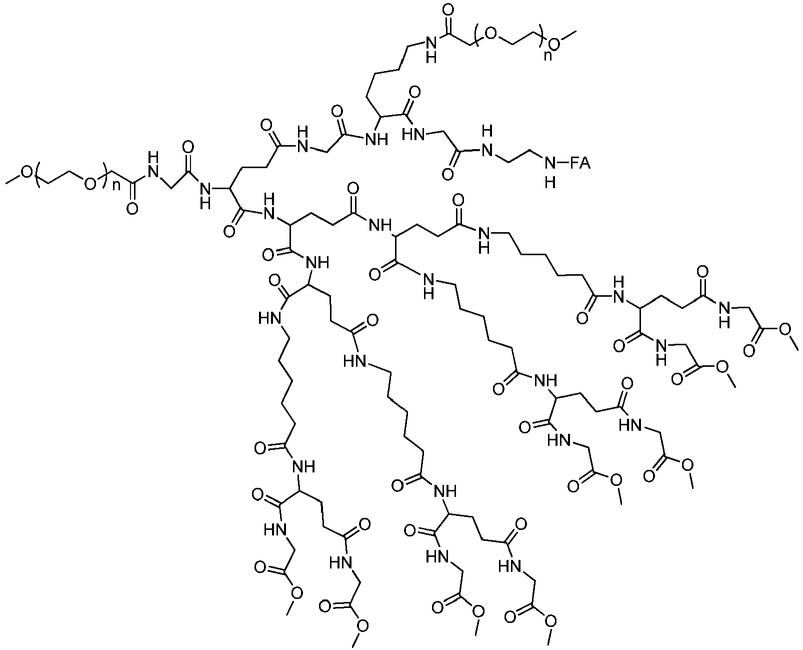

Product 49-170 (0.81 g, 0.0630 mmol), HBTU (0.04 g, 0.0945 mmol) and HOBT (0.01 g, 0.0945 mmol) were weighed and added in a flask with Product 49-110 (0.06 g, 0.1260 mmol) and then dissolved with an appropriate amount of DMF, and the mixed solution was placed at -5 °C; DIEA (1.04 mL, 6.2856 mmol) was slowly added dropwise; after addition and reaction for 30 min, the reaction solution was taken out, and the obtained solution was stirred overnight at room temperature to react. After the reaction was completed, n-hexane (25 mL) and methyl tert-butyl ether (200 mL) were first added for precipitation, the supernatant was discarded; n-hexane and methyl tert-butyl ether were added again for precipitation; such operations were repeated three times, and then suction filtering was carried out to obtain a solid product, and the obtained solid product was dissolved with dichloromethane and methanol; the operations of dry sample loading, column chromatography, and elution with 5-12% methanol /dichloromethane were carried out; the elution product was then collected, concentrated and dried, thus obtaining the product (0.66 g, 78.57%).
49-194

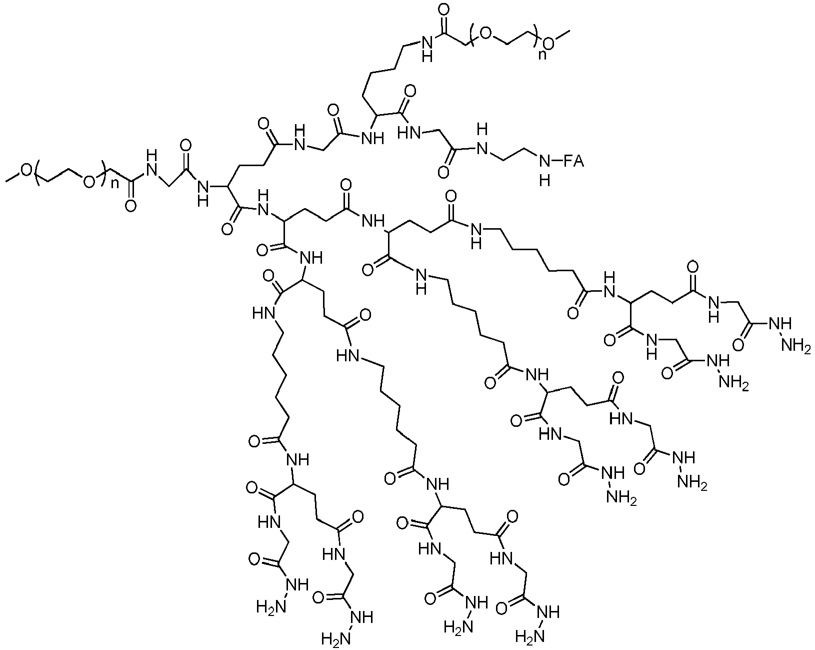

Product 49-190 (0.66 g, 0.0495 mmol) was added in a dry 250 mL round-bottomed flask, and then dissolved completely with hydrazine hydrate (0.003 g, 0.7452 mmol) and methanol, and the obtained solution was stirred at room temperature to react overnight. After the reaction was completed, n-hexane (25 mL) and methyl tert-butyl ether (200 mL) were first added for precipitation, and the supernatant was discarded; n-hexane and methyl tert-butyl ether were added again for precipitation; such operations were repeated three times, and the solid was collected, concentrated, and dried in a vacuum oven, thus obtaining the product (1.23 g being extra-quota product).
49-199

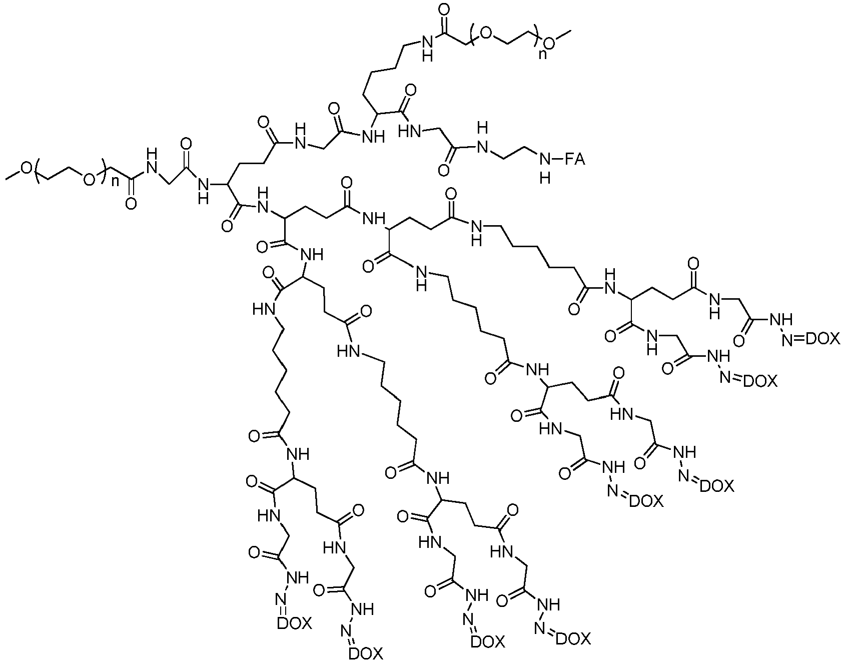

Product 49-194 (0.66 g, 0.0495 mmol) was added in a dry 500 mL round-bottomed flask and then dissolved with anhydrous methanol; glacial acetic acid (0.28 mL, 4.968 mmol) and doxorubicin (DOX, 0.43 g, 0.7452 mmol) were added in the flask, and the reaction solution was stirred at room temperature overnight to react. After the reaction was completed, n-hexane (25 mL) and methyl tert-butyl ether (200 mL) were first added for precipitation, the supernatant was discarded; n-hexane and methyl tert-butyl ether were added again for precipitation; such operations were repeated three times, and the filtering was then carried out to obtain a solid product, and the obtained solid product was dissolved with dichloromethane and methanol, the operations of dry sample loading, column chromatography, and gradient elution with 5-12% methanol /dichloromethane were carried out; the elution product was then collected, concentrated and dried, thus obtaining the product (0.52 g, 49.05 %).

¹H-NMR (600 MHz, DMSO-*d*₆) δ 8.02 - 7.93 (m, 16H), 7.84 - 7.74 (m, 15H), 7.71 - 7.63 (m, 13H), 7.51 - 7.30 (m, 25H), 5.53 - 5.39 (m, 22H), 5.01 - 4.78 (m, 29H), 4.59 - 4.52 (m, 13H), 4.17 - 3.98 (m, 59H), 3.64 - 3.61 (m, 39H), 3.51 (s, 1254H), 3.24 (s, 36H), 2.64 - 2.62 (m, 13H), 2.39 - 2.37 (m, 12H), 1.57- 1.55(m, 29H), 1.50 - 1.39 (m, 58H), 1.25 - 1.10 (m, 35H).

### Synthesis of Compound 47-122

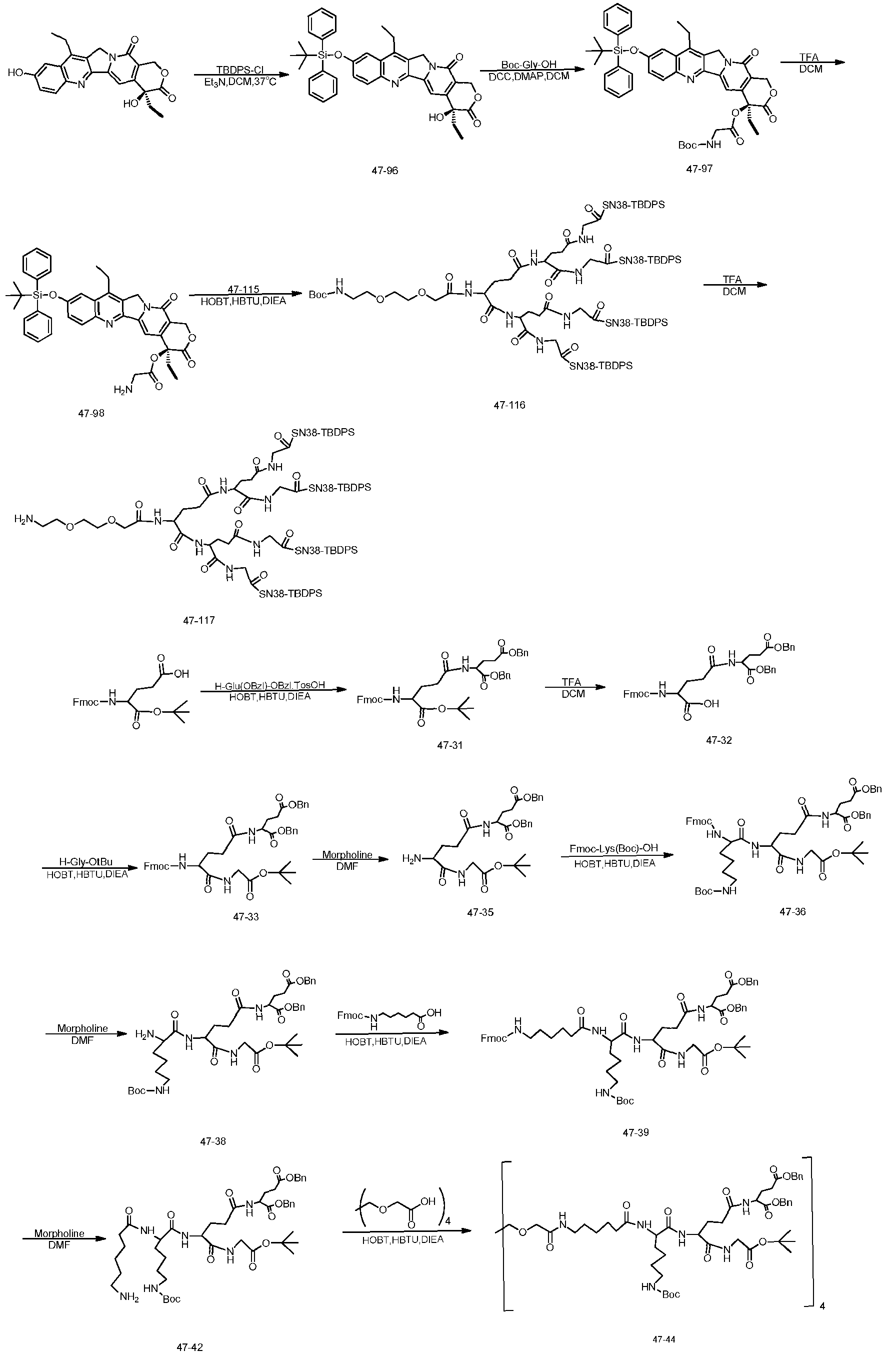

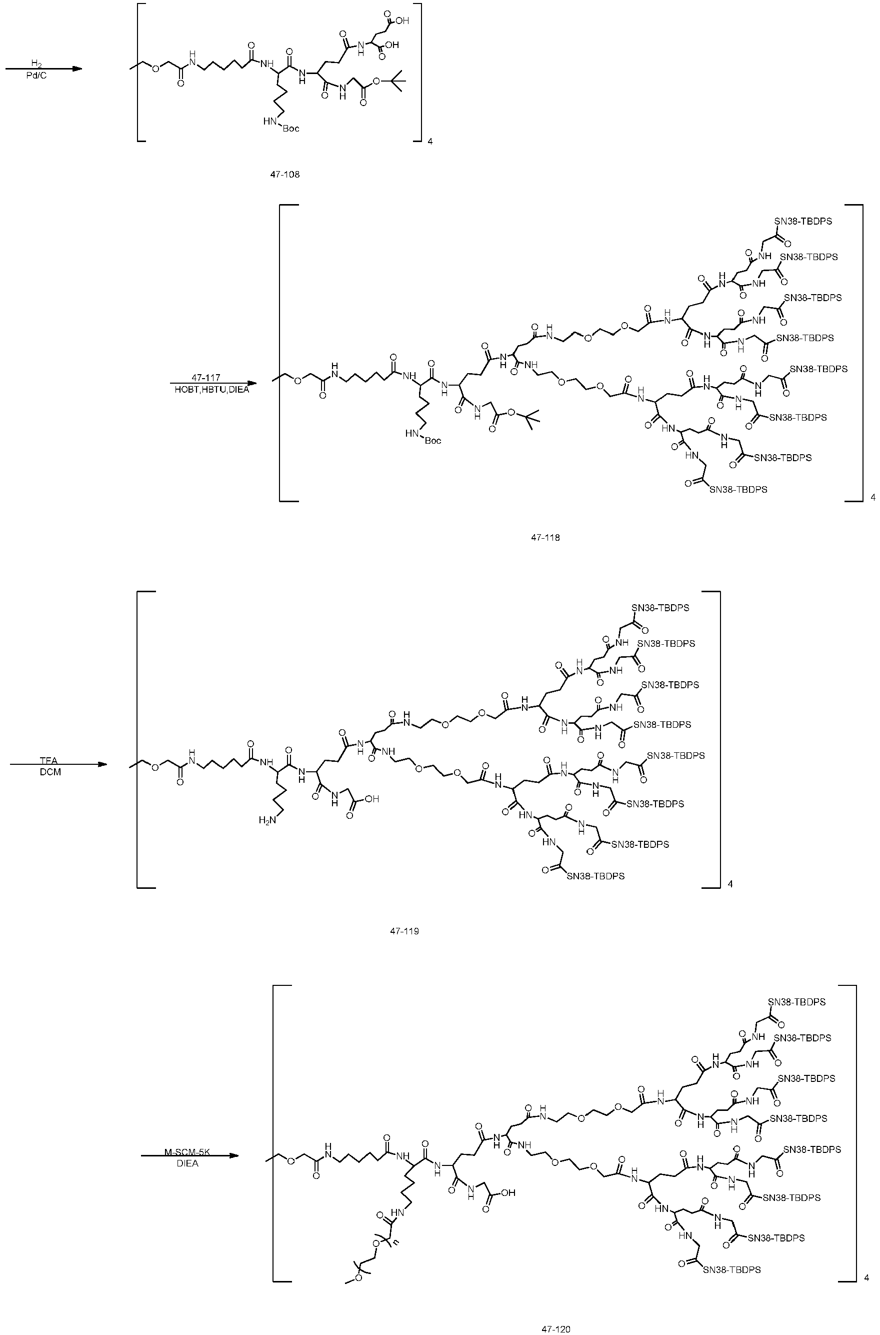

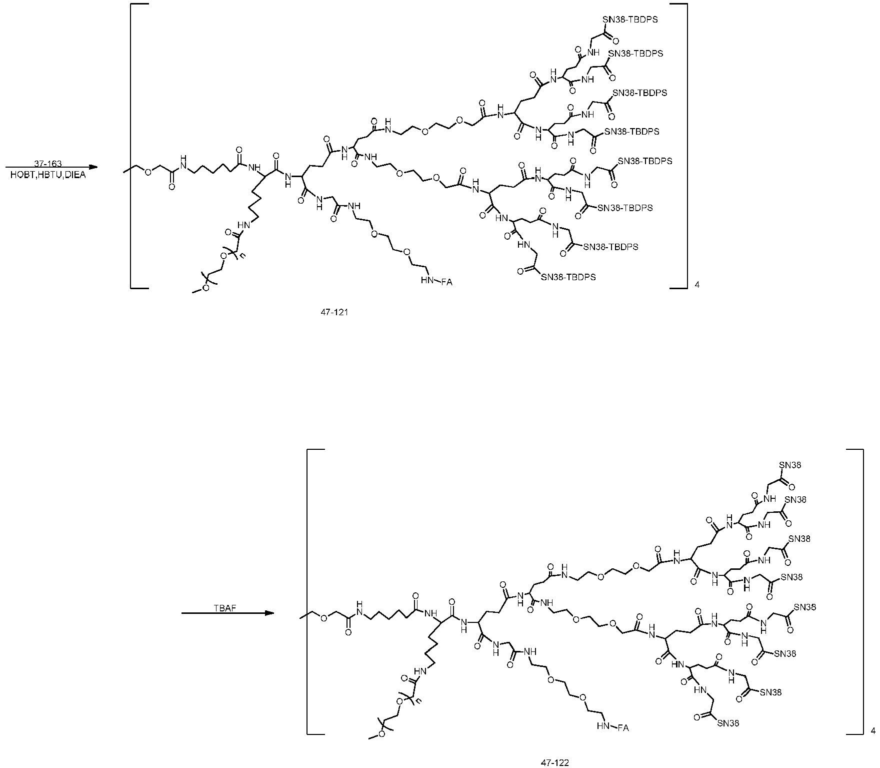

47-31

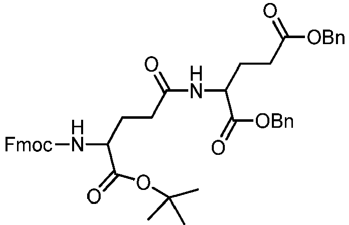

Fmoc-Glu- (OtBu) (OH) (3.0 g, 7.0510 mmol, purchased from Ark Pharm), Glu-OBn (3.6987 g, 7.4036 mmol, purchased from Ark Pharm), HBTU (4.0110 g, 10.5765 mmol) and HOBT (1.4292 g, 10.5765 mmol) were added to a 500 ml flask and then dissolved with an appropriate amount of DMF (50 mL), and the obtained solution was stirred at 0 °C for 30 min to react. DIEA (5.2439 mL, 31.7295 mmol) was slowly added dropwise, and the obtained solution was further stirred at 0 °C overnight to react. At the end of the reaction, the reaction solution was taken out, and deionized water (200 mL) was added to the reaction solution, extraction with ethyl acetate (100 mL × 3) was carried out, and the obtained organic phases were combined, washed twice with a saturated sodium chloride solution (200 mL), then concentrated and evaporated to dryness, and dried in a vacuum oven, thus obtaining the crude product.
47-32

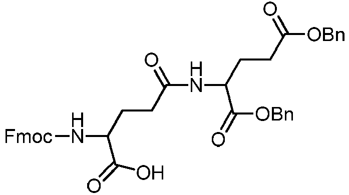

Product 47-31 (7.0510 mmol) was added in a 500 ml flask and dissolved with an appropriate amount of dichloromethane and TFA (7.8557 mL, 105.7050 mmol), and the reaction solution was stirred overnight at room temperature to react. At the end of the reaction, the reaction solution was taken out and evaporated to be oily; a saturated sodium bicarbonate solution was added to adjust the pH to alkaline, and the obtained solution was extracted with ethyl acetate (100 mL × 3); the obtained organic phases were combined, washed twice with a saturated sodium chloride solution (200 mL), then concentrated and evaporated to dryness, and dried in a vacuum oven, thus obtaining the crude product.
47-33

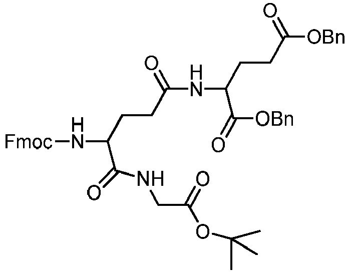

Product 47-32 (7.0510 mmol), H-Gly-OtBu (1.2411 g, 7.4036 mmol, purchased from Accela), HBTU (4.0110 g, 10.5765 mmol) and HOBT (1.4292 g, 10.5765 mmol) were added to a 500 ml flask and then dissolved with an appropriate amount of DMF (50 mL), and the obtained solution was stirred at 0 °C for 30 min to react. DIEA (5.2439 mL, 31.7295 mmol) was slowly added dropwise, and the obtained solution was further stirred at 0 °C overnight. At the end of the reaction, the reaction solution was taken out, deionized water (200 mL) was added to the reaction solution, extraction with ethyl acetate (100 mL × 3) was carried out, and the obtained organic phases were combined, washed twice with a saturated sodium chloride solution (200 mL), then concentrated and evaporated to dryness, and dried in a vacuum oven, thus obtaining the crude product.
47-35

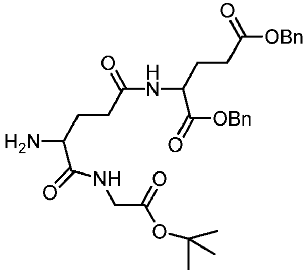

Morpholine and a small amount of DMF were added and dissolved in a 500 ml flask with Product 47-33, and the obtained solution was stirred at room temperature for 1 h to react. At the end of the reaction, the reaction solution was taken out; deionized water (200 mL) was added to the reaction solution, and extraction with ethyl acetate (100 mL × 3) was carried out; the obtained organic phases were combined, washed twice with a saturated sodium chloride solution (200 mL), then concentrated and evaporated to dryness, and dried in a vacuum oven, thus obtaining the crude product.
47-36

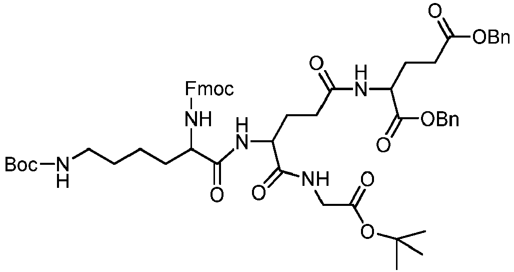

Product 47-35 (7.0510 mmol), Fmoc-Lys (Boc)-OH (3.4689 g, 7.4036 mmol), HBTU (4.0110 g, 10.5765 mmol) and HOBT (1.4292 g, 10.5765 mmol) were added to a 500 ml flask and then dissolved with an appropriate amount of DMF (50 mL), and the obtained solution was stirred at 0 °C for 30 min to react. DIEA (5.2439 mL, 31.7295 mmol) was slowly added dropwise, and the obtained solution was further stirred at 0 °C overnight. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), then concentrated and evaporated to dryness, and dried in a vacuum oven, thus obtaining the crude product.
47-38

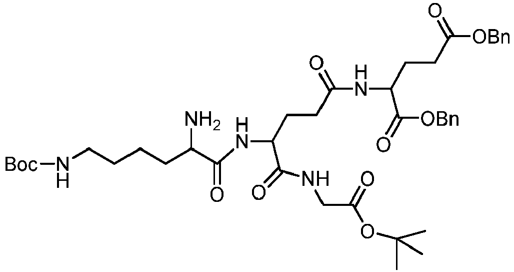

Morpholine and a small amount of DMF were added and dissolved in a 500 ml flask with Product 47-36, and the obtained solution was stirred at room temperature for 1 h to react. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), then concentrated and evaporated to dryness, and dried in a vacuum oven, thus obtaining the crude product.
47-39

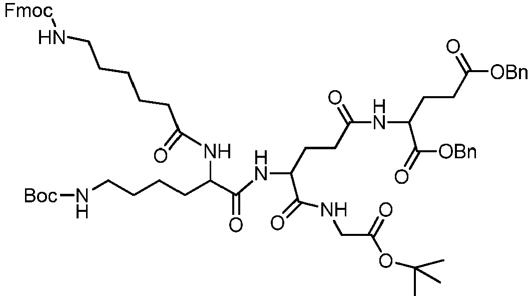

Product 47-38 (7.0510 mmol), Product 49-17 (2.6165 g, 7.4036 mmol), HBTU (4.0110 g, 10.5765 mmol) and HOBT (1.4292 g, 10.5765 mmol) were added to a 500 ml flask and then dissolved with an appropriate amount of DMF (50 mL), and the obtained solution was stirred at 0 °C for 30 min to react. DIEA (2.2439 mL, 31.7295 mmol) was slowly added dropwise, and the obtained solution was further stirred at 0 °C overnight. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), and concentrated; silica gel powder was added to the obtained solution, and the solution was then evaporated to dryness to be powder; column chromatography, and gradient elution with (1%-3% CH₃OH, others CH₂Cl₂) were carried out; the elution product was collected and concentrated, thus obtaining 4.6 g of Product 47-39 with a yield of 57%.
MALDI-TOF MS:[M+Na⁺] 1155.56
47-42

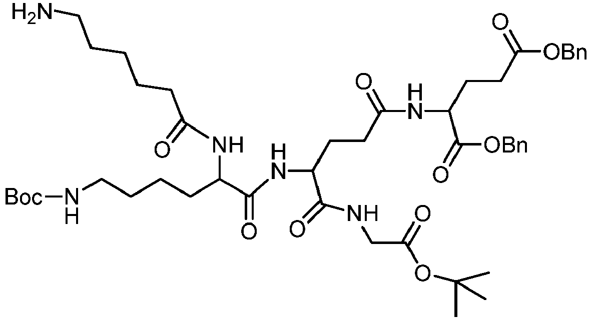

Morpholine (3.5 ml, 40.5884 mmol) and a small amount of DMF were added and dissolved in a 500 ml flask with Product 47-39 (4.6 g, 4.0588 mmol), and the obtained solution was stirred at room temperature for 1 h to react. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), then concentrated and evaporated to dryness, and dried in a vacuum oven, thus obtaining the crude product.
47-44

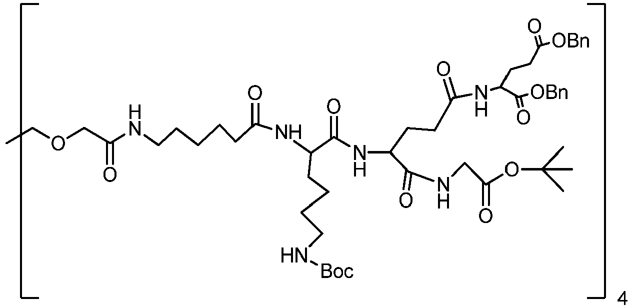

Product 47-42 (4.0588 mmol), Product 37-172 (0.9225 mmol), HBTU (2.0991 g, 5.5350 mmol) and HOBT (0.7479 g, 5.5350 mmol) were added in a 500 ml flask, and then dissolved with an appropriate amount of DMF (50 mL), and the obtained solution was stirred at -5 °C for 30 min to react; then, DIEA (2.7443 mL, 16.6050 mmol) was slowly added dropwise, the obtained solution was first stirred at -5 °C for 1 h, and then stirred at room temperature overnight to react. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), and concentrated; silica gel powder was then added to the obtained solution and the solution was evaporated to be powder; column chromatography and gradient elution with (2%-5% CH₃OH, others CH₂Cl₂) were carried out; the elution product was collected and concentrated. 1.6 g of Product 47-44 was obtained with a yield of 43%.

¹H-NMR (600 MHz, DMSO-*d*₆) δ 9.09 - 7.70 (m, 24H), 7.37 - 7.33 (m, 40H), 5.12 - 5.06 (m, 16H), 4.55 - 4.50 (m, 8H), 4.36 - 4.17 (m, 13H), 3.88 - 3.84 (m, 8H), 3.79 - 3.63 (m, 9H), 3.17 - 2.59 (m, 28H), 2.45 - 1.70 (m, 50H), 1.38 - 1.35 (m, 72H), 1.24 -1.21(m,24H).
47-108

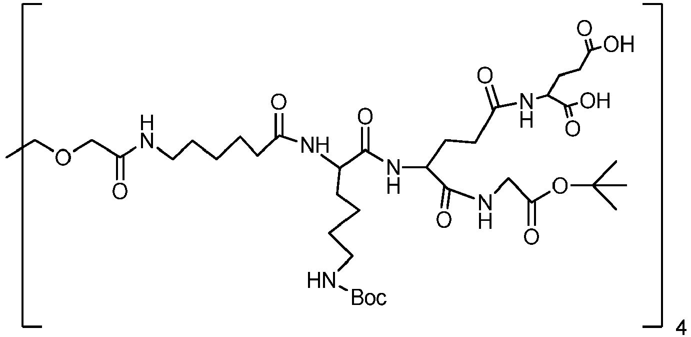

Product 47-44 (0.2964 g, 0.0752 mmol) and 10% Pd/C (0.02 g) were added into a hydrogenation reactor and then dissolved with DMF (30 mL); the hydrogenation reaction device was then sealed to perform the "three pumping and three charging" operation, so that the pressure on the hydrogenation reaction device was read as 0.18 MPa, and then the obtained solution reacted overnight at room temperature. At the end of the reaction, the reaction solution was filtered with diatomaceous earth; the filter cake was washed with DMF (20 mL × 3), thus obtaining Product 47-108 with a yield of 100%.
47-96

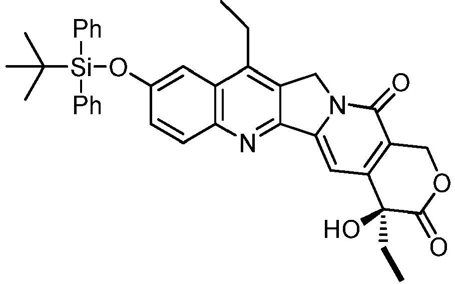

SN38 (15.00 g, 38.23 mmol) was added in a 1000 mL round-bottomed flask and then dissolved with 150 ml of dichloromethane; tert-butyldiphenylchlorosilane (59.64 ml, 229.36 mmol) and triethylamine (31.88 ml, 229.36 mmol) were added, and the reaction solution was stirred overnight in an oil bath at 37 °C to react. At the end of the reaction, the reaction solution was evaporated to be viscous, and n-hexane (150 ml) was added to precipitate the product to a solid; the solid was filtered by suction, and then dried, thus obtaining 23.15 g of Product 47-96 with a yield of 96%.
47-97

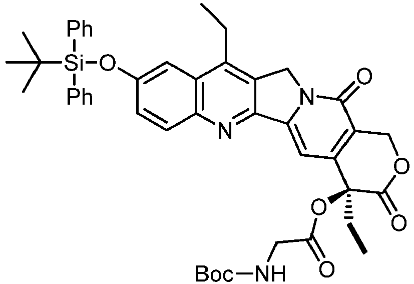

Product 47-96 (23.15 g, 36.70 mmol), Boc-Gly-OH (8.71 g, 49.70 mmol) and DMAP (0.94 g, 7.65 mmol) were added in a 1000 mL round-bottomed flask and then dissolved with dichloromethane (150 mL), and the mixed solution was stirred at 0 °C for 30 min to react. DCC (39.41 g, 191.15 mmol in total) was then added in three steps with an interval of 30 min. After that, reaction took place at 0 °C and continued for 2 h, and then the reaction device was placed at room temperature, and the reaction solution was stirred overnight to react. At the end of the reaction, n-hexane (200 mL) and petroleum ether (50 mL) were added for precipitation, and such operations were repeated three times; the solid product was obtained by filtering, and dried in a vacuum oven, thus obtaining 27.53 g of Product 47-97 with a yield of 94%.
47-98

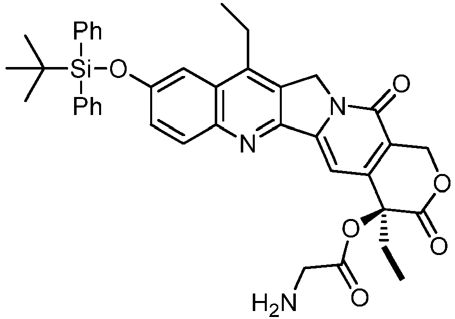

Product 47-97 (27.53 g, 34.50 mmol) was added in a 1000 mL round-bottomed flask and then dissolved with dichloromethane (50 ml), trifluoroacetic acid (28.40 ml, 382.30 mmol) was added, and the reaction solution reacted at room temperature. At the end of the reaction, the reaction solution was taken out, deionized water (200 mL) was added, and the reaction solution was extracted with ethyl acetate (100 mL × 3), the obtained organic phases were combined, the organic phase was washed with a saturated sodium chloride solution (200 mL × 2), and concentrated; silica gel powder was then added to the obtained solution and the solution was evaporated to be powder; column chromatography and gradient elution with (1%-3% CH₃OH, others CH₂Cl₂) were carried out; the elution product was collected and concentrated. 16.98 g of Product 47-98 was obtained with a yield of 72%.
47-115

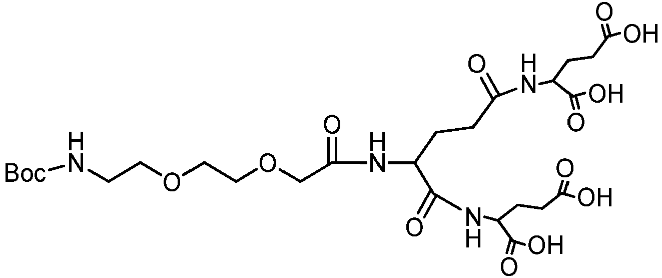

Product 35-85 (3.0 g, 2.9670 mmol) and 10% Pd/C (0.08 g) were added into a hydrogenation reactor and then dissolved with DMF (30 mL); the hydrogenation reaction device was then sealed to perform the "three pumping and three charging" operation, so that the pressure on the hydrogenation reaction device was read as 0.18 MPa, and the obtained solution reacted overnight at room temperature. At the end of the reaction, the reaction solution was filtered with diatomaceous earth; the filter cake was washed with DMF (20 mL × 3), thus obtaining Product 47-115 with a yield of 100%.
47-116

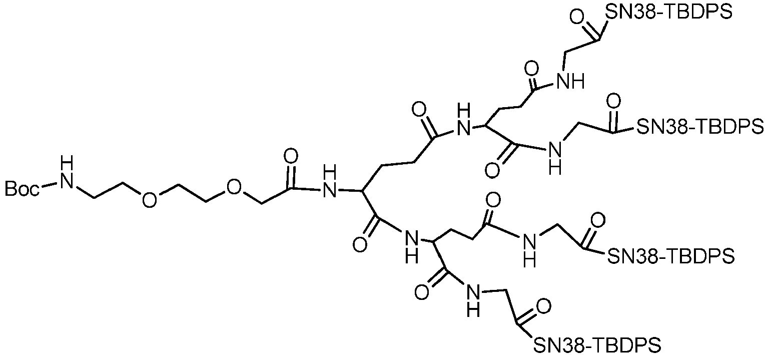

Product 47-115 (0.2964 mmol), Product 47-98 (8.58 g, 12.4614 mmol), HBTU (6.76 g, 17.8020 mmol) and HOBT (2.41 g, 17.8020 mmol) were added to a 500 ml flask and then dissolved with an appropriate amount of DMF (50 mL), and the obtained solution was stirred at -5 °C for 30 min to react. DIEA (10.79 mL, 65.2740 mmol) was slowly added dropwise, and the obtained solution was further stirred at -5 °C overnight to react. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), and concentrated; silica gel powder was then added to the obtained solution and the solution was evaporated to be powder; column chromatography and gradient elution with (1%-2% CH₃OH, others CH₂Cl₂) were carried out; the elution product was collected and concentrated. 4.97 g of Product 47-116 was obtained with a yield of 50%.
47-117

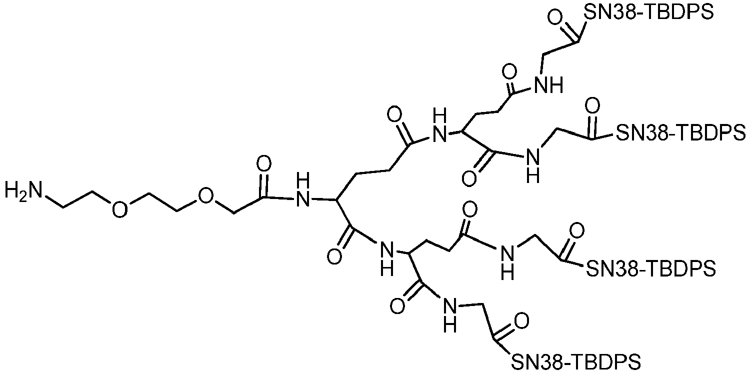

Product 47-116 (4.97 g, 1.4925 mmol) was added in a 1000 mL round-bottomed flask and then dissolved with dichloromethane (50 ml), trifluoroacetic acid (1.11 ml, 14.93 mmol) was added, and the mixed solution reacted at room temperature. At the end of the reaction, n-hexane (100 mL) and methyl tert-butyl ether (400 mL) were first added for precipitation, and such operations were repeated three times; the solid product was obtained by filtering, and the obtained solid product was dissolved with dichloromethane (100 mL) and methanol (10 mL); the operations of dry sample loading, column chromatography, and elution with 1%-3% methanol/dichloromethane were carried out; the elution product was collected, concentrated, evaporated to dryness, and dried in a vacuum oven, thus obtaining 3.5 g of Product 47-117 with a yield of 73%.
47-118

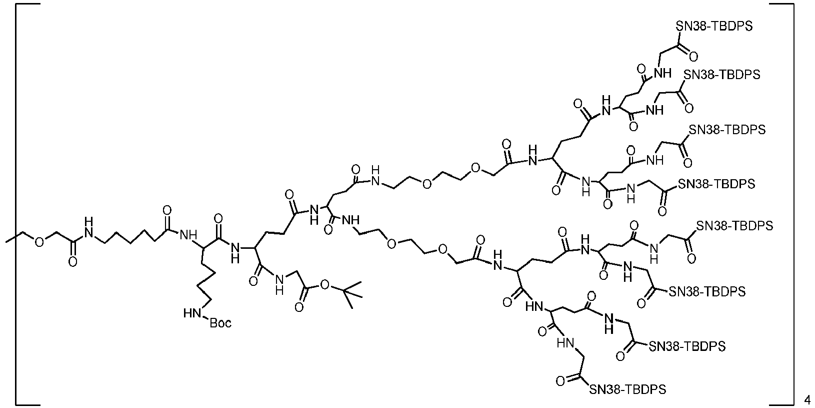

Product 47-108 (0.0752 mmol), Product 47-117 (2.04 g, 0.6317 mmol), HBTU (0.3423 g, 0.9024 mmol) and HOBT (0.1220 g, 0.9024 mmol) were added in a 250 mL round-bottomed flask and then dissolved with an appropriate amount of DMF (50 mL); the mixed solution was stirred at - 5 °C for about 30 min. then, DIEA (0.5469 mL, 3.3088 mmol) was slowly added dropwise; at the end of the addition, the obtained solution reacted at -5 °C for 2 h, and then the reaction device was placed at -5 °C and the reaction solution was stirred at -5 °C overnight to react. At the end of the reaction, n-hexane (100 mL) and methyl tert-butyl ether (400 mL) were added for precipitation, and such operations were repeated three times; a solid product was obtained by filtering, and the obtained solid product was dissolved with dichloromethane (100 mL) and methanol (10 mL); the operations of dry sample loading, column chromatography, and elution with 4%-15% methanol/dichloromethane were carried out; the elution product was collected, concentrated, evaporated to dryness, and dried in a vacuum oven, thus obtaining 1.5655 g of Product 47-118 with a yield of 72%.
47-119

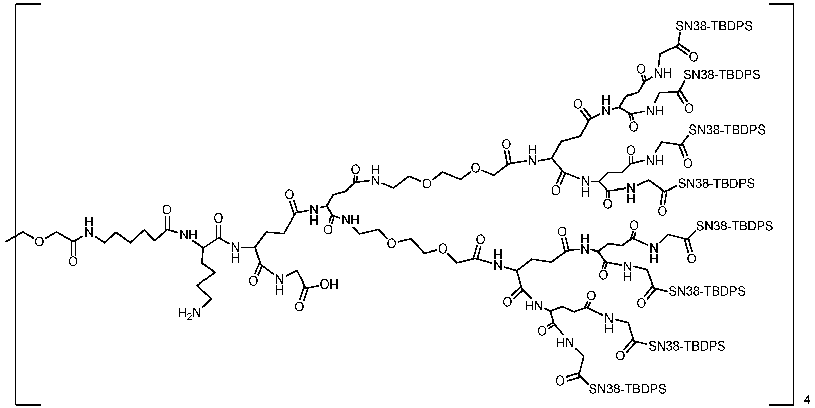

Product 47-118 (1.5655 g, 0.0541 mmol) was added in a 250 mL flask and then dissolved with dichloromethane (8 mL) and TFA (0.33 ml, 4.33 mmol), and the mixed solution was stirred at room temperature in the dark to react overnight. At the end of the reaction, the reaction solution was evaporated with a rotary evaporator to obtain an oily product, methyl tert-butyl ether (60 mL) was then added; the reaction solution was precipitated to obtain a powdery solid; suction filtering was carried out, and the filter cake was washed with methyl tert-butyl ether (40 mL × 3) and then collected, and dried in a vacuum oven. 1.33 g of crude Product 47-119 was obtained with a yield of 87%.
47-120

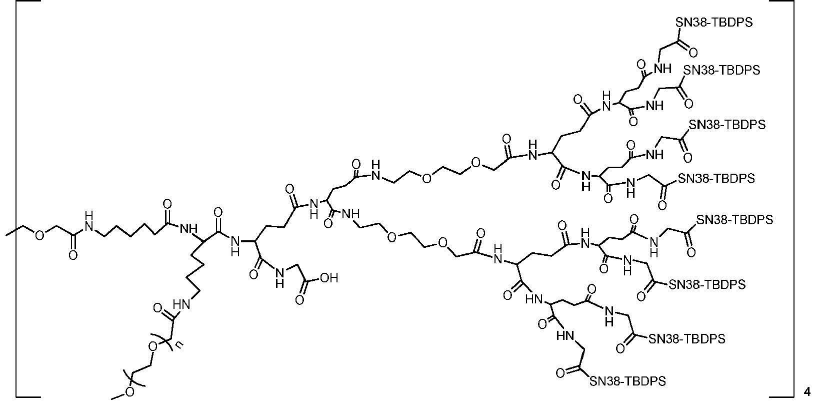

M-SCM-5K (1.0450 g, 0.2073 mmol) was added in a 250 mL round-bottomed flask and then dissolved with an appropriate amount of DMF (50 mL); DIEA (0.7785 ml, 4.71 mmol) was added, and the obtained solution was stirred at -5 °C for 30 min to react. The DMF solution (50 ml) of Product 47-119 (1.33 g, 0.0471 mmol) was slowly added dropwise, and the obtained solution was slowly stirred for 7 days at room temperature in the dark to react. At the end of the reaction, n-hexane (100 mL) and methyl tert-butyl ether (80 mL) were added for precipitation to obtain a solid product, suction filtering was carried out, and the filter cake was washed with methyl tert-butyl ether (40 mL × 3), collected, and dissolved with a mixed solution of methanol/dichloromethane (1:4) (100 mL); silica gel powder (20 g) was added to the obtained solution, and the solution was then evaporated to be powder; the operations of dry sample loading, column chromatography, and gradient elution with a mixed solution (3%-18% methanol/dichloromethane) were carried out; the elution product was then collected, concentrated, and dried in a vacuum oven. 1.8 g of Product 47-120 was obtained with a yield of 79%.
47-121

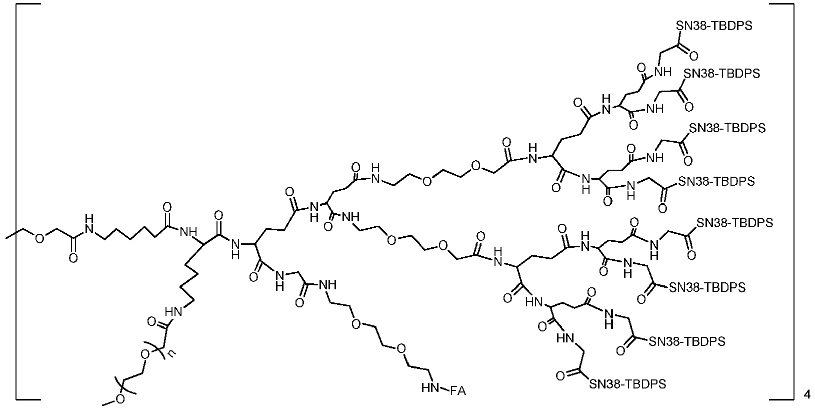

Product 47-120 (1.8 g, 0.0373 mmol), Product 37-163 (0.1066 g, 0.1864 mmol), HBTU (0.0849 g, 0.2238 mmol) and HOBT (0.0302 g, 0.2238 mmol) were added in a 250 mL round-bottomed flask and then dissolved with DMF (50 mL), and the mixed solution was stirred at -5 °C for 30 min. Then DIEA (0.1110 mL, 0.6714 mmol) was slowly added dropwise, and at the end of the addition, the obtained solution reacted at low temperature to react for 2 h, and then the reaction device was placed at room temperature and the reaction solution was stirred overnight to react. At the end of the reaction, n-hexane (100 mL) and methyl tert-butyl ether (80 mL) were added for precipitation to obtain a solid product, and suction filtering was carried out; the filter cake was washed with methyl tert-butyl ether (40 mL × 3), collected, and dissolved with a mixed solution of methanol/dichloromethane (1:4) (100 mL); silica gel powder (20 g) was then added to the obtained solution and the solution was evaporated to be powder; the operations of dry sample loading, column chromatography and gradient elution with a mixed solution (3%-18% methanol/dichloromethane) were carried out; the elution product was collected, concentrated and dried in a vacuum oven. 1.44 g of Product 47-121 was obtained with a yield of 76%.
47-122

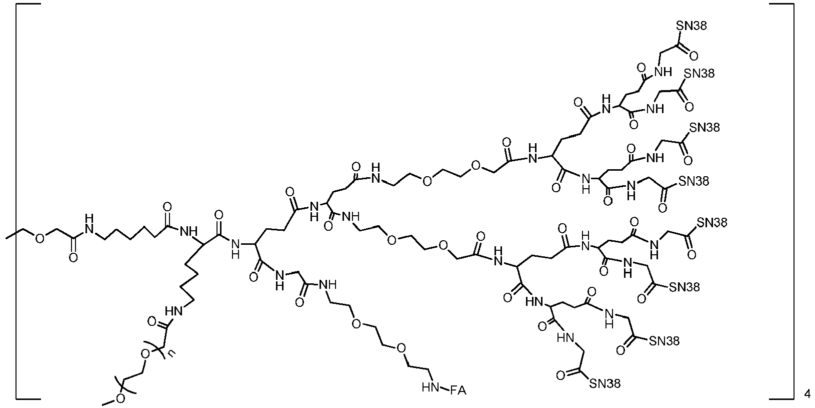

Product 47-121 (0.0284 mmol) in a 250 mL flask was dissolved by THF (8 ml) and dilute hydrochloric acid (8 ml, 0.05 mmol/L); TBAF (4.75 g, 18.1427 mmol) was added, and the obtained solution was stirred at room temperature in the dark for 3 h to react. At the end of the reaction, the reaction solution was evaporated to dryness and then dissolved with DMF (8 ml), then precipitated with isopropanol, and the above operations were repeated three times; and then the obtained solution was dissolved with anhydrous ethanol and a small amount of dichloromethane; methyl tert-butyl ether was added for precipitation, and the above operations were repeated three times; the resulting solid was collected, and dried in a vacuum oven, thus obtaining 1.09 g of Product 47-122 with a yield of 84%.

¹H-NMR (600 MHz, DMSO-*d*₆) δ 8.64 - 6.70 (m, 220H), 5.72 - 4.90 (m,108H), 4.71 - 3.57 (m, 320H), 3.54 - 3.46(m, 1681H), 3.25 - 2.67 (m, 170H), 2.40 - 1.32 (m, 238H), 1.29 - 1.17 (m, 96H), 1.04 - 0.69 (m,86H).

### Synthesis of Compound 49-166 (Control)

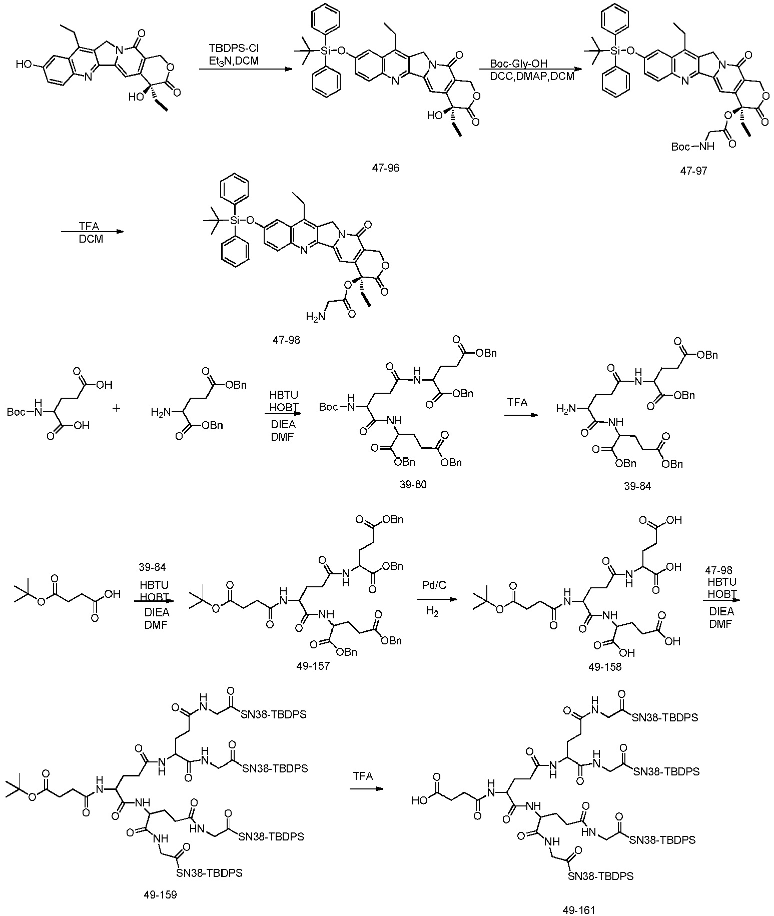

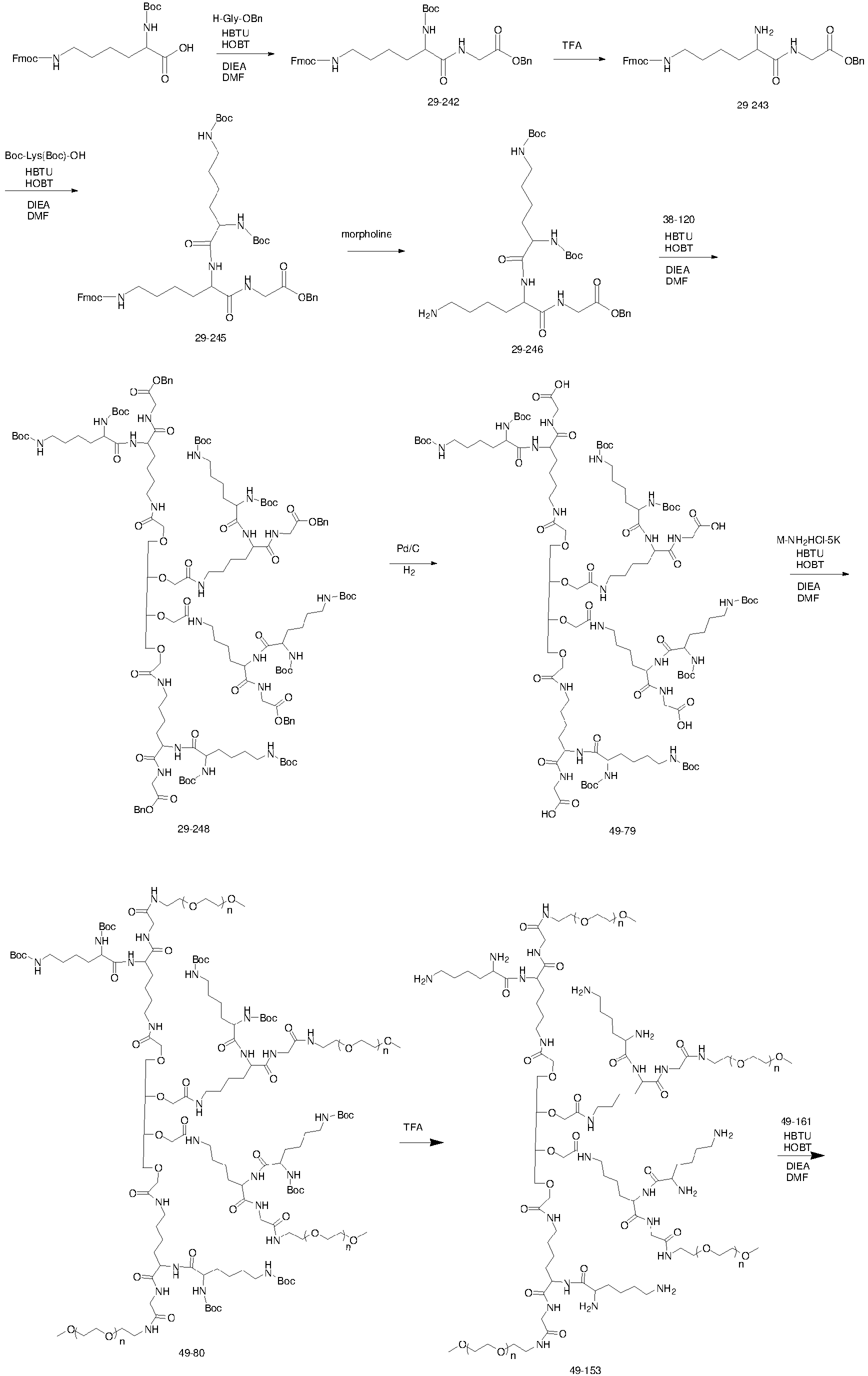

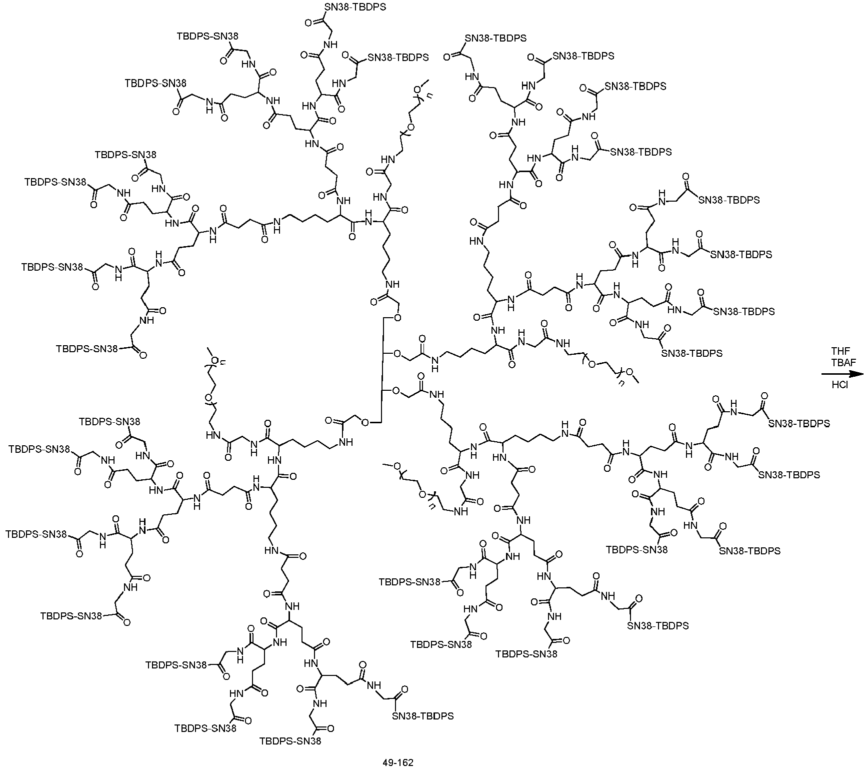

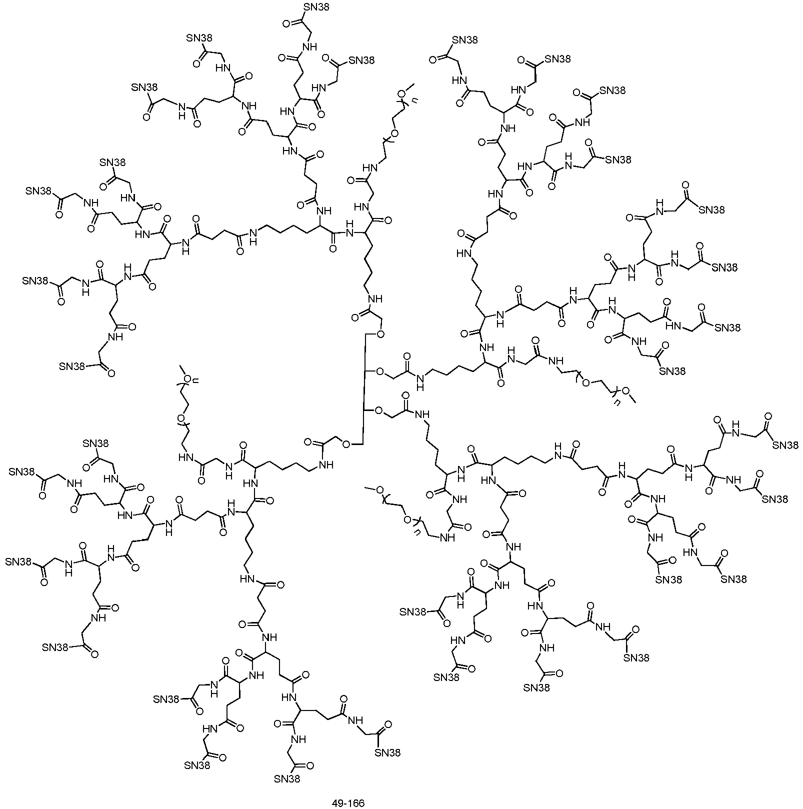

39-80

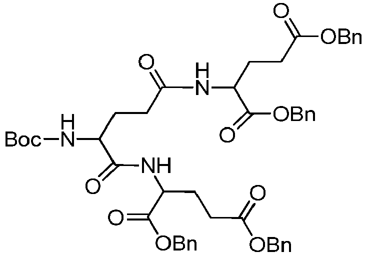

Boc-Glu-OH (5.0 g, 20.22 mmol, purchased from Aladdin), H-Glu(OBzl)-OBzl·TsOH (21.2 g, 42.46 mmol, purchased from Ark Pharm), HOBT (8 g, 60.66 mmol) and HBTU (23 g, 60.66 mmol) were weighed and added to a 250 mL flask and then dissolved with DMF (80 mL), and the ultrasonic treatment was carried out to completely dissolve the reactants; the obtained solution was stirred for 30 min at -5 °C; DIEA (30 mL, 181 mmol) was slowly added dropwise, and the obtained solution reacted at a low temperature to the end. At the end of the reaction, the reaction solution was taken out; deionized water (100 mL) was added to the reaction solution, and extraction with ethyl acetate (100 mL × 3) was carried out; the obtained organic phases were combined, washed twice with a saturated sodium chloride solution (100 mL), and finally concentrated and evaporated to dryness. The operations of dry sample loading, column chromatography, and gradient elution with 40% ethyl acetate/petroleum ether -50% ethyl acetate/petroleum ether were carried out; the elution product was then collected, concentrated and evaporated to dryness.
39-84

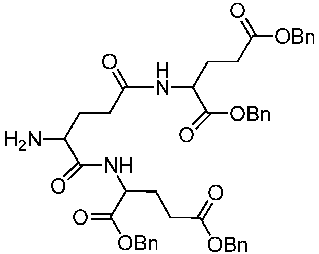

Product 39-80 (19.2 mmol) was weighed and dissolved by ultrasonic with dichloromethane (5 mL) and TFA (14 mL, 192 mmol); a ground glass stopper was used, and the obtained solution was stirred to react at room temperature. At the end of the reaction, the reaction solution was taken out, a saturated sodium bicarbonate solution (300 mL) was added to the reaction solution, and extraction with ethyl acetate (100 mL × 3) was carried out several times; the obtained organic phases were combined, washed twice with a saturated sodium chloride solution (100 mL), and finally concentrated and evaporated to dryness.
47-96

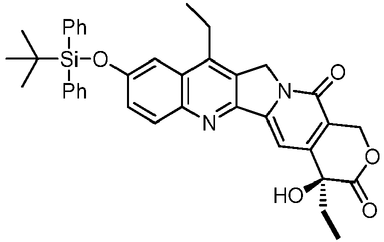

7-Ethyl-10-hydroxycamptothecin (SN38, 15.00 g, 38.23 mmol) was added in a 1000 mL round-bottomed flask and then dissolved with 150 ml of dichloromethane; tert-butyldiphenylchlorosilane (59.64 ml, 229.36 mmol, purchased from Accela), and triethylamine (31.88 ml, 229.36 mmol) were added, and the reaction solution was stirred overnight in an oil bath at 37 °C to react. At the end of the reaction, the reaction solution was evaporated to be viscous, and n-hexane (150 ml) was added to precipitate the product to a solid; the solid was filtered by suction, and then dried, thus obtaining the product (23.15 g, 96%).
47-97

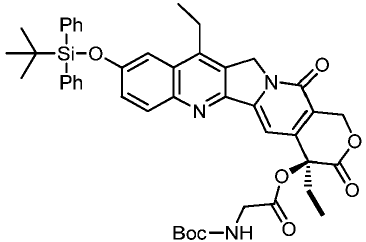

Product 47-96 (23.15 g, 36.70 mmol), Boc-Gly-OH (8.71 g, 49.70 mmol, purchased from Aladdin) and DMAP (0.94 g, 7.65 mmol) were added in a 1000 mL round-bottomed flask and then dissolved with dichloromethane (150 mL), and the obtained solution was stirred at 0 °C for 30 min to react. DCC (39.41 g, 191.15 mmol) was then added in three steps with an interval of 30 min. After that, reaction took place at 0 °C and continued for 2 h, and then the reaction device was placed at room temperature, and the reaction solution was stirred overnight to react. At the end of the reaction, n-hexane (200 mL) and petroleum ether (50 mL) were added for precipitation, and such operations were repeated three times, and the solid product was obtained by filtering, and dried in a vacuum oven, thus obtaining the product (27.53 g, 94%).
47-98

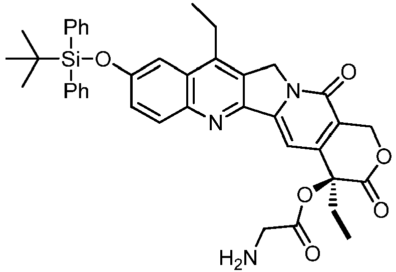

Product 47-97 (27.53 g, 34.50 mmol) was added in a 1000 mL round-bottomed flask and dissolved with dichloromethane (50 ml), trifluoroacetic acid (28.40 ml, 382.30 mmol) was added, and the reaction solution reacted at room temperature. At the end of the reaction, the reaction solution was taken out, and deionized water (200 mL) was added to the reaction solution; the obtained solution was extracted with ethyl acetate (100 mL × 3), the obtained organic phases were combined, the organic phase was washed with a saturated sodium chloride solution (200 mL × 2), and concentrated; silica gel powder was then added to the obtained solution and the solution was evaporated to be powder; column chromatography and gradient elution with (1%-3% CH₃OH, others CH₂Cl₂) were carried out; the elution product was collected and concentrated, thus obtaining the product (16.98 g, 72%).
29-242

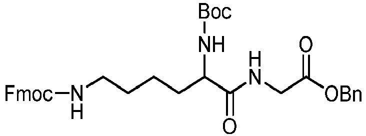

Fmoc-Lys (Boc)-OH (5.0 g, 10.6714 mmol, purchased from Aladdin), H-Gly-OBn (3.7802 g, 11.2050 mmol, purchased from Innochem), HBTU (6.0705 g, 16.0072 mmol) and HOBT (2.1630 g, 16.0072 mmol) were added to a 500 ml flask and then dissolved with DMF (50 mL), and the mixed solution was stirred at 0 °C for 30min. DIEA (7.9371 mL, 48.0215 mmol) was slowly added dropwise, and then stirred at 0 °C to further react overnight. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), then concentrated and evaporated to dryness, and dried in a vacuum oven, thus obtaining the crude product.
29-243

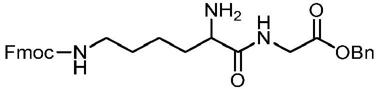

Product 29-242 (6.57 g, 10.6714 mmol) was added in a 500 ml flask and then dissolved with an appropriate amount of dichloromethane and TFA (7.9248 mL, 106.714 mmol),and the reaction solution was stirred overnight at room temperature to react. At the end of the reaction, the reaction solution was taken out and evaporated to be oily; a saturated sodium bicarbonate solution was added to adjust the pH to alkaline, and the obtained solution was extracted with ethyl acetate (100 mL × 3); the obtained organic phases were combined, washed twice with a saturated sodium chloride solution (200 mL), then concentrated and evaporated to dryness, and dried in a vacuum oven, thus obtaining the crude product.
29-245

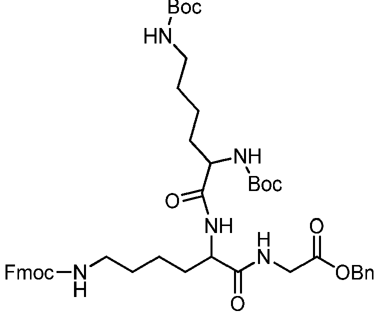

Boc-Lys (Boc)-OH (4.2805 g, 11.7385 mmol, purchased from Ark Pharm), Product 29-243 (5.50 g, 10.6714 mmol), HBTU (6.0705 g, 16.0072 mmol) and HOBT (2.1630 g, 16.0072 mmol) were added to a 500 ml flask and then dissolved with DMF (50 mL), and the obtained solution was stirred at 0 °C for 30 min to react. DIEA (7.9371 mL, 48.0215 mmol) was slowly added dropwise, and the obtained solution was further stirred at 0 °C overnight to react. At the end of the reaction, the reaction solution was taken out; deionized water (200 mL) was added to the reaction solution, and extraction with ethyl acetate (100 mL × 3) was carried out; the obtained organic phases were combined, washed twice with a saturated sodium chloride solution (200 mL), then concentrated and evaporated to dryness, and dried in a vacuum oven, thus obtaining the crude product.
29-246

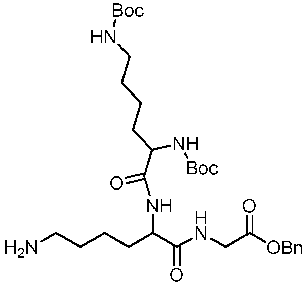

Morpholine (9.24 mL, 106.714 mmol) was added in a 500 ml flask with Product 29-245 (9.0 g, 10.6714 mmol) and dissolved with DMF (10 mL), and the mixed solution was stirred for 1 h at room temperature to react. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), and concentrated; silica gel powder was then added to the obtained solution and the solution was evaporated to be powder; column chromatography and gradient elution with (1% ammonia water, 4%-5% methanol, others dichloromethane) were carried out; the elution product was collected and concentrated. 3.7 g of Product 29-246 was obtained with a yield of 56%.
29-248

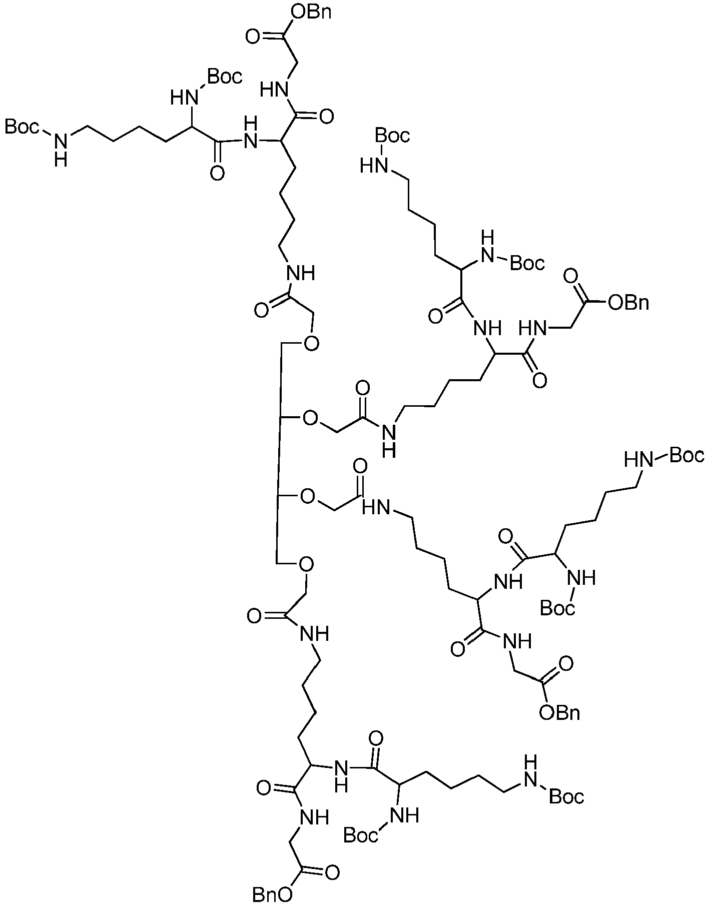

Product 38-120 (0.39 g, 1.0966 mmol), Product 29-246 (3.0 g, 4.8249 mmol), HBTU (2.4951 g, 6.5795 mmol) and HOBT (0.8891 g, 6.5795 mmol) were added in a 500 ml flask and then dissolved with DMF (50 mL), and the obtained solution was stirred at -5 °C for 30 min to react; then, DIEA (3.2624 mL, 19.7384 mmol) was slowly added dropwise, the obtained solution was first stirred at -5 °C for 1 h to react, and then stirred at room temperature overnight. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), and concentrated; silica gel powder was then added to the obtained solution and the solution was evaporated to be powder; column chromatography and gradient elution with (50%-80% ethyl acetate, others petroleum ether) were carried out; the elution product was collected and concentrated, thus obtaining the product (1.6 g, 53%).
49-79

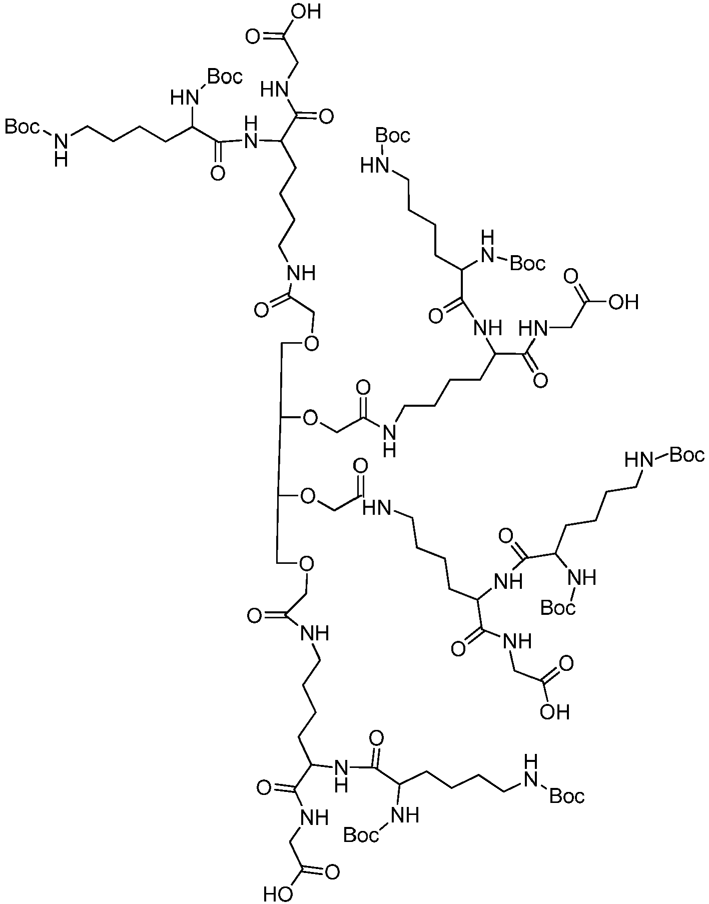

Product 29-248 (0.52 g, 0.1878 mmol) and 10% Pd/C (0.0,5 g) were added into a hydrogenation reactor and then dissolved with DMF (30 mL); the hydrogenation reaction device was then sealed to perform the "three pumping and three charging" operation, so that the pressure on the hydrogenation reaction device was read as 0.18 MPa, and then the obtained solution reacted overnight at room temperature. At the end of the reaction, the reaction solution was filtered with diatomaceous earth; the filter cake was washed with DMF (20 mL × 3), thus obtaining the DMF solution of the product as the raw material for the next step.
49-80

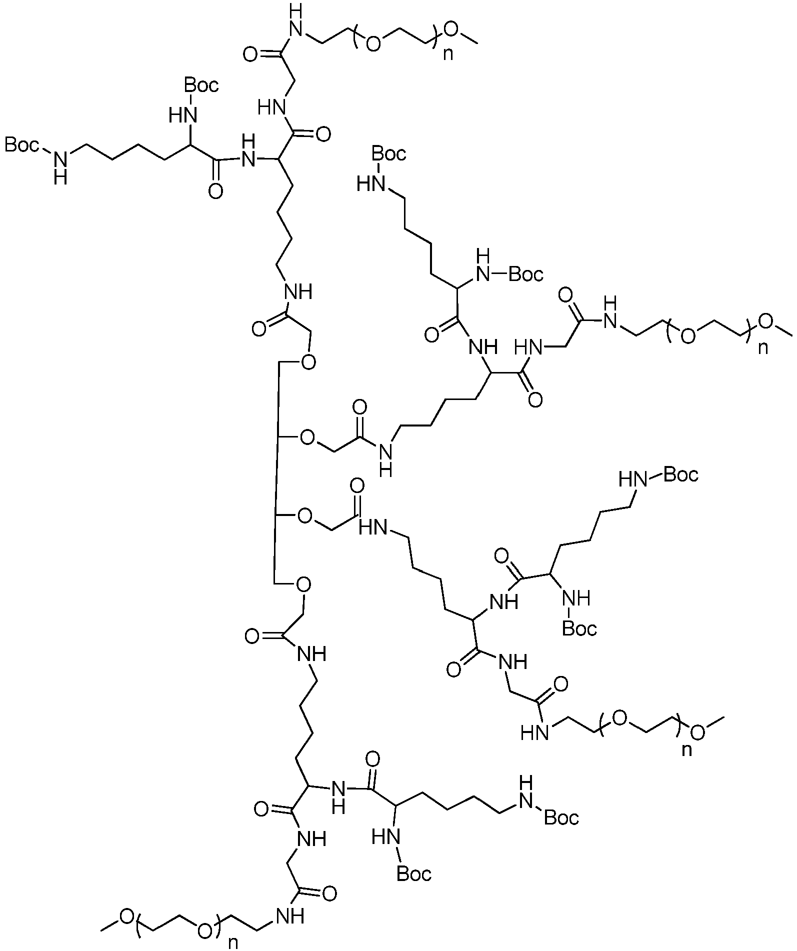

Product 49-79 (0.45 g, 0.1878 mmol), M-NH₂HCl-5K (4.91 g, 0.9389 mmol, purchased from JenKem), HBTU (0.43 g, 1.1237 mmol) and HOBT (0.15 g, 1.1267 mmol) were added in a 500 ml flask and then dissolved with DMF (60 mL), and the obtained solution was stirred at -5 °C for 30 min to react. DIEA (0.56 mL, 3.3780 mmol) was slowly added dropwise, and the obtained solution was first stirred at -5 °C for 1 h to react, and then slowly stirred for 3 days at room temperature in the dark to react. At the end of the reaction, n-hexane (100 mL) was added, the reaction solution was then shaken, and the supernatant was discarded; and such operations were repeated three times. Methyl tert-butyl ether (80 mL) and a small amount of n-hexane (10 mL) were added, the reaction solution was then shaken, and the supernatant was discarded; the above operations were repeated three times, and the powdery solid in the reaction solution was separated out by precipitation, suction filtering was carried out, the filter cake was washed with methyl tert-butyl ether (40 mL × 3) and then collected, and dissolved with a mixed solution of methanol/dichloromethane (1:4) (100 mL); silica gel powder (20 g) was then added to the obtained solution and the solution was evaporated to be powder; the operations of dry sample loading, column chromatography and gradient elution with a mixed solution (1% ammonia water/ 3%-7% methanol/dichloromethane) were carried out; the elution product was collected, concentrated and dried in a vacuum oven, thus obtaining the product (3.2 g, 73.73%).
49-153

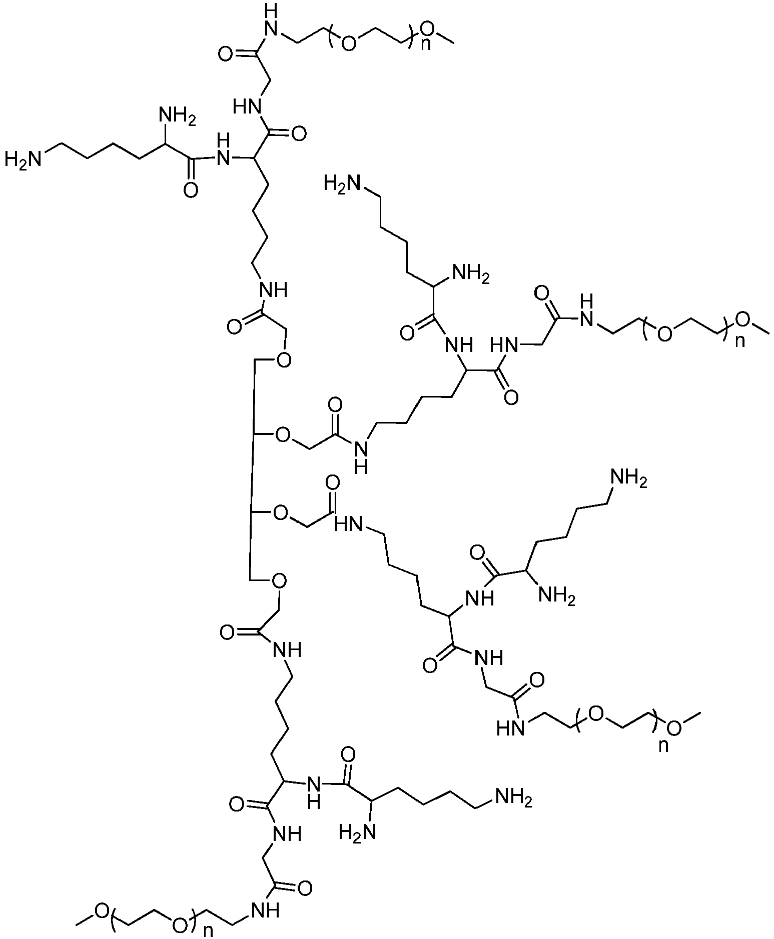

Product 49-80 (3.2 g, 0.1383 mmol) was added in a 250 mL flask and then dissolved with dichloromethane (5 mL) and TFA (0.82 mL, 11.0640 mmol), and the obtained solution was slowly stirred overnight at room temperature in the dark to react. At the end of the reaction, the reaction solution was evaporated with a rotary evaporator to obtain an oily product; methyl tert-butyl ether (60 mL) was then added, the reaction solution was precipitated to obtain a powdery solid, and suction filtering was carried out; the filter cake was washed with methyl tert-butyl ether (40 mL × 3) and then collected and dissolved with a mixed solution of methanol/dichloromethane (1:4) (100 mL); silica gel powder (20 g) was then added to the obtained solution and the solution was evaporated to be powder; the operations of dry sample loading, column chromatography and gradient elution with a mixed solution (3%-12% methanol/dichloromethane) were carried out; the elution product was collected, concentrated and dried in a vacuum oven, thus obtaining the product (1.74 g, 56.35 %).
49-157

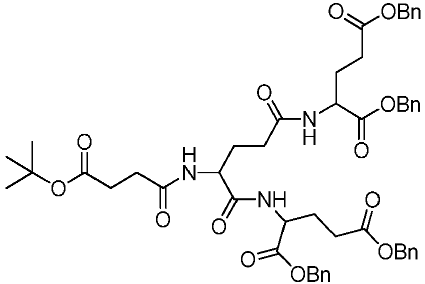

Product 39-84 (5.16 g, 6.7433 mmol), mono-tert-butyl succinic acid (1.40 g, 8.0920 mmol, purchased from Accela), HBTU (3.84 g, 10.1149 mmol) and HOBT (1.36 g, 10.1149 mmol) were added in a 500 ml flask and then dissolved with DMF (50 mL), and the obtained solution was stirred at -5 °C for 30 min to react; then, DIEA (10.03 mL, 60.6897 mmol) was slowly added dropwise, the obtained solution was first stirred at -5 °C for 1 h to react, and then stirred at room temperature overnight. At the end of the reaction, the reaction solution was taken out, deionized water (200 mL) was added to the reaction solution, and extraction with ethyl acetate (100 mL × 3) was carried out; the obtained organic phases were combined, washed twice with a saturated sodium chloride solution (200 mL), and concentrated; silica gel powder was then added to the obtained solution and the solution was evaporated to be powder; column chromatography and gradient elution with (50%-90% ethyl acetate, others petroleum ether) were carried out; the elution product was collected and concentrated, thus obtaining the product (5.66 g, 90.99%).
49-158

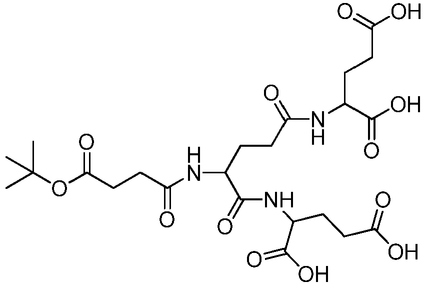

Product 49-157 (2.8 g, 3.0367 mmol) and 10% Pd/c (0.08 g) were added into a hydrogenation reactor and then dissolved with DMF (30 mL); the hydrogenation reaction device was then sealed to perform the "three pumping and three charging" operation, so that the pressure on the hydrogenation reaction device was read as 0.18 MPa, and then the obtained solution reacted overnight at room temperature. At the end of the reaction, the reaction solution was filtered with diatomaceous earth; the filter cake was washed with DMF (20 mL × 3), thus obtaining the DMF solution of the product as the raw material for the next step.
49-159

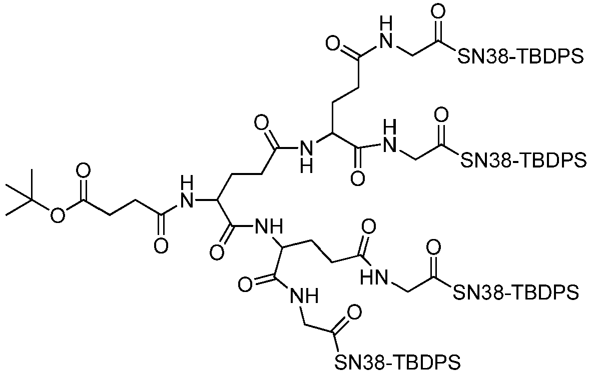

Product 49-158 (1.71 g, 3.0367 mmol), Product 47-98 (8.77 g, 12.7541 mmol), HBTU (6.90 g, 18.2202 mmol) and HOBT (2.46 g, 18.2202 mmol) were added in a 500 ml flask and then dissolved with DMF (60 mL), and the obtained solution was stirred at -5 °C for 30 min to react; then, DIEA (13 mL, 78.9542 mmol) was slowly added dropwise, the obtained solution was first stirred at -5 °C for 1 h to react, and then stirred at room temperature overnight. At the end of the reaction, n-hexane (100 mL) was added, the reaction solution was then shaken, and the supernatant was discarded; and such operations were repeated three times. Methyl tert-butyl ether (80 mL) and a small amount of n-hexane (10 mL) were added, the reaction solution was then shaken, and the supernatant was discarded; the above operations were repeated three times, and the powdery solid in the reaction solution was separated out by precipitation; suction filtering was carried out, the filter cake was washed with methyl tert-butyl ether (40 mL × 3) and then collected, and dissolved with a mixed solution of methanol/dichloromethane (1:4) (100 mL); silica gel powder (60 g) was then added to the obtained solution and the solution was evaporated to be powder; the operations of dry sample loading, column chromatography and gradient elution with a mixed solution (3%-7% methanol/dichloromethane) were carried out; the elution product was collected, concentrated and dried in a vacuum oven, thus obtaining the product (11.3 g being extra-quota product).
49-161

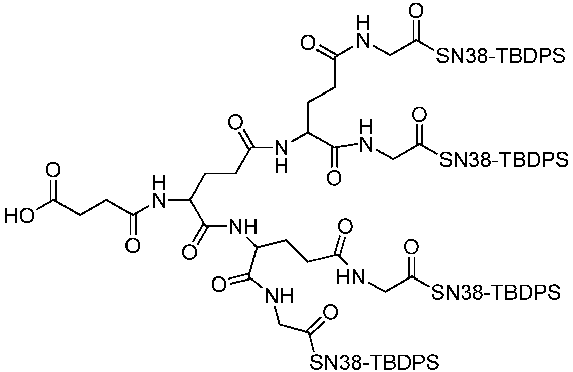

Product 49-159 (9.84 g, 3.0367 mmol) was added in a 250 mL flask and then dissolved with dichloromethane (8 mL) and TFA (8 mL), and the reaction solution was stirred overnight at room temperature to react. At the end of the reaction, the reaction solution was evaporated with a rotary evaporator to obtain an oily product; methyl tert-butyl ether (60 mL) was then added, and the reaction solution was precipitated to obtain a powdery solid; suction filtering was carried out, the filter cake was washed with methyl tert-butyl ether (40 mL × 3) and then collected and dissolved with a mixed solution of methanol/dichloromethane (1:4) (100 mL); silica gel powder (60 g) was then added to the obtained solution and the solution was evaporated to be powder; the operations of dry sample loading, column chromatography and gradient elution with a mixed solution (1%-4% methanol/dichloromethane) were carried out; the elution product was collected, concentrated and dried in a vacuum oven, thus obtaining the product (2.7 g, 27.92%).
49-162

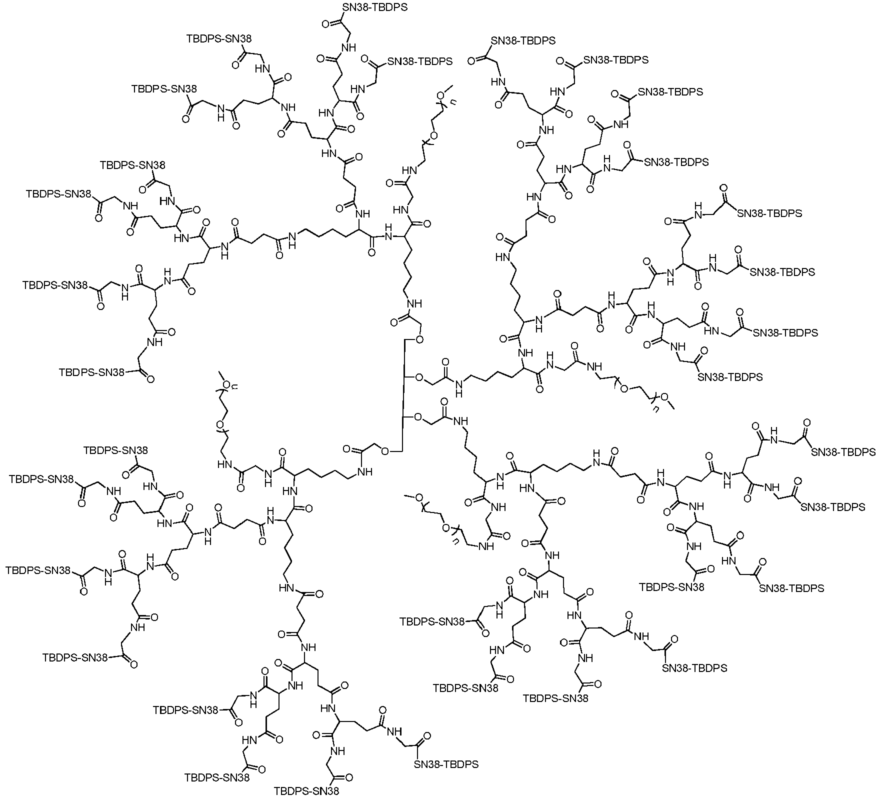

Product 49-153 (1.19 g, 0.0535 mmol), Product 49-161 (1.5 g, 0.4710 mmol), HBTU (0.24 g, 0.6422 mmol) and HOBT (0.08 g, 0.6422 mmol) were added in a 500 ml flask and then dissolved with DMF (60 mL), and the obtained solution was stirred at -5 °C for 30 min to react. DIEA (0.60 mL, 3.6395 mmol) was slowly added dropwise, and the obtained solution was first stirred at -5 °C for 1 h to react, and then slowly stirred overnight at room temperature in the dark to react. At the end of the reaction, n-hexane (100 mL) was added, and the reaction solution was then shaken, and the supernatant was discarded; such operations were repeated three times. Methyl tert-butyl ether (80 mL) and a small amount of n-hexane (10 mL) were then added, the reaction solution was then shaken, and the supernatant was discarded; the above operations were repeated three times, and the powdery solid in the reaction solution was separated out by precipitation; suction filtering was carried out, the filter cake was washed with methyl tert-butyl ether (40 mL × 3) and then collected, and dissolved with a mixed solution of methanol/dichloromethane (1:4) (100 mL); silica gel powder (20 g) was then added to the obtained solution and the solution was evaporated to be powder; the operations of dry sample loading, column chromatography and gradient elution with a mixed solution (3%-15% methanol/dichloromethane) were carried out; the elution product was collected, concentrated and dried in a vacuum oven, thus obtaining the product (0.95 g, 37.25%).
49-166

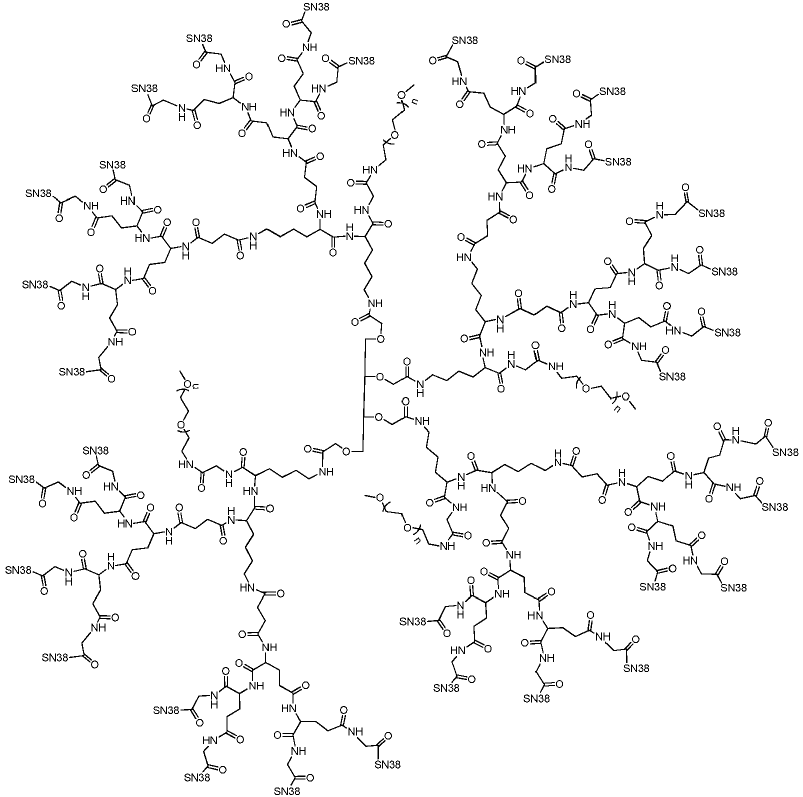

Product 49-162 (0.95 g, 0.0199 mmol) in a 500 ml flask was dissolved by ultrasonic with THF (10 ml) and dilute hydrochloric acid (10 ml, 0.05 mmol/L); TBAF (0.5 g, 1.9133 mmol) was then added, and the obtained solution was stirred in the dark at room temperature for 3 h to react. At the end of the reaction, the reaction solution evaporated to dryness and then dissolved with DMF (5 ml); isopropanol was added for precipitation, and the above operations were repeated three times; the obtained solution was then dissolved with anhydrous ethanol and a small amount of dichloromethane; methyl tert-butyl ether was added for precipitation, and the above operations were repeated three times; the resulting solid was collected, and dried in a vacuum oven, thus obtaining the product (0.75 g, 93.75%).

¹H-NMR (600 MHz, DMSO-*d*₆) δ8.44 - 8.22 (m, 24H), 8.05 - 7.89 (m, 46H), 7.44 - 7.27 (m,78H), 5.53 - 5.34 (m, 50H), 5.31 - 5.08 (m, 52H), 4.55 - 4.44 (m, 15H), 4.29 - 4.13 (m, 31H), 4.11 - 3.89 (m, 71H), 3.89 - 3.80 (m,23H), 3.78 - 3.60 (m, S2H), 3.55 -3.44(m,1892H),3.11 - 2.75 (m, 100H), 2.36 - 2.21 (m, 28H), 2.20 - 1.97 (m,91H), 1.61 - 1.49 (m, 34H), 1.31 - 1.16(m,136H), 0.97 - 0.91(m, 30H), 0.90-0.76(m,79H).

### Synthesis of 39-226

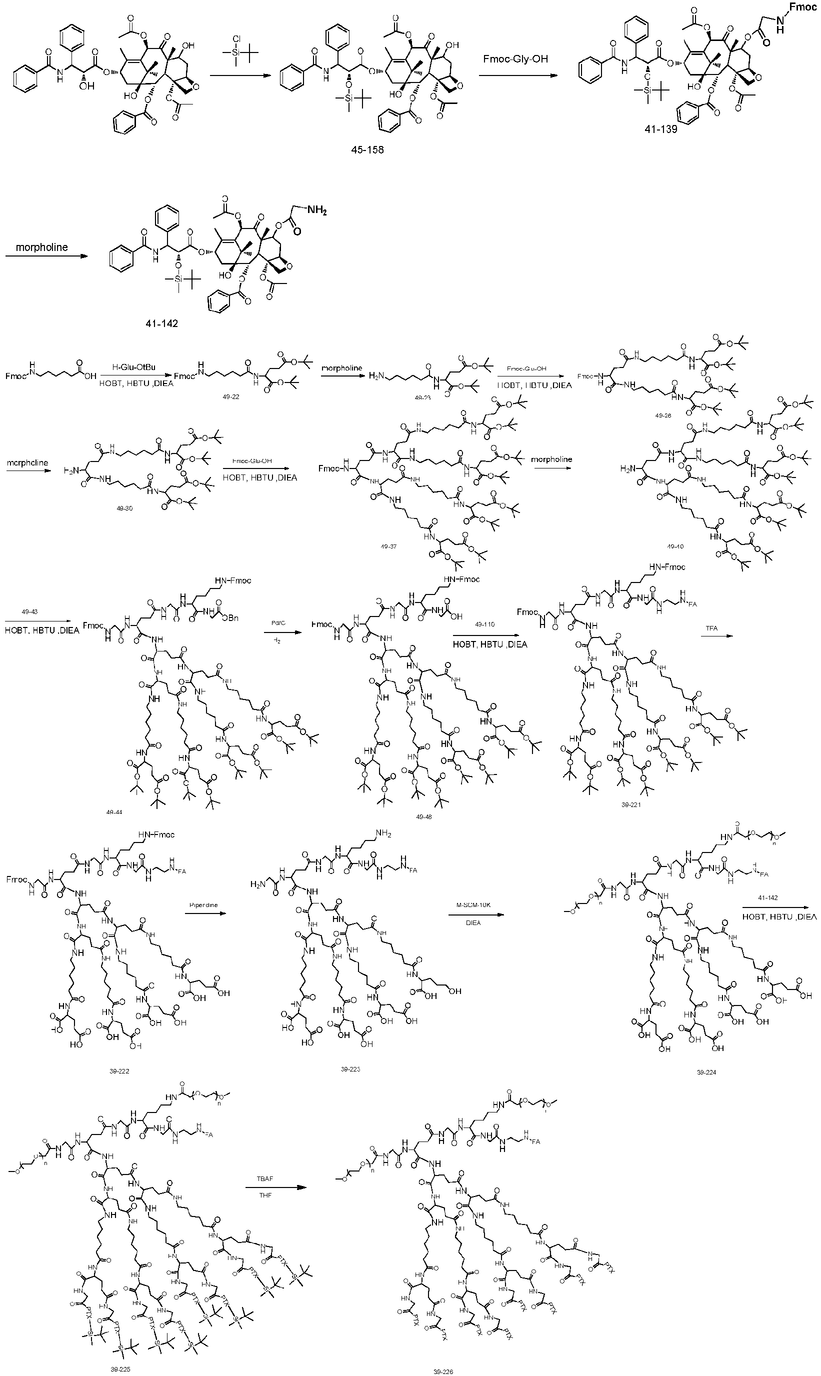

45-158

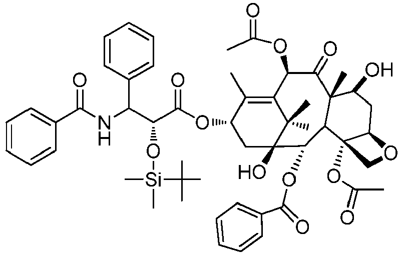

PTX (10 g, 11.71 mmol) and imidazole (3.986 g, 58.5 mmol) were weighed and added to a 500 mL flask and then dissolved with DMF (100 mL); DCC (10.59 g, 70.26 mmol) was added under the protection of N₂, and the reaction solution was stirred at room temperature to react. At the end of the reaction, extraction with a saturated ammonium chloride solution and dichloromethane was carried out; the organic phase was concentrated, and evaporated to dryness, thus obtaining 11.3 g of the product.
41-139

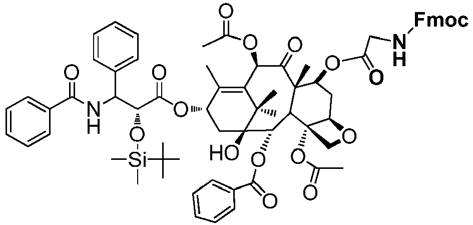

Product 45-158 (11.34 g, 11.71 mmol), Fmoc-Gly-OH (3.48 g, 11.71 mmol) and DMAP (0.285, 2.34 mmol) were added in a 500 mL round-bottomed flask and then dissolved with anhydrous dichloromethane (50 mL) and anhydrous DMF (25 mL); then DCC was added at 0 °C, and the mixed solution was stirred at room temperature for 30 min to react. After the reaction was completed, the reaction solution was diluted with dichloromethane, washing with saturated NH₄Cl was carried out once, and the organic phases were collected; washing with deionized water was carried out once, and the organic phases were collected; and washing with saturated sodium chloride was carried out once, and the organic phases were collected, and then dried with anhydrous sodium sulfate, the operations of dry sample loading, column chromatography, and elution with 3%methanol/dichloromethane were carried out; the elution product was then collected, concentrated and dried, thus obtaining 14.1 g of the product with a yield of 90 %
41-142

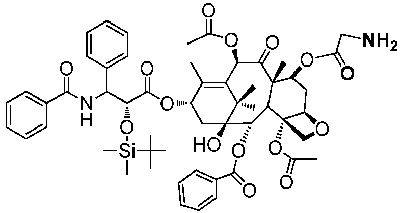

Product 41-139 (14.1 g, 11.3 mmol) in a flask was dissolved completely with DMF by ultrasonic vibration; morpholine (19.6 mL, 226 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL*3), the obtained organic phases were combined, evaporated into a solid, and the solid product was then dissolved with a mixed solution of methanol/dichloromethane (1/4) (100 mL); silica gel powder (100 mL) was then added to the obtained solution and the solution was evaporated to be powder; the operations of dry sample loading, column chromatography and gradient elution with a mixed solution (1% ammonia water/3%-5% methanol/dichloromethane) were carried out; the elution product was collected, concentrated and dried in a vacuum oven. 9.3 g of the product was obtained with a yield of 93%.
49-22

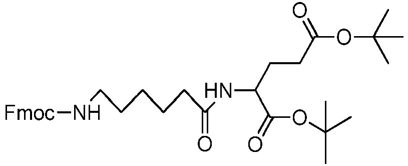

H-Glu(OtBu)-OtBu·HCl (4.08 g, 13.8116 mmol, purchased from Leyan), HBTU (7.85 g, 20.7174 mmol) and HOBT (2.80 g, 20.7174 mmol) were weighed and added in a flask with Product 49-17 (3.5 g, 9.9035 mmol) and then dissolved with an appropriate amount of DMF, and the obtained solution was placed at -5 °C; then, DIEA (10.27 mL, 62.1522 mmol) was slowly added dropwise; after addition and reaction for 30 min, the reaction solution was taken out, and the obtained solution was stirred overnight at room temperature to react. At the end of the reaction, the dichloromethane in the reaction solution was evaporated to dryness; a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and dried in a vacuum oven, thus obtaining the product (9.73 g being extra-quota product).
49-23

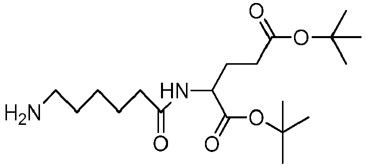

Product 49-22 (5.89 g, 9.9035 mmol) in a flask was dissolved completely with DMF by ultrasonic vibration; morpholine (8.62 mL, 99.035 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and the solid product was then dissolved with a mixed solution of methanol/dichloromethane (1:4) (100 mL); silica gel powder (100 mL) was added to the obtained solution, and the solution was then evaporated to dryness to obtain a powdery solid; the operations of dry sample loading, column chromatography, and elution with a mixed solution (1% ammonia water/3% methanol/dichloromethane) were carried out; the elution product was then collected, concentrated, and dried in a vacuum oven. The Product (3.48 g, 94.56 %) was obtained. 49-28

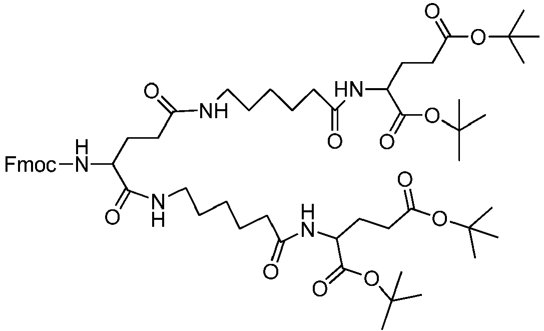

Product 49-23 (3.48 g, 9.3423 mmol), HBTU (5.06 g, 13.3461 mmol) and HOBT (1.80 g, 13.3461 mmol) were weighed and added in a flask with Fmoc-Glu-OH (1.64 g, 4.4487 mmol) and then dissolved with an appropriate amount of DMF, and the obtained solution was placed at -5 °C; then, DIEA (6.62 mL, 40.0383 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and dried in a vacuum oven, thus obtaining the product (6.5 g being extra-quota product).
49-30

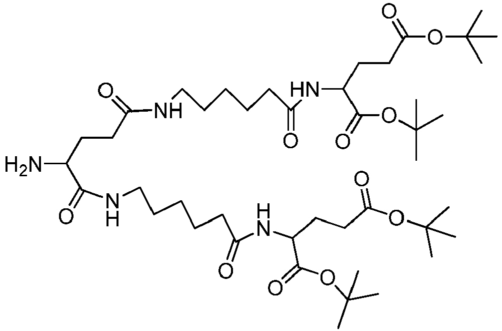

Product 49-28 (4.80 g, 4.4487 mmol) in a flask was dissolved completely with DMF by ultrasonic vibration; morpholine (3.87 mL, 44.487 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (5.8 g being extra-quota product).
49-37

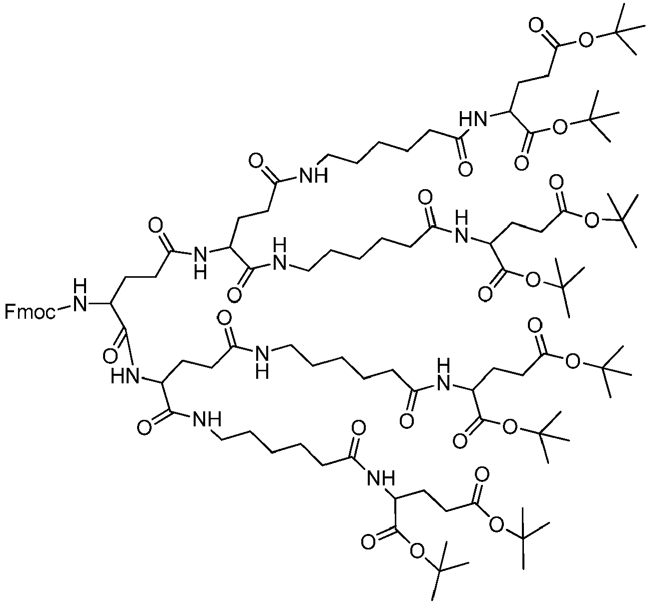

Fmoc-Glu-OH (0.71 g, 1.9342 mmol), HBTU (2.2 g, 5.8027 mmol) and HOBT (0.78 g, 5.8027 mmol) were weighed and added in a flask with Product 49-30 (3.8 g, 4.4487 mmol) and then dissolved with an appropriate amount of DMF, and the obtained solution was placed at -5 °C; then, DIEA (2.88 mL, 17.4079 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (4.5 g being extra-quota product).
49-40

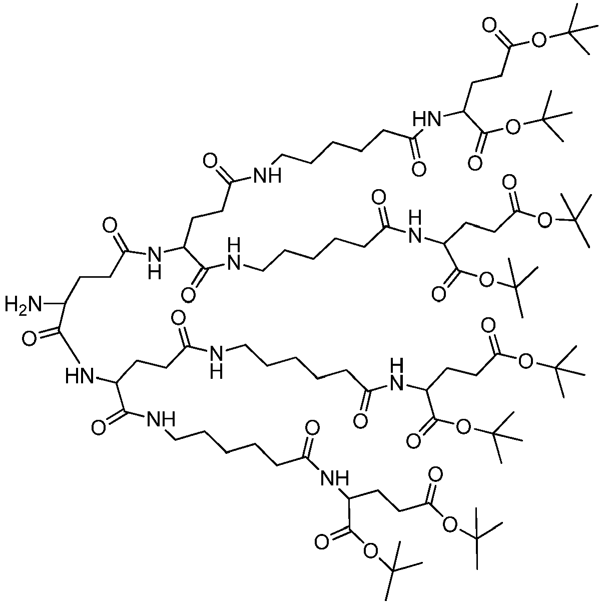

Product 49-37 (3.96 g, 1.9342 mmol) in a flask was dissolved completely with DMF by ultrasonic vibration; morpholine (1.68 mL, 19.342 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and the solid product was then dissolved with a mixed solution of methanol/dichloromethane (1:4) (100 mL); silica gel powder (100 mL) was then added to the obtained solution and the solution was evaporated to be powder; the operations of dry sample loading, column chromatography and gradient elution with a mixed solution (1% ammonia water/3%-5% methanol/dichloromethane) were carried out; the elution product was collected, concentrated and dried in a vacuum oven, thus obtaining the product (1.7668 g, 50.47 %).
49-44

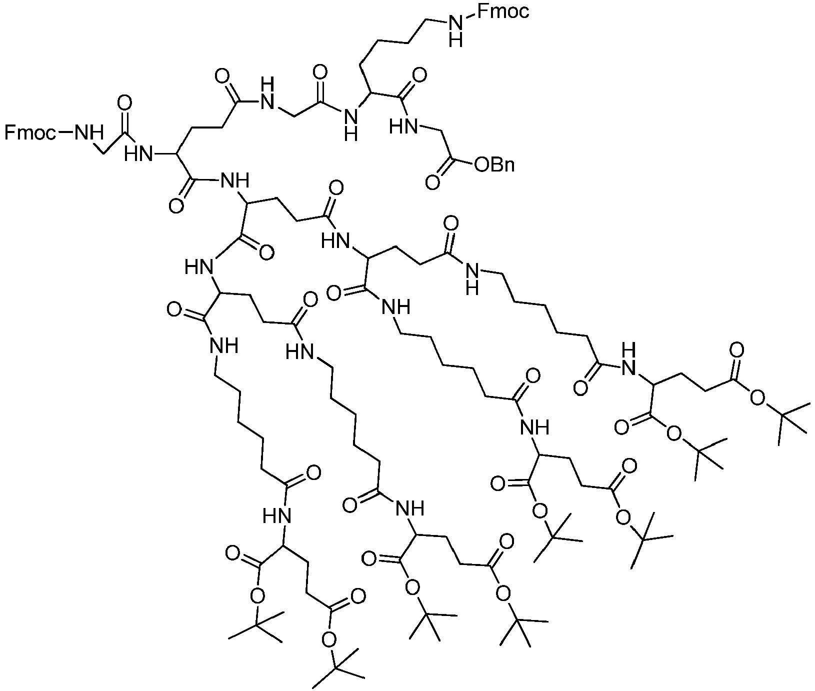

Product 49-40 (0.77 g, 0.4206 mmol), HBTU (0.24 g, 0.6308 mmol) and HOBT (0.08 g, 0.6308 mmol) were weighed and added in a flask with Product 49-43 (0.49 g, 0.5047 mmol) and then dissolved with an appropriate amount of DMF, and the mixed solution was placed at -5 °C; then, DIEA (0.1 mL, 1.8925 mmol) was slowly added dropwise; after reaction for 30 min, the reaction solution was taken out, and the obtained solution was stirred overnight at room temperature to react. At the end of the reaction, n-hexane (25 mL) and methyl tert-butyl ether (200 mL) were first added for precipitation, and the supernatant was discarded; n-hexane and methyl tert-butyl ether were added again for precipitation; such operations were repeated three times, and the filtering was then carried out to obtain a solid product, and the obtained solid product was dissolved with a mixed solvent (dichloromethane and methanol), the operations of dry sample loading, column chromatography, and elution with a mixed solution (3-5% methanol/dichloromethane) were carried out; the elution product was collected, concentrated and dried in a vacuum oven. The product (0.68 g, 58.12%) was obtained.

¹H-NMR (600 MHz, DMSO-*d*₆) δ 8.46 - 7.96 (m, 12H), 7.93- 7.83 (m, 4H), 7.79 - 7.59 (m, 6H), 7.53 (s, 1H), 7.46 - 7.17 (m, 14H), 5.11 (s, 2H), 4.32 - 4.06 (m, 15H), 3.95 - 3.78 (m,3H), 3.11-2.90 (m, 11H), 2.26 - 2.03 (m, 23H), 1.92 - 1.66 (m, 16H), 1.47 (s, 10H), 1.38 (s, 72H), 1.29-1.10 (m, 25H). 49-48

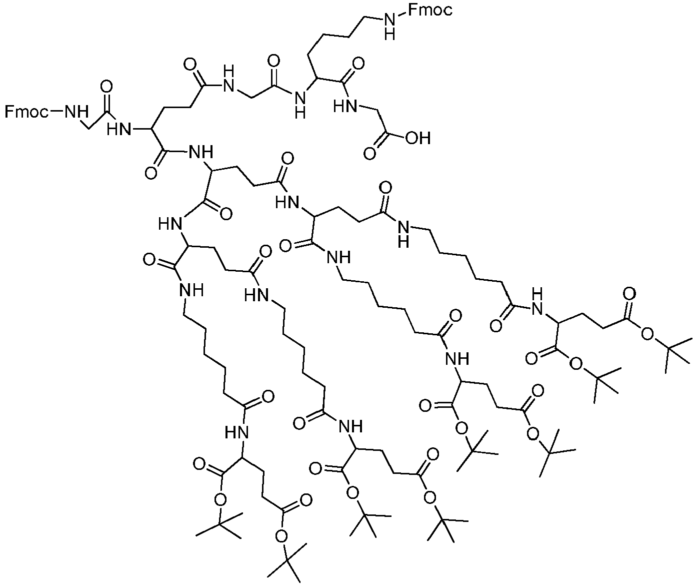

Product 49-44 (0.68 g, 0.2440 mmol) was added into a hydrogenation reactor and then 10% Pd/C (0.05 g) and DMF (20 mL) were added; hydrogen was introduced to a pressure of 300 Psi in the reactor, and the reaction solution was stirred overnight at room temperature to react. At the end of the reaction, the reaction solution was filtered with diatomaceous earth, and the filter cake was washed three times with DMF (15 mL × 3); the elution solution was added in a 250 mL round-bottomed flask, and the product was obtained, as the raw material for the next step. 39-221

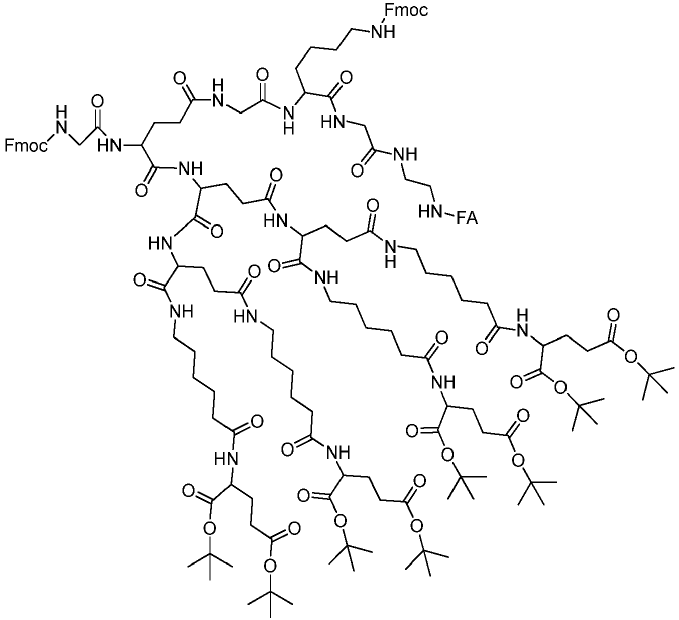

Product 49-48 (0.79 g, 0.2440 mmol) in a flask was dissolved completely with DMF by ultrasonic vibration; Product 49-110 (0.1 g, 0.2440 mmol), HBTU (0.138 g, 0.366 mmol) and HOBT (0.0.049 g, 0.366 mmol) were added, and the reaction solution was stirred at room temperature to react. At the end of the reaction, n-hexane (25 mL) and methyl tert-butyl ether (200 mL) were first added for precipitation, and the supernatant was discarded; n-hexane and methyl tert-butyl ether were added again for precipitation; such operations were repeated three times, and the filtering was then carried out to obtain a solid product, and the obtained solid product was dissolved with a mixed solvent (dichloromethane and methanol), the operations of dry sample loading, column chromatography, and elution with a mixed solution (1% ammonia water/8-12% methanol/dichloromethane) were carried out; the elution product was collected, concentrated and dried in a vacuum oven. The product was obtained. 39-222

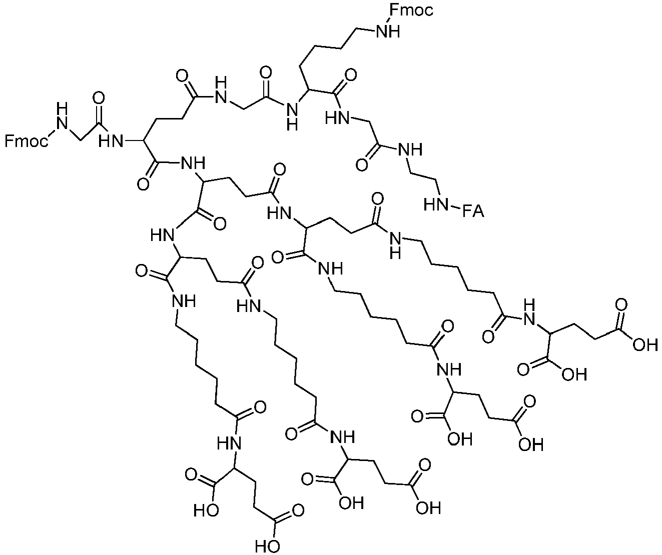

Product 39-221 (0.1248 mmol) were weighed and added to a 250 mL flask and then dissolved with an appropriate amount of dichloromethane (5 mL) and TFA (2.22 mL, 29.952), and the mixed solution was stirred to react. At the end of the reaction, n-hexane (25 mL) and methyl tert-butyl ether (200 mL) were first added for precipitation, and the supernatant was discarded; n-hexane and methyl tert-butyl ether were added again for precipitation; such operations were repeated three times, suction filtering was carried out, and the solid product was collected, thus obtaining the product (0.35 g). 39-223

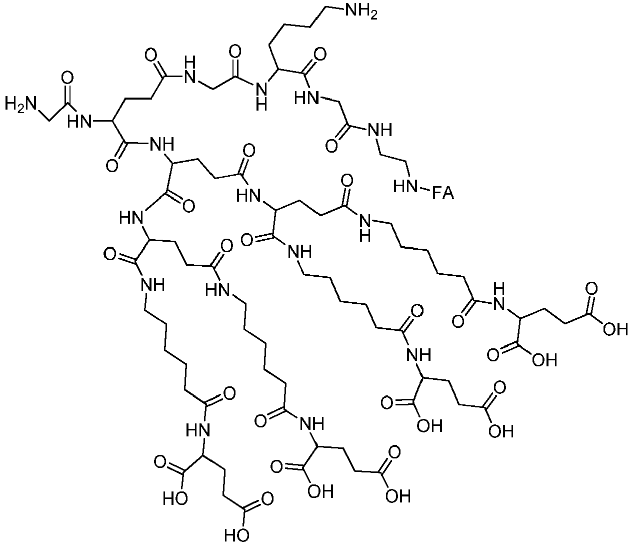

Product 39-222 (0.1248 mmol) was weighed and added to a 250 mL flask and then dissolved with morpholine (2.6 mL, 29.952), and the mixed solution was stirred to react. At the end of the reaction, n-hexane (25 mL) and methyl tert-butyl ether (200 mL) were first added for precipitation, and the supernatant was discarded; n-hexane and methyl tert-butyl ether were added again for precipitation; such operations were repeated three times, suction filtering was carried out, and the solid product was collected, thus obtaining the product (0.35 g). 39-224

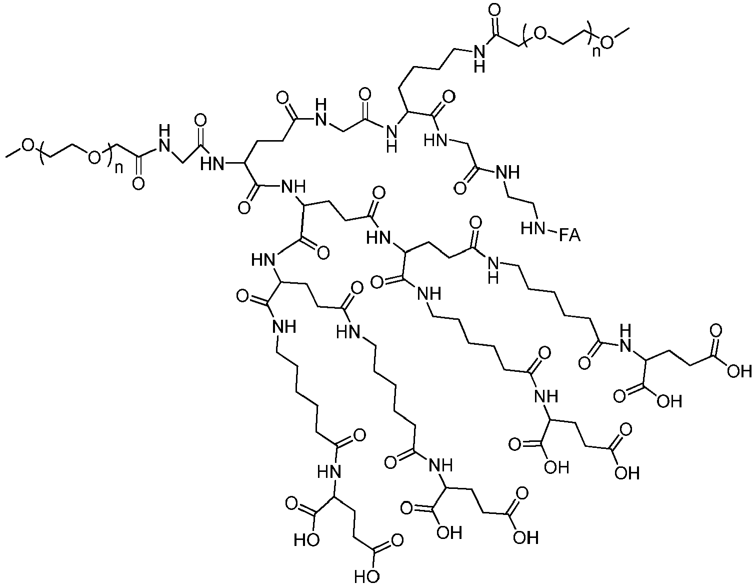

Product 39-223 (0.35 g, 0.1248 mmol) was added in a 250 mL flask and then dissolved with DMF (20 mL), and M-SCM-10K (2.75 g, 0.2745 mmol, purchased from JenKem) was added and dissolved by ultrasonic vibration; DIEA (1 mL) was added and the obtained solution was slowly stirred at room temperature to react for one week in the dark. After the reaction was completed, n-hexane (25 mL) and methyl tert-butyl ether (200 mL) were first added for precipitation, and the supernatant was discarded; n-hexane and methyl tert-butyl ether were added again for precipitation; such operations were repeated three times, and the filtering was then carried out to obtain a solid product, and the obtained solid product was dissolved with a mixed solvent (dichloromethane and methanol); the operations of dry sample loading, column chromatography, and gradient elution with 1% ammonia water/6% methanol/dichloromethane-1%ammonia water:10% methanol/dichloromethane were carried out, the elution product was collected, concentrated and dried in a vacuum oven, thus obtaining the product (1.38 g). 39-225

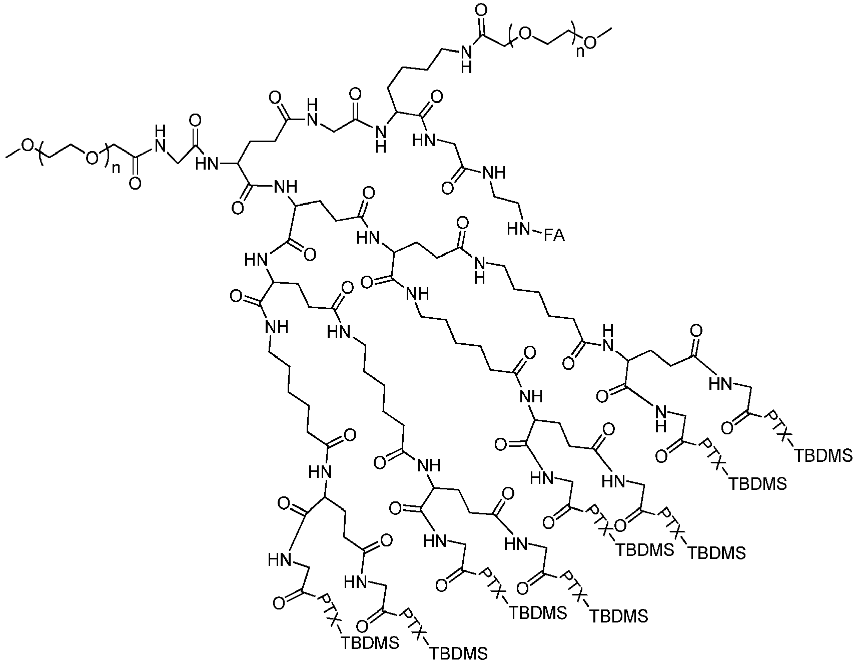

Product 39-224 (1.38 g, 0.0625 mmol), HBTU (0.035 g, 0.0938 mmol) and HOBT (0.012 g, 0.0938 mmol) were weighed and added in a flask with Product 41-142 (1.66 g, 0.6250 mmol) and then dissolved with an appropriate amount of DMF, and the mixed solution was placed at -5 °C; DIEA (0.046 mL, 0.2813 mmol) was slowly added dropwise; after addition and reaction for 30 min, the reaction solution was taken out, and the obtained solution was stirred overnight at room temperature to react. At the end of the reaction, n-hexane (25 mL) and methyl tert-butyl ether (200 mL) were first added for precipitation, and the supernatant was discarded; n-hexane and methyl tert-butyl ether were added again for precipitation; such operations were repeated three times, suction filtering was carried out, and the solid product was collected, thus obtaining the product (0.76 g, 40.21%).

¹H-NMR (600 MHz, DMSO-*d*₆) δ 7.99 - 7.37 (m, 133H), 7.24 - 7.11 (m, 20H), 6.74 - 6.60 (m, 10H), 5.46 - 5.38 (m, 16H), 4.98 - 4.92 (m, 8H), 4.80 - 4.73 (m, 16H), 4.31 - 4.17 (m, 19H), 4.03 (s, 21H), 3.51 - 3.50 (m, 1836H), 3.24 - 3.23 (m, 10H), 3.18 - 2.57 (m, 16H), 2.48 - 1.71 (m, 130H), 1.66 - 1.49 (m, 72H), 1.48 - 1.39 (m, 29H), 1.04 - 0.94 (m, 48H), 0.79 (s, 73H), 0.03 (s, 48H). 39-226

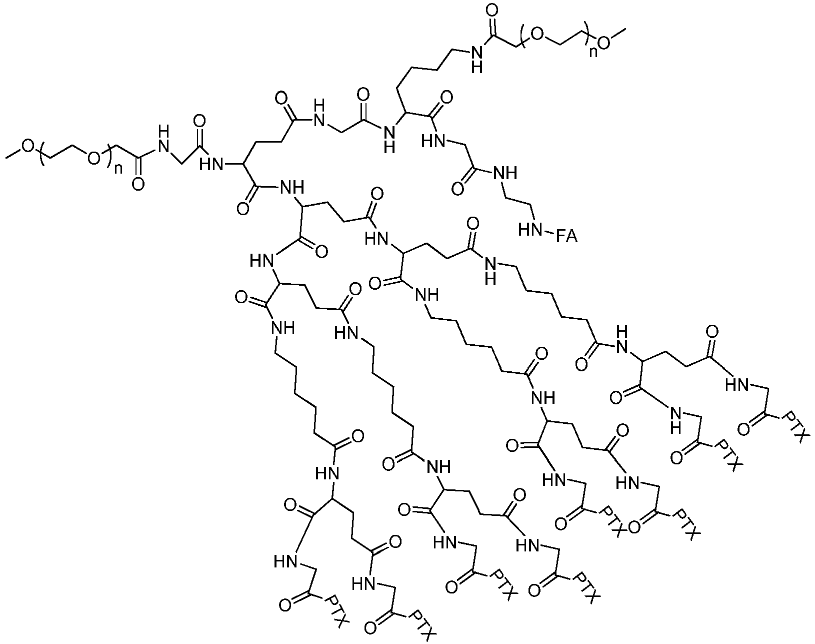

Product 39-225 (0.76 g, 0.0251 mmol) and TBAF (0.13 g, 0.5228 mmol) were added in a 500 mL round-bottomed flask and then dissolved with THF (10 mL), and the reaction solution was stirred at room temperature for 1.5 h to react. After the reaction was completed, the reaction solution was concentrated under reduced pressure, and the solid product was obtained, and dissolved with DMF (5 mL); isopropanol was added for precipitation, and the above operations were repeated three times; the solid product was obtained by filtering, and dissolved with dichloromethane (10 mL); methyl tert-butyl ether was added for precipitation, and such operations were repeated three times, and a solid was obtained; the obtained solid was dried, thus obtaining the product (0.65 g, 89.04%). ¹H-NMR (600 MHz, DMSO-*d*₆) δ 8.17 - 7.86 (m, 59H), 7.75 - 7.19 (m, 104H), 5.43 - 5.40 (m, 16H), 4.95 (s, 8H), 4.86 - 4.50 (m, 16H), 4.32 - 3.83 (m, 44H), 3.52 - 3.50 (m, 1978H), 3.24 - 3.23 (m, 10H), 3.01 - 2.87 (m, 16H), 2.42 - 2.02 (m, 120H), 1.95 - 1.59 (m, 119H), 1.05 - 0.96 (m, 48H).

### Synthesis of Product 45-145:

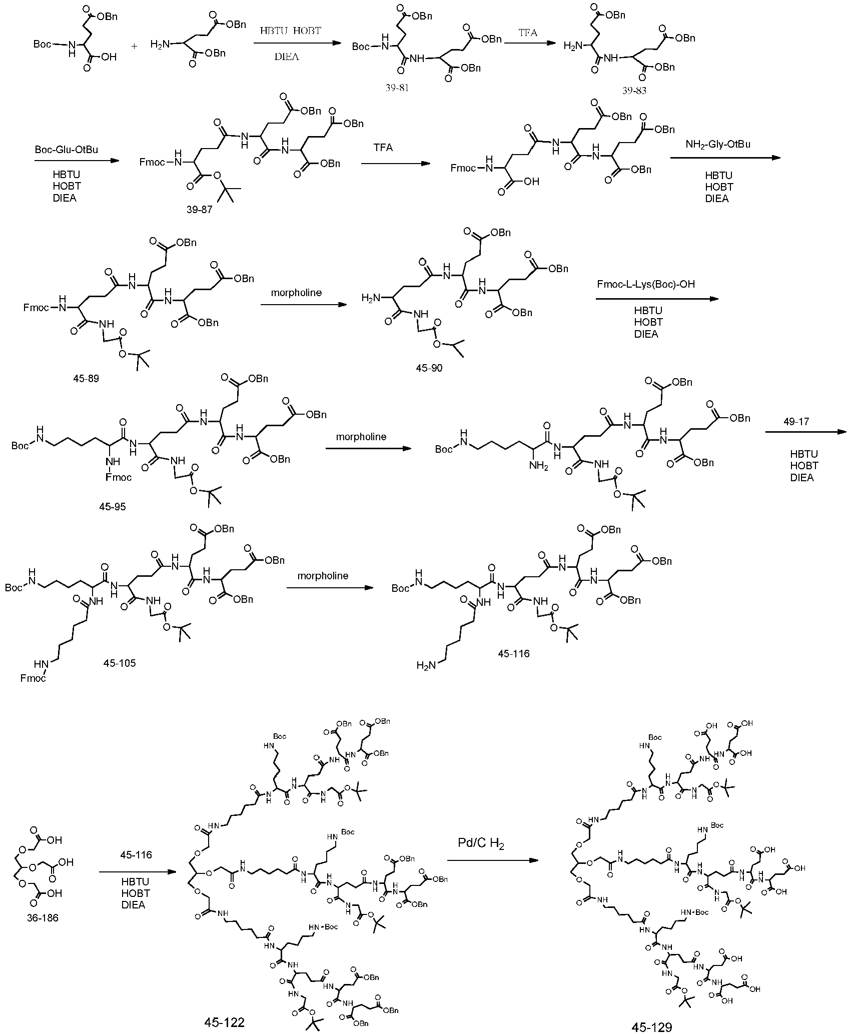

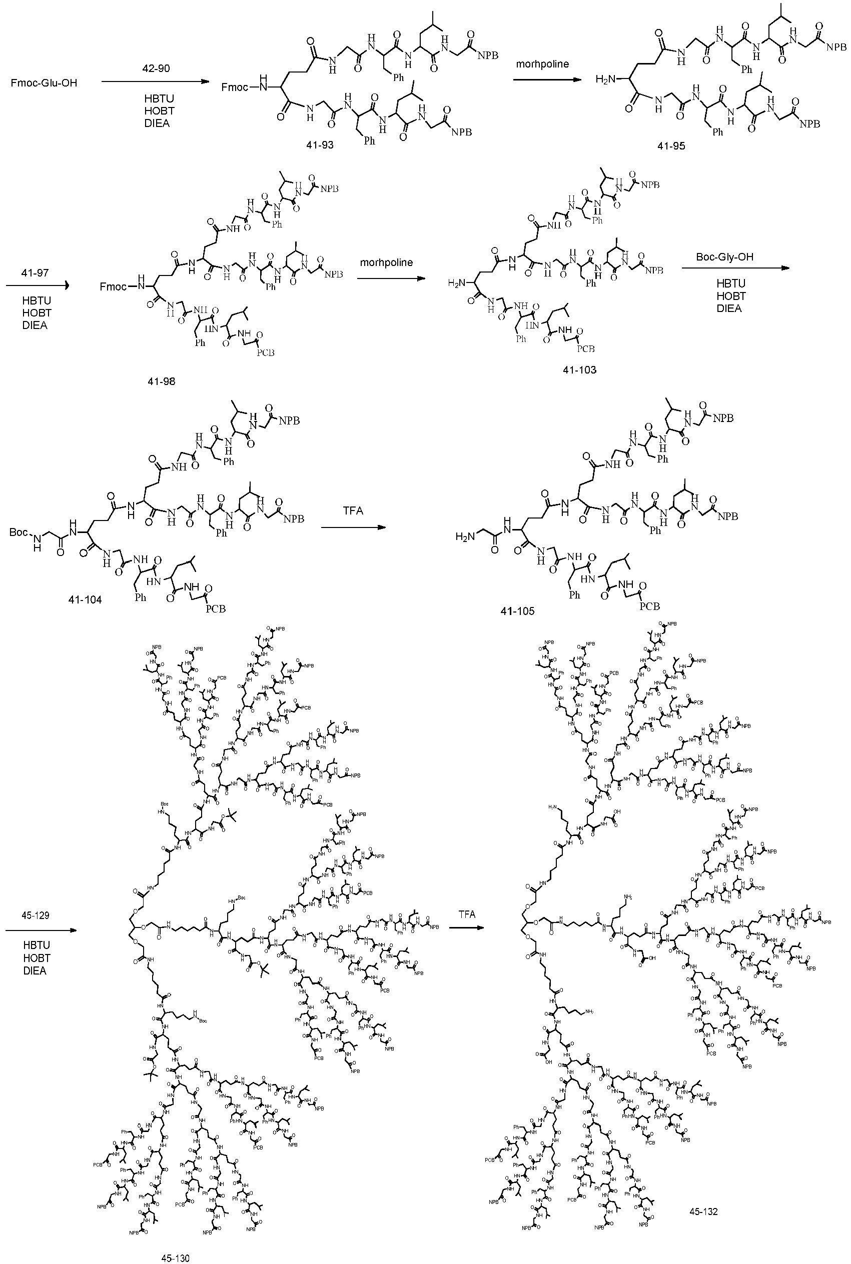

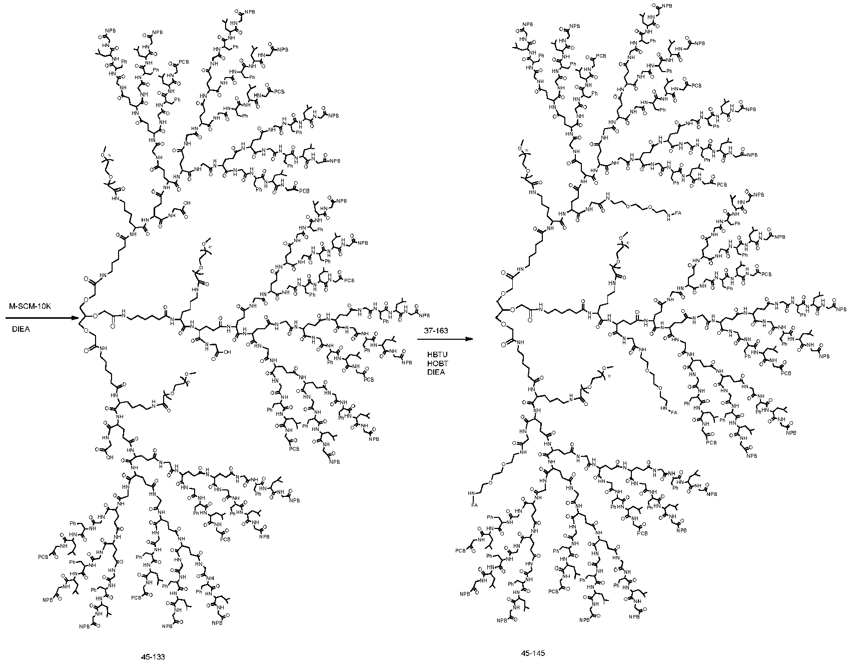

41-92

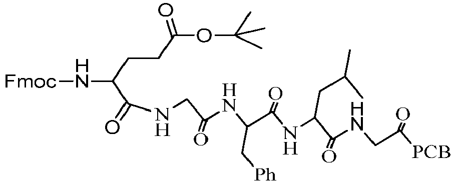

Fmoc-Glu(OtBu)-OH (3.3522 g, 7.7879 mmol, purchased from Ark Pharm), Product 36-98 (6.8 g, 8.2728 mmol), HBTU (4.7061 g, 12.4092 mmol) and HOBT (1.6767 g, 12.4092 mmol) were added in a 250 mL flask and then dissolved with DMF (20 mL), and the obtained solution was stirred at low temperature (-5 °C) and constant temperature for 30 min to react. Then DIEA (6.2 mL, 37.2276 mmol) was slowly added dropwise, and then the mixed solution was further stirred at this temperature for 3 h to react. At the end of the reaction, methyl tert-butyl ether (30 mL) and n-hexane (200 mL) were added to precipitate, suction filtering was carried out, and the solid powder was transferred to a 500 mL round-bottomed flask, and dissolved with 20% methanol/dichloromethane; silica gel powder was added to the obtained solution, and the solution was then evaporated to dryness, column chromatography, and elution with 2% methanol/dichloromethane were carried out. 8.9 g of the product was obtained with a yield of 100%. 41-97

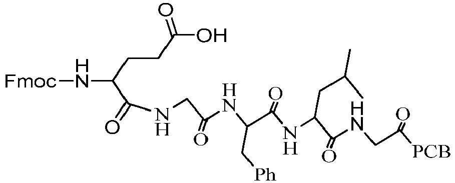

Product 41-92 (8.9 g, 7.2447 mmol) and dichloromethane (20 mL) were added in a flask, and then TFA (8 mL, 108.6709 mmol) was added, and the mixed solution was stirred at room temperature for 3 h to react. At the end of the reaction, the reaction solution was first evaporated to remove dichloromethane; methyl tert-butyl ether (30 mL) and n-hexane (200 mL) were added to precipitate, suction filtering was carried out, and the solid powder was transferred to a 500 mL round-bottomed flask, and evaporated to dryness, thus obtaining 8.5 g of the product with a yield of 100%. 41-93

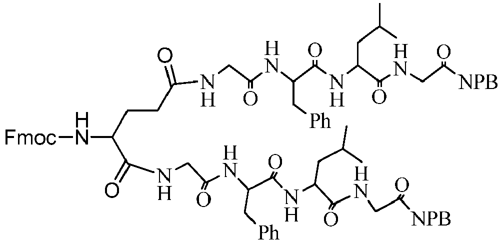

Fmoc-Glu-OH (1.6433 g, 4.4485 mmol, purchased from Aladdin), GFLG-NPB (synthesized according to the synthesis method of Product 42-90, 6.8 g, 9.7867 mmol), HBTU (5.0615 g, 13.3455 mmol) and HOBT (1.8032 g, 13.3455 mmol) were added in a 250 mL flask and then dissolved with DMF (50 mL), and the mixed solution was stirred at a low-temperature constant temperature for 30 min to react. DIEA (6.6 mL, 40.0365 mmol) was slowly added dropwise, and the mixed solution was further stirred at this temperature for 3 h to react. At the end of the reaction, methyl tert-butyl ether (30 mL) and n-hexane (200 mL) were added to precipitate, suction filtering was carried out, and the solid powder was transferred to a 500 mL round-bottomed flask, and evaporated to dryness, thus obtaining 10.2 g of the product with a yield of 100%. 41-95

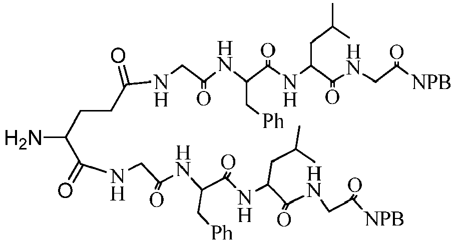

Product 41-93 (7.6 g, 4.4485 mmol) was dissolved with DMF (40 mL), and then morpholine (7.8 mL, 88.97 mmol) was added, and the mixed solution was stirred at room temperature for 1 h to react. At the end of the reaction, ethyl acetate (100 mL) and n-hexane (200 mL) were added to precipitate; suction filtering was then carried out, and the solid powder was transferred to a 500 mL round-bottomed flask; silica gel powder was added to the obtained solution, and the solution was then evaporated to dryness for column chromatography, and elution with 3% methanol/dichloromethane were carried out. 4.4 g of the product was obtained with a yield of 65.7%. 41-98

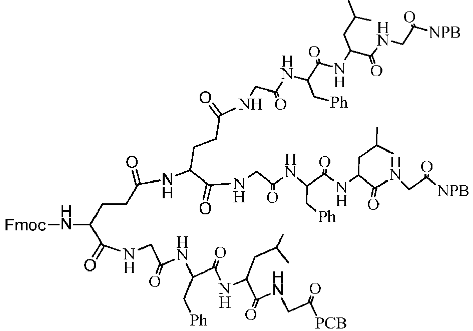

Product 41-95 (4.4 g, 2.9319 mmol), Product 41-97 (3.6121 g, 3.0785 mmol), HBTU (1.6679 g, 4.3979 mmol) and HOBT (0.5492 g, 4.3979 mmol) were added in 250 mL flask and then dissolved with DMF (20 mL), and the mixed solution was stirred at low temperature (0 °C) and constant temperature for 30 min to react. DIEA (2.2 mL, 13.1936 mmol) was slowly added dropwise, and the mixed solution was further stirred at this temperature for 3 h to react. At the end of the reaction, methyl tert-butyl ether (30 mL) and n-hexane (200 mL) were added to precipitate, suction filtering was then carried out, and the solid powder was transferred to a 2 L round-bottomed flask, and evaporated to dryness, thus obtaining 7.7873 g of the product. 41-103

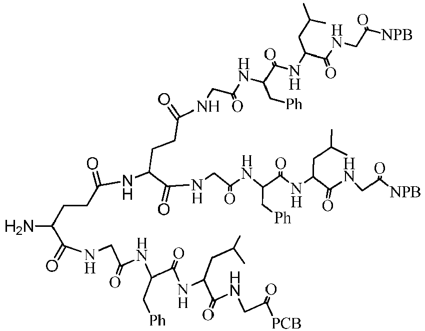

Product 41-98 (6.2 g, 2.3343) was dissolved with DMF (20 mL), and then morpholine (6 mL, 70.0298 mmol) was added, and the mixed solution was stirred at room temperature for 1 h to react. At the end of the reaction, methyl tert-butyl ether (100 mL) and n-hexane (200 mL) were added to precipitate, suction filtering was then carried out, and the solid powder was transferred to a 500 mL round-bottomed flask, and evaporated to dryness, thus obtaining 5.68 g of the product. 41-104

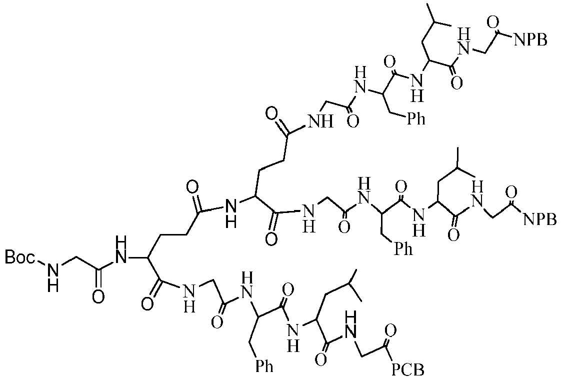

Product 41-103 (5.5 g, 2.2598 mmol), Boc-Gly-OH (0.4157 g, 2.3728 mmol, purchased from Aladdin), HBTU (1.2855 g, 3.3897 mmol) and HOBT (0.4580 g, 3.3897 mmol) were added in 250 mL flask and then dissolved with DMF (20 mL), and the mixed solution was stirred at low temperature (-5 °C) and constant temperature for 30 min to react. DIEA (1.7 mL, 10.1692 mmol) was slowly added dropwise, and the mixed solution was further stirred at this temperature for 3 h to react. At the end of the reaction, ethyl acetate (30 mL) and n-hexane (200 mL) were added to precipitate, suction filtering was then carried out, and such operations were repeated multiple times, and the solid powder was transferred to a 2L round-bottomed flask, and evaporated to dryness for the next reaction. 41-105

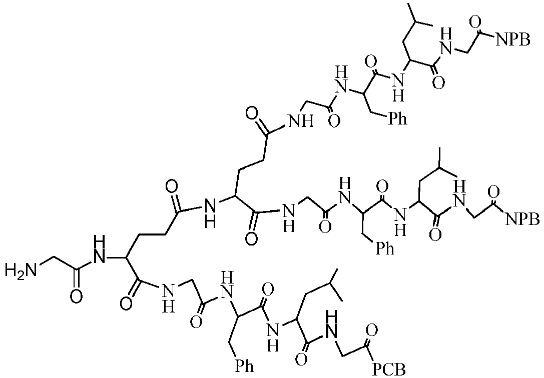

Product 41-104 (5.8 g, 2.2598 mmol) and dichloromethane (20 mL) were added in a flask, and TFA (5 mL, 67.2110 mL) was slowly added dropwise, and the mixed solution was stirred at room temperature for 3 h to react. At the end of the reaction, dichloromethane was removed with a rotary evaporator; ethyl acetate (30 mL) and n-hexane (200 mL) were added to precipitate, suction filtering was then carried out, and the above operations were repeated multiple times; the solid powder was transferred to a 500 mL round-bottomed flask, and evaporated to dryness, thus obtaining 5.5 g of the product with a yield of 98%. 39-81

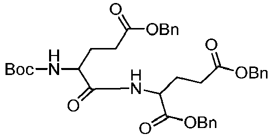

N-Boc-L-glutamic acid-5-benzyl ester (10 g, 29.6 mmoL, purchased from Aladdin), H-Glu (Obzl)-Obzl TsOH (16.2 g, 32.6 mmol, purchased from Ark Pharm), HOBT (6 g, 44.4 mmoL) and HBTU (16.8 g, 44.4 mmoL) were weighed and added to a 250 mL flask and then dissolved in DMF solution (80 mL); ultrasonic treatment was carried out to completely dissolve the reactants, and the obtained solution was stirred for 30 min at -5 °C; DIEA (22 mL, 133.4 mmoL) was slowly added dropwise, and the obtained solution reacted at this temperature until to the end. At the end of the reaction, the reaction solution was taken out, and deionized water (100 mL) was added to the reaction solution; extraction with ethyl acetate (100 mL × 3) were carried out several times, and the obtained organic phases were combined, washed twice with a saturated sodium chloride solution (100 mL × 2), and then concentrated and evaporated to dryness. The operations of dry sample loading, column chromatography and elution with 30% ethyl acetate/petroleum ether were carried out; the elution product was collected, then concentrated and evaporated to dryness, thus obtaining the product. 39-83

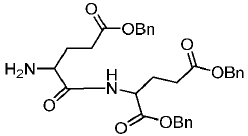

Product 39-81 (29.6 mmol) was weighed and dissolved by ultrasonic with dichloromethane (5 mL) and TFA (22 mL, 296 mmol), a ground glass stopper was used, and the obtained solution was stirred to react at room temperature. At the end of the reaction, the reaction solution was taken out; a saturated sodium bicarbonate solution (300 mL) was added to the reaction solution, extraction with ethyl acetate (100 mL × 3) were carried out several times, and the obtained organic phases were combined, washed twice with a saturated sodium chloride solution (100 mL), and concentrated and evaporated to dryness. 39-87

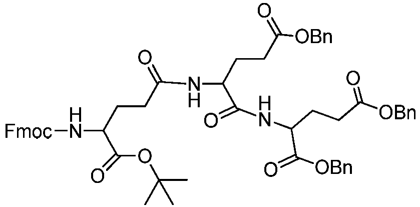

Boc-Glu(OtBu)-OH (12.6 g, 29.6 mmoL, purchased from Accela), Product 39-83 (29.6 mmol), HOBT (5.9 g, 44.4 mmoL) and HBTU (16.8 g, 133.2 mmoL) were weighed and added in a 250 mL flask and then dissolved in DMF solution (70 mL); ultrasonic treatment was carried out to completely dissolve the reactants, and the obtained solution was stirred for 30 min at -5 °C. DIEA (22 mL, 86.85 mmol) was slowly added dropwise, and the obtained solution reacted at a low temperature to the end. At the end of the reaction, the reaction solution was taken out; deionized water (100 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness. The operations of dry sample loading, column chromatography and gradient elution with 40%-50% ethyl acetate/petroleum ether were carried out; the elution product was collected, then concentrated and evaporated to dryness. 39-88

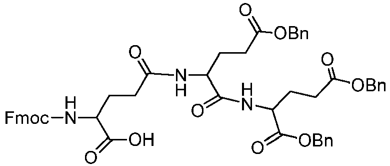

Product 39-87 (29.6 mmol) was weighed and dissolved by ultrasonic with dichloromethane (5 mL) and TFA (22 mL, 296 mmol), a ground glass stopper was used, and the obtained solution was stirred to react at room temperature. At the end of the reaction, the reaction solution was taken out; a saturated sodium bicarbonate solution (300 mL) was added to the reaction solution, and extraction with ethyl acetate (100 mL × 3) were carried out several times; the obtained organic phases were combined, washed twice with a saturated sodium chloride solution (100 mL), and then concentrated and evaporated to dryness, thus obtaining 26 g of the product. 45-89

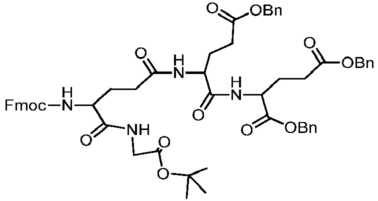

The reactant Product 39-88 (26 g, 29.6 mmol), NH₂-Gly-OtBu·HCl (4.96 g, 29.6 mmol, purchased from Accela), HBTU (16.8383 g, 44.4 mmol) and HOBT (5.999 g, 44.4 mmol) were added in a 250 mL flask and dissolved with DMF (30 mL), and the mixed solution was stirred at -5 °C for 30 min; DIEA (22 mL, 133.2 mmol) was slowly added dropwise, and the obtained solution reacted at a low temperature to the end. At the end of the reaction, the reaction solution was transferred to a 1 L separatory funnel; ethyl acetate (200 mL) and deionized water (150 mL) were added to separate the organic phase, and the aqueous phase was extracted twice with ethyl acetate (100 mL×2) until there was no product in the aqueous phase; the organic phases were combined, washed once with a saturated saline solution (100 mL), and then concentrated and evaporated to dryness, thus obtaining 29.9 g of the product. 45-90

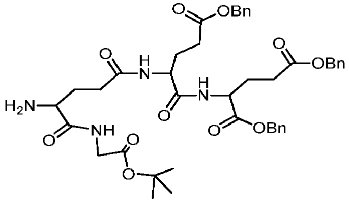

The reactant Product 45-89 (29.9 g, 29.6 mmol) was added in a 250 mL flask, and DMF solution (20 mL) and morpholine (63.162 mL, 725 mmol) were then added in sequence; and the mixed solution was stirred at room temperature to react. Three hours later, the reaction was completed, and the reaction solution was transferred to a 2 L separatory funnel; a saturated saline solution (150 mL) and ethyl acetate (200 mL) were added to separate the organic phase; the aqueous phase was extracted three times with ethyl acetate (50 mL×3) until there was no product in the aqueous phase; the obtained organic phases were combined, and washing with a saturated saline solution (50 mL×2) was carried out twice, and the organic phase was evaporated to dryness, thus obtaining 23 g of the product. 45-95

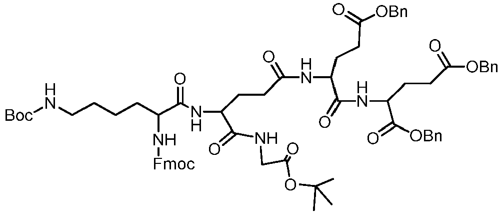

Fmoc-L-Lys(Boc)-OH (15.256 g, 32.56 mmol, purchased from Accela), Product 45-90 (23 g, 29.6 mmol), HBTU (16.838 g, 44.4 mmol) and HOBT (5.999 g, 44.4 mmol) were weighed and added in a 500 mL flask and then dissolved in DMF solution (50 mL), and the obtained solution was stirred at -5 °C for 30 min. DIEA (22.1 mL, 133.2 mmol) was slowly added dropwise, and the obtained solution was stirred at -5 °C overnight to react. At the end of the reaction, extraction with purified water and ethyl acetate was carried out, and the organic phase was concentrated; the operations of dry sample loading, column chromatography and gradient elution with 1%-4%methanol/dichloromethane were carried out. 25 g of the product was obtained. 45-103

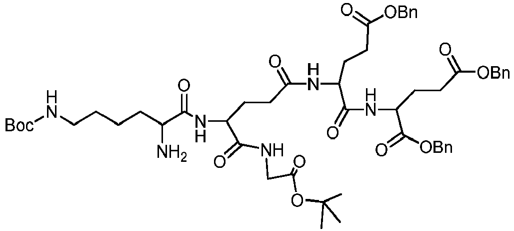

The reactant Product 45-95 (25 g, 20.17 mmol) was added in a 250 mL flask, and DMF solution (20 mL) and morpholine (35 mL, 403.3mmoL) were added, and the mixed solution was stirred at room temperature to react. Three hours later, the reaction ended. A saturated saline solution (150 mL) and ethyl acetate (200 mL) were added to separate the organic phase, the aqueous phase was extracted three times with ethyl acetate (50 mL×3) until there was no product in the aqueous phase; the obtained organic phases were combined, and washing with a saturated saline solution (50 mL×2) was carried out twice, and the organic phase was evaporated to dryness, column chromatography, and gradient elution with 2%-8% methanol/dichloromethane were carried out, thus obtaining 18.8 g of the product with a yield of 91.7%. 45-105

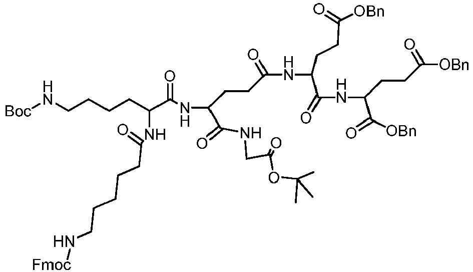

Product 49-17 (4 g, 8.822 mmol), Product 45-103 (8.97 g, 8.822 mmol), HBTU (5.018 g, 13.233 mmol) and HOBT (1.788 g, 13.233 mmol) were weighed and added in a 500 mL flask and then dissolved in DMF solution (40 mL), and the obtained solution was stirred at -5 °C for 30 min. DIEA (6.5869 mL, 39.699 mmol) was slowly added dropwise, and the obtained solution was stirred at -5 °C overnight to react. At the end of the reaction, purified water was added, and a solid product was precipitated; the obtained solid product was filtered by suction and dried, column chromatography, and gradient elution with 20%-50% ethyl acetate/petroleum ether and 0.5%-5% methanol/dichloromethane were carried out, thus obtaining 10.3 g of the product with a yield of 86%. 45-116

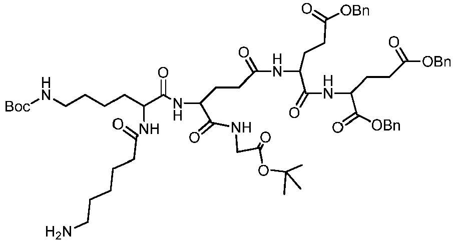

The reactant Product 45-105 (10.3 g, 7.615 mmol) was added in a 250 mL flask, and DMF solution (50 mL) and morpholine (13.27 mL, 152.3mmoL) were added in sequence, and the mixed solution was stirred at room temperature to react. At the end of the reaction, purified water was added to the reaction solution, suction filtering was carried out, and the obtained product was dried, thus obtaining 4.3 g of the product with a yield of 50%. 45-122

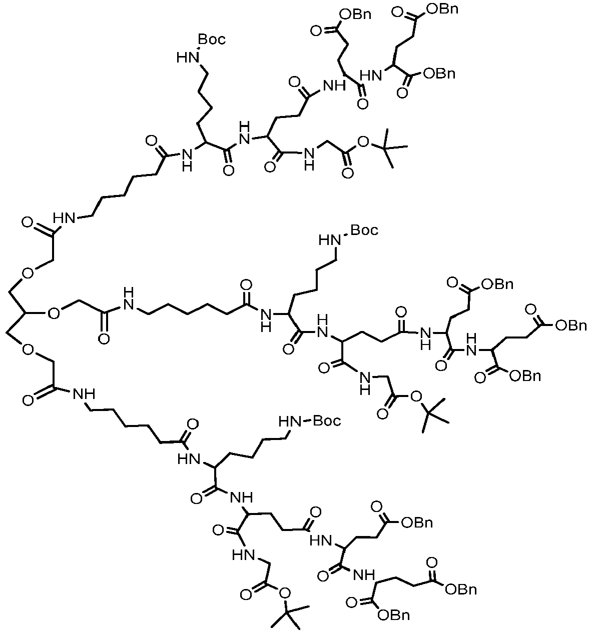

Product 45-116 (4.304 g, 3.808 mmol), HBTU (1.6247 g, 4.284 mmol) and HOBT (0.5789 g, 4.284 mmol) were weighed and added in a 250 mL flask and dissolved with the DMF solution of Product 36-186 (0.952 mmol, synthesized according to the synthesis method of Product 37-172, just replace pentaerythritol to glycerol), and the obtained solution was stirred at -5 °C for 30 min. DIEA (2.1 mL, 12.852 mmol) was slowly added dropwise, and the obtained solution was stirred at -5 °C overnight to react. At the end of the reaction, purified water was added to the reaction solution, and suction filtering was carried out; the operations of dry sample loading, column chromatography and elution with 4%methanol/dichloromethane were carried out. 2 g of the product was obtained with a yield of 58.82%.

¹H-NMR (600 MHz, DMSO-*d*₆) δ 8.45-8.44 (m, 2H), 8.17-8.16 (m, 2H), 7.95-7.94 (m, 5H), 7.86-7.85 (m, 3H), 7.75-7.74 (m, 2H), 7.35-7.30 (m, 49H), 6.72-6.70 (m, 2H), 5.10-5.08 (m, 16H), 4.53-4.52 (m, 4H), 4.32-4.30 (m, 6H), 4.21 -4.20(m, 5H), 3.86 (s, 4H), 3.75-3.73 (m, 3H), 3.67-3.65 (m, 2H), 3.06-3.04 (m, 5H), 2.86-2.83 (m, 6H), 2.44-2.37 (m, 22H), 2.11-2.04 (m, 14H), 1.89 - 1.88(m, 8H), 1.75-1.74 (m,5H), 1.62-1.60 (m, 3H), 1.54 - 1.14 (m, 88H). 45-129

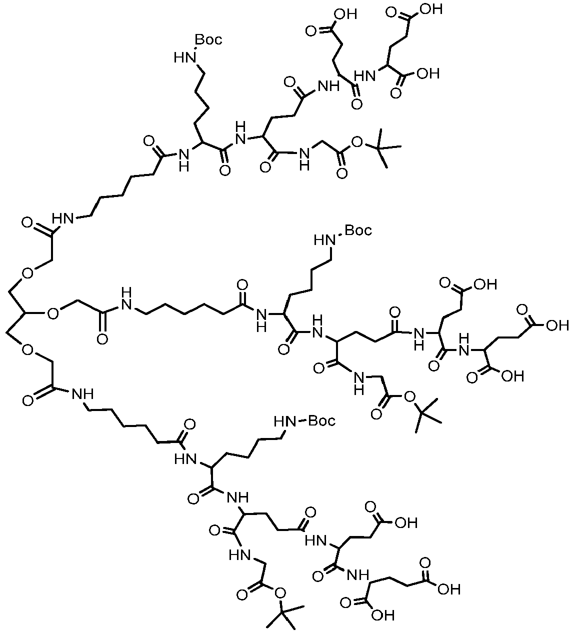

Product 45-122 (0.1736 g, 0.0850 mmol) and 10% Pd/C (75 mg) were added in a hydrogenation reactor and then DMF (35 mL) was slowly added with stirring until the reactant product was dissolved; hydrogen (300 psi) was introduced, and the obtained solution was stirred at room temperature overnight. Next day, the reaction solution was filtered with a sand core funnel filled with diatomaceous earth to remove Pd/C, thus obtaining the DMF solution of the product, for direct use in the next reaction. 45-130

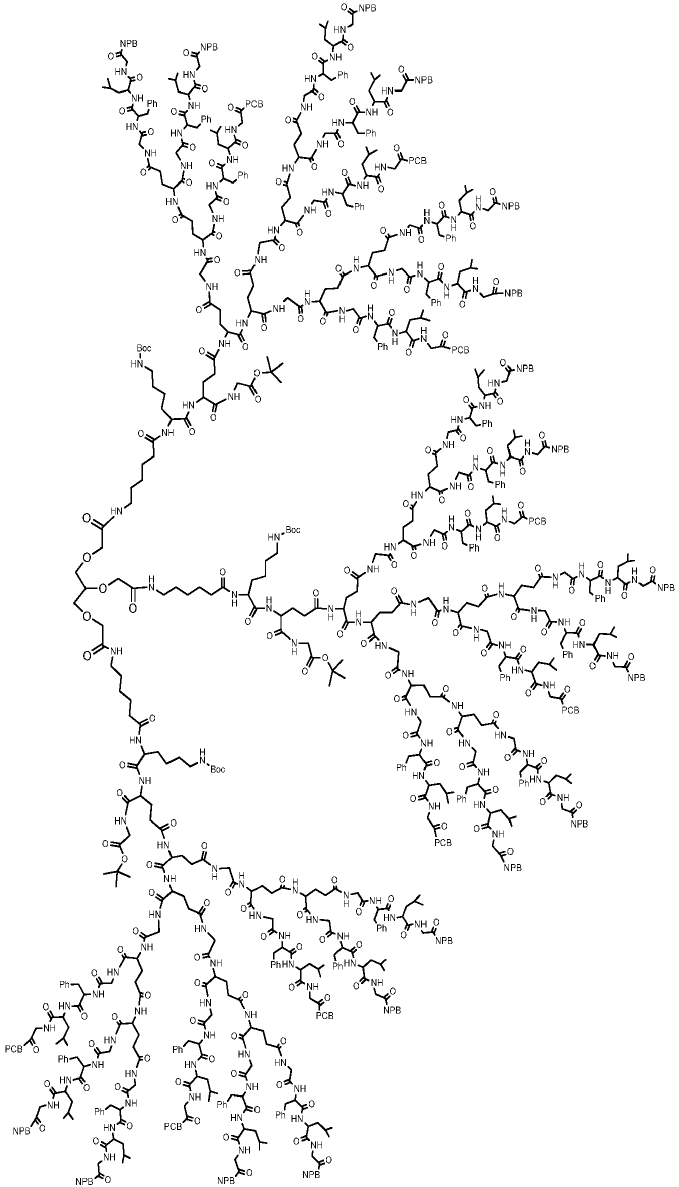

Product 41-105 (1.2 g, 0.4818 mmol), HBTU (0.2467 g, 0.6507 mmol) and HOBT (0.0879 g, 0.6507 mmol) were weighed and added in a 500 mL flask and dissolved with the DMF solution of Product 45-129 (0.0482 mmol), and the obtained solution was stirred at -5 °C for 30 min. DIEA (0.3227 mL, 1.9521 mmol) was slowly added dropwise, and the obtained solution was stirred at - 5 °C overnight to react. At the end of the reaction, purified water was added, and a solid product was precipitated; the obtained solid product was filtered by suction and dried, thus obtaining 1.2 g of the product. 45-132

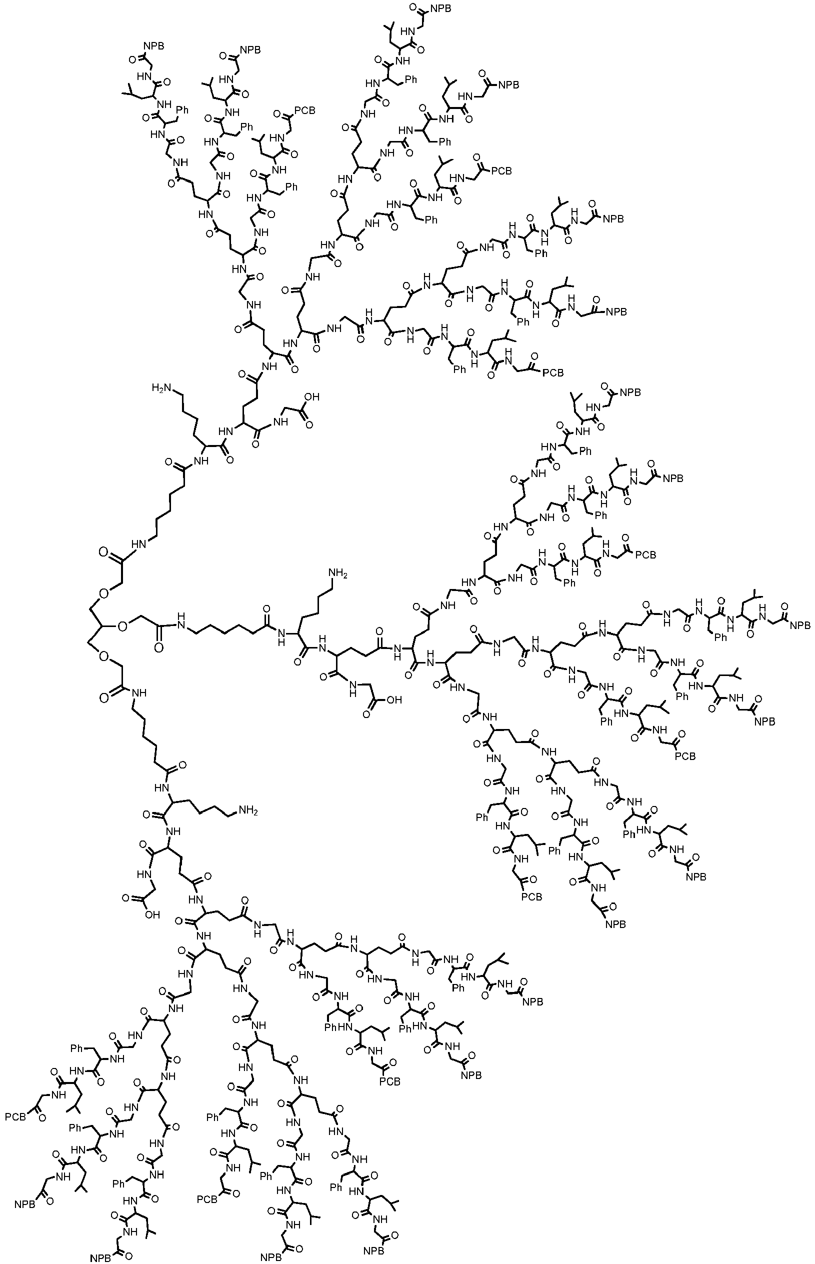

Product 45-130 (1.3 g, 0.0519 mmol) was weighed and added in a 250 mL flask and then dissolved by ultrasonic with dichloromethane (30 mL), and then TFA (0.15 mL, 2.076 mmol) was added, and the obtained solution was stirred at room temperature overnight. Next day, the reaction solution was first evaporated to remove dichloromethane, then precipitated with methyl tert-butyl ether, and filtered by suction, thus obtaining 1.2 g of the product. 45-133

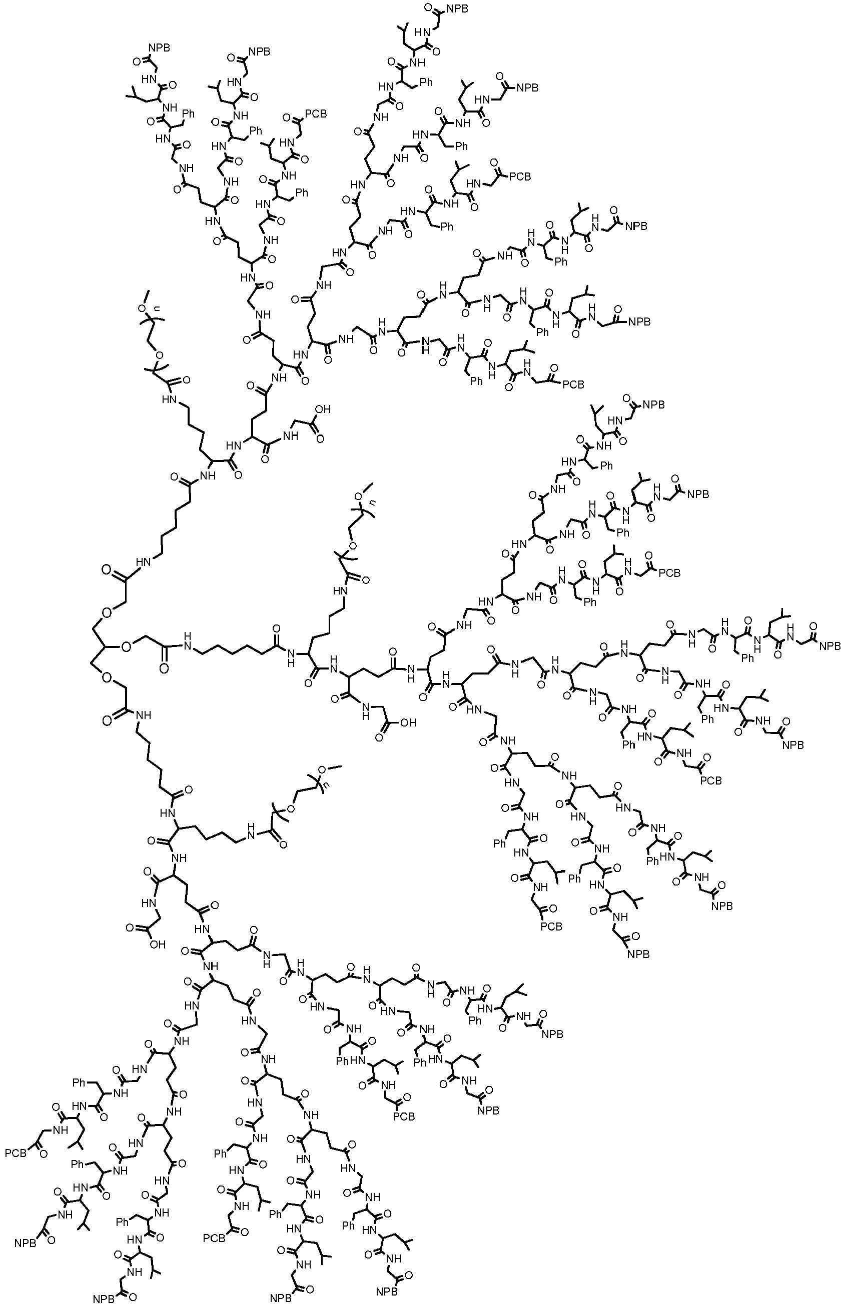

Product 45-132 (1.2 g, 0.028 mmol) was weighed and dissolved with DMF (30 mL) and DIEA (0.3226 mL, 1.952 mmol) was added dropwise and then dissolved by ultrasonic with M-SCM-10K (1.5 g, 0.1462 mmol), and the obtained solution slowly reacted in the dark. At the end of the reaction, methyl tert-butyl ether (150 mL) and n-hexane (70 mL) were added in the reaction solution, and a solid product was precipitated; the obtained solid product was filtered by suction, and dissolved with 20% methanol/dichloromethane; silica gel powder (10 g) was added to the obtained solution, and the solution was evaporated to dryness, column chromatography, and gradient elution with 5%-8% methanol/dichloromethane were carried out, thus obtaining 1.4 g of the product with a yield of 51%.

¹H-NMR (600 MHz, DMSO-*d*₆) δ 9.28-9.26 (m, 13H), 9.07 - 8.72 (m, 98H), 8.57-8.55 (m, 12H), 8.36 - 7.78 (m, 223H), 7.73 - 7.45 (m, 23H), 7.39 - 7.01 (m, 147H), 4.23-4.01 (m, 38H), 3.78-3.76(m, 162H), 3.51-3.49 (m, 2727H), 2.89-2.87 (m, 88H), 2.70-2.68(m, 92H), 2.45-2.44 (m, 46H),2.31- 2.27 (m, 57H), 1.91-1.48 (m, 136H), 1.31-1.18 (m, 293H), 0.90-0.84 (m, 144H). 45-145

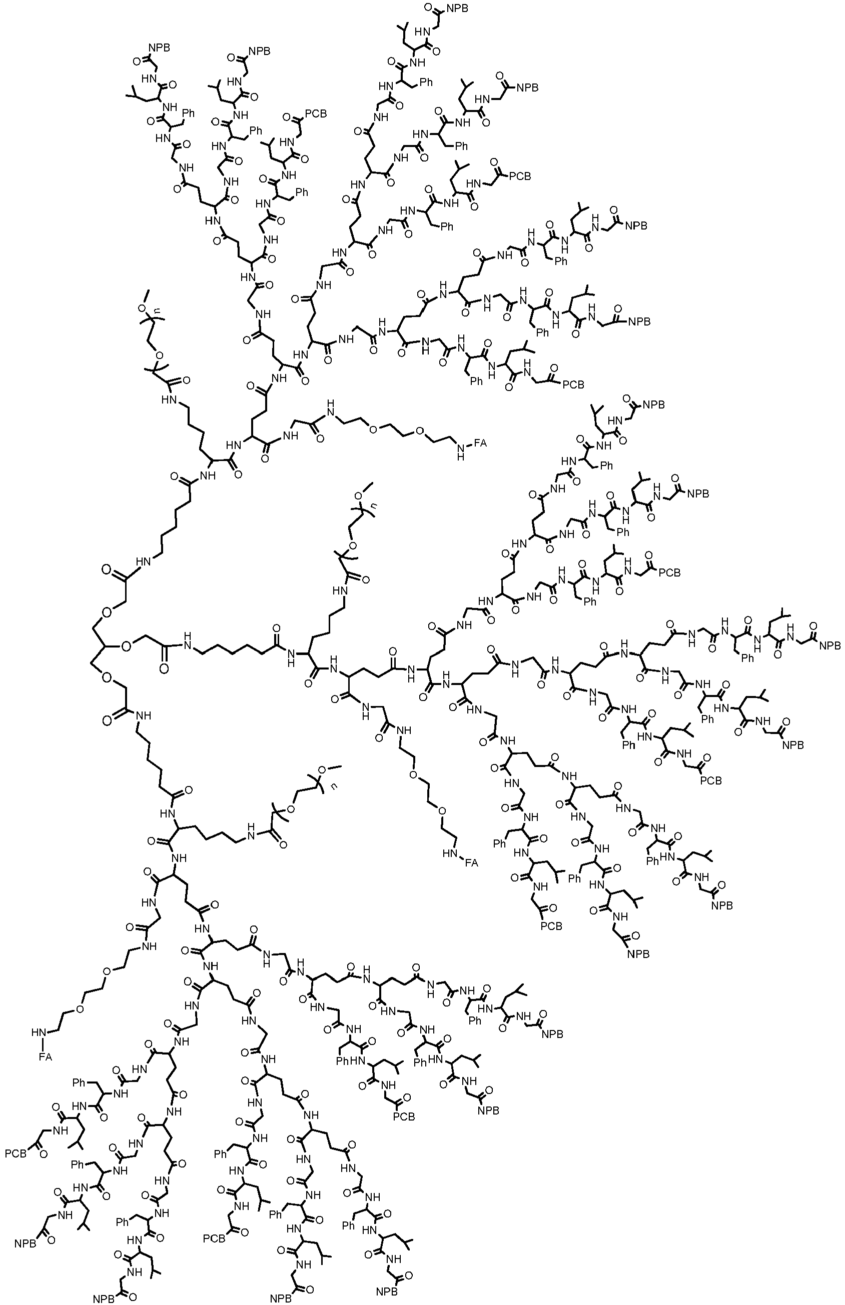

Product 45-133 (1.4 g, 0.025 mmol), Product 37-163 (0.0643 g, 0.1125 mmol), HBTU (0.0427 g, 0.1125 mmol) and HOBT (0.0152 g, 0.0025 mmol) were weighed and added in a 250 mL flask and then dissolved in DMF solution, and the obtained solution was stirred at -5 °C for 30 min. DIEA (0.0560 mL, 0.3375 mmol) was slowly added dropwise, and the obtained solution reacted at -5 °C for 2 h and then transferred to room temperature to react. At the end of the reaction, methyl tert-butyl ether and n-hexane were added to precipitate the reaction solution; suction filtering was then carried out, the operations of dry sample loading, column chromatography and elution with 5% methanol/dichloromethane were carried out. 0.5 g of the product was obtained with a yield of 35.7%.

¹H-NMR (600 MHz, DMSO-*d*₆) δ 10.10-10.07(m, 10H), 9.32-9.27 (m, 23H), 8.94-8.93 (m, 12H), 8.56-8.53 (m, 18H), 8.18-7.89 (m, 260H), 7.65-7.50 (m, 57H), 7.28-7.14 (m, 150H), 4.57-4.37 (m, 49H), 4.05-3.85 (m, 61H), 3.74 - 3.60 (m, 43H), 3.51-3.49 (m, 2702H), 3.17-3.06 (m, 100H), 2.89-2.73 (m,198H), 2.28-2.14 (m, 138H), 1.91-1.48 (m, 318H), 0.90-0.81 (m, 172H).

### Synthesis of Compound 39-138

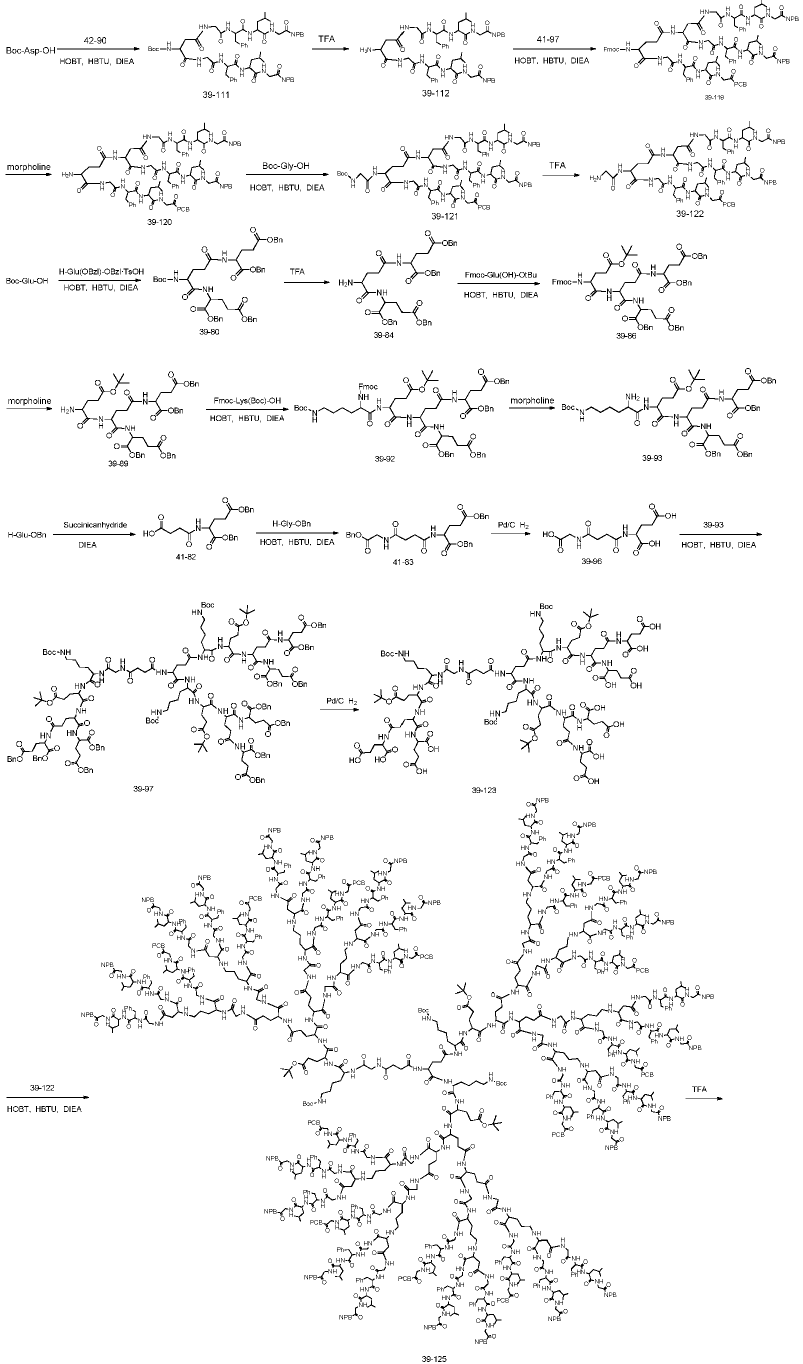

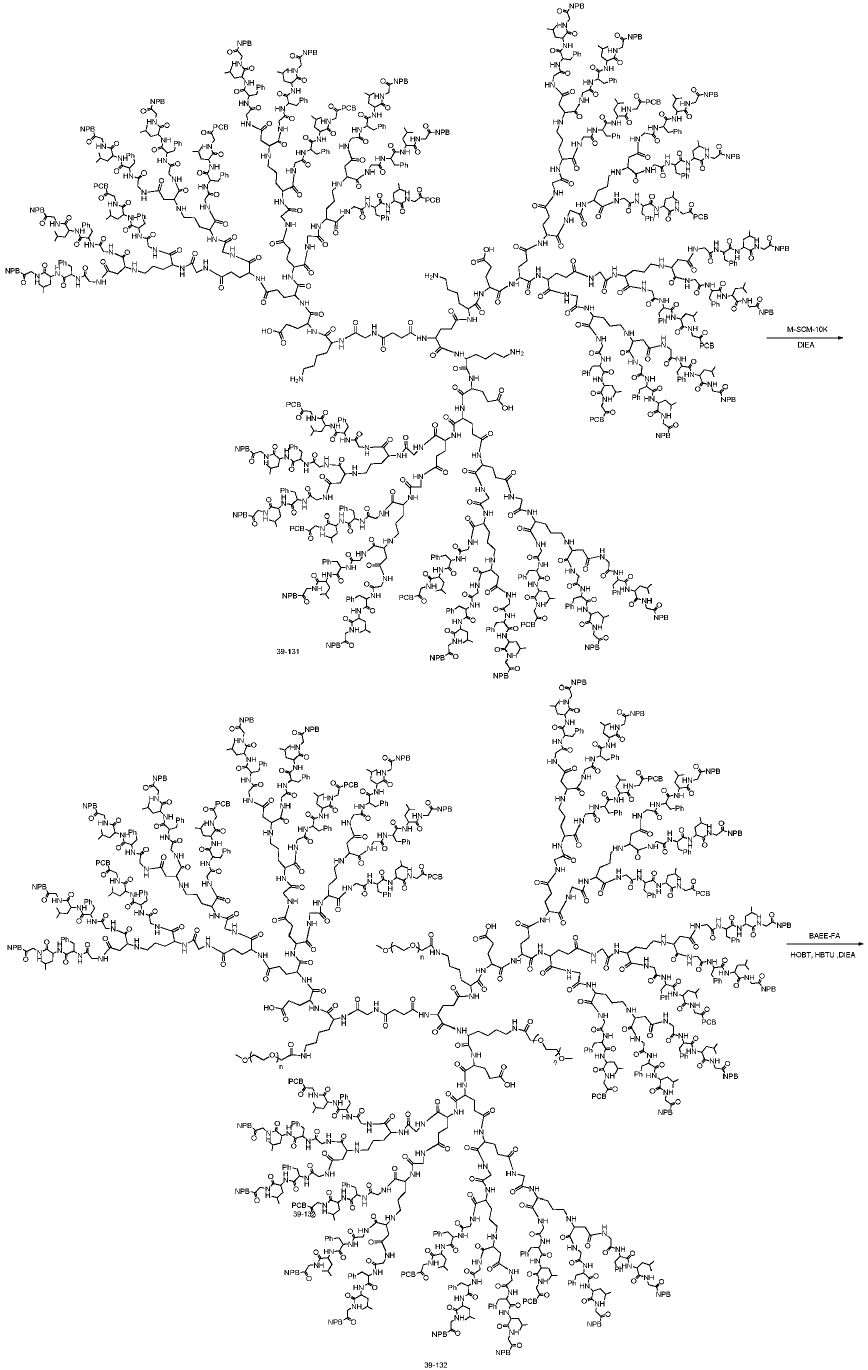

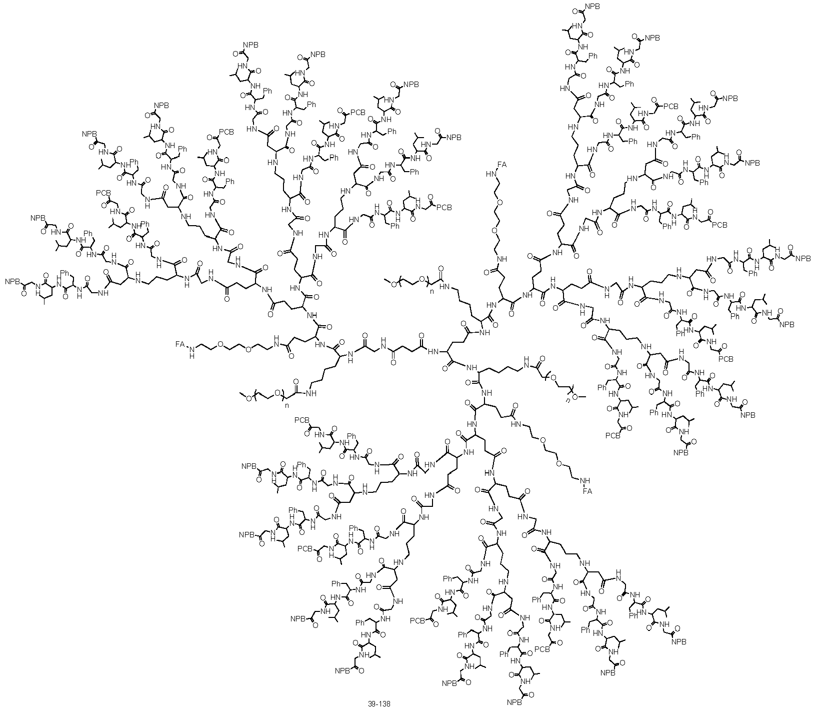

39-80

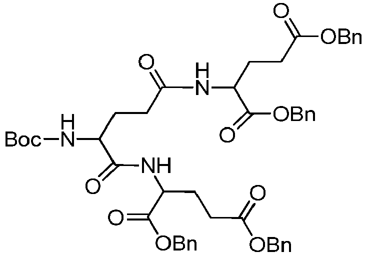

Boc-Glu-OH (5.0 g, 20.22 mmol), H-Glu(OBzl)-OBzl·TsOH (21.2 g, 42.46 mmol, purchased from Ark Pharm), HOBT (8 g, 60.66 mmol) and HBTU (23 g, 60.66 mmol) were weighed and added in a 250 mL flask and then dissolved in DMF solution (80 mL), and the ultrasonic treatment was carried out to completely dissolve the reactants; the obtained solution was stirred for 30 min at - 5 °C. DIEA (30 mL, 181 mmol) was slowly added dropwise, and the obtained solution reacted at a low temperature to the end. At the end of the reaction, the reaction solution was taken out; deionized water (100 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness. The operations of dry sample loading, column chromatography and gradient elution with 40% ethyl acetate/petroleum ether-50% ethyl acetate/petroleum ether were carried out; the elution product was collected, then concentrated and evaporated to dryness. 39-84

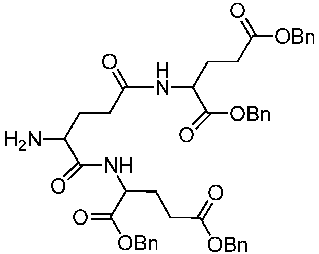

Product 39-80 (19.2 mmol) was weighed and dissolved by ultrasonic with dichloromethane (5 mL) and TFA (14 mL, 192 mmol), a ground glass stopper was used, and the obtained solution was stirred to react at room temperature. At the end of the reaction, the reaction solution was taken out, a saturated sodium bicarbonate solution (300 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness. 39-86

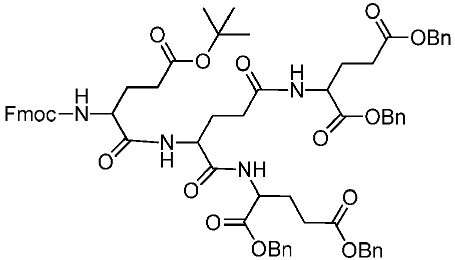

Fmoc-Glu (OH)-OtBu (8.2 g, 19.3 mmol, purchased from JenKem), Product 39-84 (19.2 mmol), HOBT (3.9 g, 28.95 mmol) and HBTU (10.9 g, 28.95 mmol) were weighed and added in a 250 mL flask and then dissolved in DMF solution (70 mL), and the ultrasonic treatment was carried out to completely dissolve the reactants; the obtained solution was stirred for 30 min at -5 °C. DIEA (14.3 mL, 86.85 mmol) was slowly added dropwise, and the obtained solution reacted at a low temperature to the end. At the end of the reaction, the reaction solution was taken out, deionized water (100 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness. The operations of dry sample loading, column chromatography and gradient elution with 40% ethyl acetate/petroleum ether -50% ethyl acetate/petroleum ether were carried out; the elution product was collected, then concentrated and evaporated to dryness. 39-89

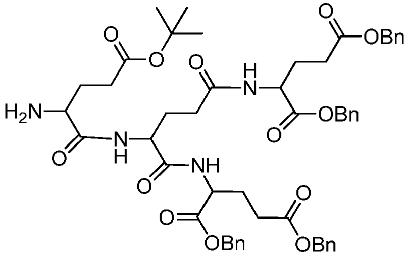

Product 39-86 (19.2 mmol) was dissolved in DMF (40 mL) and morpholine (25 mL, 289.5 mmol) was added, and the mixed solution was stirred at room temperature to react. At the end of the reaction, the reaction solution was taken out; deionized water (100 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness. The operations of dry sample loading, column chromatography and gradient elution with 40% ethyl acetate/petroleum ether -50% ethyl acetate/petroleum ether were carried out; the elution product was collected, then concentrated and evaporated to dryness. 39-92

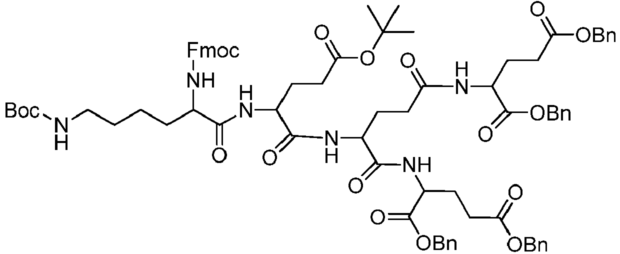

Fmoc-Lys (Boc)-OH (9.04 g, 19.3 mmol), Product 39-89 (19.2 mmol), HOBT (3.9 g, 28.95 mmol) and HBTU (10.9 g, 28.95 mmol) were weighed and added in a 250 mL flask and then dissolved in DMF solution (70 mL), and the ultrasonic treatment was carried out to completely dissolve the reactants; the obtained solution was stirred for 30 min at -5 °C. DIEA (14.3 mL, 86.85 mmol) was slowly added dropwise, and the obtained solution reacted at a low temperature to the end. At the end of the reaction, the reaction solution was taken out; deionized water (100 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness. The operations of dry sample loading, column chromatography and gradient elution with 40% ethyl acetate/petroleum ether -50% ethyl acetate/petroleum ether were carried out; the elution product was collected, then concentrated and evaporated to dryness. 39-93

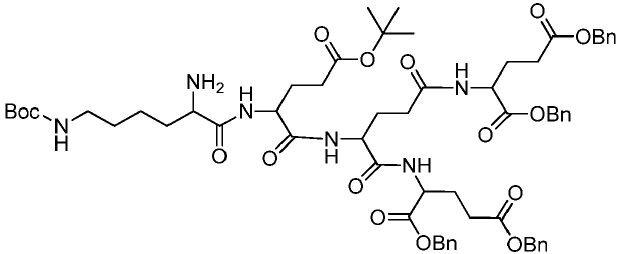

Product 39-92 (19.2 mmol) was dissolved in DMF (40 mL) and then morpholine (25 mL, 289.5 mmol) was added, and the mixed solution was stirred at room temperature to react. At the end of the reaction, the reaction solution was taken out; deionized water (100 mL) was added to the reaction solution, extraction with ethyl acetate (100 mL × 3) were carried out several times, and the obtained organic phases were combined, the organic phase was washed twice with a saturated sodium chloride solution (100 mL), finally concentrated and evaporated to dryness. The operations of dry sample loading, column chromatography and gradient elution with 80% ethyl acetate/petroleum ether -50% ethyl acetate/petroleum ether were carried out; the elution product was collected, then concentrated and evaporated to dryness. 41-82

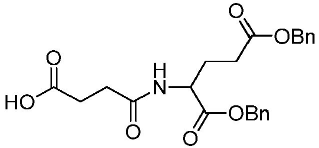

Succinic anhydride and H-Glu(OBzl)-OBzl (5 g, 10.0084 mmol, purchased from Ark Pharm) were added in a 250 mL flask and then dissolved with DMF (20 mL), and the mixed solution was stirred at low temperature (0 °C) and constant temperature for 0.5 h to react. DIEA (8.3 mL, 50.0420 mmol) was slowly added dropwise, and the mixed solution was further stirred at this temperature for 3 h to react. The reaction solution was taken out and stirred at room temperature to react. Sample was taken for TLC to determine the completion of the reaction. The next step of the reaction was then carried out. 41-83

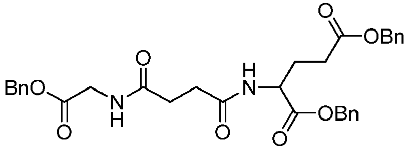

Product 41-82, H-Gly-OBzl (4.0521 g, 12.0108 mmol, purchased from Innochem), HBTU (5.6934 g, 15.0126 mmol) and HOBT (2.0285 g, 15.6934 mmol) were added in a 250 mL flask and then dissolved with 20 mL of DMF, and the mixed solution was stirred at low temperature (0 °C) and constant temperature for 0.5 h to react. DIEA (7.5 mL, 45.0378 mmol) was slowly added dropwise, and the mixed solution was further stirred at this temperature for 3 h to react. The reaction solution was taken out and stirred at room temperature to react. Sample was taken for TLC to determine the completion of the reaction. Extraction with ethyl acetate (150 mL), deionized water (200 mL) and a saturated sodium bicarbonate solution (200 mL) was carried out, and the organic phase was separated, the aqueous phase was further extracted and separated, and such operations were repeated multiple times until there was no product in the organic phase. The obtained organic phases were combined, washed with a saturated saline solution (300 mL), collected and concentrated, and evaporated to dryness; silica gel powder was added to the obtained solution, and the solution was then evaporated to dryness; the operations of dry sample loading, and column chromatography with 40% ethyl acetate/petroleum ether. 2.4 g of the product was obtained. 39-96

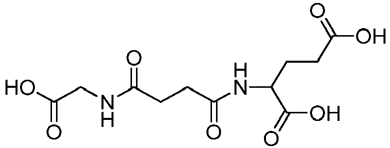

Product 41-83 (1.5 g, 2.61 mmol) was added in a hydrogenation reactor and then 10% Pd/C (0.07 g) was added, and the mixture was dissolved with DMF (20 mL); hydrogen was introduced to a pressure of 300 Psi in the reactor, and the reaction solution was stirred overnight at room temperature to react. At the end of the reaction, the reaction solution was filtered with diatomaceous earth; the filter cake was washed with DMF (15 mL × 3), and the elution solution was added in a 250 mL round-bottomed flask, as the raw material for the next step. 39-97

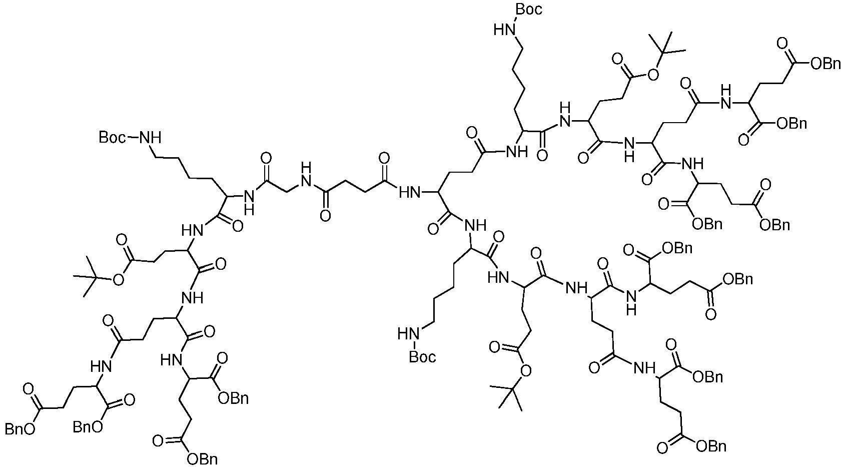

Product 39-93 (6.0 g, 5.08 mmol), Product 39-96 (1.54 mmol), HOBT (0.84 g, 6.28 mmol) and HBTU (2.38 g, 6.28 mmol) were weighed and added in a 250 mL flask and then dissolved in DMF solution (30 mL), and the ultrasonic treatment was carried out to completely dissolve the reactants; the obtained solution was stirred for 30 min at -5 °C. DIEA (3.1 mL, 18.85 mmol) was slowly added dropwise, and the obtained solution reacted at a low temperature to the end. At the end of the reaction, methyl tert-butyl ether (150 mL) and n-hexane (100 mL) were added in the reaction solution, and a solid product was precipitated; the obtained solid product was filtered by suction, the operations of dry sample loading, column chromatography and elution with 2% methanol/dichloromethane were carried out; the elution product was then collected, concentrated and evaporated to dryness, thus obtaining 2.0 g of the product.

¹H-NMR (600 MHz, DMSO-*d*₆) δ 8.27 - 7.89 (m, 20H), 7.38 - 7.27 (m, 60H), 5.14 - 5.02 (m, 24H), 4.36 - 4.22 (m, 12H), 3.08 (s, 2H), 2.91 - 2.78 (m, 18H), 2.74 - 2.59 (m, 18H), 2.24 - 1.82 (m, 48H), 1.36 - 1.32 (m, 54H), 1.10 (s, 6H). 39-111

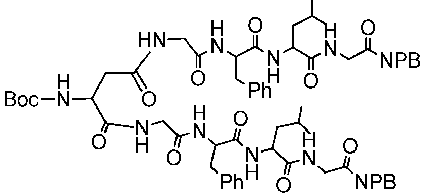

Boc-Asp-OH (1.32 g, 5.68 mmol, purchased from damas-beta), GFLG-NPB (synthesized according to the synthesis method of Product 42-90, 8.3 g, 11.94 mmol), HOBT (2.29 g, 17.0 mmol) and HBTU (6.4 g, 17.0 mmol) were weighed and added in a 250 mL flask and then dissolved in DMF solution (70 mL), and the ultrasonic treatment was carried out to completely dissolve the reactants; the obtained solution was stirred for 30 min at -5 °C. DIEA (8.4 mL, 51.1 mmol) was slowly added dropwise, and the obtained solution reacted at a low temperature to the end. At the end of the reaction, methyl tert-butyl ether (150 mL) and n-hexane (100 mL) were added in the reaction solution, and a solid product was precipitated; the obtained solid product was filtered by suction and dried in vacuum, thus obtaining the product. 39-112

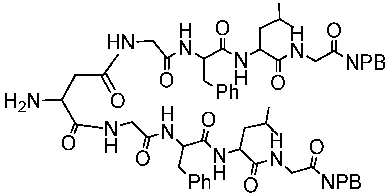

Product 39-111 (5.68 mmol) was weighed and dissolved by ultrasonic with dichloromethane (5 mL) and TFA (6.3 mL, 85.2 mmol), a ground glass stopper was used, and the obtained solution was stirred to react at room temperature. At the end of the reaction, the reaction solution was concentrated, methyl tert-butyl ether (150 mL) and n-hexane (100 mL) were added in the reaction solution, and a solid product was precipitated; the obtained solid product was filtered by suction and dried in vacuum, thus obtaining 5.3 g of the product. 39-119

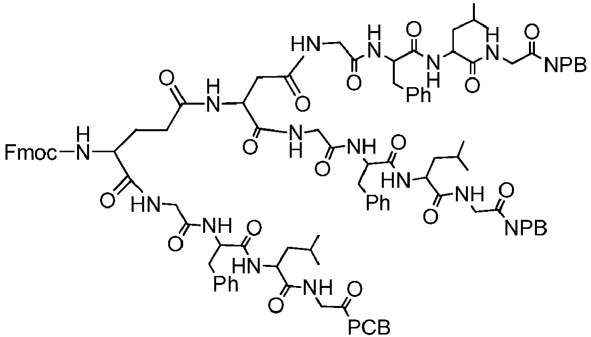

Product 39-112 (5.3 g, 3.62 mmol), Product 41-97 (3.2 g, 3.62 mmol), HOBT (0.73 g, 5.43 mmol) and HBTU (2.0 g, 5.43 mmol) were weighed and added in a 250 mL flask and then dissolved in DMF solution (70 mL), and the ultrasonic treatment was carried out to completely dissolve the reactants; the obtained solution was stirred for 30 min at -5 °C. DIEA (2.69 mL, 16.29 mmol) was slowly added dropwise, and the obtained solution reacted at a low temperature to the end. At the end of the reaction, methyl tert-butyl ether (150 mL) and n-hexane (100 mL) were added in the reaction solution, and a solid product was precipitated; the obtained solid product was filtered by suction and dried in vacuum, thus obtaining 7 g of the product. 39-120

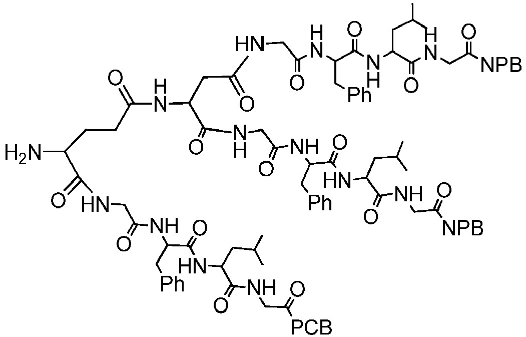

Product 39-119 (3.6 mmol) was dissolved with DMF (40 mL), and morpholine (6.2 mL, 72 mmol) was then added, and the mixed solution was stirred at room temperature to react. At the end of the reaction, methyl tert-butyl ether (150 mL) and n-hexane (100 mL) were added to precipitate the reaction solution, and a solid product was then obtained; the obtained solid product was filtered by suction and dried in vacuum, thus obtaining 7 g of the product. 39-121

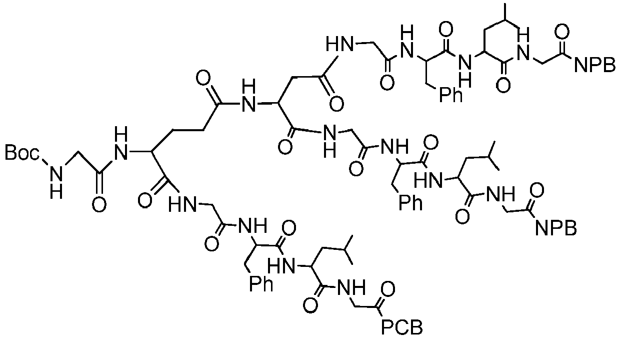

Product 39-120 (3.62 mmol), Boc-Gly-OH (0.63 g, 3.62 mmol, purchased from Aladdin), HOBT (0.73 g, 5.43 mmol) and HBTU (2.0 g, 5.43 mmol) were weighed and added in a 250 mL flask and then dissolved in DMF solution (70 mL), and the ultrasonic treatment was carried out to completely dissolve the reactants; the obtained solution was stirred for 30 min at -5 °C. DIEA (2.69 mL, 16.29 mmol) was slowly added dropwise, and the obtained solution reacted at a low temperature to the end. At the end of the reaction, methyl tert-butyl ether (150 mL) and n-hexane (100 mL) were added in the reaction solution, and a solid product was precipitated; the obtained solid product was filtered by suction and dried in vacuum, thus obtaining the product. 39-122

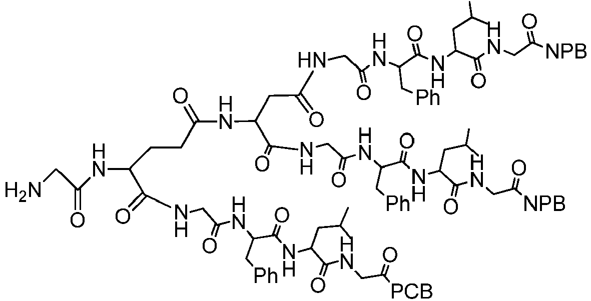

Product 39-121 (3.62 mmol) was weighed and dissolved by ultrasonic with dichloromethane (5 mL) and TFA (8.0 mL, 108.6 mmol), a ground glass stopper was used, and the obtained solution was stirred to react at room temperature. At the end of the reaction, the reaction solution was concentrated; methyl tert-butyl ether (150 mL) and n-hexane (100 mL) were added to precipitate the reaction solution, and a solid product was then obtained; the obtained solid product was filtered by suction and dried in vacuum, thus obtaining 3.0 g of the product. 39-123

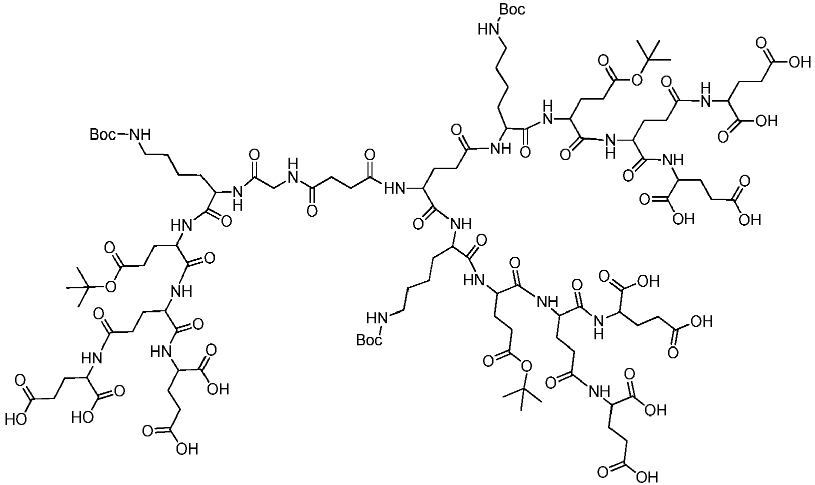

The reactant Product 39-97 (0.5 g, 0.13 mmol) was weighed and added in a micro-reactor, 10% Pd/C (70 mg) was added, and then dissolved in DMF solution (30 mL); H₂ (300psi) was introduced, and the obtained solution was stirred to react. At the end of the reaction, the reaction solution was filtered by suction with diatomaceous earth as a filter cake to remove Pd/C; the diatomaceous earth was washed 3-4 times with DMF to obtain the DMF solution of the product for the next reaction. 39-125

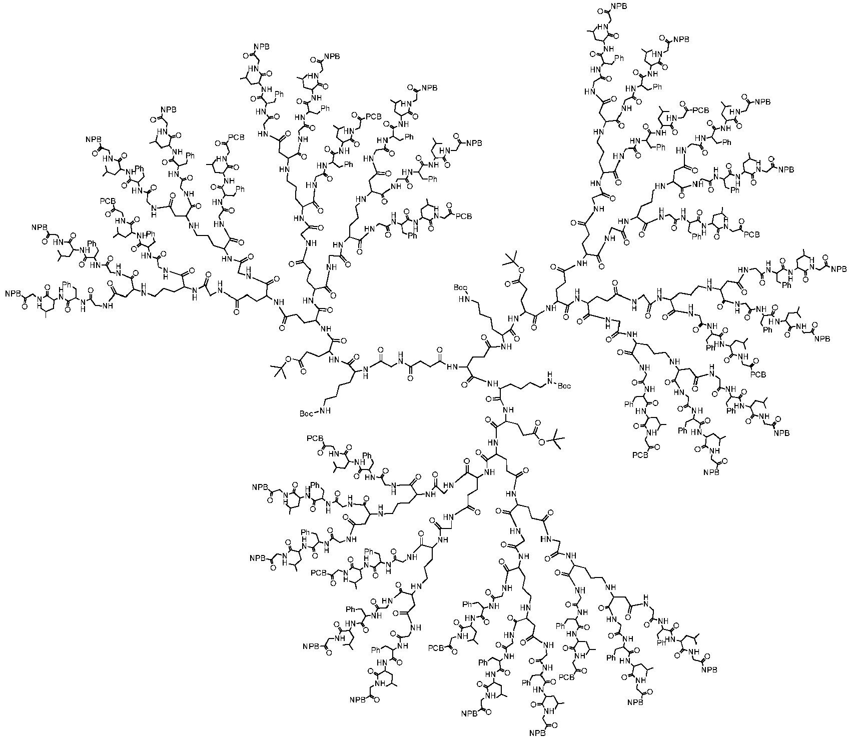

Product 39-122 (3.0 g, 1.17 mmol), Product 39-123 (0.09 mmol), HOBT (0.21 g, 1.62 mmol) and HBTU (0.61 g, 1.58 mmol) were weighed and added in a 250 mL flask and then dissolved in DMF solution (70 mL), and the ultrasonic treatment was carried out to completely dissolve the reactants; the obtained solution was stirred for 30 min at -5 °C. DIEA (0.80 mL, 4.86 mmol) was slowly added dropwise, and the obtained solution reacted at a low temperature to the end. At the end of the reaction, methyl tert-butyl ether (150 mL) and n-hexane (100 mL) were added in the reaction solution, and a solid product was precipitated; the obtained solid product was filtered by suction and dried in vacuum, thus obtaining the product. 39-131

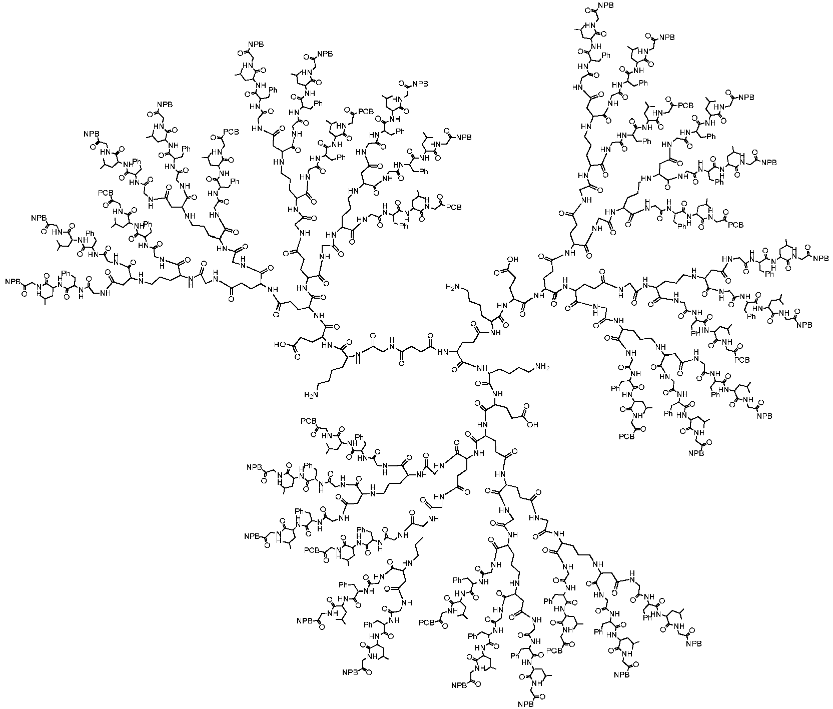

Product 39-125 (0.09 mmol) was weighed and dissolved by ultrasonic with dichloromethane (5 mL) and TFA (0.20 mL, 2.7 mmol), a ground glass stopper was used, and the obtained solution was stirred to react at room temperature. At the end of the reaction, the reaction solution was concentrated; methyl tert-butyl ether (150 mL) and n-hexane (100 mL) were added in the reaction solution, and a solid product was precipitated; the obtained solid product was filtered by suction and dried in vacuum, thus obtaining 1 g of the product. 39-132

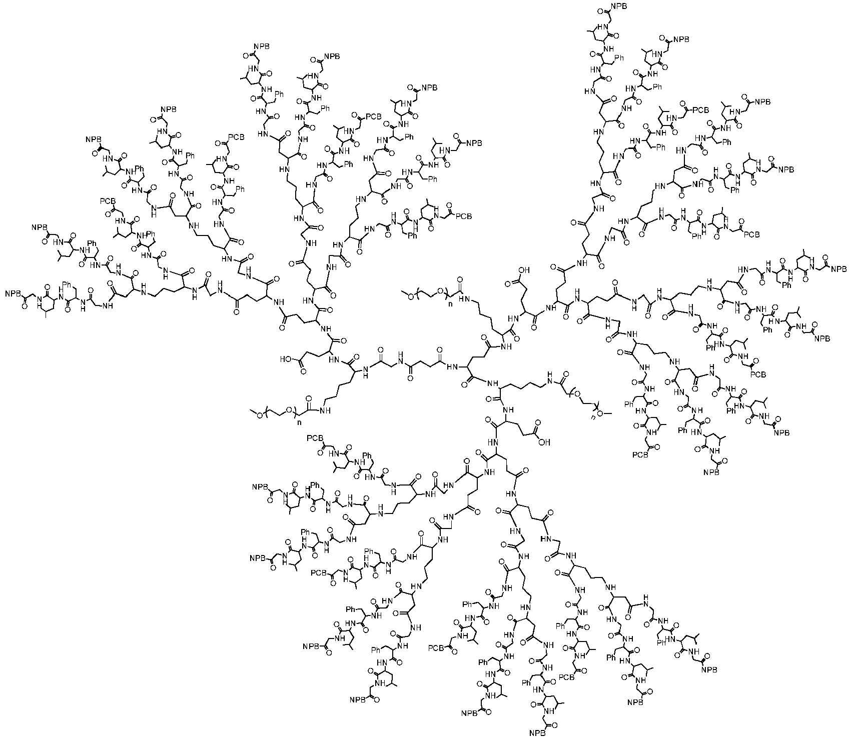

Compound 39-131 (1 g, 0.029 mmol) and M-SCM-10K (0.90 g, 0.0907 mmol, purchased from JenKem) were added to a 250 mL flask and then dissolved with DMF (30 mL); DIEA (1 mL) was added, and the obtained solution was slowly stirred in the dark. At the end of the reaction, the reaction solution was concentrated; methyl tert-butyl ether (150 mL) and n-hexane (100 mL) were added to separate out a solid product, suction filtering and evaporated to dryness were carried out, thus obtaining 1.2 g of the product.

¹H-NMR (600 MHz, DMSO-*d*₆) δ 10.15 - 10.00 (m, 10H), 9.36 - 8.47 (m, 62H), 8.40 - 7.74 (m, 302H), 7.63 - 7.07 (m, 265H), 7.01 - 6.87 (m, 3H), 6.74 - 6.62 (m, 6H), 4.64 - 4.29 (m, 122H), 4.25 - 3.75 (m, 185H), 3.74 - 3.60 (m, 178H), 3.51 - 3.50 (m, 2923H), 3.44 - 3.37 (m, 37H), 3.26 - 3.22 (m, 8H), 3.21 - 3.01 (m, 74H), 2.92 - 2.86 (m, 12H), 2.85 - 2.56 (m, 112H), 2.46 - 1.42 (m, 410H), 0.88 - 0.79 (m, 216H). 39-138

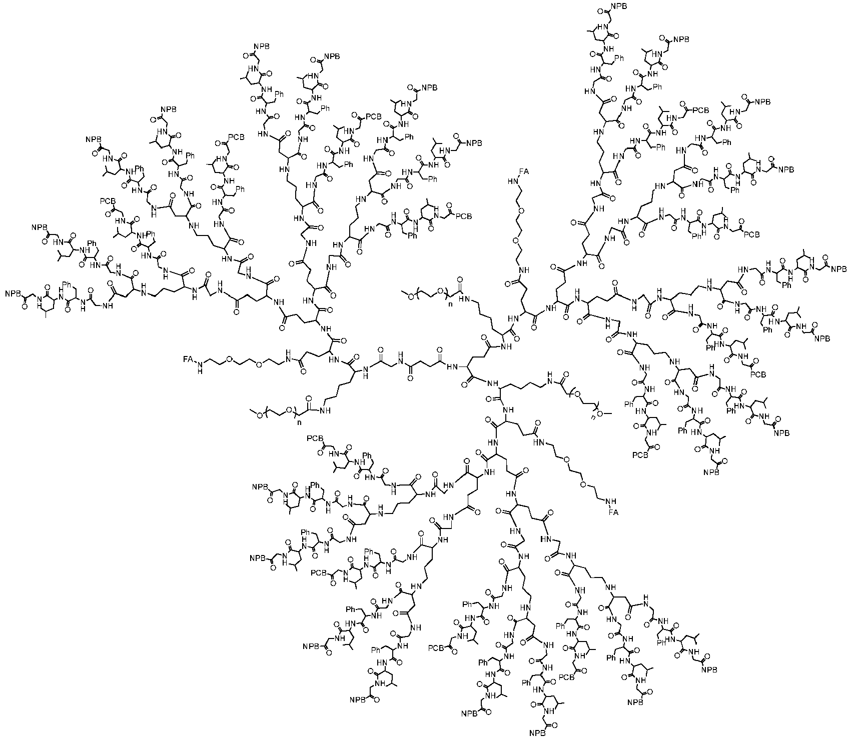

Product 39-132 (1.2 g, 0.018 mmol), Product 37-163 (0.0579 mmol), HOBT (0.035 g, 0.26 mmol) and HBTU (0.098 g, 0.26 mmol) were weighed and added in a 250 mL flask and then dissolved in DMF solution (70 mL), and the ultrasonic treatment was carried out to completely dissolve the reactants; the obtained solution was stirred for 30 min at -5 °C. DIEA (0.129 mL, 0.78 mmol) was slowly added dropwise, and the obtained solution reacted at a low temperature to the end. At the end of the reaction, methyl tert-butyl ether (150 mL) and n-hexane (100 mL) were added in the reaction solution, and a solid product was precipitated; the obtained solid product was filtered by suction and dried in vacuum, thus obtaining 0.4 g of the product.

¹H-NMR (600 MHz, DMSO-*d*₆) δ 10.15 - 10.00 (m, 10H), 9.31 - 9.25 (m, 22H), 8.96 - 8.92 (m, 10H), 8.58 - 8.54 (m, 23H), 8.24 - 7.99 (m, 246H), 7.98 - 7.92 (m, 8H), 7.89 - 7.87 (m, 25H), 7.29 - 6.96 (m, 325H), 4.63 - 4.32 (m, 119H), 4.11 - 3.71 (m, 256H), 3.51 - 3.50 (m, 2776H), 3.25 - 2.97 (m, 268H), 2.71 - 2.58 (m, 90H), 2.44 - 2.02 (m, 150H), 1.98 - 1.35 (m, 330H), 0.91 - 0.77 (m, 216H).

### Synthesis of Product 45-80:

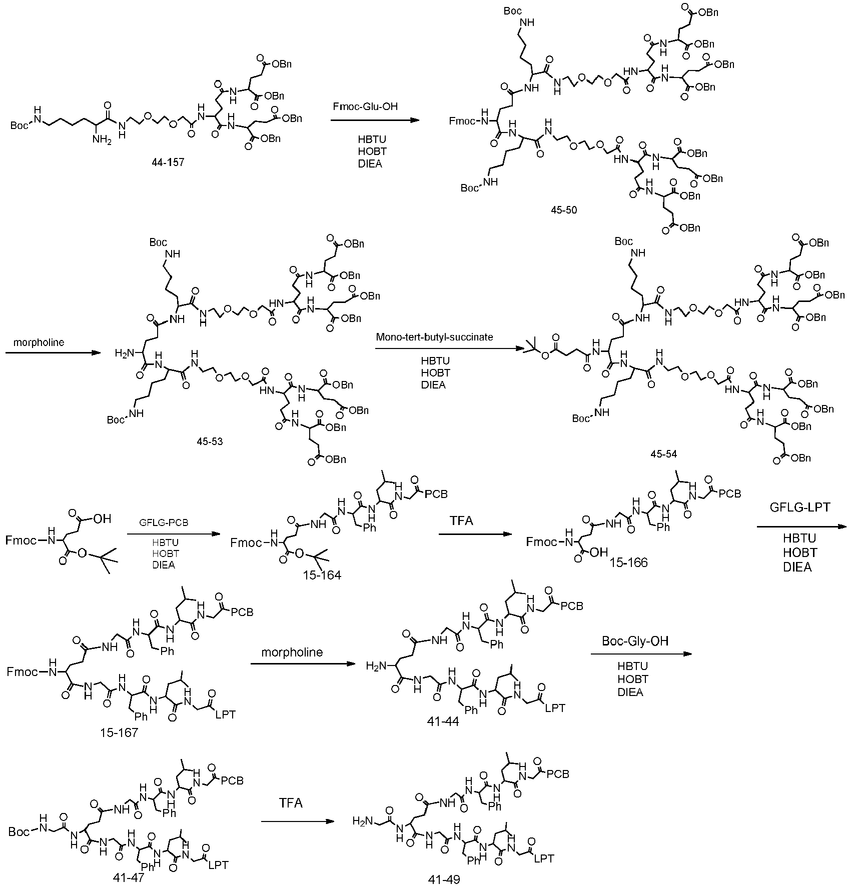

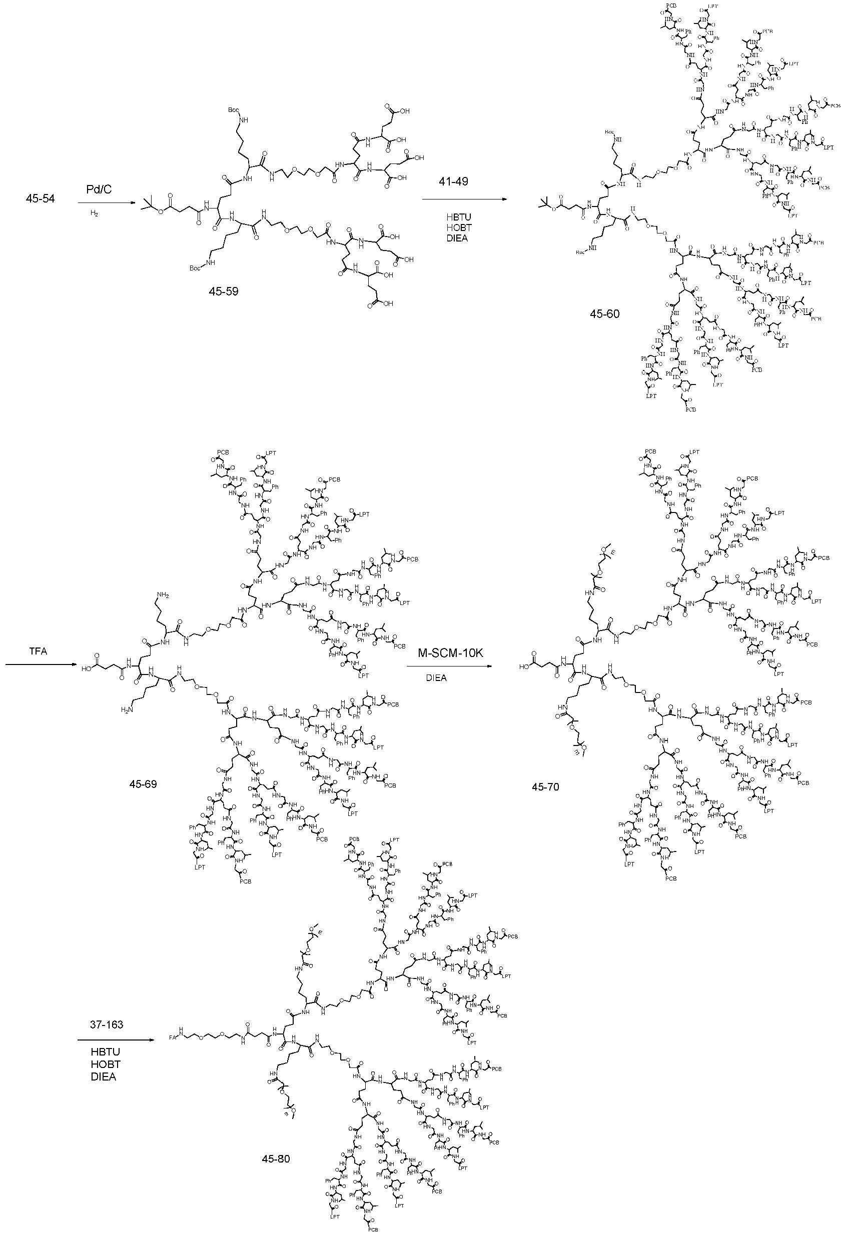

45-50

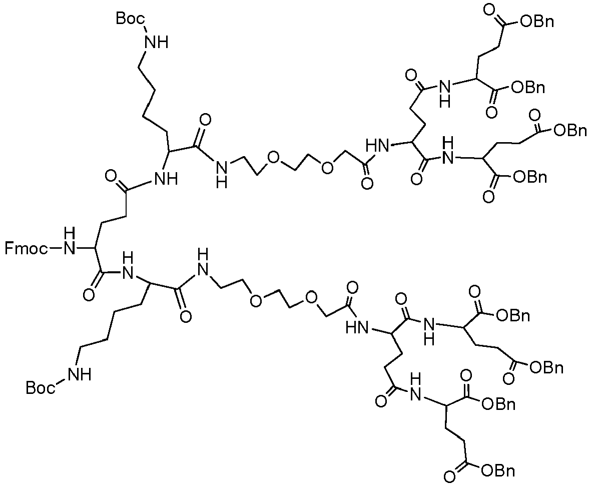

Fmoc-Glu-OH (0.766 g, 2.0747 mmol, purchased from Aladdin), Product 44-157 (5.2 g, 4.5642 mmol), HBTU (2.36 g, 6.22 mmol) and HOBT (0.84 g, 6.22 mmol) were weighed and added in a 250 mL flask and then dissolved in DMF solution (40 mL), and the obtained solution was stirred at -5 °C for 30 min. DIEA (3 mL, 18.6723 mmol) was slowly added dropwise, and the obtained solution was stirred at -5 °C overnight to react. At the end of the reaction, extraction with purified water and ethyl acetate was carried out, and the organic phase was concentrated, the operations of dry sample loading, column chromatography and elution with 2% methanol/dichloromethane were carried out, thus obtaining 5.4 g of the product with a yield of 96%. 45-53

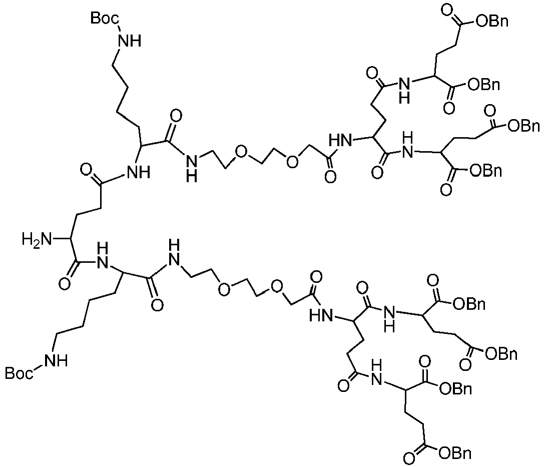

Product 45-50 (5.2 g, 1.99 mmol) was weighed and added in a 250 mL flask and then dissolved by ultrasonic with DMF solution (20 mL); morpholine (3.4689 mL, 39.8 mmol), was added and the mixed solution was stirred at room temperature to react. Two hours later, the reaction solution was extracted with ethyl acetate and purified water; the organic phase was concentrated, the operations of dry sample loading, column chromatography and gradient elution with 1% ammonia water: 4%-5% methanol/dichloromethane were carried out. The elution product was evaporated to dryness, thus obtaining 2.6 g of the product with a yield of 55%. 45-54

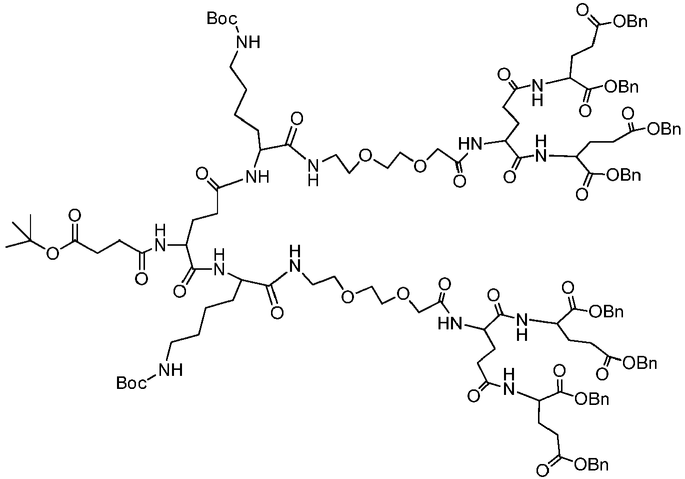

Mono-tert-butyl succinate (0.0383 g, 0.22 mmol, purchased from Accela), Product 45-53 (0.5 g, 0.2 mmol), HBTU (0.1138 g, 0.3 mmol) and HOBT (0.04 g, 0.3 mmol) were weighed and added in a 250 mL flask and then dissolved in DMF solution (20 mL), and the obtained solution was stirred at -5 °C for 30 min. DIEA (0.0653 mL, 0.9 mmol) was slowly added dropwise, and the obtained solution was stirred at -5 °C overnight to react. At the end of the reaction, extraction with purified water and ethyl acetate was carried out; the organic phase was concentrated, the operations of dry sample loading, column chromatography and elution with 2%-4% methanol/dichloromethane were carried out, thus obtaining 0.32 g of the product with a yield of 64%. 15-164

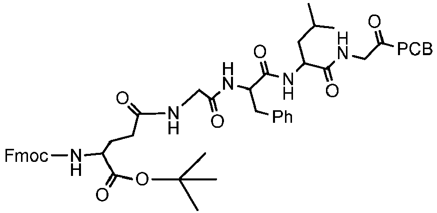

Fmoc-Glu-OtBu (1.0870 g, 2.5548 mmol, purchased from Accela), GFLG-PCB (synthesized according to the synthesis method of Product 36-98, 2 g, 2.4332 mmol), HBTU (2.7683 g, 7.2996 mmol) and HOBT (0.9863 g, 7.2996 mmol) were added in sequence in a 250 mL flask and then dissolved with DMF (20 ml), and the mixed solution was stirred at low temperature (0 °C) and constant temperature for 30 min to react. DIEA (1.8 mL, 10.9494 mmol) was slowly added dropwise, and the mixed solution reacted at 0 °C overnight. At the end of the reaction, methyl tert-butyl ether (150 mL) and n-hexane (50 mL) were added to precipitate, suction filtering and evaporation to dryness were carried out, the obtained product was used for the next reaction. 15-166

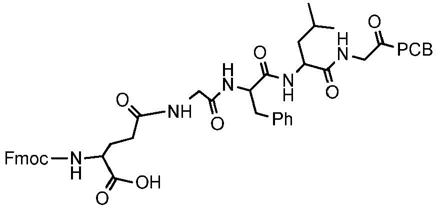

Product 15-164 and dichloromethane (25 mL) were added in a flask, and then TFA (5.4 mL, 72.9960 mmol) was added, and the mixed solution was stirred at room temperature for 3 h to react. At the end of the reaction, methyl tert-butyl ether (150 mL) and n-hexane (50 mL) were added to precipitate, suction filtering and evaporation to dryness were carried out, the obtained product was used for the next reaction. 15-167

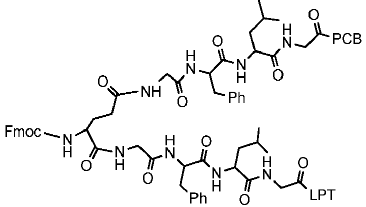

Product 15-166, GFLG-LPT (synthesized according to the synthesis method of Product 36-145, 2.3 g, 2.4332 mmol), HBTU (1.3842 g, 3.6498 mmol) and HOBT (0.4932 g, 3.6498 mmol) were added in sequence in a 250 mL flask and then dissolved with DMF (25 ml), and the mixed solution was stirred at low temperature (0 °C) and constant temperature for 0.5 h to react. DIEA (1.8 mL, 10.9494 mmol) was slowly added dropwise, and the reaction solution was taken out and stirred at room temperature for 3 h to react. Sample was taken for TLC, and the flask was taken out; methyl tert-butyl ether (300 mL) and n-hexane (50 mL) were added to precipitate, suction filtering and evaporation to dryness were carried out, the obtained product was used for the next reaction. 41-44

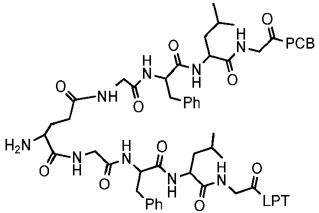

Product 15-167 was dissolved with DMF, and morpholine (6.4 mL, 72.9960 mmol) was added, and the mixed solution was stirred at room temperature for 1 h to react. Sample was taken for TLC to determine the completion of the reaction, and the flask was taken out; methyl tert-butyl ether (100 mL) and n-hexane (50 mL) were added to precipitate, operations of suction filtering, drying, adding silica gel powder and dissolving were carried out, and the solution was evaporated to dryness, column chromatography, and elution with 1% ammonia water+5% methanol/dichloromethane were carried out, thus obtaining 3.1 g of the product. 41-47

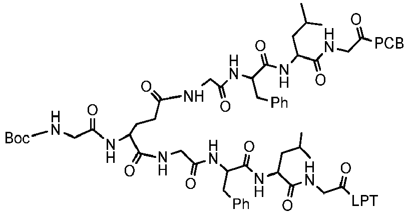

Product 41-44 (3.1 g, 1.6415 mmol), Boc-Gly-OH (0.2876 g, 1.6415 mmol), HBTU (0.9338 g, 2.4622 mmol) and HOBT (0.3327 g, 2.4622 mmol) were added in sequence in a 250 mL flask and then dissolved with DMF (15 ml), and the mixed solution was stirred at low temperature (0 °C) and constant temperature for 0.5 h to react. DIEA (1.2 mL, 7.3866 mmol) was slowly added dropwise, and the reaction solution was taken out and stirred at room temperature for 3 h to react. Sample was taken for TLC. The flask was taken out, methyl tert-butyl ether (300 mL) and n-hexane (50 mL) were added to precipitate, operations of suction filtering, adding silica gel powder and dissolving were carried out, and the solution was evaporated to dryness, column chromatography, and elution with 1% ammonia water+3% methanol/dichloromethane were carried out, thus obtaining 1.9 g of the product. 41-49

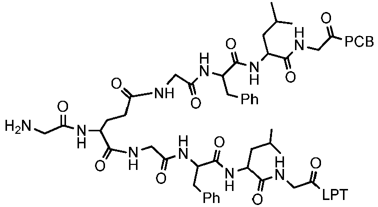

Product 41-47 and CH₂Cl₂ (20 mL) were added in a flask and TFA (2 mL, 26.3966 mmol) was slowly added dropwise, and the reaction solution was taken out and stirred at room temperature for 3 h to react. Sample was taken for TLC to determine the completion of the reaction. Methyl tert-butyl ether (300 mL) and n-hexane (50 mL) were added to precipitate, operations of suction filtering, evaporation to dryness, adding silica gel powder and dissolving were carried out, and the solution was evaporated to dryness, column chromatography, and gradient elution with 1% ammonia water+3%-8% methanol/dichloromethane were carried out, thus obtaining 1.2 g of the product. 45-59

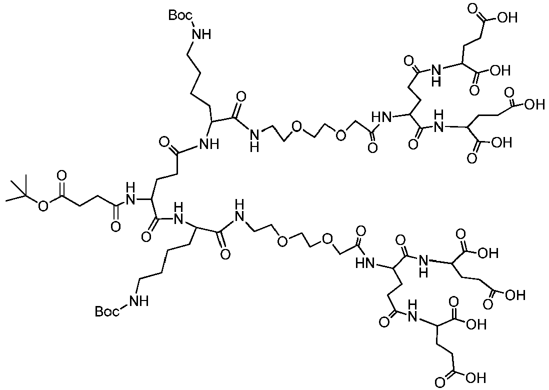

Product 45-54 (0.1025 mg, 0.04026 mmol) and 10% Pd/C (40 mg) were added in a hydrogenation reactor, and then DMF (30 mL) was slowly added with stirring until the reactant was dissolved; hydrogen (300 psi) was introduced, and the obtained solution was stirred at room temperature overnight. Next day, suction filtering was carried out with a suction funnel filled with diatomaceous earth to remove the Pd/C, thus obtaining the DMF solution of Product 45-59, for direct use in the next reaction. 45-60

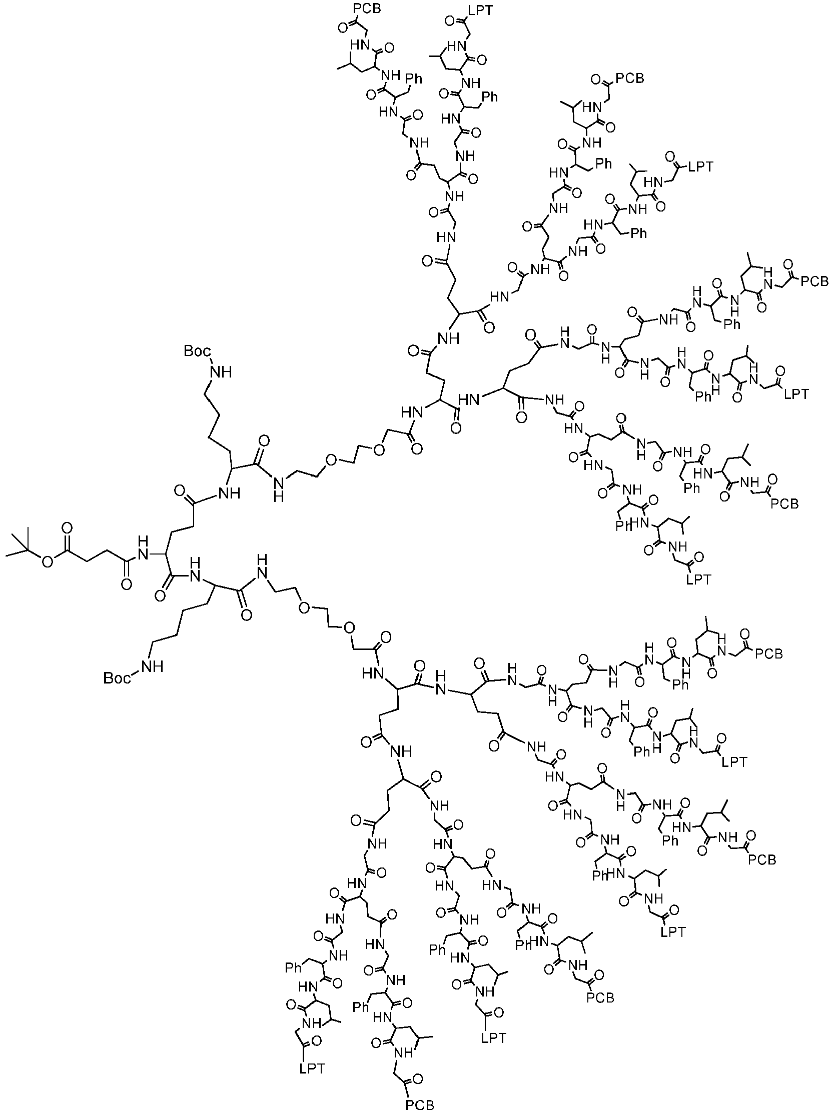

Product 41-49 (0.7 g, 0.3623 mmol), HBTU (0.1832 g, 0.4831 mmol) and HOBT (0.0653 g, 0.4831 mmol) were weighed and added in a 250 mL flask and then dissolved in the DMF solution of Product 45-59, and the obtained solution was stirred at -5 °C for 30 min. DIEA (0.2396 mL, 1.4494 mmol) was slowly added dropwise, and the obtained solution was stirred at -5 °C overnight to react. At the end of the reaction, methyl tert-butyl ether and n-hexane were added to precipitate the reaction solution, suction filtering was carried out, and the operations of dry sample loading, column chromatography and elution with 1% ammonia water: 5%-6% methanol/dichloromethane were carried out, thus obtaining 0.4 g of the product with a yield of 58%. 45-69

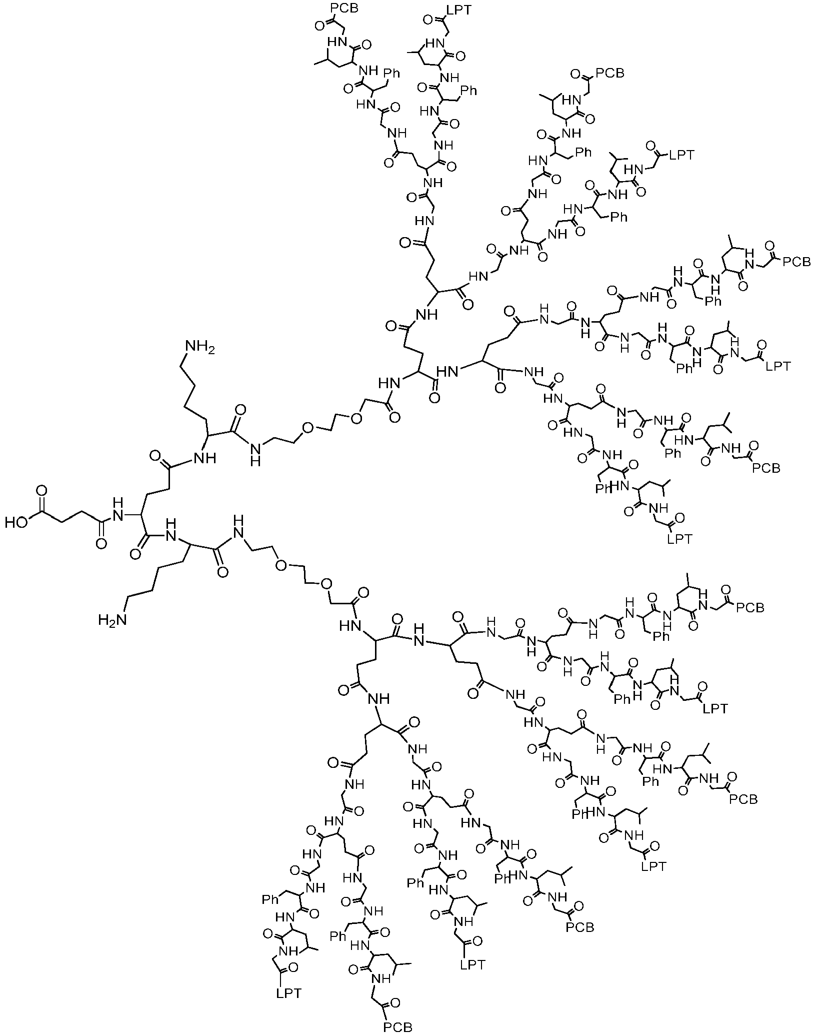

Product 45-60 (0.4 g, 0.073 mmol) was weighed and added in a 250 mL flask and then dissolved by ultrasonic with dichloromethane (10 mL), and TFA (0.0854 mL, 1.15 mmol) was then added, and the mixed solution was stirred at room temperature overnight. Next day, methyl tert-butyl ether and n-hexane were added to precipitate the reaction solution, suction filtering was carried out, and the operations of dry sample loading, column chromatography and gradient elution with 1% ammonia water+8%-10% methanol/dichloromethane were carried out, thus obtaining 0.28 g of the product with a yield of 72%. 45-70

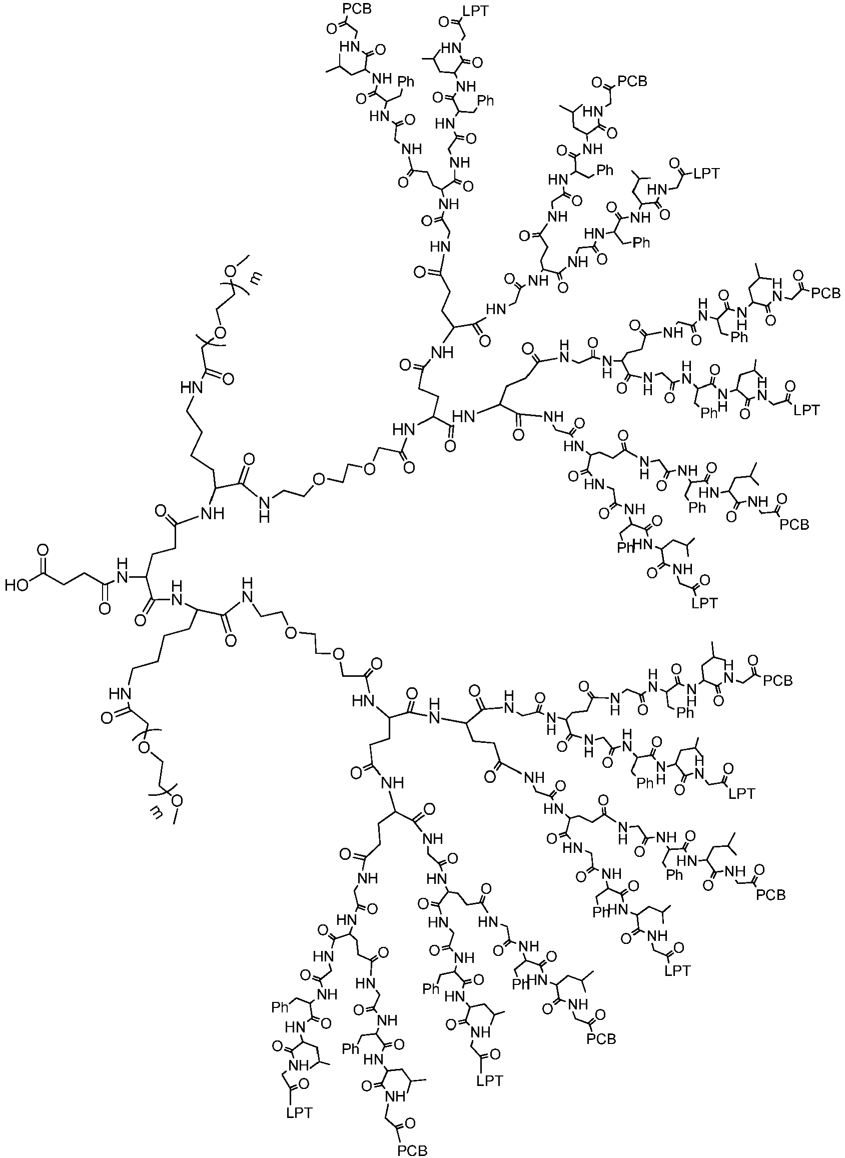

Product 45-69 (0.2 g, 0.01177 mmol) was dissolved in DMF (15 mL) and DIEA (0.0195 mL, 0.1177 mmol) was added dropwise, and then the mixed solution was dissolved by ultrasonic with M-SCM-10K (0.25 g, 0.0241 mmol, purchased from JenKem), and the obtained solution slowly reacted in the dark. At the end of the reaction, methyl tert-butyl ether (150 mL) and n-hexane (70 mL) were added to the reaction solution to separate out a solid product, suction filtering was carried out, and the obtained solid was dissolved with 20% methanol/dichloromethane; silica gel powder (3 g) was added to the obtained solution, and the solution was evaporated to dryness, column chromatography, and gradient elution with 1% ammonia water+5%-7% methanol/dichloromethane were carried out, thus obtaining 0.3 g of the product with a yield of 67%.

¹H-NMR (600 MHz, DMSO-*d*₆) δ 10.13-9.92 (m, 25H), 9.05-8.95 (m, 21H), 8.82-8.78 (m, 9H), 8.56-8.54 (m, 9H), 8.20-7.75(m, 142H), 7.48-7.10 (m, 200H), 7.07-6.90 (m, 10H), 6.70-6.68 (m, 15H),6.53-6.44(m, 10H), 5.81-5.76 (m, 10H), 5.28-5.26 (m, 10H), 4.75-4.71 (m, 6H), 4.57-4.52 (m, 7H), 4.36-4.23 (m, 18H), 3.80 -3.78(m, 32H), 3.51 -3.49(m, 1963H), 3.24 -3.22(m, 35H), 2.90-2.88 (m, 63H), 2.74-2.68 (m,65H), 5.42-2.39 (m, 21H), 2.30-2.28 (m,12H), 1.34-1.30 (m, 58H), 1.24-1.22 (m, 131H), 0.90-0.86(m, 96H). 45-80

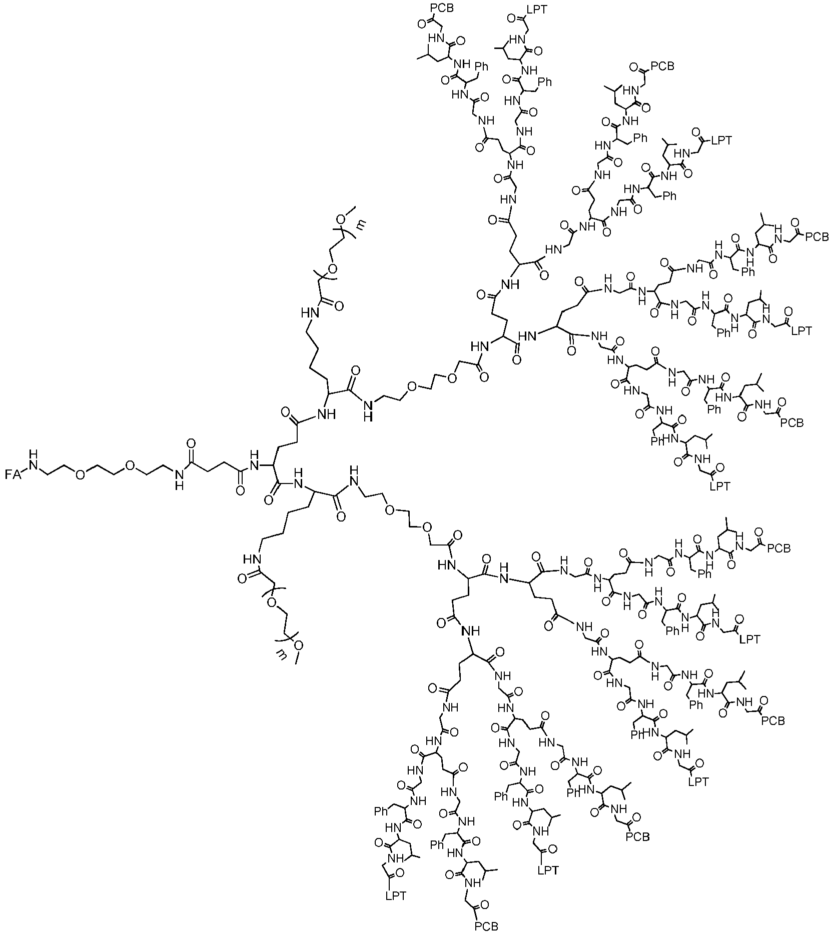

Product 45-70 (0.25 g, 0.00659 mmol), Product 37-163 (0.006 g, 0.0105 mmol), HBTU (0.0062 g, 0.0165 mmol) and HOBT (0.0022 g, 0.0165 mmol) were weighed and added in a 250 mL flask and then dissolved in DMF solution, and the obtained solution was stirred at -5 °C for 30 min. DIEA (0.027 mL, 0.1648 mmol) was slowly added dropwise, and the obtained solution reacted at -5 °C for 2 h and then transferred to room temperature. At the end of the reaction, methyl tert-butyl ether and n-hexane were added to precipitate the reaction solution, suction filtering was carried out, and the operations of dry sample loading, column chromatography and elution with 1% ammonia water+6%-10% methanol/dichloromethane were carried out, thus obtaining 0.22 g of the product with a yield of 88%.

¹H-NMR (600 MHz, DMSO-*d*₆) δ 10.11-10.10 (m, 6H), 9.84-9.82 (m, 6H), 9.04-8.98 (m, 20H), 8.75-8.54 (m, 14H), 8.28- 7.94 (m, 91H), 7.95-7.73 (m, 33H), 7.47-7.42 (m, 10H), 7.40 - 6.99 (m, 259H),6.90-6.88 (m, 2H), 6.69-6.67 (m, 26H), 5.33 - 5.24 (m, 20H), 4.74-4.58 (m, 21H), 4.25-4.22 (m, 20H), 3.81-3.79 (m, 18H), 3.51-3.49 (m, 1900H), 3.26-3.24 (m, 28H), 2.41 -2.39(m, 12H), 2.30 - 2.29 (m, 13H), 1.99 1.95(m, 30H),1.39- 1.34 (m, 96H), 1.30-1.23 (m, 212H), 0.90-0.86 (m, 96H).

### Synthesis of Compound 49-137

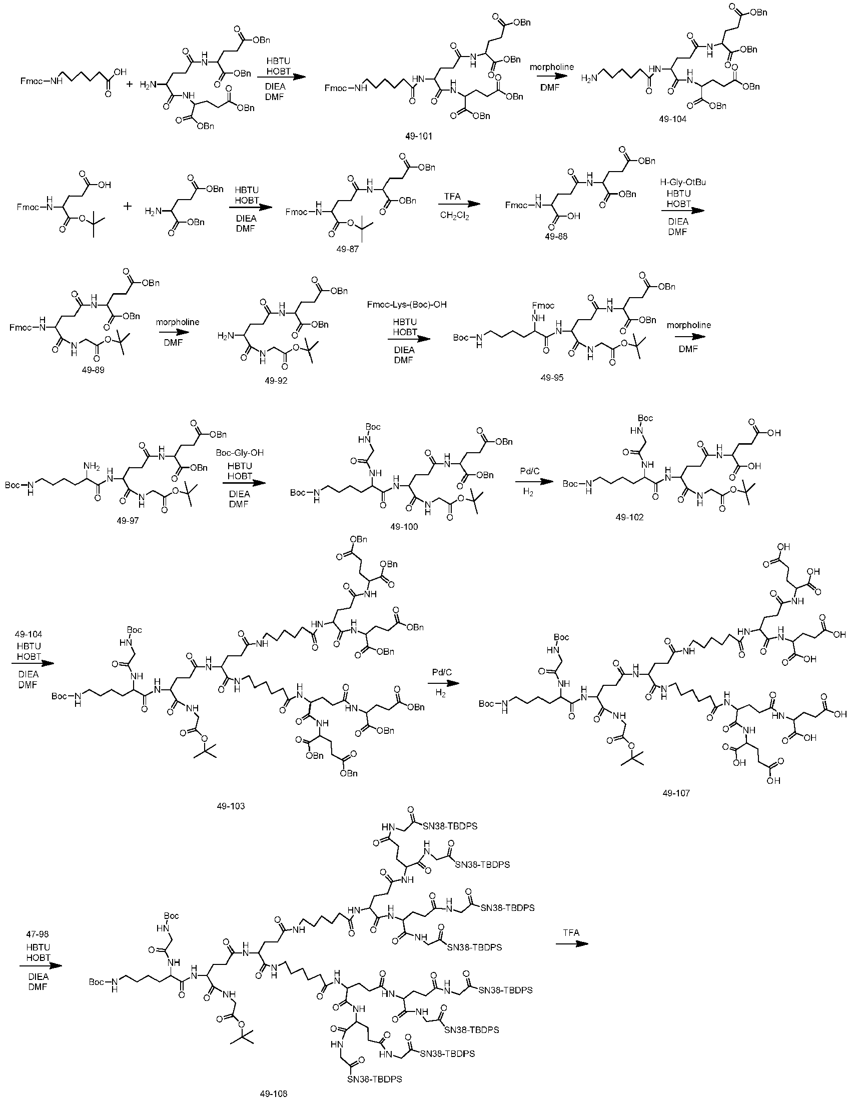

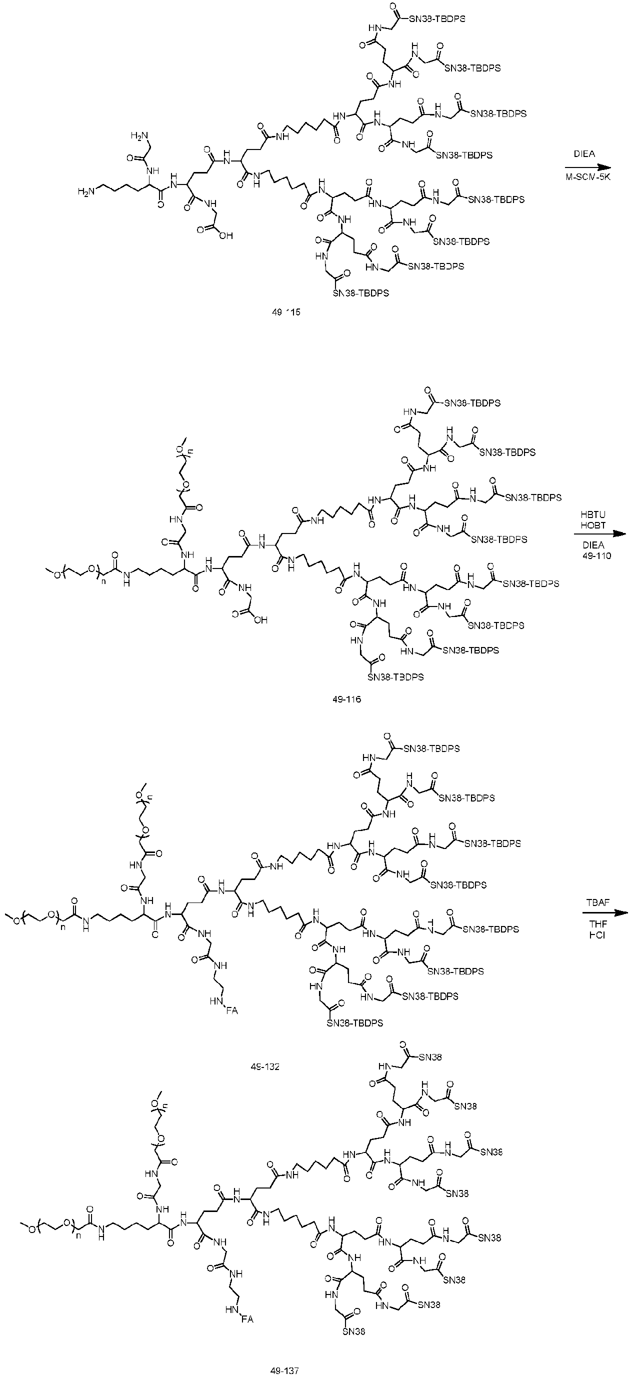

49-87

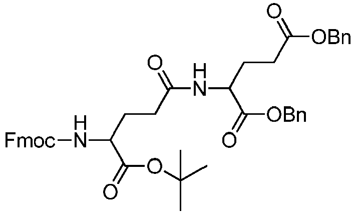

Fmoc-Glu(OtBu)(OH) (3 g, 7.0510 mmol, purchased from Ark Pharm), HBTU (4.01 g, 10.5765 mmol) and HOBT (1.43 g, 10.5765 mmol) were weighed and added in a flask with H-Glu(OBzl)·OBzl·TosOH (3.70 g, 7.4036 mmol, purchased from Ark Pharm) and then dissolved with an appropriate amount of DMF, and the obtained solution was placed at -5 °C; DIEA (5.24 mL, 31.7296 mmol) was slowly added dropwise; after addition and reaction for 30 min, the reaction solution was taken out, and the obtained solution was stirred overnight at room temperature to react. At the end of the reaction, saturated NaCl (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (7.32 g being extra-quota product). 49-88

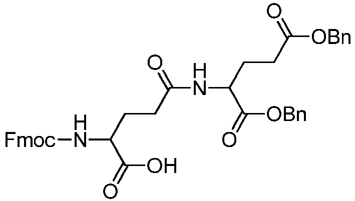

Dichloromethane was added in a flask with Product 49-87 (5.18 g, 7.0510 mmol) and dissolved completely by ultrasonic vibration; TFA (5.23 mL, 70.510 mmol) was added, and the reaction solution was stirred at room temperature overnight to react. At the end of the reaction, dichloromethane and most of the TFA were evaporated to dryness; a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and dried in a vacuum oven, thus obtaining the product (6.89 g being extra-quota product). 49-89

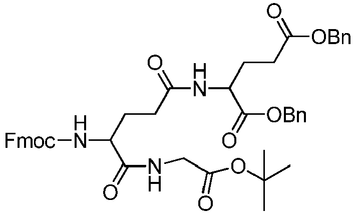

Product 49-88 (4.78 g, 7.0510 mmol), HBTU (4.01 g, 10.5765 mmol) and HOBT (1.43 g, 10.5765 mmol) were weighed and added in a flask with H-Gly-OtBu (1.24 g, 7.4036 mmol, purchased from Accela) and then dissolved with an appropriate amount of DMF, and the obtained solution was placed at -5 °C; then, DIEA (5.24 mL, 31.7296 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (6.38 g being extra-quota product). 49-92

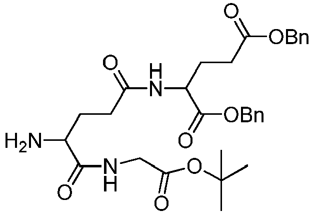

Product 49-89 (5.58 g, 7.0510 mmol) in a flask was dissolved completely with DMF by ultrasonic vibration; morpholine (6.14 mL, 70.510 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (5.6 g being extra-quota product). 49-95

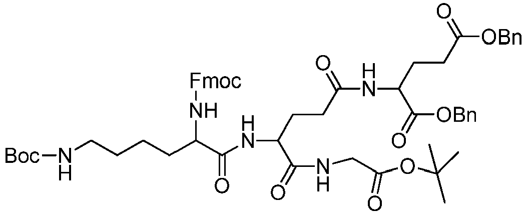

Product 49-92 (4.01 g, 7.0510 mmol), HBTU (4.01 g, 10.5765 mmol) and HOBT (1.43 g, 10.5765 mmol) were weighed and added in a flask with Fmoc-Lys (Boc)-OH (3.6 g, 7.7561 mmol) and then dissolved with an appropriate amount of DMF, and the obtained solution was placed at -5 °C; then, DIEA (5.24 mL, 31.7296 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (8.69 g being extra-quota product). 49-97

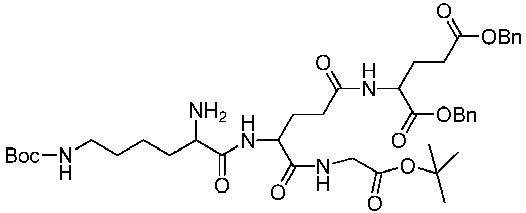

Product 49-95 (7.19 g, 7.0510 mmol) in a flask was dissolved completely with DMF by ultrasonic vibration; morpholine (6.14 mL, 70.510 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, evaporated into a solid, and dried in a vacuum oven, thus obtaining the product (5.6 g being extra-quota product). 49-100

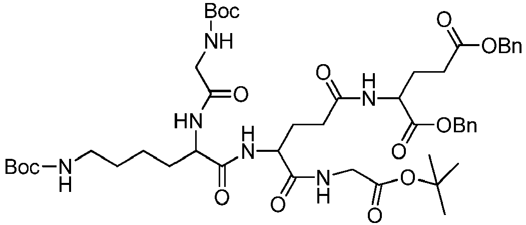

Product 49-97 (5.62 g, 7.0510 mmol), HBTU (4.01 g, 10.5765 mmol) and HOBT (1.43 g, 10.5765 mmol) were weighed and added in a flask with Boc-Gly-OH (1.36 g, 7.7561 mmol, purchased from Aladdin), and then dissolved with an appropriate amount of DMF, and the obtained solution was placed at -5 °C; then, DIEA (5.24 mL, 31.7296 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and the solid product was then dissolved with a mixed solution of methanol/dichloromethane (1:4) (100 mL); silica gel powder (100 mL) was then added to the obtained solution and the solution was evaporated to be powder; the operations of dry sample loading, column chromatography and gradient elution with a mixed solution (1%-3% methanol/dichloromethane) were carried out; the elution product was collected, concentrated and dried in a vacuum oven, thus obtaining the product (2.5 g, 37.15 %). 49-102

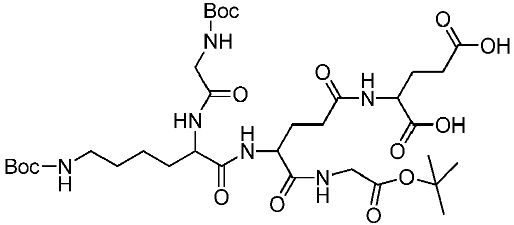

Product 49-100 (1.5 g, 1.5705 mmol) was added in a hydrogenation reactor and then 10% Pd/C (0.05 g) was added, and the mixture was dissolved with DMF (20 mL); hydrogen was introduced to a pressure of 300 Psi in the reactor, and the reaction solution was stirred overnight at room temperature to react. At the end of the reaction, the reaction solution was filtered with diatomaceous earth, and the filter cake was washed three times with DMF (15 mL × 3); the elution solution was added in a 250 mL round-bottomed flask, and the product was obtained, as the raw material for the next step. 49-101

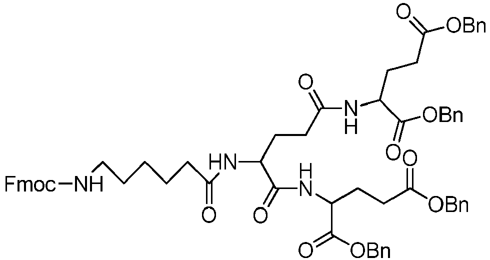

Product 39-84 (8 g, 10.4459 mmol), HBTU (5.94 g, 15.6689 mmol) and HOBT (2.12 g, 15.6689 mmol) were weighed and added in a flask with Product 49-17 (3.69 g, 10.4459 mmol) and then dissolved with an appropriate amount of DMF, and the obtained solution was placed at -5 °C; then, DIEA (7.77 mL, 47.0066 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and the solid product was then dissolved with a mixed solution of methanol/dichloromethane (1:4) (100 mL); silica gel powder (100 mL) was added to the obtained solution, and the solution was then evaporated to dryness to obtain a powdery solid; the operations of dry sample loading, column chromatography, and elution with 100% dichloromethane were carried out; the elution product was then collected, concentrated, and dried in a vacuum oven. The product (9.6 g, 83.48 %) was obtained. 49-104

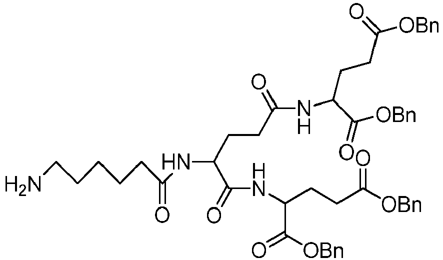

Product 49-101 (9.6 g, 8.7193 mmol) in a flask was dissolved completely with DMF by ultrasonic vibration; morpholine (7.59 mL, 87.193 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered; the organic phases were collected, and the aqueous phase was then washed with EA (200 mL × 3); the obtained organic phases were combined, and evaporated into a solid. The solid product was then dissolved with a mixed solution of methanol/dichloromethane (1:4) (100 mL); silica gel powder (100 mL) was then added to the obtained solution and the solution was evaporated to be powder; the operations of dry sample loading, column chromatography and gradient elution with a mixed solution (3%-5% methanol/dichloromethane) were carried out; the elution product was collected, concentrated and dried in a vacuum oven. The product (5.3 g, 69.19 %) was obtained. 49-103

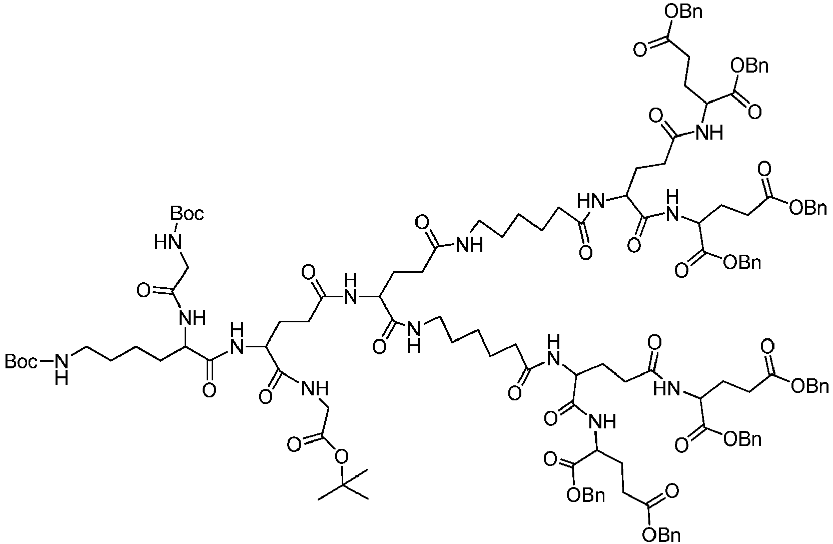

Product 49-102 (1.21 g, 1.5705 mmol), HBTU (0.89 g, 2.3558 mmol) and HOBT (0.32 g, 2.3558 mmol) were weighed and added in a flask with Product 49-104 (3.03 g, 3.4551 mmol) and then dissolved with an appropriate amount of DMF, and the obtained solution was placed at -5 °C; then, DIEA (2.34 mL, 14.1345 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), and the obtained organic phases were combined, evaporated into a solid, and then dissolved with a mixed solution of methanol/dichloromethane (1:4) (100 mL); silica gel powder (100 mL) was then added to the obtained solution and the solution was evaporated to be powder; the operations of dry sample loading, column chromatography and elution with a mixed solution (2%-4% methanol/dichloromethane) were carried out; the elution product was collected, concentrated and dried in a vacuum oven. The product (2.6 g, 66.49 %) was obtained.

¹H-NMR (600 MHz, DMSO-*d*₆) δ 8.42 - 8.26 (m, 4H), 8.20 - 8.10 (m, 1H), 7.99 - 7.72 (m, 6H), 7.40 - 7.26 (m, 40H), 6.96 (s, 1H), 6.71 (s, 1H), 5.17 - 4.99 (m, 17H), 4.39 - 4.13 (m, 9H), 3.82 - 3.50 (m, 3H), 3.10 - 2.81 (m, 7H), 2.65 - 2.57 (m, 2H), 2.48 - 2.30 (m, 9H), 2.22 - 1.59 (m, 31H), 1.46 - 1.32 (m, 27H), 1.28 - 1.05 (m, 10H). 49-107

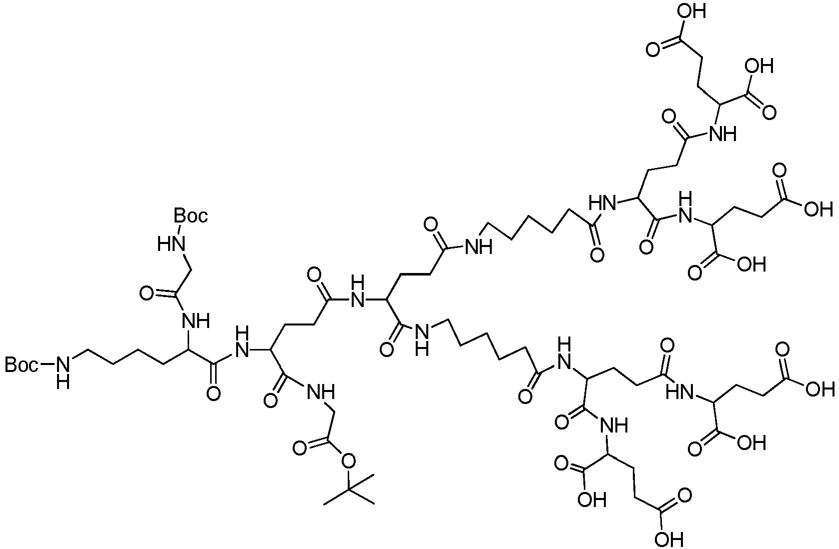

Product 49-103 (2.6 g, 1.0416 mmol) was added in a hydrogenation reactor and then 10% Pd/C (0.05 g) was added, and the mixture was dissolved with DMF (20 mL); hydrogen was introduced to a pressure of 300 Psi in the reactor, and the reaction solution was stirred overnight at room temperature to react. At the end of the reaction, the reaction solution was filtered with diatomaceous earth, and the filter cake was washed three times with DMF (15 mL × 3); the elution solution was added in a 250 mL round-bottomed flask, and the product was obtained, as the raw material for the next step. 49-108

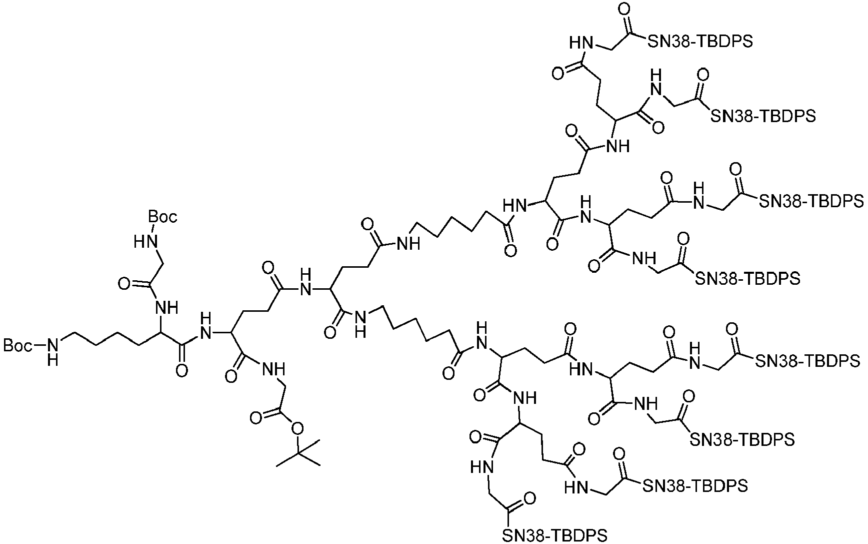

Product 47-98 (6.02 g, 8.7494 mmol), HBTU (0.89 g, 2.3558 mmol) and HOBT (0.32 g, 2.3558 mmol) were weighed and added to the flask with Product 49-107 (1.85 g, 1.0416 mmol) and then dissolved with an appropriate amount of DMF, and the obtained solution was placed at -5 °C; then, DIEA (1.55 mL, 9.3744 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3); the obtained organic phases were combined, and evaporated into a solid; the solid product was then dissolved with a mixed solution of methanol/dichloromethane (1:4) (100 mL); silica gel powder (100 mL) was then added to the obtained solution and the solution was evaporated to be powder; the operations of dry sample loading, column chromatography and elution with a mixed solution (2%-6% methanol/dichloromethane) were carried out; the elution product was collected, concentrated and dried in a vacuum oven. The product (7.43 g, 100%) was obtained.

¹H-NMR (600 MHz, DMSO-*d*₆) δ 8.10 - 7.85 (m, 15H), 7.79 - 7.70 (m, 33H), 7.55 - 7.38 (m, 61H), 7.13 - 6.95 (m, 17H), 5.55 - 5.29 (m, 17H), 5.26 - 4.90 (m, 18H), 4.34 - 3.81 (m, 24H), 3.17 - 2.54 (m, 43H), 2.22 - 1.98 (m, 36H), 1.93 - 1.52 (m, 15H), 1.50 - 1.05 (m, 99H), 0.94 - 0.58 (m, 53H). 49-115

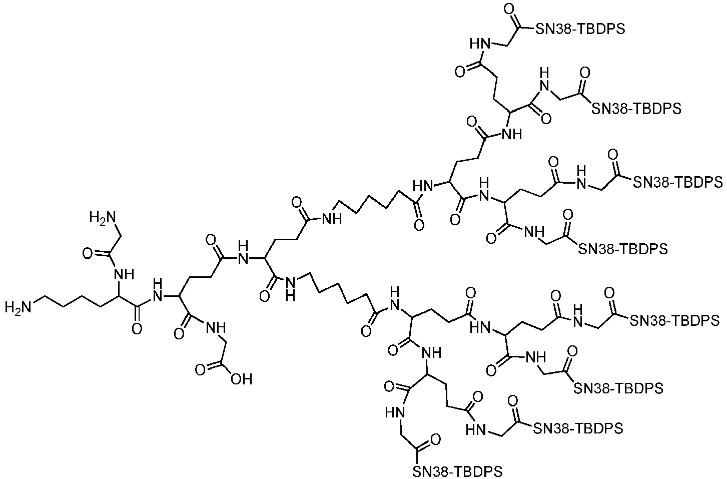

Dichloromethane was added in a flask with Product 49-108 (7.43 g, 1.0416 mmol) and dissolved completely by ultrasonic vibration; TFA (0.77 mL, 10.416 mmol) was added, and the reaction solution was stirred at room temperature overnight to react. At the end of the reaction, dichloromethane and most of the TFA were evaporated to dryness, and then methyl tert-butyl ether (200 mL) was added for precipitation, the supernatant was discarded, and such operations were repeated three times; filtering was then carried out to obtain a solid product, and the obtained solid product was dissolved with a mixed solvent (dichloromethane and methanol), the operations of dry sample loading, column chromatography, and elution with a mixed solution (3-5% methanol/dichloromethane) were carried out; the elution product was collected, concentrated and dried in a vacuum oven. The product (2.9 g, 40.50%) was obtained. 49-116

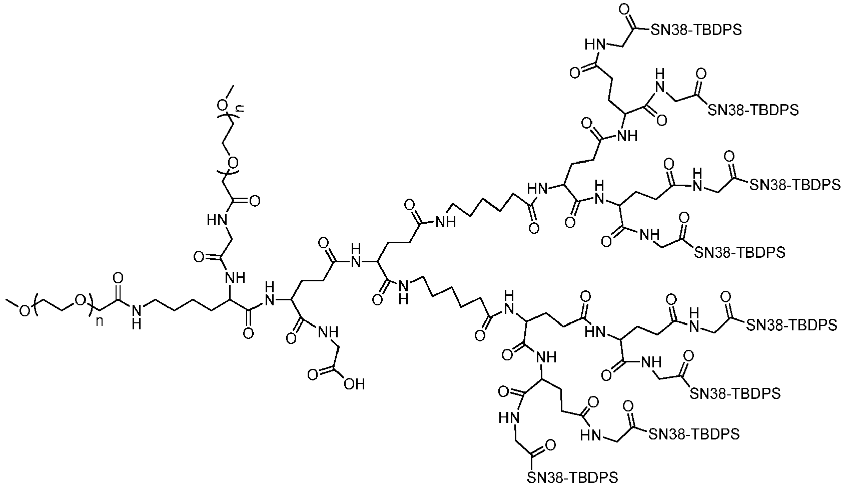

Product 49-115 (1 g, 0.1454 mmol) was added in a 250 mL flask and then dissolved with DMF (20 mL), and M-SCM-5K (1.70 g, 0.3198 mmol, purchased from JenKem) was added and dissolved by ultrasonic vibration, and the obtained solution was placed at -5 °C; DIEA (0.72 mL, 4.3512 mmol) was slowly added dropwise; after addition and reaction for 30 min, the reaction solution was taken out, and the obtained solution was slowly stirred at room temperature to react for one week in the dark. After the reaction was completed, n-hexane (25 mL) and methyl tert-butyl ether (200 mL) were first added for precipitation, and the supernatant was discarded; n-hexane and methyl tert-butyl ether were added again for precipitation; such operations were repeated three times, and the filtering was then carried out to obtain a solid product; the obtained solid product was dissolved with a mixed solvent (dichloromethane and methanol), the operations of dry sample loading, column chromatography, and elution with a mixed solution (6-10% methanol/dichloromethane) were carried out; the elution product was collected, concentrated and dried in a vacuum oven. The product (1.3 g, 51.79%) was obtained.

¹H-NMR (400 MHz, DMSO-*d*₆) δ 8.59 - 7.85 (m, 67H), 7.59 - 6.88 (m, 67H), 5.58 - 5.16 (m, 47H), 4.15 - 3.93 (m, 37H), 3.51- 3.49 (m, 1093H), 3.26 - 3.23 (m, 15H), 3.11 - 2.70 (m, 59H), 2.07 (s, 32H), 1.50 - 1.11 (m, 60H), 0.93 - 0.80 (m, 26H). 49-132

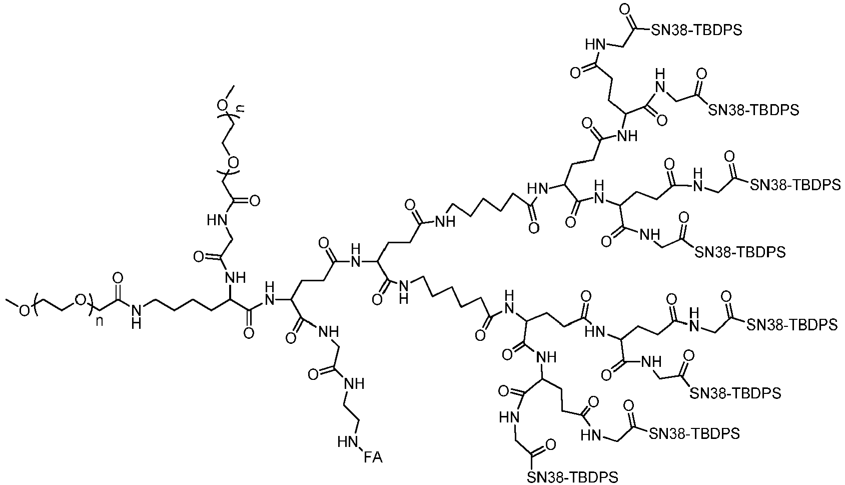

Product 49-110 (0.09 g, 0.1506 mmol), HBTU (0.04 g, 0.1129 mmol) and HOBT (0.02 g, 0.1129 mmol) were weighed and added in a flask with Product 49-116 (1.3 g, 0.0753 mmol) and then dissolved with an appropriate amount of DMF, and the obtained solution was placed at -5 °C; then, DIEA (0.35 mL, 2.1084 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, and evaporated into a solid; the solid product was then dissolved with a mixed solution of methanol/dichloromethane (1:4) (100 mL); silica gel powder (100 mL) was then added to the obtained solution and the solution was evaporated to be powder; the operations of dry sample loading, column chromatography and elution with a mixed solution (2%-8% methanol/dichloromethane) were carried out; the elution product was collected, concentrated and dried in a vacuum oven. The product (0.9 g, 67.16%) was obtained. 49-137

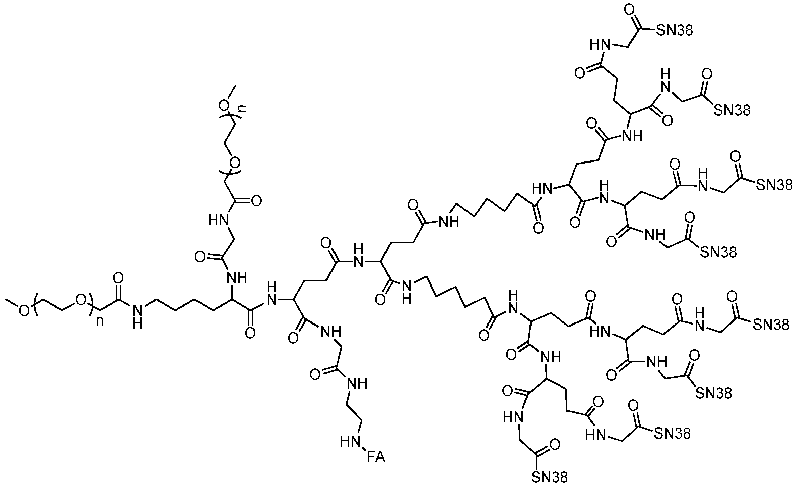

Product 49-132 (0.9 g, 0.0505 mmol), TBAF (0.21 g, 0.8080 mmol), THF (20 mL) and dilute HCl solution (20 mL) were added in a 500 mL round-bottomed flask, and the reaction solution was stirred at room temperature overnight to react. After the reaction was completed, the reaction solution was concentrated under reduced pressure, and the solid product was obtained. The obtained solid was dissolved with DMF (5 mL), precipitated with isopropanol, the above operations were repeated three times, and the solid product was obtained by filtering; The obtained solid was dissolved with dichloromethane (10 mL), precipitated with methyl tert-butyl ether, and such operations were repeated three times to obtain a solid product, and the obtained solid was dried, thus obtaining the product (0.68 g, 83.95%).

¹H-NMR (600 MHz, DMSO-*d*₆) δ 8.48 - 8.28 (m, 35H), 8.04 - 7.86 (m, 34H), 7.44 - 7.28 (m, 32H), 7.05 - 6.89 (m, 26H), 6.64 - 6.52 (m, 16H), 5.48 - 5.35 (m, 30H), 5.28 - 5.12 (m, 28H), 4.15 - 3.98 (m, 34H), 3.51 (s, 1091H), 2.07 (s, 51H), 1.48 - 1.09 (m, 84H), 0.93 - 0.78 (m, 40H).

### Synthesis of Product 43-197

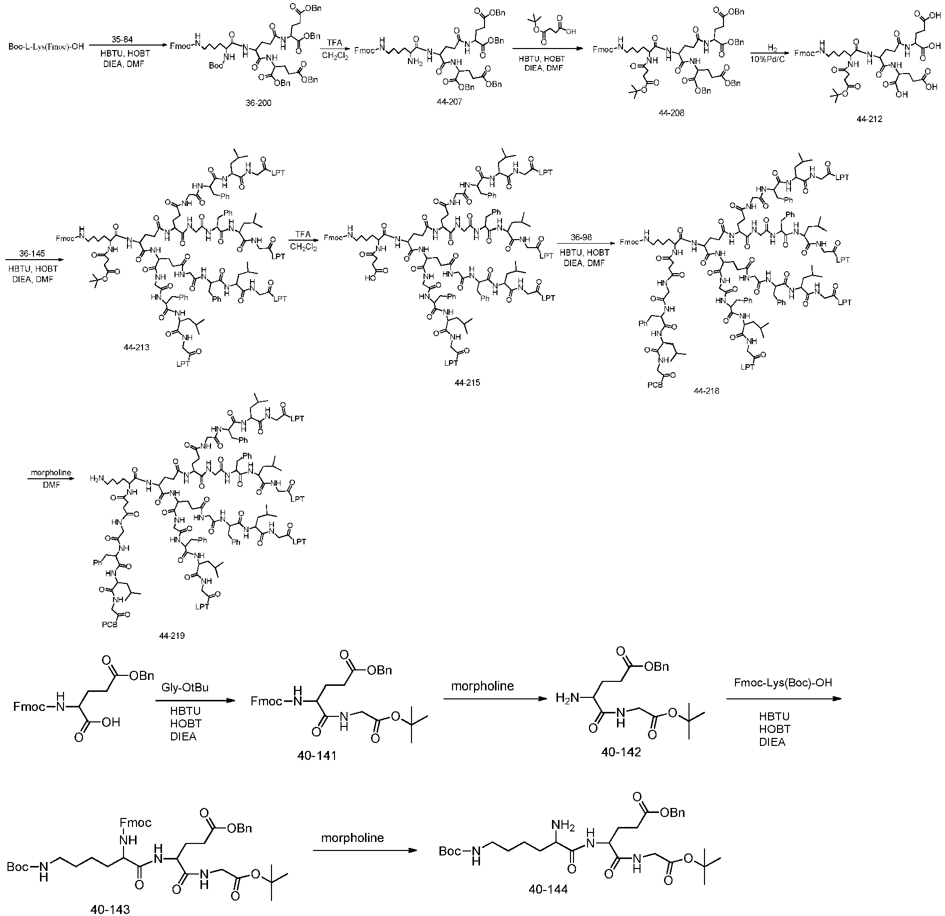

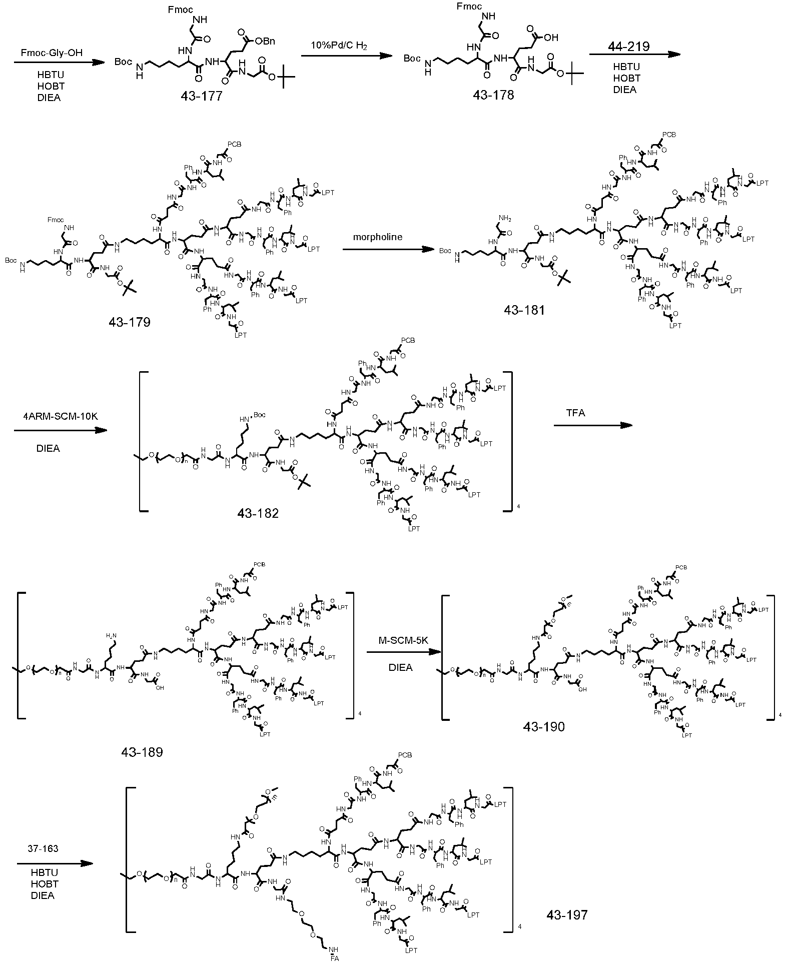

36-200

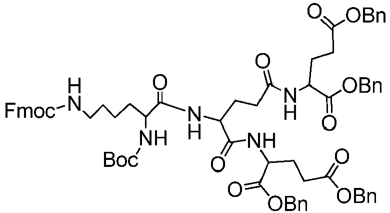

Product 35-84 (8.99 g, 11.7386 mmol), Boc-L-Lys(Fmoc)-OH (5.0 g, 10.6714 mmol), HBTU (6.0706 g, 16.0072 mmol) and HOBT (2.1629 g, 16.0072 mmol) were added in a 500 mL round-bottomed flask and then dissolved with DMF (80 mL), and the obtained solution in the flask was stirred at -5 °C for about 30 min; then, DIEA (10.6 mL, 64.0287 mmol) was slowly added dropwise, and at the end of the addition, the reaction solution in the flask was stirred at -5 °C for about 3 h. At the end of the reaction, the reaction solution was transferred to a 1 L separatory funnel, a saturated sodium chloride solution (300 mL) and ethyl acetate (200 mL) were then added, the obtained solution was shaken and extracted, and the aqueous phase and the organic phase were separated; ethyl acetate (100 mL) was then added to the aqueous phase, the obtained solution was shaken and extracted, and the aqueous phase and the organic phase were separated. Then, the obtained organic phases were combined, a saturated sodium chloride solution (300 mL) was added to the organic phase, the obtained solution was shaken and extracted, and the aqueous phase and the organic phase were separated; deionized water (300 mL) was added to the organic phase, the obtained solution was shaken and extracted, and the aqueous phase and the organic phase were separated. Finally, the organic phase was concentrated, evaporated to dryness, and dried, thus obtaining 12.9804 g of Product 36-200. 44-207

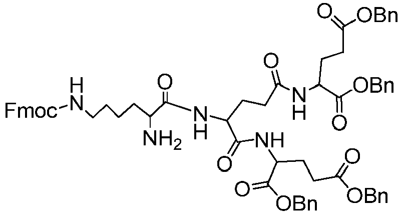

Product 36-200 (11.9 g, 9.7832 mmol) was added in a 500 mL round-bottomed flask and then dissolved with dichloromethane (20 mL), TFA (10.9 mL, 146.748 mmol) was then added with stirring, and the obtained solution in the flask was stirred at room temperature to react. At the end of the reaction, the reaction solution was concentrated at reduced pressure to remove dichloromethane; methyl tert-butyl ether (150 mL) was then added to precipitate the reaction solution and a powdery solid was obtained; the obtained solid was filtered, washed with methyl tert-butyl ether (100 mL), thus obtaining 8.351 g of Product 44-207 with a yield of 76.47%. 44-208

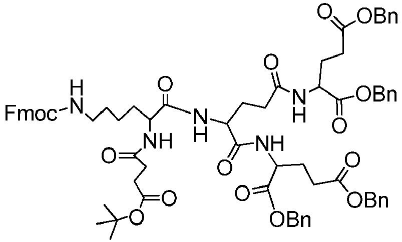

Mono-tert-butyl succinate (1.5638 g, 8.9775 mmol), Product 44-207 (8.351 g, 7.4812 mmol), HBTU (4.2558 g, 11.2218 mmol) and HOBT (1.5163 g, 11.2218 mmol) were weighed and added in a 500 mL round-bottomed flask and then dissolved in DMF solution (80 mL), and the obtained solution in the flask was stirred at -5 °C for about 30 min; then, DIEA (9.9 mL, 59.8499 mmol) was slowly added dropwise, and at the end of the addition, the reaction solution in the flask was stirred at -5 °C for about 3 h. At the end of the reaction, the reaction solution was transferred to a 1 L separatory funnel, a saturated sodium chloride solution (300 mL) and ethyl acetate (200 mL) were then added, the obtained solution was shaken and extracted, and the aqueous phase and the organic phase were separated; ethyl acetate (100 mL) was added to the aqueous phase, the obtained solution was shaken and extracted, and the aqueous phase and the organic phase were separated. Then, the obtained organic phases were combined, a saturated sodium chloride solution (300 mL) was then added to the organic phase, the obtained solution was shaken and extracted, and the aqueous phase and the organic phase were separated; deionized water (300 mL) was then added to the organic phase, the obtained solution was shaken and extracted, and the aqueous phase and the organic phase were separated. Finally, the organic phase was concentrated, evaporated to dryness, and dissolved with a mixed solvent (100 mL) (20% methanol/dichloromethane); silica gel powder (50 mL) was added to the obtained solution, and the solution was then evaporated to dryness; the operations of dry sample loading, column chromatography, and elution with an eluent (2%-8% methanol: 98%-92% dichloromethane) were carried out; the elution product was then collected, concentrated, and evaporated to dryness. 2.5268 g of Product 44-208 was obtained with a yield of 35.85%. 44-212

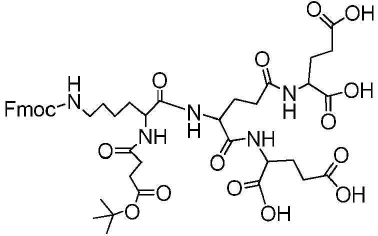

Product 44-208 (2.5268 g, 1.9858 mmol) and 10% Pd/C (60 mg) were added in a hydrogenation reactor and then dissolved with DMF (30 mL); the hydrogenation reactor was then sealed, hydrogen was introduced to a pressure of 2.2 MPa in the reactor; the hydrogenation reactor was placed at room temperature and the rection solution was stirred overnight. At the end of the reaction, the reactor was taken out, and the reaction solution was evenly added dropwise to a sand core funnel filled with compacted diatomaceous earth, suction filtering was carried out, and the diatomaceous earth was washed with DMF until it did not contain any product, thus obtaining the reaction solution of the product. 44-213

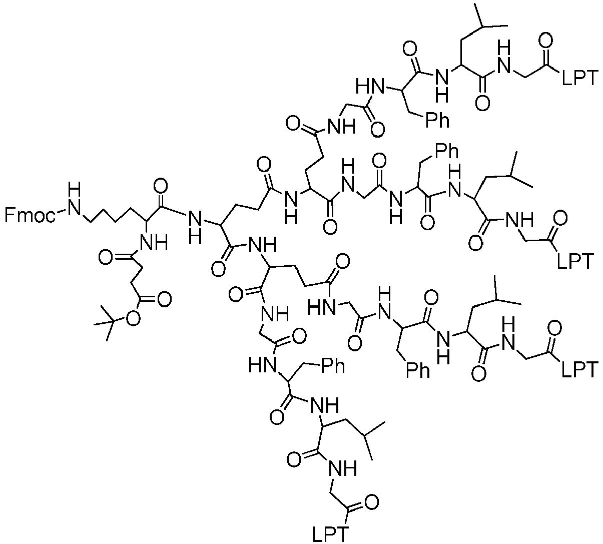

Solution of Product 44-212 (1.0846 g, 1.1893 mmol), Product 36-145 (5.0 g, 5.2329 mmol), HBTU (2.7062 g, 7.1358 mmol) and HOBT (0.9642 g, 7.1358 mmol) were added in a 500 mL round-bottomed flask and then dissolved with DMF (80 mL); the obtained solution in the flask was stirred at -5 °C for about 30 min; then, DIEA (3.5 mL, 21.4074 mmol) was slowly added dropwise; at the end of the addition, the reaction solution in the flask was stirred at -5 °C for 1 h to react, and finally stirred at room temperature for 3 h to react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product, and the process of precipitation was repeated three times to obtain an oily solid; the oily solid was dissolved with dichloromethane (10 mL), precipitated with methyl tert-butyl ether (150 mL) to obtain a powdery solid, filtering was carried out, and the obtained solid was washed with methyl tert-butyl ether (60 mL), and finally dried in an oven, thus obtaining 5.5443 g of Product 44-213. 44-215

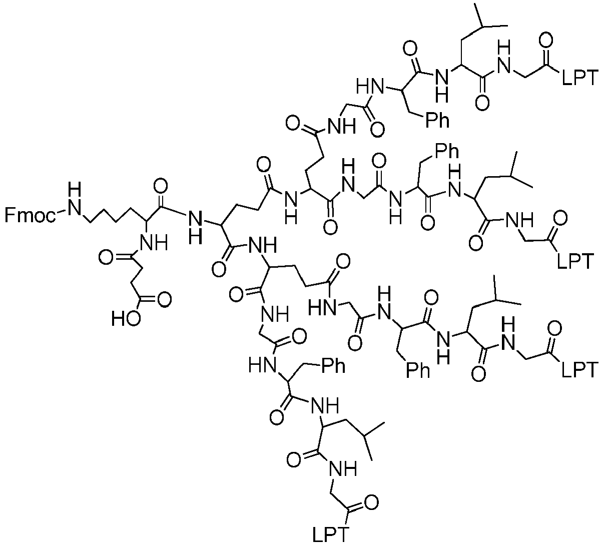

Product 44-213 (5.5443 g, 1.1893 mmol) was added in a 500 mL round-bottomed flask and then dissolved with dichloromethane (20 mL), TFA (1.3 mL, 17.8395 mmol) was then added with stirring, and the obtained solution in the flask was stirred at room temperature to react. At the end of the reaction, the reaction solution was concentrated at reduced pressure to remove dichloromethane. Methyl tert-butyl ether (150 mL) was added to precipitate the reaction solution and a powdery solid was obtained, filtering was then carried out, and the obtained solid was washed with methyl tert-butyl ether (100 mL), thus obtaining 5.0 g of Product 44-215 with a yield of 91.28%. 44-218

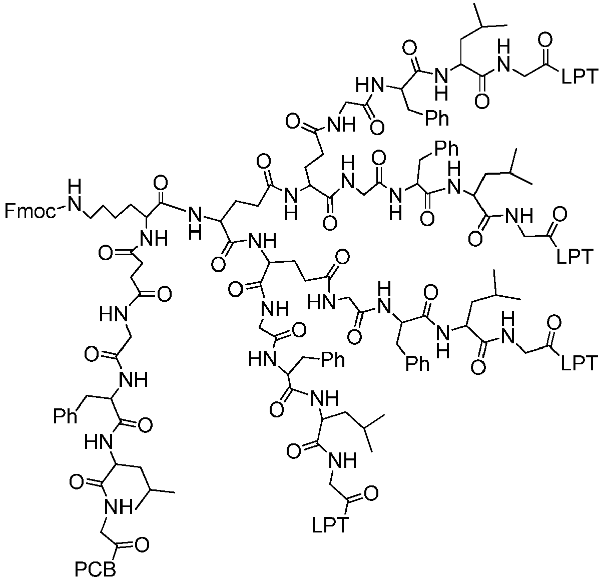

Product 44-215 (5.0 g, 1.0856 mmol), Product 36-98 (1.16 g, 1.4113 mmol), HBTU (0.6176 g, 1.6284 mmol) and HOBT (0.22 g, 1.6284 mmol) were added in a 500 mL round-bottomed flask and then dissolved with DMF (80 mL), and the obtained solution in the flask was stirred at -5 °C for about 30 min; then, DIEA (1.1 mL, 6.5136 mmol) was slowly added dropwise; at the end of the addition, the reaction solution in the flask was stirred at -5 °C for 1 h, and finally stirred at room temperature for 3 h to react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product; the process of precipitation was repeated three times to obtain an oily solid; the oily solid was dissolved with dichloromethane (10 mL), and then precipitated with methyl tert-butyl ether (150 mL) to obtain a powdery solid; filtering was carried out, and the solid was washed with methyl tert-butyl ether (60 mL), and finally dried in a vacuum oven, thus obtaining 5.8728 g of Product 44-218. 44-219

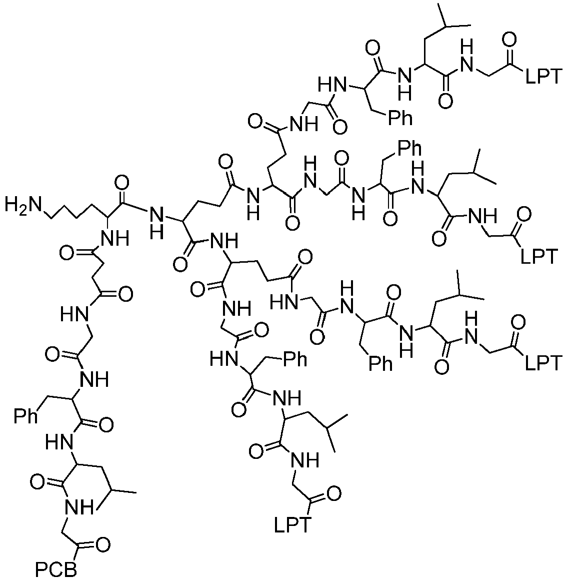

Product 44-218 (5.8728 g, 1.0856 mmol) was added in a 500 mL round-bottomed flask and then dissolved with DMF (20 mL), morpholine (1.9 mL, 21.7120 mmol) was then added with stirring, and the obtained solution in the flask was stirred at room temperature for 2 h to react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product. Such operations were repeated five times to obtain an oily solid. The oily solid was dissolved with dichloromethane (10 mL), and precipitated with methyl tert-butyl ether (150 mL) to obtain a powdery solid; filtering was carried out to obtain a solid product, and the obtained solid product was washed with methyl tert-butyl ether (60 mL), and dissolved with a mixed solvent (100 mL) (20% methanol/dichloromethane); silica gel powder (60 mL) was added to the obtained solution, and the solution was then evaporated to dryness; the operations of dry sample loading, column chromatography, and elution with an eluent (1% ammonia water: 3%-15% methanol: 96%-84% dichloromethane) were carried out; the elution product was then collected, concentrated, evaporated to dryness, and dried. 0.8841 g of Product 44-219 was obtained with a yield of 15.7%. 40-141

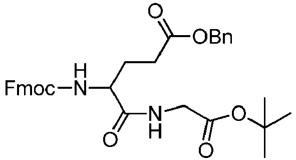

N-Fmoc-D-Glutamic Acid 5-Benzyl Ester (5 g, 10.8 mmol, purchased from damas-beta), HBTU (6.14 g, 16.2 mmol) and HOBT (2.1 g, 16.2 mmol) were weighed and added in a flask with H-Gly-OtBu (1.81 g, 10.8 mmol, purchased from Accela), and then dissolved with an appropriate amount of DMF, and the obtained solution was stirred at -5 °C for 30 min; DIEA (8 mL, 48.6 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), the obtained organic phases were combined, evaporated into a solid, and dried in a vacuum oven, thus obtaining the product (6.38 g being extra-quota product). 40-142

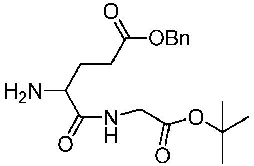

Product 40-141 (6 g, 10.8 mmol) in a flask was dissolved completely with DMF by ultrasonic vibration; morpholine (19 mL, 216 mmol) was added, and the reaction solution was stirred at room temperature for 2 h to react. At the end of the reaction, a saturated NaCl solution (200 mL) and EA (200 mL) were added to the reaction solution, extraction was carried out and the reaction solution was stood still to be layered; the organic phases were collected; the aqueous phase was then washed with EA (200 mL × 3); the obtained organic phases were combined, evaporated into a solid, and dried in a vacuum oven, thus obtaining the product (5 g being extra-quota product). 40-143

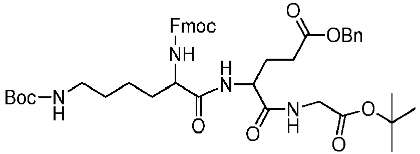

Product 40-142 (4 g, 10.8 mmol), HBTU (6.14 g, 16.2 mmol) and HOBT (2.12 g, 16.2 mmol) were weighed and added in a flask with Fmoc-Lys (Boc)-OH (5 g, 10.8 mmol, purchased from Accela), and then dissolved with an appropriate amount of DMF, and the obtained solution was placed at -5 °C; then, DIEA (8 mL, 48.6 mmol) was slowly added dropwise; after addition and reaction for 30 min, the reaction solution was taken out, and the obtained solution was stirred overnight at room temperature to react. At the end of the reaction, a saturated NaCl solution (200 mL) and EA (200 mL) were added to the reaction solution, extraction was carried out and the reaction solution was stood still to be layered, and the organic phases were collected; the aqueous phase was then washed with EA (200 mL × 3), and the obtained organic phases were combined, evaporated into a solid, and dried in a vacuum oven, thus obtaining the product (8.69 g, being extra-quota product 0.05 g). 40-144

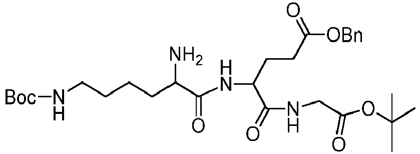

Product 40-143 (8.6 g, 10.8 mmol) in a flask was dissolved completely with DMF by ultrasonic vibration; morpholine (28 mL, 324 mmol) was added, and the reaction solution was stirred at room temperature for 2 h to react. At the end of the reaction, a saturated NaCl solution (200 mL) and EA (200 mL), were added to the reaction solution, extraction was carried out and the reaction solution was stood still to be layered, and the organic phases were collected; the aqueous phase was then washed with EA (200 mL × 3), the obtained organic phases were combined, evaporated into a solid, and dried in a vacuum oven, thus obtaining 6 g of the product with a yield of 96.15%. 43-177

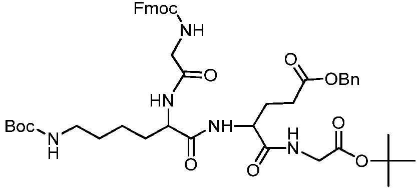

Fmoc-Gly-OH (1.0275 g, 3.456 mmol, purchased from Accela), Product 40-144 (2 g, 3.456 mmol), HBTU (1.9660 g, 5.184 mmol) and HOBT (0.7004 g, 5.184 mmol) were weighed and added in a 250 mL round-bottomed flask and then dissolved in DMF solution (50 mL), and the mixed solution was stirred at 0 °C for about 30 min; then, DIEA (2.5705 mL, 15.5520 mmol) was slowly added dropwise, and at the end of the addition, the reaction solution was further stirred at 0 °C overnight to react. At the end of the reaction, the reaction solution was first transferred to a 1 L separatory funnel, a saturated sodium chloride solution (300 mL) and ethyl acetate (200 mL) were added, and the obtained solution was shaken; the organic phase was separated, extracted once with ethyl acetate (100 mL); the obtained organic phases were combined, and concentrated to a small amount; silica gel powder was then added to the obtained solution, and the solution was then evaporated to dryness; the operations of dry sample loading, column chromatography, and gradient elution with 50%-100% ethyl acetate/petroleum ether were carried out. 2.3 g of the product was obtained with a yield of 79.31%. 43-178

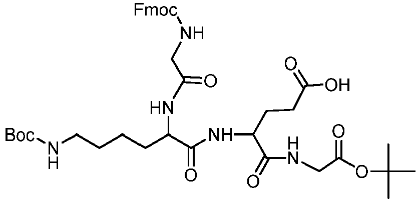

Product 43-177 (0.3308 g, 0.3855 mmol) and 10% Pd/C (20 mg) were added in a hydrogenation reactor and then dissolved with DMF (35 mL); hydrogen (2 MPa) was introduced in the reactor, and the mixed solution then was stirred overnight at room temperature to react. At the end of the reaction, the reaction solution was filtered through a sand core funnel filled with diatomaceous earth, the diatomaceous earth was washed three times with DMF (20 mL × 3), and the DMF solution was combined, as the raw material for the next step. 43-179

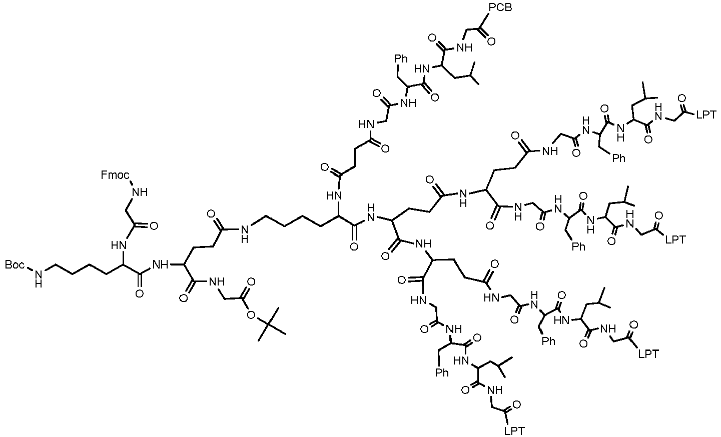

Product 44-219 (2 g, 0.3855 mmol), HBTU (0.2193 g, 0.5783 mmol) and HOBT (0.0781 g, 0.5783 mmol) were added in a 500 mL flask, and the DMF solution of Product 43-178 (150 mL) was added to dissolve the reactants, and the obtained solution was stirred for about 30 min at -5 °C; then, DIEA (0.2878 mL, 1.7348 mmol) was slowly added dropwise, and at the end of the addition, the obtained solution was stirred at -5 °C overnight to react. At the end of the reaction, methyl tert-butyl ether (100 mL) and n-hexane (100 mL) were added to separate out a solid product; the solid product was filtered by suction, and washed with methanol, thus obtaining 1.5 g of the product with a yield of 68.18% 43-181

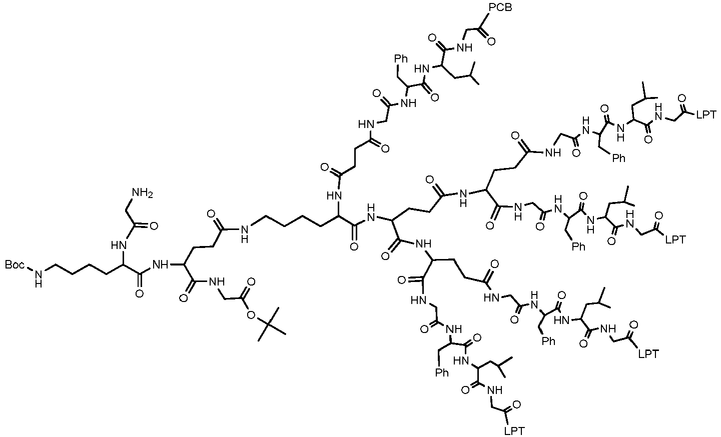

Product 43-179 (1.5 g, 0.2526 mmol) was added in a 500 mL flask and then DMF (30 mL) and morpholine (0.4402 mL, 5.0528 mmol) were added and dissolved completely by ultrasonic vibration, and the mixed solution was stirred at room temperature to react. At the end of the reaction, methyl tert-butyl ether (150 mL) and n-hexane (100 mL) were added to the reaction solution to separate out a solid product, suction filtering was carried out, and the filter cake was washed with methanol, thus obtaining 1.2 g of the product with a yield of 83.13%. 43-182

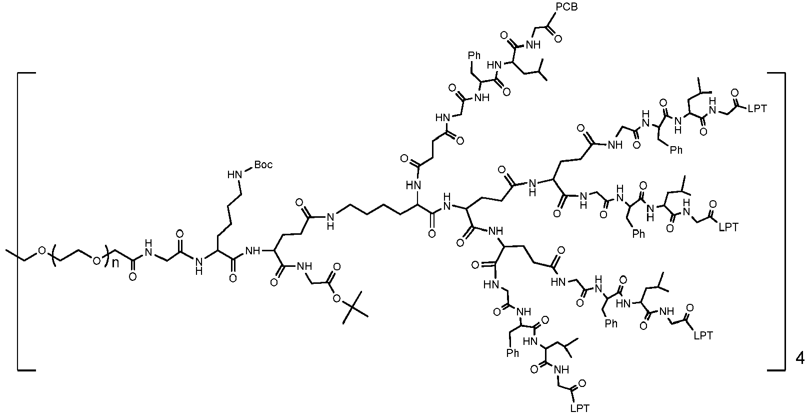

Product 43-181 (1.2 g, 0.2100 mmol) was weighed and added in a 500 mL flask and then dissolved in DMF (20 mL), dissolved by ultrasonic with DIEA (0.1445 mL, 0.874 mmol) and 4ARM-SCM-10K (0.4766 g, 0.0437 mmol, purchased from Jenkem Technology), and the obtained solution slowly reacted in the dark. At the end of the reaction, methyl tert-butyl ether (200 mL) was added to the reaction solution to separate out a solid product, and the solid product was filtered by suction, and dissolved with 20% methanol/dichloromethane; silica gel powder (5 g) was added to the obtained solution, and the solution was evaporated to dryness, column chromatography, and gradient elution with 10%-20% methanol/dichloromethane were carried out, thus obtaining 0.9 g of the product with a yield of 64.29%. 43-189

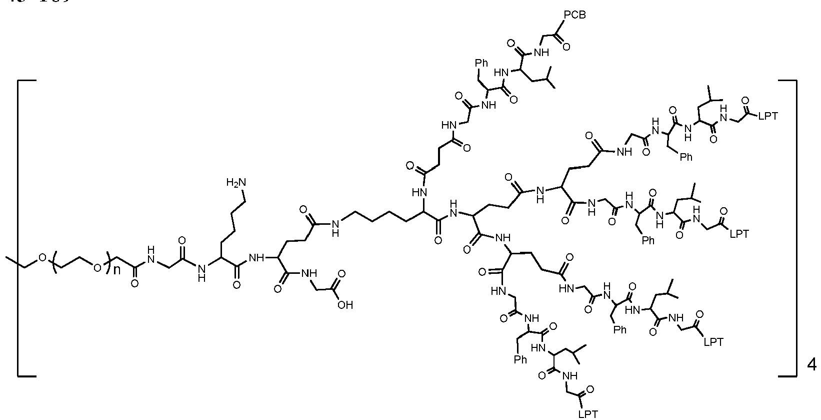

Product 43-182 (0.9 g, 0.0270 mmol) was added to a 500 mL flask, dichloromethane (15 mL) and TFA (0.1606 mL, 2.1625 mmol) were then added in sequence, and the obtained solution was treated by ultrasonic until completely dissolved; a ground glass stopper was used, and the mixed solution was stirred at room temperature to react. At the end of the reaction, the reaction solution was first evaporated to remove dichloromethane, and dissolved 2 min by ultrasonic with methyl tert-butyl ether (150 mL) to separate out a solid product, suction filtering was carried out, and the solid product was dissolved with 20% methanol/dichloromethane solvent; silica gel powder (5 g) was added to the obtained solution, and the solution was then evaporated to dryness with a rotary evaporator; the operations of dry sample loading, column chromatography, and gradient elution with 1% ammonia water+5%-15% methanol/dichloromethane were carried out. 0.6 g of the product was obtained with a yield of 68.03%. 43-190

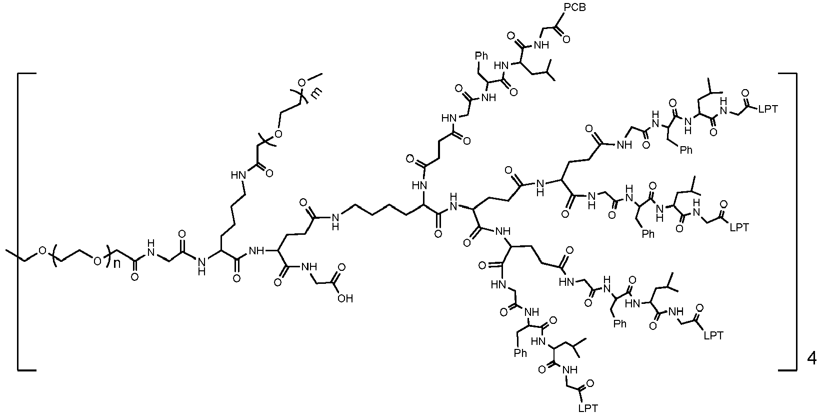

Product 43-189 (0.6 g, 0.0184 mmol) was weighed and added in a 250 mL flask and then dissolved in DMF (20 mL), dissolved by ultrasonic with DIEA (0.0608 mL, 0.3680 mmol) and M-SCM-5K (0.4044 g, 0.0773 mmol, purchased from Jenkem Technology), and the obtained solution slowly reacted in the dark. At the end of the reaction, methyl tert-butyl ether (100 mL) was added to the reaction solution to separate out a solid product; the solid product was filtered by suction, and dissolved with 10% methanol/dichloromethane; silica gel powder was added, and the obtained solution was evaporated to dryness, column chromatography, and gradient elution with 1% ammonia water+5%-7% methanol/dichloromethane were carried out. 0.8 g of the product was obtained with a yield of 81.63%. 43-197

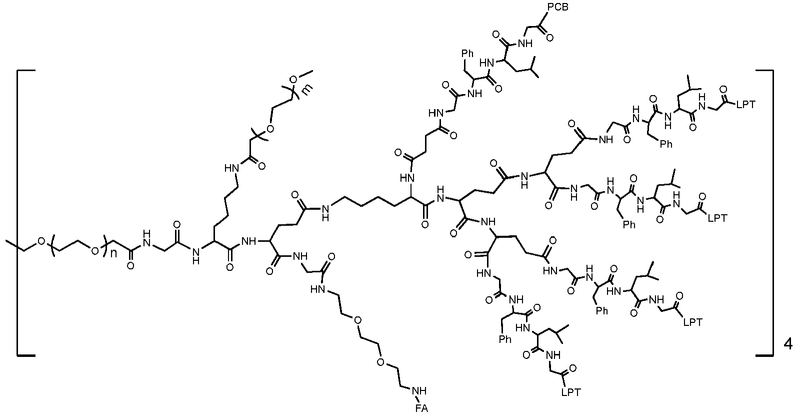

Product 43-190 (0.8 g, 0.0149 mmol), Product 37-163 (0.0376 g, 0.0658 mmol), HBTU (0.0339 g, 0.0894 mmol) and HOBT (0.0121 g, 0.0894 mmol) were weighed and added in a 250 mL flask and then dissolved in DMF solution, and the obtained solution was stirred at -5 °C for 30 min. DIEA (0.0443 mL, 0.2682 mmol) was slowly added dropwise, and the obtained solution reacted at -5 °C for 2 h and then stirred in the dark at room temperature. At the end of the reaction, methyl tert-butyl ether and n-hexane were added to precipitate the reaction solution, suction filtering was carried out, the operations of dry sample loading, column chromatography and elution with 1% ammonia water+6%-10% methanol/dichloromethane were carried out. 0.5 g of the product was obtained with a yield of 62.5%.

¹H-NMR (600 MHz, DMSO-d₆) δ 9.87-9.82 (m, 10H), 9.31-9.27 (m, 2H), 8.57-8.71(m, 10H), 8.57-8.52 (m, 13H), 8.22-8.13 (m, 32H), 8.13-8.01 (m,54H),8.00-7.91 (m,48H), 7.90-7.83 (m, 16H), 7.80-7.73 (m, 19H), 7.72-7.69 (m, 13H), 7.65-7.61(m,3H), 7.60-7.57(m, 3H), 7.53-7.40(m, 20H), 7.39-7.26(m, 56H), 7.25-7.18(m, 142H), 7315-7.09(m, 62H), 6.72-6.62(m, 10H),6.55-6.50 (m,5H), 5.29-5.21 (m, 32H), 4.79-4.66 (m, 40H), 4.62-4.50 (m,41H), 4.41-4.31(m,42H), 4.27-4.17(m,52H),4.16-4.08(m, 55H), 4.06-3.99 (m, 46H), 3.83-3.78(m,146H), 3.53-3.45 (m,3010H), 3.10-2.98(m, 52H), 2.93-2.87 (m, 15H), 2.82-2.77(m, 15H),2.64-2.59 (m, 60H),2.41-2.35(m, 31H), 2.17-2.07 (m,9H), 189-1.76 (m, 13H), 1.64-1.55 (m, 23H), 1.52-1.45(m, 38H), 1.36-1.31(m, 17H), 1.26-1.17 (m, 16H), 1.11-1.05(m, 4H),0.93-0.73(m, 120H).

### Chemical synthesis route of 50-47

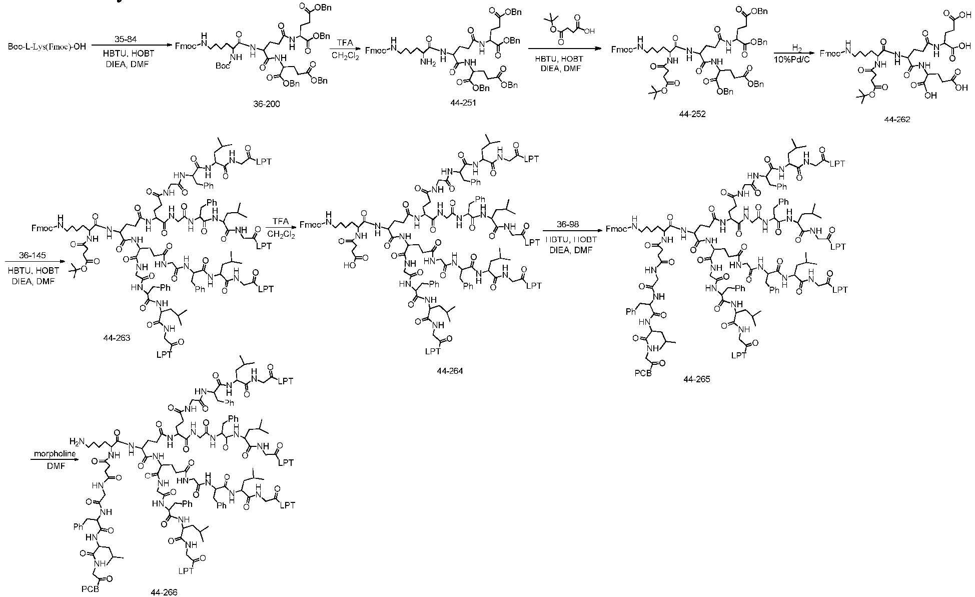

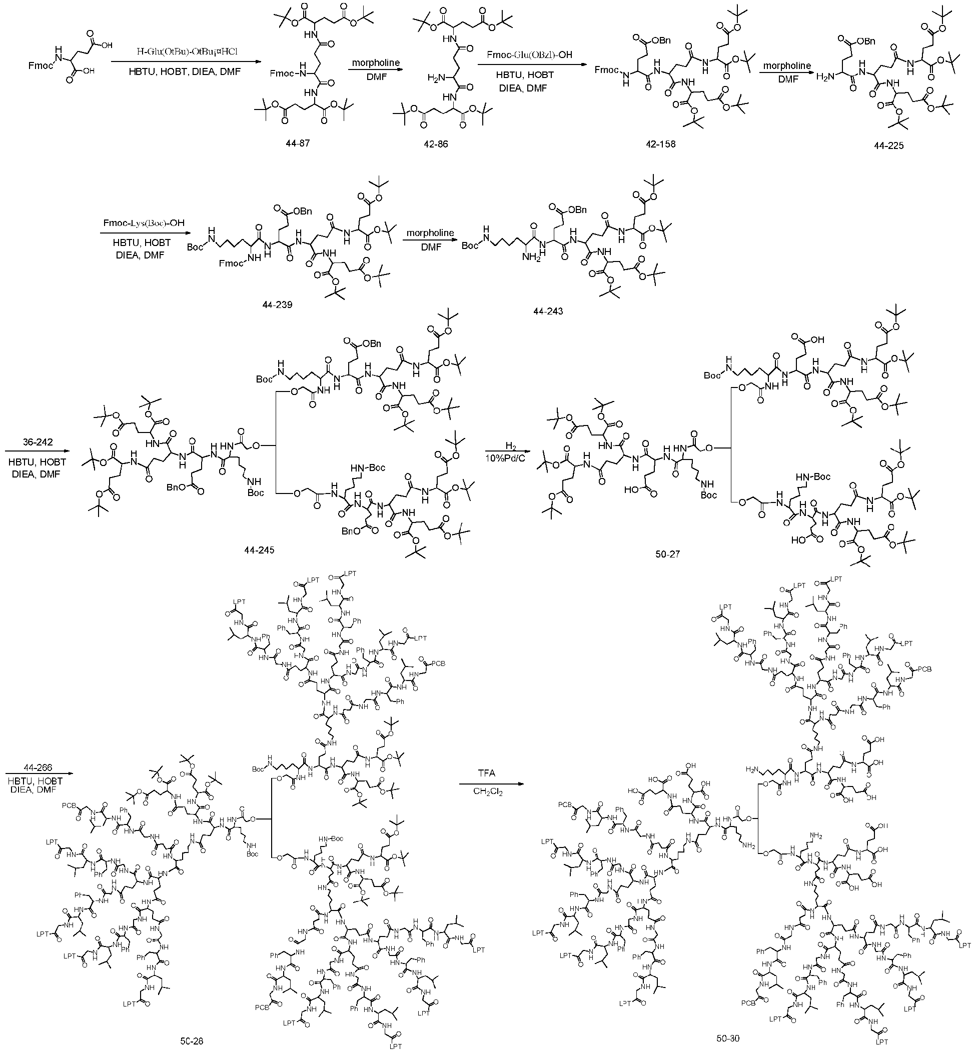

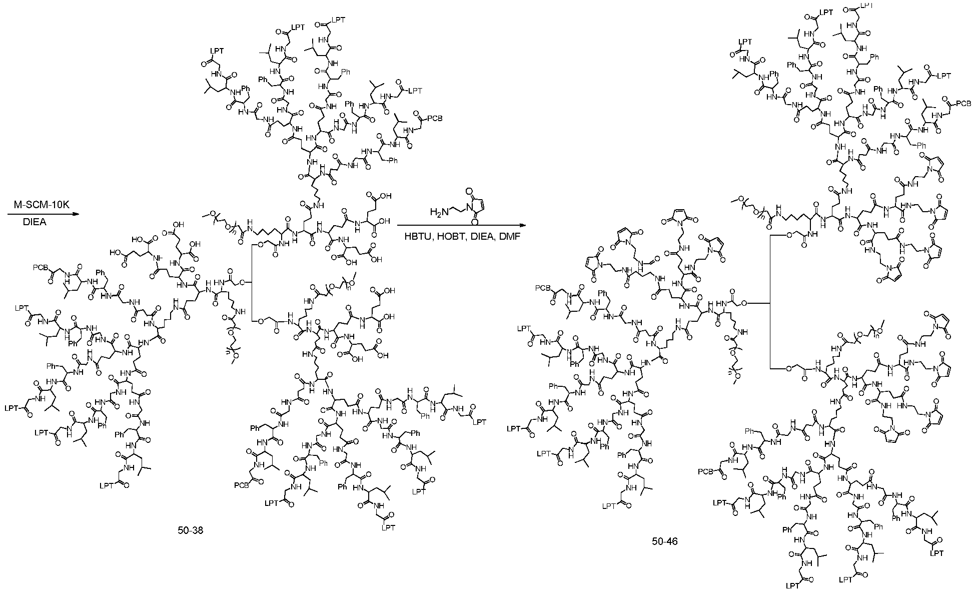

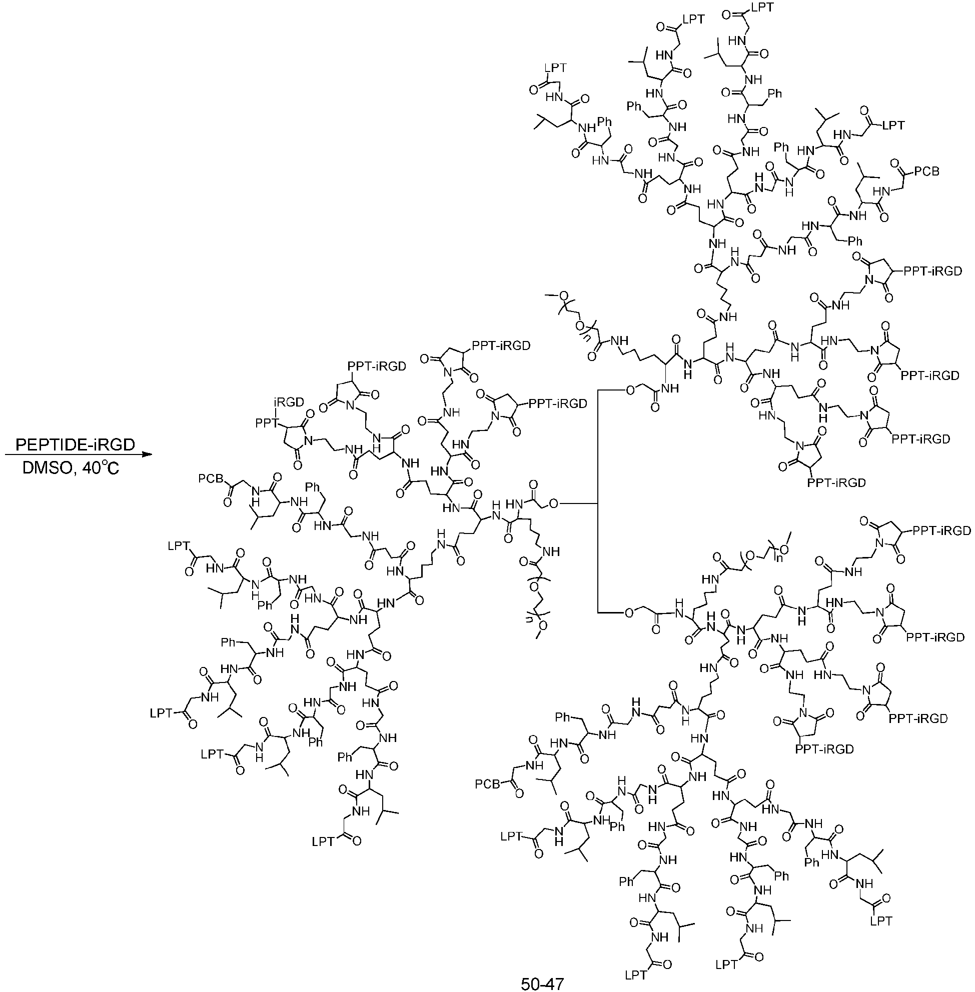

36-200

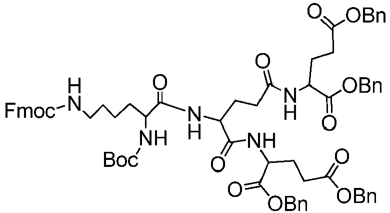

Product 35-84 (8.99 g, 11.7386 mmol), Boc-L-Lys(Fmoc)-OH (5.0 g, 10.6714 mmol, purchased from Aladdin), HBTU (6.0706 g, 16.0072 mmol) and HOBT (2.1629 g, 16.0072 mmol) were added in a 500 mL round-bottomed flask and then dissolved with DMF (80 mL), and the obtained solution in the flask was stirred at -5 °C for about 30 min; then, DIEA (10.6 mL, 64.0287 mmol) was slowly added dropwise, and at the end of the addition, the reaction solution in the flask was stirred at -5 °C for 3 h to react. At the end of the reaction, the reaction solution was transferred to a 1 L separatory funnel, a saturated sodium chloride solution (300 mL) and ethyl acetate (200 mL) were then added, the obtained solution was shaken and extracted, and the aqueous phase and the organic phase were separated. Ethyl acetate (100 mL) was then added to the aqueous phase, the obtained solution was shaken and extracted, and the aqueous phase and the organic phase were separated. Then, the obtained organic phases were combined and a saturated sodium chloride solution (300 mL) was then added to the organic phase; the obtained solution was then shaken and extracted, and the aqueous phase and the organic phase were separated. Then, deionized water (300 mL) was then added to the organic phase, the obtained solution was shaken and extracted, and the aqueous phase and the organic phase were separated. Finally, the organic phase was concentrated, evaporated to dryness, and dried. 12.9804 g of Product 36-200 was obtained finally. 44-251

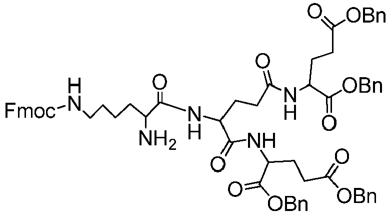

Product 36-200 (6.921 g, 5.6899 mmol) was added in a 250 mL round-bottomed flask and then dissolved with dichloromethane (20 mL), TFA (6.3 mL, 85.3482 mmol) was then added with stirring, and the obtained solution in the flask was stirred at room temperature to react. At the end of the reaction, the reaction solution was concentrated at reduced pressure to remove dichloromethane. Methyl tert-butyl ether (200 mL) was added and a powdery solid was then precipitated; the suction filtering was carried out and the filter cake was washed three times with methyl tert-butyl ether, and dried. 6.35 g of Product 44-251 was obtained . 44-252

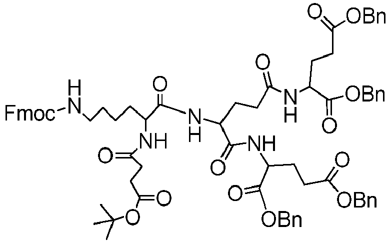

Mono-tert-butyl succinate (1.1893 g, 6.8279 mmol, purchased from Accela), Product 44-251 (6.3574 g, 5.6899 mmol), HBTU (3.2368 g, 8.5348 mmol) and HOBT (1.1532 g, 8.5348 mmol) were weighed and added in a 250 mL round-bottomed flask and then dissolved in DMF solution (60 mL), and the obtained solution in the flask was stirred at -5 °C for about 30 min; then, DIEA (5.6 mL, 34.1394 mmol) was slowly added dropwise, and at the end of the addition, the reaction solution in the flask was stirred at -5 °C overnight to react. At the end of the reaction, the reaction solution was transferred to a 1 L separatory funnel; a saturated sodium chloride solution (300 mL) and ethyl acetate (200 mL) were then added, the obtained solution was shaken and extracted, and the aqueous phase and the organic phase were separated; ethyl acetate (100 mL) was then added to the aqueous phase, the obtained solution was then shaken and extracted, and the aqueous phase and the organic phase were separated. Then, the obtained organic phases were combined and a saturated sodium chloride solution (300 mL) was then added to the organic phase; the obtained solution was then shaken and extracted, and the aqueous phase and the organic phase were separated. Then, deionized water (300 mL) was then added to the organic phase, the obtained solution was shaken and extracted, and the aqueous phase and the organic phase were separated. Finally, the organic phase was concentrated, evaporated to dryness and dissolved with a mixed solvent (100 mL) (20% methanol/dichloromethane); silica gel powder (50 mL) was added to the obtained solution, and the solution was then evaporated to dryness; the operations of dry sample loading, column chromatography, and elution with an eluent (50% ethyl acetate and 50% petroleum ether) were carried out; the elution product was then collected, concentrated, evaporated to dryness, and dried. 5.4136 g of Product 44-252 was obtained with a yield of 75%. 44-262

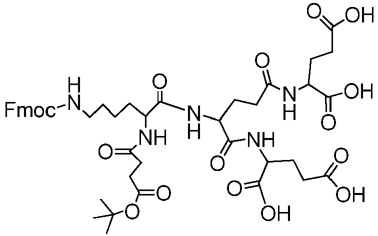

Product 44-252 (1.737 g, 1.3651 mmol) and 10% Pd/C (80 mg) were added in a hydrogenation reactor and then dissolved with DMF (30 mL), and the hydrogenation reactor was then sealed; hydrogen was introduced to a pressure of 2.2 MPa in the reactor; the hydrogenation reactor was placed at room temperature and the rection solution was stirred overnight. At the end of the reaction, the reactor was taken out, and the reaction solution was evenly added dropwise to a sand core funnel filled with compacted diatomaceous earth, suction filtering was carried out, and the diatomaceous earth was washed with DMF until it did not contain any product, thus obtaining the reaction solution of the product. 44-263

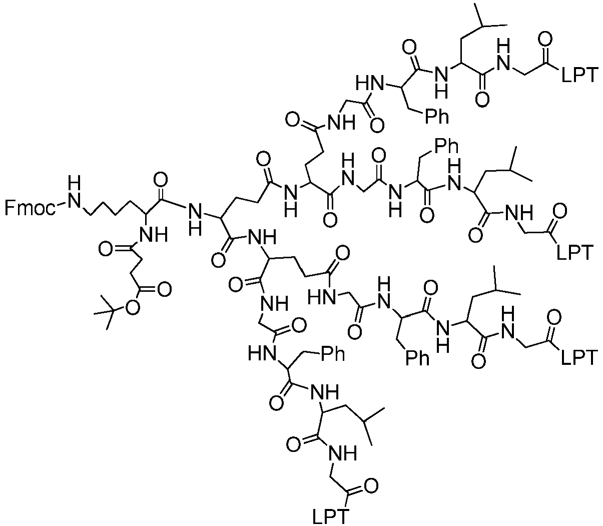

Product 44-262 (1.2449 g, 1.3651 mmol), Product 36-145 (6.0 g, 6.2795 mmol), HBTU (3.1062 g, 8.1905 mmol) and HOBT (1.1067 g, 8.1905 mmol) were added in a 500 mL round-bottomed flask and then dissolved with DMF (80 mL); the obtained solution in the flask was stirred at -5 °C for about 30 min; then, DIEA (4.1 mL, 24.5720 mmol) was slowly added dropwise; at the end of the addition, the reaction solution in the flask was stirred at -5 °C for 1 h to react, and finally stirred at room temperature for 3 h to react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product; the process of precipitation was repeated three times to obtain an oily solid; the oily solid was dissolved with dichloromethane (10 mL), and then precipitated with methyl tert-butyl ether (150 mL) to obtain a powdery solid; filtering was carried out to obtain a solid product; the solid was washed with methyl tert-butyl ether (60 mL), and finally dried in an oven, thus obtaining 6.36 g of Product 44-263. 44-264

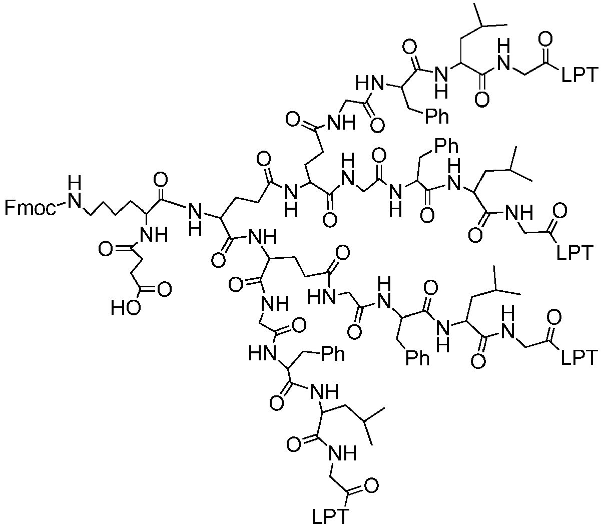

Product 44-263 (6.3639 g, 1.3651 mmol) was added in a 500 mL round-bottomed flask and then dissolved with dichloromethane (20 mL), TFA (1.5 mL, 20.4765 mmol) was then added with stirring, and the obtained solution in the flask was stirred at room temperature to react. At the end of the reaction, the reaction solution was concentrated at reduced pressure to remove dichloromethane; methyl tert-butyl ether (150 mL) was then added to precipitate the reaction solution and a powdery solid was obtained, filtering was carried out; the obtained solid was washed with methyl tert-butyl ether (100 mL), and dried, thus obtaining 6.4 g of Product 44-264. 44-265

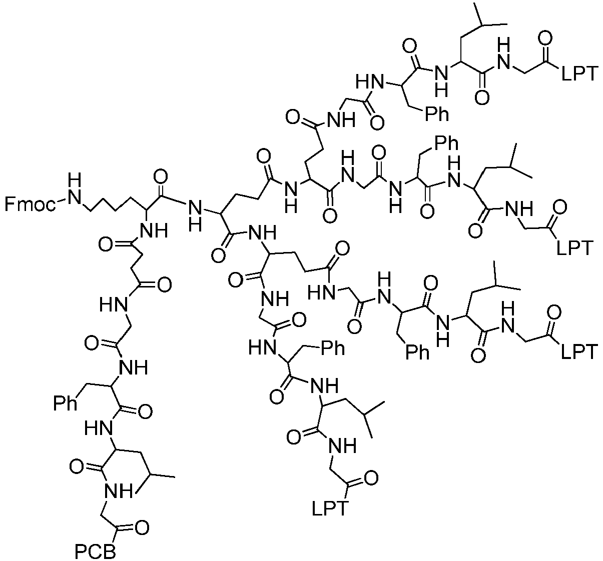

Product 44-264 (6.2873 g, 1.3651 mmol), Product 36-98 (1.6831 g, 2.0477 mmol), HBTU (0.7766 g, 2.0477 mmol) and HOBT (0.2767 g, 2.0477 mmol) were added in a 500 mL round-bottomed flask and then dissolved with DMF (50 mL); the obtained solution in the flask was stirred at -5 °C for about 30 min; then, DIEA (1.4 mL, 8.1906 mmol) was slowly added dropwise; at the end of the addition, the reaction solution in the flask was stirred at -5 °C for 1h to react, and finally stirred at room temperature for 3 h to react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product; the process of precipitation was repeated three times to obtain an oily solid; the oily solid was dissolved with dichloromethane (10 mL), and then precipitated with methyl tert-butyl ether (150 mL) to obtain a powdery solid; filtering was carried out to obtain a solid product, and the obtained solid product was washed with methyl tert-butyl ether (60 mL), and finally dried in an oven, thus obtaining 7.3848 g of Product 44-265. 44-266

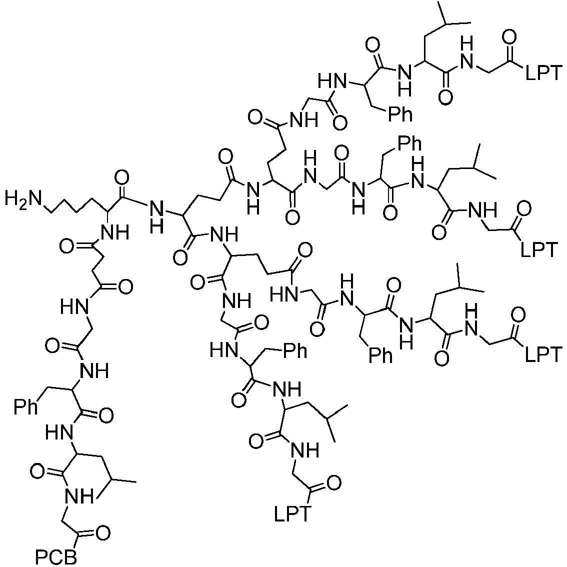

Product 44-265 (7.3848 g, 1.3651 mmol) was added in a 500 mL round-bottomed flask and then dissolved with DMF (30 mL), morpholine (1.8 mL, 20.4765 mmol) was then added with stirring, and the obtained solution in the flask was stirred at room temperature for 2 h to react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product. Such operations were repeated five times to obtain an oily solid. The oily solid was dissolved with dichloromethane (10 mL), and then precipitated with methyl tert-butyl ether (150 mL) to obtain a powdery solid; filtering was carried out to obtain a solid product, and the obtained solid product was washed with methyl tert-butyl ether (60 mL), and dissolved with a mixed solvent (100 mL) (20% methanol/dichloromethane); silica gel powder (60 mL) was added to the obtained solution, and the solution was then evaporated to dryness; the operations of dry sample loading, column chromatography, and elution with an eluent (20% methanol: 80% dichloromethane) were carried out; the elution product was then collected, concentrated, evaporated to dryness, and dried. 2.0 g of Product 44-266 was obtained with a yield of 28.4%. 44-87

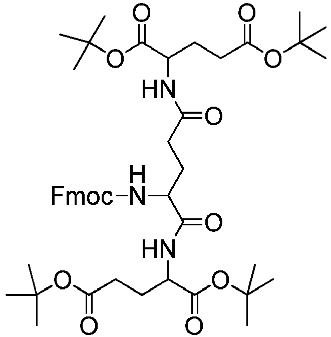

Fmoc-Glu-OH (5.0 g, 13.5355 mmol, purchased from Aladdin), HBTU (15.3996 g, 40.6064 mmol), HOBT (5.4867 g, 40.6064 mmol) and L-glutamic acid di-tert-butyl ester hydrochloride (8.8083 g, 29.7780 mmol, purchased from Innochem) were added in a 1 L round-bottomed flask and then dissolved with DMF (100 mL); the obtained solution in the flask was stirred at -5 °C for about 30 min; then, DIEA (26.8 mL, 162.4256 mmol) was slowly added dropwise; at the end of the addition, the reaction solution in the flask was further stirred at -5 °C for 2 h, and finally stirred at room temperature overnight to react. At the end of the reaction, the reaction solution was transferred to a 2 L separatory funnel, a saturated sodium chloride solution (400 mL) and ethyl acetate (300 mL) were then added, the obtained solution was shaken, stood still, and extracted, and the aqueous phase and the organic phase were separated; ethyl acetate (200 mL) was then added to the aqueous phase, the obtained solution was shaken, stood still, and extracted, and the aqueous phase and the organic phase were separated; the obtained organic phases were combined, a saturated sodium chloride solution (300 mL) was then added to the organic phase, the obtained solution was shaken and extracted, and the aqueous phase and the organic phase were separated. Then, the organic phase was concentrated, evaporated to dryness, and dried. 11.5325 g of Product 44-87 was obtained. 42-86

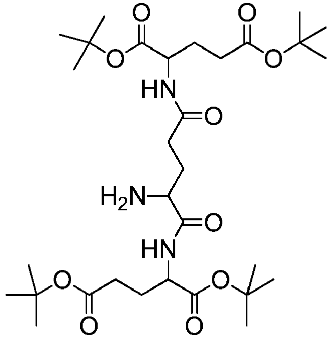

Product 44-87 (11.5325 g, 13.5355 mmol) was added in a 500 mL round-bottomed flask and then dissolved with DMF (30 mL), morpholine (17.7 mL, 203.0325 mmol) was then added with stirring, and finally the obtained solution in the flask was stirred at room temperature for 2 h to react. At the end of the reaction, the reaction solution was first transferred to a 1 L separatory funnel; a saturated sodium chloride aqueous solution (300 mL) and ethyl acetate (200 mL) were added, and the obtained solution was shaken and extracted; the aqueous phase was separated; ethyl acetate (100 mL) was added to the aqueous phase, and the obtained solution was shaken and extracted; the aqueous phase was separated, and the obtained organic phases were combined; a saturated sodium chloride aqueous solution (300 mL) was added again to the organic phase, and the obtained solution was shaken and extracted; the aqueous phase was separated. Then, deionized water (300 mL) was added to the organic phase, and the obtained solution was shaken and extracted, the aqueous phase was separated. Finally, the organic phase was concentrated, evaporated to dryness, dissolved with a mixed solvent (30 mL) (20% methanol/dichloromethane); silica gel powder (50 mL) was added to the obtained solution, and the solution was then evaporated to dryness; the operations of dry sample loading, column chromatography, and elution with an eluent (0%-6% methanol: 100%-98% dichloromethane) were carried out; the elution product was then collected, concentrated, evaporated to dryness, and dried. 6.2281 g of Product 42-86 was obtained with a yield of 73.0%. 42-158

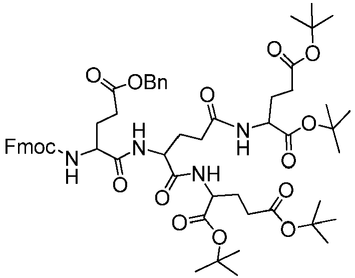

Fmoc-Glu(OBzl)-OH (5.0 g, 10.8816 mmol, purchased from Ark Pharm), Product 42-86 (7.5383 g, 11.9698 mmol), HBTU (6.1901 g, 16.3224 mmol) and HOBT (2.2055 g, 16.3224 mmol) were added in a 500 mL round-bottomed flask and then dissolved with DMF (80 mL); the obtained solution in the flask was stirred at -5 °C for about 30 min; then, DIEA (8.1 mL, 48.9673 mmol) was slowly added dropwise; at the end of the addition, the reaction solution in the flask was stirred at -5 °C for 1 h to react, and finally stirred at room temperature for 3 h to react. At the end of the reaction, the reaction solution was transferred to a 1 L separatory funnel, a saturated sodium chloride solution (400 mL) and ethyl acetate (300 mL) were then added, the obtained solution was shaken and extracted, and the aqueous phase and the organic phase were separated; ethyl acetate (200 mL) was then added to the aqueous phase, the obtained solution was shaken and extracted, and the aqueous phase and the organic phase were separated. Then, the obtained organic phases were combined, a saturated sodium chloride solution (300 mL) was then added to the organic phase, the obtained solution was shaken and extracted, and the aqueous phase and the organic phase were separated. Then, deionized water (300 mL) was added to the organic phase, the obtained solution was shaken and extracted, and the aqueous phase and the organic phase were separated. Finally, the organic phase was concentrated, evaporated to dryness, and dried, thus obtaining 13.1818 g of Product 42-158. 44-225

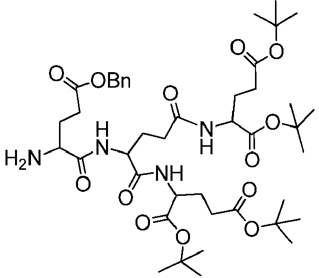

Product 42-158 (13.1818 g, 12.3050 mmol) was added in a 500 mL round-bottomed flask and then dissolved with DMF (30 mL), morpholine (16 mL, 184.575 mmol) was then added with stirring, and the obtained solution in the flask was stirred at room temperature for 2 h to react. At the end of the reaction, n-hexane (150 mL) and methyl tert-butyl ether (30 mL) were first added to precipitate the reaction solution, and the supernatant was discarded; n-hexane (150 mL) and methyl tert-butyl ether (30 mL) were added again to precipitate the lower oily product; the process of precipitation was repeated three times to obtain an oily solid; the oily solid was dissolved with dichloromethane (10 mL), and then precipitated with methyl tert-butyl ether (150 mL) to obtain a powdery solid; filtering was carried out to obtain a solid product, and the obtained solid product was washed with methyl tert-butyl ether (60 mL), and finally dried in an oven, thus obtaining 10.4472 g of Product 44-225. 44-239

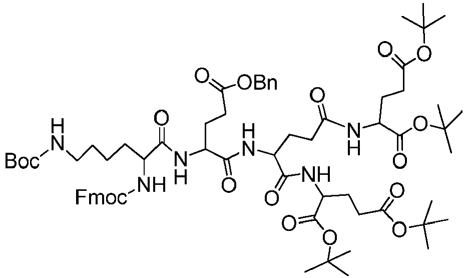

Product 44-225 (10.4472 g, 12.3050 mmol), Fmoc-Lys(Boc)-OH (4.1181 g, 8.7893 mmol, purchased from Accela), HBTU (4.9999 g, 13.1839 mmol) and HOBT (1.7814 g, 13.1839 mmol) were added in a 500 mL round-bottomed flask and then dissolved with DMF (100 mL), and the obtained solution in the flask was stirred at -5 °C for about 30 min; then, DIEA (8.0 mL, 48.3411 mmol) was slowly added dropwise, and at the end of the addition, the reaction solution in the flask was stirred at -5 °C for 3 h to react. At the end of the reaction, the reaction solution was transferred to a 1 L separatory funnel, a saturated sodium chloride solution (400 mL) and ethyl acetate (300 mL) were then added, the obtained solution was shaken and extracted, and the aqueous phase and the organic phase were separated; ethyl acetate (200 mL) was added to the aqueous phase, the obtained solution was shaken and extracted, and the aqueous phase and the organic phase were separated. Then, the obtained organic phases were combined, a saturated sodium chloride solution (300 mL) was added to the organic phase, the obtained solution was shaken and extracted, and the aqueous phase and the organic phase were separated. Then, deionized water (300 mL) was added to the organic phase, the obtained solution was shaken and extracted, and the aqueous phase and the organic phase were separated. Finally, the organic phase was concentrated, evaporated to dryness, dried, and then dissolved with a mixed solution (20% methanol/dichloromethane); silica gel powder was added to the obtained solution, and the solution was then evaporated to dryness to obtain a powdery solid; the operations of dry sample loading, column chromatography, and elution with a mixed solution (20% methanol/dichloromethane) were carried out; the elution product was then collected, concentrated, and dried. 11.42 g of Product 44-239 was obtained. 44-243

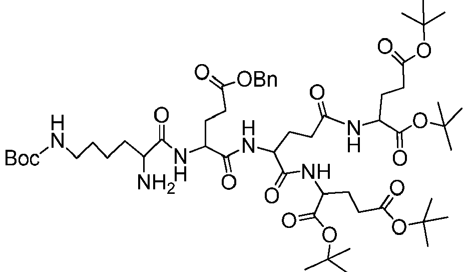

Product 44-239 (11.42 g, 10.3 mmol) was added in a 500 mL round-bottomed flask and then dissolved with DMF (10 mL), morpholine (13.5 mL, 154.6 mmol) was then added with stirring, and the obtained solution in the flask was stirred at room temperature for 2 h to react. At the end of the reaction, the reaction solution was transferred to a 1 L separatory funnel, a saturated sodium bicarbonate aqueous solution (400 mL) and ethyl acetate (300 mL) were then added, the obtained solution was shaken and extracted, and the aqueous phase and the organic phase were separated; ethyl acetate (200 mL) was then added to the aqueous phase, the obtained solution was shaken and extracted, and the aqueous phase and the organic phase were separated. Then, the obtained organic phases were combined; deionized water (400 mL) was added to the organic phase, the obtained solution was shaken and extracted, and the aqueous phase and the organic phase were separated; deionized water (300 mL) was added again to the organic phase, the obtained solution was shaken and extracted, and the aqueous phase and the organic phase were separated. Finally, the organic phase was concentrated and evaporated to dryness. The solid product was dissolved with a mixed solvent (100 mL) (20% methanol/dichloromethane); silica gel powder (50 mL) was added to the obtained solution, and the solution was then evaporated to dryness; the operations of dry sample loading, column chromatography, and elution with an eluent (a mixed solution of 20%-90% ethyl acetate and petroleum ether) were carried out, and the liquid was collected; the elution product was concentrated, evaporated to dryness, and dried, thus obtaining 7.219 g of Product 44-243 with a yield of 65%. 44-245

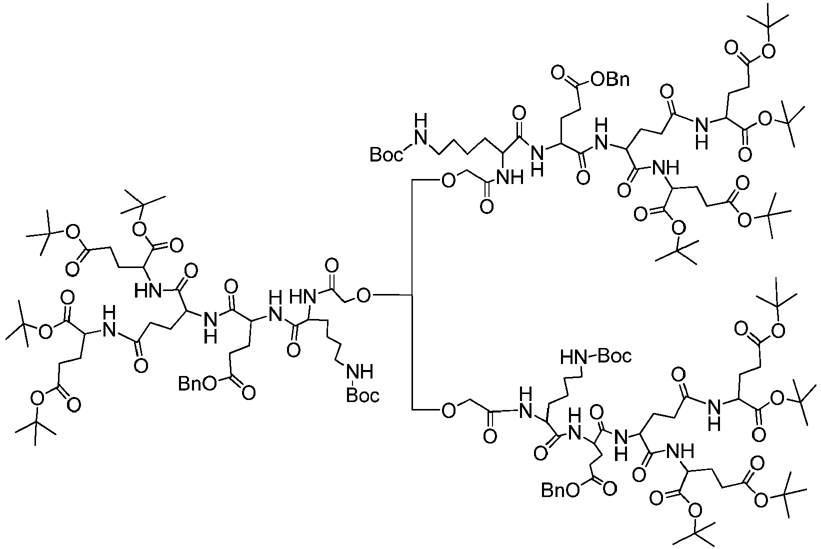

Product 44-243 (6.1723 g, 5.7294 mmol), solution of Product 36-242 (0.4237 g, 1.5915 mmol), HBTU (2.7160 g, 7.1617 mmol) and HOBT (0.9677 g, 7.1617 mmol) were added in a 250 mL round-bottomed flask and then dissolved with DMF (100 mL), and the obtained solution in the flask was stirred at -5 °C for about 30 min; then, DIEA (3.7 mL, 22.5437 mmol) was slowly added dropwise; at the end of the addition, the reaction solution in the flask was stirred at -5 °C for 1 h to react, and finally stirred at room temperature for 3 h to react. At the end of the reaction, the reaction solution was transferred to a 1 L separatory funnel, a saturated sodium bicarbonate aqueous solution (400 mL) and ethyl acetate (200 mL) were then added, the obtained solution was shaken and extracted, and the aqueous phase and the organic phase were separated; ethyl acetate (100 mL) was then added to the aqueous phase, the obtained solution was shaken and extracted, and the aqueous phase and the organic phase were separated. Then, the obtained organic phases were combined, deionized water (400 mL) was added to the organic phase, the obtained solution was shaken and extracted, and the aqueous phase and the organic phase were separated; deionized water (300 mL) was added again to the organic phase, the obtained solution was shaken and extracted, and the aqueous phase and the organic phase were separated. Finally, the organic phase was concentrated and evaporated to dryness. The solid product was dissolved with a mixed solvent (80 mL) (20% methanol/dichloromethane); silica gel powder (50 mL) was added to the obtained solution, and the solution was then evaporated to dryness; the operations of dry sample loading, column chromatography, and elution with an eluent (1%-8% methanol: 99%-92% dichloromethane) were carried out; the elution product was then collected, concentrated, evaporated to dryness, and dried. 2.3346 g of Product 44-245 was obtained with a yield of 43%. 50-27

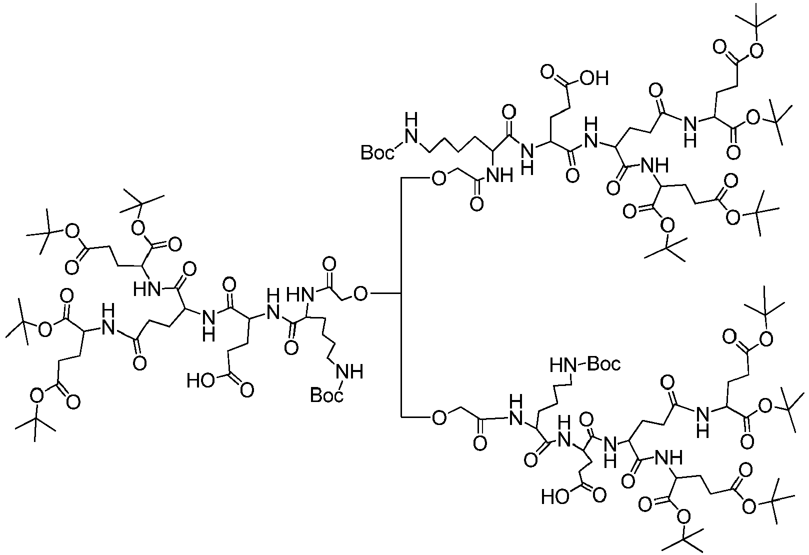

Product 44-245 (0.4023 g, 0.1168) and 10% Pd/C (50 mg) were added in a hydrogenation reactor and then dissolved with DMF (30 mL), and the hydrogenation reactor was then sealed; hydrogen was introduced to a pressure of 2.0 MPa in the reactor; the hydrogenation reactor was placed at room temperature and the rection solution was stirred overnight. At the end of the reaction, the reactor was taken out, and the reaction solution was evenly added dropwise to a sand core funnel filled with compacted diatomaceous earth, suction filtering was carried out, and the diatomaceous earth was washed with DMF until it did not contain any product, thus obtaining the reaction solution of the product. 50-28

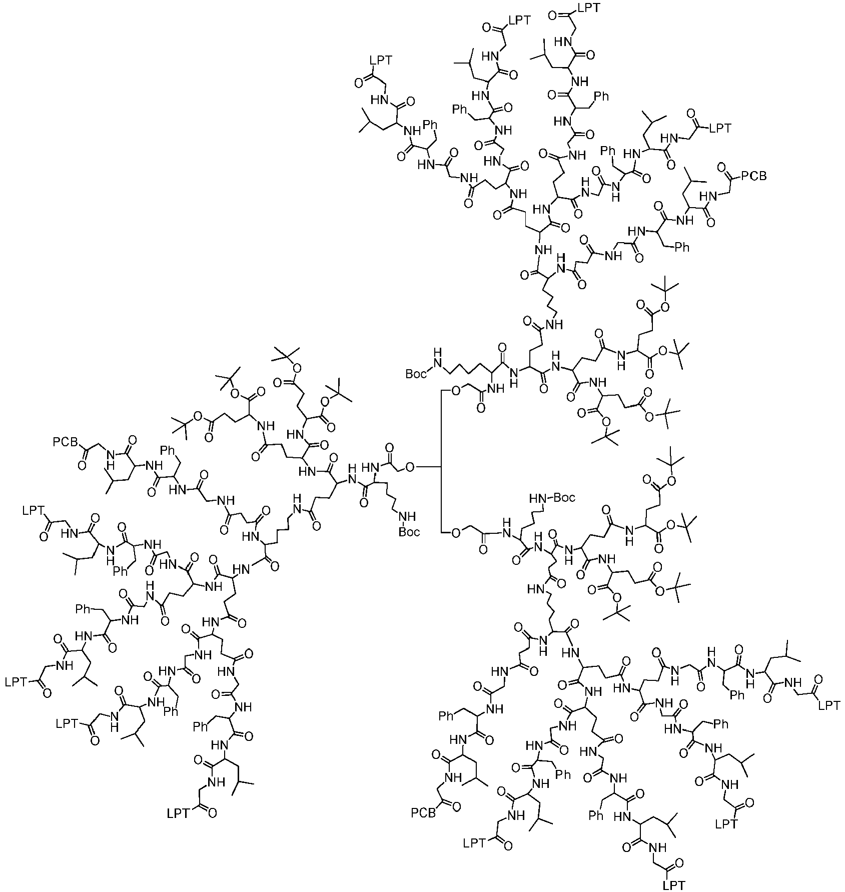

Solution of Product 50-27 (0.3707 g, 0.1168 mmol), Product 44-266 (2.0 g, 0.3855 mmol), HBTU (0.2 g, 0.5256 mmol) and HOBT (0.07 g, 0.5256 mmol) were added in a 250 mL round-bottomed flask and then dissolved with DMF (50 mL); the obtained solution in the flask was stirred at -5 °C for about 30 min; then, DIEA (0.7 mL, 4.4982 mmol) was slowly added dropwise; at the end of the addition, the reaction solution in the flask was stirred at -5 °C for 1 h to react, and finally stirred at room temperature for 3 h to react. At the end of the reaction, n-hexane (150 mL) and methyl tert-butyl ether (30 mL) were first added to precipitate the reaction solution, the supernatant was discarded, n-hexane (150 mL) and methyl tert-butyl ether (30 mL) were added again to precipitate the lower oily product. Such operations were repeated six times to obtain an oily solid. The oily solid was dissolved with dichloromethane (10 mL), and then precipitated with methyl tert-butyl ether (150 mL) to obtain a powdery solid; filtering was carried out to obtain a solid product; the obtained solid product was washed with methyl tert-butyl ether (60 mL), and finally dried in an oven, thus obtaining 2.18 g of Product 50-28. 50-30

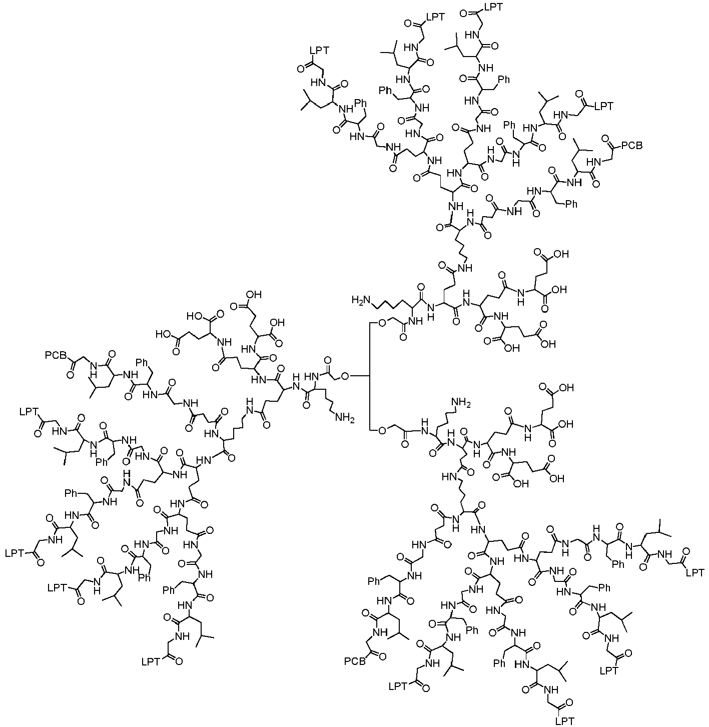

Product 50-28 (2.18 g, 0.1168 mmol) was added in a 250 mL round-bottomed flask and then dissolved with dichloromethane (15 mL), TFA (10 mL, 134.65 mmol) was then added with stirring, and the obtained solution in the flask was stirred at room temperature to react. At the end of the reaction, the reaction solution was concentrated at reduced pressure to remove dichloromethane. Methyl tert-butyl ether (150 mL) was added to precipitate the reaction solution and a powdery solid was obtained, filtering was carried out, and the obtained solid was washed with methyl tert-butyl ether (100 mL), and dried, thus obtaining 2.06 of Product 50-30. 50-38

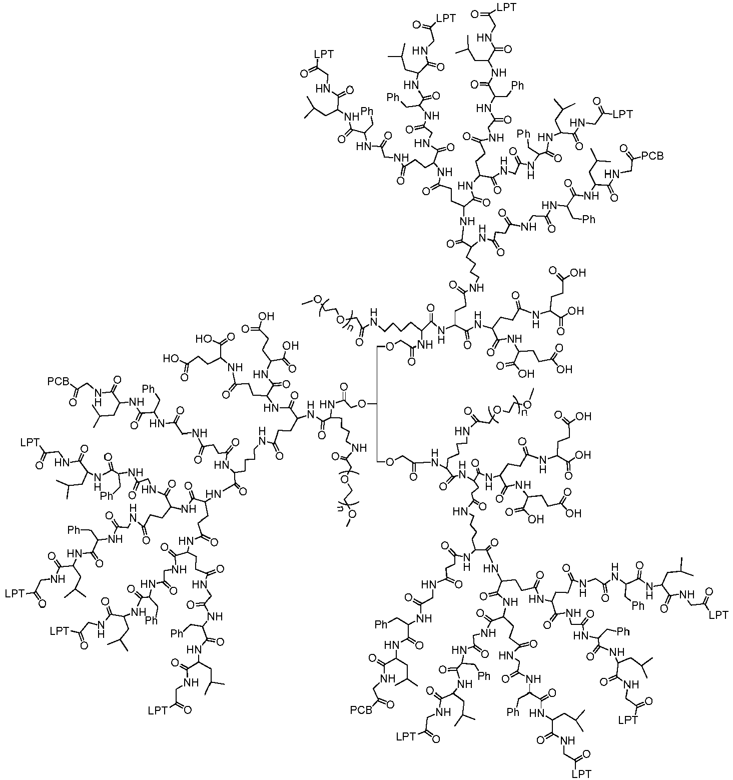

Product 50-30 (1.0 g, 0.056 mmol) and M-SCM-10K (1.98 g, 0.1863 mmol, purchased from JenKem) were added in a 250 mL round-bottomed flask and then dissolved with DMF (30 mL), and the obtained solution in the flask was stirred at -5 °C for about 30 min to react; then, DIEA (0.5 mL, 2.8305 mmol) was slowly added dropwise; at the end of the addition, the reaction solution was stirred at -5 °C for 10 min to react, and then transferred to room temperature to react for one week. At the end of the reaction, n-hexane (150 mL) and methyl tert-butyl ether (20 mL) were added to precipitate the reaction solution, the supernatant was discarded, n-hexane (150 mL) and methyl tert-butyl ether (30 mL) were added again to precipitate the lower oily product, the process of precipitation was repeated three times to obtain an oily solid; the oily solid was dissolved with dichloromethane (10 mL), and then precipitated with methyl tert-butyl ether (60 mL) to obtain a powdery solid, and filtering was carried out to obtain a solid product. The filter cake was dissolved with a mixed solvent (100 mL) (20% methanol/dichloromethane); 50 mL of silica gel powder was added to the obtained solution, and the solution was then evaporated to dryness; the operations of dry sample loading, column chromatography, and elution with an eluent (a mixed solution of 3%-8% methanol/dichloromethane) were carried out; the elution product was then collected, concentrated, and evaporated to dryness. 0.72 g of Product 50-38 was obtained with a yield of 27%. 50-46

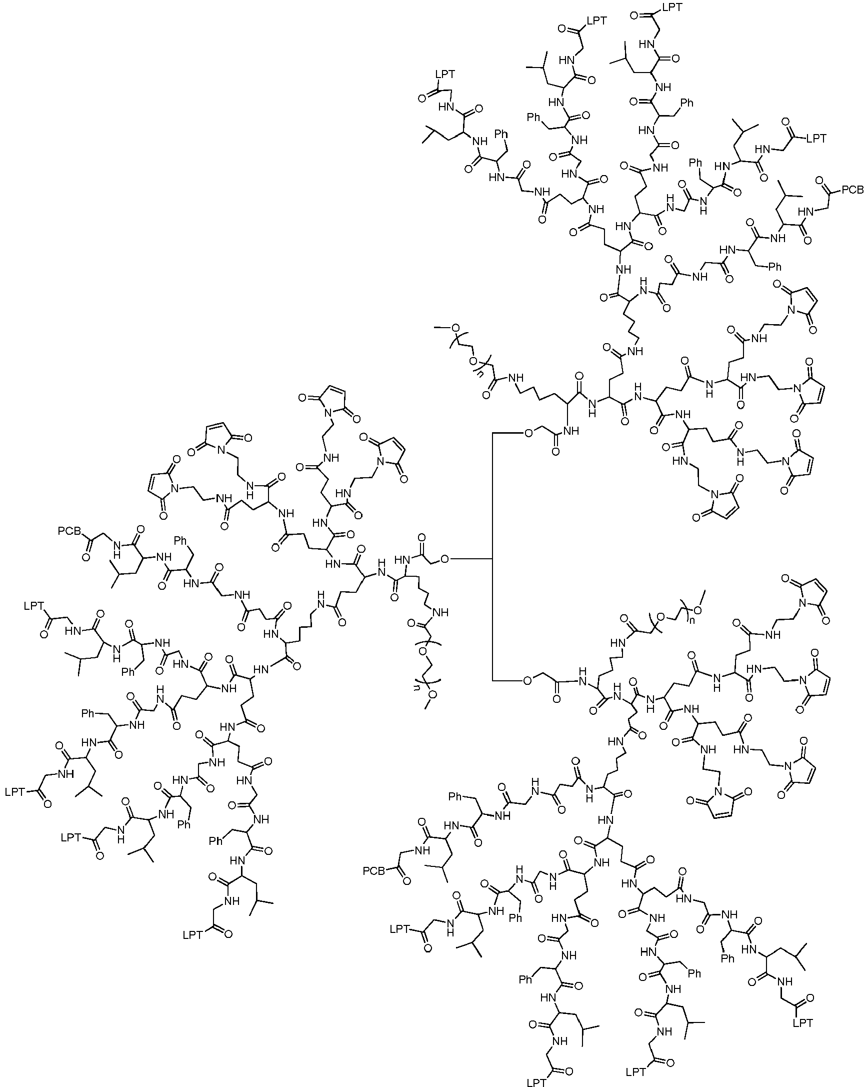

Product 50-38 (0.72 g, 0.01477 mmol), N-(2-aminoethyl)maleimide hydrochloride (0.052 g, 0.2954 mmol), HBTU (0.1 g, 0.2659 mmol) and HOBT (0.036 g, 0.2659 mmol) were added in a 250 mL round-bottomed flask and then dissolved with DMF (20 mL); the obtained solution in the flask was stirred at -5 °C for about 30 min; then, DIEA (0.16 mL, 0.9748 mmol) was slowly added dropwise; at the end of the addition, the reaction solution in the flask was stirred at -5 °C for 1 h, and finally stirred in the dark at room temperature overnight to react. At the end of the reaction, n-hexane (200 mL) and methyl tert-butyl ether (60 mL) were first added to precipitate the reaction solution, the supernatant was discarded, n-hexane (150 mL) and methyl tert-butyl ether (30 mL) were added again to precipitate the lower oily product, the process of precipitation was repeated three times to obtain an oily solid; the oily solid was dissolved with dichloromethane (10 mL), and then precipitated with methyl tert-butyl ether (150 mL) to obtain a powdery solid; filtering was carried out to obtain a solid product, and the obtained solid product was washed with methyl tert-butyl ether (60 mL), and dried, thus obtaining 0.3 g of Product 50-46 with a yield of 41%. 50-47

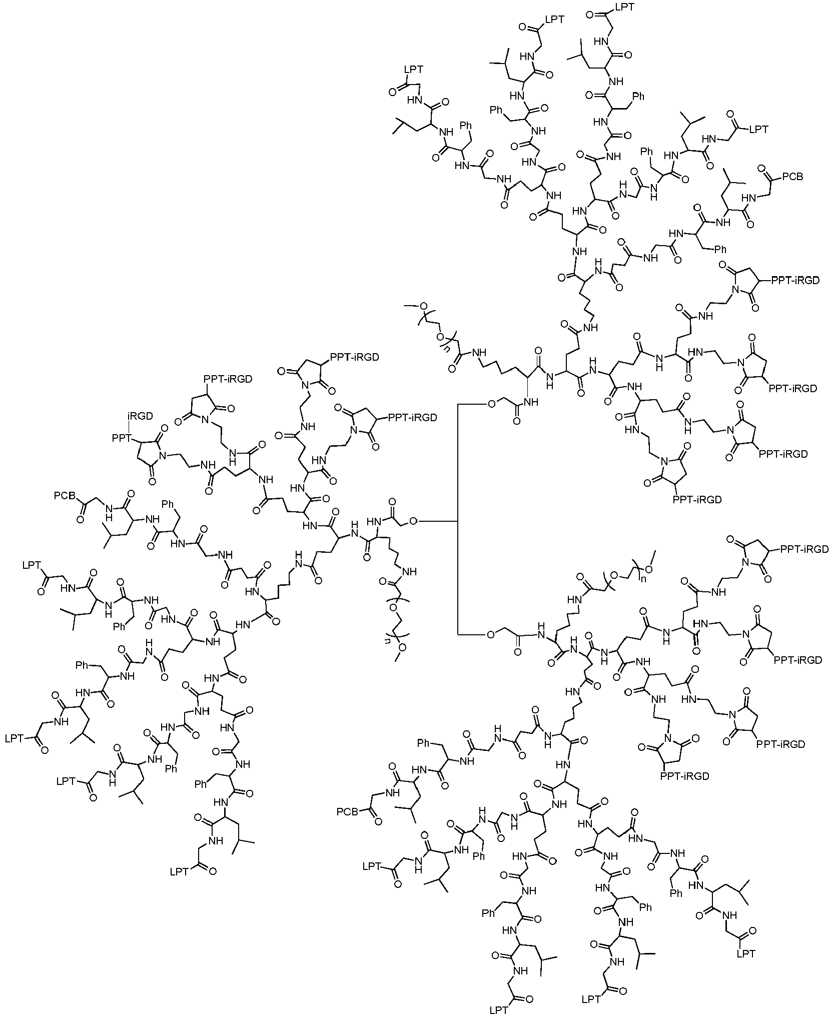

Product 50-46 (0.3 g, 0.006 mmol) and PPT-iRGD (0.16 g, 0.089 mmol, Nanjing Taiye), were added in a 250 mL round-bottomed flask and then dissolved with DMSO (30 mL); the obtained solution in the flask was stirred at 40 °C in an oil bath in the dark for 2 days. At the end of the reaction, n-hexane (150 mL) and methyl tert-butyl ether (30 mL) were first added to precipitate the reaction solution, and the supernatant was discarded; n-hexane (200 mL) and methyl tert-butyl ether (60 mL) were added again to precipitate the lower oily product. Suh operations were repeated five times to obtain a powdery solid. Filtering was carried out to obtain a solid product, and the obtained solid product was washed with methyl tert-butyl ether (60 mL), and dissolved with a mixed solvent (60 mL) (20% methanol/dichloromethane); silica gel powder (65 mL) was added to the obtained solution, and the solution was then evaporated to dryness; the operations of dry sample loading, column chromatography, and elution with an eluent (1% ammonia water/6%-8% methanol/91%-93% dichloromethane) were carried out; the elution product was then collected, concentrated, evaporated to dryness, and dried. 0.19 g of Product 50-47 was obtained with a yield of 46%.

¹H-NMR (600 MHz, DMSO-d₆) δ 9.85- 9.72 (m, 19H), 9.15- 9.03 (m, 3H), 8.94- 8.85 (m, 3H), 8.84- 8.76 (m, 19H), 8.65- 8.46 (m, 19H), 8.30- 8.24 (m, 37H), 8.16- 8.09 (m, 48H), 8.00- 7.93 (m, 41H), 7.89- 7.81 (m, 19H), 7.72- 7.52 (m, 16H), 7.47- 7.42 (m, 29H), 7.35 - 7.26 (m, 69H), 7.22 - 7.16 (m, 152H), 7.11- 7.01 (m, 72H), 4.82- 4.72 (m, 32H), 4.68- 4.43 (m, 27H), 4.40 - 4.09 (m, 102H), 4.01 - 3.74 (m, 56H), 3.66 - 3.58 (m, 109H), 3.51- 3.42 (m, 2901H), 3.30- 3.25 (m, 47H), 3.13 - 2.97 (m, 128H), 2.98 - 2.68 (m, 127H), 2.45 - 2.18 (m, 101H), 2.17 - 1.93 (m, 68H), 1.94 - 1.73 (m, 90H), 1.54- 1.46 (m, 172H), 1.36 - 1.11 (m, 346H), 0.93 - 0.77 (m, 257H).

### Chemical synthesis of 50-65

### Synthesis route of 50-65

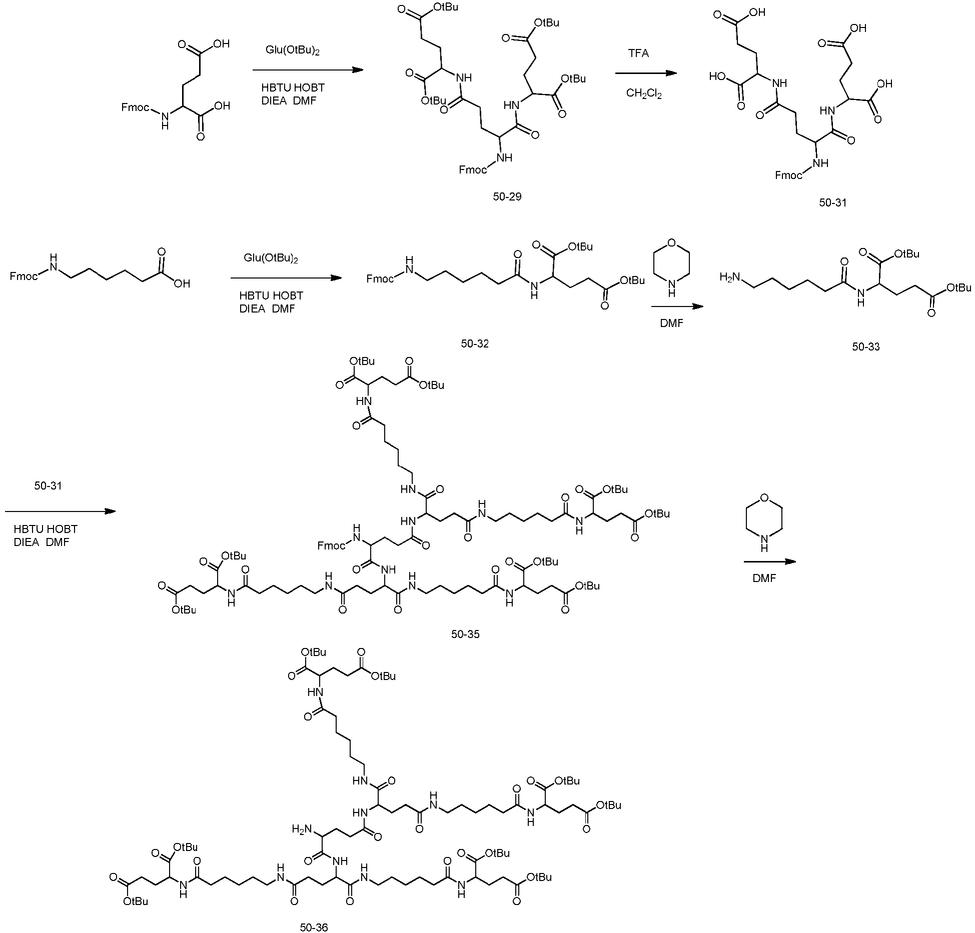

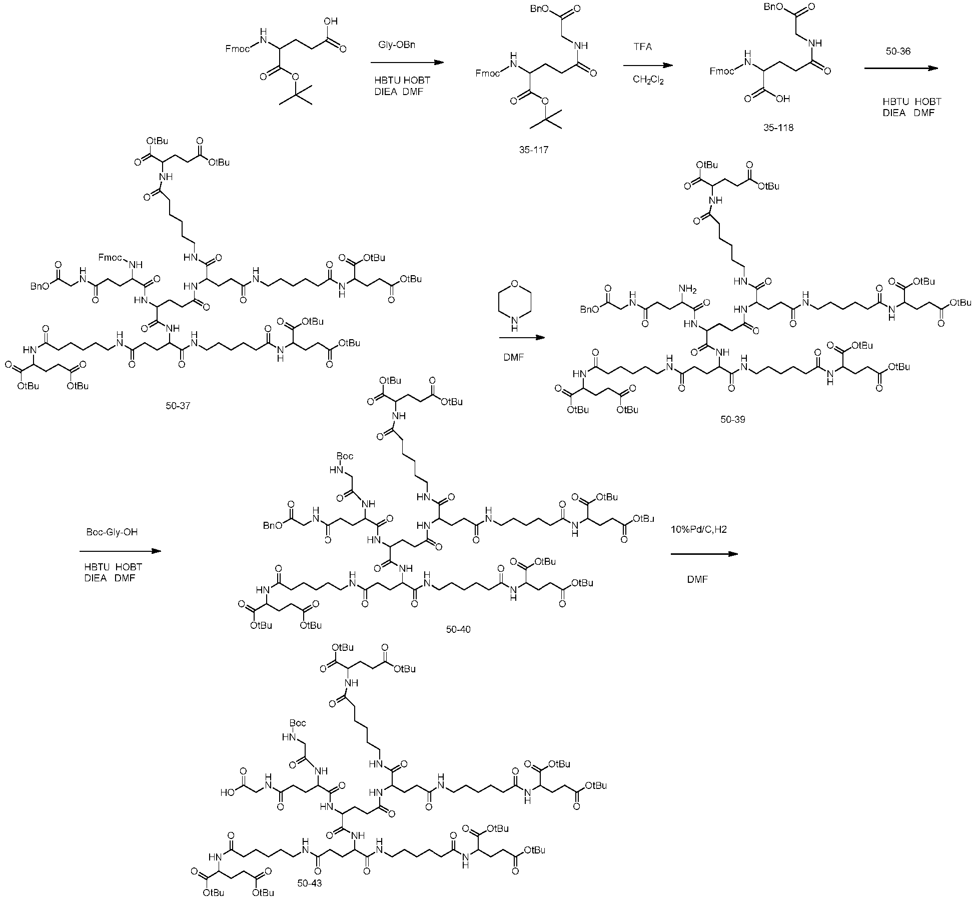

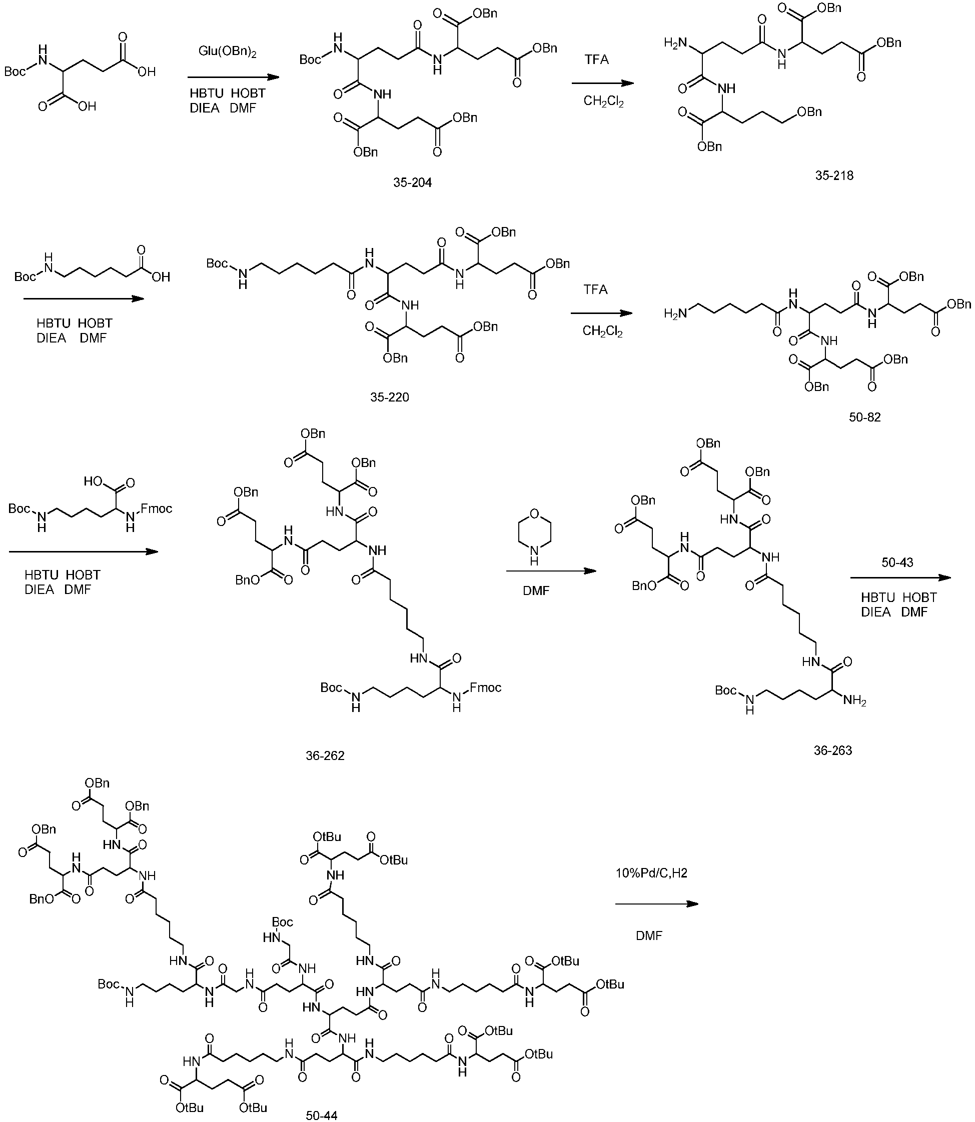

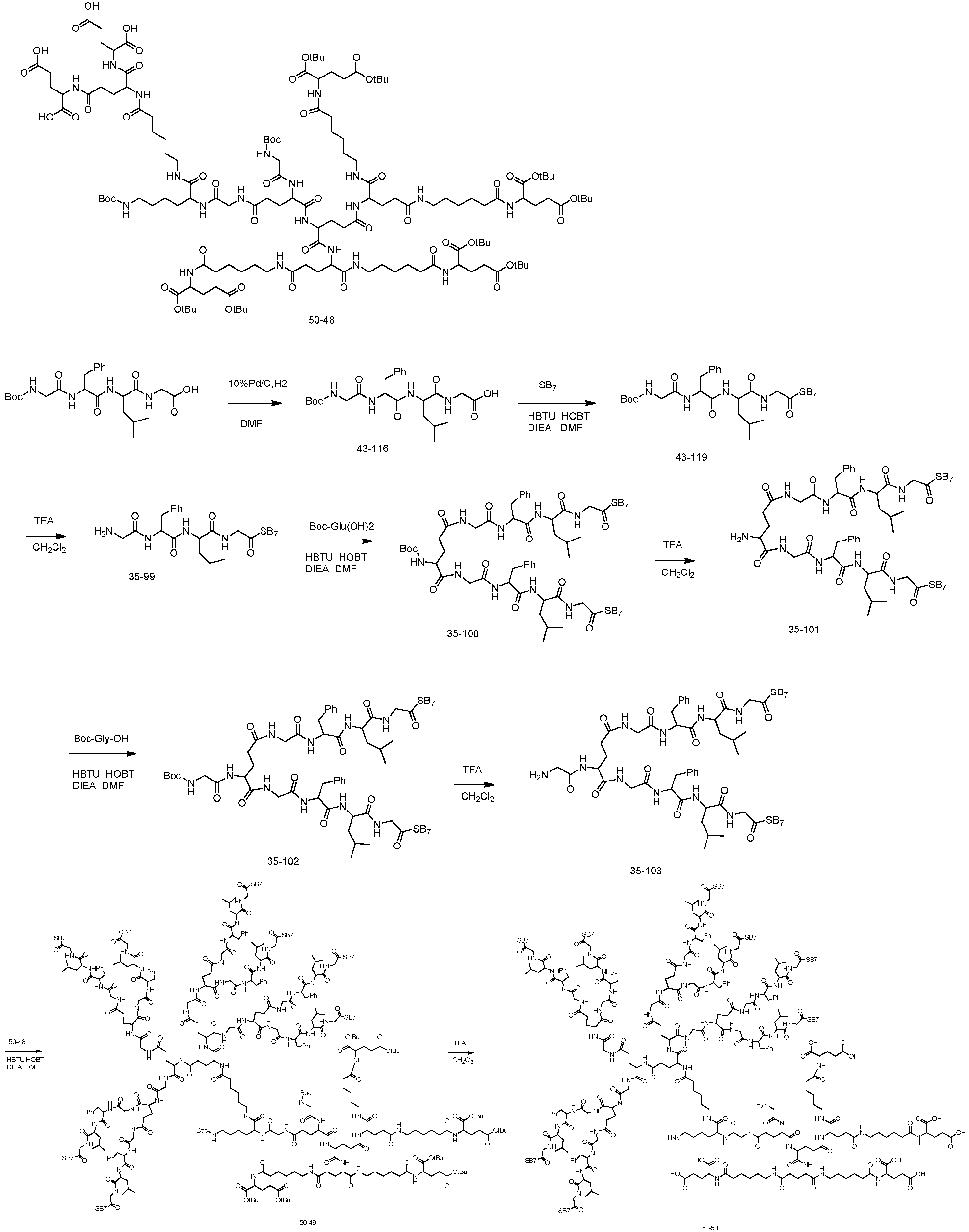

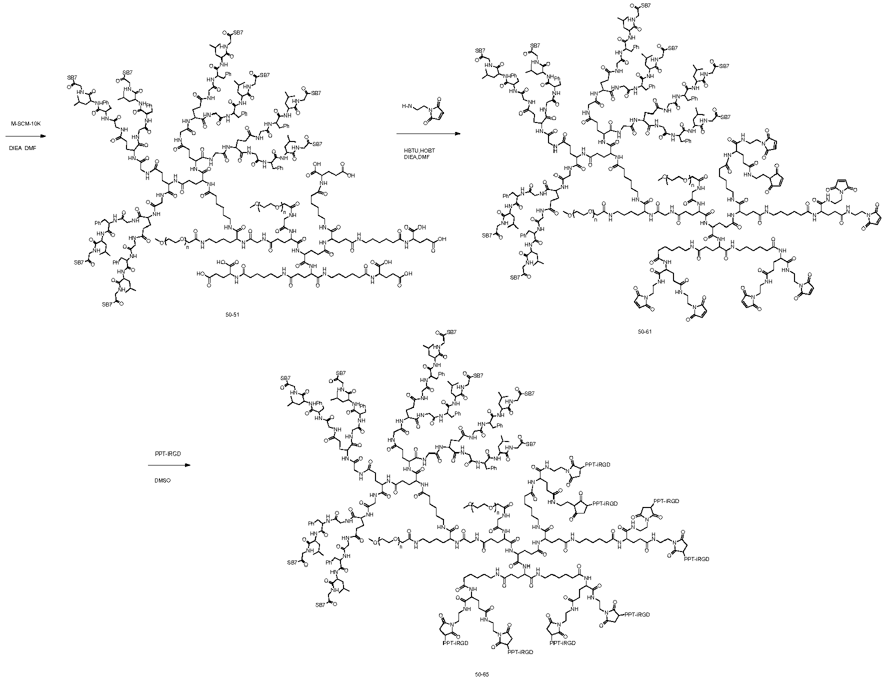

50-29

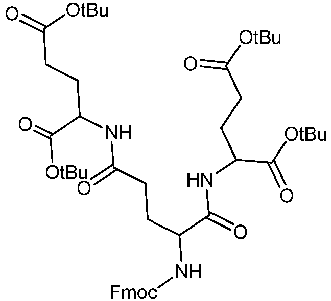

Fmoc-Glu(OH)₂ (2.425 g, 6.5647 mmol, purchased from Aladdin), Glu(OtBu).HCl (3.9 g, 13.129 mmol, purchased from Leyan), HBTU (7.5 g, 19.7 mmol), and HOBT (2.6 g, 19.7 mmol) were added to a 250 mL flask and dissolved with DMF (35 mL), and the obtained solution was stirred for about 30 min at 0 °C; then, DIEA (9.7 mL, 59.1 mmol) was slowly added dropwise, and at the end of the addition, then the obtained solution was further stirred at 0 °C overnight to react. At the end of the reaction, the reaction solution was transferred to a 1 L separatory funnel, purified water (200 mL) and ethyl acetate (250 mL) were added, and the obtained solution was shaken and extracted, evaporated to dryness, and dried in a vacuum oven, thus obtaining 5.6 g of Product 50-29 50-31

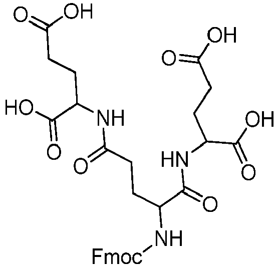

Product 50-29 (5.6 g, 6.5647 mmol) was added in a 250 mL flask and then dissolved with dichloromethane (20 mL), and TFA (9.8 mL, 131.294 mmol) was then added, and the reaction solution was stirred overnight at room temperature to react. At the end of the reaction, the reaction solution was concentrated, methyl tert-butyl ether (50 mL) and n-hexane (150 mL) were added to precipitate the reaction solution many times to obtain an oily product, the obtained product was dried, thus obtaining 4.1 g of Product 50-31. 50-32

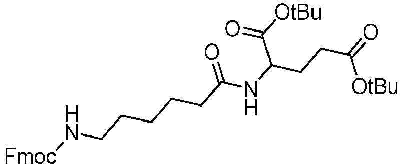

Fmoc-6-aminocaproic-acid (4.53 g, 12.81 mmol, prepared by the reaction of Fmoc-Cl with 6-aminocaproic acid in DMF solvent under DIEA conditions), Glu(OtBu)₂.HCl (3.64 g, 12.81 mmol, purchased from Leyan), HBTU (7.3 g, 19.215 mmol) and HOBT (2.6 g, 19.215 mmol) were added to a 250 mL flask and dissolved with DMF (50 mL), and the obtained solution was stirred for about 30 min at 0 °C; then, DIEA (11.6 mL, 70.455 mmol) was slowly added dropwise, and at the end of the addition, then the obtained solution was further stirred at 0 °C overnight to react. At the end of the reaction, the reaction solution was transferred to a 1 L separatory funnel, purified water (200 mL) and ethyl acetate (250 mL) were added, and the obtained solution was shaken and extracted, evaporated to dryness, and dried in a vacuum oven, thus obtaining 7.6 g of Product 50-32. 50-33

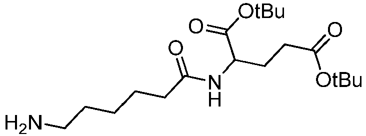

Product 50-32 (7.6 g, 12.81 mmol) was added in a 250 mL flask and then dissolved with DMF (40 mL), and morpholine (11.1 mL, 128.1 mmol) was added, and the reaction solution was stirred overnight at room temperature to react. At the end of the reaction, the reaction solution was transferred to a 1 L separatory funnel and extracted with purified water (200 mL) and ethyl acetate (200 mL) to obtain an organic phase; the aqueous phase was washed with ethyl acetate (200 mL × 1), and the obtained organic phases were combined, concentrated, and then dried, thus obtaining 4.8 g of Product 50-33. 50-35

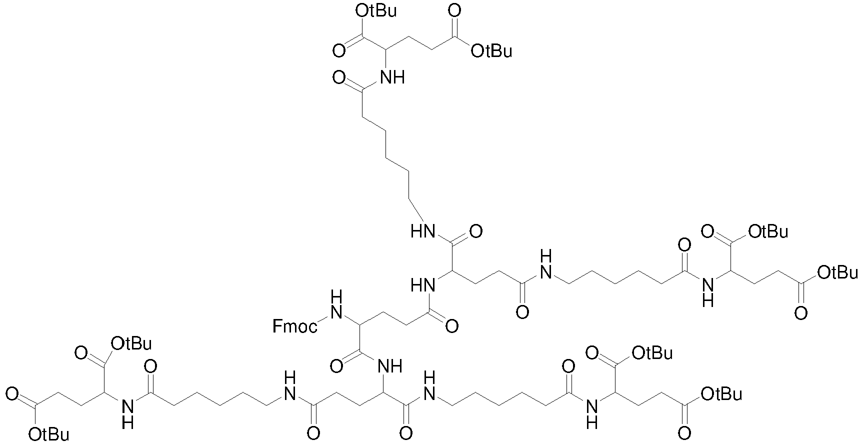

Product 50-31 (1.83 g, 2.911 mmol), Product 50-33 (4.8 g, 12.81 mmol), HBTU (6.6 g, 17.46 mmol) and HOBT (2.36 g, 17.46 mmol) were added to a 250 mL flask and dissolved with DMF (40 mL), and the obtained solution was stirred for about 30 min at 0 °C; then, DIEA (11.6 mL, 70.455 mmol) was slowly added dropwise, and at the end of the addition, then the obtained solution was further stirred at 0 °C overnight to react. At the end of the reaction, the reaction solution was transferred to a 1 L separatory funnel; a saturated sodium chloride solution (200 mL) and ethyl acetate (200 mL) were added, and the obtained solution was shaken and extracted, evaporated to dryness, and dissolved with methanol (20 mL) and dichloromethane (100 mL); silica gel powder (30 g) was added to the obtained solution, and the solution was then evaporated to dryness to obtain a powdery solid; the operations of dry sample loading, column chromatography, and elution with a mixed solution (3%-5% methanol/dichloromethane) were carried out; the elution product was then collected, concentrated, and dried in a vacuum oven. 2.2 g of Product 50-35 was obtained with a yield of 42%. 50-36

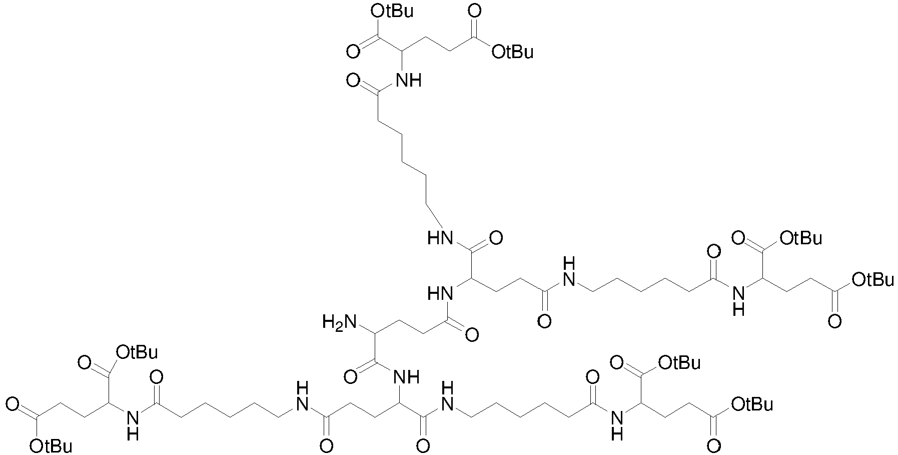

Product 50-35 (2.2 g, 1.0756 mmol) was added in a 250 mL flask and then dissolved with DMF (40 mL), morpholine (1.87 mL, 21.51 mmol) was then added, and the reaction solution was stirred at room temperature for about 1 h. At the end of the reaction, n-hexane (150 mL) and methyl tert-butyl ether (50 mL) were added to precipitate the solution three times, and a solid product was then precipitated; the solution was filtered and the product was washed, and dried in a vacuum oven, thus obtaining 1.96 g of Product 50-36. 35-117

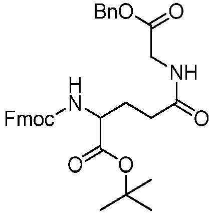

Fmoc-Glu(OtBu) (3.4 g, 8.0 mmol, purchased from Ark pharm), Gly-OBn.HCl (1.8 g, 8.8 mmol, purchased from Accela), HBTU (4.6 g, 12 mmol) and HOBT (1.6 g, 12 mmol) were added to a 250 mL flask and dissolved with DMF (50 mL), and the obtained solution was stirred for about 30 min at 0 °C; then, DIEA (7.3 mL, 44 mmol) was slowly added dropwise, and at the end of the addition, then the obtained solution was further stirred at 0 °C overnight to react. At the end of the reaction, the reaction solution was transferred to a 1 L separatory funnel, purified water (200 mL) and ethyl acetate (250 mL) were added, and the obtained solution was shaken and extracted, evaporated to dryness, and dried in a vacuum oven, thus obtaining 4.5 g of Product 35-117. 35-118

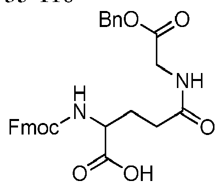

Product 35-117 (4.5 g, 8 mmol) was added in a 250 mL flask and then dissolved with dichloromethane (10 mL), TFA (9 mL, 120 mmol) was then added, and the reaction solution was stirred overnight at room temperature to react. At the end of the reaction, the reaction solution was concentrated. Methyl tert-butyl ether (5100 mL) was added, and a powdery solid was then precipitated; the suction filtering was carried out and the filter cake was dissolved with methanol (10 mL) and dichloromethane (40 mL); methyl tert-butyl ether (150 mL) was added and a powdery solid was then precipitated; the suction filtering was carried out and the filter cake was dried. 4 g of Product 35-118 was obtained. 50-37

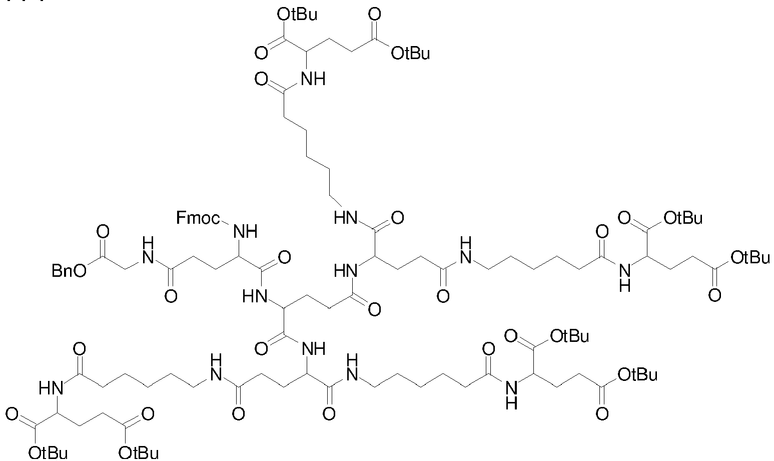

Product 50-36 (1.96 g, 1.0756 mmol), Product 35-118 (0.4 g, 0.8605 mmol), HBTU (0.6 g, 1.6314 mmol) and HOBT (0.218 g, 1.6314 mmol) were added to a 250 mL flask and dissolved with DMF (45 mL), and the obtained solution was stirred for about 20 min at -0 °C; then, DIEA (2.1 mL, 12.5865 mmol) was slowly added dropwise, and at the end of the addition, then the obtained solution was further stirred at 0 °C overnight to react. At the end of the reaction, n-hexane (100 mL) and methyl tert-butyl ether (30 mL) were added to precipitate the solution three times, and a solid product was then precipitated; the solution was filtered and the product was washed, and dried in a vacuum oven, thus obtaining 3.8 g of Product 50-37. 50-39

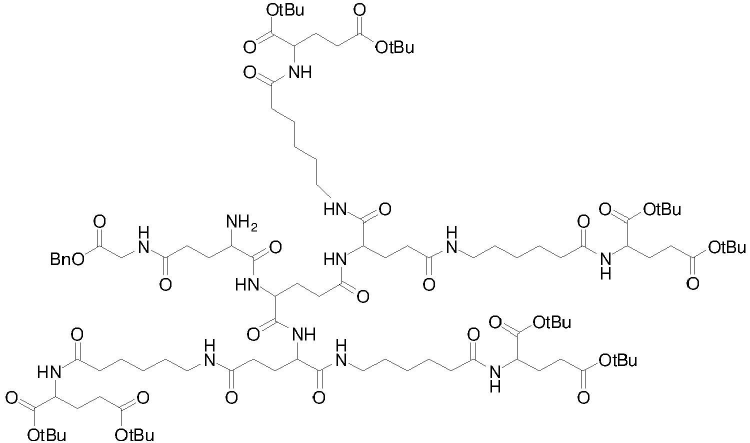

Product 50-37 (1.997 g, 0.8605 mmol) was added in a 250 mL flask and then dissolved with DMF (40 mL), morpholine (1.87 mL, 21.5125 mmol) was then added, and the reaction solution was stirred at room temperature for about 1 h. At the end of the reaction, n-hexane (150 mL) and methyl tert-butyl ether (50 mL) were added to precipitate the reaction solution 4 times and a solid product was obtained; the solid product was filtered, washed, and dried in a vacuum oven, thus obtaining 1.8 g of Product 50-39. 50-40

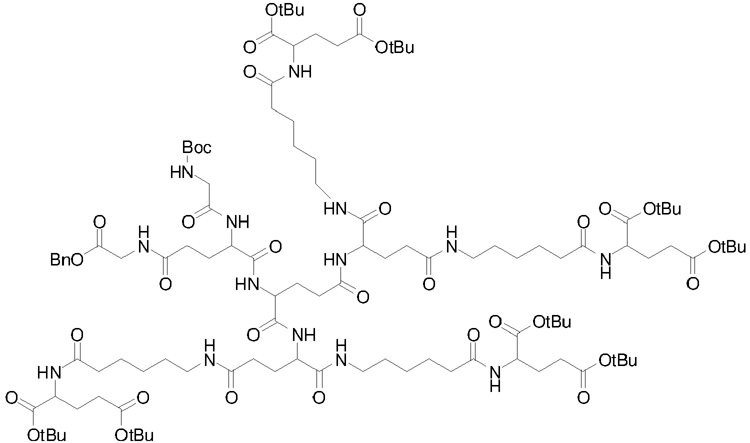

Product 50-39 (1.8 g, 0.8605 mmol), Boc-Gly-OH (0.18 g, 1.0326 mmol, purchased from Ark pharm), HBTU (0.49 g, 1.2908 mmol) and HOBT (0.17 g, 1.2908 mmol) were added to a 250 mL flask and dissolved with DMF (35 mL), and the obtained solution was stirred for about 20 min at -5 °C; then, DIEA (0.6 mL, 3.8723 mmol) was slowly added dropwise, and at the end of the addition, the obtained solution was stirred at -5 °C overnight to react. At the end of the reaction, n-hexane (100 mL) and methyl tert-butyl ether (30 mL) were added to precipitate the reaction solution 3 times and a solid product was obtained; the solid product was filtered, and dissolved with methanol (20 mL) and dichloromethane (100 mL); silica gel powder (10 g) was added to the obtained solution, and the solution was then evaporated to dryness to obtain a powdery solid; the operations of dry sample loading, column chromatography, and elution with a mixed solution (6%-10% methanol/dichloromethane) were carried out; the elution product was then collected, concentrated, and dried in a vacuum oven. 0.6 g of Product 50-40 was obtained with a yield of 32%. 50-43

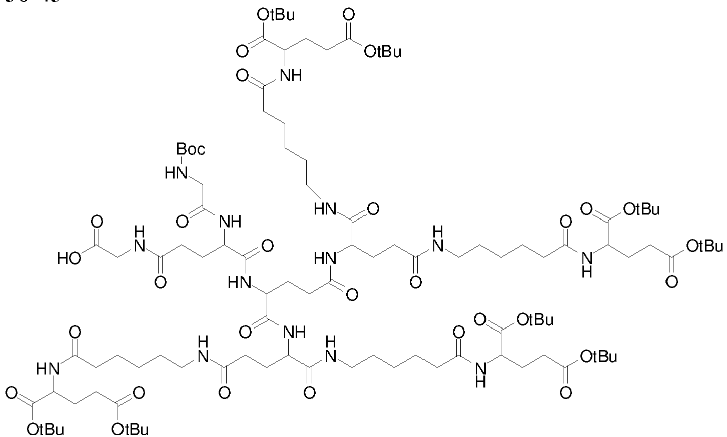

Product 50-40 (0.6 g, 0.266 mmol) and 10% Pd/C (0.021 g) were added in a hydrogenation reactor and then dissolved with DMF (30 mL); hydrogen was introduced to a pressure of Pa=1.8 MPa in the reactor, and the mixed solution then was stirred overnight at room temperature to react. At the end of the reaction, the reaction solution was filtered with diatomaceous earth; the diatomaceous earth was washed with DMF (20 mL × 3), and the DMF solution was combined, as the raw material for the next step. 35-204

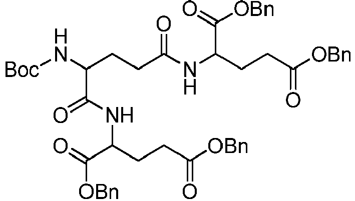

Boc-Glu-OH (7 g, 28.3 mmol, purchased from Ark pharm), Glu(OBn)₂.TosOH (31 g, 62.6 mmol, purchased from Ark pharm), HBTU (32 g, 84.9 mmol), and HOBT (11.5 g, 84.9 mmol) were added to a 250 mL flask and dissolved with DMF (200 mL), and the obtained solution was stirred for about 30 min at -5 °C; then, DIEA (42 mL, 254.7 mmol) was slowly added dropwise, and at the end of the addition, then the obtained solution was stirred at room temperature overnight to react. At the end of the reaction, the reaction solution was transferred to a 1 L separatory funnel; a saturated sodium bicarbonate solution (200 mL) and ethyl acetate (250 mL) were added, and the obtained solution was shaken and extracted, evaporated to dryness, and dried in a vacuum oven, thus obtaining 24.8 g of Product 35-204. 35-218

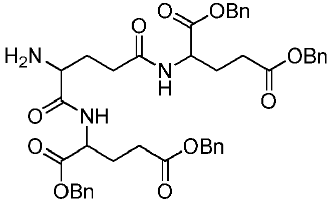

Product 35-204 (24.5 g, 28.3 mmol) was added in a 250 mL flask and then dissolved with dichloromethane (20 mL), TFA (30 mL, 404.2 mmol) was then added, and the reaction solution was stirred overnight at room temperature to react. At the end of the reaction, the reaction solution was concentrated. Methyl tert-butyl ether (200 mL) was added and a powdery solid was then precipitated; the suction filtering was carried out and the filter cake was dissolved with methanol (10 mL) and dichloromethane (40 mL); methyl tert-butyl ether (150 mL) was added and a powdery solid was then precipitated; the suction filtering was carried out and the filter cake was dried, thus obtaining 21.7 g of Product 35-218 35-220

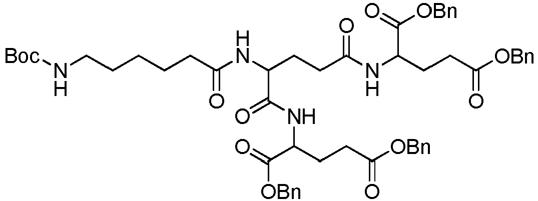

Product 35-218 (21.7 g, 28.3 mmol), N-Boc-6-aminocaproic acid (5.9 g, 25.7 mmol, prepared by the reaction of Boc₂O with 6-aminocaproic acid in dichloromethane solvent under triethylamine conditions), HBTU (14.6 g, 38.55 mmol) and HOBT (5.2 g, 38.55 mmol) were added to a 250 mL flask and dissolved with DMF (200 mL), and the obtained solution was stirred for about 30 min at -5 °C; then, DIEA(19 mL, 115.65 mmol) was slowly added dropwise; and at the end of the addition, then the obtained solution was stirred at room temperature overnight to react. At the end of the reaction, the reaction solution was transferred to a 1 L separatory funnel; a saturated sodium bicarbonate solution (200 mL) and ethyl acetate (250 mL) were added, and the obtained solution was shaken and extracted, evaporated to dryness, and dried in a vacuum oven, thus obtaining 25.2 g of Product 35-220. 50-82

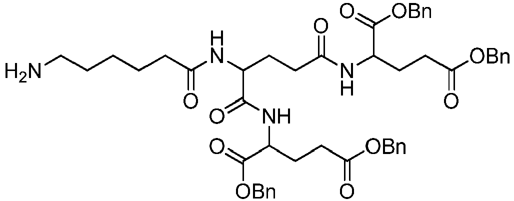

Product 35-220 (6 g, 6.128 mmol) was added in a 250 mL flask and then dissolved with dichloromethane (10 mL), TFA (4.6 mL, 61.28 mmol) was then added, and the reaction solution was stirred overnight at room temperature to react. At the end of the reaction, the reaction solution was concentrated; methyl tert-butyl ether (200 mL),was added and a powdery solid was then precipitated; the suction filtering was carried out and the filter cake was dissolved with methanol (10 mL) and dichloromethane (40 mL); methyl tert-butyl ether (200 mL) was added and a powdery solid was then precipitated; the suction filtering was carried out and the filter cake was dried, thus obtaining 4.5 g of Product 50-82 with a yield of 83%. 36-262

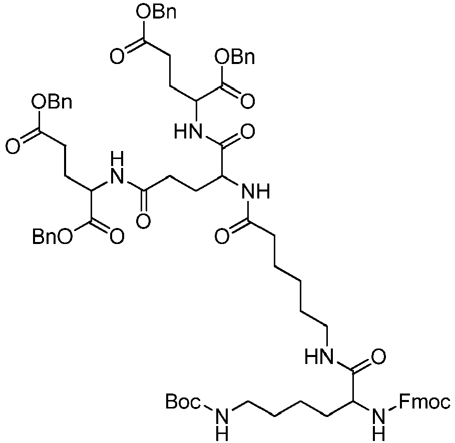

Product 50-82 (4.5 g, 5.119 mmol), Fmoc-Lys(Boc)-OH (2 g, 4.266 mmol, purchased from Aladdin), HBTU (2.4 g, 6.4 mmol) and HOBT (0.86 g, 6.4 mmol) were added to a 250 mL flask and dissolved with DMF (100 mL), and the obtained solution was stirred for about 30 min at 0 °C; then, DIEA (19 mL, 115.65 mmol) was slowly added dropwise, and at the end of the addition, the obtained solution was further stirred overnight. At the end of the reaction, the reaction solution was transferred to a 1 L separatory funnel; a saturated sodium chloride solution (200 mL) and ethyl acetate (250 mL) were added, and the obtained solution was shaken, extracted and evaporated to dryness; the solid product was dissolved with methanol (20 mL) and dichloromethane (100 mL); silica gel powder (10 g) was added to the obtained solution, and the solution was then evaporated to dryness to obtain a powdery solid; the operations of dry sample loading, column chromatography, and elution with a mixed solution (5%-8% methanol/dichloromethane) were carried out; the elution product was then collected, concentrated, and dried in a vacuum oven. 4.8 g of Product 36-262 was obtained with a yield of 84%. 36-263

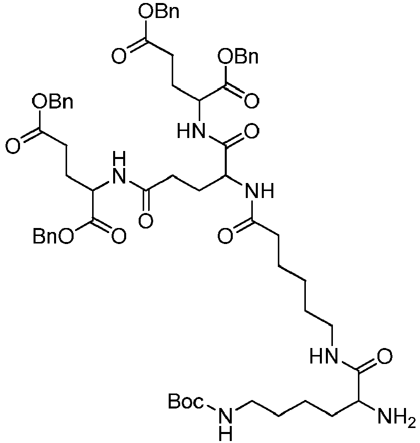

Product 36-262 (4.8 g, 3.6 mmol) was added in a 250 mL flask and then dissolved with DMF (40 mL), morpholine (6.3 mL, 72 mmol) was added, and the reaction solution was stirred overnight at room temperature to react. At the end of the reaction, the reaction solution was transferred to a 1 L separatory funnel and extracted with purified water (200 mL) and ethyl acetate (200 mL) to obtain an organic phase; the aqueous phase was washed with ethyl acetate (200 mL × 1), and the obtained organic phases were combined, concentrated, and then dried, thus obtaining 4 g of Product 36-263. 50-44

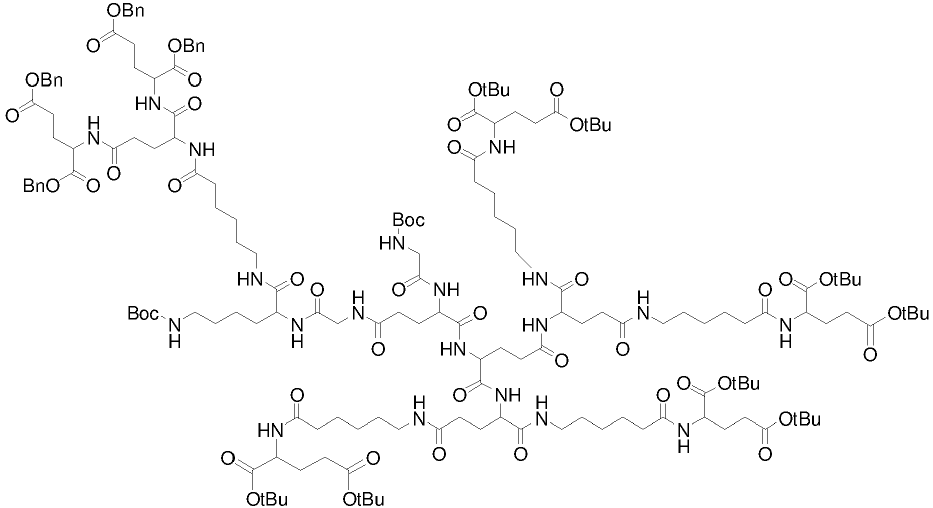

Product 50-43 (0.576 g, 0.266 mmol), Product 36-263 (0.383 g, 0.3458 mmol), HBTU (0.151 g, 0.399 mmol) and HOBT (0.054 g, 0.399 mmol) were added in a 100 mL flask and then dissolved with DMF (30 mL), and the obtained solution was stirred for about 20 min at -5 °C; then, DIEA (0.197 mL, 1.197 mmol) was slowly added dropwise, and at the end of the addition, the obtained solution was first stirred at -5 °C for 1 h to react, and then placed at room temperature overnight. At the end of the reaction, n-hexane (100 mL) and methyl tert-butyl ether (30 mL) were added to precipitate the reaction solution 4 times and a solid product was obtained; the solid product was filtered, and dissolved with methanol (20 mL) and dichloromethane (100 mL); silica gel powder (10 g) was added to the obtained solution, and the solution was then evaporated to dryness to obtain a powdery solid; the operations of dry sample loading, column chromatography, and elution with a mixed solution (1% ammonia water and 3%-8% methanol/dichloromethane) were carried out; the elution product was then collected, concentrated, and dried in a vacuum oven. 0.46 g of Product 50-44 was obtained with a yield of 53%.

¹H-NMR (600 MHz, DMSO-d₆) δ 8.38 (d, J = 7.3 Hz, 1H), 8.37- 8.24 (m, 1H), 8.19- 8.05 (m, 6H), 7.96- 7.86 (m, 9H), 7.73- 7.64 (m, 1H), 7.57 - 7.54 (m, 2H), 7.44 - 7.40 (m, 1H), 7.36 - 7.32 (m, 19H), 5.14 - 5.00 (m, 9H), 4.64- 4.53 (m, 1H), 4.43 - 4.25 (m, 4H), 3.92 - 3.72 (m, 3H), 3.72 - 3.58 (m, 15H), 3.50- 3.42 (m, 6H), 3.34- 3.16 (m, 14H), 3.06 - 2.92 (m, 12H), 2.89 - 2.84 (m, 6H), 2.79- 2.65 (m, 6H), 2.62 (s, 1H), 2.55- 2.49 (m, 2H), 2.46 - 2.36 (m, 5H), 2.23 - 2.02 (m, 7H), 1.92 - 1.83 (m, 4H), 1.71-1.63 (m, 4H), 1.51-1.42 (m, 3H), 1.40 -1.33 (m, 14H), 1.31 - 1.21 (m, 90H), 1.24- 1.21 (m, 12H), 1.12 (t, J = 4.8 Hz, 1H). 50-48

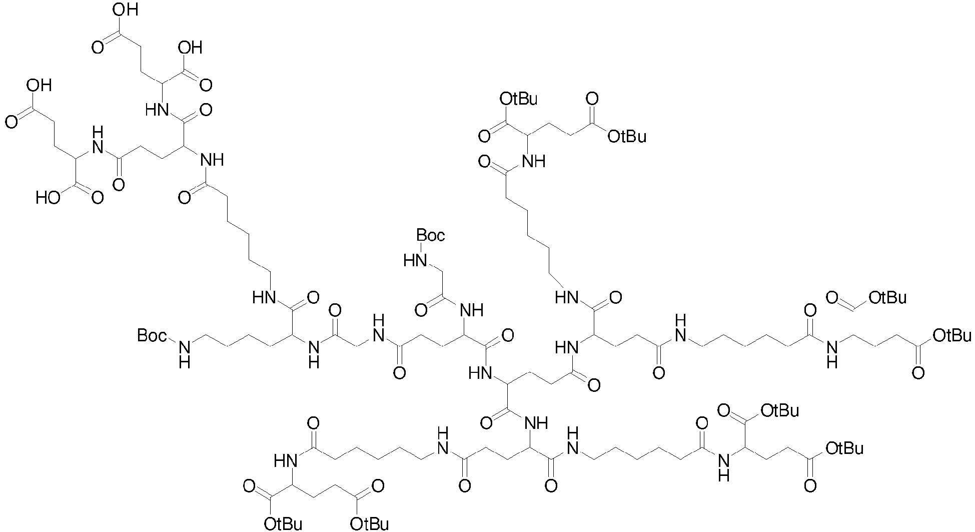

Product 50-44 (0.46 g, 0.1413 mmol) and 10% Pd/C (0.02 g) were added in a hydrogenation reactor and then dissolved with DMF (30 mL); hydrogen was introduced to a pressure of Pa=1.8 MPa in the reactor, and the mixed solution then was stirred overnight at room temperature to react. At the end of the reaction, the reaction solution was filtered with diatomaceous earth; the diatomaceous earth was washed with DMF (20 mL × 3), and the DMF solution was combined, as the raw material for the next step. 50-49

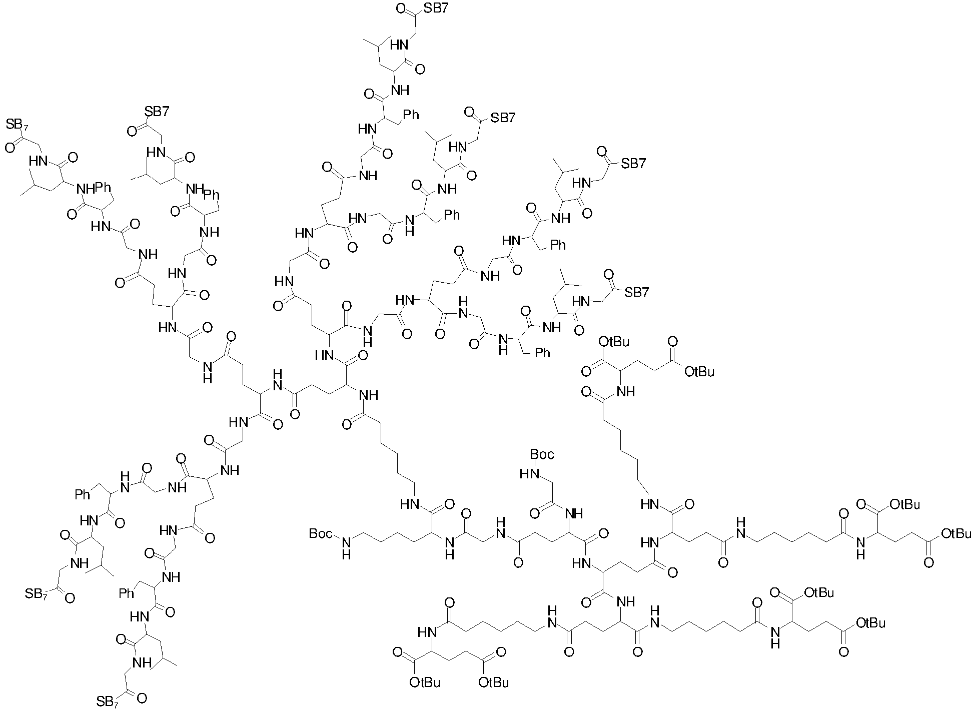

Product 50-48 (0.409 g, 0.1413 mmol), Product 50-45 (1.34 g, 0.6783 mmol, synthesized according to the synthesis method of Product 35-103), HBTU (0.32 g, 0.8478 mmol) and HOBT (0.11 g, 0.8478 mmol) were added in a 100 mL flask and then dissolved with DMF (20 mL), and the obtained solution was stirred for about 20 min at -5 °C; then, DIEA (0.16 mL, 0.9748 mmol) was slowly added dropwise; at the end of the addition, the obtained solution was first stirred at - 5 °C for 1.5 h to react, and then placed at room temperature overnight. At the end of the reaction, n-hexane (100 mL) and methyl tert-butyl ether (30 mL) was added to precipitate the reaction solution 3 times and a solid product was obtained, the solid product was filtered, and the filter cake was dissolved with methanol (20 mL) and dichloromethane (100 mL); silica gel powder (10 g) was added to the obtained solution, and the solution was then evaporated to dryness to obtain a powdery solid; the operations of dry sample loading, column chromatography, and elution with a mixed solution (1% ammonia water/5%-10% methanol/dichloromethane) were carried out; the elution product was then collected, concentrated, and dried in a vacuum oven. 0.86 g of Product 50-49 was obtained with a yield of 57% 50-50

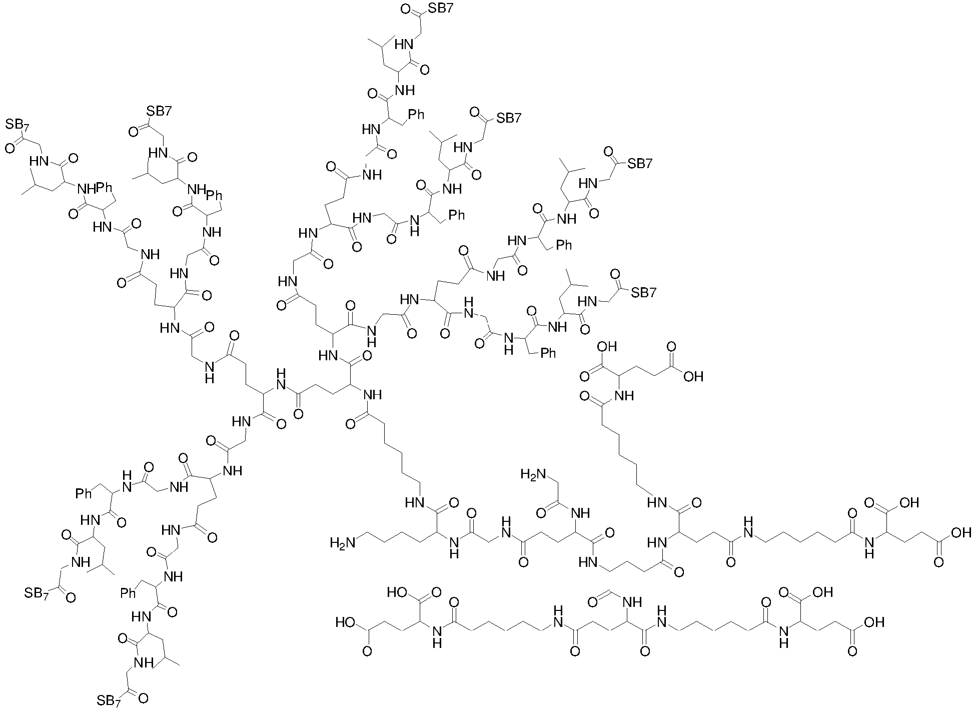

Product 50-49 (0.86 g, 0.08 mmol) was added in a 100 mL flask and then dissolved with dichloromethane (10 mL), TFA (15 mL, 202.1 mmol) was then added, and the reaction solution was stirred overnight at room temperature to react. At the end of the reaction, the reaction solution was concentrated; methyl tert-butyl ether (50 mL) was added and a powdery solid was then precipitated; the suction filtering was carried out and the filter cake was dissolved with methanol (10 mL) and dichloromethane (40 mL); methyl tert-butyl ether (150 mL) was added and a powdery solid was then precipitated; the suction filtering was carried out and the filter cake was dried, thus obtaining 0.78 g of Product 50-50. 50-51

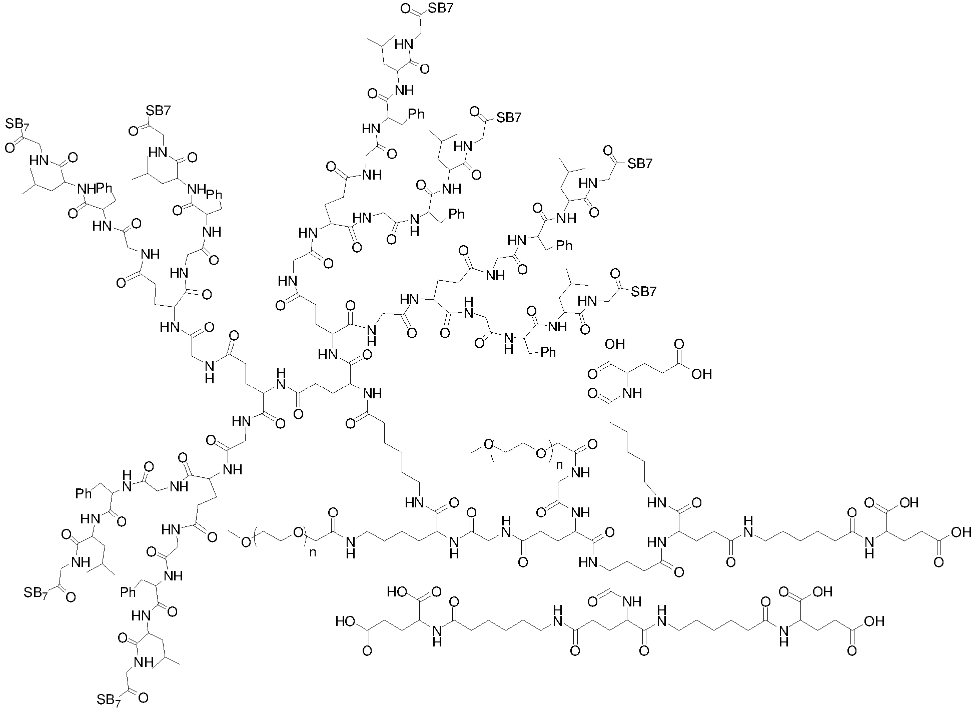

Product 50-50 (0.5 g, 0.0497 mmol) was added in a 2100 mL flask and then dissolved with DMF (25 mL); DIEA (1.5 mL, 8.946 mmol) was added, and the obtained solution was stirred for 20 min; M-SCM-10K (1.16 g, 0.1095 mmol) was added, and the obtained solution was slowly stirred at room temperature to react for one week in the dark. At the end of the reaction, n-hexane (100 mL) and methyl tert-butyl ether (50 mL) were added, and the precipitation was carried out 3 times to obtain an oily product; the product was dissolved with methanol (20 mL) and dichloromethane (100 mL); silica gel powder (10 g) was added to the obtained solution, and the solution was then evaporated to dryness to obtain a powdery solid; the operations of dry sample loading, column chromatography, and elution with a mixed solution (1% ammonia water/5%-8% methanol/dichloromethane) were carried out; the elution product was then collected, concentrated, and dried in a vacuum oven. 0.8 g of Product 50-51 was obtained with a yield of 53%. 50-61

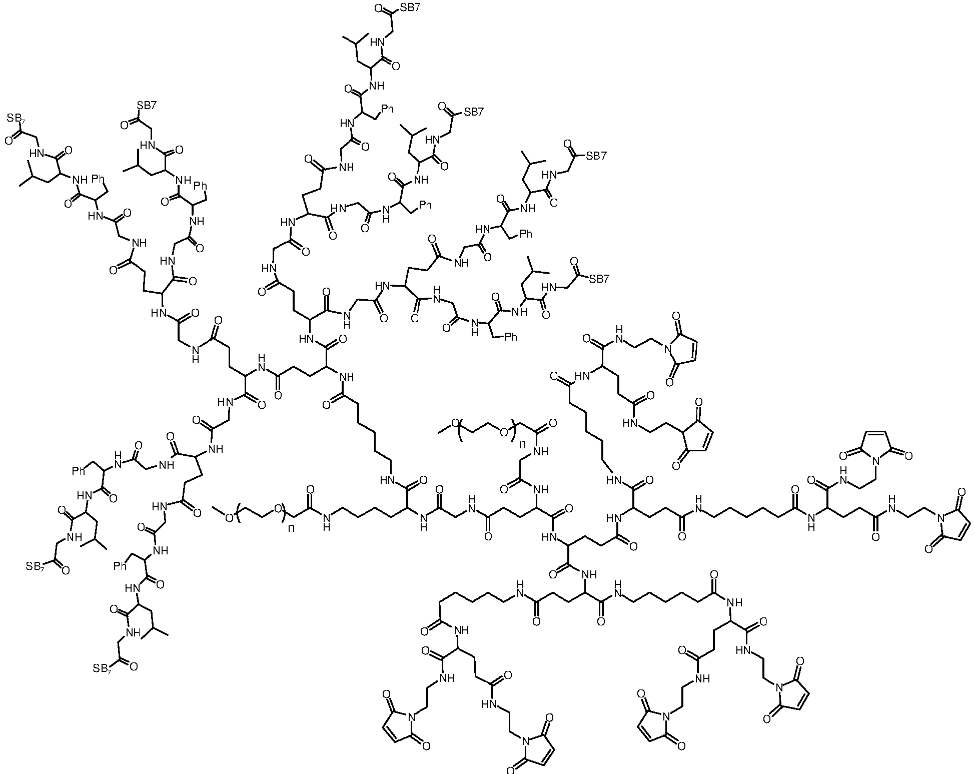

Product 50-51 (0.8 g, 0.0257 mmol), N-maleimide-ethylenediamine (0.04 g, 0.2264 mmol), HBTU (0.117 g, 0.3084 mmol) and HOBT (0.042 g, 0.3084 mmol) were added in a 100 mL flask and then dissolved with DMF (60 mL), and the obtained solution was stirred for about 20 min at -5 °C; then, DIEA (0.19 mL, 1.1308 mmol) was slowly added dropwise; at the end of the addition, the obtained solution was first stirred at -5 °C for 1 h to react, and then placed at room temperature overnight. At the end of the reaction, n-hexane (100 mL) and methyl tert-butyl ether (30 mL) were added to precipitate the solution four times, and a powdery solid was then precipitated; the suction filtering was carried out and the filter cake was dried, thus obtaining 0.75 g of Product 50-61. 50-65

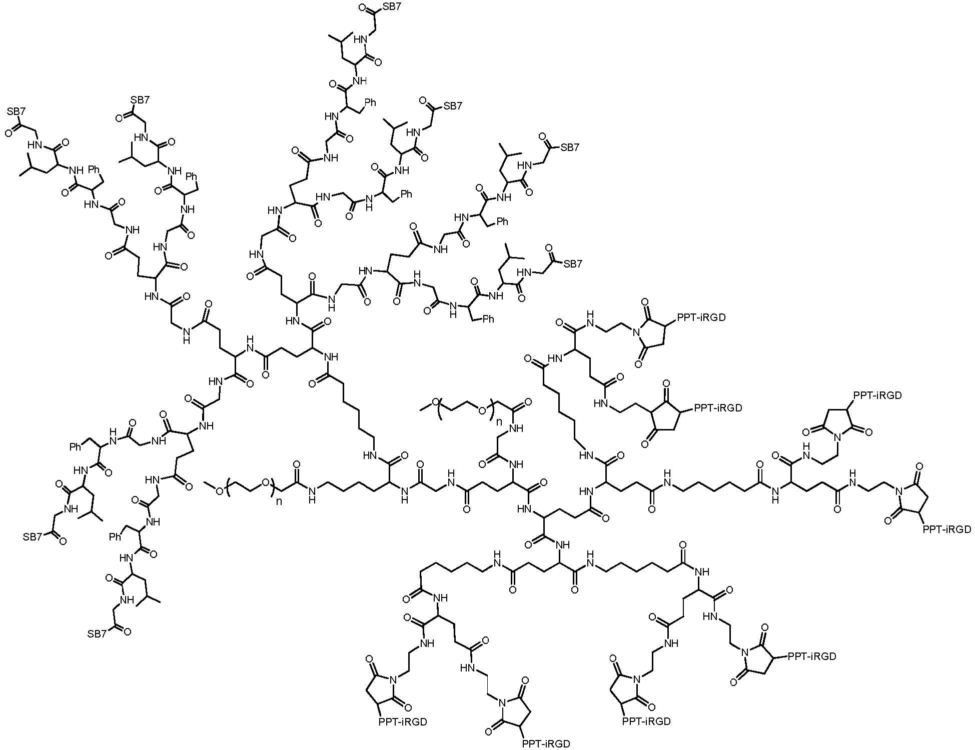

Product 50-61 (0.51 g, 0.0159 mmol) was added in a 100 mL flask and then dissolved with PPT-IRGD (0.224 g, 0.14 mmol) and DMSO (35 mL), and the obtained solution was stirred at 40 °C for 2 days. At the end of the reaction, ethyl acetate (40 mL) and n-hexane (200 mL) were added to precipitate the reaction solution, and the precipitation was carried out 5 times to obtain a viscous product; methyl tert-butyl ether (150 mL) was added, and a solid product was precipitated; suction filtering was then carried out, and the filter cake was dissolved with methanol (20 mL) and dichloromethane (100 mL); silica gel powder (15 g) was then added to the obtained solution, and the solution was then evaporated to dryness to obtain a powdery solid; the operations of dry sample loading, column chromatography, and elution with a mixed solution (1% ammonia water and 5-10% methanol/dichloromethane) were carried out; the elution product was then collected, concentrated, and dried in a vacuum oven, thus obtaining 0.16 g of Product 50-65 with a yield of 23%.

¹H-NMR (600 MHz, DMSO-d₆) δ 9.16- 9.05 (m, 4H), 8.29- 8.18 (m, 25H), 8.13 - 8.04 (m, 49H), 7.94- 7.76 (m, 13H), 7.63- 7.45 (m, 25H), 7.32-7.24 (m, 139H), 7.22- 7.06 (m, 79H), 6.79 - 6.64 (m, 4H), 4.61- 4.43 (m, 18H), 4.27- 4.12 (m, 44H), 3.99- 3.86 (m, 21H), 3.77 - 3.58 (m, 80H), 3.51- 3.49 (m, 1945H), 3.45 - 3.41 (m, 22H), 3.38- 3.26(m, 42H), 3.24- 3.19 (m, 13H), 3.17-3.15 (m, 9H), 3.12 - 3.07 (m, 15H), 3.08 - 2.96 (m, 32H), 2.88- 2.76 (m, 79H), 2.72- 2.68 (m, 52H), 2.62- 2.59 (m, 20H), 2.53- 2.45 (m, 48H), 2.38 - 2.26 (m, 38H), 2.22 - 2.01 (m, 45H), 1.87- 1.67 (m, 53H), 1.60- 1.48 (m, 73H), 1.44- 1.35 (m, 65H), 1.28 - 1.05 (m, 125H), 0.95- 0.75 (m, 172H), 0.63 - 0.48 (m, 37H).

### Synthesis of Compound 60-43

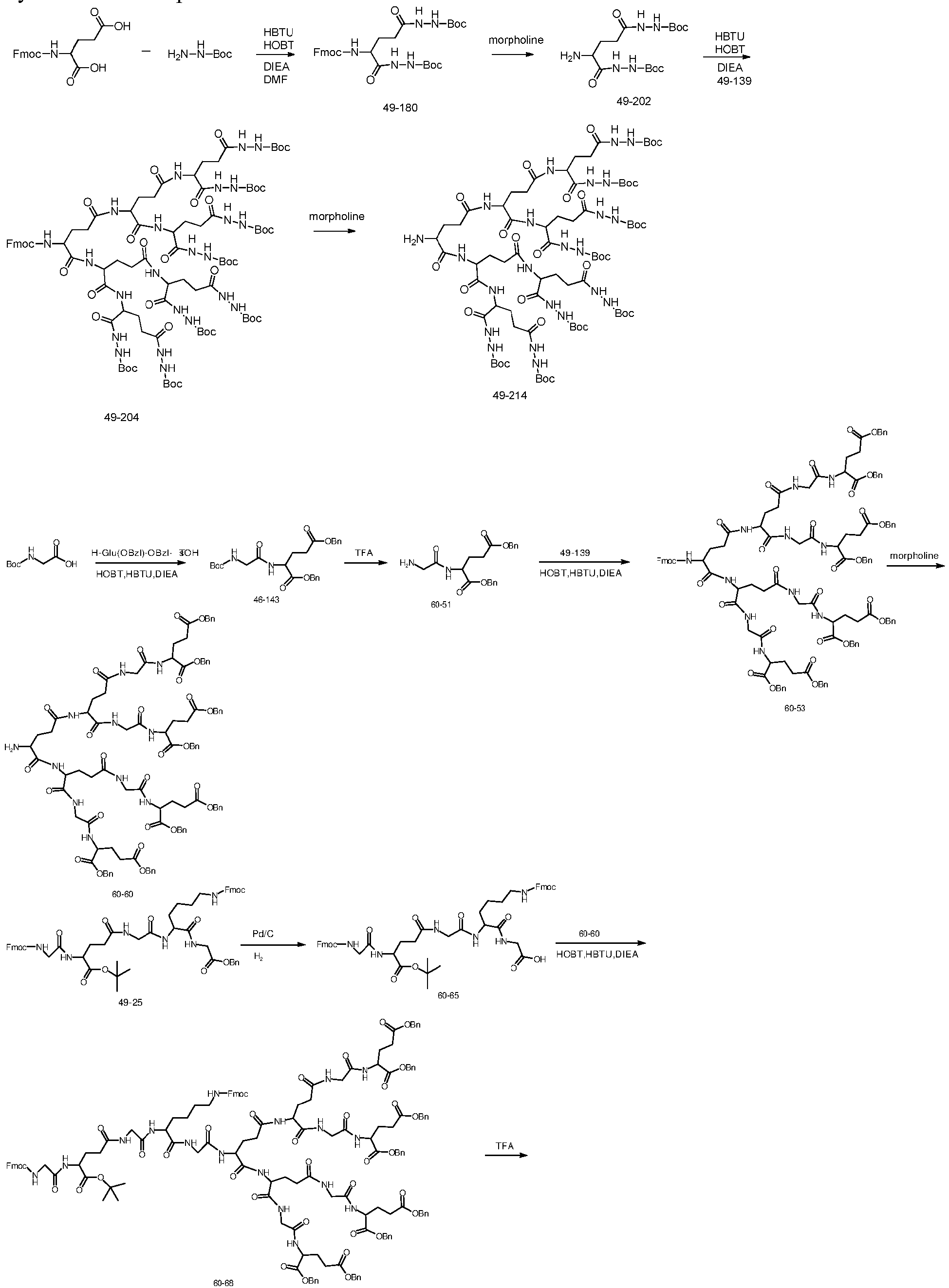

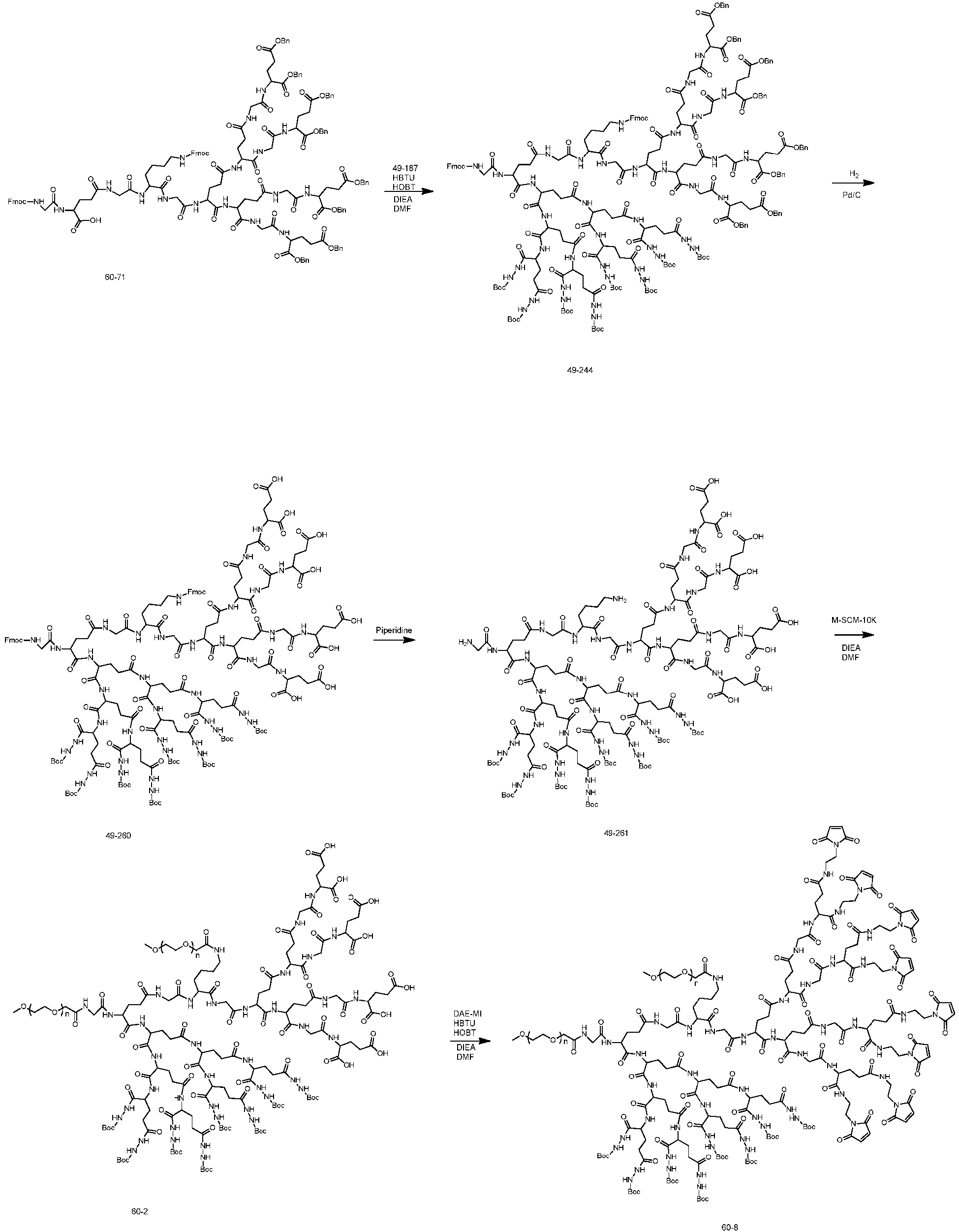

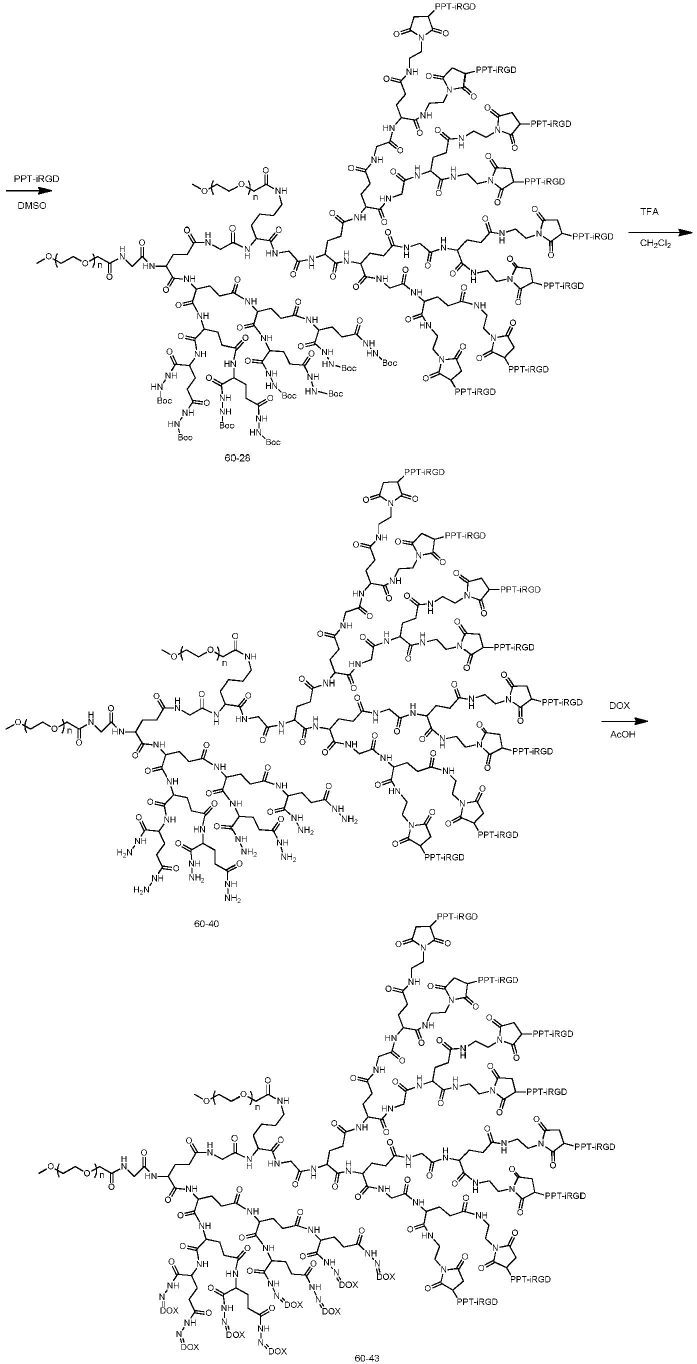

49-180

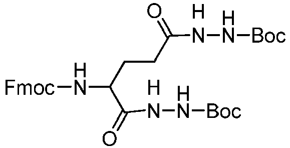

Fmoc-Glu-OH (3 g, 8.1219 mmol, purchased from Aladdin), HBTU (9.23 g, 24.3658 mmol) and HOBT (3.28 g, 24.3658 mmol) were weighed and added in a flask with Boc-NHNH₂ (2.25 g, 17.0560 mmol, purchased from Innochem) and then dissolved with an appropriate amount of DMF, and the obtained solution was placed at -5 °C; then, DIEA (0.81 mL, 4.9216 mmol) was slowly added dropwise; after addition and reaction for 30 min, the reaction solution was taken out, and the obtained solution was stirred overnight at room temperature to react. At the end of the reaction, n-hexane (25 mL) and methyl tert-butyl ether (200 mL) were first added for precipitation, and the supernatant was discarded; n-hexane and methyl tert-butyl ether were added again for precipitation, and such operations were repeated three times, and the suction filtering was then carried out to obtain a solid product, and the obtained solid product was collected and dried in a vacuum oven, thus obtaining the product (6.2 g being extra-quota product). 49-202

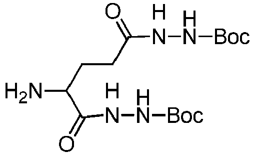

Product 49-180 (4.80 g, 8.1219 mmol) in a flask was dissolved completely with DMF by ultrasonic vibration; morpholine (7.07 mL, 81.219 mmol) was added, and the reaction solution was stirred at room temperature for 2 h to react. At the end of the reaction, n-hexane (25 mL) and methyl tert-butyl ether (200 mL) were first added for precipitation, and the supernatant was discarded; n-hexane and methyl tert-butyl ether were added again for precipitation, and such operations were repeated three times; and the suction filtering was then carried out to obtain a solid product, and the obtained solid product was dissolved with a mixed solvent (dichloromethane and methanol), the operations of dry sample loading, column chromatography, and elution with a mixed solution (3-8% methanol/dichloromethane) were carried out; the elution product was collected, concentrated and dried in a vacuum oven, thus obtaining the product (2.3 g, 75.66%). 49-204

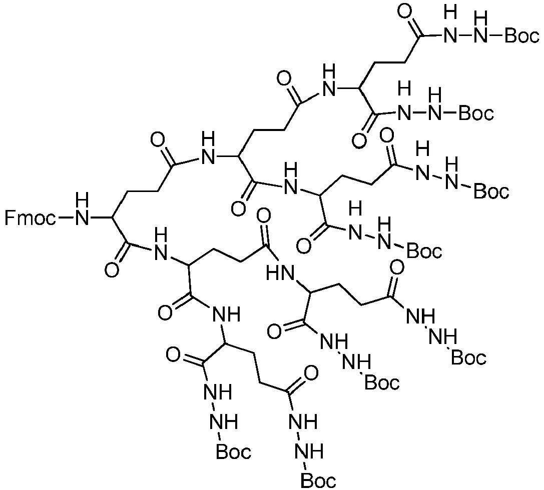

Product 49-202 (2.3 g, 6.1265 mmol), HBTU (3.17 g, 8.3543 mmol) and HOBT (1.13 g, 8.3543 mmol) were weighed and added in a flask with Product 49-139 (0.87 g, 1.3924 mmol) and then dissolved with an appropriate amount of DMF, and the mixed solution was placed at -5 °C; then, DIEA (4.14 mL, 25.0632 mmol) was slowly added dropwise; after addition and reaction for 30 min, the reaction solution was taken out, and the obtained solution was stirred overnight at room temperature to react. At the end of the reaction, n-hexane (25 mL) and methyl tert-butyl ether (200 mL) were first added for precipitation, and the supernatant was discarded; n-hexane and methyl tert-butyl ether were added again for precipitation, and such operations were repeated three times; and the suction filtering was then carried out to obtain a solid product, and the obtained solid product was collected, and dried in a vacuum oven, thus obtaining the product (3.6 g being extra-quota product). 49-214

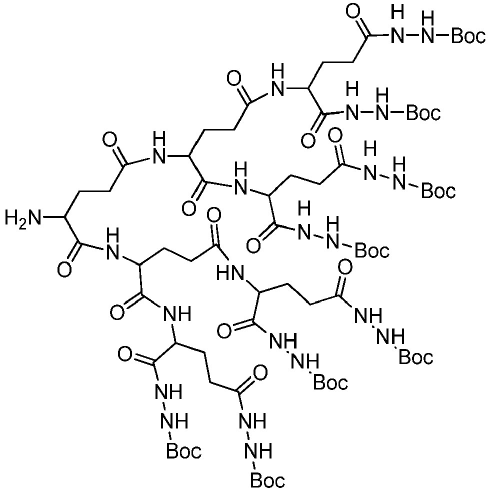

Product 49-204 (2.86 g, 1.3924 mmol) in a flask was dissolved completely with DMF by ultrasonic vibration; morpholine (1.21 mL, 13.924 mmol) was added, and the reaction solution was stirred at room temperature for 2 h to react. At the end of the reaction, n-hexane (25 mL) and methyl tert-butyl ether (200 mL) were first added for precipitation, and the supernatant was discarded; n-hexane and methyl tert-butyl ether were added again for precipitation, and such operations were repeated three times; and the suction filtering was then carried out to obtain a solid product, and the obtained solid product was dissolved with a mixed solvent (dichloromethane and methanol), the operations of dry sample loading, column chromatography, and elution with a mixed solution (4-8% methanol/dichloromethane) were carried out; the elution product was collected, concentrated and dried in a vacuum oven, thus obtaining the product (2.2 g, 86.27%). 46-143

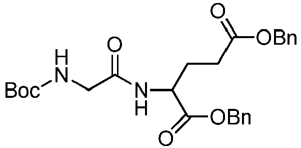

Boc-Gly-OH (5.25 g, 30 mmol, purchased from Aladdin), H-Glu(OBzl)-OBzl TsOH (15 g, 30 mmol, purchased from Ark Pharm), HOBT(6.08 g, 45 mmol) and HBTU (17.08 g, 45 mmol) were weighed and added in a 250 mL flask and then dissolved in DMF solution (80 mL), and the ultrasonic treatment was carried out to completely dissolve the reactants; the obtained solution was stirred for 30 min at -5 °C. DIEA (22.3 mL, 135 mmol) was slowly added dropwise, and the obtained solution reacted at a low temperature to the end. At the end of the reaction, the reaction solution was taken out, deionized water (100 mL) was added to the reaction solution, and extraction with ethyl acetate (100 mL × 3) were carried out several times; the obtained organic phases were combined, washed twice with a saturated sodium chloride solution (100 mL), and then concentrated and evaporated to dryness. The operations of dry sample loading, column chromatography and gradient elution with 20% ethyl acetate/petroleum ether -50% ethyl acetate/petroleum ether were carried out; the elution product was then collected, concentrated and evaporated to dryness, thus obtaining 13 g of the product with a yield of 89%. 60-51

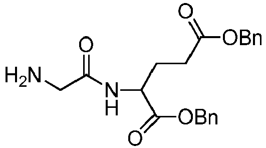

Product 46-143 (19.2 mmol) was weighed and dissolved by ultrasonic with dichloromethane (5 mL) and TFA (22 mL, 300 mmol), a ground glass stopper was used, and the obtained solution was stirred to react at room temperature. At the end of the reaction, the reaction solution was taken out; a saturated sodium bicarbonate solution (300 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness, thus obtaining 13 g of the product 60-53

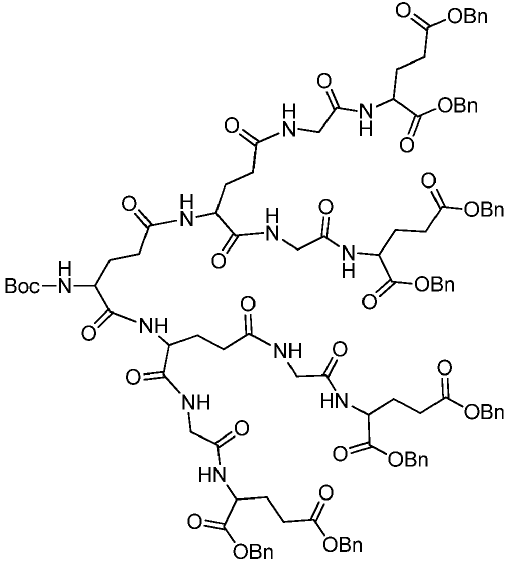

Product 60-51 (10 g, 26.01 mmol), Product 49-139 (5.91 mmol), HOBT (4.79 g, 35.4 mmol) and HBTU (13.45 g, 35.4 mmol) were weighed and added in a 250 mL flask and then dissolved in DMF solution (80 mL); ultrasonic treatment was carried out to completely dissolve the reactants, and the obtained solution was stirred for 30 min at -5 °C; DIEA (17.5 mL, 106.2 mmol) was slowly added dropwise, and the obtained solution reacted at a low temperature to the end. At the end of the reaction, the reaction solution was taken out; deionized water (100 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness. The operations of dry sample loading, column chromatography and gradient elution with 20% ethyl acetate/petroleum ether -50% ethyl acetate/petroleum ether were carried out; the elution product was then collected, concentrated and evaporated to dryness, thus obtaining 8 g of the product with a yield of 66%. 60-60

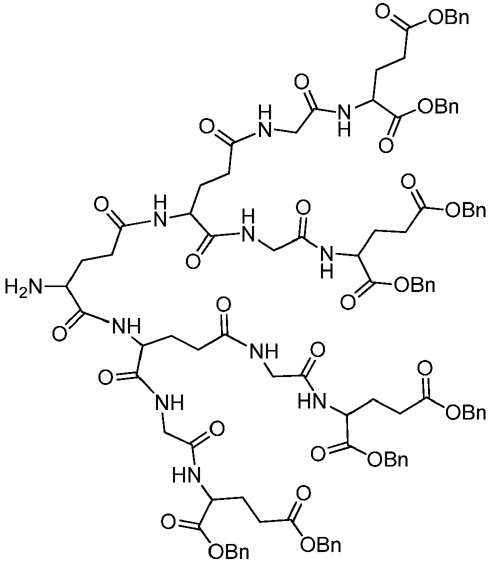

Product 60-53 (8 g, 3.82 mmol) was dissolved with DMF (40 mL), morpholine (3.3 mL, 38.2 mmol) was then added, and the mixed solution was stirred at room temperature to react. At the end of the reaction, methyl tert-butyl ether (150 mL) and n-hexane (100 mL) were added to precipitate the reaction solution, and a solid product was obtained; the obtained solid product was filtered by suction and dried in vacuum, thus obtaining 7.1 g of the product. 60-65

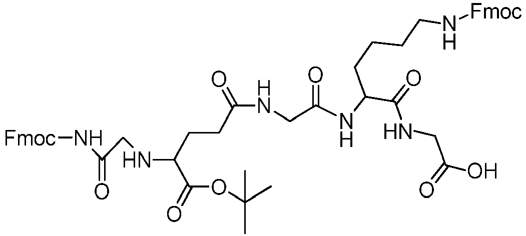

Product 49-25 (3.96 g, 3.82 mmol) was added in a hydrogenation reactor and then 10% Pd/C (0.07 g) was added, and the mixture was dissolved with DMF (20 mL); hydrogen was introduced to a pressure of 300 Psi in the reactor, and the reaction solution was stirred overnight at room temperature to react. At the end of the reaction, the reaction solution was filtered with diatomaceous earth; the filter cake was washed with DMF (15 mL × 3), and the elution solution was added in a 250 mL round-bottomed flask, as the raw material for the next step. 60-68

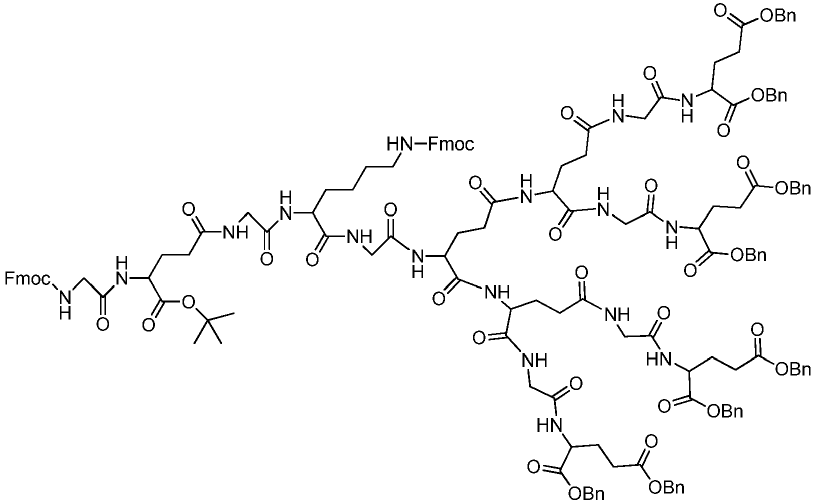

Product 60-65 (3.82 mmol), Product 60-60 (7.1 g, 3.82 mmol), HOBT (0.77 g, 5.73 mmol) and HBTU (2.17 g, 5.73 mmol) were weighed and added in a 250 mL flask and then dissolved in DMF solution (80 mL), and the ultrasonic treatment was carried out to completely dissolve the reactants; the obtained solution was stirred for 30 min at -5 °C; DIEA (2.8 mL, 17.19 mmol) was slowly added dropwise, and the obtained solution reacted at a low temperature to the end. At the end of the reaction, the reaction solution was taken out; deionized water (100 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness. The operations of dry sample loading, column chromatography and gradient elution with 20% ethyl acetate/petroleum ether -50% ethyl acetate/petroleum ether were carried out; the elution product was then collected, concentrated and evaporated to dryness, thus obtaining 8 g of the product with a yield of 75%. 60-71

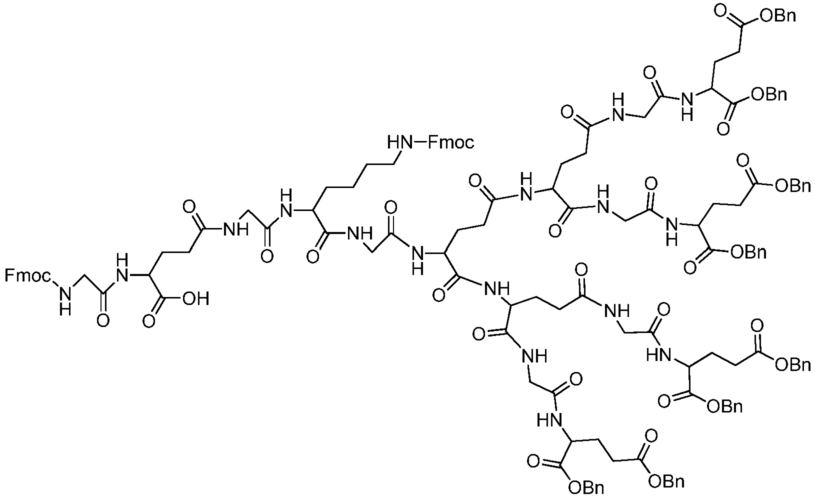

Product 60-68 (8.0 g, 2.85 mmol) was weighed and dissolved by ultrasonic with dichloromethane (5 mL) and TFA (6.3 mL, 85.7 mmol), a ground glass stopper was used, and the obtained solution was stirred to react at room temperature. At the end of the reaction, the reaction solution was concentrated; methyl tert-butyl ether (150 mL) and n-hexane (100 mL) were added in the reaction solution, and a solid product was precipitated; the obtained solid product was filtered by suction and dried in vacuum, thus obtaining 7.8 g of the product. 49-244

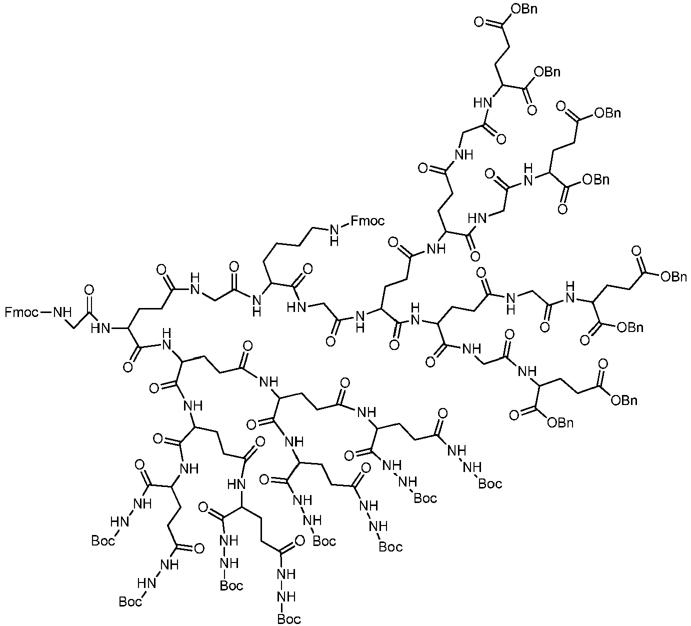

Product 60-71 (3 g, 1.0937 mmol), HBTU (0.62 g, 1.6405 mmol) and HOBT (0.22 g, 1.6405 mmol) were weighed and added in a flask with Product 49-187 (2.55 g, 1.3124 mmol) and then dissolved with an appropriate amount of DMF, and the mixed solution was placed at -5 °C; then, DIEA (0.81 mL, 4.9216 mmol) was slowly added dropwise; after addition and reaction for 30 min, the reaction solution was taken out, and the obtained solution was stirred overnight at room temperature to react. At the end of the reaction, n-hexane (25 mL) and methyl tert-butyl ether (200 mL) were first added for precipitation, and the supernatant was discarded; n-hexane and methyl tert-butyl ether were added again for precipitation, and such operations were repeated three times; and the suction filtering was then carried out to obtain a solid product, and the obtained solid product was dissolved with a mixed solvent (dichloromethane and methanol); the operations of dry sample loading, column chromatography, and elution with a mixed solution (3-5% methanol/dichloromethane) were carried out; the elution product was collected, concentrated and dried in a vacuum oven. The product (3.2 g, 62.74%) was obtained. 49-260

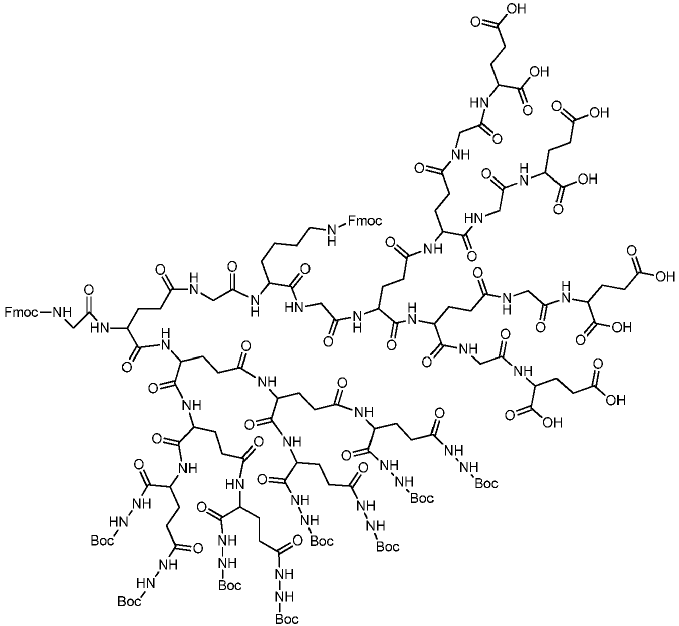

Product 49-244 (2 g, 0.4284 mmol) was added in a hydrogenation reactor and then 10% Pd/C (0.05 g) was added, and the mixture was dissolved with DMF (20 mL); hydrogen was introduced to a pressure of 300 Psi in the reactor, and the reaction solution was stirred overnight at room temperature to react. At the end of the reaction, the reaction solution was filtered with diatomaceous earth; the filter cake was washed with three times with DMF (15 mL × 3); the elution solution was added in a 250 mL round-bottomed flask, and the product was obtained, as the raw material for the next step. 49-261

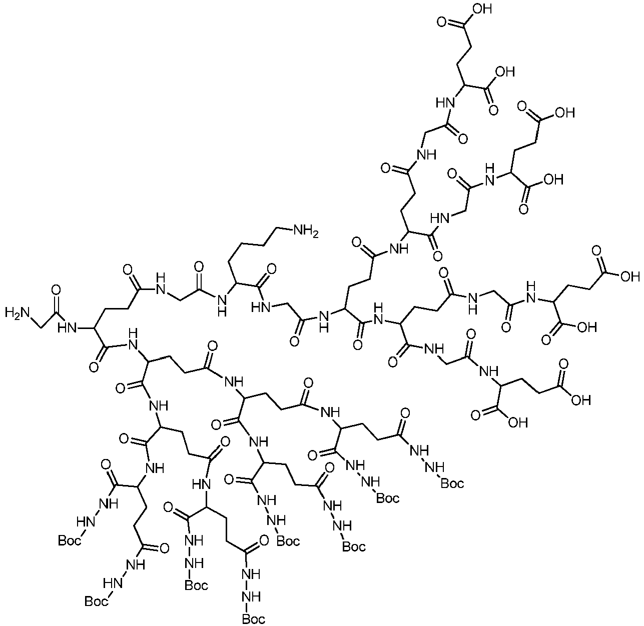

Product 49-260 (1.69 g, 0.4284 mmol) in a flask was dissolved completely with DMF by ultrasonic vibration; piperidine (0.73 mL, 8.568 mmol) was added, and the reaction solution was stirred at room temperature for 2 h to react. At the end of the reaction, the reaction solution was concentrated at reduced pressure, and the DMF solution of the product was obtained, as the raw material for the next step. 60-2

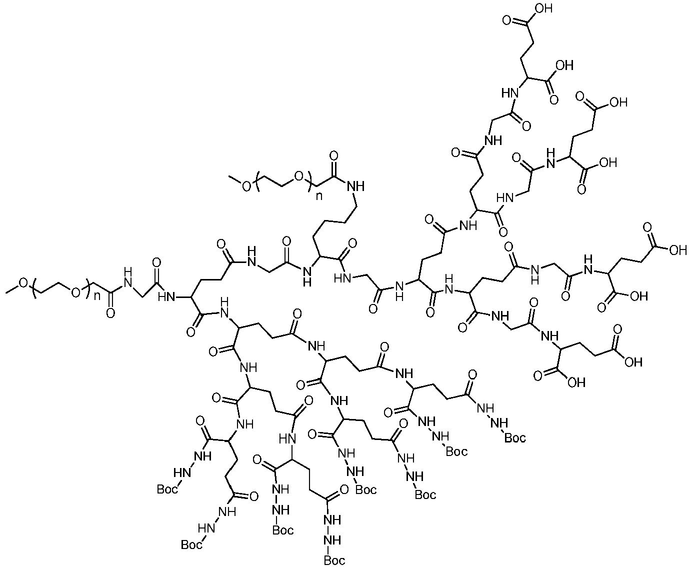

Product 49-261 (0.5 g, 0.1427 mmol) was added in a 250 mL flask and then dissolved with DMF (20 mL), and the mixed solution was slowly added dropwise to the DMF solution containing DIEA (0.42 mL, 2.5686 mmol) and M-SCM-10K (3.14 g, 0.3139 mmol, purchased from JenKem), and the obtained solution was slowly stirred at room temperature to react for one week in the dark. After the reaction was completed, n-hexane (25 mL) and methyl tert-butyl ether (200 mL) were first added for precipitation, and the supernatant was discarded; n-hexane and methyl tert-butyl ether were added again for precipitation; and such operations were repeated three times, and the suction filtering was then carried out to obtain a solid product, and the obtained solid product was collected, and dried in a vacuum oven, thus obtaining the product (3.7 g being extra-quota product). 60-8

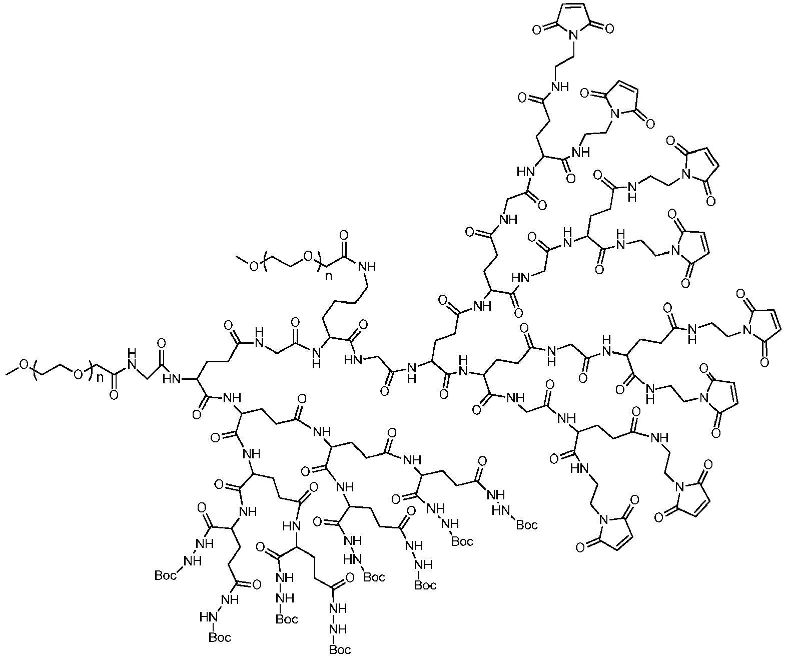

Product 60-2 (3.35 g, 0.1427 mmol), HBTU (0.65 g, 1.7124 mmol) and HOBT (0.23 g, 1.7124 mmol) were weighed and added in a flask with DAE-MI (0.18 g, 1.2558 mmol, purchased from Shanghai Leiqi Bio), and then dissolved with an appropriate amount of DMF, and the mixed solution was placed at -5 °C; then, DIEA (0.85 mL, 5.1372 mmol) was slowly added dropwise; after addition and reaction for 30 min, the reaction solution was taken out, and the obtained solution was stirred overnight at room temperature to react. At the end of the reaction, n-hexane (25 mL) and methyl tert-butyl ether (200 mL) were first added for precipitation, and the supernatant was discarded; n-hexane and methyl tert-butyl ether were added again for precipitation, and such operations were repeated three times; and the filtering was then carried out to obtain a solid product, and the obtained solid product was dissolved with a mixed solvent (dichloromethane and methanol), the operations of dry sample loading, column chromatography, and elution with a mixed solution (3-5% methanol/dichloromethane) were carried out; the elution product was collected, concentrated and dried in a vacuum oven. The product (1.3 g, 37.25%) was obtained. 60-28

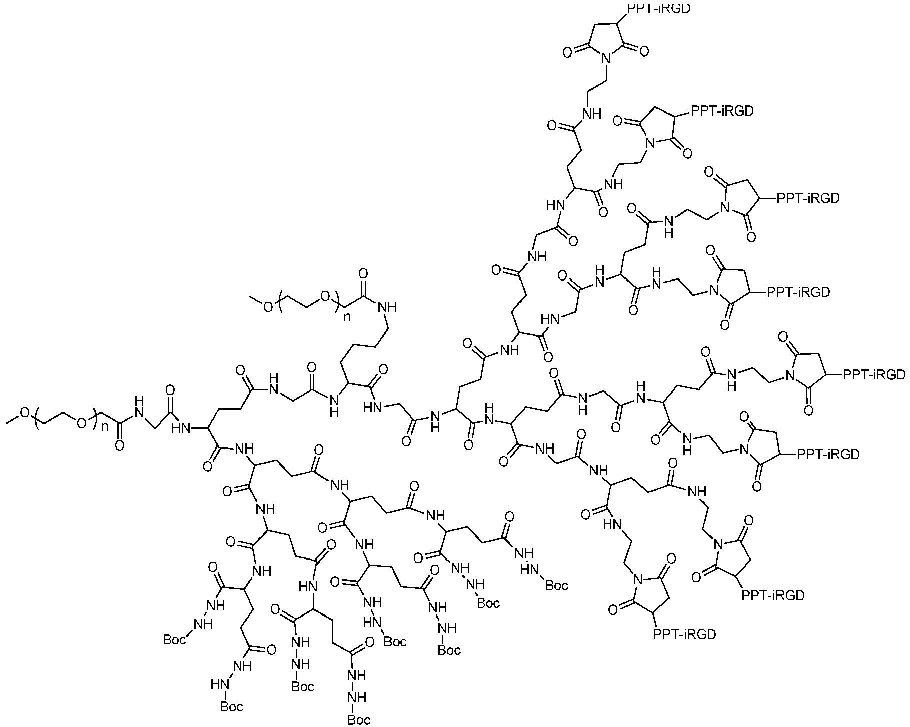

Product 60-8 (0.21 g, 0.0082 mmol) was added in a dry 250 mL round-bottomed flask and then dissolved completely with PPT-iRGD (0.12 g, 0.0721 mmol) and DMSO (20 mL); the obtained solution was stirred at 40 °C to react for 3 days. After the reaction was completed, ethyl acetate (25 mL) and methyl tert-butyl ether (200 mL) were first added for precipitation, and the supernatant was discarded; n-hexane and methyl tert-butyl ether were added again for precipitation, and such operations were repeated three times, and the resulting solid was collected, concentrated, and dried in a vacuum oven, thus obtaining the product (0.32 g being extra-quota product). 60-40

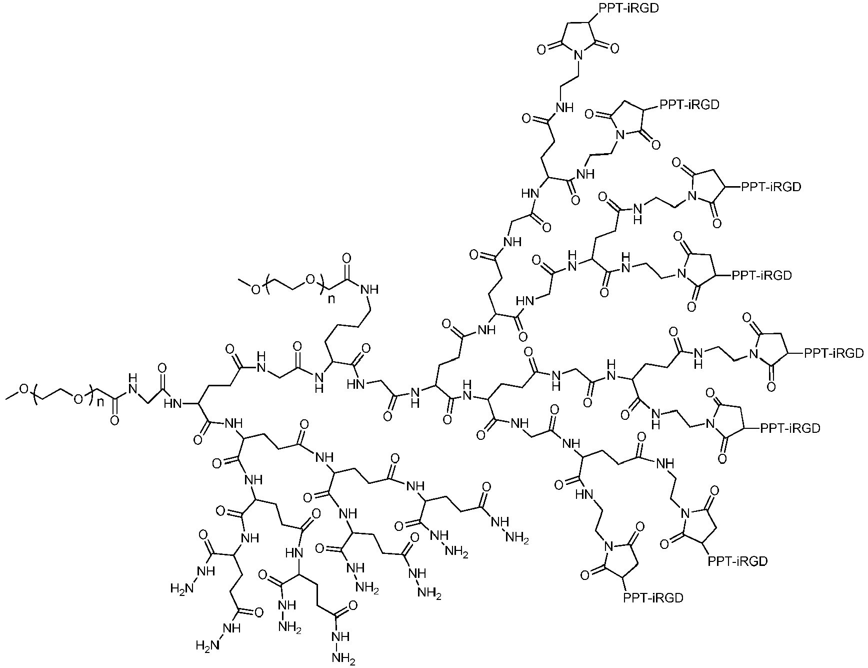

Product 60-28 (0.13 g, 0.0034 mmol) was added in a 500 mL flask, dichloromethane (20 mL) and TFA (3 mL) were added in sequence, and the reaction solution was stirred overnight at room temperature to react. At the end of the reaction, the reaction solution was concentrated at reduced pressure; methyl tert-butyl ether (200 mL) was then added and the powdery product was precipitated; suction filtering was then carried out, and the resulting product was then dried, thus obtaining 0.42 g of the product being extra-quota product. 60-43

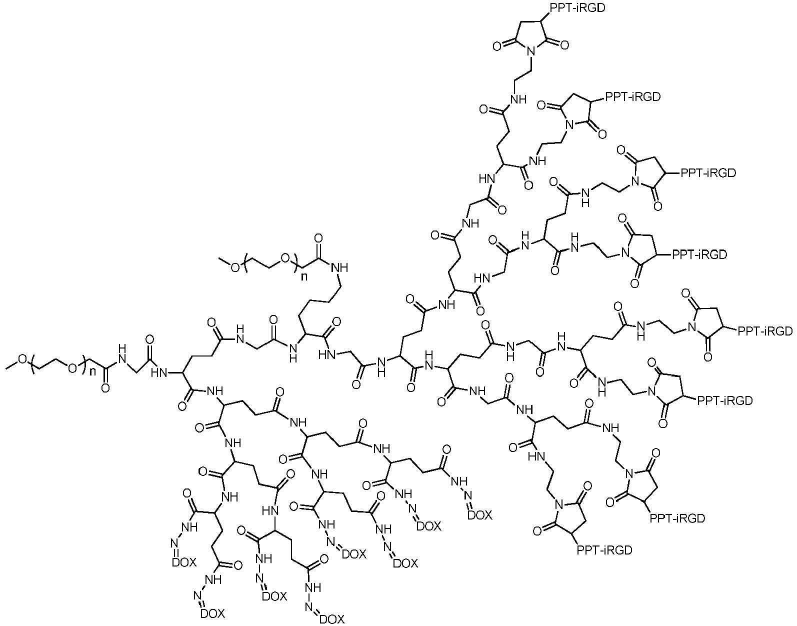

Product 60-40 (0.13 g, 0.0034 mmol) was added in a dry 500 mL round-bottomed flask and then dissolved with anhydrous methanol; acetic acid (0.12 mL, 2.124 mmol) and DOX (0.02 g, 0.034 mmol) were then added in the flask, and the reaction solution was stirred at room temperature overnight to react. After the reaction was completed, n-hexane (25 mL) and methyl tert-butyl ether (200 mL) were first added for precipitation, and the supernatant was discarded; n-hexane and methyl tert-butyl ether were added again for precipitation, and such operations were repeated three times, and the filtering was then carried out to obtain a solid product, and the obtained solid product was dissolved with dichloromethane and methanol, the operations of dry sample loading, column chromatography and elution with 5-12%methanol /dichloromethane were carried out; the elution product was then collected, concentrated and dried, thus obtaining the product (0.07 g, 49.29%). ¹H-NMR (600 MHz, DMSO-*d*₆) δ 8.81 - 8.10 (m, 41H), 8.06 - 7.66 (m, 33H), 7.62 - 6.81 (m, 136H), 4.72 - 4.05 (m, 106H), 4.01 - 3.61 (m, 133H), 3.59 - 3.43 (m, 1260H), 3.26 - 3.04 (m, 68H), 2.95 - 2.70 (m, 54H), 2.67 - 2.53 (m, 367H), 2.44 - 2.36 (m, 13H), 2.19 - 1.83 (m, 77H), 1.64 - 1.43 (m, 78H), 1.41 - 1.18 (m, 60H), 0.93 - 0.78 (m, 80H).

### Chemical synthesis route of 48-124

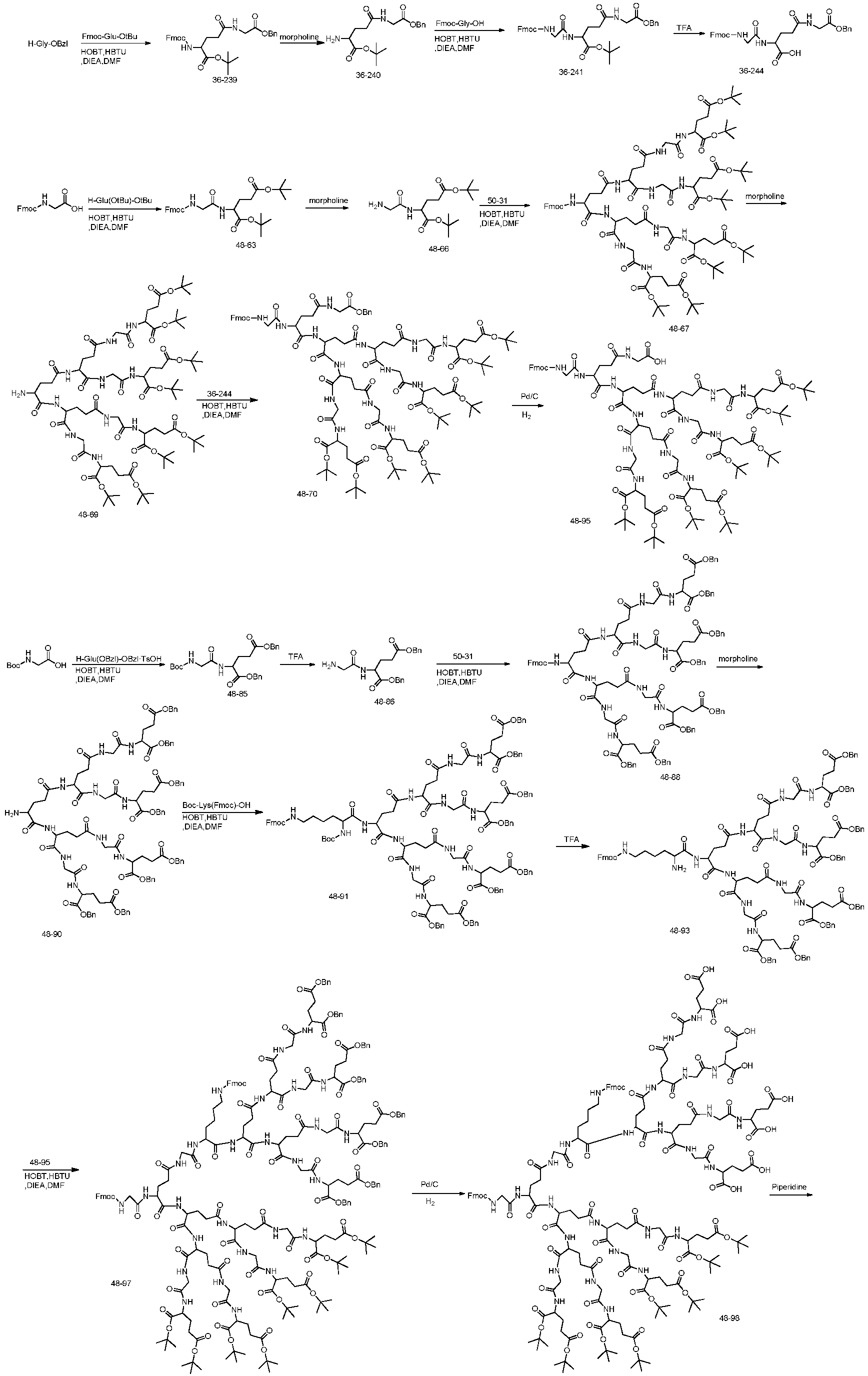

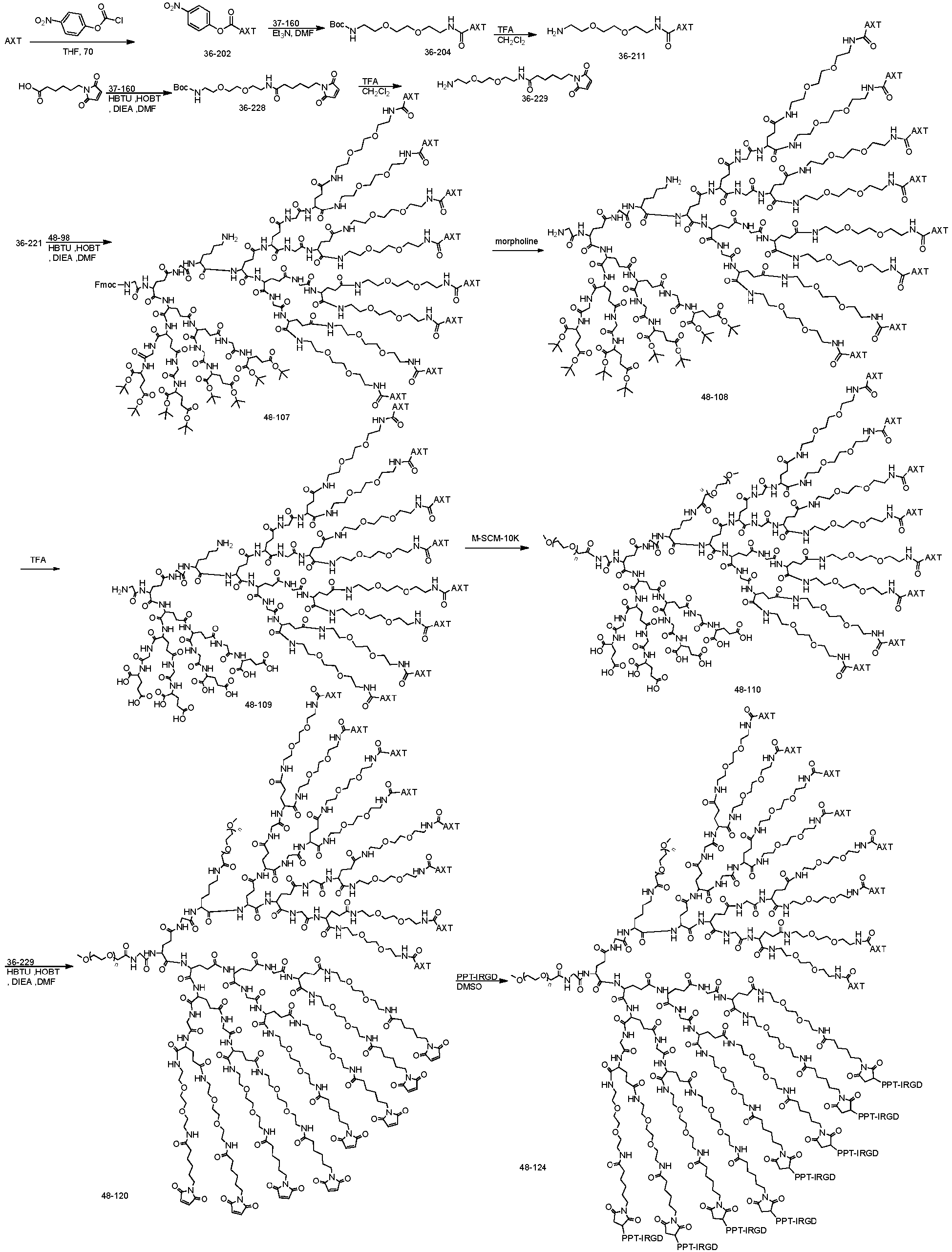

36-202

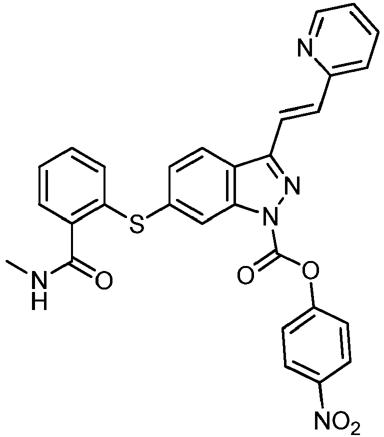

Axitinib (AXT, 6 g, 15.5251 mmol) and 4-nitrophenyl chloroformate (9.38 g, 46.5754 mmol) were added in a 500 mL double-neck flask, 300 mL of THF was added to obtain a homogeneous phase, and the reaction solution reacted in an oil bath at 70 °C, and the solvent was refluxed with a condenser tube, and then the reaction solution was stirred overnight to react. At the end of the reaction, n-hexane (100 mL × 3) was added for precipitation, and the lower oily solid was dissolved with a small amount of dichloromethane; methyl tert-butyl ether was added, and a powdery solid was then precipitated; the suction filtering was carried out and the filter cake was washed with methyl tert-butyl ether (50 mL × 3), and then dissolved with a mixed solution (20% methanol: 80% dichloromethane) (200 mL); silica gel powder (60 mL) was added to the obtained solution, and the solution was then evaporated to dryness to obtain a powdery solid; the operations of dry sample loading, column chromatography, and elution with a mixed solution (0%-20% methanol/dichloromethane) were carried out; the elution product was then collected, concentrated, and evaporated to dryness. 4.7 g of Product 36-202 was obtained with a yield of 55%. 36-204

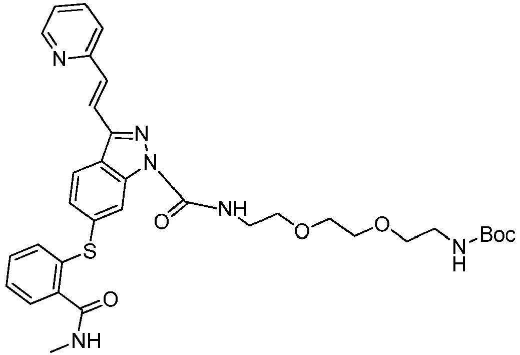

Product 36-202 (4.7 g, 8.5251 mmol) and Product 37-160 (2.1 g, 8.5251 mmol) were added in a 500 mL double-neck flask and then dissolved with 30 mL DMF, and the mixed solution was placed at room temperature; triethylamine (3.6 mL, 25.5634 mmol) was added, and the obtained solution was stirred at room temperature overnight to react. At the end of the reaction, the reaction solution was transferred to a 1 L separatory funnel and extracted with deionized water (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); and the obtained organic phases were combined, washed with a saturated sodium chloride solution (100 ml × 2), and evaporated to dryness, thus obtaining 7 g of Product 36-204. 36-211

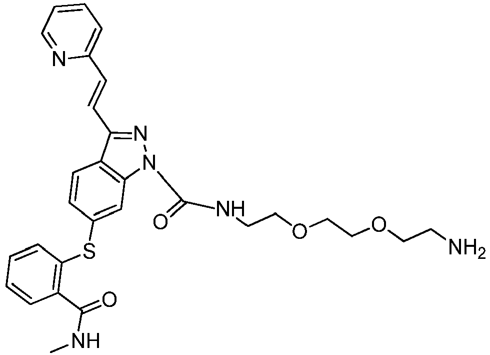

Product 36-204 (7 g, 8.5211 mmol) was added in a 500 mL flask, dichloromethane (20 mL) and TFA (6 mL, 85.2110 mmol) were added in sequence, and the reaction solution was stirred overnight at room temperature to react. At the end of the reaction, the reaction solution was concentrated to 10 mL; methyl tert-butyl ether (200 mL) was then added and the powdery product was precipitated; suction filtering was then carried out, and the filter cake was washed with methyl tert-butyl ether (50 mL × 3), and dissolved with a mixed solution (20% methanol: 80% dichloromethane) (200 mL); silica gel powder (60 mL) was added to the obtained solution, and the solution was then evaporated to dryness to obtain a powdery solid; the operations of dry sample loading, column chromatography, and elution with a mixed solution (1%-8% methanol/dichloromethane) were carried out; the elution product was then collected, concentrated, and evaporated to dryness. 4.5 g of Product 36-211 was obtained with a yield of 94.3%. 36-228

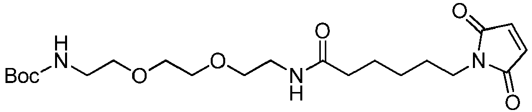

6-maleimidohexanoic acid (3 g, 14.2032 mmol, purchased from Energy), Product 37-160 (3.9 g, 15.6235 mmol), HBTU (8 g, 21.3048 mmol) and HOBT (2.8 g, 21.3048 mmol) were added to a 250 mL flask and dissolved with 30 mL of DMF, and the obtained solution was stirred at -5 °C for 30 min to react; then, DIEA (10.5 mL, 63.9144 mmol) was slowly added dropwise, and at the end of the addition, the reaction continued for 1 h, and the reaction solution was stirred at room temperature overnight to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); and the obtained organic phases were combined, washed with a saturated sodium chloride solution (100 ml ×2), and evaporated to dryness, thus obtaining 10.6 g of Product 36-228. 36-229

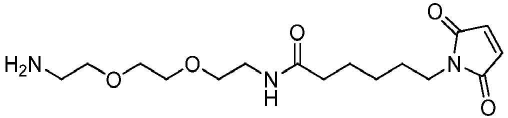

Product 36-228 (10.6 g, 14.2032 mmol) was added in a 500 mL flask, dichloromethane (20 mL) and TFA (10.5 mL, 142.032 mmol) were added in sequence, and the reaction solution was stirred overnight at room temperature to react. At the end of the reaction, the reaction solution was concentrated to 10 mL; methyl tert-butyl ether (200 mL) was then added and the powdery product was precipitated; suction filtering was then carried out, and the filter cake was washed with methyl tert-butyl ether (50 mL × 3), and dissolved with a mixed solution (20% methanol: 80% dichloromethane) (200 mL); silica gel powder (60 mL) was added to the obtained solution, and the solution was then evaporated to dryness to obtain a powdery solid; the operations of dry sample loading, column chromatography, and elution with a mixed solution (4%-40% methanol/dichloromethane) were carried out; the elution product was then collected, concentrated, and evaporated to dryness. 1.5 g of Product 36-229 was obtained with a yield of 30.9%. 36-239

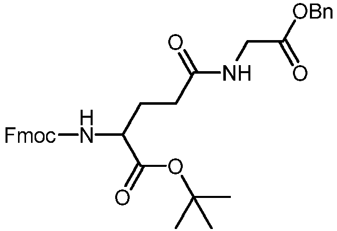

Fmoc-Glu-OtBu (4.2 g, 9.9182 mmol), H-Gly-OBzl HCl (2 g, 9.9182 mmol, purchased from Innochem), HBTU (5.6 g, 14.8773 mmol) and HOBT (2 g, 14.8773 mmol) were added in a 250 mL flask and dissolved with 30 mL of DMF, and the obtained solution was stirred at -5 °C for 30 min to react; then, DIEA (9 mL, 54.5499 mmol) was slowly added dropwise, and at the end of the addition, the reaction continued for 1 h, and the reaction solution was stirred at room temperature overnight to react. At the end of the reaction, the reaction solution was taken out; deionized water (100 mL) was added to the reaction solution, extraction with ethyl acetate (100 mL × 3) were carried out several times, and the obtained organic phases were combined, washed twice with a saturated sodium chloride solution (100 mL), and then concentrated and evaporated to dryness, thus obtaining 7.8 g of Product 36-239. 36-240

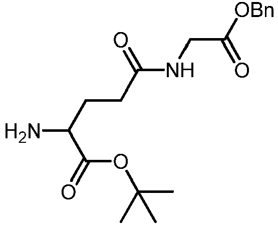

Product 36-239 (7.8 g, 9.9182 mmol) was added in a 250 mL flask and then dissolved with DMF, morpholine (8.6 mL, 99.182 mmol) was then added, and the reaction solution was stirred overnight at room temperature to react. At the end of the reaction, the reaction solution was transferred to a 1 L separatory funnel and extracted with saturated sodium bicarbonate (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, then washed with a saturated sodium chloride solution (100 ml × 2), and evaporated to dryness, thus obtaining 5.4 g of Product 36-240. 36-241

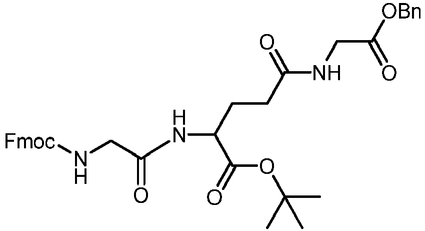

Product 36-240 (5.4 g, 9.9182 mmol), Fmoc-Gly-OH (2.6 g, 8.9264 mmol), HBTU (5.6 g, 14.8773 mmol) and HOBT (2 g, 14.8773 mmol) were added to a 250 mL flask and dissolved with 30 mL of DMF, and the obtained solution was stirred at -5 °C for 30 min to react; then, DIEA (7.3 mL, 44.6319 mmol) was slowly added dropwise, and at the end of the addition, the reaction continued for 1 h, and the reaction solution was stirred at room temperature overnight to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); and the obtained organic phases were combined, washed with a saturated sodium chloride solution (100 ml × 2), and evaporated to dryness, thus obtaining 4.86 g of Product 36-241 with a yield of 78.3% . 36-244

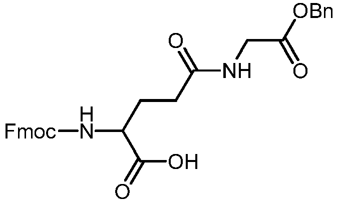

Product 36-241 (4.86 g, 9.9182 mmol) was added in a 500 mL flask, dichloromethane (20 mL) and TFA (7.36 mL, 99.182 mmol) were added in sequence, and the reaction solution was stirred overnight at room temperature to react. At the end of the reaction, the reaction solution was concentrated to 10 mL; methyl tert-butyl ether (200 mL) was then added and the powdery product was precipitated; suction filtering was then carried out, and then dried, thus obtaining 3.45 g of Product 36-244 with a yield of 86.6%. 48-63

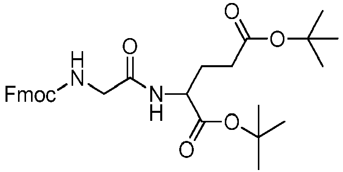

Fmoc-Gly-OH (10.74 g, 36.32566 mmol), L-glutamic acid di-tert-butyl ester hydrochloride (10.8 g, 36.3256 mmol), HBTU (15.2 g, 39.9580 mmol) and HOBT (5.4 g, 39.9580 mmol) were dissolved with an appropriate amount of DMF, and the obtained solution was placed at -5 °C; then, DIEA (10.27 mL, 62.1522 mmol) was slowly added dropwise; after addition and reaction for 30 min, the reaction solution was taken out, and the obtained solution was stirred overnight at room temperature to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, washed with a saturated sodium chloride solution (100 ml × 2), evaporated to dryness, and then dissolved with dichloromethane (200 mL); silica gel powder (60 mL) was added to the obtained solution, and the solution was then evaporated to dryness to obtain a powdery solid; the operations of dry sample loading, column chromatography, and elution with a mixed solution of 20%-40% ethyl acetate in petroleum ether were carried out, and the elution product was collected, concentrated and evaporated to dryness, thus obtaining 19.4 g of Product 48-63 with a yield of 99.5%. 48-66

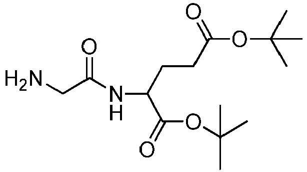

Product 48-63 (9.5 g, 36.0173 mmol) in a flask was dissolved completely with DMF by ultrasonic vibration; morpholine (31.4 mL, 360.1730 mmol) was added to the obtained solution, and the reaction solution was stirred at room temperature for 2 h to react. At the end of the reaction, the reaction solution was transferred to a 1 L separatory funnel and extracted with saturated sodium bicarbonate (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, washed with a saturated sodium chloride solution (100 ml × 2), and evaporated to dryness, thus obtaining 6.8 g of Product 48-66. 48-67

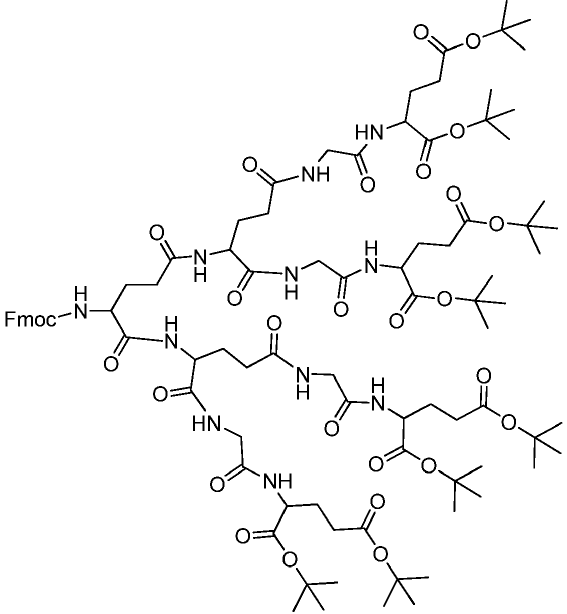

Product 48-66 (5.5 g, 17.5270 mmol), Product 50-31 (2.2 mmol, 3.5054 mmol), HBTU (7.9 g, 21.0324 mmol) and HOBT (2.8 g, 21.0324 mmol) were weighed and added in a 250 mL flask and then dissolved in DMF solution (80 mL), and the ultrasonic treatment was carried out to completely dissolve the reactants; the obtained solution was stirred for 30 min at -5 °C. DIEA (12.8 mL, 77.1188 mmol) was slowly added dropwise, and the obtained solution reacted at a low temperature to the end. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, washed with a saturated sodium chloride solution (100 ml × 2), and evaporated to dryness, thus obtaining 11 g of Product 48-67. 48-69

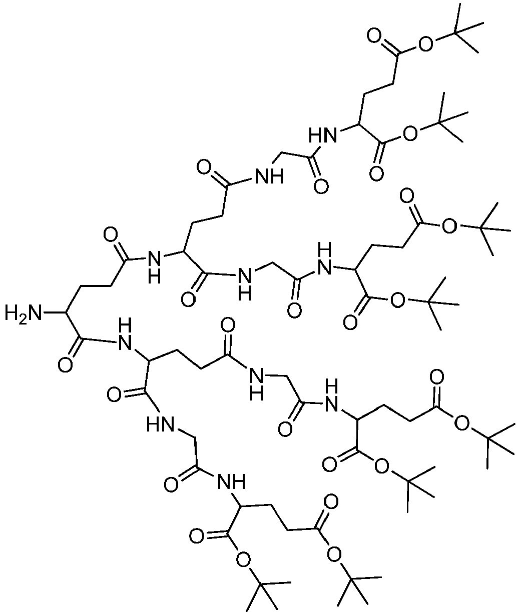

Product 48-67 (11 g, 6.0402 mmol) was dissolved with DMF (40 mL), then morpholine (5.3 mL, 60.4020 mmol) was added, and the mixed solution was stirred at room temperature to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, washed with a saturated sodium chloride solution (100 ml × 2), evaporated to dryness, and then dissolved with dichloromethane (200 mL); silica gel powder (60 mL) was added to the obtained solution, and the solution was then evaporated to dryness to obtain a powdery solid; the operations of dry sample loading, column chromatography, and elution with a mixed solution (0.5% ammonia water/4%-7% methanol/dichloromethane) were carried out; the elution product was then collected, concentrated, and evaporated to dryness. 3.9 g of Product 48-69 was obtained with a yield of 70%. 48-70

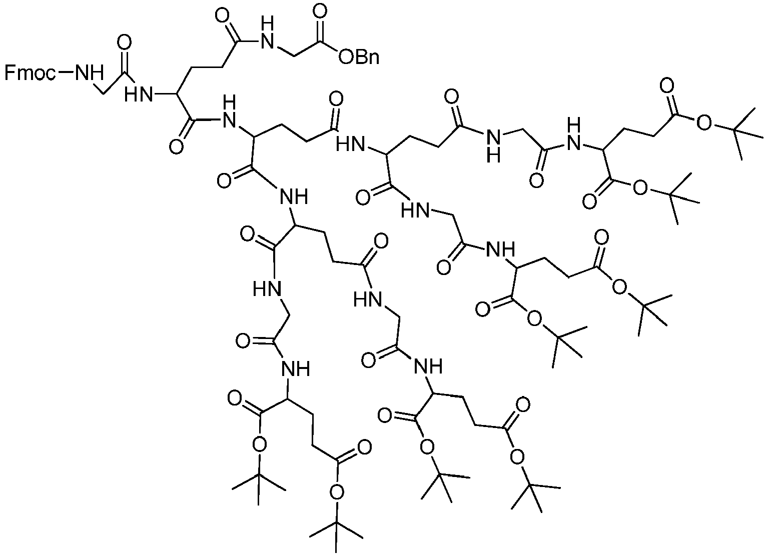

Product 36-244 (1.31 g, 2.4392 mmol), Product 48-69 (3.9 g, 2.4392 mmol), HBTU (1.4 g, 3.6588 mmol) and HOBT (0.5 g, 3.6588 mmol) were added to a 250 mL flask and dissolved with 30 mL of DMF, and the obtained solution was stirred at -5 °C for 30 min to react; then, DIEA (2.2 mL, 13.4157 mmol) was slowly added dropwise, and at the end of the addition, the reaction continued for 1 h, and the reaction solution was stirred at room temperature overnight to react. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, washed with a saturated sodium chloride solution (100 ml × 2), and evaporated to dryness, thus obtaining 6 g of Product 48-70. 48-85

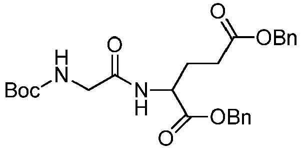

Boc-Gly-OH (3 g, 17.1252 mmol), H-Glu(OBzl)-OBzl·TsOH (8.5 g, 17.1252 mmol), HOBT (3.4 g, 25.1878 mmol) and HBTU (9.7 g, 25.6878 mmol) were weighed and added in a 250 mL flask and then dissolved in DMF solution (80 mL), and the ultrasonic treatment was carried out to completely dissolve the reactants; the obtained solution was stirred for 30 min at -5 °C. DIEA (12.7 mL, 77.0634 mmol) was slowly added dropwise, and the obtained solution reacted at a low temperature to the end. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, washed with a saturated sodium chloride solution (100 ml × 2), evaporated to dryness, and then dissolved with dichloromethane (200 mL); silica gel powder (60 mL) was added to the obtained solution, and the solution was then evaporated to dryness to obtain a powdery solid; the operations of dry sample loading, column chromatography, and elution with a mixed solution of 30%-40% ethyl acetate in petroleum ether were carried out, and the elution product was collected, concentrated and evaporated to dryness, thus obtaining 8.5 g of Product 48-85. 48-86

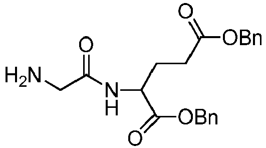

Product 48-85 (8.5 mmol, 17.1296 mmol) was weighed and dissolved by ultrasonic with dichloromethane (5 mL) and TFA (12.7 mL, 171.2960 mmol), a ground glass stopper was used, and the obtained solution was stirred to react at room temperature. At the end of the reaction, the reaction solution was concentrated to 10 mL, and methyl tert-butyl ether (200 mL) was added to obtain a viscous solid; and then dissolved with 2-3 mL of dichloromethane, precipitated with n-hexane and methyl tert-butyl ether, and such operations were repeated twice; the obtained solid was filtered, and then dried, thus obtaining 8.5 g of Product 48-86. 48-88

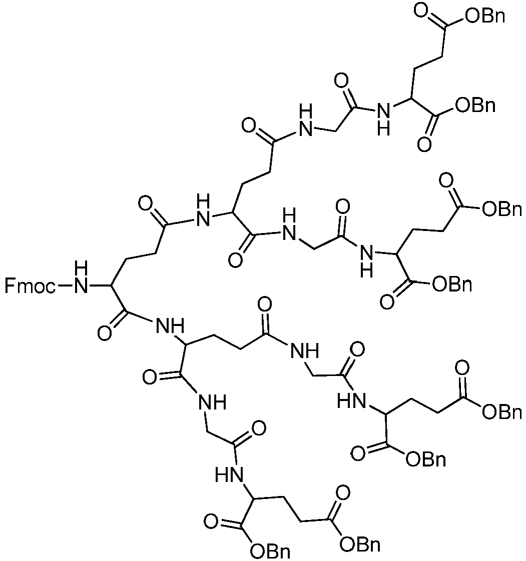

Product 48-86 (5.95 g, 15.4876 mmol), Product 50-31 (2.43, 3.8719 mmol), HOBT (3.15 g, 23.2314 mmol) and HBTU (8.8 g, 23.2314 mmol) were weighed and added in a 250 mL flask and then dissolved in DMF solution (80 mL), and the ultrasonic treatment was carried out to completely dissolve the reactants; the obtained solution was stirred for 30 min at -5 °C. DIEA (16.7 mL, 100.6694 mmol) was slowly added dropwise, and the obtained solution reacted at a low temperature to the end. At the end of the reaction, the reaction solution was transferred to a 1 L separatory funnel and extracted with saturated sodium bicarbonate (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, washed with a saturated sodium chloride solution (100 ml × 2), evaporated to dryness, and then dissolved with dichloromethane (200 mL); silica gel powder (50 mL) was added to the obtained solution, and the solution was then evaporated to dryness to obtain a powdery solid; the operations of dry sample loading, column chromatography, and elution with a mixed solution (4.5%-6% methanol/dichloromethane) were carried out; the elution product was then collected, concentrated, and evaporated to dryness. 4.4 g of Product 48-88 was obtained with a yield of 54%. 48-90

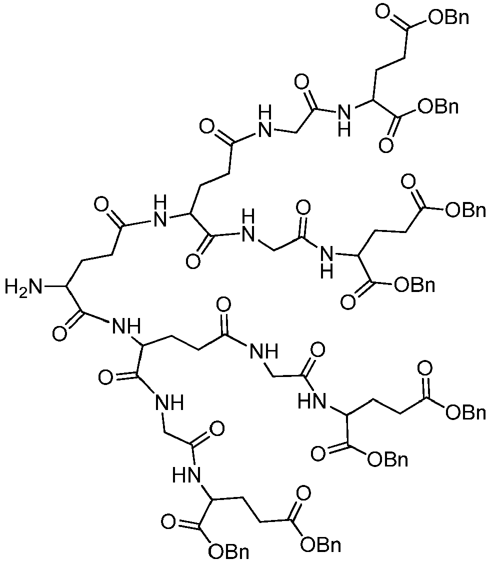

Product 48-88 (2.7 g, 1.2898 mmol) was dissolved in DMF (20 mL) and then morpholine (1.13 mL, 12.8986 mmol) was added, and the mixed solution was stirred at room temperature to react. At the end of the reaction, methyl tert-butyl ether (150 mL) and n-hexane (100 mL) were added to precipitate the reaction solution, and a solid product was obtained; the obtained solid product was filtered by suction and dried, thus obtaining 3 g of Product 48-90. 48-91

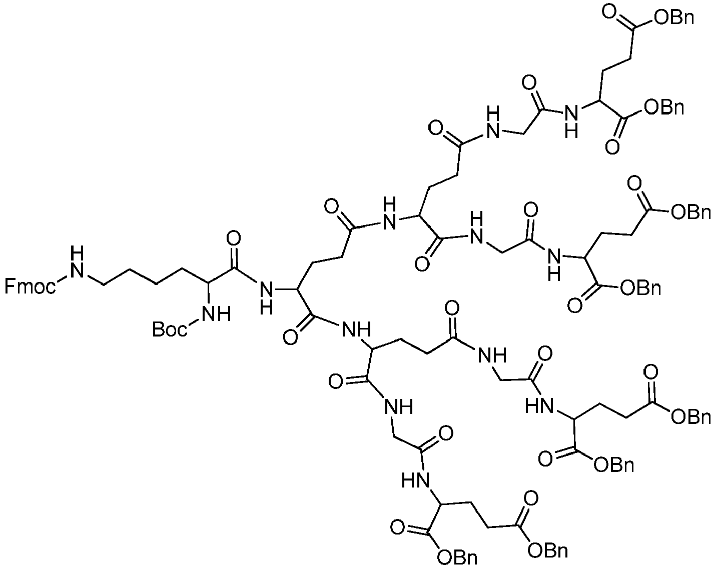

Product 48-90 (2.4 g, 1.2827 mmol), Boc-Lys(Fmoc)-OH (0.6 g, 1.2827 mmol), HOBT (0.26 g, 1.9241 mmol) and HBTU (0.73 g, 1.9241 mmol) were weighed and added in a 250 mL flask and then dissolved in DMF solution (40 mL), and the ultrasonic treatment was carried out to completely dissolve the reactants; the obtained solution was stirred for 30 min at -5 °C. DIEA (1 mL, 5.7721 mmol) was slowly added dropwise, and the obtained solution reacted at a low temperature to the end. At the end of the reaction, n-hexane (150 mL) and methyl tert-butyl ether (30 mL) were first added for precipitation, the supernatant was discarded; n-hexane (150 mL) and methyl tert-butyl ether (50 mL) were added again to the lower oily solution, and such operations were repeated three times to obtain an oily product; methyl tert-butyl ether (200 mL) was added, and a powdery solid was then precipitated; the suction filtering was carried out and the filter cake was washed with methyl tert-butyl ether (50 mL × 3), and then dissolved with a mixed solution (20% methanol: 80% dichloromethane) (200 mL); silica gel powder (60 mL) was added to the obtained solution, and the solution was then evaporated to dryness to obtain a powdery solid; the operations of dry sample loading, column chromatography, and elution with a mixed solution (4%-5% methanol/dichloromethane) were carried out; the elution product was then collected, concentrated, and evaporated to dryness. 2.3 g of Product 48-91 was obtained with a yield of 79.5%. 48-93

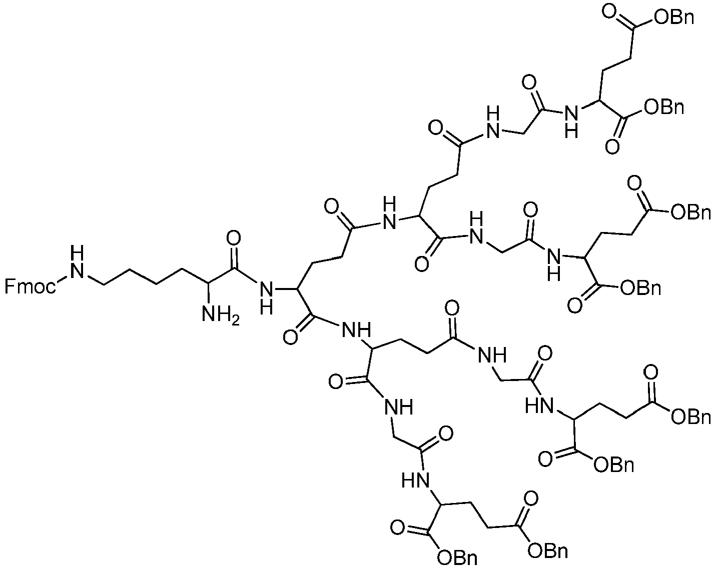

Product 48-91 (2.3g, 0.9907mmol) was weighed and dissolved completely by ultrasonic with dichloromethane (5 mL) and TFA (0.75 mL, 9.9072 mmol), a ground glass stopper was used, and the obtained solution was stirred to react at room temperature. At the end of the reaction, methyl tert-butyl ether (150 mL) and n-hexane (100 mL) were added to precipitate the reaction solution, and a solid product was obtained; the obtained solid product was filtered by suction and dried, thus obtaining 2.4 g of Product 48-93. 48-95

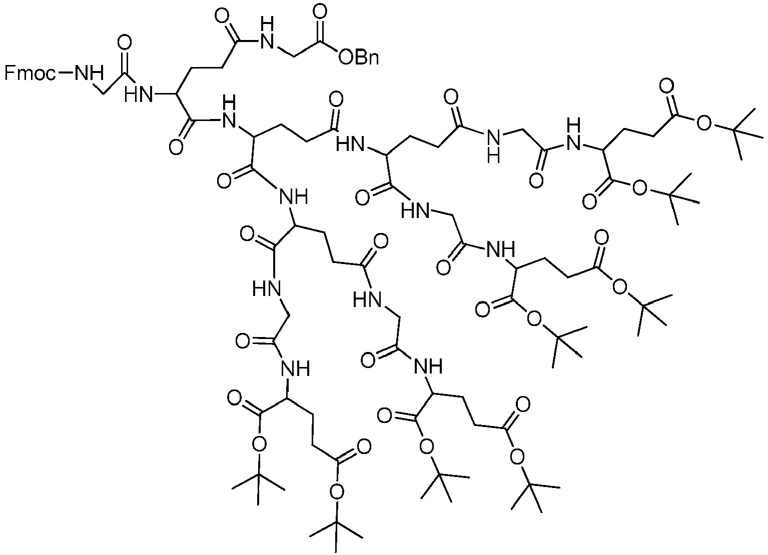

Product 48-70 (1.2 g, 0.5570 mmol) was added in a hydrogenation reactor and then 10% Pd/C (0.07 g) was added, and the mixture was dissolved with DMF (20 mL); hydrogen was introduced to a pressure of 1.4 MPa in the reactor, and the reaction solution was stirred overnight at room temperature to react. At the end of the reaction, the reaction solution was filtered with diatomaceous earth; the filter cake was washed with DMF (15 mL × 3), and the elution solution was added in a 250 mL round-bottomed flask, as the raw material for the next step. 48-97

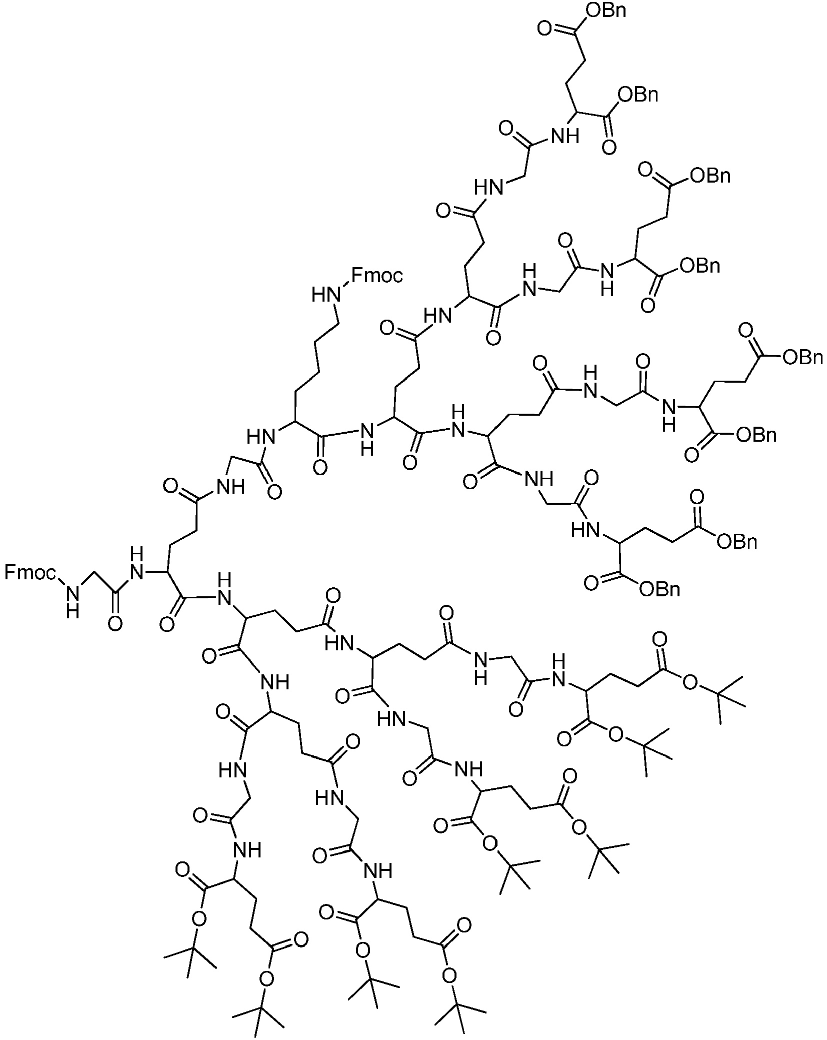

Product 48-95 (1.15 g, 0.5570 mmol), Product 48-93 (1.25 g, 0.5570 mmol), HOBT (0.12 g, 0.8355 mmol) and HBTU (0.32 g, 0.8355 mmol) were weighed and added in a 250 mL flask and then dissolved in DMF solution (80 mL), and the ultrasonic treatment was carried out to completely dissolve the reactants; the obtained solution was stirred for 30 min at -5 °C. DIEA (0.5 mL, 3.0635 mmol) was slowly added dropwise, and the obtained solution reacted at a low temperature to the end. At the end of the reaction, the reaction solution was transferred to a 1 L separatory funnel and extracted with a saturated saline solution (200 mL) and ethyl acetate (200 mL) to obtain an organic phase; the aqueous phase was washed with ethyl acetate (200 mL × 2), and the obtained organic phases were combined, washed with a saturated saline solution (200 mL × 2); the obtained organic phases were combined again, washed with a saturated sodium chloride solution (100 ml × 2), evaporated to dryness, and then dissolved with dichloromethane (200 mL); silica gel powder (30 mL) was added to the obtained solution, and the solution was then evaporated to dryness to obtain a powdery solid; the operations of dry sample loading, column chromatography, and elution with a mixed solution (3%-10% methanol/dichloromethane) were carried out; the elution product was then collected, concentrated, and evaporated to dryness. 1.3 g of Product 48-97 was obtained with a yield of 54.8%. 48-98

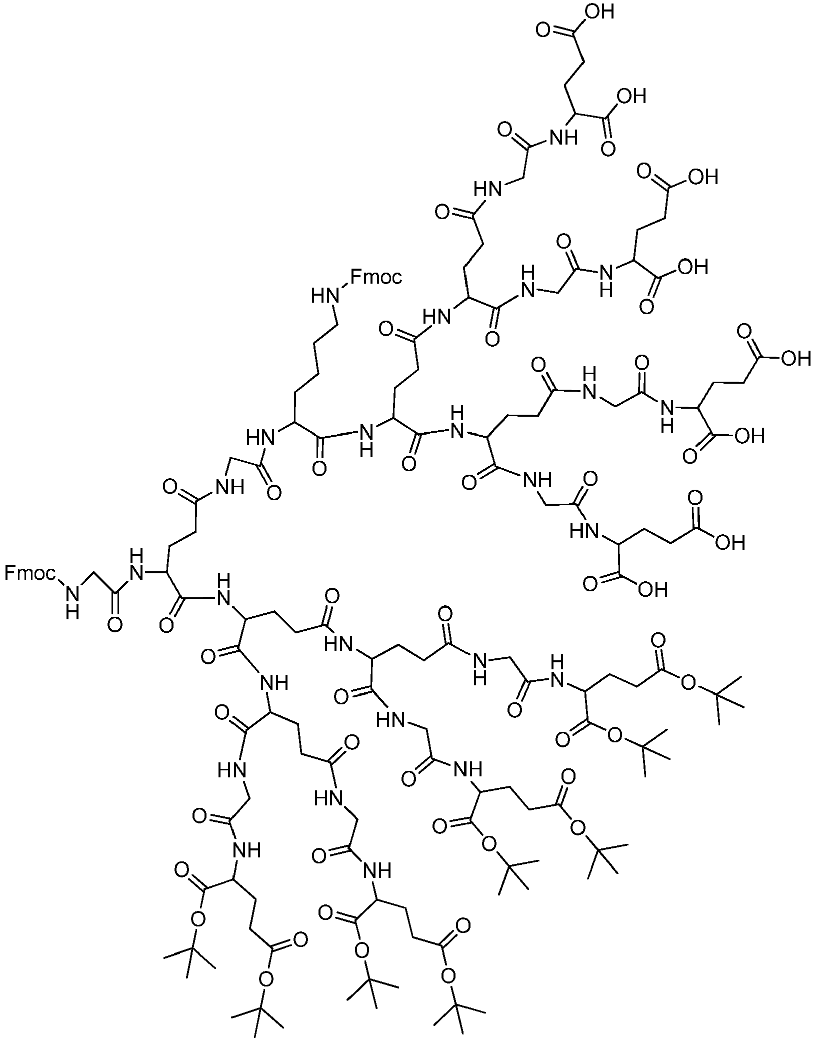

Product 48-97 (1 g, 0.2343 mmol) was added in a hydrogenation reactor and then 10% Pd/C (0.07 g) was added, and the mixture was dissolved with DMF (20 mL); hydrogen was introduced to a pressure of 1.4 MPa in the reactor, and the reaction solution was stirred overnight at room temperature to react. At the end of the reaction, the reaction solution was filtered with diatomaceous earth; the filter cake was washed with DMF (15 mL × 3), and the elution solution was added in a 250 mL round-bottomed flask, as the raw material for the next step. 48-107

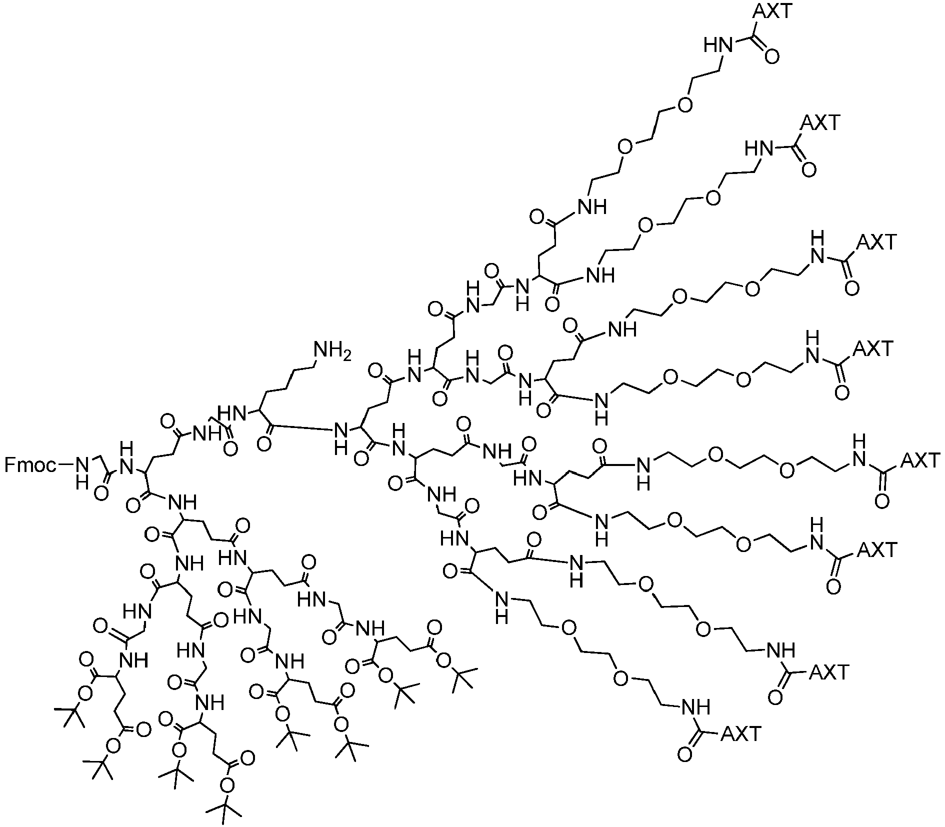

Product 48-98 (0.89 g, 0.2331 mmol), Product 36-211 (1.05 g, 1.8644 mmol), HBTU (1.06 g, 2.7966 mmol) and HOBT (0.38 g, 2.7966 mmol) were added to a 250 mL flask and dissolved with DMF 30 mL of DMF, and the obtained solution was stirred at -5 °C for 30 min to react; then, DIEA (1.38 mL, 8.3899 mmol) was slowly added dropwise, and at the end of the addition, the reaction continued for 1 h, and the reaction solution was stirred at room temperature overnight to react. At the end of the reaction, n-hexane (100 mL × 3) was added for precipitation, and the lower oily solid was dissolved with a small amount of dichloromethane; methyl tert-butyl ether was added, and a solid product was obtained; the obtained solid product was dried, thus obtaining 1.9 g of Product 48-107. 48-108

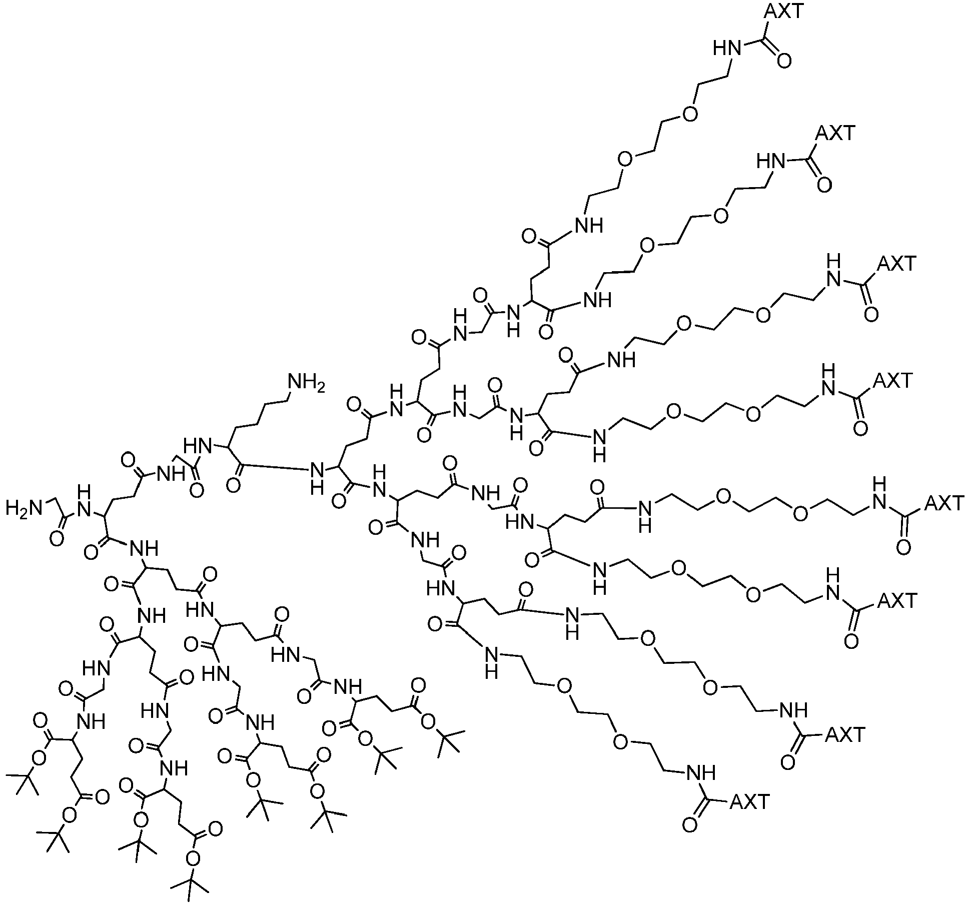

Product 48-107 (1.9 g, 0.2282 mmol) was added in a 250 mL flask and then dissolved with DMF, morpholine (0.39 mL, 4.5639 mmol) was then added, and the reaction solution was stirred overnight at room temperature to react. At the end of the reaction, n-hexane (100 mL × 3) was added for precipitation, and the lower oily solid was dissolved with a small amount of dichloromethane; methyl tert-butyl ether was added, and a solid product was obtained; the obtained solid product was dried, thus obtaining 1.5 g of Product 48-108 with a yield of 88%. 48-109

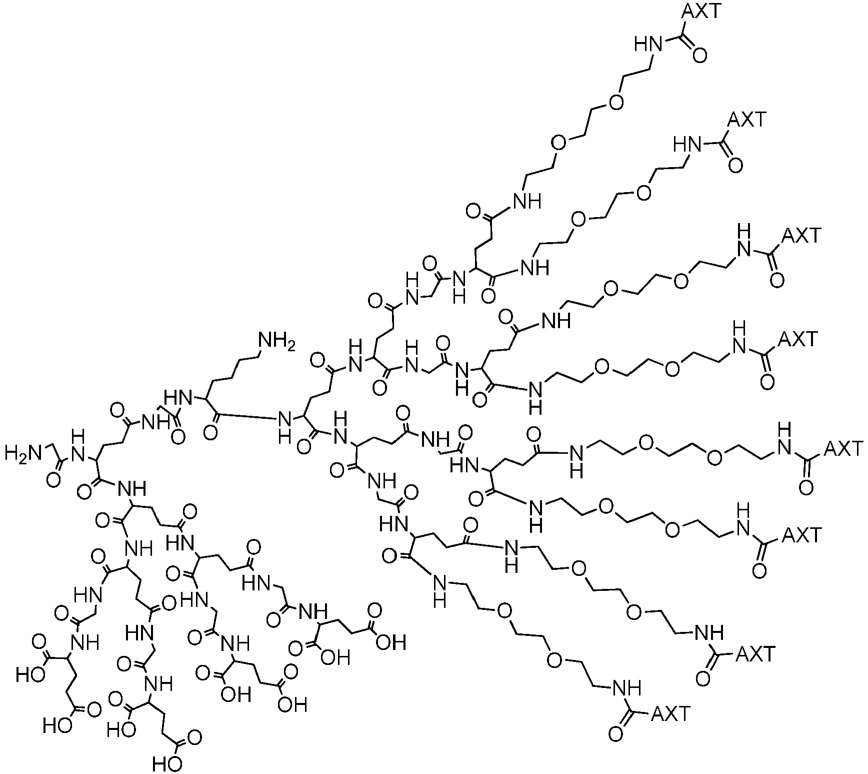

Product 48-108 (1.5 g, 0.2270 mmol) was added in a 500 mL flask, dichloromethane (20 mL) and TFA (1.35 mL, 18.1635 mmol) were added in sequence, and the reaction solution was stirred overnight at room temperature to react. At the end of the reaction, the reaction solution was concentrated to 10 mL; methyl tert-butyl ether (200 mL) was then added and the powdery product was precipitated; suction filtering was then carried out, and the resulting product was then dried, thus obtaining 1.5 g of Product 48-109. 48-110

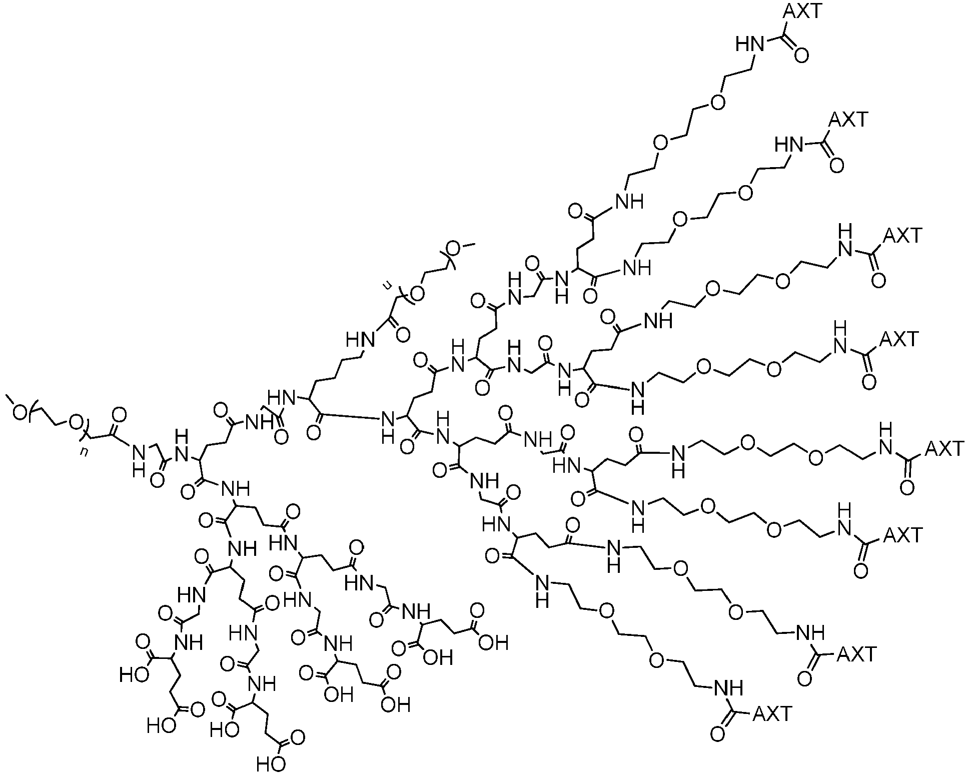

Product 48-109 (0.6 g, 0.08544 mmol) was added in a 250 mL flask and then dissolved with 20 mL of DMF, and the obtained solution was stirred at -5 °C for 30 min to react; then, DIEA (0.28 mL, 1.7088 mmol) was slowly added dropwise; M-SCM-10K (2 g) was added then, and the obtained solution was slowly stirred at room temperature to react for one week in the dark. At the end of the reaction, n-hexane (50 mL × 3) was first added; when the lower oily solution was only a small amount, methyl tert-butyl ether (20 mL) was added to precipitate the reaction solution, a solid product was obtained, and the filter cake was dissolved with methanol/dichloromethane (1: 5); 30 mL of silica gel powder was then added to the obtained solution, and the solution was then evaporated to dryness; the operations of dry sample loading, column chromatography, and elution with an eluent (5%-10% methanol: 95%-90% dichloromethane) were carried out; the elution solution was then collected, concentrated and evaporated to dryness. 0.42 g of Product 48-110 was obtained with a yield of 17.5%. 48-120

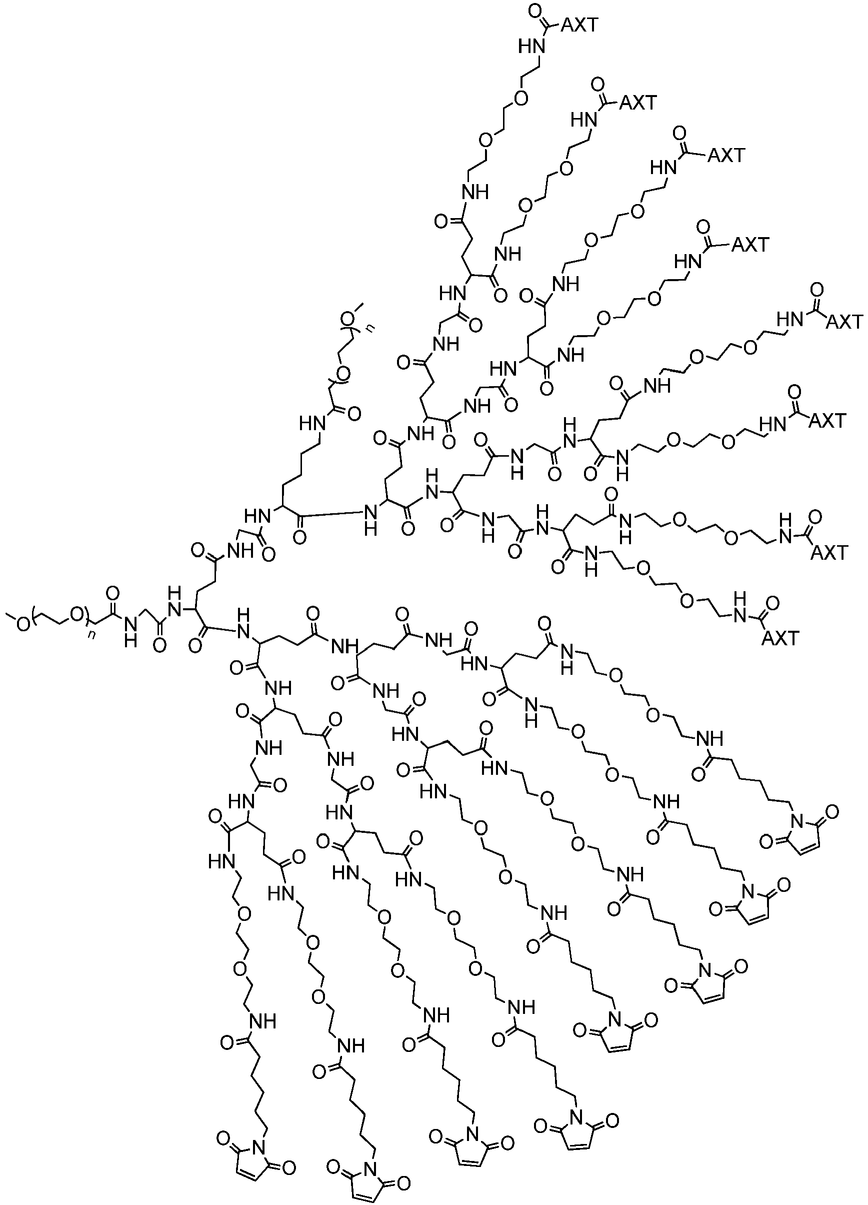

Product 48-110 (0.42 g, 0.01498 mmol), Product 36-229 (0.1 g, 0.2996 mmol), HBTU (0.7 g, 1.7970 mmol) and HOBT (0.2 g, 1.7970 mmol) were added to a 250 mL flask and dissolved with 30 mL of DMF, and the obtained solution was stirred at -5 °C for 30 min to react; then, DIEA (0.9 mL, 5.3920 mmol) was slowly added dropwise, and at the end of the addition, the reaction continued for 1 h, and the reaction solution was stirred at room temperature overnight to react. At the end of the reaction, n-hexane (50 mL × 3) was first added, when the lower oily solution was a small amount, methyl tert-butyl ether (20 mL) was added to precipitate the reaction solution, a solid product was obtained, and the filter cake was dissolved with methanol/dichloromethane (1: 5); 30 mL of silica gel powder was added to the obtained solution, and the solution was then evaporated to dryness; the operations of dry sample loading, column chromatography, and elution with an eluent (5%-10% methanol: 95%-90% dichloromethane) were carried out; the elution solution was then collected, concentrated and evaporated to dryness. 0.39 g of Product 48-120 was obtained with a yield of 85%. 48-124

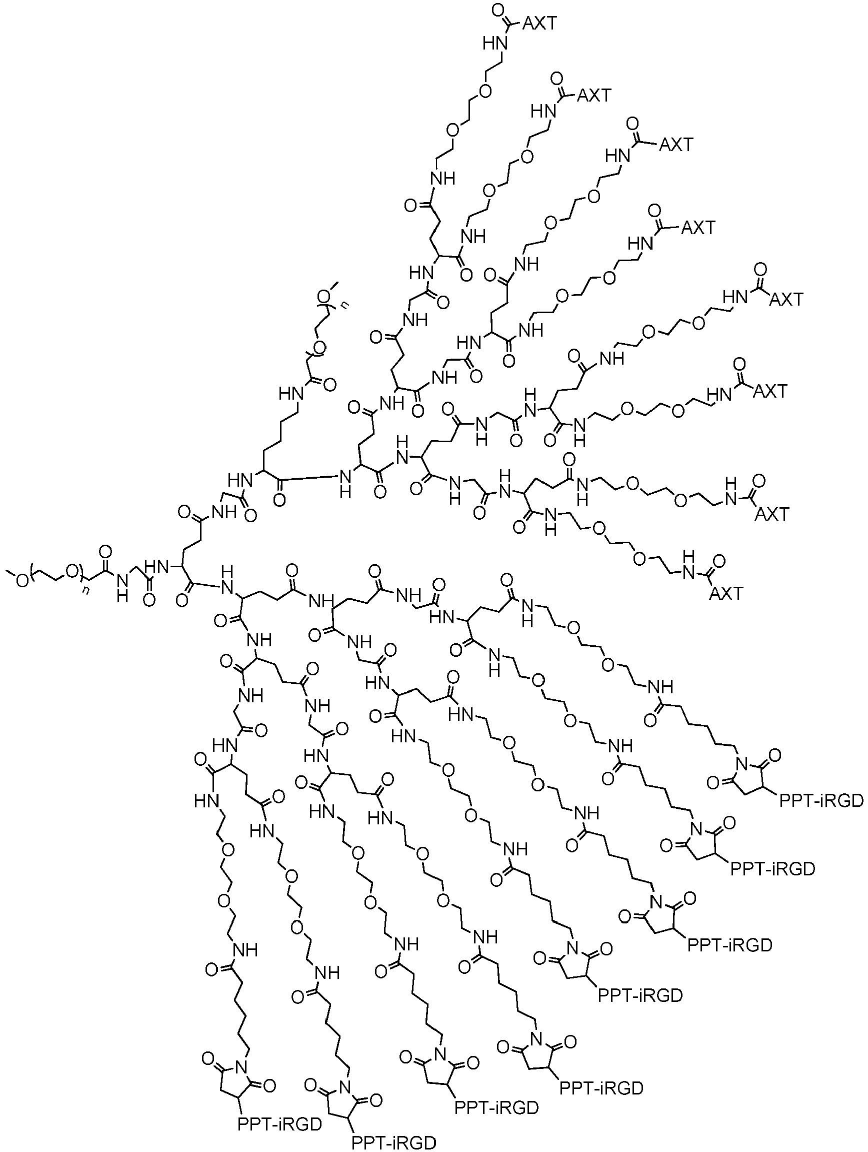

Product 48-120 (0.39 g, 0.01274 mmol) was added in a 500 mL flask and then dissolved with DMSO (30 mL), PPT-IRGD (0.2 g, 0.1274 mmol) was then added, and the obtained solution was slowly stirred at 40 °C to react for 2 days in the dark. At the end of the reaction, n-hexane (100 mL) and methyl tert-butyl ether (30 mL) were added, and the supernatant was discarded; n-hexane (100 mL) and methyl tert-butyl ether (30 mL) were added to the lower liquid, and such operations were repeated three times to obtain an oily; methyl tert-butyl ether (100 mL) was added, and a solid product was precipitated; suction filtering was then carried out, and the filter cake was washed with methyl tert-butyl ether (30 mL × 3), and dissolved with a solution of methanol (40 mL) and dichloromethane (160 mL); silica gel powder (15 g) was added to the obtained solution, and the solution was then evaporated to dryness to obtain a powdery solid; the operations of dry sample loading, column chromatography, and gradient elution with a mixed solution (5%-20% methanol/dichloromethane) were carried out; the elution product was then collected, concentrated, and evaporated to dryness. 0.18 g of Product 48-124 was obtained with a yield of 32.7%.

¹H-NMR (600 MHz, DMSO-*d*₆) δ 9.04 -9.00(m, 1H), 7.95 -7.85(m, 90H), 7.81-7.76 (m, 90H), 7.70-7.66 (m, 5H), 7.51-7.44 (m, 93H), 7.40 -7.31(m, 5H), 7.13-7.11 (m, 2H), 7.00-6.96 (m, 2H), 6.67-6.60 (m, 2H), 6.21-6.19 (m, 43H), 4.10-3.96 (m, 73H), 3.58 - 3.45 (m, 417H), 3.40-3.36 (m, 1031H), 3.27-3.21 (m, 155H), 3.18-3.06 (m, 281H), 2.90-2.77 (m, 563H), 2.58-2.51 (m, 54H), 2.06 -2.01(m, 92H), 1.61 - 1.42 (m, 193H), 1.35-1.33 (m, 22H), 1.24-1.22 (m, 144H).

### Synthesis of Product 43-226

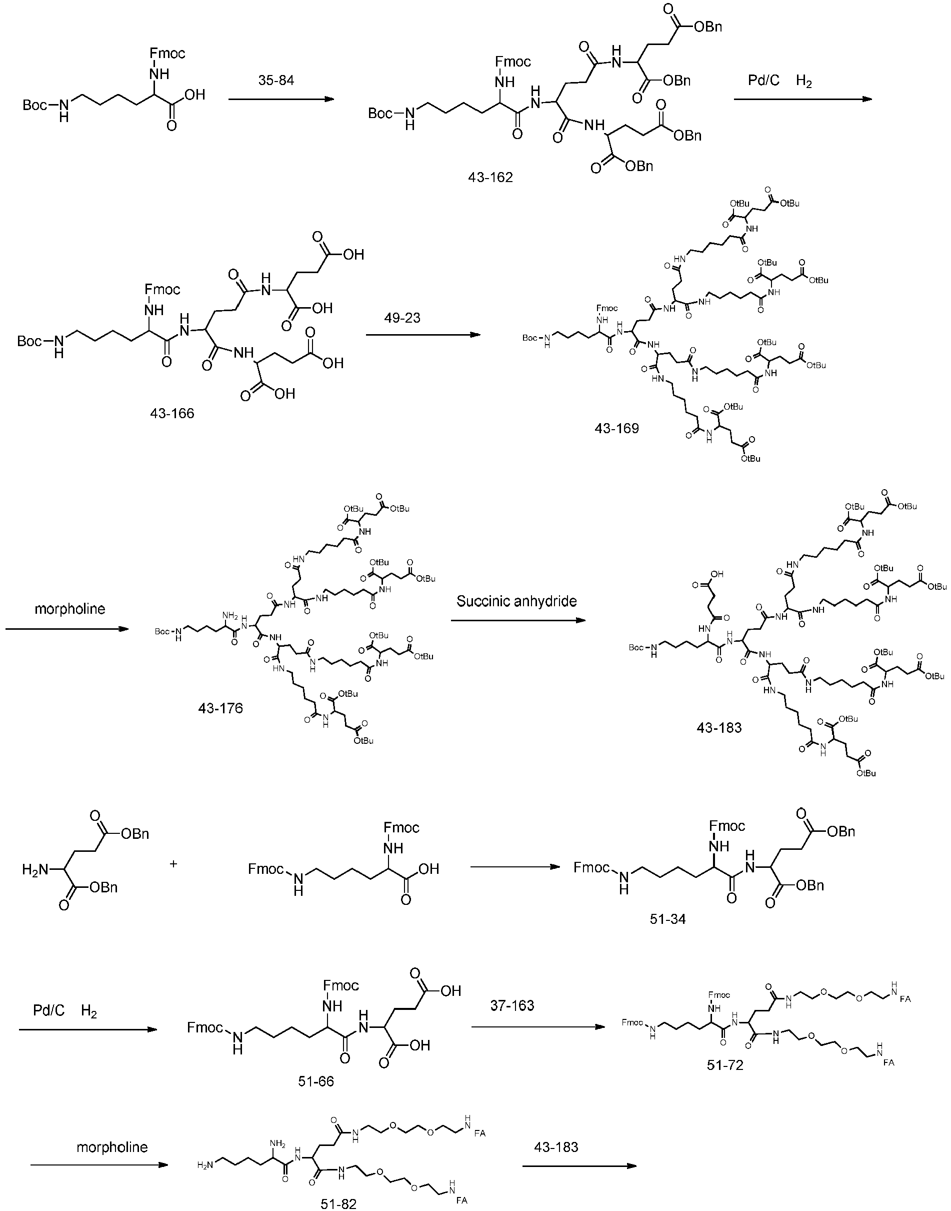

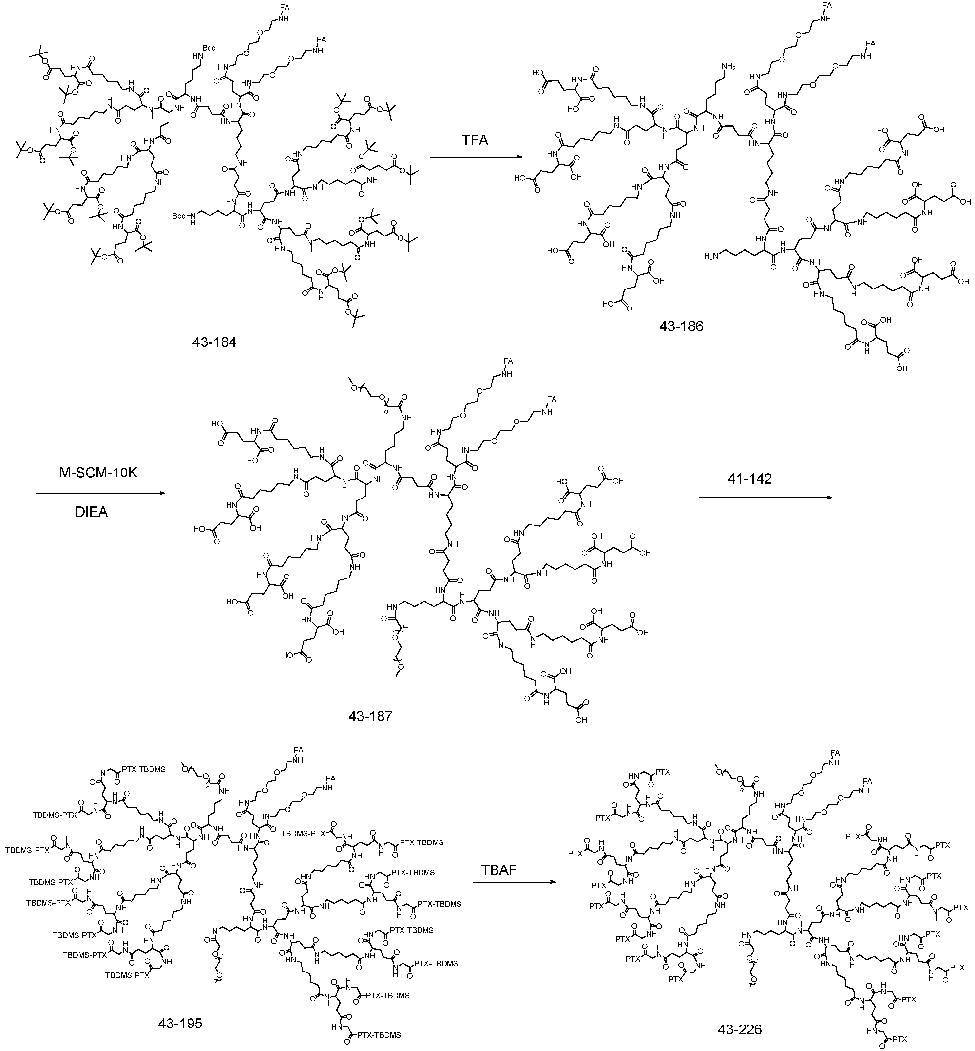

43-162

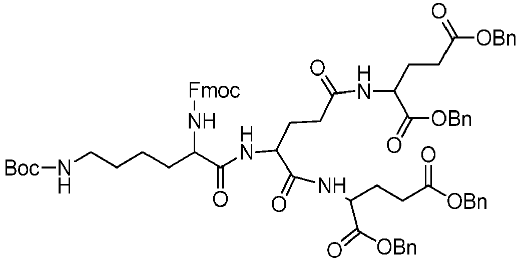

Product 35-84 (8.99 g, 11.7386 mmol), Fmoc-Lys(Boc)-OH (5.0 g, 10.6714 mmol, purchased from Aladdin), HBTU (6.0706 g, 16.0072 mmol) and HOBT (2.1629 g, 16.0072 mmol) were added in a 500 mL round-bottomed flask and then dissolved with DMF (80 mL), and the obtained solution in the flask was stirred at -5 °C for about 30 min; then, DIEA (10.6 mL, 64.0287 mmol) was slowly added dropwise, and at the end of the addition, the reaction solution in the flask was stirred at -5 °C for about 3 h. At the end of the reaction, the reaction solution was transferred to a 1 L separatory funnel, a saturated sodium chloride solution (300 mL) and ethyl acetate (200 mL) were added, the obtained solution was shaken and extracted, and the aqueous phase and the organic phase were separated; ethyl acetate (100 mL) was then added to the aqueous phase, the obtained solution was shaken and extracted, and the aqueous phase and the organic phase were separated. Then, the obtained organic phases were combined, a saturated sodium chloride solution (300 mL) was added to the organic phase, the obtained solution was shaken and extracted, and the aqueous phase and the organic phase were separated; deionized water (300 mL) was then added to the organic phase, the obtained solution was shaken and extracted, and the aqueous phase and the organic phase were separated. Finally, the organic phase was concentrated, evaporated to dryness, and dried, thus obtaining 12.98 g of the product. 43-166

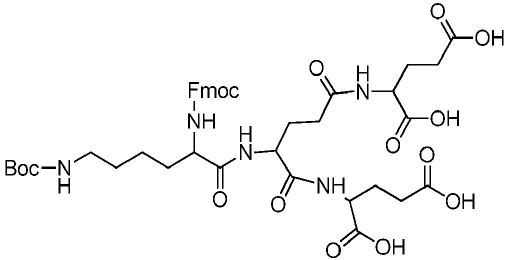

Product 43-162 (3 g, 2.4664 mmol) and 10% Pd/C (100 mg) were added in a hydrogenation reactor and then dissolved with DMF (30 mL), and the hydrogenation reactor was then sealed; hydrogen was introduced to a pressure of 1.8 MPa in the reactor; the hydrogenation reactor was placed at room temperature and the rection solution was stirred overnight. At the end of the reaction, the reactor was taken out, and the reaction solution was evenly added dropwise to a sand core funnel filled with compacted diatomaceous earth, suction filtering was carried out, and the diatomaceous earth was washed with DMF three times, thus obtaining the reaction solution of the product. 43-169

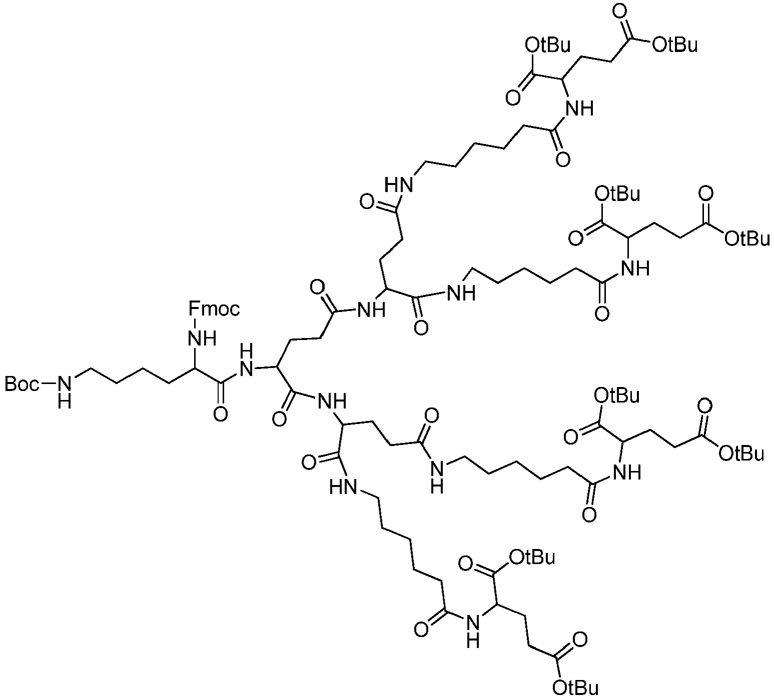

Product 43-166 (2.4664 mmol), Product 49-23 (4.04 g, 10.8522 mmol), HBTU (5.61 g, 14.7984 mmol) and HOBT (2.00 g, 14.7984 mmol) were added in a 500 mL round-bottomed flask and then dissolved with DMF (150 mL), and the obtained solution in the flask was stirred at -5 °C for about 30 min; then, DIEA (7.34 mL, 44.3952 mmol) was slowly added dropwise, and at the end of the addition, the reaction solution in the flask was stirred at -5 °C for about 3 h. At the end of the reaction, the reaction solution was transferred to a 1 L separatory funnel, a saturated sodium chloride solution (300 mL) and ethyl acetate (200 mL) were added, the obtained solution was shaken and extracted, and the aqueous phase and the organic phase were separated; ethyl acetate (100 mL) was added to the aqueous phase, the obtained solution was shaken and extracted, and the aqueous phase and the organic phase were separated. Then, the obtained organic phases were combined, a saturated sodium chloride solution (300 mL) was further added to the organic phase, the obtained solution was shaken and extracted, and the aqueous phase and the organic phase were separated; deionized water (300 mL) was then added to the organic phase, the obtained solution was shaken and extracted, and the aqueous phase and the organic phase were separated. The organic phase was concentrated and evaporated to dryness, and the solid product was dissolved with methanol (30 mL) and dichloromethane (120 mL); silica gel powder (50 g) was then added to the obtained solution, and the solution was then evaporated to dryness to obtain a powdery solid; the operations of dry sample loading, column chromatography, and elution with a mixed solution (3%-7% methanol/dichloromethane) were carried out. 4.9 g of the product was obtained. 43-176

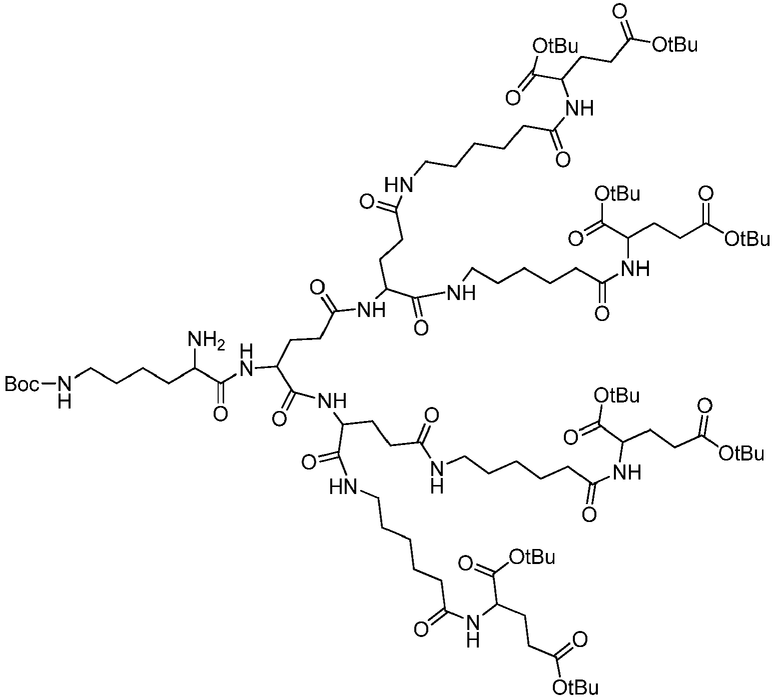

Product 43-169 (4.43 g, 1.95 mmol) were added in a 250 mL flask and then dissolved with DMF (30 mL), morpholine (1.7 mL, 19.5 mmol) was then added, and the mixed solution was stirred for 1 h at room temperature to react. At the end of the reaction, the reaction solution was transferred to a 1 L separatory funnel and extracted with deionized water (200 mL) and ethyl acetate (200 mL) to obtain an organic phase; the aqueous phase was washed with ethyl acetate (200 mL × 2), and the obtained organic phases were combined, washed with a saturated saline solution (200 mL × 2), then concentrated and evaporated to dryness; the operations of dry sample loading, column chromatography and gradient elution with 5%-10% methanol/dichloromethane were carried out, thus obtaining 4.3 g of the product. 43-183

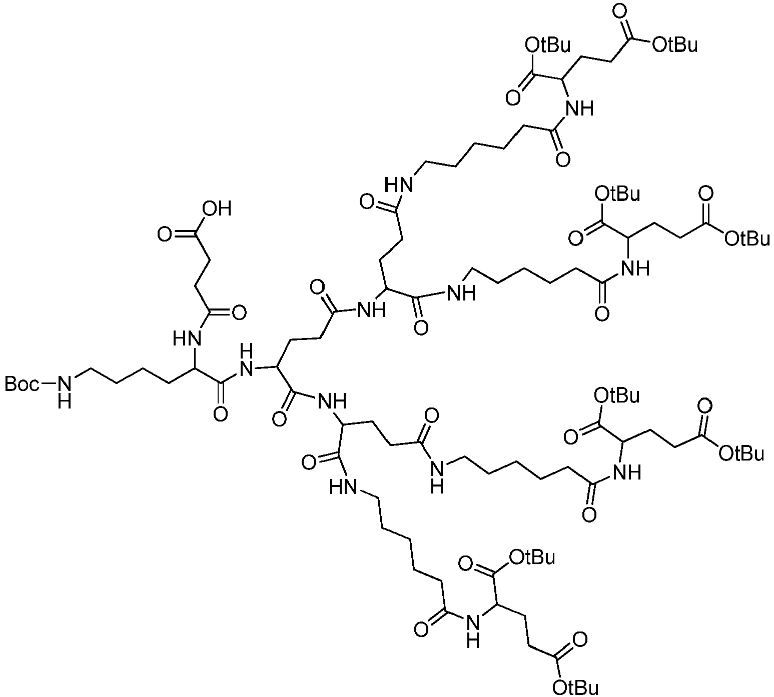

Product 43-176 (4.0 g, 1.95 mmol) was added in a 250 mL flask and then dissolved with DMF (30 mL), DIEA (4.4 mL, 26.64 mmol) was then added dropwise, and the mixed solution was stirred for 1 h; succinic anhydride (0.59 g, 5.85 mmol) was then added, and the reaction solution was stirred at room temperature overnight to react. At the end of the reaction, the reaction solution was transferred to a 1 L separatory funnel and extracted with deionized water (200 mL) and ethyl acetate (200 mL) to obtain an organic phase; the aqueous phase was washed with ethyl acetate (200 mL × 4), and the obtained organic phases were combined, washed with a saturated saline solution (200 mL × 2), then concentrated and evaporated to dryness, thus obtaining 3.6 g of the product. 51-34

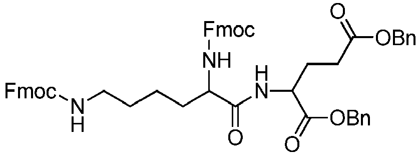

Fmoc-Lys (Fmoc)-OH (5 g, 8.46 mmol, purchased from Innochem), HBTU (4.8 g, 12.6 mmol), HOBT (1.7 g, 12.6 mmol) and H-Glu (OBzl)- OBzl•TOsOH (4.22 g, 8.46 mmol, purchased from Ark Pharm) were added in a 1000 mL flask and then dissolved with DMF (200 mL), and the obtained solution was stirred for about 30 min at -5 °C; then, DIEA (12.51 mL, 75.69 mmol) was slowly added dropwise; at the end of the addition, the reaction solution was first stirred at -5 °C to react for 1 h, and then stirred at room temperature to further react overnight. At the end of the reaction, the reaction solution was transferred to a 2 L separatory funnel and extracted with a saturated sodium bicarbonate solution (600 mL) and ethyl acetate (300 mL); the aqueous phase was washed with ethyl acetate (200 mL × 1), and the obtained organic phases were combined, washed with a saturated saline solution (200 mL × 2), then concentrated and evaporated to dryness, thus obtaining 7.6 g of the product. 51-66

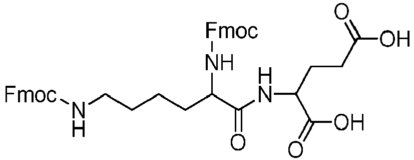

Product 51-34 (1.5 g, 1.66 mmol) and 10% Pd/C (70 mg) were added in a hydrogenation reactor and then dissolved with DMF (30 mL), and the hydrogenation reactor was then sealed; hydrogen was introduced to a pressure of 1.8 MPa in the reactor, and the hydrogenation reactor was placed at room temperature and the rection solution was stirred overnight. At the end of the reaction, the reactor was taken out, and the reaction solution was evenly added dropwise to a sand core funnel filled with compacted diatomaceous earth; then suction filtering was carried out, and the diatomaceous earth was washed with DMF three times, thus obtaining the reaction solution of the product. 51-72

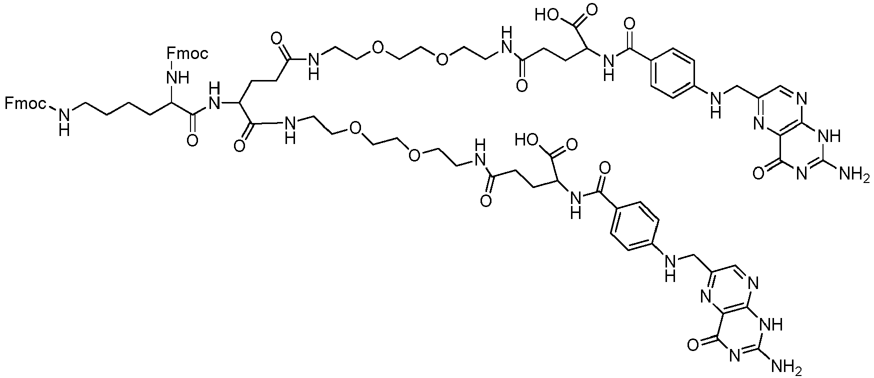

Product 51-66 (1.66 mmol), Product 37-163 (1.95 g, 3.41 mmol), HBTU (1.25 g, 3.32 mmol) and HOBT (0.44 g, 3.32 mmol) were added in a 500 mL round-bottomed flask and then dissolved with DMF (150 mL), and the obtained solution in the flask was stirred at -5 °C for about 30 min; then, DIEA (1.64 mL, 9.96 mmol) was slowly added dropwise, and at the end of the addition, the reaction solution in the flask was stirred at -5 °C for about 3 h. At the end of the reaction, methyl tert-butyl ether (200 mL) and n-hexane (300 mL) were added, and a solid product was precipitated; suction filtering was then carried out, and the filter cake was washed with dichloromethane (10 mL × 2), and dried in vacuum, thus obtaining 2.0 g of the product. 51-82

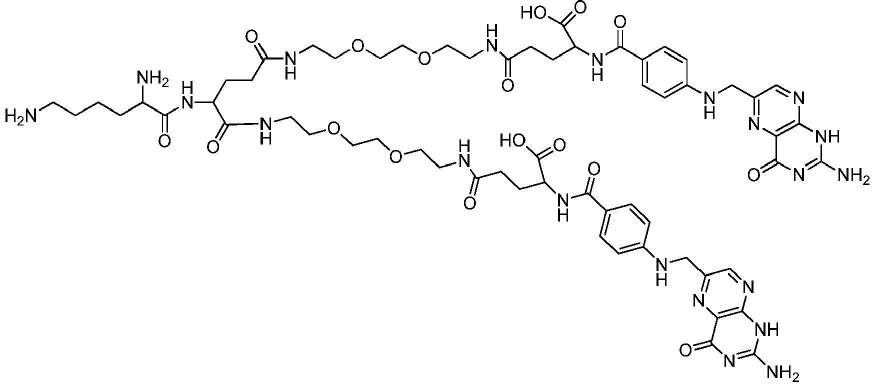

Product 51-72 (2.0 g, 1.09 mmol) was added in a 250 mL flask and then dissolved with DMF (30 mL), morpholine (2.8 mL, 32.8 mmol) was then added, and the mixed solution was stirred for 1 h at room temperature to react. At the end of the reaction, methyl tert-butyl ether (200 mL) and n-hexane (300 mL) were added, and a solid product was precipitated; suction filtering was then carried out, and the filter cake was washed with dichloromethane (10 mL × 2), and dried in vacuum, thus obtaining 1.0 g of the product. 43-184

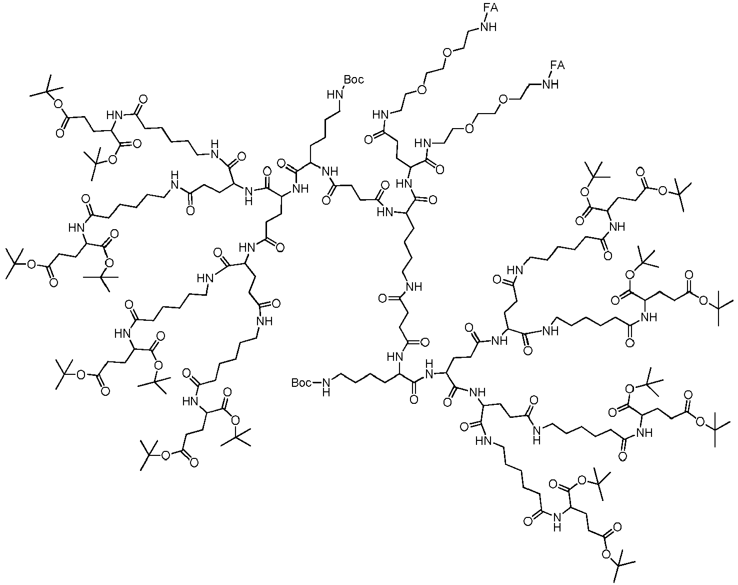

Product 43-183 (0.3424 g, 0.159 mmol), Product 51-82 (0.1 g, 0.0072 mmol), HBTU (0.08 g, 0.2169 mmol) and HOBT (0.029 g, 0.2169 mmol) were added to a 500 mL round-bottomed flask and then dissolved with DMF (150 mL), and the obtained solution in the flask was stirred at -5 °C for about 30 min; then, DIEA (0.1 mL, 0.65 mmol) was slowly added dropwise, and at the end of the addition, the reaction solution in the flask was stirred at -5 °C for about 3 h. At the end of the reaction, methyl tert-butyl ether (200 mL) and n-hexane (300 mL) was added, a solid product was precipitated, suction filtering was then carried out, and the filter cake was washed with methyl tert-butyl ether (150 mL × 2), and dried, thus obtaining 0.3 g of the product with a yield of 75%. 43-186

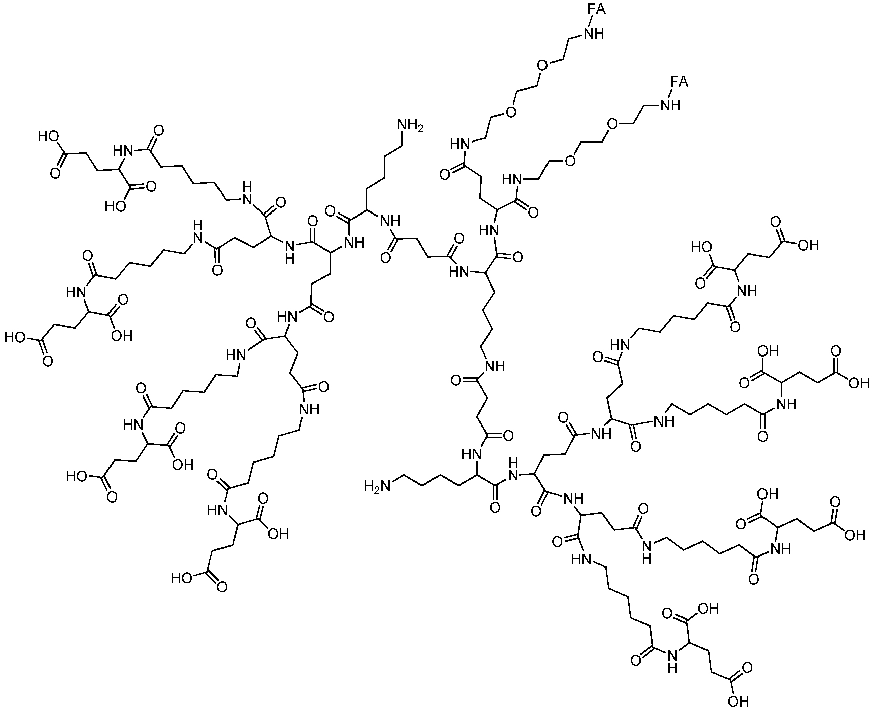

Product 43-184 (0.3 g, 0.053 mmol) was added in a 500 mL round-bottomed flask and then dissolved with dichloromethane (20 mL), TFA (1 mL, 14.33 mmol) was then added with stirring, and the obtained solution in the flask was stirred at room temperature to react overnight. At the end of the reaction, the reaction solution was first concentrated and evaporated to remove dichloromethane. Then, methyl tert-butyl ether (150 mL) was added for precipitation, a powdery solid was obtained, and the filtering was carried out. The filter cake was washed with methyl tert-butyl ether (60 mL) and dried in an oven. 0.2 g of the product was obtained with a yield of 83%. 43-187

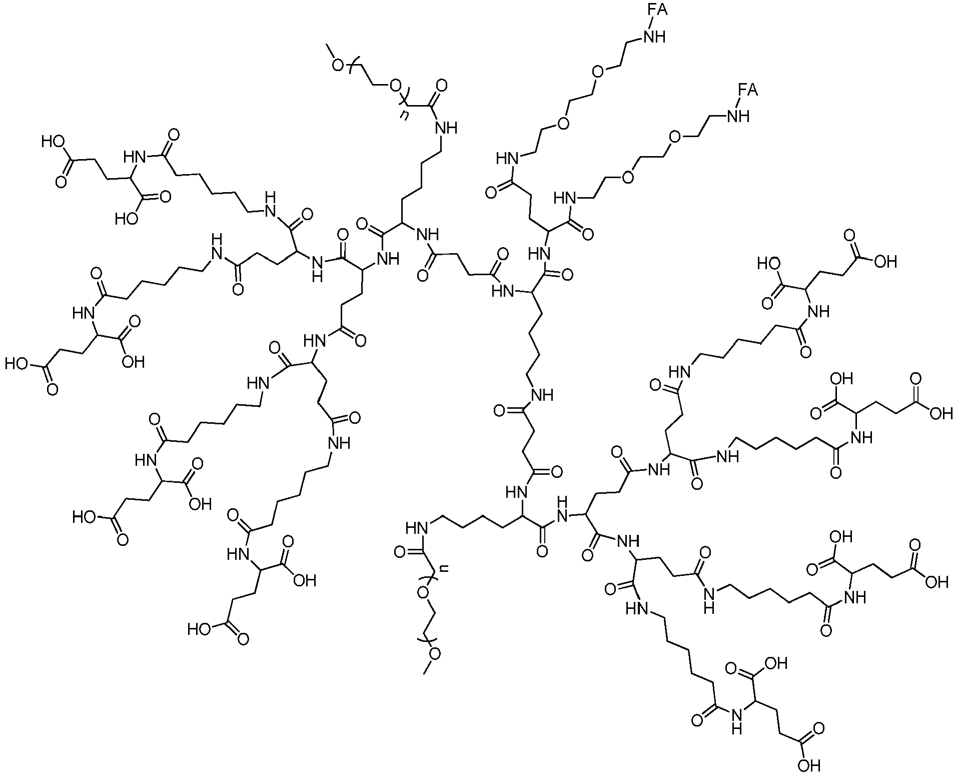

Product 43-186 (0.087 g, 0.019 mmol) was added in a 250 mL flask and then dissolved with DMF (40 mL), and the obtained solution was reacted for about 30 min at -5 °C; DIEA (0.31 mL, 1.9 mmol) was slowly added dropwise; the obtained solution was stirred at low temperature for 10 min and M-SCM-10K (0.45 g, 0.04 mmol, purchased from JenKem) was added, and the obtained solution was slowly stirred for one week at room temperature in the dark to react. At the end of the reaction, methyl tert-butyl ether (200 mL) and n-hexane (70 mL) were added to separate out a solid product, suction filtering was carried out, and the filter cake was washed with methyl tert-butyl ether (40 mL × 3), and dried in a vacuum oven, thus obtaining 0.44 g of the product with a yield of 91%.

¹H-NMR (600 MHz, DMSO-d₆) δ 8.71-8.52 (m, 4H), 8.35-7.66 (m, 42H), 7.417-7.22(m, 6H), 6.99-6.64(m, 15H), 4.51-4.03 (m, 53H), 3.86-3.68 (m, 34H), 3.51-3.47(m, 1864H), 3.01 -2.88(m, 12H), 2.78-2.57 (m, 10H), 2.27 - 2.02 (m, 28H), 1.91 - 1.67 (m, 18H), 1.49 - 1.34 (m, 27H), 1.26 - 1.07 (m, 60H). 43-195

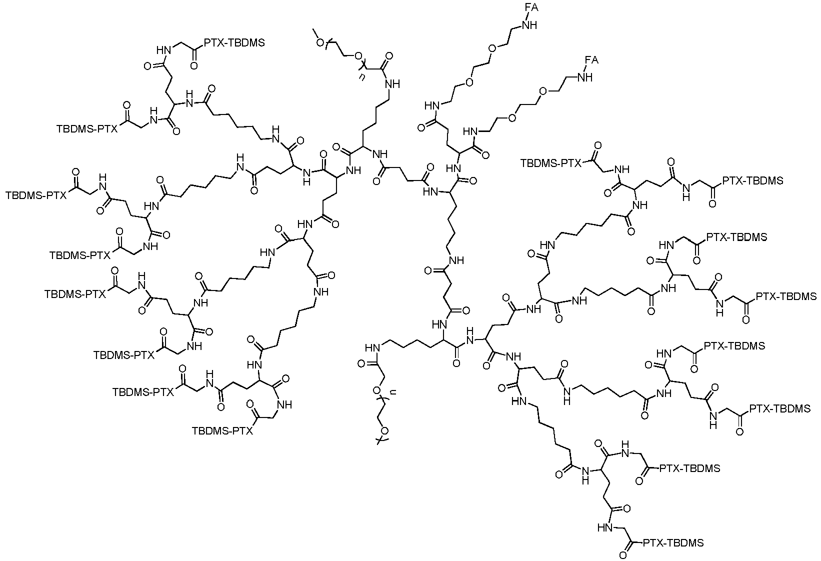

Product 41-142 (0.3 g, 0.3 mmol), Product 43-187 (0.44 g, 0.017 mmol), HBTU (0.15 g, 0.408 mmol) and HOBT (0.055 g, 0.408 mmol) were added to a 500 mL round-bottomed flask and then dissolved with DMF (150 mL), and the obtained solution in the flask was stirred at -5 °C for about 30 min; then, DIEA (0.25 mL, 1.53 mmol) was slowly added dropwise, and at the end of the addition, the reaction solution in the flask was stirred at -5 °C for about 3 h. At the end of the reaction, methyl tert-butyl ether (200 mL) and n-hexane (300 mL) was added, and a solid product was precipitated; suction filtering was then carried out, and the filter cake was washed with methyl tert-butyl ether (150 mL × 2), and dissolved with dichloromethane (160 mL) and methanol (40 mL); silica gel powder (30 g) was added to the obtained solution, and the solution was then evaporated to dryness to obtain a powdery solid; the operations of dry sample loading, column chromatography, and elution with a mixed solution (1% triethylamine+5%-10% methanol/dichloromethane) were carried out; the elution product was collected, concentrated and dried. 0.2 g of the product was obtained. 43-226

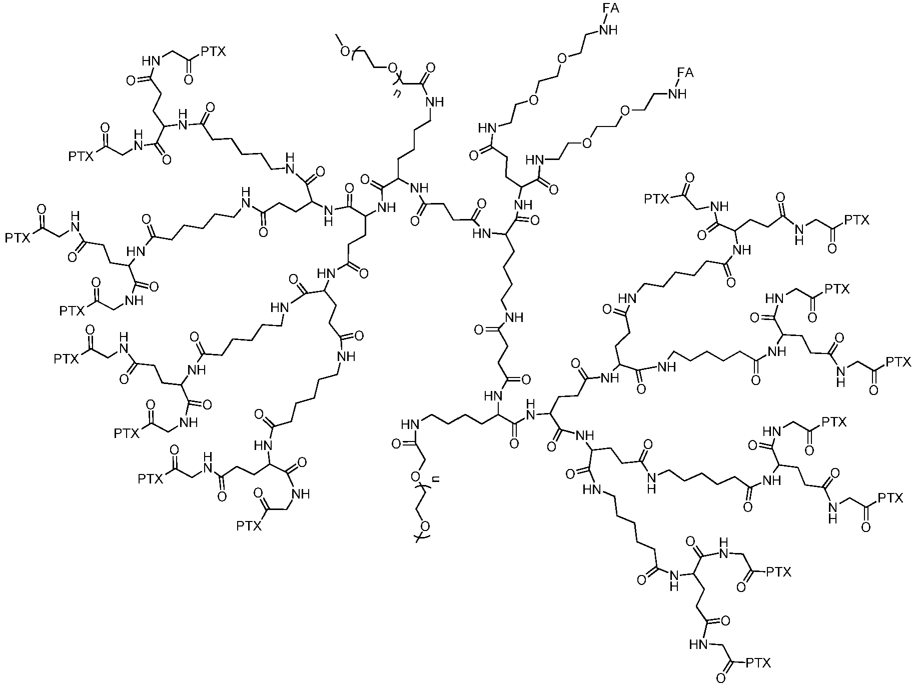

Product 43-195 (0.2 g, 0.0059 mmol) and TBAF (0.44 g, 1.42 mmol) were added in a 500 mL round-bottomed flask and then dissolved with THF (20 mL), and the reaction solution was stirred at room temperature to react overnight. At the end of the reaction, the reaction solution was concentrated under reduced pressure, dissolved with DMF (5 mL), and precipitated with isopropanol; the above operations were repeated three times, and the solid product was obtained by filtering, the operations of dry sample loading, column chromatography and gradient elution with 10%-20% methanol/dichloromethane were carried out, thus obtaining 0.1 g of the product.

¹H-NMR (600 MHz, DMSO-*d*₆) δ 8.97-8.77 (m, 14H), 8.27-7.84 (m, 103H), 7.75 -7.18(m, 190H), 7.10-6.99 (m, 16H), 6.20-5.86 (m, 45H), 5.49 - 5.37 (m, 48H), 4.95-4.81 (m, 33H), 4.60-4.40 (m, 10H), 4.26-4.04 (m, 22H), 3.86-3.70 (m, 24H),3.51-3.47(m, 1864H), 3.11 - 2.90 (m, 54H), 2.77-2.62 (m, 29H), 2.41-2.37 (m, 43H), 2.24-2.10 (m, 120H), 1.90-1.59(m, 194H), 1.46-1.20 (m, 82H), 1.06-0.90 (m, 114H).

### Synthesis of Product 51-103

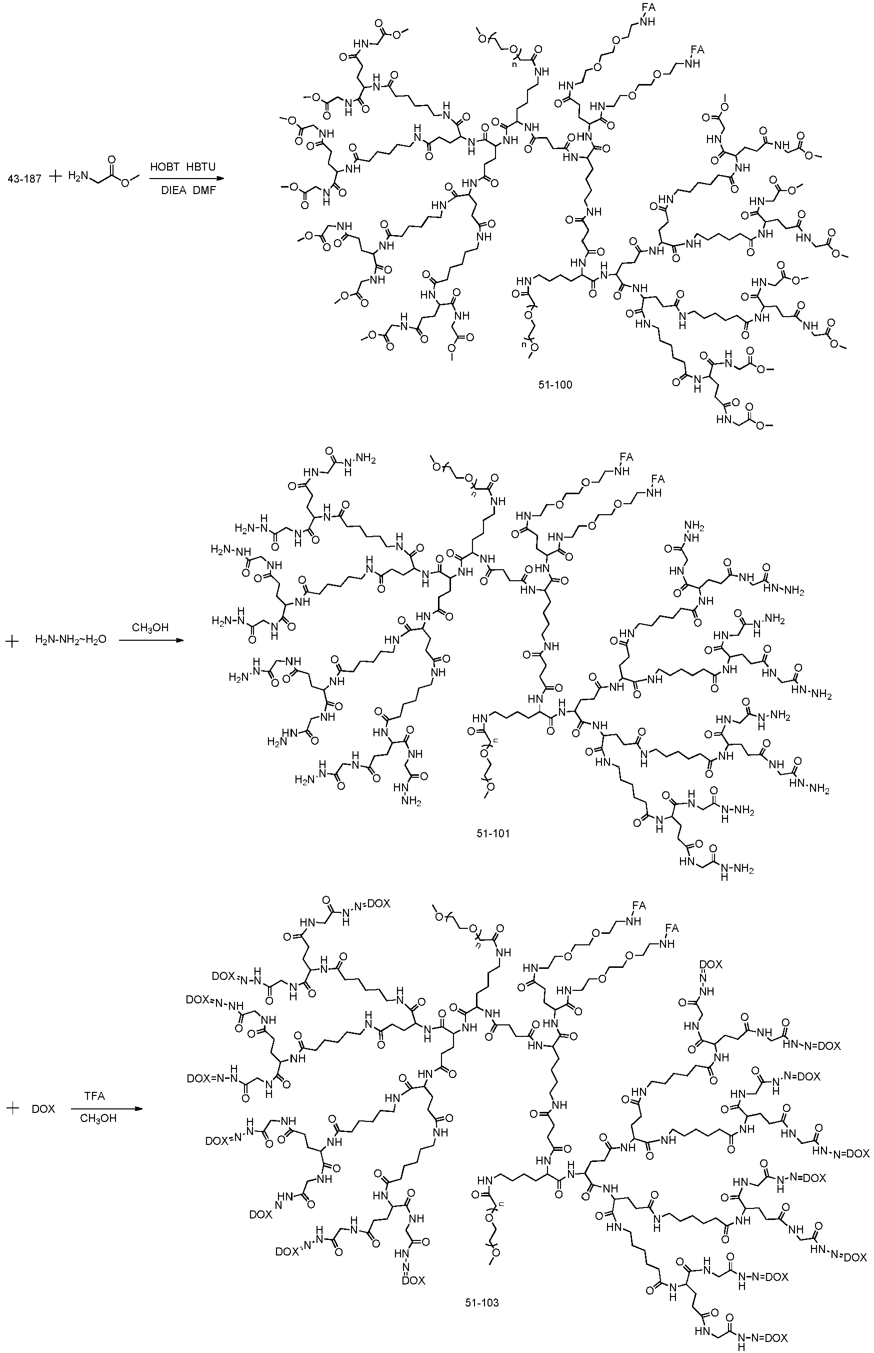

51-100

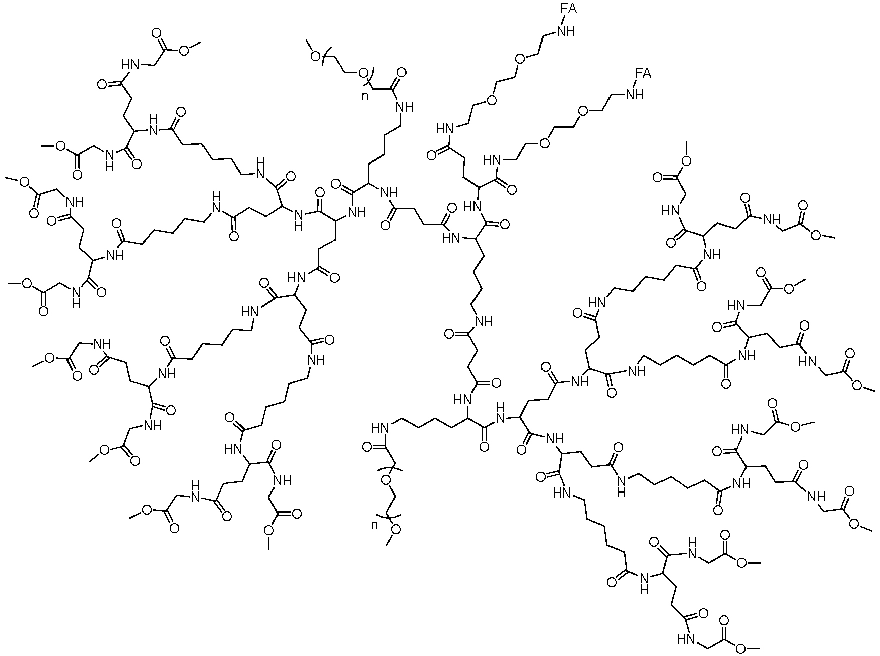

Product 43-187 (0.535 g, 0.021 mmol), Glycine methyl ester hydrochloride (0.047 g, 0.0378 mmol), HBTU (0.19 g, 0.504 mmol) and HOBT (0.068 g, 0.504 mmol) were added to a 500 mL round-bottomed flask and then dissolved with DMF (150 mL); the obtained solution in the flask was stirred at -5 °C for about 30 min; then, DIEA (0.249 mL, 1.512 mmol) was slowly added dropwise; at the end of the addition, the reaction solution in the flask was stirred at -5 °C for about 3 h. At the end of the reaction, methyl tert-butyl ether (200 mL) and n-hexane (300 mL) were added to layer the reaction solution, and the supernatant was discarded; methyl tert-butyl ether (200 mL) and n-hexane (300 mL) were further added to the lower oily product, and such operations were repeated three times to obtain a small amount of the oily product; methyl tert-butyl ether (250 mL) was added, and a solid product was precipitated; suction filtering was then carried out, and the filter cake was washed with methyl tert-butyl ether (150 mL × 2), and dissolved with dichloromethane (160 mL) and methanol (40 mL); silica gel powder (30 g) was then added to the obtained solution and the solution was evaporated to dryness to obtain a powdery solid; the operations of dry sample loading, column chromatography and elution with a mixed solution (5%-8% methanol/dichloromethane) were carried out; the elution product was collected, concentrated and dried. 0.5590 g of the product was obtained. 51-101

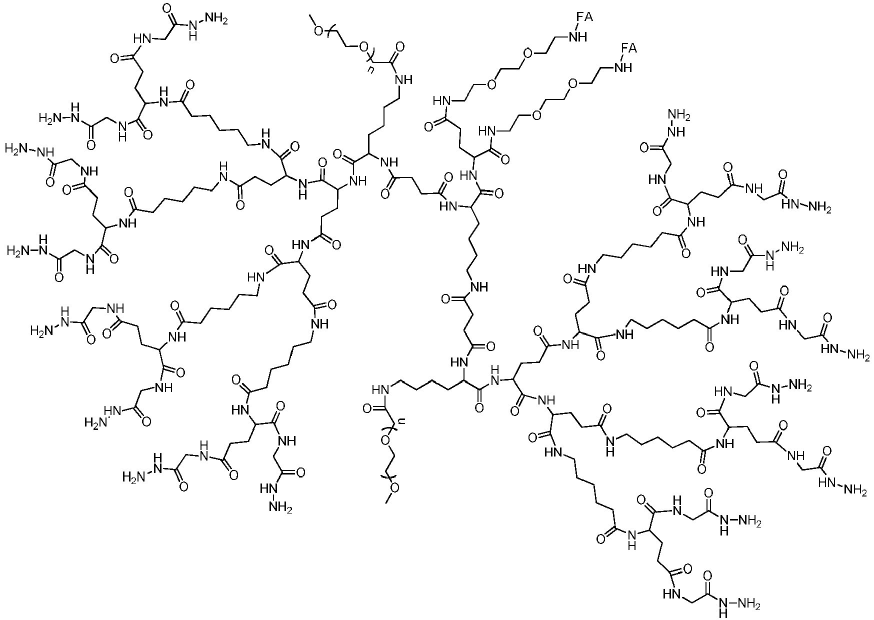

Product 51-100 (0.021 mmol) was added in a 250 mL round-bottomed flask and then dissolved with methanol (80 mL), and then hydrazine hydrate (0.08 mL, 1.68 mmol) was added to the mixed solution; finally, the obtained solution in the flask was stirred at room temperature overnight to react. At the end of the reaction, the reaction solution was first concentrated and evaporated. Then, methyl tert-butyl ether (150 mL) was added for precipitation, a powdery solid was obtained, and the filtering was carried out. The filter cake was washed with methyl tert-butyl ether (60 mL) and dried in an oven. 51-103

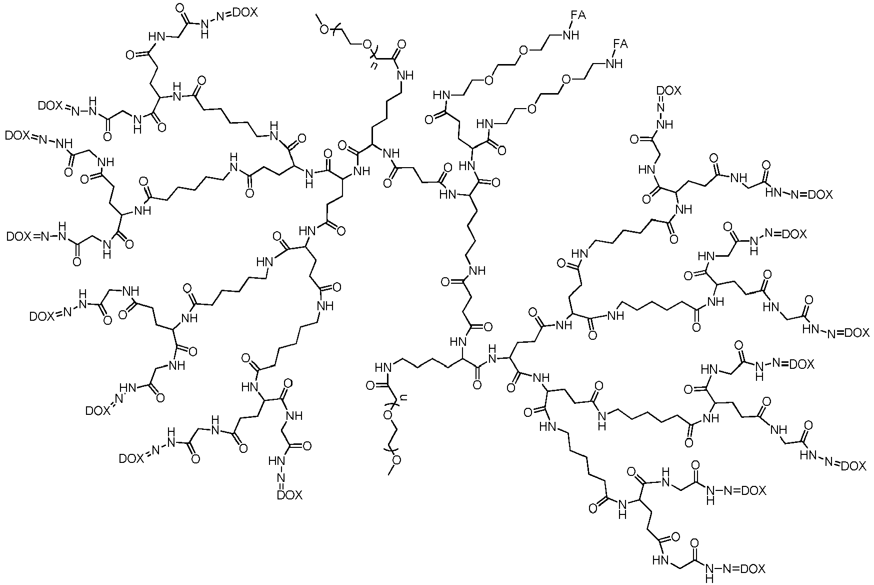

Product 51-101 (0.021 mmol) was added in a dry 500 mL round-bottomed flask and then dissolved with anhydrous methanol; then, TFA (0.249 mL, 3.36 mmol) and DOX (0.219 g, 18 mmol) were added, and the obtained solution was stirred at room temperature overnight to react. After the reaction was completed, n-hexane (25 mL) and methyl tert-butyl ether (200 mL) were first added for precipitation, and the supernatant was discarded; n-hexane and methyl tert-butyl ether were added again for precipitation, and such operations were repeated three times; the filtering was then carried out to obtain a solid product, and the obtained solid product was dissolved with dichloromethane and methanol; the operations of dry sample loading, column chromatography and elution with 6-10% methanol/dichloromethane were carried out; the elution product was then collected, concentrated and dried, thus obtaining 0.2 g of the product.

¹H-NMR (600 MHz, DMSO-d₆) δ 7.99 -7.61 (m, 121H), 6.32 - 4.81 (m, 82H), 4.65 - 4.04 (m, 57H), 4.02 - 3.85 (m, 65H), 3.87 - 3.60 (m, 68H), 3.50 (s, 1404H), 3.26 - 2.59 (m, 95H), 2.42 - 1.31 (m, 200H), 1.23 (s, 48H), 1.20 - 0.94 (m, 76H).

### Example 2 Efficacy test of polyethylene glycol conjugated drug on BALB/c nude mouse subcutaneous transplantation tumor models of human breast cancer MDA-MB-231 cells

### 1. Preparation method:

### Test sample:

1) a proper amount of polyethylene glycol conjugated drug (such as 49-166) which was used as a control and synthesized according to the present invention was taken, and a proper amount of normal saline was added to prepare a solution with a proper concentration.
2) a proper amount of polyethylene glycol conjugated drug to be tested (such as 47-122) was taken, and a proper amount of normal saline was added to prepare a solution with a proper concentration.

Negative control: normal saline was directly used.

The prepared test samples and control sample were preserved at 2-8°C or in an ice box before administration, and the residual test samples and control sample after administration were treated as medical waste.

### 2. Cells and experimental animals

Human breast cancer cell MDA-MB-231: it was from the Cell Resource Center of Institute of Basic Medicine of Chinese Academy of Medical Sciences, cultured under the conditions of RPMI1640 + 10% FBS, 37°C, 5% CO₂.
Animal species & strain: BALB/c nude mice
Animal level: SPF level
Animal source: Beijing Vital River Laboratory Animal Technology Co., Ltd.
Animal age at tumor inoculation: about 4-5 weeks.
Animal weight at tumor inoculation: about 15-18 g. The weights of animals of the same sex were between 80-120% of the average weight.
Animal sex and number: female, 96 mice were purchased, 48 modeling animals were screened for final experiments, and the remaining animals were either handed over to veterinarian or euthanized.

Animals were reared in an independent ventilation system (IVC), at most 6 animals of the same group in each cage, and an SPF level animal house was provided, with the environmental conditions controlled as follows: room temperature 20-26°C, 40-70% of relative humidity and illumination with 12 hours light dark alternation. During quarantine domestication and testing period, qualified mouse feed (manufacturer: Beijing Keao Xieli Feed Co., Ltd.) was provided each day. The animals ate freely and drunk water freely.

### 3. Model establishment

MDA-MB-231 cells were revived, and cell passage amplification was carried out. When amplified to a sufficient number, the cells in the logarithmic growth phase were collected for cell inoculation.

According to the actual cell number, the cells were adjusted to have a concentration of 1×10⁸/mL, and mixed with Matrigel (Matrix Basement Membrane Matrix, BD Co.) at a volume ratio of 1:1, to obtain a cell suspension with a concentration of 5×10⁷/mL. The cell suspension was inoculated subcutaneously in the right armpit of 96 mice at 0.2 mL per mouse. The tumor growth was observed after inoculation, and 48 tumorigenic animals with the tumor volume of 77.30-292.27 mm³ were finally screened and used for the test.

### 4. Animal grouping and dosing

The tumorigenic animals were randomly divided into 7 groups according to the tumor volume and the body weight, including: group 1 (negative control group, normal saline), group 6 (49-166, 48 mg/kg), group 7 (47-122, 51 mg/kg), 6 animals in each group. The animals were intravenously injected, for administration of each group at D1, D4, D7, D10, D13, D18, D21, D24, and at D27 the animals were euthanized.

### 5. Experimental results

### 5.1 General clinical observation

Observation frequency and time: during the experiment, all animals underwent gross observations twice daily.

Observation contents: including mental state, behavioral activities, food intake and the like of the animals.

### 5.2 Body weight

### Test animal: all animals

Test time: after receiving, before inoculation, the day of grouping (i.e., D1, the day of first administration), 2 times per week after first administration, and before euthanasia, the animals were weighed. The animals were also weighed when they died accidentally or when they were dying or euthanized.

### 5.3 Measurement of tumor diameter:

### Test animal: all animals

Test time: the day of grouping (i.e., D1, the day of first administration), 2 times per week after first administration, and before euthanasia, the long and short diameters of tumor were measured using a slide caliper and recorded, and the tumor volume was calculated.

The tumor volume was calculated according to the following formula: $V = 1 / 2 \times long diameter \times short {diameter}^{2}$

During the experiment, the general clinical symptoms of the animals were observed 2 times every day, and the body weight and tumor diameter were measured 10 times in total. The tumor was stripped after euthanization, and the tumor weight was weighed. The tumor volume, relative tumor volume RTV, relative tumor proliferation rate T/C% and tumor weight inhibition rate IR_{TW}% were calculated.

### 6. Analysis and evaluation of results

Statistical analysis: in this experiment, statistical software SPSS13.0 was used to process the data, and the measured data was expressed by mean value ± standard error. The specific analysis process was as follows

One-way analysis of variance (ANOVA) was used to carry out the statistical analysis. Comparative analysis between groups was performed by Tukey test if ANOVA had statistical significance (P ≤ 0.05) and variance was homogeneous, and comparative analysis between groups was performed by Dunnett's T3 test if variance was inhomogeneous.

### 6.1 Evaluation of therapeutic efficacy based on the tumor volume

The relative tumor volume (RTV) and the relative tumor proliferation rate T/C% were calculated according to the following formula: $RTV = {V}_{t} / {V}_{0}$
Vₜ: tumor volume obtained by measuring tumor every day
V₀: initial tumor volume (before administration)
T/C%= average RTV of the administration group/average RTV of the control group×100 %

If the relative tumor proliferation rate T/C% of the administration group was ≤ 40 %, and the RTV of the administration group was effective compared with the RTV of the negative control group (P ≤ 0.05), tumor growth inhibition effect was achieved; on the other hand, if T/C% was > 40%, tumor growth was not inhibited.

### 6.2 Evaluation of therapeutic efficacy based on the tumor weight

After the experiment, tumor nodules were stripped and weighed, and the differences in tumor weight among the groups were compared to further calculate the tumor inhibition rate IR_{TW}. IR_{TW}>60% was taken as an effective reference indicator. The calculation was conducted according to the following formula: ${IR}_{TW} \left(%\right) = \left({W}_{Control group}-{W}_{Administration group}\right) / {W}_{Control group} \times 100 %$

### 6.3 Gross anatomy observation

The animals died during the experiment and the euthanized animals after observation period were subjected to gross anatomy for observation of their main organs, to see if there are obvious abnormalities visible to the naked eye.

### 6.4 Photograph recording

The pictures of the euthanized animals and tumors were taken.

The concrete results were as follows:
Throughout the experiment, no obvious abnormality was seen in each group of the animals. The body weight of each group of the animals slowly increased during the experiment, and no significant difference was seen between each test sample group and the group 1 (P>0.05).

In the negative control group (group 1), the tumor gradually increased throughout the experiment, by the end of the experiment (D27), the group 1 had an average tumor volume of 2962.92±2176.59 mm³ and an average RTV of 19.14±12.01; the average tumor volumes of the groups 6, 7 were 1690.27±785.04 mm³, 1041.24±291.96 mm³ respectively, and the average RTVs thereof were 13.03±5.43, 8.12±2.34 respectively.

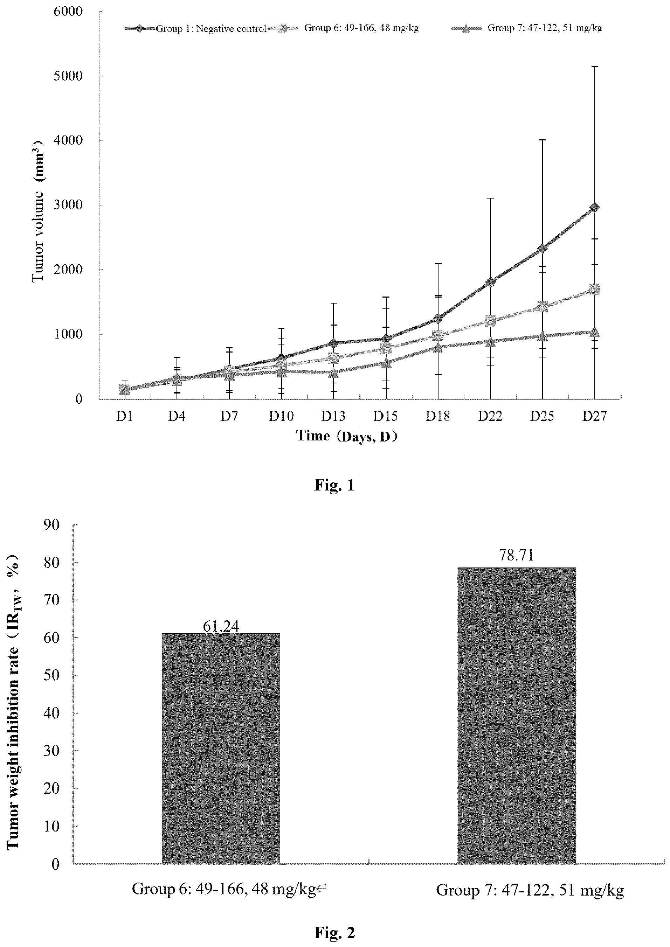

The tumor growth trend of each group is shown in FIG. 1.

By the end of the experiment (D27), the T/C% values of the groups 6, 7 were 68.08%, 42.41% respectively, and the IR_{TW}% values thereof were 31.92%, 57.59% respectively.

At the end of the experiment, the tumors of the animals were weighed after euthanasia. The average tumor weights of the groups 1, 6, 7 were 2.555±2.207 g, 0.990±0.399 g, 0.544±0.127 g respectively. The IR_{TW}% of the groups 6, 7 were 61.24%, 78.71% respectively.

The schematic diagram of the tumor weight inhibition rate of each group is shown in FIG. 2.

Conclusion: under the experimental conditions, 49-166 at a dose of 48 mg/kg and 47-122 at a dose of 51 mg/kg, which were respectively administered by tail vein injection, had an obvious inhibition effect on the growth of the BALB/c nude mouse subcutaneous transplantation tumor of human breast cancer MDA-MB-231 cells, and the tumor inhibition effect of 47-122 was obviously superior to that of 49-166.

The inventors found out through the experiments and the results that the polyethylene glycol conjugated drug of the present invention had significant antitumor effects on subcutaneous transplantation tumor (such as BALB/c nude mouse subcutaneous transplantation tumor of human breast cancer MDA-MB-231 cells).

## Claims

1. A polyethylene glycol conjugated drug of formula (A) or a pharmaceutically acceptable salt thereof, 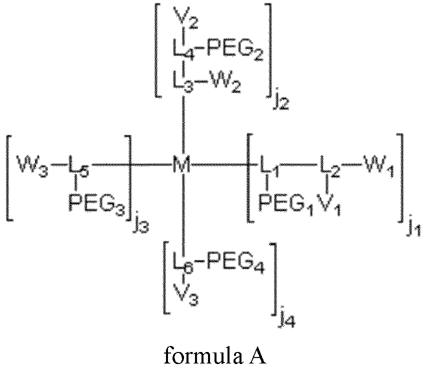 wherein:
M is selected from 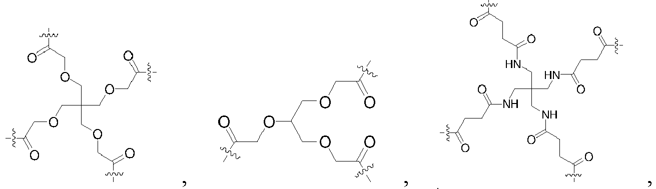 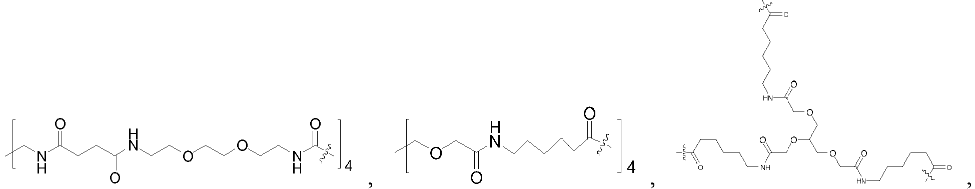 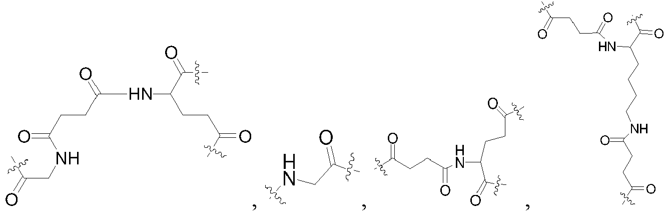 or PEGₘ; PEGₘ is a single-arm or multi-arm (such as four-arm, eight-arm, preferably four-arm) polyethylene glycol segment; PEGₘ is connected to L₁, L₃, L₅ or L₆ through a carbonyl group; the number-average molecular weight of PEGₘ is 5k-40k, preferably 5k-10k or 10k-40k, more preferably 5k or 10k;
j₁, j₂, j₃ and j₄ each independently are 0, 1, 2, 3, 4, 5 or 6, preferably 0, 1, 2, 3 or 4, and j₁, j₂, j₃ and j₄ are not 0 at the same time;
PEG₁, PEG₂, PEG₃ and PEG₄ are single-arm polyethylene glycol segments; PEG₁, PEG₂, PEG₃ and PEG₄ are connected to L₁, L₄, L₅ and L₆ through a carbonyl group respectively; each of the number-average molecular weight of PEG₁, PEG₂, PEG₃ and PEG₄ independently is 5k-40k, preferably 5k-10k or 10k-40k, more preferably 5k, 10k or 40k;
L₁, L₂, L₃, L₄, L₅ and L₆ each independently are a direct bond, 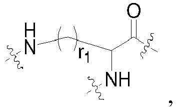 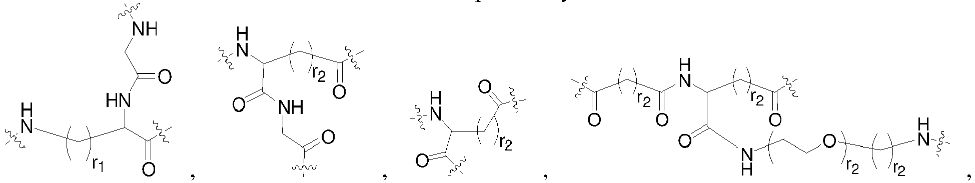 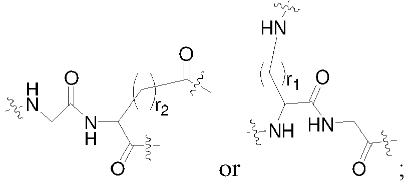 L₁, L₂, L₃, L₄, L₅ and L₆ are not direct bonds at the same time, and when L₆ is a direct bond, PEG₄ does not exist, and V3 is directly connected to M;
each r₁ independently is 1, 2, 3, 4, 5 or 6, preferably 1, 2, 3 or 4, more preferably 3 or 4;
each r₂ independently is 1, 2, 3, 4, 5 or 6, preferably 1, 2, 3 or 4, more preferably 1 or 2;
preferably, L₁, L₂ L₃, L₄, L₅ and L₆ each independently are a direct bond, 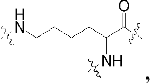 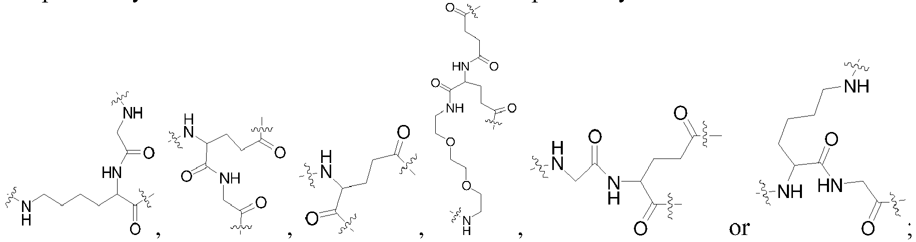 L₁, L₂, L₃, L₄, L₅ and L₆ are not direct bonds at the same time, and when L₆ is a direct bond, PEG₄ does not exist, and V3 is directly connected to M;
or preferably, L₁ is 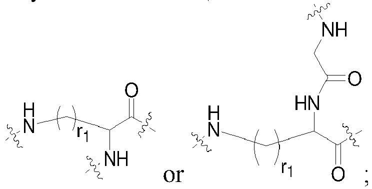 each r₁ independently is 1, 2, 3, 4, 5 or 6, preferably 1, 2, 3 or 4, more preferably 3 or 4;
more preferably, L₁ is 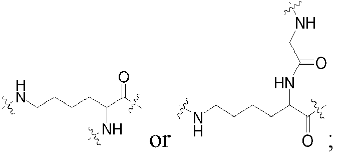
or preferably, L₂ is 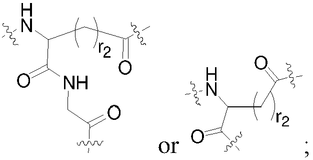 each r₂ independently is 1, 2, 3, 4, 5 or 6, preferably 1, 2, 3 or 4, more preferably 1 or 2;
more preferably, L₂ is 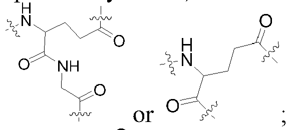
or preferably, L₃ is 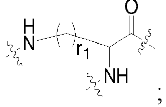 r₁ is 1, 2, 3, 4, 5 or 6, preferably 1, 2, 3 or 4, more preferably 3 or 4;
more preferably, L₃ is 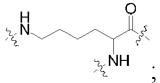
or preferably, L₄ is 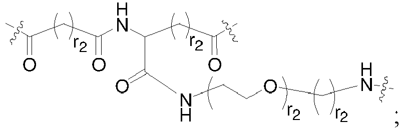 each r₂ independently is 1, 2, 3, 4, 5 or 6, preferably 1 2, 3 or 4, more preferably 1 or 2;
more preferably, L₄ is 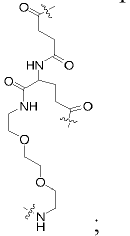
or preferably, L₅ is 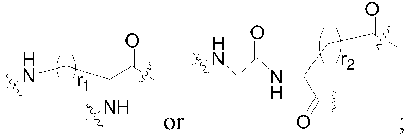 r₁ is 1, 2, 3, 4, 5 or 6, preferably 1, 2, 3 or 4, more preferably 3 or 4; r₂ is 1, 2, 3, 4, 5 or 6, preferably 1, 2, 3 or 4, more preferably 1 or 2;
more preferably, L₅ is 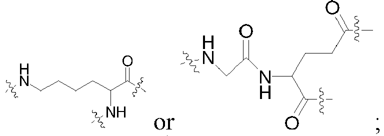
or preferably, L₆ is a direct bond, 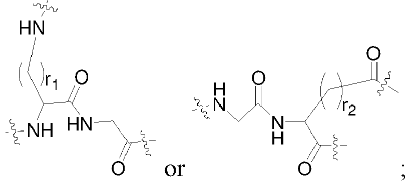 r₁ is 1, 2, 3, 4, 5 or 6, preferably 1, 2, 3 or 4, more preferably 3 or 4; r₂ is 1, 2, 3, 4, 5 or 6, preferably 1, 2, 3 or 4, more preferably 1 or 2; and when L₆ is a direct bond, PEG₄ does not exist, and V3 is directly connected to M;
more preferably, L₆ is a direct bond, 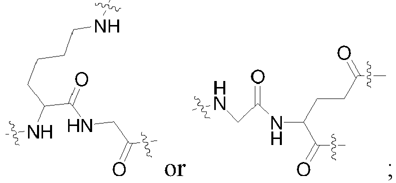 and when L₆ is a direct bond, PEG₄ does not exist, and V3 is directly connected to M;
V₁ is -L_{1V}-T₁ or 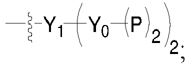
V₂ is -L_{2V}-T₂;
V₃ is -P₃, 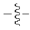 Y₀-(P₃)₂, or 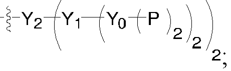
P is -Lv-T;
P₃ is -L_{3V}-T₃;
Liv, L_{2V} and L_{3V} each independently are 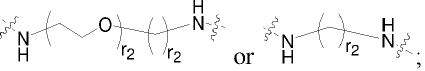 each r₂ independently is 1, 2, 3, 4, 5 or 6, preferably 1, 2, 3 or 4, more preferably 1 or 2;
preferably, Liv, L_{2V} and L_{3V} each independently are 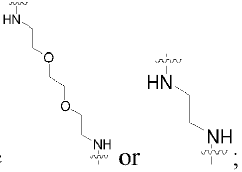
T₁, T₂ and T₃ are 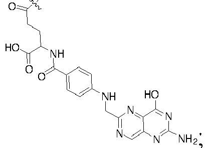
Y₂, Y₁ and Y₀ each independently are 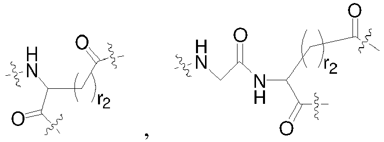 or 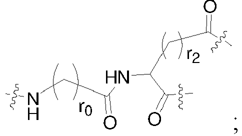 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
preferably, Y₂, Y₁ and Y₀ each independently are 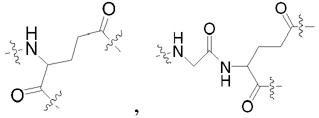 or
L_{V} is 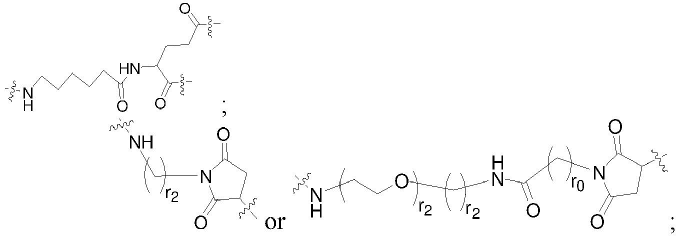 r₀is 1, 2, 3, 4, 5 or 6, preferably
3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
preferably, Lv is 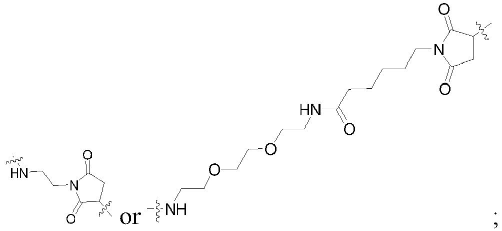 T is 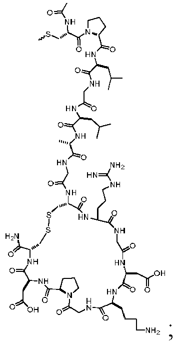 W₁, W₂ and W₃ each independently are 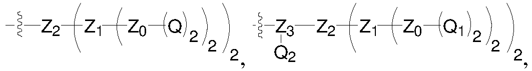 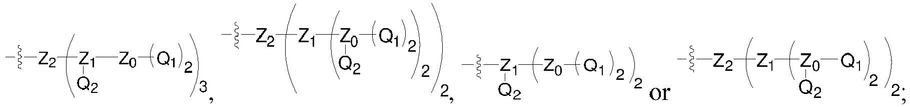 Z₃, Z₂, Z₁ and Z₀ each independently are 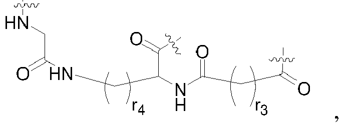 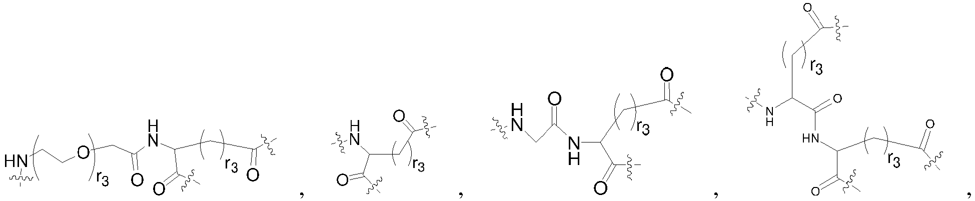 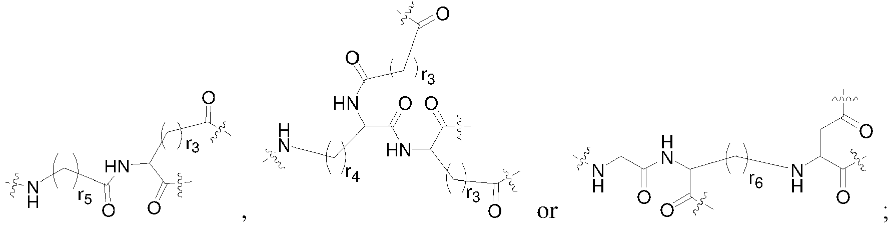 each r₃ independently is 1, 2, 3, 4, 5 or 6, each r₃ independently is preferably 1, 2, 3 or 4, each r₃ independently is more preferably 1 or 2; each r₄ independently is 1, 2, 3, 4, 5 or 6, each r₄ independently is preferably 1, 2, 3 or 4, each r₄ independently is more preferably 3 or 4; r₅ is 1, 2, 3, 4, 5 or 6, preferably 3, 4, 5 or 6, more preferably 5 or 6; r₆ is 1, 2, 3, 4, 5 or 6, preferably 2, 3, 4 or 5, more preferably 3 or 4;
preferably, Z₃, Z₂, Z₁ and Z₀ each independently are 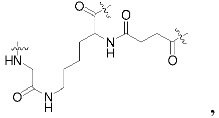 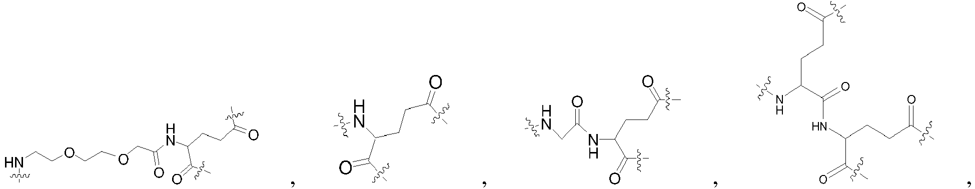 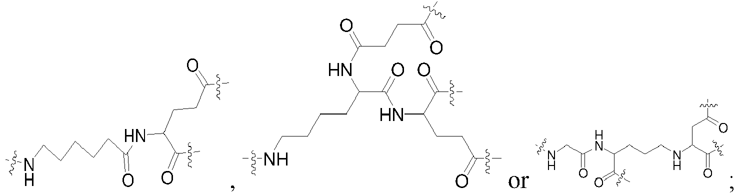
Q is -N-AC;
Q1 is -N1-AC1;
Q2 is -N2-AC2;
N, N1 and N2 each independently are 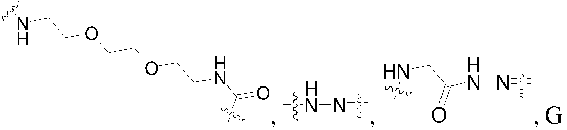 or GFLG;
AC, AC1 and AC2 are anti-cancer drug molecules; preferably, AC, AC1 and AC2 each independently are SB7, NPB, SN38, LPT, PCB, DOX, PTX or AXT.

2. The polyethylene glycol conjugated drug or a pharmaceutically acceptable salt thereof according to claim 1, wherein the polyethylene glycol conjugated drug having a structure of formula (I),
wherein: 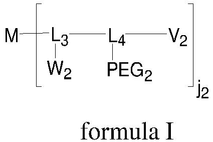
M is selected from 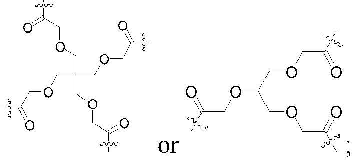
j₂ is 3 or 4;
PEG₂ is a single-arm polyethylene glycol segment; PEG₂ is connected to L₄ through a carbonyl group; the number-average molecular weight of PEG₂ is 5k-40k, preferably 5k-10k or 10k-40k, more preferably 10k;
L₃ is 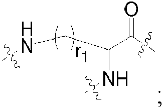 r₁ is 1, 2, 3, 4, 5 or 6, preferably 1, 2, 3 or 4, more preferably 3 or 4,
preferably, L₃ is 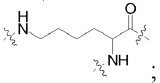
L₄ is 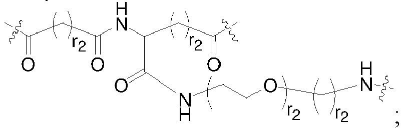 each r₂ independently is 1, 2, 3, 4, 5 or 6, each r₂ independently is preferably 1, 2, 3 or 4, each r₂ independently is more preferably 1 or 2,
preferably, L₄ is 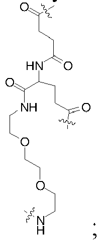
V₂ is -L_{2V}-T₂,
L_{2V} is 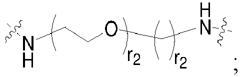 each r₂ independently is 1, 2, 3, 4, 5 or 6, each r₂ independently is preferably 1, 2, 3 or 4, each r₂ independently is more preferably 1 or 2,
preferably, L_{2V} is 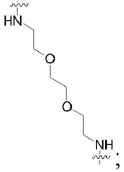
T₂ is 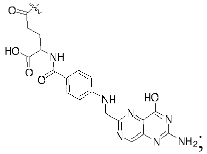
W₂ is 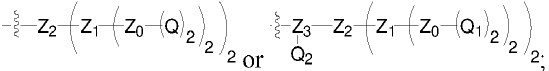
Z₃, Z₂, Z₁ and Z₀ each independently are 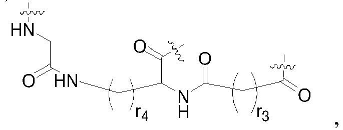 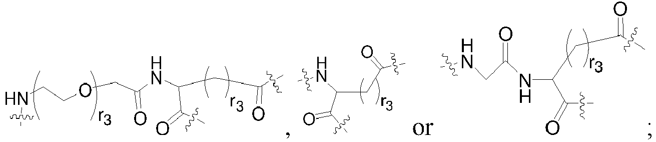 each r₃ independently is 1, 2, 3, 4, 5 or 6, each r₃ independently is preferably 1, 2, 3 or 4, each r₃ independently is more preferably 1 or 2; r₄ is 1, 2, 3 4, 5 or 6, preferably 1 2, 3 or 4, more preferably 3 or 4;
preferably, Z₃ is 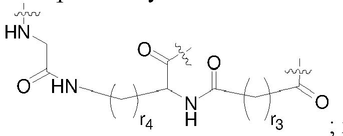 r₃ is 1 or 2; r₄ is 3 or 4;
more preferably, Z₃ is 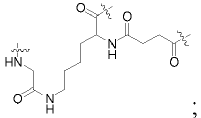
preferably, Z₂ is 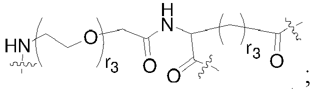 each r₃ independently is 1 or 2,
more preferably, Z₂ is 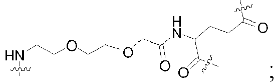
preferably, Z₁ is 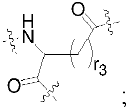 r₃ is 1 or 2,
more preferably, Z₁ is 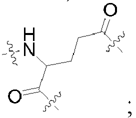
preferably, Z₀ is 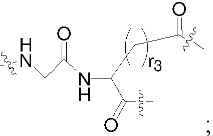 r₃ is 1 or 2,
more preferably, Z₀ is 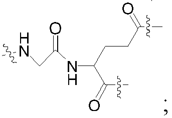
Qis -N-AC, Q1 is -N1-AC1, Q2 is -N2-AC2;
N, N1 and N2 each independently are G or GFLG, preferably, N, N1 and N2 are GFLG;
AC, AC1 and AC2 each independently are SB7, NPB, SN38, LPT, PCB, DOX, PTX or AXT, preferably, AC, AC1 and AC2 each independently are LPT or PCB, more preferably, AC1 is LPT, AC and AC2 are PCB.

3. The polyethylene glycol conjugated drug or a pharmaceutically acceptable salt thereof according to claim 1, wherein the polyethylene glycol conjugated drug having a structure of formula (II), 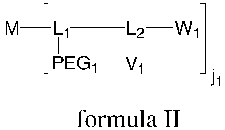 wherein:
M is selected from 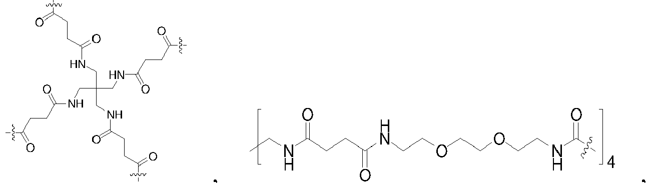 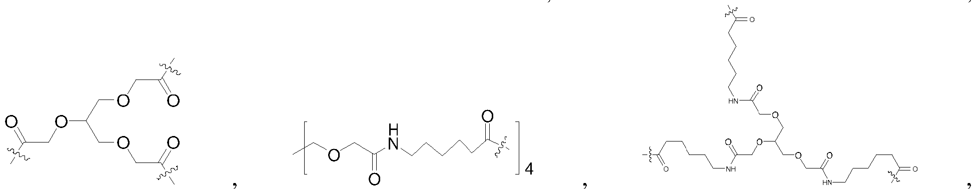 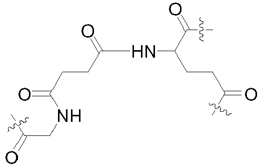 or PEGₘ; PEGₘ is a single-arm or multi-arm (such as four-arm, eight-arm, preferably four-arm) polyethylene glycol segment; PEGₘ is connected to L₁ through a carbonyl group; the number-average molecular weight of PEGₘ is 5k-40k, preferably 5k-10k or 10k-40k, more preferably 5k or 10k;
j₁ is 3 or 4;
PEG₁ is a single-arm polyethylene glycol segment; PEG₁ is connected to L₁ through a carbonyl group; the number-average molecular weight of PEG₁ is 5k-40k, preferably 5k-10k or 10k-40k, more preferably 5k, 10k or 40k;
L₁ is 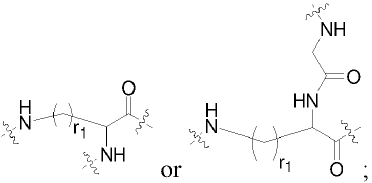 each r₁ independently is 1, 2, 3, 4, 5 or 6, each r₁ independently is preferably 1, 2, 3 or 4, each r₁ independently is more preferably 3 or 4;
preferably, L₁ is 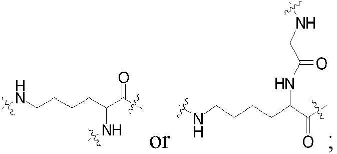
L₂ is 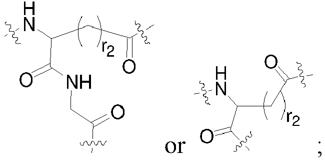 each r₂ independently is 1, 2, 3, 4, 5 or 6, each r₂ independently is preferably 1, 2, 3 or 4, each r₂ independently is more preferably 1 or 2;
preferably, L₂ is 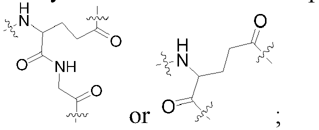
V₁ is -L_{1V}-T₁;
L_{1V} is 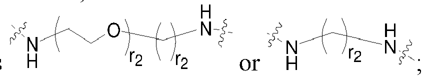 each r₂ independently is 1, 2, 3, 4, 5 or 6, each r₂ independently is preferably 1, 2, 3 or 4, each r₂ independently is more preferably 1 or 2;
preferably, Liv is 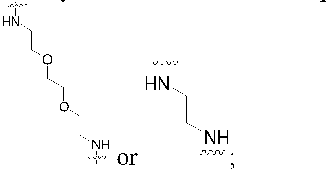
T₁ is 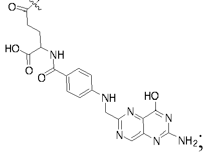 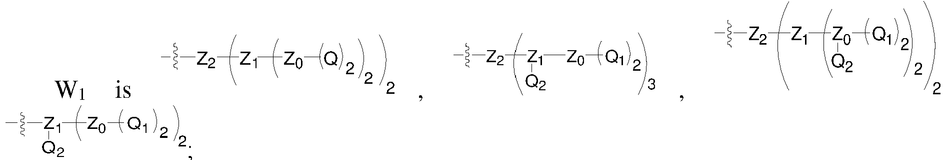 or
Z₂, Z₁ and Z₀ each independently are 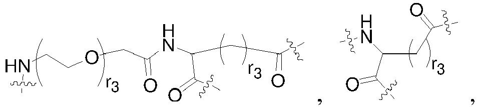 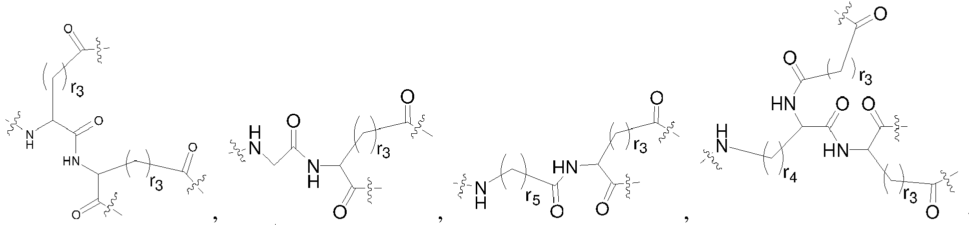 or 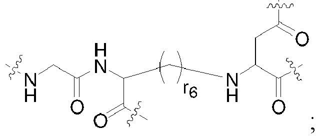 each r₃ independently is 1, 2, 3, 4, 5 or 6, each r₃ independently is preferably 1, 2, 3 or 4, each r₃ independently is more preferably 1 or 2; r₄ is 1, 2, 3, 4, 5 or 6, preferably 1, 2, 3 or 4, more preferably 3 or 4; r₅ is 1, 2, 3, 4, 5 or 6, preferably 3, 4, 5 or 6, more preferably 5 or 6; r₆ is 1, 2, 3, 4, 5 or 6, preferably 2, 3, 4 or 5, more preferably 3 or 4;
preferably, Z₂ is 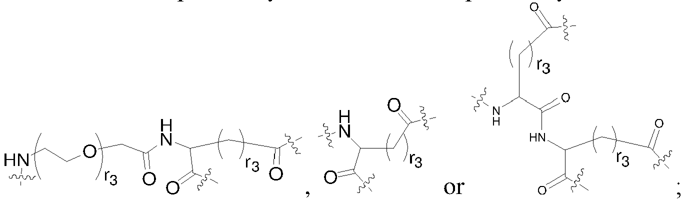 each r₃ independently is 1 or 2
more preferably, Z₂ is 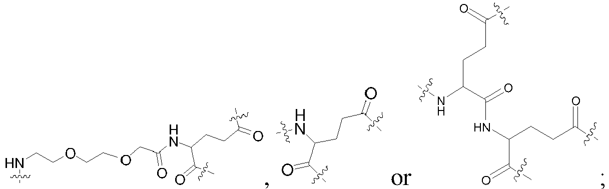
preferably, Z₁ is 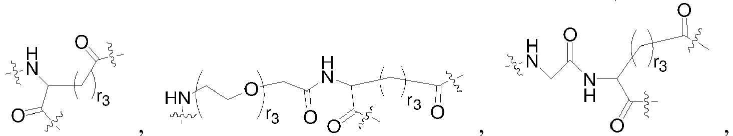 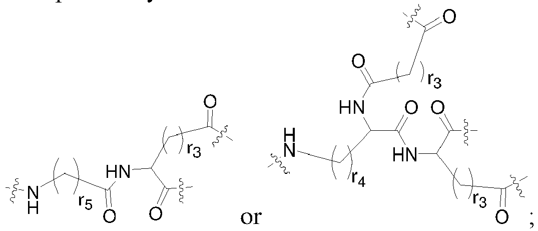 each r₃ independently is 1 or 2, r₄ is 3 or 4, r₅ is 5 or 6;
more preferably, Z₁ is 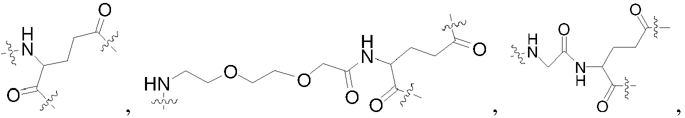 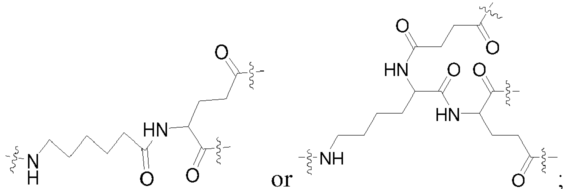
preferably, Z₀ is 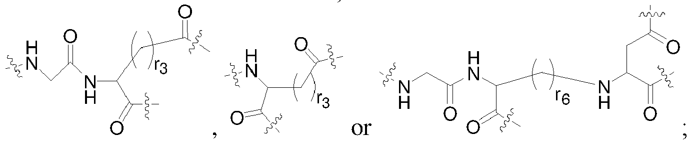 each r₃ independently is 1 or 2; r₆ is 3 or 4;
more preferably, Z₀ is 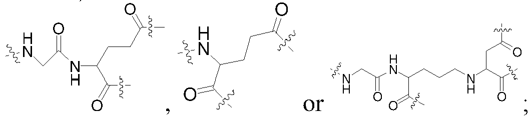
Qis -N-AC, Q1 is -N1-AC1, Q2 is -N2-AC2;
N, N1 and N2 each independently are G or GFLG;
AC, AC1 and AC2 each independently are SB7, NPB, SN38, LPT, PCB, DOX, PTX or AXT; preferably, AC, AC1 and AC2 each independently are SB7, NPB, SN38, LPT or PCB, more preferably, AC is SB7, NPB or SN38, AC1 is NPB or LPT, AC2 is PCB.

4. The polyethylene glycol conjugated drug or a pharmaceutically acceptable salt thereof according to claim 1, wherein, the polyethylene glycol conjugated drug having a structure of formula (III), 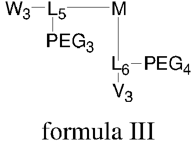 wherein:
M is 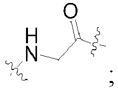
PEG₃ and PEG₄ are single-arm polyethylene glycol segments; PEG₃ and PEG₄ are connected to L₅ and L₆ through a carbonyl group respectively; each of the number-average molecular weight of PEG₃ and PEG₄ independently is 5k-40k, preferably 5k-10k or 10k-40k, more preferably 5k or 10k;
L₅ is 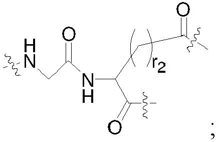 r₂ is 1, 2, 3, 4, 5 or 6, preferably 1, 2, 3 or 4, more preferably 1 or 2;
preferably, L₅ is 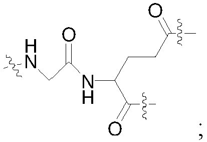
L₆ is 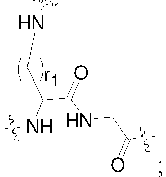 r₁ is 1, 2, 3, 4, 5 or 6, preferably 1, 2, 3 or 4, more preferably 3 or 4,
preferably, L₆ is 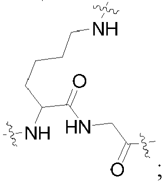
V₃ is -L_{3V}-T₃;
L_{3V} is 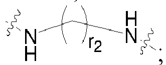 r₂ is 1, 2, 3, 4, 5 or 6, preferably 1, 2, 3 or 4, more preferably 1 or 2,
preferably, L_{3V} is 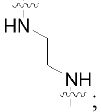
T₃ is 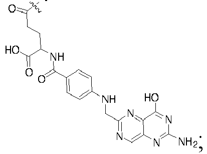
W₃ is 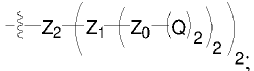
Z₂, Z₁ and Z₀ each independently are 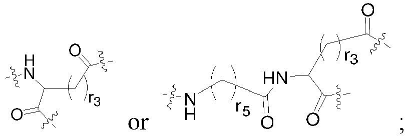 each r₃ independently is 1, 2, 3, 4, 5 or 6, each r₃ independently is preferably 1, 2, 3 or 4, each r₃ independently is more preferably 1 or 2; r₅ is 1, 2, 3, 4, 5 or 6, preferably 3, 4, 5 or 6, more preferably 5 or 6;
preferably, Z₁ and Z₂ are 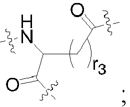 each r₃ independently is 1 or 2;
more preferably, Z₁ and Z₂ are 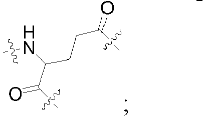
preferably, Z₀ is 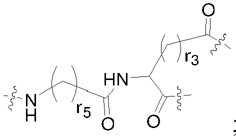 ; r₃ is 1 or 2, r₅ is 5 or 6;
more preferably, Z₀ is 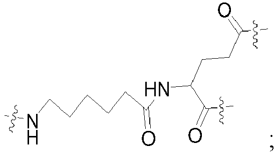
Q is -N-AC;
Nis G, 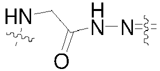 or GFLG; preferably, N is G or 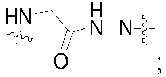
AC is SB7, NPB, SN38, LPT, PCB, DOX, PTX or AXT; preferably, AC is DOX or PTX.

5. The polyethylene glycol conjugated drug or a pharmaceutically acceptable salt thereof according to claim 1, wherein the polyethylene glycol conjugated drug having a structure of formula (IV), 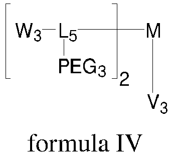 wherein:
M is 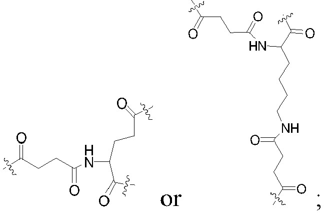
PEG₃ is a single-arm polyethylene glycol segment; PEG₃ is connected to L₅ through a carbonyl group; the number-average molecular weight of PEG₃ is 5k-40k, preferably 5k-10k or 10k-40k, more preferably 10k;
L₅ is 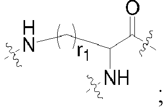 r₁ is 1, 2, 3, 4, 5 or 6, preferably 1, 2, 3 or 4, more preferably 3 or 4;
preferably, L₅ is 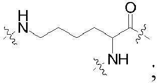
V₃ is or -P₃;
Y₀ is 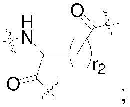 r₂ is 1, 2, 3, 4, 5 or 6, preferably 1, 2, 3 or 4, more preferably 1 or 2,
preferably, Y₀ is 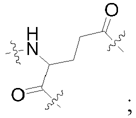
P₃ is -L_{3V}-T₃;
L_{3V} is 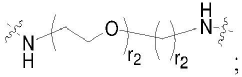 each r₂ independently is 1, 2, 3, 4, 5 or 6, each r₂ independently is preferably 1, 2, 3 or 4, each r₂ independently is more preferably 1 or 2;
preferably, L_{3V} is 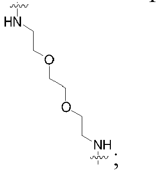
T₃ is 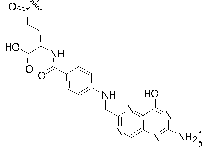
W₃ is 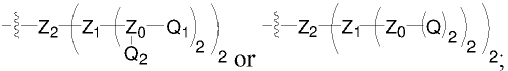
Z₂, Z₁ and Z₀ each independently are 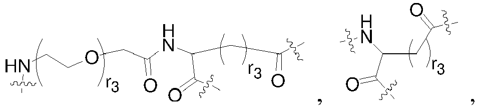 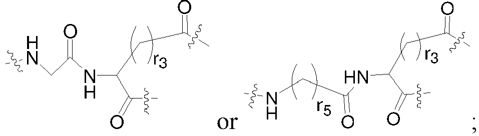 each r₃ independently is 1, 2, 3, 4, 5 or 6, each r₃ independently is preferably 1, 2, 3 or 4, each r₃ independently is more preferably 1 or 2; r₅ is 1, 2, 3, 4, 5 or 6, preferably 3, 4, 5 or 6, more preferably 5 or 6;
preferably, Z₂ is 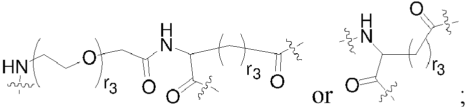 each r₃ independently is 1 or 2;
more preferably, Z₂ is 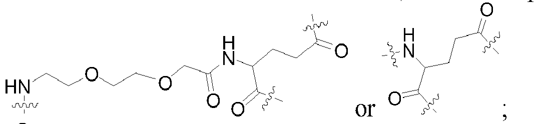
preferably, Z₁ is 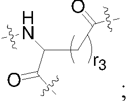 r₃ is 1 or 2;
more preferably, Z₁ is 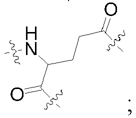
preferably, Z₀ is 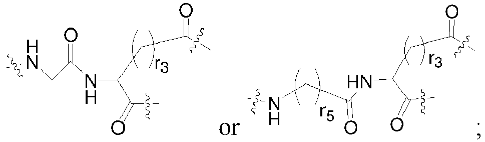 each r₃ independently is 1 or 2; r₅ is 5 or 6;
more preferably, Z₀ is 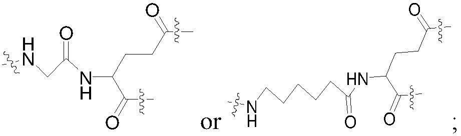
Qis -N-AC, Q1 is -N1-AC1, Q2 is -N2-AC2;
N, N1 and N2 each independently are G, GFLG or 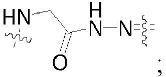 preferably, N1 and N2 are GFLG; preferably, N is G or 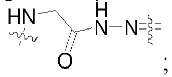
AC, AC1 and AC2 each independently are SB7, NPB, SN38, LPT, PCB, DOX, PTX or AXT; preferably, AC1 and AC2 each independently are PCB or LPT; preferably, AC is PTX or DOX.

6. The polyethylene glycol conjugated drug or a pharmaceutically acceptable salt thereof according to claim 1, wherein the polyethylene glycol conjugated drug having a structure of formula (V), 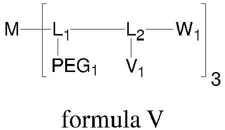 wherein:
M is 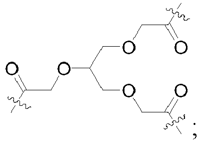
PEG₁ is a single-arm polyethylene glycol segment; PEG₁ is connected to L₁ through a carbonyl group; the number-average molecular weight of PEG₁ is 5k-40k, preferably 5k-10k or 10k-40k, more preferably 10k;
L₁ is 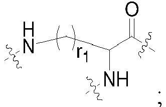 r₁ is 1, 2, 3 4, 5 or 6, preferably 1, 2, 3 or 4, more preferably 3 or 4,
preferably, L₁ is 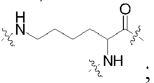
L₂ is 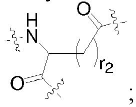 r₂ is 1, 2, 3, 4, 5 or 6, preferably 1, 2, 3 or 4, more preferably 1 or 2,
preferably, L₂ is 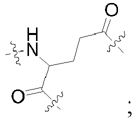
V₁ is 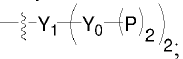
Y₁ and Y₀ are 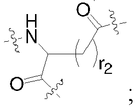 each r₂ independently is 1, 2, 3, 4, 5 or 6, each r₂ independently is preferably 1, 2, 3 or 4, each r₂ independently is more preferably 1 or 2;
preferably, Y₁ and Y₀ are 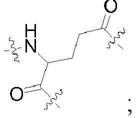
P is -Lv-T;
L_{V} is 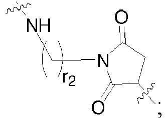 r₂ is 1, 2, 3, 4, 5 or 6, preferably 1, 2, 3 or 4, more preferably 1 or 2;
preferably, Lv is 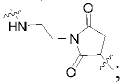
T is 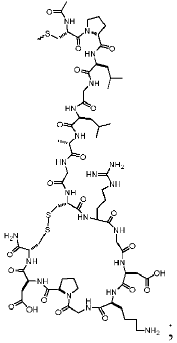
W₁ is ;
Z₁ and Z₀ each independently are 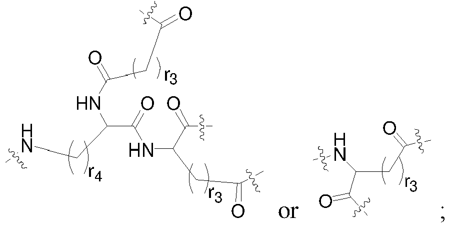 each r₃ independently is 1, 2, 3, 4, 5 or 6, each r₃ independently is preferably 1, 2, 3 or 4, each r₃ independently is more preferably 1 or 2; r₄ is 1, 2, 3, 4, 5 or 6, preferably 1, 2, 3 or 4, more preferably 3 or 4;
preferably, Z₁ is 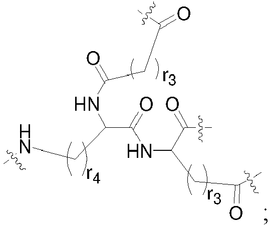 each r₃ independently is 1 or 2; r₄ is 3 or 4;
more preferably, Z₁ is 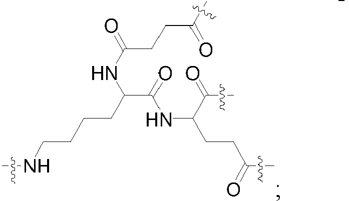
preferably, Z₀ is 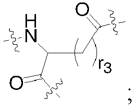 r₃ is 1 or 2;
more preferably, Z₀ is 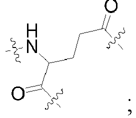
Q1 is -N1-AC1, Q2 is -N2-AC2;
N1 and N2 each independently are G or GFLG, preferably, N1 and N2 are GFLG;
AC1 and AC2 each independently are SB7, NPB, SN38, LPT, PCB, DOX, PTX or AXT; preferably, AC1 and AC2 each independently are PCB or LPT; more preferably, AC1 is LPT, and AC2 is PCB.

7. The polyethylene glycol conjugated drug or a pharmaceutically acceptable salt thereof according to claim 1, wherein the polyethylene glycol conjugated drug having a structure of formula (VI), 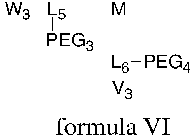 wherein:
M is 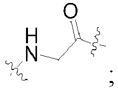
PEG₃ and PEG₄ are single-arm polyethylene glycol segments; PEG₃ and PEG₄ are connected to L₅ and L₆ through a carbonyl group respectively; each of the number-average molecular weight of PEG₃ and PEG₄ independently is 5k-40k, preferably 5k-10k or 10k-40k, more preferably 10k;
L₅ and L₆ each independently are 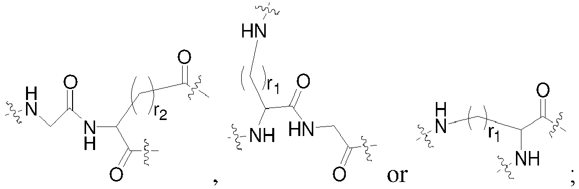 each r₁ independently is 1, 2, 3, 4, 5 or 6, each r₁ independently is preferably 1, 2, 3 or 4, each r₁ independently is more preferably 3 or 4; r₂ is 1, 2, 3, 4, 5 or 6, preferably 1, 2, 3 or 4, more preferably 1 or 2,
preferably, L₅ and L₆ each independently are 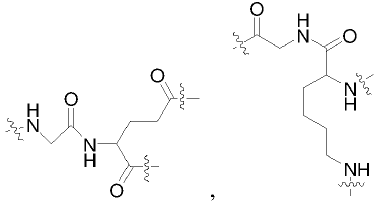 or 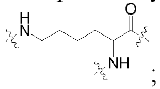
V₃ is 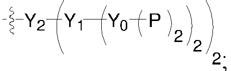
Y₂, Y₁ and Y₀ each independently are 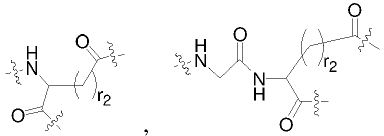 or 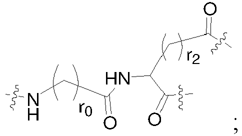 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
preferably, Y₂ and Y₁ are 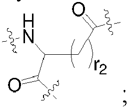 each r₂ independently is 1 or 2;
more preferably, Y₂ and Y₁ are 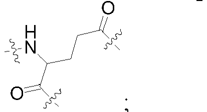
preferably, Y₀ is 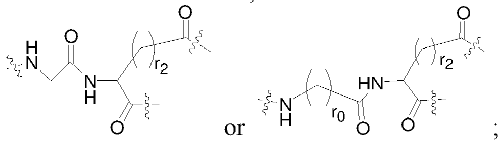 r₀ is 5 or 6; each r₂ independently is 1 or 2;
more preferably, Y₀ is 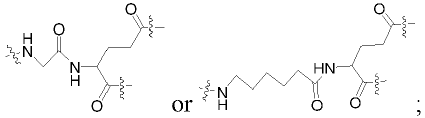
P is -Lv-T,
L_{V} is 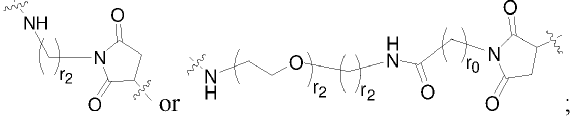 r₀ is 1, 2, 3, 4, 5 or 6, preferably 3, 4, 5 or 6, more preferably 5 or 6; each r₂ independently is 1, 2, 3, 4, 5 or 6, each r₂ independently is preferably 1, 2, 3 or 4, each r₂ independently is more preferably 1 or 2;
preferably, Lv is 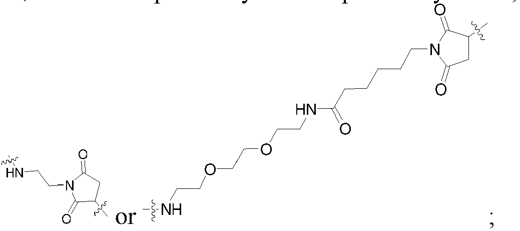
T is 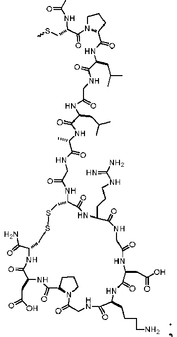
W₃ is 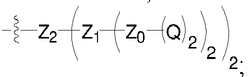
Z₂, Z₁ and Z₀ each independently are 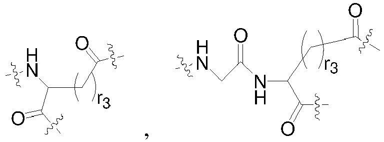 or 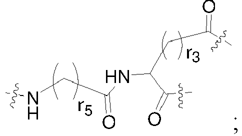 each r₃ independently is 1, 2, 3, 4, 5 or 6, each r₃ independently is preferably 1, 2, 3 or 4, each r₃ independently is more preferably 1 or 2; r₅ is 1, 2, 3, 4, 5 or 6, preferably 3, 4, 5 or 6, more preferably 5 or 6;
preferably, Z₂ is 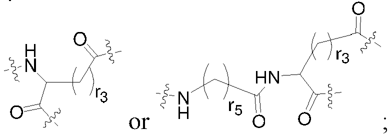 each r₃ independently is 1 or 2; r₅ is 5 or 6;
more preferably, Z₂ is 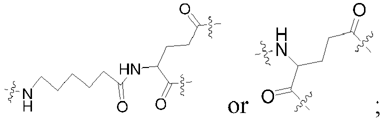
preferably, Z₁ is 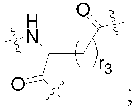 r₃ is 1 or 2;
more preferably, Z₁ is 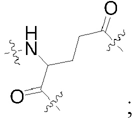
preferably, Z₀ is 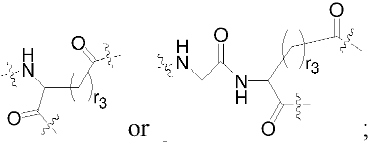 each r₃ independently is 1 or 2;
more preferably, Z₀ is 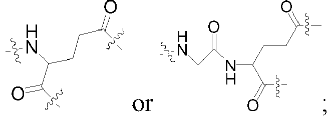
Q is -N-AC;
N is 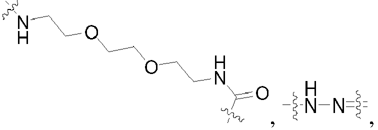 G or GFLG;
AC is SB7, NPB, SN38, LPT, PCB, DOX, PTX or AXT; preferably, AC is SB7, LPT, PTX, DOX or AXT.

8. The polyethylene glycol conjugated drug or a pharmaceutically acceptable salt thereof according to claim 1, wherein the polyethylene glycol conjugated drug is selected from:
| Numbe r | Structure | | | |
|---|---|---|---|---|
| 37-184 | 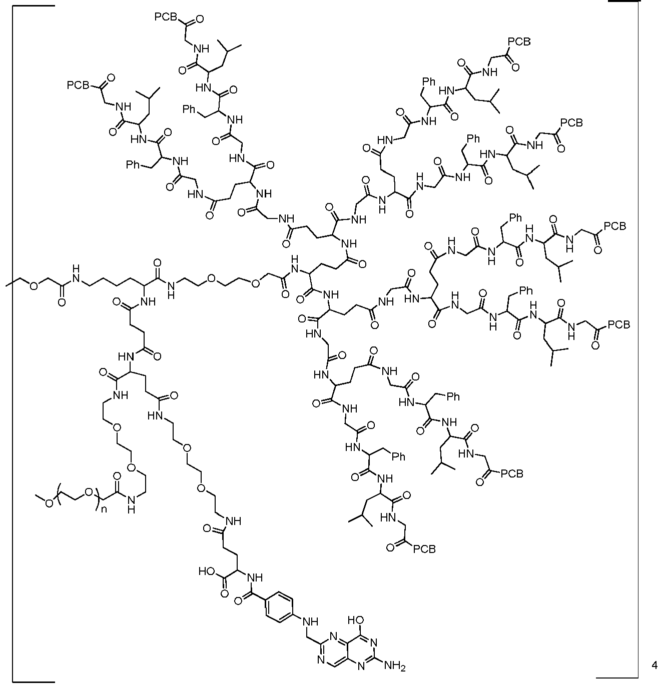 | | | |
| | wherein the number-average molecular weight of | 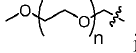 | is 10k; | |
| 36-257 | 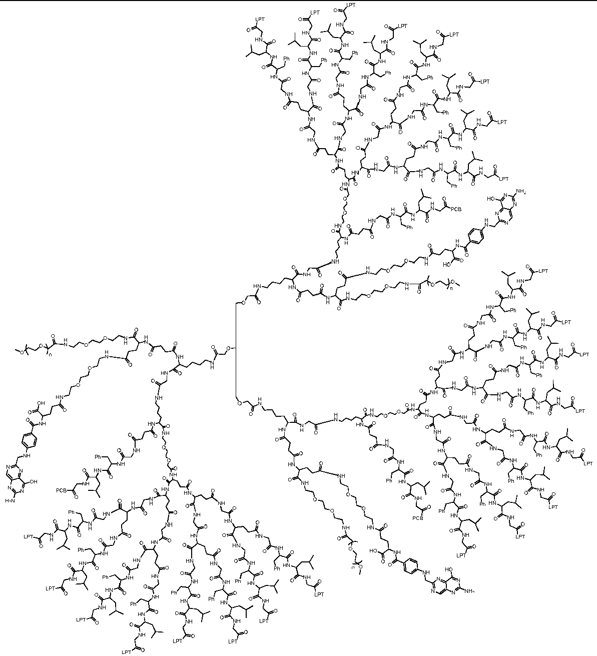 | | | |
| | wherein the number-average molecular weight of | 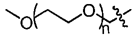 | is 10k; | |
| 43-154 | | | | |
| | 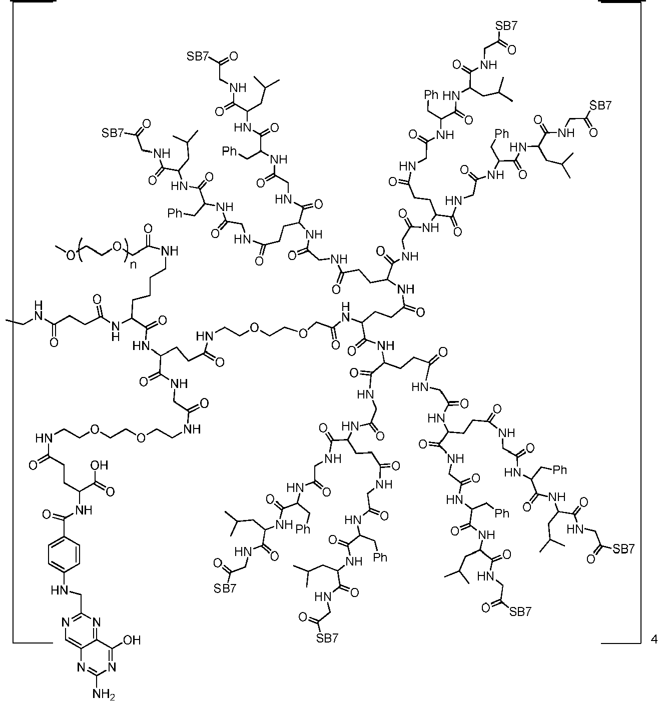 | | | |
| | wherein the number-average molecular weight of | 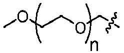 | is 10k; | |
| 37-221 | 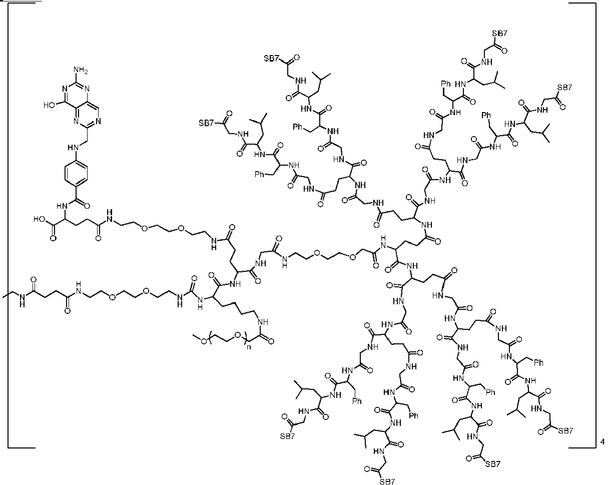 | | | |
| | wherein the number-average molecular weight of | 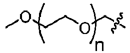 | is 10k; | |
| 44-174 | | | | |
| | 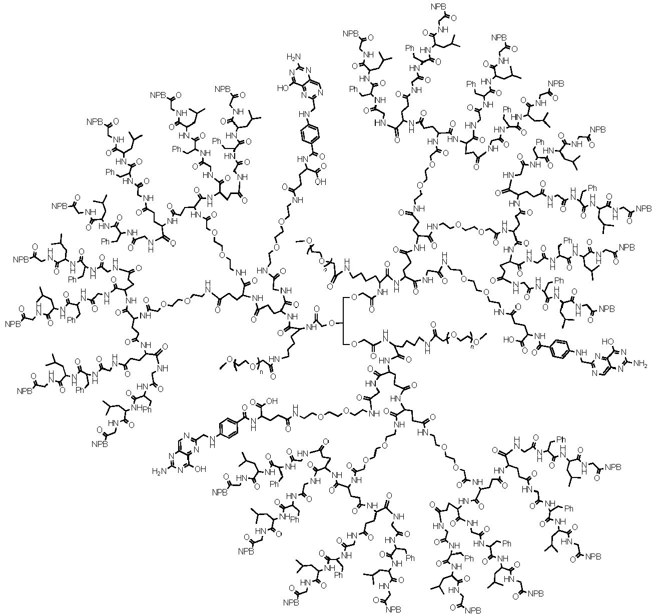 | | | |
| | wherein the number-average molecular weight of | 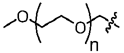 | is 10k; | |
| 49-199 | 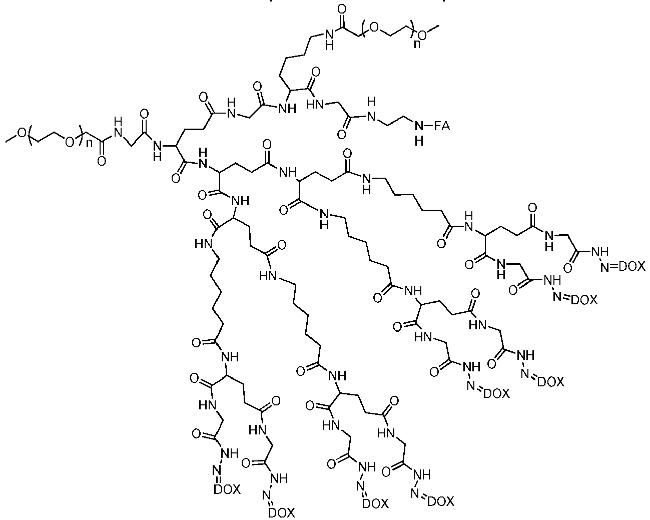 | | | |
| | wherein the number-average molecular weight of | 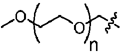 | is 5k; | |
| 47-122 | 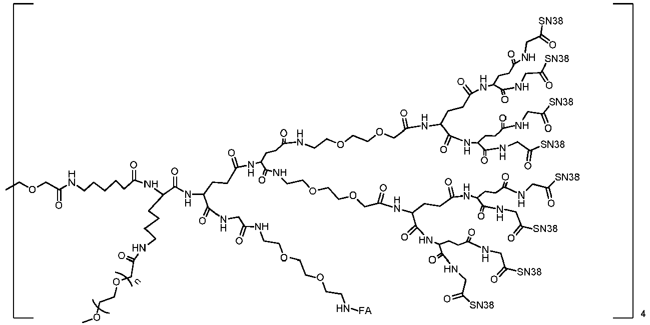 | | | |
| | wherein the number-average molecular weight of | 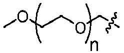 | is 5k; | |
| 39-226 | 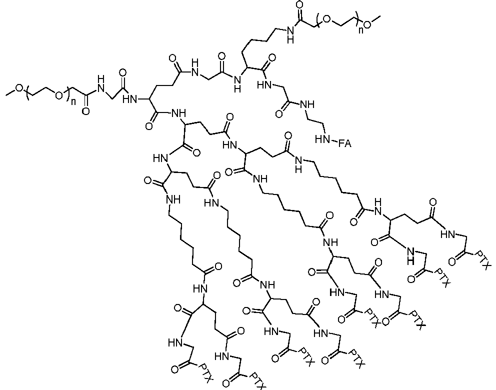 | | | |
| | wherein the number-average molecular weight of | 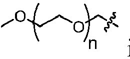 | is 10k; | |
| 45-145 | 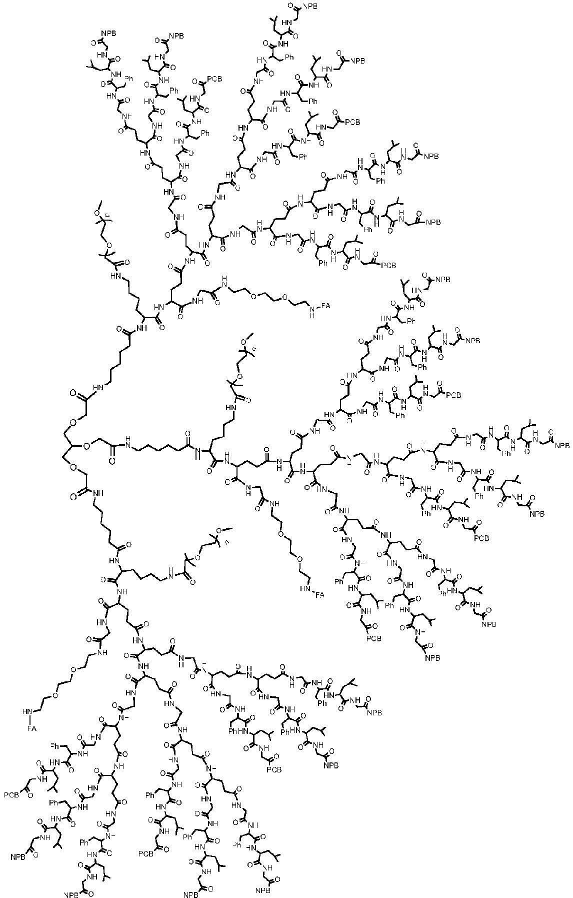 | | | |
| | wherein the number-average molecular weight of | 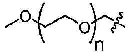 is | 10k; | |
| 39-138 | 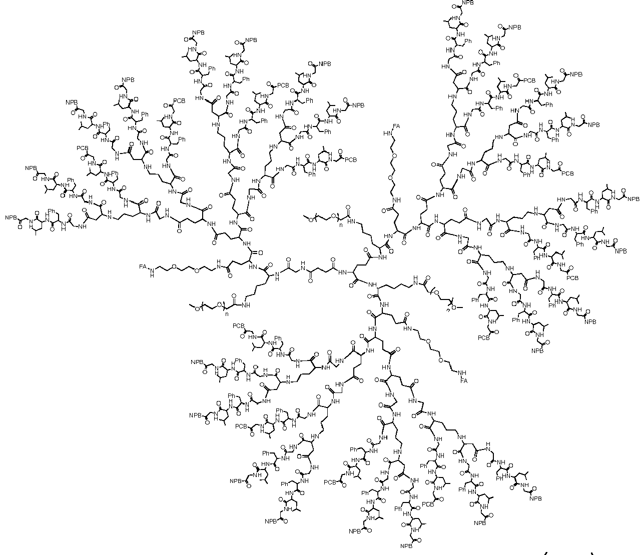 | | | |
| | wherein the number-average molecular weight of | 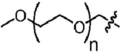 | is 10k; | |
| 45-80 | 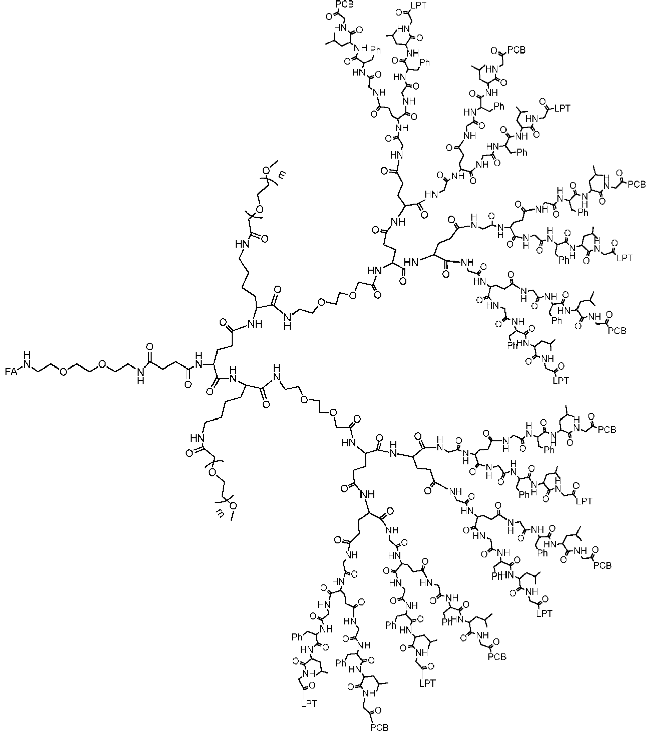 | | | |
| | wherein the number-average molecular weight of | 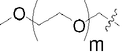 | is 10k; | |
| 49-137 | 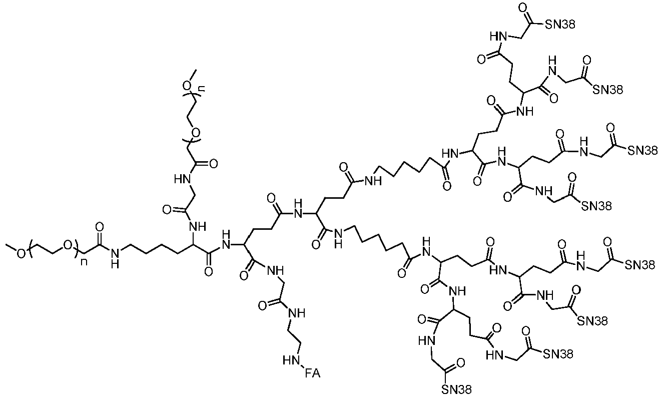 | | | |
| | wherein the number-average molecular weight of | | 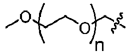 | is 5k; |
| 43-197 | 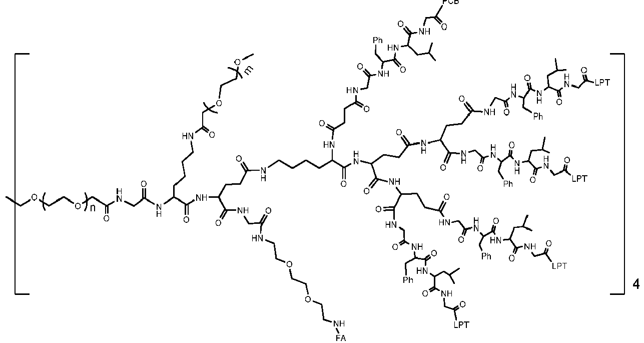 | | | |
| | wherein the number-average molecular weight of | | 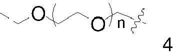 | is 10k, and the |
| | number-average molecular weight of is | 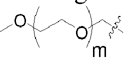 | 5k; | |
| 50-47 | 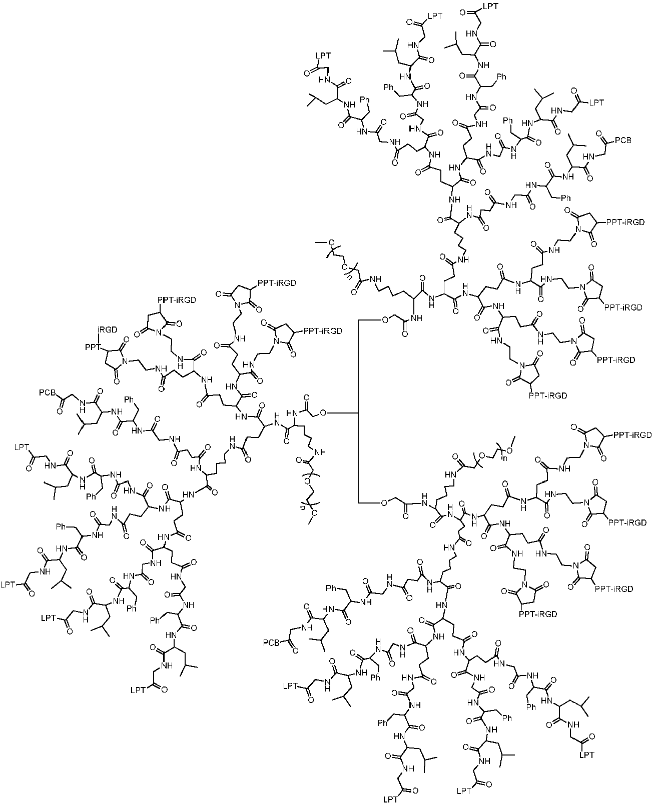 | | | |
| | wherein the number-average molecular weight of | 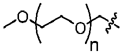 | is 10k; | |
| 50-65 | 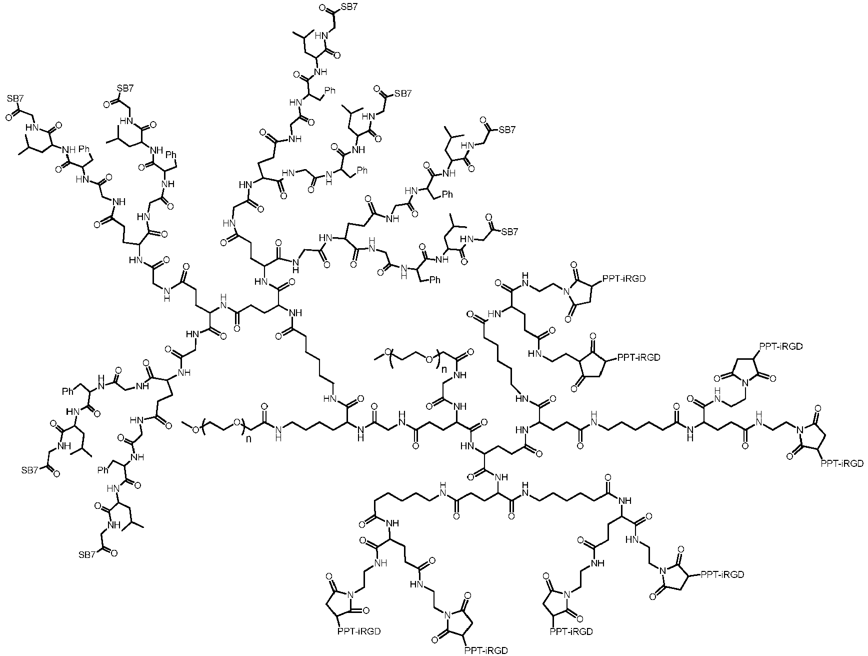 | | | |
| | wherein the number-average molecular weight of | 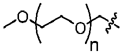 | is 10k; | |
| 60-43 | 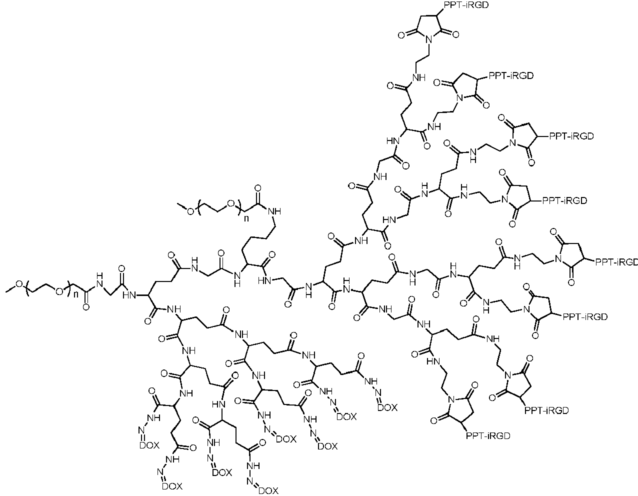 | | | |
| | wherein the number-average molecular weight of | 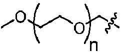 | is 10k; | |
| 48-124 | 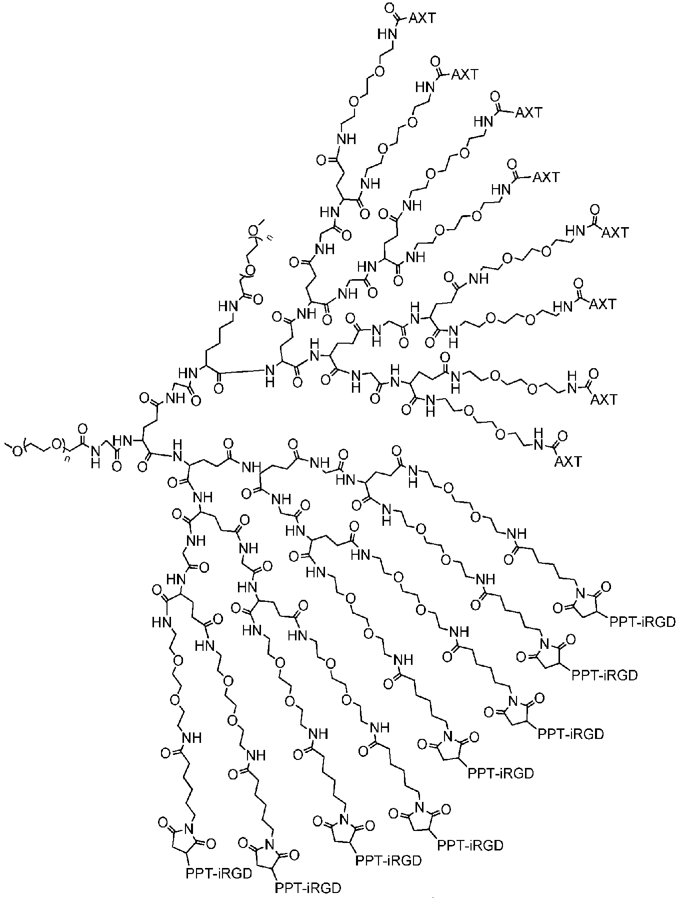 | | | |
| | wherein the number-average molecular weight of | 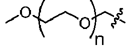 | is 10k; | |
| 43-226 | 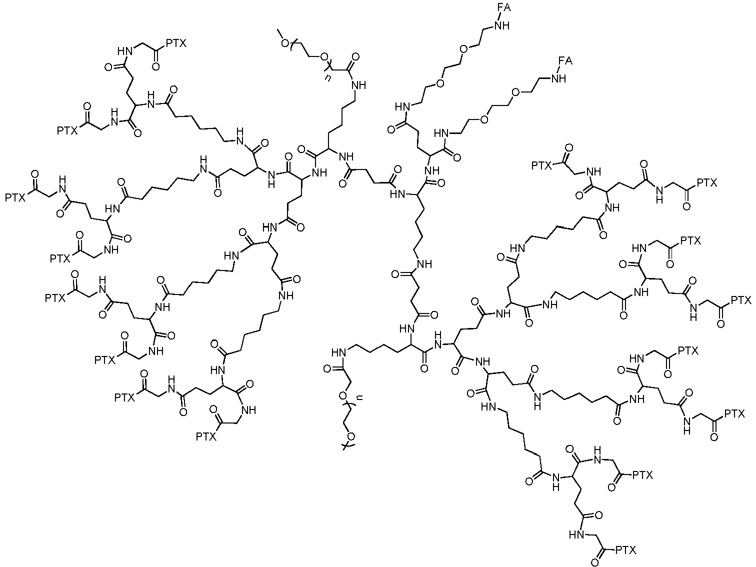 | | | |
| | wherein the number-average molecular weight of | 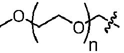 | is 10k; | |
| 51-103 | 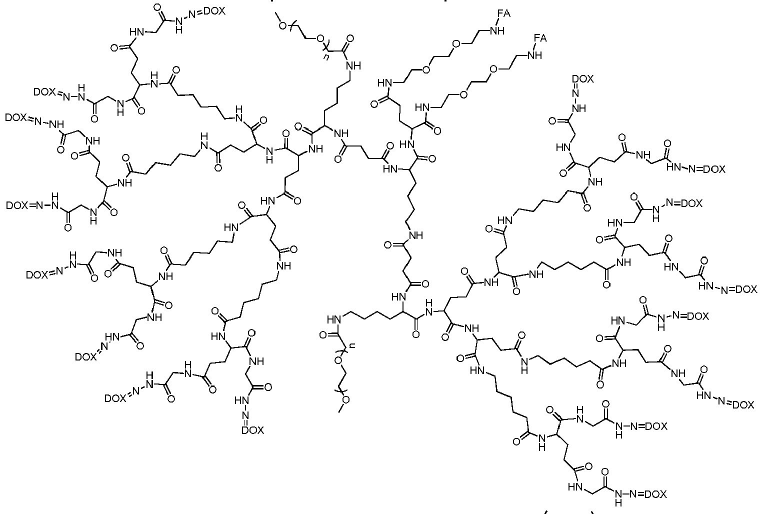 | | | |
| | wherein the number-average molecular weight of | 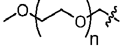 | is 10k. | |

9. An intermediate for preparing the polyethylene glycol conjugated drug or a pharmaceutically acceptable salt thereof according to any one of claims 1-8, the intermediate being selected from:
| Number | Structure |
|---|---|
| 37-166 | 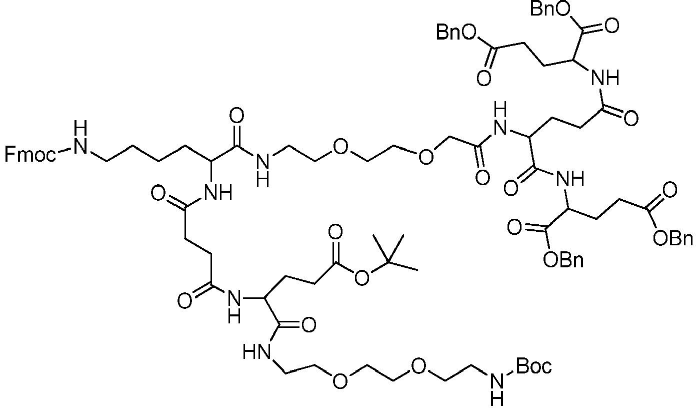 |
| 36-144 | 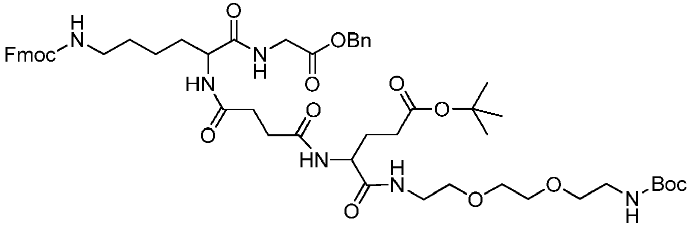 |
| 43-140 | 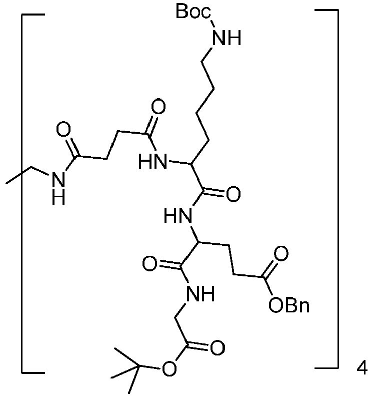 |
| 43-135 | 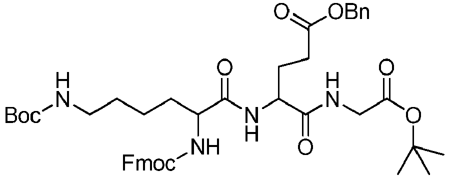 |
| 37-212 | 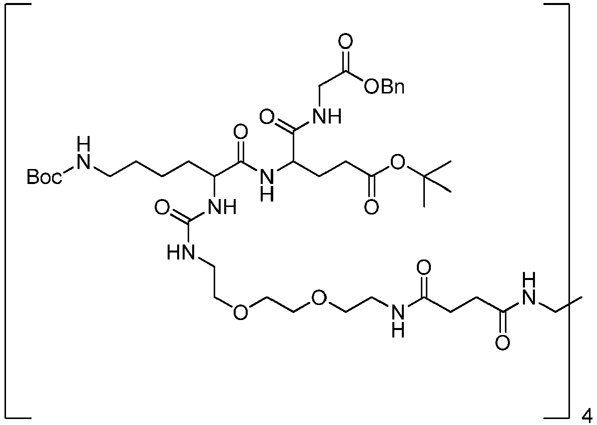 |
| 44-140 | 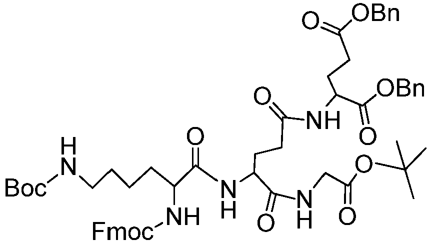 |
| 44-150 | 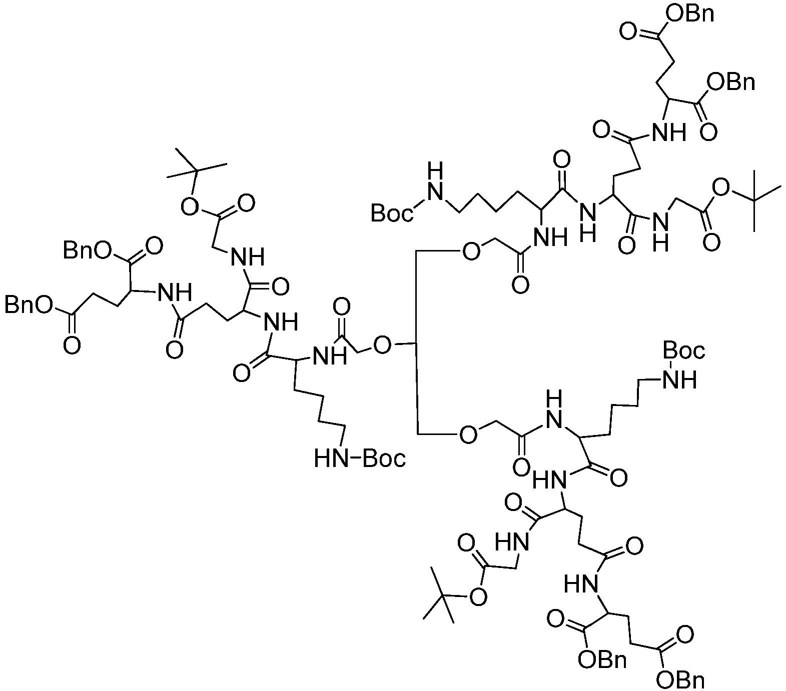 |
| 49-25 | 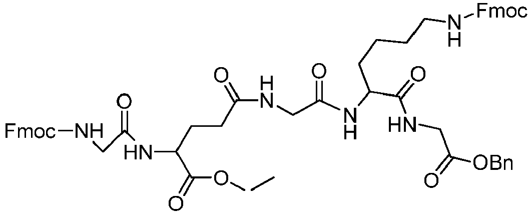 |
| 47-36 | 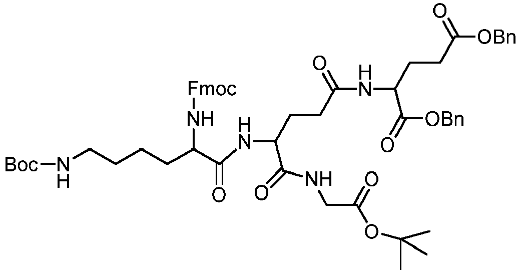 |
| 47-39 | 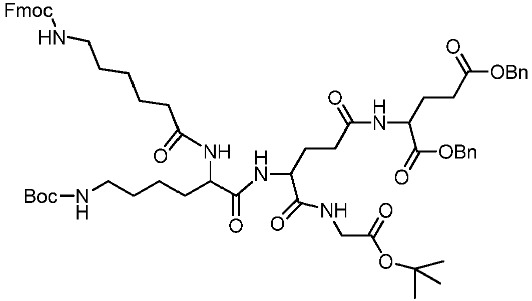 |
| 47-44 | 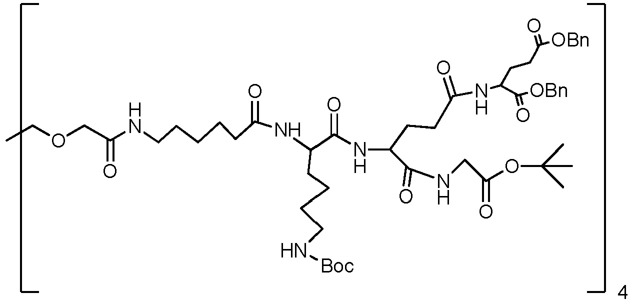 |
| 49-44 | 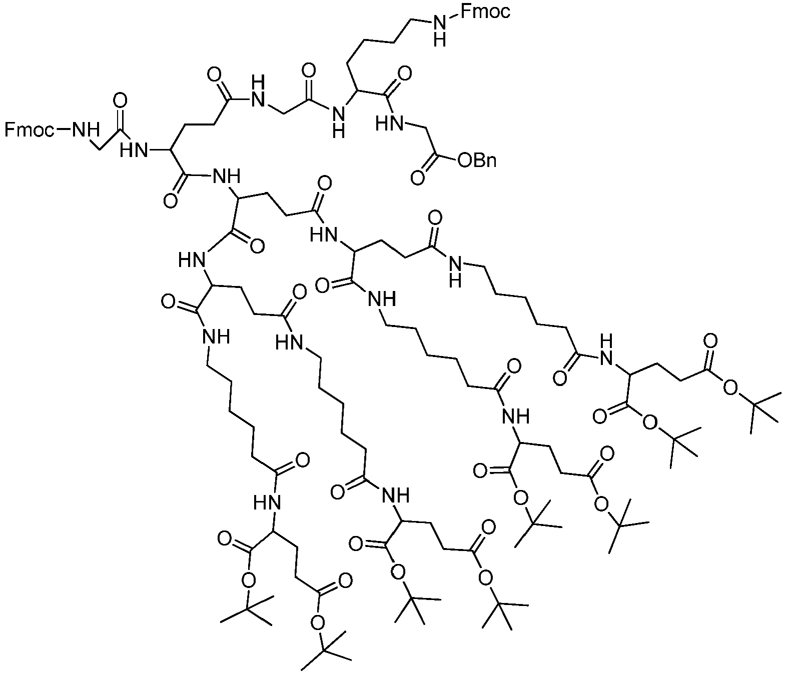 |
| 45-95 | 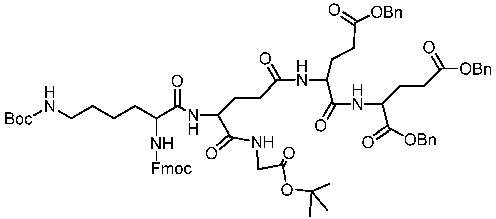 |
| 45-105 | 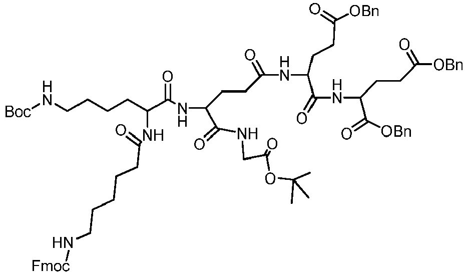 |
| 45-122 | 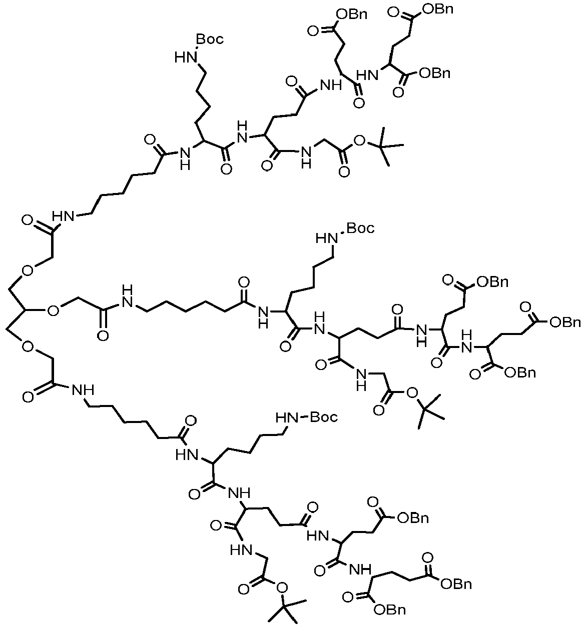 |
| 39-92 | 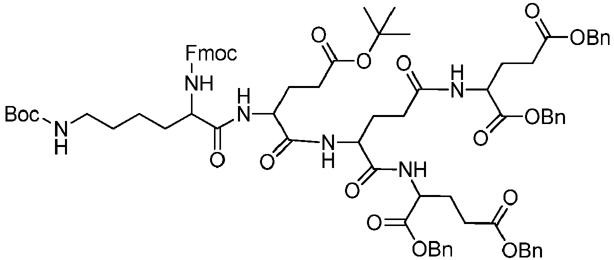 |
| 39-97 | 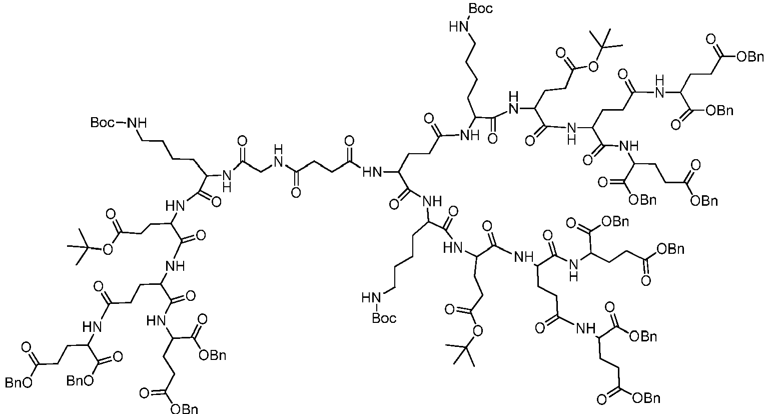 |
| 45-50 | 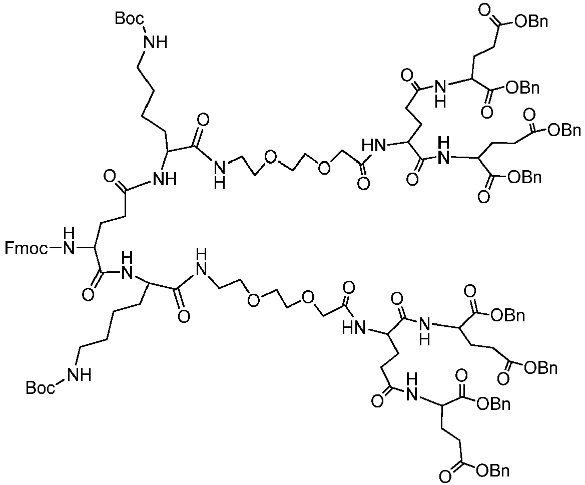 |
| 45-54 | 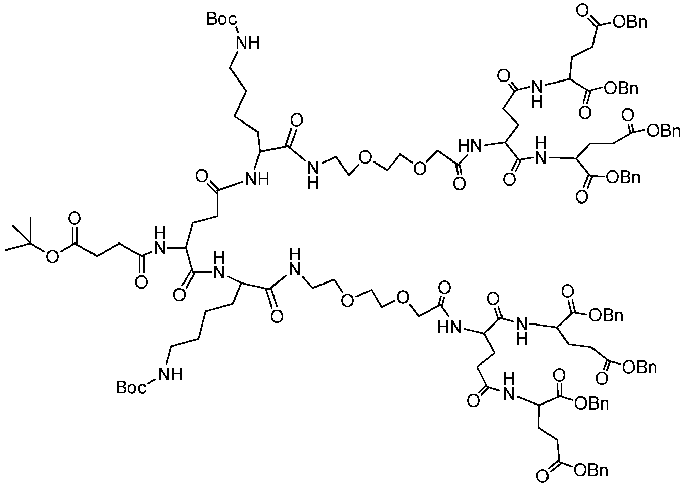 |
| 49-100 | 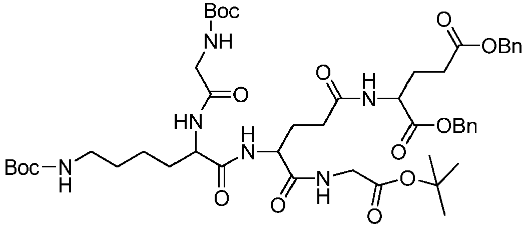 |
| 49-103 | 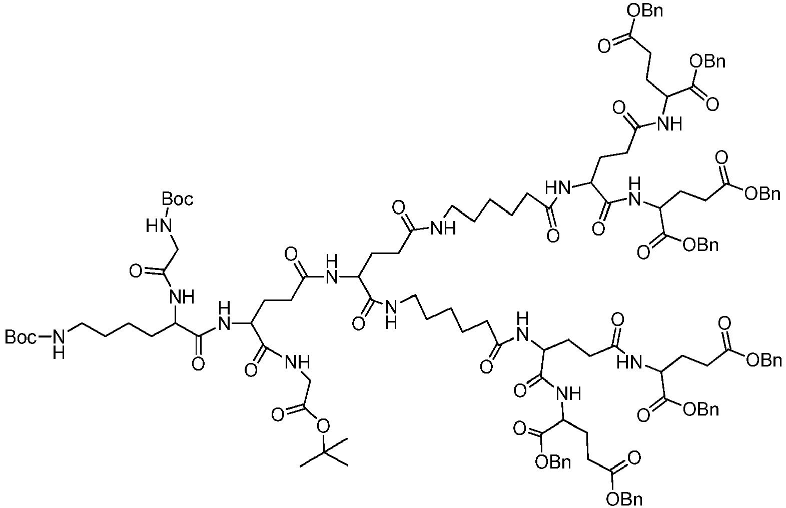 |
| 44-239 | 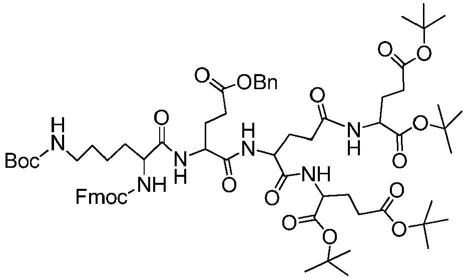 |
| 44-245 | 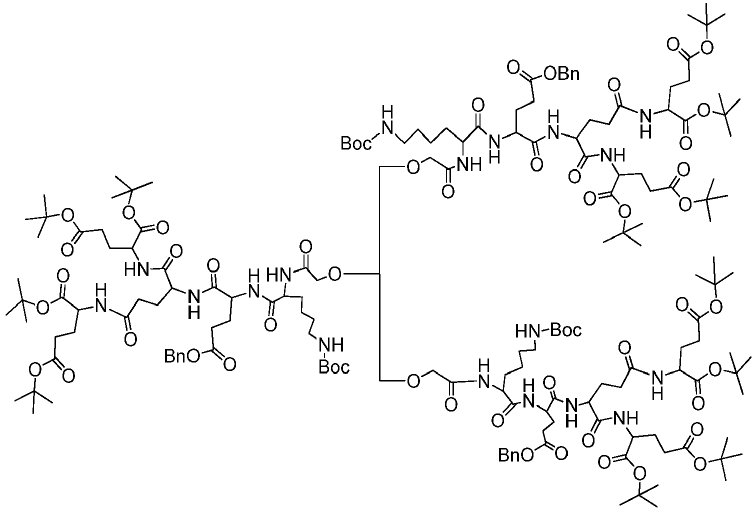 |
| 50-44 | 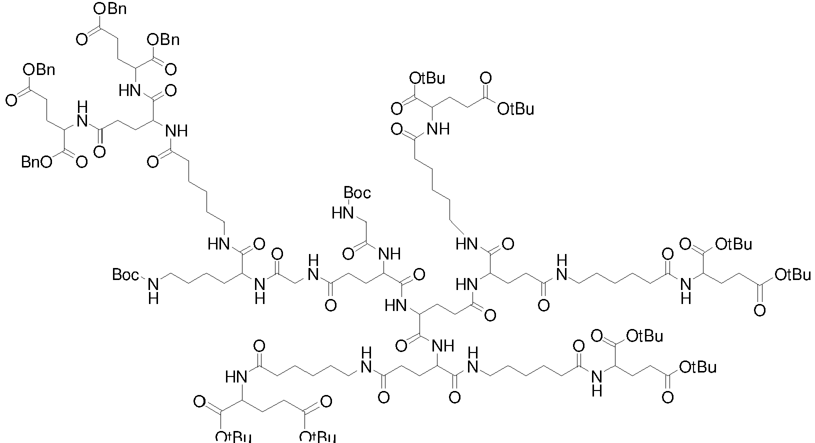 |
| 49-244 | 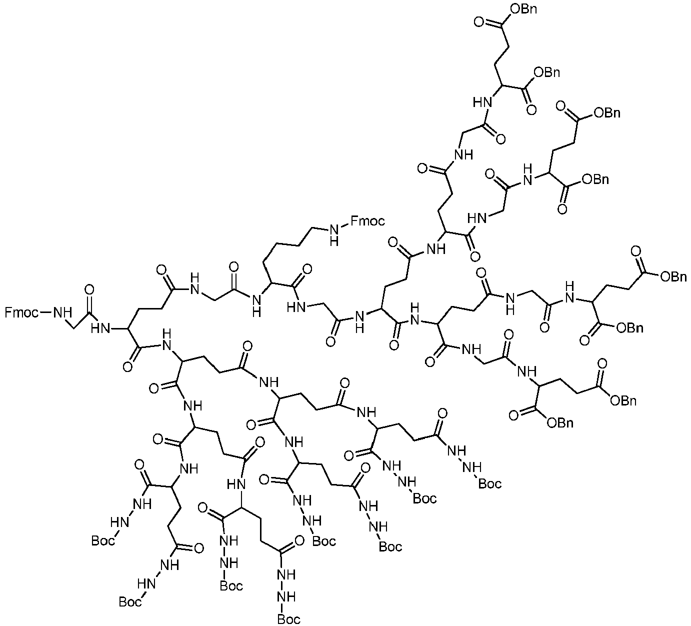 |
| 48-97 | 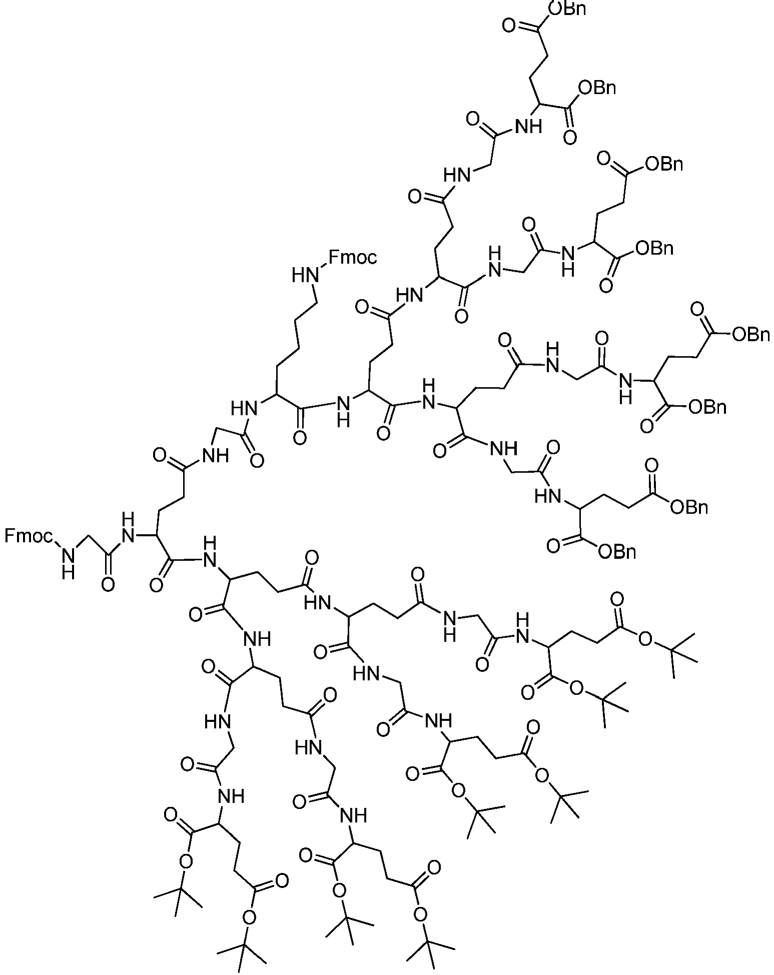 |

10. A method for preparing the polyethylene glycol conjugated drug or a pharmaceutically acceptable salt thereof according to claim 2, comprising the following steps:
(1) preparing the intermediate 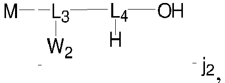 wherein M L₃ L₄, W₂ and j₂ are as defined in claim 2, and 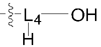 has carboxyl group and amino group at its terminal;
preparing the intermediate V₂-H, wherein, V₂ is as defined in claim 2, and V₂-H has amino group at its terminal;
(2) allowing PEG with carboxyl group or activated carboxyl group and the intermediate 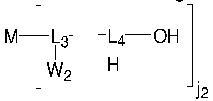 to carry out amidation reaction, to obtain the intermediate 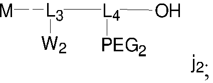 and
(3) allowing the intermediate V₂-H with amino group and the intermediate 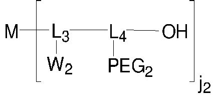 to carry out amidation reaction, to obtain the polyethylene glycol conjugated drug according to claim 2.

11. A method for preparing the polyethylene glycol conjugated drug or a pharmaceutically acceptable salt thereof according to claim 3, comprising the following steps:
(1) preparing the intermediate 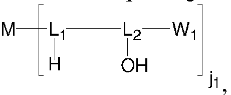 wherein M L₁, L₂, W₁ and j₁ are as defined in claim 3, and 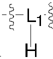 has amino group at its terminal, 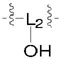 has carboxyl group at its terminal;
preparing the intermediate V₁-H, wherein, V₁ is as defined in claim 3, and V₁-H has amino group at its terminal;
(2) allowing PEG with carboxyl group or activated carboxyl group and the intermediate 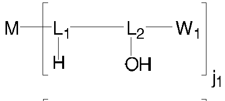 to carry out amidation reaction, to obtain the intermediate 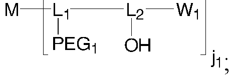 and
(3) allowing the intermediate V₁-H with amino group and the intermediate 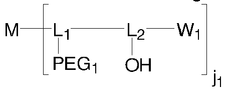 to carry out amidation reaction, to obtain the polyethylene glycol conjugated drug according to claim 3.

12. A method for preparing the polyethylene glycol conjugated drug or a pharmaceutically acceptable salt thereof according to claim 5, comprising the following steps:
(1) preparing the intermediate 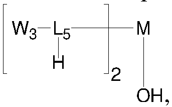 wherein, M, L₅ and W₃ are as defined in claim 5, and 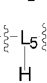 has amino group at its terminal, 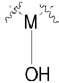 has carboxyl group at its terminal;
preparing the intermediate V₃-H, wherein, V₃ is as defined in claim 5, and V₃-H has amino group at its terminal;
(2) allowing PEG with carboxyl group or activated carboxyl group and the intermediate 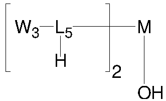 to carry out amidation reaction, to obtain the intermediate 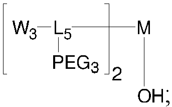 and
(3) allowing the intermediate V₃-H with amino group and the intermediate 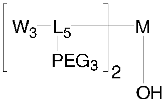 to carry out amidation reaction, to obtain the polyethylene glycol conjugated drug according to claim 5.

13. A method for preparing the polyethylene glycol conjugated drug or a pharmaceutically acceptable salt thereof according to claim 6, comprising the following steps:
(1) preparing the intermediate 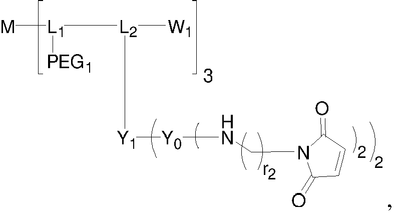 wherein, M, L₁, L₂, PEG₁, W₁, Y₁, Y₀ and r₂ are as defined in claim 6; and
(2) allowing the intermediate 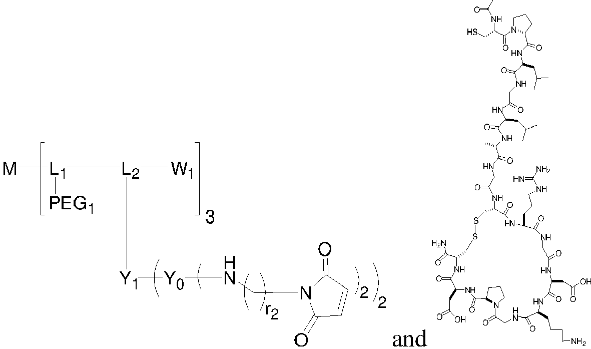 to carry out addition reaction, to obtain the polyethylene glycol conjugated drug according to claim 6.

14. A method for preparing the polyethylene glycol conjugated drug or a pharmaceutically acceptable salt thereof according to claim 7, comprising the following steps:
(1) preparing the intermediate 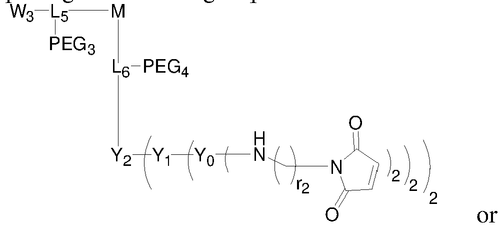 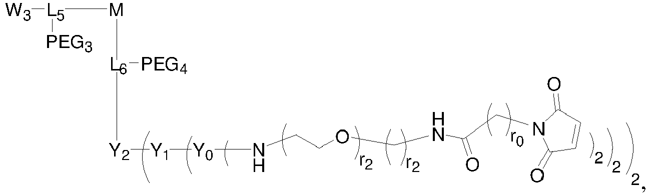 wherein, M, L₅, L₆, PEG₃, PEG₄, W₃, Y₂, Y₁, Y₀, r₀ and r₂ are as defined in claim 7; and
(2) allowing the intermediate 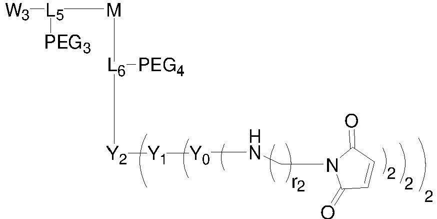 or 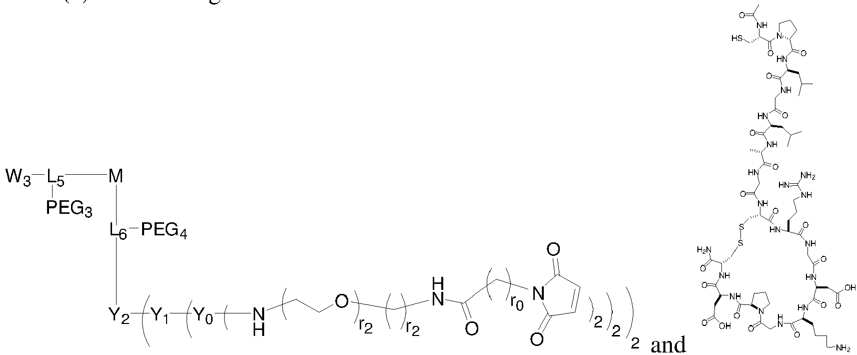 to carry out addition reaction, to obtain the polyethylene glycol conjugated drug according to claim 7.

15. A pharmaceutical composition comprising the polyethylene glycol conjugated drug or a pharmaceutically acceptable salt thereof according to any one of claims 1-8; optionally, the composition further comprises one or more pharmaceutically acceptable excipients.

16. Use of the polyethylene glycol conjugated drug or a pharmaceutically acceptable salt thereof according to any one of claims 1-8 in the preparation of a medicament for treating and/or preventing a disease, wherein the disease refers to a disease treated by the active ingredient in the polyethylene glycol conjugated drug;
preferably, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.
